# EUROPEAN PATENT APPLICATION

(11) **EP 3 974 427 A1**
(43) Date of publication of application: **30.03.2022**
(21) Application number: 20808745.2
(22) Date of filing: 19.05.2020
(51) Int. Cl.: C07D 471/04, A61K 31/519, A61P 35/00, A61P 19/02, A61P 29/00

(54) **PYRIDO-PYRIMIDIN DERIVATIVES AND PHARMACEUTICAL COMPOSITION, FOR USE IN PREVENTING OR TREATING PI3 KINASE RELATED DISEASES, COMPRISING SAME AS ACTIVE INGREDIENT**

(30) Priority: 20.05.2019 KR 20190058895
(71) Applicant: Boryung Pharmaceutical Co., Ltd., Seoul 03127 (KR)
(72) Inventor: KIM, Hak Do, Gunpo-Si Gyeonggi-do 15815 (KR); LEE, Seong Guk, Suwon-Si Gyeonggi-do 16336 (KR); LEE, Hee Jin, Ansan-si Gyeonggi-do 15560 (KR); CHOUNG, Won Ken, Seoul 06361 (KR); YANG, Deok Mo, Siheung-Si Gyeonggi-do 15033 (KR); KIM, Seong Heon, Seoul 07981 (KR); LEE, Suk Ho, Hwaseong-si Gyeonggi-do 18379 (KR)
(74) Representative: HGF
(86) International application number: PCT/IB2020/054719
(87) International publication number: WO 2020/234756

(57) **Abstract**

The present invention relates to pyrido-pyrimidin derivatives expressed by chemical formula (X), an isomer thereof or pharmaceutically acceptable salts thereof, a solvate or hydrate thereof, a pharmaceutical composition comprising same as an active ingredient, and the use thereof.

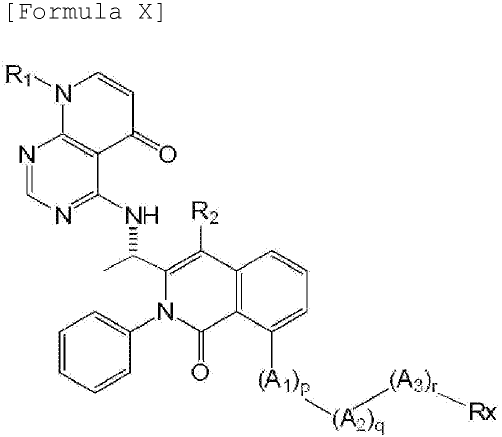

## Description

### [Technical Field]

The present invention relates to a pyrido-pyrimidine derivative, a pharmaceutical composition including the same as an effective ingredient, and a use thereof.

### [Background Art]

Phosphatidylinositol 3-kinase (PI3 kinase; PI3K) is a lipid kinase, which phosphorylates lipid molecules instead of proteins, and plays an important role in cell survival, signal transduction, control of membrane trafficking, etc. If a problem occurs to such control, a cancer, inflammatory disease, autoimmune disease, etc. occurs.

Cell signaling via 3'-phosphorylated phosphoinositide is involved in various cellular processes, for example, malignant cell transformation, growth factor signaling, inflammation, and immunity. PI3 kinase, which is an enzyme responsible for generating these phosphorylated signaling products, has been confirmed to have an activity associated with a viral oncoprotein and growth factor receptor tyrosine kinase, which phosphorylates phosphatidylinositol (PI) and phosphorylated derivatives thereof at 3'-OH of an inositol ring.

The amount of phosphatidylinositol-3,4,5-triphosphate (PIP3), which is a primary product of PI3K activation, increases when cells are treated with various stimuli. This includes signaling through receptors for most growth factors and multiple inflammatory stimuli, hormones, neurotransmitters and antigens, and thus activation of PI3K represents one of signaling associated with the activation of cell surface receptors in mammals, if not the most predominant. Thus, PI3K activation is involved in a wide range of cellular responses including cell growth, migration, differentiation, and apoptosis.

PI3K is an enzyme that phosphorylates position no. 3 (3-OH) of the inositol ring moiety of phosphatidylinositol by using adenosine triphosphate (ATP). Specifically, PI3K phosphorylates the 3'-OH position of the inositol ring of phosphatidyl inositide to phosphorylate PIP2 to PIP3, and this PIP3 serves as an anchoring site of protein kinases including pleckstrin homology. These protein kinases in turn regulate important cellular functions. Among the PIP3-binding protein kinases, what is most important is AKT or protein kinase B (PKB), which is serine/threonine kinase, and regulates the growth, survival, division, etc., of cells through downstream mTOR, GSK3β, Foxo 3a, p70S6K and NF-κB.

Through initial purification and molecular cloning of PI3K, it was found that PI3K is a heterodimer consisting of p85 and p110 subunits. Based on sequence homology and substrate specificity, there is class I, which is classified into class 1A and class IB.

Class 1A includes PI3Kα, PI3Kβ, and PI3Kδ, and class 1A is downstream of receptor tyrosine kinase (RTK). Class IB includes PI3Kγ, which is downstream of the G protein coupled receptor. Each consists of a separate 110 kDa catalytic subunit and a regulatory subunit.

More specifically, three catalytic subunits, that is, p110α, p110β and p110δ, include an ATP binding domain, each interacting with the same regulatory subunit p85 and being activated by receptor tyrosine kinase. In contrast, in case of PI3Kγ, p110γ interacts with another regulatory subunit p101 and is activated by the heterotrimeric G-protein. A regulatory domain includes a domain that allows anchoring to a cell surface receptor.

When ATP binding is inhibited, phosphorylation of PIP2 is inhibited, and PIP3 is not produced. Then, this prevents important regulatory proteins such as AKT from anchoring to the cell membrane, resulting in non-functioning. Thus, inhibition of this catalytic subunit and ATP binding site thereof is one of the main targets for drug development.

As described below, the expression patterns of each of these PI3Ks in human cells and tissues are also quite different. PI3Kα and PI3Kβ have a wide tissue distribution, whereas PI3Kγ is mainly expressed in leukocytes, but is also found in skeleton muscle, liver, pancreas and heart. PI3Kδ is expressed only in spleen, thymus, and peripheral blood lymphocytes. Judging from this expression pattern, PI3Kα and PI3Kβ have a high correlation with cancer, and PI3Kγ and PI3Kδ are highly correlated with adaptive immune systems such as rheumatoid arthritis (RA), systemic lupus erythematosus (SLE) and hematologic malignancy.

Specifically, mutations in p110α have been identified in several solid tumors. For example, amplification mutations in alpha are associated with 50% of ovarian cancer, cervical cancer, lung cancer and breast cancer, and activation mutations are associated with at least 50% of intestinal cancer and at least 25% of breast cancer. p110β is involved in thrombus formation, and compounds related to p110γ are being developed as immunosuppressive agents for autoimmune diseases, which include rheumatoid arthritis, systemic lupus erythematosus or the like.

In addition, it was found that p110δ may be used to play a key role in B and T cell activation and, furthermore, δ is also partially involved in neutrophil migration and ready neutrophil respiratory burst, causing a partial blocking of antigen-IgE-mediated mast cell degranulation. Thus, p110δ is emerging as an important mediator of a number of key inflammatory responses known to be involved in abnormal inflammatory diseases including, but not limited to, autoimmune diseases and allergies. In support of this concept, there is a growing amount of data for the evaluation of p110δ targets obtained from studies using both genetic tools and pharmacological agents. In addition, it was found that inhibition of delta significantly ameliorates inflammation and disease in a murine asthma model using ovalbumin-induced airway inflammation. Rituximab and belimumab, which are monoclonal antibodies of PI3Kδ, have a great effect on RA and SLE, respectively.

In addition, it has recently been found that PI3K is involved in lung and ear infections. Although the mechanism has not yet been fully identified, the overexpressed p110δ-AKT-mTOR pathway accelerates aerobic glycosis and degrades the function and survival of lymphocytes, thereby lowering an immune response. Although chronic inflammation is not unique to autoimmune diseases, it was found that the level of PI3Kδ and phosphorylated-AKT rises in chronic obstructive pulmonary disease (COPD). This means that high-level expression of PI3Kδ and phosphorylated-AKT is strongly associated with inflammation as well as immune diseases.

Accordingly, it is suggested that inhibition of PI3Kδ may be used not only in the treatment of autoimmune diseases such as rheumatoid arthritis (RA) and systemic lupus erythematosus (SLE), but also in the treatment of chronic non-autoimmune diseases such as chronic obstructive pulmonary disease (COPD).

Recently, there has been a report on research results for developing a compound having a novel structure capable of selectively inhibiting PI3K. Specifically, Patent Document 1 discloses a compound that has PI3K enzyme inhibitory activity and is useful for cancer treatment. And Patent Document 2 describes that 4-morpholino-substituted bicyclic heteroaryl compound has an inhibitory effect on PI3K activity.

Meanwhile, DNA-dependent protein kinase (DNA-PK) is a nuclear serine/threonine protein kinase that belongs to a group of PI3K-associated kinase proteins and is activated upon binding to DNA. It is known that DNA-PK is composed of a large catalytic subunit called DNA-PKcs and a regulatory component called Ku. An increase in DNA-PK activity has been proven in vitro and in vivo, which is correlated with the resistance of tumor cells to IR and bifunctional alkylating agents (Muller C. etc., Blood, 92, 2213-2219(1998), Sirzen F. etc., Eur. J. Cancer, 35, 111-116(1999)), and thus increased DNA-PK activity has been suggested as a mechanism for cellular and tumor resistance.

### [Prior Art References]

### [Patent Documents]

(Patent Document 1) International Publication No. WO 2004/048365
(Patent Document 2) European Patent No. 1,277,738

### [Disclosure of Invention]

### [Technical Problem]

An object of the present invention is to provide a pyrido-pyrimidine derivative, isomers thereof or pharmaceutically acceptable salts thereof, solvates thereof or hydrates thereof.

Other object of the present invention is to provide a method for preparing the pyrido-pyrimidine derivative, isomers thereof or pharmaceutically acceptable salts thereof, solvates thereof or hydrates thereof.

Another object of the present invention is to provide a pharmaceutical composition comprising the pyrido-pyrimidine derivative, isomers thereof or pharmaceutically acceptable salts thereof, solvates thereof or hydrates thereof as an effective ingredient.

Still another object of the present invention is to provide a pharmaceutical composition for preventing or treating PI3 kinase-associated diseases, containing the pyrido-pyrimidine derivative, isomers thereof or pharmaceutically acceptable salts thereof, solvates thereof or hydrates thereof as an effective ingredient.

Still another object of the present invention is to provide a method for preventing or treating PI3 kinase-associated diseases comprising administering a therapeutically effective amount of the pyrido-pyrimidine derivative, isomers thereof or pharmaceutically acceptable salts thereof, solvates thereof or hydrates thereof into a subject.

Still another object of the present invention is to provide a use of the pyrido-pyrimidine derivative, isomers thereof or pharmaceutically acceptable salts thereof, solvates thereof or hydrates thereof for preventing or treating PI3 kinase-associated diseases.

Still another object of the present invention is to provide a use of the pyrido-pyrimidine derivative, isomers thereof or pharmaceutically acceptable salts thereof, solvates thereof or hydrates thereof, in the manufacture of a medicament for preventing or treating PI3 kinase-associated diseases.

### [Technical Solution]

According to the present invention, a pyrido-pyrimidine derivative, isomers thereof or pharmaceutically acceptable salts thereof, solvates thereof or hydrates thereof may be represented by formula X below: 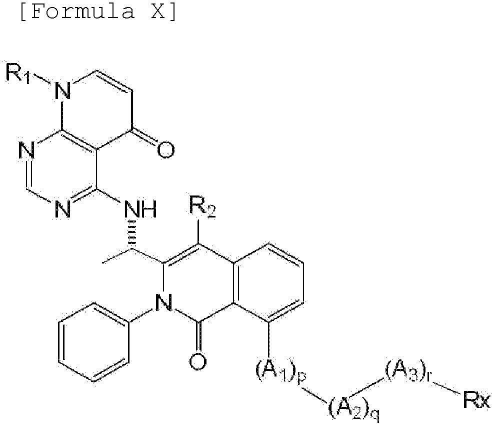 in above formula X,
R₁ is a hydrogen atom or C₂₋₁₂ heteroaryl group, in which at least one or more hydrogen atoms of the C₂₋₁₂ heteroaryl group may be each independently substituted with a halogen atom or -O-Ra (in which Ra is H, NH₂, OH, -CF₃ or C₁₋₁₀ alkyl group) ;
R₂ is a hydrogen atom, a halogen atom or C₃₋₁₀ cycloalkyl group;
Rx is R₃ or Rcy;
R₃ is a hydrogen atom, a halogen atom, -CN, OH, -O-Ra (in which Ra is H, NH₂, OH, -CF₃ or C₁₋₁₀ alkyl group), -NH₂, -CF₃ or C₁₋₁₀ alkyl group;
Rcy is C₃₋₁₀ cycloalkyl, C₃₋₁₀ cycloalkenyl, C₂₋₁₀ heterocycloalkyl, C₂₋₁₀ heterocycloalkenyl, 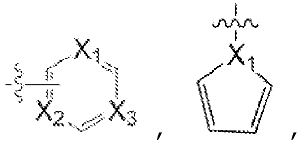 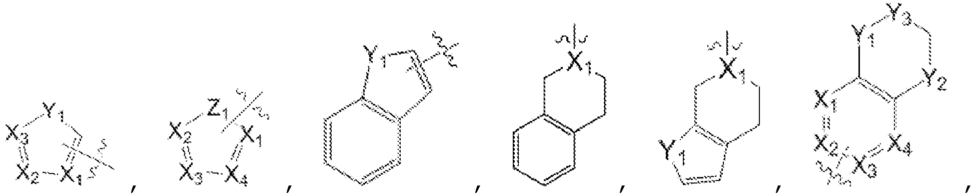 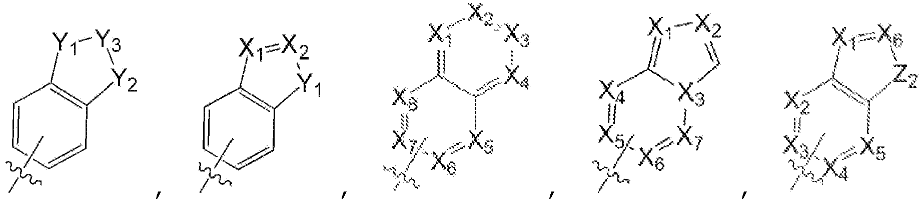 or 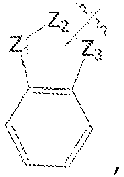
at least one or more hydrogen atoms of Rcy may be substituted or unsubstituted with R₄,
R₄ is a halogen atom, -CN, -OH, -NH₂, -NO₂, -CF₃, -O-Ra, - N(Ra)(Rb), -S(=O)(=N-Ra) -Rb, -S(Ra)₃, -S-Ra, -S(=O)₂-Ra, -C(=O)-Ra, -C(=O)-N(Ra)(Rb), -C(=O)-O-Ra, -N(Ra)-C(=O)-Rb, -N(Ra)-C(=O)-O-Rb, -N(Ra)-S(=O)₂-Rb, C₁₋₁₀ alkyl group or -(A₄)ₛ-(A₅)ₜ-R₅, which are the same as or different from each other if R₄ substituted for Cy is 2 or more, and Ra and Rb of R₄ are each independently H, NH₂, OH, - CF₃ or C₁₋₁₀ alkyl group;
in R₄, at least one or more hydrogen atoms of C₁₋₁₀ alkyl group may be substituted or unsubstituted with -OH, C₁₋₁₀ alkoxy, -CN, - CF₃ or -N(Ra)(Rb), in which Ra and Rb are each independently H, NH₂, OH, -CF₃ or C₁₋₁₀ alkyl group;
A₁ and A₃ are each independently C₁₋₁₀ alkylene, C₂₋₁₀ alkenylene or C₂₋₁₀ alkynylene, in which at least one or more hydrogen atoms of A₁ and A₃ may be each independently substituted or unsubstituted with a halogen atom, -OH, -NO₂ or -CF₃;
A₂ is -N(Ra)-, -N(Ra)-C(=O)-, -N(Ra)-C(=O)-O-, -C(=O)-N(Ra)-, -N(Ra)-S(=O)-, -N(Ra)-S(=O)₂-, -C(Ra)=N-, -S(=O)(=N-Ra)-, -C(=O)-, -C(=O)-O-, -O-, -S-, -S(=O)-, -S(=O)₂- or -B(Ra)-, in which Ra of A₂ is H, NH₂, OH, -CF₃ or C₁₋₁₀ alkyl group;
A₄ is C₁₋₁₀ alkylene, C₂₋₁₀ alkenylene or C₂₋₁₀ alkynylene;
A₅ is -N(Ra)-, -N(Ra)-S(=O)₂-, -C(=O)-, -O-Ra-, -O-, or -S-, in which Ra of A₅ is H, NH₂, OH, -CF₃ or C₁₋₁₀ alkyl group;
R₅ is C₃₋₁₀ cycloalkyl, C₃₋₁₀ cycloalkenyl, C₂₋₁₀ heterocycloalkyl, C₂₋₁₀ heterocycloalkenyl, 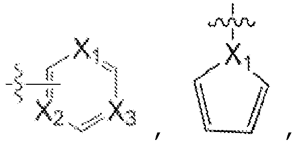 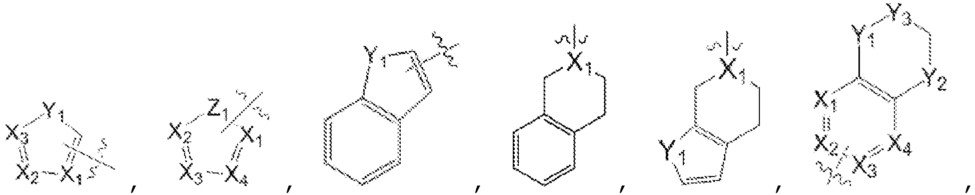 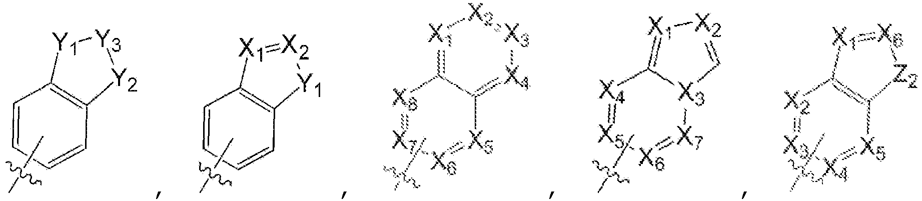 or 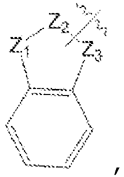 and at least one or more hydrogen atoms of R₅ may be substituted or unsubstituted with a halogen atom, NH₂, O-Ra (Ra is H, NH₂, OH, -CF₃ or C₁₋₁₀ alkyl group) or C₁₋₁₀ alkyl group;
in each of Rcy and R₅, X₁ to X₈ are each independently N or CH;
Y₁ and Y₂ are each independently NH, O or S, and Y₃ is C=O;
Z₁ to Z₃ are each independently NH or CH₂; and
p, q, r, s and t are each independently an integer of 0 or 1.

In one embodiment, the compound represented by above formula X may be a compound represented by formula 1 or 2 below: 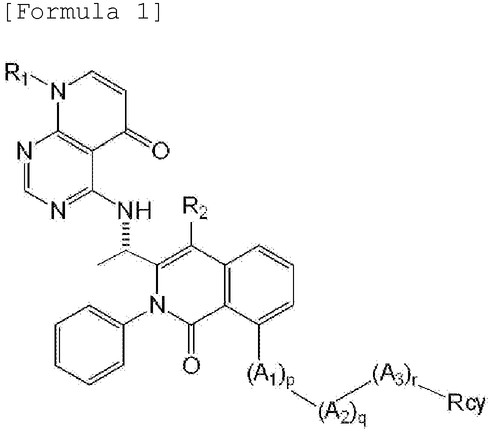 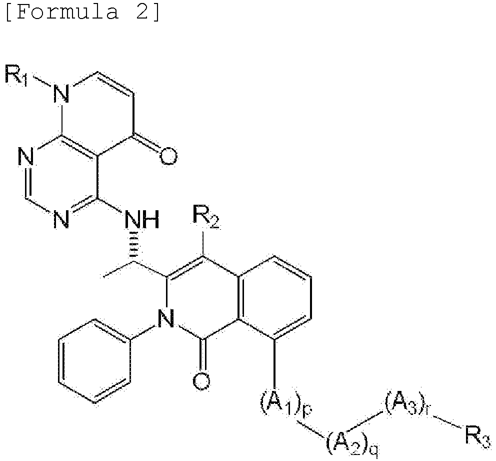 in above formula 1 or 2,
R₁ to R₃, A₁ to A₃, Rcy, Ra, Rb, p, q and r are the same as defined above.

In one embodiment, in above formula X, R₁ to R₃, A₁ to A₃, Rcy, Ra, Rb, p, q and r are the same as defined above, wherein
Rx of formula X is Rcy, p, r and q are 0, Rcy is 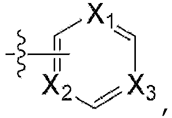 and X₁ to X₃ are CH, in which, if H is unsubstituted with R₄, R₂ is Cl as a halogen atom;
Rx of formula X is Rcy, p and r are 0, q is 1, A₂ is N(Ra)-,
Ra is a hydrogen atom, Rcy is C₂₋₁₀heterocycloalkyl or 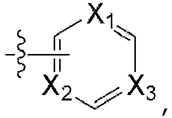 and if X₁ to X₃ are CH, R₂ is Cl as a halogen atom;
if Rx of formula X is R₃; p, q and r are 0; and R₃ is a hydrogen atom, R₂ is Cl as a halogen atom;
if Rx of formula X is R₃; p, q and r are 0; and R₃ is a hydrogen atom, R₁ is C₂₋₁₂ heteroaryl group, in which at least one or more hydrogen atoms of C₂₋₁₂ heteroaryl group may be each independently substituted with a halogen atom or -O-Ra (in which Ra is C₁₋₁₀ alkyl) ;
if Rx of formula X is R₃; p, q and r are 0; and R₃ is CN, R₂ is a hydrogen atom or Cl as a halogen atom or a hydrogen atom;
if Rx of formula X is R₃; p, q and r are 0; and R₃ is a halogen atom, R₂ is Br as a halogen atom or C₃₋₁₀ cycloalkyl group;
if Rx of formula X is R₃; p and r are 0, q is 1; A₂ is N(Ra)-, S-, O- or S(=O)₂-; R₃ is C₁₋₁₀ alkyl; and Ra is a hydrogen atom or C₁₋ₗ₀ alkyl, R₂ is Cl as a halogen atom; and
if Rx of formula X is R₃; p, r and q are 1; A₁ is C₁₋₁₀ alkylene; A₂ is O-; and if A₃ is C₁₋₁₀ alkylene, R3 is OH.

In one example, the compound represented by above formula X may be a compound represented by formula 1-1 below: 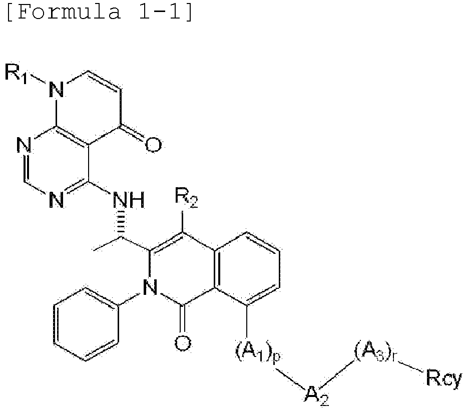 in above formula 1-1,
R₁ is H or C₂₋₁₂ heteroaryl group, in which at least one or more hydrogen atoms of the C₂₋₁₂ heteroaryl group may be each independently substituted with a halogen atom or -O-Ra (in which Ra is H, NH₂, OH, -CF₃ or C₁₋₁₀ alkyl group) ;
R₂ is H, a halogen atom or C₃₋₁₀ cycloalkyl group;
Rcy is C₃₋₁₀ cycloalkyl, C₃₋₁₀ cycloalkenyl, C₂₋₁₀ heterocycloalkyl, C₂₋₁₀ heterocycloalkenyl, 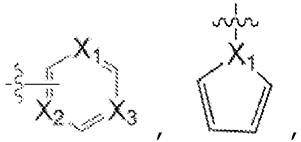 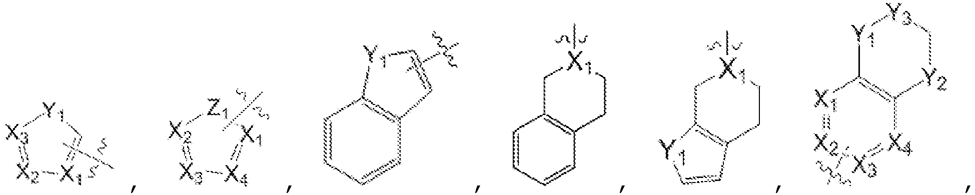 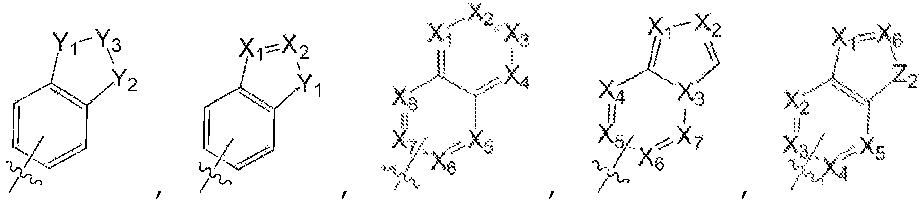 or 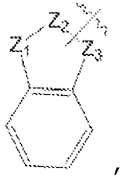
at least one or more hydrogen atoms of Rcy may be substituted or unsubstituted with R₄;
R₄ is a halogen atom, -CN, -OH, NO₂, -CF₃, -O-Ra, -N(Ra)(Rb), -S(=O)(=N-Ra) -Rb, -S(Ra)₃, -S-Ra, S(=O)₂-Ra, -C(=O)-Ra, -C(=O)-N(Ra)(Rb), -C(=O)-O-Ra, -N(Ra)-C(=O)-Ra, -N(Ra)-C(=O)-O-Ra, - N(Ra)-S(=O)₂-Rb, C₁₋₁₀ alkyl group or -(A₄)ₛ-(A₅)ₜ-R₅, which are the same as or different from each other if R₄ substituted for Cy is 2 or more, and Ra and Rb of R₄ are each independently H, NH₂, OH, - CF₃ or C₁₋₁₀ alkyl group;
in R₄, at least one or more hydrogen atoms of C₁₋₁₀ alkyl group may be substituted or unsubstituted with OH, C₁₋₁₀ alkoxy, CN, -CF₃ or -N(Ra)(Rb), in which Ra and Rb of R₄ are each independently H, NH₂, OH, -CF₃ or C₁₋₁₀ alkyl group;
A₁ and A₃ are each independently C₁₋₁₀ alkylene, C₂₋₁₀ alkenylene or C₂₋₁₀ alkynylene, in which at least one or more hydrogen atoms of A₁ and A₃ may be each independently substituted with a halogen atom, -OH, NO₂ or -CF₃;
A₂ is N(Ra)-, N(Ra)-C(=O)-, N(Ra)-C(=O)-O-, C(=O)-N(Ra)-, N(Ra)-S(=O)-, N(Ra)-S(=O)₂-, C(Ra)=N-, S(=O)(=N-Ra) -, C(=O)-, C(=O)-O-, O-, S-, S(=O)-, S(=O)₂- or B(Ra)-, in which Ra of A₂ is H, NH₂, OH, -CF₃ or C₁₋₁₀ alkyl group;
A₄ is C₁₋₁₀ alkylene, C₂₋₁₀ alkenylene or C₂₋₁₀ alkynylene;
A₅ is N(Ra)-, N(Ra)-S(=O)₂-, C(=O)-, O-Ra-, O-, or S-, in which Ra of A₅ is H, NH₂, OH, -CF₃ or C₁₋₁₀ alkyl group;
R₅ is C₃₋₁₀ cycloalkyl, C₃₋₁₀ cycloalkenyl, C₂₋₁₀ heterocycloalkyl, C₂₋₁₀ heterocycloalkenyl, 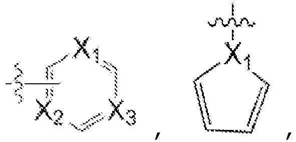 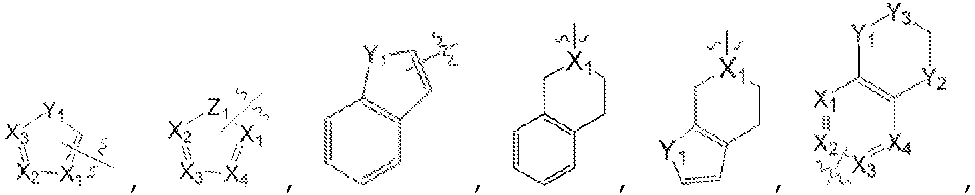 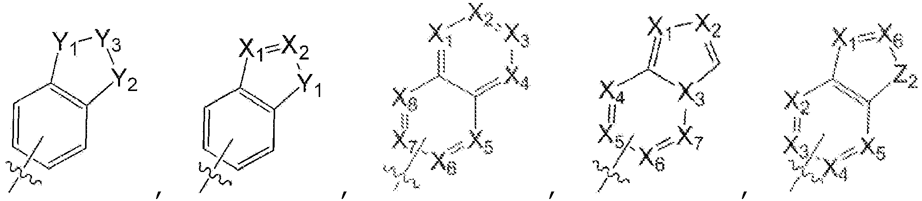 or 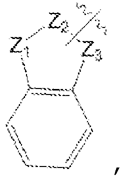 and at least one or more hydrogen atoms of R₅ may be substituted with a halogen atom, NH₂, OH or C₁₋₁₀ alkyl group;
in each of Rcy and R₅, X₁ to X₈ are each independently N or CH,
Y₁ and Y₂ are each independently NH, O or S, and Y₃ is C=O;
Z₁ to Z₃ are each independently NH or CH₂; and
p, r, s and t are each independently an integer of 0 or 1.

In one embodiment, the compound represented by above formula X may be a compound represented by formula 1-2 below: 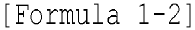 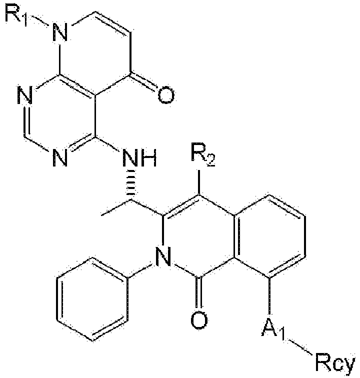 in above formula 1-2,
R₁ is H or C₂₋₁₂ heteroaryl group, in which at least one or more hydrogen atoms of the C₂₋₁₂ heteroaryl group may be each independently substituted with a halogen atom or -O-Ra (in which Ra is H, NH₂, OH, -CF₃ or C₁₋₁₀ alkyl group) ;
R₂ is H, a halogen atom or C₃₋₁₀ cycloalkyl group;
Rcy is C₃₋₁₀ cycloalkyl, C₃₋₁₀ cycloalkenyl, C₂₋₁₀ heterocycloalkyl, C₂₋₁₀ heterocycloalkenyl, 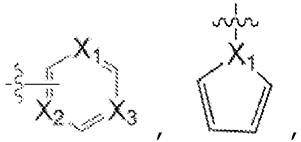 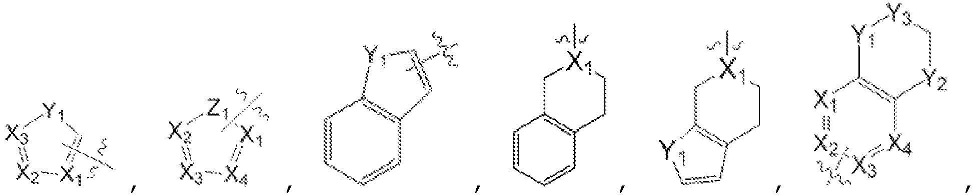 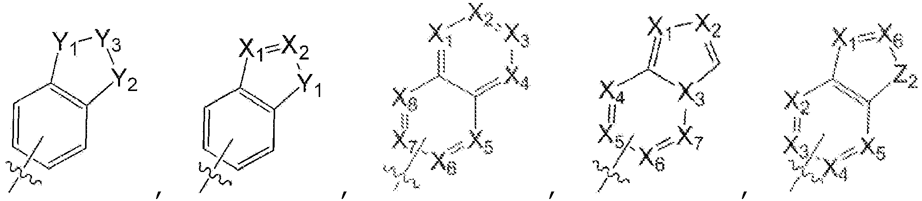 or 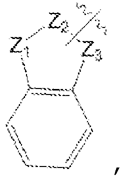
at least one or more hydrogen atoms of Rcy may be substituted or unsubstituted with R₄;
R₄ is a halogen atom, -CN, -OH, NO₂, -CF₃, O-Ra, -N(Ra)(Rb), S(=O) (=N-Ra) -Rb, S(Ra)₃, S-Ra, S (=O)₂-Ra, C(=O)-Ra, C(=O)-N(Ra) (Rb), C (=O)-O-Ra, N(Ra) -C (=O)-Ra, N(Ra) -C (=O)-O-Ra, N(Ra) - S(=O)₂-Rb, C₁₋₁₀ alkyl group or - (A₄)ₛ-(A₅)ₜ-R₅, which are the same as or different from each other if R₄ substituted for Cy is 2 or more, and Ra and Rb of R₄ are each independently H, NH₂, OH, -CF₃ or C₁₋₁₀ alkyl group;
in R₄, at least one or more hydrogen atoms of C₁₋₁₀ alkyl group may be substituted with OH, C₁₋₁₀ alkoxy, CN, -CF₃ or -N(Ra)(Rb), in which Ra and Rb are each independently H, NH₂, OH, -CF₃ or C₁₋₁₀ alkyl group;
A₁ is C₂₋₁₀ alkenylene or C₂₋₁₀ alkynylene, in which at least one or more hydrogen atoms of A₁ may be each independently substituted or unsubstituted with a halogen atom, -OH, NO₂ or -CF₃;
A₄ is C₁₋₁₀ alkylene, C₂₋₁₀ alkenylene or C₂₋₁₀ alkynylene;
A₅ is N(Ra)-, N(Ra)-S(=O)₂-, C(=O)-, O-Ra-, O-, or S-, in which Ra of A₅ is H, NH₂, OH, -CF₃ or C₁₋₁₀ alkyl group;
R₅ is C₃₋₁₀ cycloalkyl, C₃₋₁₀ cycloalkenyl, C₂₋₁₀ heterocycloalkyl, C₂₋₁₀ heterocycloalkenyl, 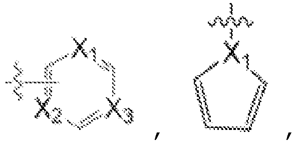 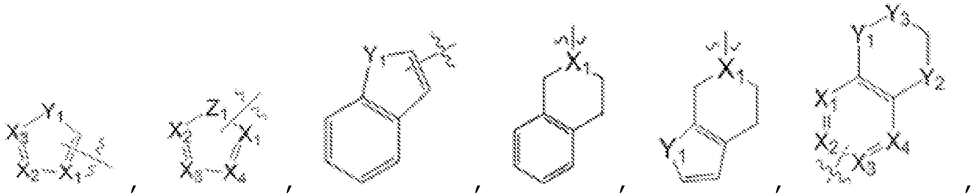 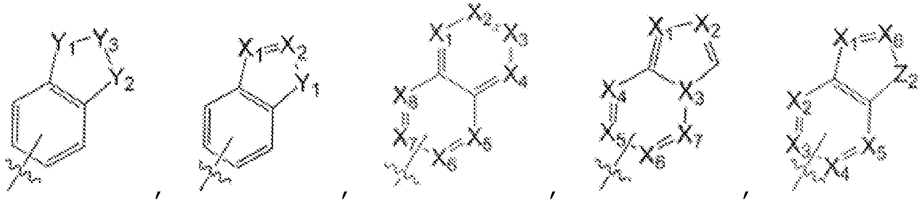 or 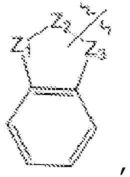 and at least one or more hydrogen atoms of R₅ may be substituted with a halogen atom, NH₂, OH or C₁₋₁₀ alkyl group;
in each of Rcy and R₅, X₁ to X₈ are each independently N or CH;
Y₁ and Y₂ are each independently NH, O or S, and Y₃ is C=O;
Z₁ to Z₃ are each independently NH or CH₂; and
s and t are each independently an integer of 0 or 1.

In one embodiment, the compound represented by above formula X may be a compound represented by formula 1-3 below: 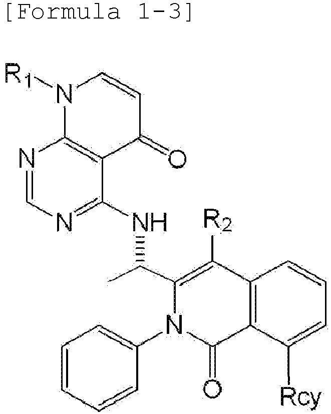 in above formula 1-3,
R₁ is H or C₂₋₁₂ heteroaryl group, in which at least one or more hydrogen atoms of the C₂₋₁₂ heteroaryl group may be each independently substituted with a halogen atom or -O-Ra (in which Ra is H, NH₂, OH, -CF₃ or C₁₋₁₀ alkyl group) ;
R₂ is H, a halogen atom or C₃₋₁₀ cycloalkyl group;
Rcy is C₃₋₁₀ cycloalkyl, C₃₋₁₀ cycloalkenyl, C₂₋₁₀ heterocycloalkyl, C₂₋₁₀ heterocycloalkenyl, 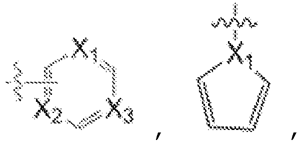 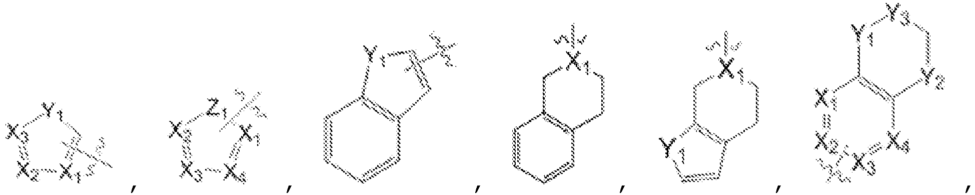 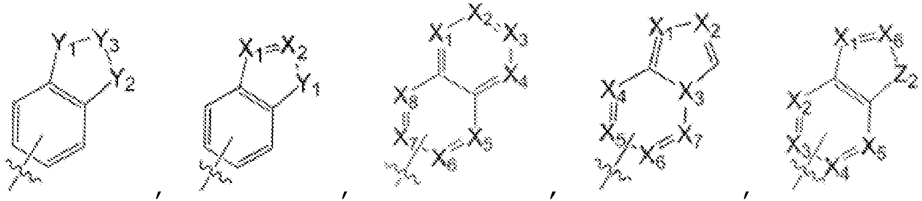 or 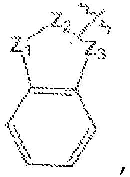
at least one or more hydrogen atoms of Rcy may be substituted or unsubstituted with R₄;
R₄ is a halogen atom, -CN, -OH, NO₂, -CF₃, O-Ra, -N(Ra)(Rb), S(=O)(=N-Ra) -Rb, S(Ra)₃, S-Ra, S(=O)₂-Ra, C(=O)-Ra, C(=O)-N(Ra)(Rb), C(=O)-O-Ra, N(Ra)-C(=O)-Ra, N(Ra)-C(=O)-O-Ra, N(Ra)-S(=O)₂-Rb, C₁₋₁₀ alkyl group or -(A₄)ₛ-(A₅)ₜ-R₅, which are the same as or different from each other if R₄ substituted for Cy is 2 or more, and Ra and Rb are each independently H, NH₂, OH, -CF₃ or C₁₋₁₀ alkyl group;
in R₄, at least one or more hydrogen atoms of C₁₋₁₀ alkyl group may be substituted or unsubstituted with OH, C₁₋₁₀ alkoxy, CN, -CF₃ or -N(Ra)(Rb), in which Ra and Rb are each independently H, NH₂, OH, -CF₃ or C₁₋₁₀ alkyl group;
A₄ is C₁₋₁₀ alkylene, C₂₋₁₀ alkenylene or C₂₋₁₀ alkynylene;
A₅ is N(Ra)-, N(Ra)-S(=O)₂-, C(=O)-, O-Ra-, O-, or S-, in which Ra of A₅ is H, NH₂, OH, -CF₃ or C₁₋₁₀ alkyl group;
R₅ is C₃₋₁₀ cycloalkyl, C₃₋₁₀ cycloalkenyl, C₂₋₁₀ heterocycloalkyl, C₂₋₁₀ heterocycloalkenyl, 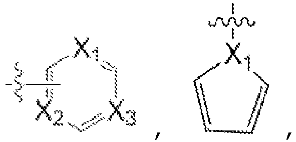 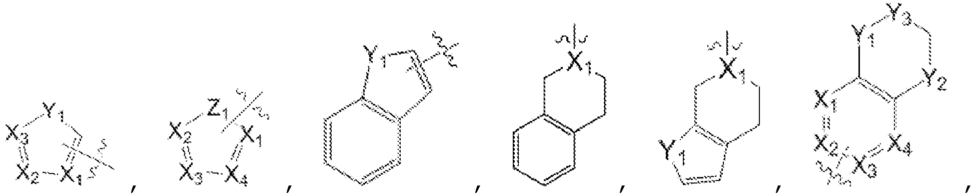 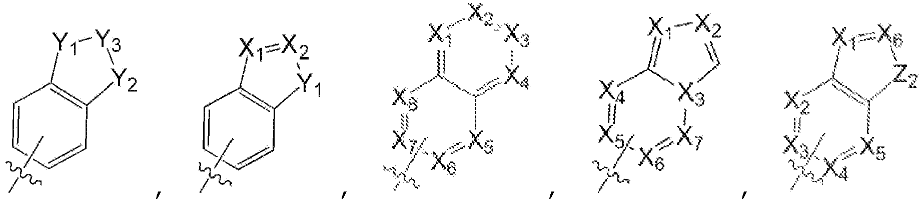 or 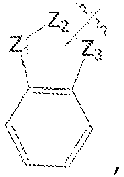 and at least one or more hydrogen atoms of R₅ may be substituted or unsubstituted with a halogen atom, NH₂, OH or C₁₋ₗ₀ alkyl group;
in each of Rcy and R₅, X₁ to X₈ are each independently N or CH,
Y₁ and Y₂ are each independently NH, O or S, and Y₃ is C=O;
Z₁ to Z₃ are each independently NH or CH₂; and
s and t are each independently an integer of 0 or 1.

In one embodiment, the compound represented by above formula X may be a compound represented by formula 1-4 below: 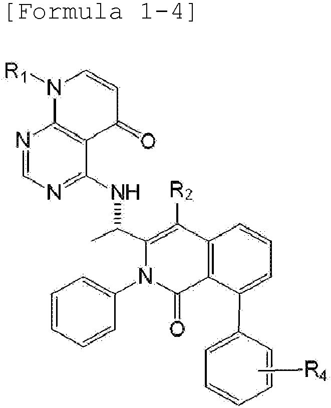 in above formula 1-4,
R₁ is H or C₂₋₁₂ heteroaryl group, in which at least one or more hydrogen atoms of the C₂₋₁₂ heteroaryl group may be each independently substituted with a halogen atom or -O-Ra (in which Ra is H, NH₂, OH, -CF₃ or C₁₋₁₀ alkyl group) ;
R₂ is H, a halogen atom or C₃₋₁₀ cycloalkyl group;
R₄ is a halogen atom, -CN, -OH, NO₂, -CF₃, O-Ra, -N(Ra)(Rb), S (=O)(=N-Ra) -Rb, S (Ra)₃, S-Ra, S(=O)₂-Ra, C(=O)-Ra, C(=O)-N(Ra) (Rb), C(=O)-O-Ra, N(Ra)-C(=O)-Ra, N(Ra)-C(=O)-O-Ra, N(Ra)-S(=O)₂-Rb, C₁₋ₗ₀ alkyl group or -(A₄)ₛ-(A₅)ₜ-R₅, which are the same as or different from each other if R₄ is 2 or more, and Ra and Rb of R₄ are each independently H, NH₂, OH, -CF₃ or C₁₋₁₀ alkyl group;
in R₄, at least one or more hydrogen atoms of C₁₋₁₀ alkyl group may be substituted or unsubstituted with OH, C₁₋₁₀ alkoxy, CN, -CF₃ or -N(Ra)(Rb), in which Ra and Rb are each independently H, NH₂, OH, -CF₃ or C₁₋₁₀ alkyl group;
A₄ is C₁₋₁₀ alkylene, C₂₋₁₀ alkenylene or C₂₋₁₀ alkynylene;
A₅ is N(Ra)-, N(Ra)-S(=O)₂-, C(=O)-, O-Ra-, O-, or S-, in which Ra of A₅ is H, NH₂, OH, -CF₃ or C₁₋₁₀ alkyl group;
R₅ is C₃₋₁₀ cycloalkyl, C₃₋₁₀ cycloalkenyl, C₂₋₁₀ heterocycloalkyl, C₂₋₁₀ heterocycloalkenyl, 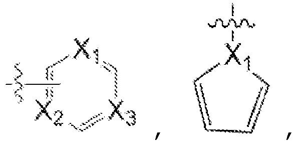 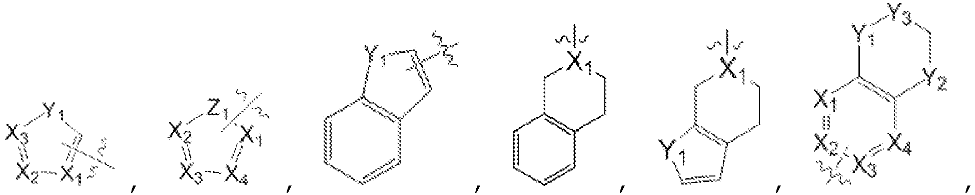 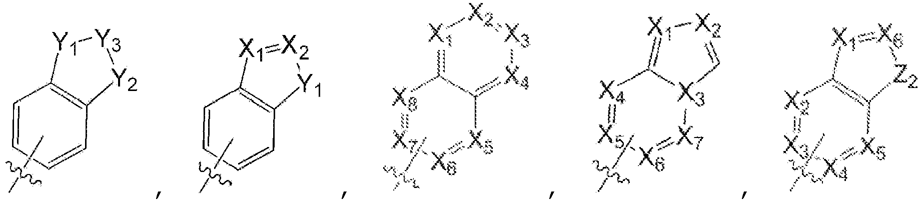 or 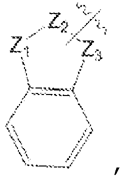 and at least one or more hydrogen atoms of R₅ may be substituted or unsubstituted with a halogen atom, NH₂, OH or C₁₋₁₀ alkyl group;
X₁ to X₈ are each independently N or CH;
Y₁ and Y₂ are each independently NH, O or S, and Y₃ is C=O;
Z₁ to Z₃ are each independently NH or CH₂; and
s and t are each independently an integer of 0 or 1.

In one embodiment, the compound represented by above formula X may be a compound represented by formula 2-1 below: 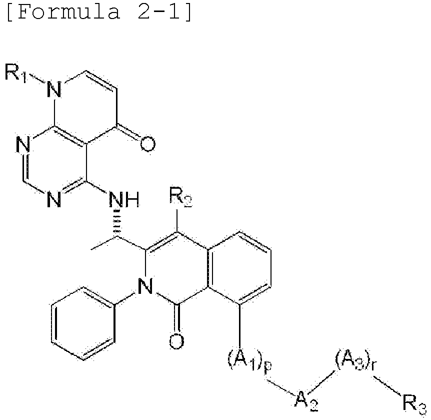 in above formula 2-1,
R₁ is H or C₂₋₁₂ heteroaryl group, in which at least one or more hydrogen atoms of the C₂₋₁₂ heteroaryl group may be each independently substituted with a halogen atom or -O-Ra (in which Ra is H, NH₂, OH, -CF₃ or C₁₋ₗ₀ alkyl group) ;
R₂ is H, a halogen atom or C₃₋₁₀ cycloalkyl group;
R₃ is H, a halogen atom, CN, OH, O-Ra (in which Ra is H, NH₂, OH, -CF₃ or C₁₋₁₀ alkyl group), NH₂, -CF₃ or C₁₋₁₀ alkyl group;
A₁ and A₃ are each independently C₁₋₁₀ alkylene, C₂₋₁₀ alkenylene or C₂₋₁₀ alkynylene, in which at least one or more hydrogen atoms of A₁ and A₃ may be each independently substituted or unsubstituted with a halogen atom, -OH, NO₂or -CF₃;
A₂ is N(Ra)-C(=O)-, N(Ra)-C(=O)-O-, C(=O)-N(Ra)-, N(Ra)-S(=O)-, N(Ra)-S(=O)₂-, C(Ra)=N-, S(=O)(=N-Ra)-, C(=O)-, C(=O)-O-, S(=O)- or B(Ra)-, in which Ra of A₂ is H, NH₂, OH, -CF₃ or C₁₋₁₀ alkyl group; and
p and r are each independently 0 or 1.

In one embodiment, the compound represented by above formula X may be a compound represented by formula 2-2 below: 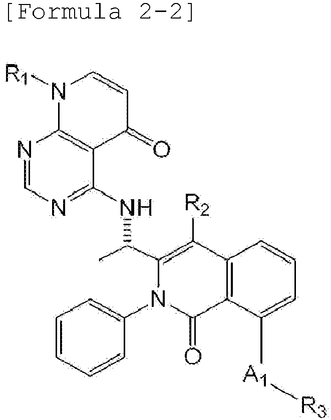 in above formula 2-2,
R₁ is H or C₂₋₁₂ heteroaryl group, in which at least one or more hydrogen atoms of the C₂₋₁₂ heteroaryl group may be each independently substituted with a halogen atom or -O-Ra (in which Ra is H, NH₂, OH, -CF₃ or C₁₋₁₀ alkyl group) ;
R₂ is H, a halogen atom or C₃₋₁₀ cycloalkyl group;
if A₁ is C₂₋₁₀ alkenylene or C₂₋₁₀ alkynylene, R₃ is H, a halogen atom, CN, OH, O-Ra (in which Ra is H, OH, -CF₃ or C₁₋₁₀ alkyl group), NH₂, -CF₃ or C₁₋₁₀ alkyl group; and
if A₁ is C₁₋₁₀ alkylene, R₃ is a halogen atom, CN, OH, O-Ra (in which Ra is H, OH, -CF₃ or C₁₋₁₀ alkyl group), NH₂ or -CF₃.

In one embodiment, Rcy and R₅ of formula X may be each independently C₃₋₁₀ cycloalkylene, C₃₋₁₀ cycloalkenyl, C₂₋₁₀ heterocycloalkylene, C₂₋₁₀ heterocycloalkenyl, 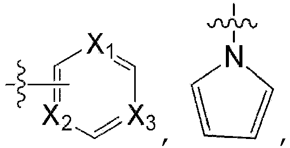 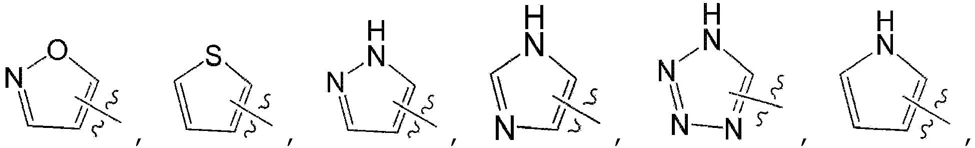 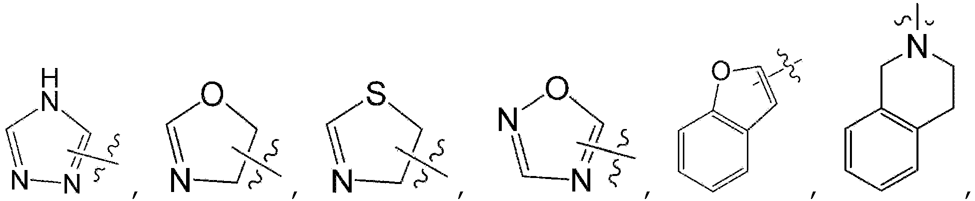 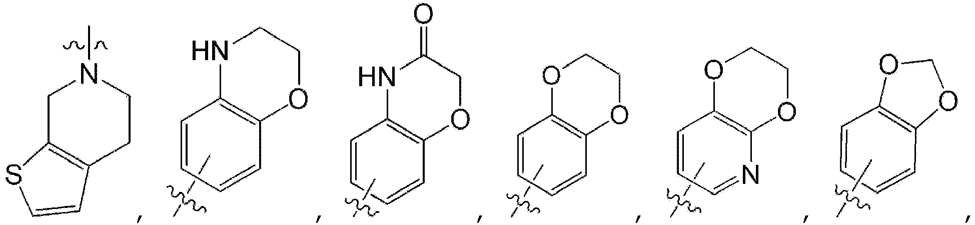 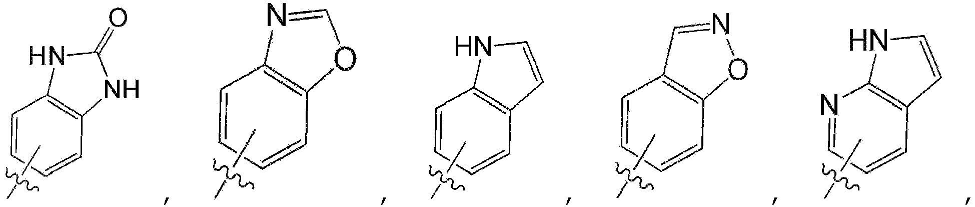 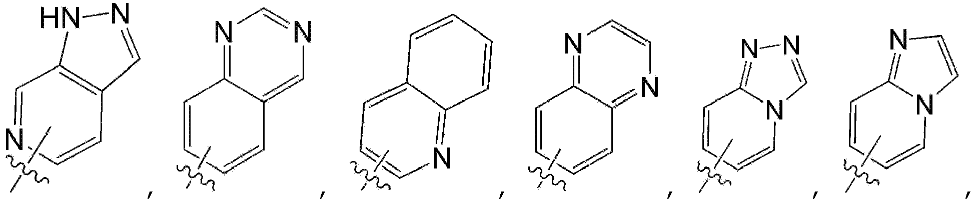 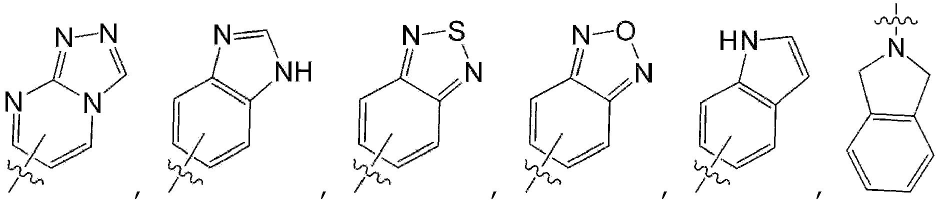 or

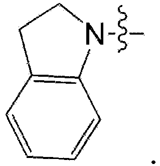

### [Advantageous Effects]

According to the present invention, a pyrido-pyrimidine derivative, isomers thereof or pharmaceutically acceptable salts thereof, solvates thereof or hydrates thereof are confirmed to show an effect of selectively inhibiting a PI3 kinase selected from the group consisting of PI3Kα, β, δ and γ and, in particular, show an excellent effect of inhibiting PI3K-δ and DNA-PK. As such, according to the present invention, a pyrido-pyrimidine derivative, isomers thereof or pharmaceutically acceptable salts thereof, solvates thereof or hydrates thereof can inhibit a downstream signaling system mediated by PI3K-δ and suppress the activity of DNA-PK at the same time to promote a cell cycle arrest and apoptosis, and thus can be advantageously used to treat or prevent cancer diseases such as liquid hematologic malignancy, ovarian cancer, cervical cancer, breast cancer, colorectal cancer, liver cancer, stomach cancer, pancreatic cancer, colon cancer, peritoneal metastatic cancer, skin cancer, bladder cancer, prostate cancer, lung cancer, osteosarcoma, fibrous tumor, brain tumor, etc.

In addition, it can be also advantageously used to treat or prevent PI3K-associated diseases such as autoimmune diseases such as rheumatoid arthritis, systemic lupus erythematosus, multiple sclerosis, diabetes, hyperthyroidism, myasthenia, autoimmune pernicious anemia, etc., respiratory diseases such as chronic obstructive pulmonary disease (COPD), rhinitis, asthma, chronic bronchitis, chronic pulmonary inflammatory disease, pleurisy, alveolitis, vasculitis, emphysema, pneumonia, bronchiectasis, etc.

### [Mode for Invention]

Hereinafter, terms used in the present application are used only to describe a certain exemplary embodiment and are not intended to limit the present invention. The terms of a singular form may comprise plural forms unless otherwise specified. In the present application, terms such as "comprising," "have" or the like shall be intended to designate a presence of features, steps, operations, components, parts or combinations thereof described herein, and shall not be construed to exclude a possible presence or addition of one or more other features, steps, operations, components, parts or combinations thereof in advance.

All the terms used herein comprising technical or scientific terms have the same meaning as commonly understood by those ordinary skilled in the art, to which the present invention pertains, unless defined otherwise. Such terms as those defined in a generally used dictionary are to be interpreted to have the meanings equal to the contextual meanings in the relevant art, and are not to be interpreted to have ideal or excessively formal meanings, unless clearly defined in the present application.

In the present specification, "C_{x-y}" may refer to having x or more and y or less carbon atoms.

In the present invention, the term "substituted" may represent a moiety having a substituent which replaces at least one hydrogen on carbon of a main chain. The "substitution" or "substituted with~" may be defined to comprise implicit conditions, in which the substitution follows a permitted valency of a substituted atom and a substituent and induces a compound stabilized by substitution, for example, a compound which is not naturally modified by rearrangement, cyclization, removal, etc.

In the present invention, "alkyl" may mean a linear (or straight-chain) saturated hydrocarbon group or a branched (or side-chain) saturated hydrocarbon group, and may comprise methyl, ethyl, n-propyl, isopropyl, n-butyl, sec-butyl, isobutyl, tert-butyl, n-pentyl, n-hexyl, n-heptyl, etc.

In the present invention, "alkylene" may mean a divalent functional group which is induced from the alkyl group as defined above.

In the present invention, "alkenyl" may mean an unsaturated hydrocarbon group comprising at least one double bond between carbons, and "alkynyl" may mean an unsaturated hydrocarbon group comprising at least one triple bond between carbons.

In the present invention, "alkenylene" may mean a divalent functional group derived from alkenyl as defined above, and "alkynylene" may mean a divalent functional group derived from alkynyl as defined above.

In the present invention, "alkoxy" may refer to -O-alkyl, in which alkyl is the same as defined above.

In the present invention, "halogen atom" may refer to F, Cl, Br or I.

In the present invention, "aryl" may comprise a monocyclic aromatic structure or a polycyclic aromatic structure, as well as a structure in which a saturated hydrocarbon ring is fused into the monocyclic or polycyclic aromatic group. Aryl may comprise a phenyl group, biphenyl, naphthalenyl, tetrahydronaphthalenyl, anthracenyl, phenanthrenyl, pyrenyl, etc.

In the present invention, "heteroaryl" may mean a monocyclic or polycyclic hetero ring in which at least one or more carbon atoms are substituted with nitrogen (N), oxygen (O) or sulfur (S) in the aryl group. Heteroaryl may comprise pyridinyl, thiophenyl, triazolyl, tetrazolyl, benzodioxolyl, benzothiazolyl, benzothiophenyl, quinolinyl, indolyl, isoindolyl, benzofuranyl, benzopyrrolyl, furanyl, pyrrolyl, thiazolyl, isothiazolyl, imidazolyl, pyrazolyl, oxazolyl, isoxazolyl, pyrazinyl, pyridazinyl, pyrimidinyl, isoquinolinyl, carbazolyl, benzoxazolyl, benzodioxazolyl, benzodioxinyl, benzimidazolyl, dihydrobenzothiophenyl, dihydrobenzofuranyl, purinyl, indolizinyl, chromanyl, chromenyl, dihydrobenzodioxinyl, etc., but is not limited thereto.

In the present invention, "cycloalkyl" may mean a saturated hydrocarbon ring generally having a specified number of carbon atoms containing a ring, and the saturated hydrocarbon ring may collectively refer to monocyclic and polycyclic structures, and a ring structure, in which at least two rings share at least one pair of carbon atoms. Cycloalkyl may comprise cyclohexyl, cycloheptanyl, cyclooctanyl, tetrahydronaphthalenyl, adamantanyl, etc., but is not limited thereto.

In the present invention, "heterocycloalkyl" may mean saturated monocyclic and polycyclic hetero rings comprising one to four hetero atoms independently selected from boron (B), nitrogen (N), oxygen (O) and sulfur (S), or a ring structure, in which at least two rings share at least one pair of carbon atoms. Heterocycloalkyl may comprise oxiranyl, oxetanyl, morpholinyl, thietanyl, pyrrolidinyl, piperidinyl, tetrahydrofuranyl, tetrahydrothiophenyl, tetrahydropyranyl, tetrahydrothiopyranyl, 6- azabicyclo[3.2.1]octanyl, etc., but is not limited thereto.

In the present invention, "heterocycloalkenyl" may mean a saturated and partially unsaturated aromatic ring comprising one to four heteroatoms independently selected from nitrogen (N), oxygen (O) and sulfur (S). Heterocycloalkenyl may comprise 4,5-dihydrooxazolyl, etc., but is not limited thereto.

The pyrido-pyrimidine derivative represented by formula X according to the present invention may comprise a compound selected from the group consisting of compounds shown in table 1 below.

**[Table 1]**

| Example s | Compound Names |
|---|---|
| 001 | (S)-4-((1-(4-bromo-8-chloro-1-oxo-2-phenyl-1,2-dihydroisoquinolin-3-yl)ethyl)amino)pyrido[2,3-d]pyrimidin-5(8H)-one |
| 002 | (S)-4-((1-(4-chloro-1-oxo-2-phenyl-8-(4,4,5,5-tetramethyl-1,3,2-dioxaboran-2-yl)-1,2-dihydroisoquinolin-3-yl)ethyl)amino)pyrido[2,3-d]pyrimidin-5(8H)-one |
| 003 | (S)-4-((1-(8-(6-(1H-pyrrol-1-yl)pyridin-3-yl)-4-chloro-1-oxo-2-phenyl-1,2-dihydroisoquinolin-3-yl)ethyl)amino)pyrido[2,3-d]pyrimidin-5(8H)-one |
| 004 | (S)-4-((1-(4-chloro-1-oxo-2-phenyl-8-(pyridin-3-yl)-1,2-dihydroisoquinolin-3-yl)ethyl)amino)pyrido[2,3-d]pyrimidin-5(8H)-one |
| 005 | (S)-4-((1-(4-chloro-8-(1-methyl-1H-pyrazol-4-yl)-1-oxo-2-phenyl-1,2-dihydroisoquinolin-3-yl)ethyl)amino)pyrido[2,3-d]pyrimidin-5(8H)-one |
| 006 | (S)-4-((1-(8-(benzofuran-2-yl)-4-chloro-1-oxo-2-phenyl-1,2-dihydroisoquinolin-3-yl)ethyl)amino)pyrido[2,3-d]pyrimidin-5(8H)-one |
| 007 | (S)-4'-chloro-3'-(1-((5-oxo-5,8-dihydropyrido[2,3-d]pyrimidin-4-yl)amino)ethyl-2'-phenyl-3,4-dihydro-1H-(2,8'-biisoquinolin)-1'(2'H)-one |
| 008 | 4-(((1S)-1-(4-chloro-1-oxo-2-phenyl-8-(3a,4,7,7a-tetrahydro-1-H-isoindol-2(3H-yl)-1,2-dihydroisoquinolin-3-yl)ethyl)amino)pyrido[2,3-d]pyrimidin-5(8H)-one |
| 009 | (S)-4-((1-(4-chloro-1-oxo-2-phenyl-8-(4-(pyridin-4-yl)piperazin-1-yl)-1,2-dihydroisoquinolin-3-yl)ethyl)amino)pyrido[2,3-d]pyrimidin-5(8H)-one |
| 010 | (S)-4-((1-(8-([1,4'-bipiperidin]-1'-yl)-4-chloro-1-oxo-2-phenyl-1,2-dihydroisoquinolin-3-yl)ethyl)amino)pyrido[2,3-d]pyrimidin-5(8H)-one |
| 011 | (S)-4-((1-(4-chloro-8-(4-(1-methylpiperidin-4-yl)piperazin-1-yl)-1-oxo-2-phenyl-1,2-dihydroisoquinolin-3-yl)ethyl) amino)p yrido[2,3-d]pyrimidin-5(8H)-one |
| 012 | (S)-4-((1-(1-oxo-2-phenyl-8-(pyridin-3-yl)-1,2-dihydroisoquinolin-3-yl)ethyl)amino)pyrido[2,3-d]pyrimidin-5(8H)-one |
| 013 | (S)-4-((1-(8-(6-methylpyridin-3-yl)-1-oxo-2-phenyl-1,2-dihydroisoquinolin-3-yl)ethyl)amino)pyrido[2,3-d]pyrimidin-5(8H)-one |
| 014 | (S)-4-((1-(8-(4-fluorophenyl)-1-oxo-2-phenyl-1,2-dihydroisoquinolin-3-yl)ethyl)amino)pyrido[2,3-d]pyrimidin-5(8H)-one |
| 015 | (S)-4-((1-(8-(1-methyl-1H-pyrazol-4-yl)-1-oxo-2-phenyl-1,2-dihydroisoquinolin-3-yl)ethyl)amino)pyrido[2,3-d]pyrimidin-5(8H)-one |
| 016 | (S)-4-((1-(4-bromo-8-(4-(dimethylamino)phenyl)-1-oxo-2-phenyl-1,2-dihydroisoquinolin-3-yl)ethyl)amino)pyrido[2,3-d]pyrimidin-5(8H)-one |
| 017 | (S)-4-((1-(4-bromo-8-(6-methylpyridin-3-yl)-1-oxo-2-phenyl-1,2-dihydroisoquinolin-3-yl)ethyl)amino)pyrido[2,3-d]pyrimidin-5(8H)-one |
| 018 | (S)-4-((1-(4-bromo-8-(4-fluorophenyl)-1-oxo-2-phenyl-1,2-dihydroisoquinolin-3-yl)ethyl)amino)pyrido[2,3-d]pyrimidin-5(8H)-one |
| 019 | (S)-4-((1-(4-bromo-8-(1-methyl-1H-pyrazol-4-yl)-1-oxo-2-phenyl-1,2-dihydroisoquinolin-3-yl)ethyl)amino)pyrido[2,3-d]pyrimidin-5(8H)-one |
| 020 | (S)-4-((1-(8-(4-(dimethylamino)phenyl)-1-oxo-2-phenyl-1,2-dihydroisoquinolin-3-yl)ethyl)amino)pyrido[2,3-d]pyrimidin-5(8H)-one |
| 021 | (S)-4-((1-(4-chloro-1-oxo-8-(2-oxo-2,3-dihydro-1H-benzo[d]imidazol-5-yl)-2-phenyl-1,2-dihydroisoquinolin-3-yl)ethyl)amino)pyrido[2,3-d]pyrimidin-5(8H)-one |
| 022 | (S)-4-((1-(4-chloro-8-(2-methylbenzo[d]oxazol-6-yl)-1-oxo-2-phenyl-1,2-dihydroisoquinolin-3-yl)ethyl)amino)pyrido[2,3-d]pyrimidin-5(8H)-one |
| 023 | (S)-4-(1-oxo-3-(1-((5-oxo-5,8-dihydropyrido[2,3-d]pyrimidin-4-yl)amino)ethyl)-2-phenyl-1,2-dihydroisoquinolin-8-yl)benzonitrile |
| 024 | (S)-4-((1-(8-(benzo[d]isoxazol-6-yl)-4-chloro-1-oxo-2-phenyl-1,2-dihydroisoquinolin-3-yl)ethyl)amino)pyrido[2,3-d]pyrimidin-5(8H)-one |
| 025 | (S)-4-((1-(4-chloro-1-oxo-2-phenyl-8-(1H-pyrrolo[2,3-b]pyridin-5-yl)-1,2-dihydroisoquinolin-3-yl)ethyl)amino)pyrido[2,3-d]pyrimidin-5(8H)-one |
| 026 | (S)-4-((1-(4-chloro-8-(2-(2-hydroxypropan-2-yl)pyrimidin-5-yl)-1-oxo-2-phenyl-1,2-dihydroisoquinolin-3-yl)ethyl) amino)p yrido[2,3-d]pyrimidin-5(8H)-one |
| 027 | (S)-4-((1-(4-chloro-1-oxo-2-phenyl-8-(1H-pyrazolo[4,3-c]pyridin-6-yl)-1,2-dihydroisoquinolin-3-yl)ethyl)amino)pyrido[2,3-d]pyrimidin-5(8H)-one |
| 028 | (S)-4-((1-(4-chloro-8-(5,6-dimethoxypyrazin-2-yl)-1-oxo-2-phenyl-1,2-dihydroisoquinolin-3-yl)ethyl)amino)pyrido[2,3-d]pyrimidin-5(8H)-one |
| 029 | (S)-4-((1-(4-chloro-1-oxo-2-phenyl-8-(4-(triazol-2-yl)phenyl)-1,2-dihydroisoquinolin-3-yl)ethyl)amino)pyrido[2,3-d]pyrimidin-5(8H)-one |
| 030 | (S)-4-((1-(4-chloro-8-(5-methoxypyrimidin-2-yl)-1-oxo-2-phenyl-1,2-dihydroisoquinolin-3-yl)ethyl)amino)pyrido[2,3-d]pyrimidin-5(8H)-one |
| 031 | (S)-6-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)nicotinonitrile |
| 032 | (S)-4-((1-(4-chloro-1-oxo-2-phenyl-8-(quinazolin-6-yl)-1,2-dihydroisoquinolin-3-yl)ethyl)amino)pyrido[2,3-d]pyrimidin-5(8H)-one |
| 033 | (S)-4-((1-(4-chloro-8-(4-(isoxazol-3-yl)phenyl)-1-oxo-2-phenyl-1,2-dihydroisoquinolin-3-yl)ethyl)amino)pyrido[2,3-d]pyrimidin-5(8H)-one |
| 034 | (S)-4-((1-(8-(6-(tert-butyl)pyridin-3-yl)-4-chloro-1-oxo-2-phenyl-1,2-dihydroisoquinolin-3-yl)ethyl)amino)pyrido[2,3-d]pyrimidin-5(8H)-one |
| 035 | (S)-4-((1-(4-chloro-1-oxo-2-phenyl-8-(pyrimidin-2-yl)-1,2-dihydroisoquinolin-3-yl)ethyl)amino)pyrido[2,3-d]pyrimidin-5(8H)-one |
| 036 | (S)-4-((1-(4-chloro-8-(3-fluoro-4,5-dihydroxyphenyl)-1-oxo-2-phenyl-1,2-dihydroisoquinolin-3-yl)ethyl) amino)pyrido[2,3-d]pyrimidin-5(8H)-one |
| 037 | (S)-4-((1-(8-([1,2,4]tetrazolo[4,3-a]pyridin-6-yl)-4-chloro-1-oxo-2-phenyl -1,2-dihydroisoquinolin-3-yl)ethyl)amino)pyrido[2,3-d]pyrimidin-5(8H)-one |
| 038 | (S)-4-((1-(4-chloro-1-oxo-2-phenyl-1,2-dihydroisoquinolin-3-yl)ethyl)amino)pyrido[2,3-d]pyrimidin-5(8H)-one |
| 039 | (S)-2-(5-(4-chloro-1-oxo-3-(1-((5-oxo-5,8-dihyropyrido[2,3-d]pyrimidin-4-yl)amino) ethyl)-2-phenyl-1,2-dihydroisoquinolin-8-yl)pyrimidin-2-yl)acetonitrile |
| 040 | (S)-4-((1-(8-([1,2,4]triazolo[4,3-a]pyrimidin-6-yl)-4-chloro-1-oxo-2-phenyl-1,2-dihydroisoquinolin-3-yl)ethyl) amino)pyrid o[2,3-d]pyrimidin-5(8H)-one |
| 041 | (S)-4-((1-(4-chloro-8-(5-methoxypyridin-2-yl)-1-oxo-2-phenyl-1,2-dihydroisoquinolin-3-yl)ethyl)amino)pyrido[2,3-d]pyrimidin-5(8H)-one |
| 042 | (S)-4-((1-(8-([1,2,4]triazolo[1,5-a]pyridin-6-yl)-4-chloro-1-oxo-2-phenyl-1,2-dihydroisoquinolin-3-yl)ethyl) amino)pyrido[2,3-d]pyrimidin-5(8H)-one |
| 043 | (S)-4-((1-(4-chloro-1-oxo-2-phenyl-1,2-dihydroisoquinolin-3-yl)ethyl)amino)-8-(5-methoxypyrimidin-2-yl)pyrido[2,3-d]pyrimidin-5(8H)-one |
| 044 | (S)-5-(4-chloro-1-oxo-3-(1-((5-oxo-5,8-dihydropyrido[2,3-d]pyrimidin-4-yl)amino) ethyl)-2-phenyl-1,2-dihydroisoquinolin-8-yl)pyrimidine-2-carbonitrile |
| 045 | (S)-4-((1-(4-chloro-8-(6-methoxypyrimidin-4-yl)-1-oxo-2-phenyl-1,2-dihydroisoquinolin-3-yl)ethyl)amino)pyrido[2,3-d]pyrimidin-5(8H)-one |
| 046 | (S)-4-((1-(4-chloro-8-(6-cyclopropylpyridin-3-yl)-1-oxo-2-phenyl-1,2-dihydroisoquinolin-3-yl)ethyl)amino)pyrido[2,3-d]pyrimidin-5(8H)-one |
| 047 | (S)-5-(4-chloro-1-oxo-3-(1-((5-oxo-5,8-dihydropyrido[2,3-d]pyrimidin-4-yl)amino) ethyl)-2-phenyl-1,2-dihydroisoquinolin-8-yl)picolinic acid |
| 048 | (S)-4-((1-(4-chloro-1-oxo-2-phenyl-8-(quinoxalin-2-yl)-1,2-dihydroisoquinolin-3-yl)ethyl)amino)pyrido[2,3-d]pyrimidin-5(8H)-one |
| 049 | (S)-4-((1-(4-chloro-8-(2,6-dimethoxypyrimidin-4-yl)-1-oxo-2-phenyl-1,2-dihydroisoquinolin-3-yl)ethyl)amino)pyrido[2,3-d]pyrimidin-5(8H)-one |
| 050 | (S)-4-((1-(4-chloro-8-(6-hydroxypyrimidin-4-yl)-1-oxo-2-phenyl-1,2-dihydroisoquinolin-3-yl)ethyl)amino)pyrido[2,3-d]pyrimidin-5(8H)-one |
| 051 | (S)-4-((1-(4-chloro-1-oxo-2-phenyl-8-(5-(trifluoromethyl)pyrazin-2-yl)-1,2-dihydroquinolin-3-yl)ethyl)amino)pyrido[2,3-d]pyrimidin-5(8H)-one |
| 052 | (S)-4-((1-(4-chloro-1-oxo-2-phenyl-8-(5-(trifluoromethyl)pyrimidin-2-yl)-1,2-dihydroisoquinolin-3-yl)ethyl)amino)pyrido[2,3-d]pyrimidin-5(8H)-one |
| 053 | (S)-4-((1-(4-chloro-8-(5-fluoropyrimidin-2-yl)-1-oxo-2-phenyl-1,2-dihydroisoquinolin-3-yl)ethyl)amino)pyrido[2,3-d]pyrimidin-5(8H)-one |
| 054 | (S)-4-((1-(4-chloro-8-(2-cyclobutylpyrimidin-5-yl)-1-oxo-2-phenyl-1,2-dihydroisoquinolin-3-yl)ethyl)amino)pyrido[2,3-d]pyrimidin-5(8H)-one |
| 055 | (S)-4-((1-(4-chloro-8-(2-(methoxymethyl)pyrimidin-5-yl)-1-oxo-2-phenyl-1,2-dihydroisoquinolin-3-yl)ethyl) amino)pyrido[2,3-d]pyrimidin-5(8H)-one |
| 056 | (S)-4-((1-(8-(2-aminopyrimidin-5-yl)-4-chloro-1-oxo-2-phenyl-1,2-dihydroisoquinolin-3-yl)ethyl)amino)pyrido[2,3-d]pyrimidin-5(8H)-one |
| 057 | 4-(((1S)-1-(4-chloro-8-(4-(1-hydroxyethyl)phenyl)-1-oxo-2-phenyl-1,2-dihydroisoquinolin-3-yl)ethyl)amino)pyrido[2,3-d]pyrimidin-5(8H)-one |
| 058 | (S)-4-((1-(4-chloro-8-(6-hydroxy-5-methylpyridin-3-yl)-1-oxo-2-phenyl-1,2-dihydroisoquinolin-3-yl)ethyl) amino)pyrido[2,3-d]pyrimidin-5(8H)-one |
| 059 | (S)-4-((1-(4-chloro-8-(4-cyclopentylpyrimidin-5-yl)-1-oxo-2-phenyl-1,2-dihydroisoquinolin-3-yl)ethyl)amino)pyrido[2,3-d]pyrimidin-5(8H)-one |
| 060 | (S)-4-((1-(8-(benzo[d][1,3]dioxol-5-yl)-4-chloro-1-oxo-2-phenyl-1,2-dihydroisoquinolin-3-yl)ethyl)amino)pyrido[2,3-d]pyrimidin-5(8H)-one |
| 061 | (S)-4-((1-(4-chloro-8-(6-methoxy-5-nitropyrimidin-3-yl)-1-oxo-2-phenyl-1,2-dihydroisoquinolin-3-yl)ethyl) amino)pyrido[2,3-d]pyrimidin-5(8H)-one |
| 062 | (S)-4-((1-(4-chloro-1-oxo-2,S-diphenyl-1,2-dihydroisoquinolin-3-yl)ethyl)amino)pyrido[2,3-d]pyrimidin-5(8H)-one |
| 063 | (S)-4-((1-(4-chloro-8-(4-fluorophenyl)-1-oxo-2-phenyl-1,2-dihydroisoquinolin-3-yl)ethyl)amino)pyrido[2,3-d]pyrimidin-5(8H)-one |
| 064 | (S)-4-((1-(4-chloro-8-(4-methoxyphenyl)-1-oxo-2-phenyl-1,2-dihydroisoquinolin-3-yl)ethyl)amino)pyrido[2,3-d]pyrimidin-5(8H)-one |
| 065 | (S)-4-((1-(8-chloro-4-cyclopropyl-1-oxo-2-phenyl-1,2-dihydroisoquinolin-3-yl)ethyl)amino)pyrido[2,3-d]pyrimidin-5(8H)-one |
| 066 | (S)-4-((1-(4-chloro-8-cyclopropyl-1-oxo-2-phenyl-1,2-dihydroisoquinolin-3-yl)ethyl)amino)pyrido[2,3-d]pyrimidin-5(8H)-one |
| 067 | (S)-4-(4-chloro-1-oxo-3-(1-((5-oxo-5,8-dihydropyrido[2,3-d]pyrimidin-4-yl)amino) ethyl)-2-phenyl-1,2-dihydroisoquinolin-8-yl)-3-methylbenzonitrile |
| 068 | (S)-4-((1-(4-chloro-1-oxo-2-phenyl-8-(4-(trifluoromethyl)phenyl)-1,2-dihydroisoquinolin-3-yl)ethyl)amino)pyrido[2,3-d]pyrimidin-5(8H)-one |
| 069 | (S)-1,4'-dichloro-3'-(1-((5-oxo-5,8-dihydropyrido[2,3-d]pyrimidin-4-yl)amino)ethyl)-2'-phenyl-(4,8'-biisoquinolin)-1'(2'H)-one |
| 070 | (S,E)-4-((1-(4-chloro-1-oxo-2-phenyl-8-styryl-1,2-dihydroisoquinolin-3-yl)ethyl)amino)pyrido[2,3-d]pyrimidin-5(8H)-one |
| 071 | (S)-4-((1-(4-chloro-8-(3,4-dichlorophenyl)-1-oxo-2-phenol-1,2-dihydroisoquinolin-3-yl)ethyl)amino)pyrido[2,3-d]pyrimidin-5(8H)-one |
| 072 | (S)-4-((1-(4-chloro-8-(4-(dimethylamino)phenyl)-1-oxo-2-phenyl-1,2-dihydroisoquinolin-3-yl)ethyl)amino)pyrido[2,3-d]pyrimidin-5(8H)-one |
| 073 | (S)-4-((1-(4-chloro-8-(1H-indol-5-yl)-1-oxo-2-phenyl-1,2-dihydroisoquinolin-3-yl)ethyl)amino)pyrido[2,3-d]pyrimidin-5(8H)-one |
| 074 | (S)-4-((1-(4-chloro-8-(3-methyl-1H-indol-5-yl)-1-oxo-2-phenyl-1,2-dihydroisoquinolin-3-yl)ethyl)amino)pyrido[2,3-d]pyrimidin-5(8H)-one |
| 075 | (S)-4-((1-(4-chloro-8-(4-morpholinophenyl)-1-oxo-2-phenyl-1,2-dihydroisoquinolin-3-yl)ethyl)amino)pyrido[2,3-d]pyrimidin-5(8H)-one |
| 076 | (S)-4-((1-(4-chloro-8-(4-isopropylpiperidin-1-yl)-1-oxo-2-phenyl-1,2-dihydroisoquinolin-3-yl)ethyl)amino)pyrido[2,3-d]pyrimidin-5(8H)-one |
| 077 | tert-butyl (S)-(1-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)piperidin-4-yl) carbamate |
| 078 | (S)-4-((1-(4-chloro-8-(4-(2-hydroxyphenyl)piperazin-1-yl)-1-oxo-2-phenyl-1,2-dihydroisoquinolin-3-yl)ethyl) amino)pyrido[2,3-d]pyrimidin-5(8H)-one |
| 079 | (S)-3-(4-chloro-1-oxo-3-(1-((5-oxo-5,8-dihydropyrido[2,3-d]pyrimidin-4-yl)amino) ethyl)-2-phenyl-1,2-dihydroisoquinolin-8-yl)benzaldehyde |
| 080 | (S)-4-(4-chloro-1-oxo-3-(1-((5-oxo-5,8-dihydropyrido[2,3-d]pyrimidin-4-yl)amino) ethyl)-2-phenyl-1,2-dihydroisoquinolin-8-yl)benzamide |
| 081 | (S)-4-(1-oxo-3-(1-((5-oxo-5,8-dihydropyrido[2,3-d]pyrimidin-4-yl)amino)ethyl)-2-phenyl-1,2-dihydroisoquinolin-8-yl)benzamide |
| 082 | (S)-4-((1-(4-chloro-8-(1,5-dimethyl-1H-pyrazol-4-yl)-1-oxo-2-phenyl-1,2-dihydroisoquinolin-3-yl)ethyl)amino)pyrido[2,3-d]pyrimidin-5(8H)-one |
| 083 | (S)-4-((1-(4-chloro-8-(1-(2-(dimethylamino)ethyl)-1H-pyrazol-4-yl)-1-oxo-2-phenyl-1,2-dihydroisoquinolin-3-yl)ethyl)amino)pyrido[2,3-d]pyrimidin-5(8H)-one |
| 084 | (S)-4-((1-(4-chloro-8-(4-chlorophenyl)-1-oxo-2-phenyl-1,2-dihydroisoquinolin-3-yl)ethyl)amino)pyrido[2,3-d]pyrimidin-5(8H)-one |
| 085 | (S)-4-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)benzaldehyde |
| 086 | (S)-4-((1-(4-chloro-8-(6-(dimethylamino)pyridin-3-yl)-1-oxo-2-phenyl-1,2-dihydroisoquinolin-3-yl)ethyl)amino)pyrido[2,3-d]pyrimidin-5(8H)-one |
| 087 | (S)-4-((1-(4-chloro-1-oxo-2-phenyl-8-(4-(pyrrolidin-1-yl)phenyl)-1,2-dihydroisoquinolin-3-yl)ethyl)amino)pyrido[2,3-d]pyrimidin-5(8H)-one |
| 088 | (S)-4-((1-(4-chloro-1-oxo-2-phenyl-8-(4-(piperidin-1-yl)phenyl)-1,2-dihydroisoquinolin-3-yl)ethyl)amino)pyrido[2,3-d]pyrimidin-5(8H)-one |
| 089 | (S)-4-((1-(4-chloro-8-(1H-indol-4-yl)-1-oxo-2-phenyl-1,2-dihydroisoquinolin-3-yl)ethyl)amino)pyrido[2,3-d]pyrimidin-5(8H)-one |
| 090 | (S)-4-((1-(8-(4-(1-methylpiperidin-4-yl)phenyl)-1-oxo-2-phenyl-1,2-dihydroisoquinolin-3-yl)ethyl)amino)pyrido[2,3-d]pyrimidin-5(8H)-one |
| 091 | (S)-2-(3-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)phenyl)acetonitrile |
| 092 | (S)-4-((1-(8-(4-(5-amino-1,3,4-thiadiazol-2-yl)phenyl)-4-chloro-1-oxo-2-phenyl-1,2-dihydroisoquinolin-3-yl)ethyl) amino)p yrido[2,3-d]pyrimidin-5(8H)-one |
| 093 | (S)-4-((1-(4-chloro-1-oxo-2-phenyl-8-(4-(thiophen-2-yl)phenyl)-1,2-dihydroisoquinolin-3-yl)ethyl)amino)pyrido[2,3-d]pyrimidin-5(8H)-one |
| 094 | (S)-4-((1-(8-(4-aminophenyl)-4-chloro-1-oxo-2-phenyl-1,2-dihydroisoquinolin-3-yl)ethyl)amino)pyrido[2,3-d]pyrimidin-5(8H)-one |
| 095 | (S)-4-((1-(4-chloro-8-(4-mercaptophenyl)-1-oxo-2-phenyl-1,2-dihydroisoquinolin-3-yl)ethyl)amino)pyrido[2,3-d]pyrimidin-5(8H)-one |
| 096 | (S)-4-((1-(4-chloro-8-(1-cyclopropyl-1H-pyrazol-4-yl)-1-oxo-2-phenyl-1,2-dihydroisoquinolin-3-yl)ethyl)amino)pyrido[2,3-d]pyrimidin-5(8H)-one |
| 097 | (S)-4-((1-(4-chloro-8-(3-chlorophenyl)-1-oxo-2-phenyl-1,2-dihydroisoquinolin-3-yl)ethyl)amino)pyrido[2,3-d]pyrimidin-5(8H)-one |
| 098 | (S)-4-((1-(4-chloro-8-(5-methylfuran-2-yl)-1-oxo-2-phenyl-1,2-dihydroisoquinolin-3-yl)ethyl)amino)pyrido[2,3-d]pyrimidin-5(8H)-one |
| 099 | (S)-4-((1-(4-chloro-8-(4-chloro-3-methoxyphenyl)-1-oxo-2-phenyl-1,2-dihydroisoquinolin-3-yl)ethyl)amino)pyrido[2,3-d]pyrimidin-5(8H)-one |
| 100 | (S)-4-((1-(8-(4-chloro-3-methoxyphenyl)-1-oxo-2-phenyl-1,2-dihydroisoquinolin-3-yl)ethyl)amino)pyrido[2,3-d]pyrimidin-5(8H)-one |
| 101 | (S)-4-((1-(4-chloro-8-(6-isopropoxypyridin-3-yl)-1-oxo-2-phenyl-1,2-dihydroisoquinolin-3-yl)ethyl)amino)pyrido[2,3-d]pyrimidin-5(8H)-one |
| 102 | (S)-4-((1-(4-chloro-1-oxo-2-phenyl-8-(pyrimidin-5-yl)-1,2-dihydroisoquinolin-3-yl)ethyl)amino)pyrido[2,3-d]pyrimidin-5(8H)-one |
| 103 | (S)-4-((1-(4-chloro-1-oxo-2-phenyl-8-(3,4,5-trimethoxyphenyl)-1,2-dihydroisoquinolin-3-yl)ethyl)amino)pyrido[2,3-d]pyrimidin-5(8H)-one |
| 104 | (S)-4-((1-(4-chloro-8-(3-chloro-5-fluorophenyl)-1-oxo-2-phenyl-1,2-dihydroisoquinolin-3-yl)ethyl)amino)pyrido[2,3-d]pyrimidin-5(8H)-one |
| 105 | (S)-4-((1-(4-chloro-1-oxo-2-phenyl-8-(4-(pyridin-4-yl)phenyl)-1,2-dihydroisoquinolin-3-yl)ethyl)amino)pyrido[2,3-d]pyrimidin-5(8H)-one |
| 106 | (S)-4-((1-(4-chloro-8-(2-chloro-3-fluorophenyl)-1-oxo-2-phenyl-1,2-dihydroisoquinolin-3-yl)ethyl)amino)pyrido[2,3-d]pyrimidin-5(8H)-one |
| 107 | (S)-4-((1-(4-chloro-8-(2-chloro-5-fluorophenyl)-1-oxo-2-phenyl-1,2-dihydroisoquinolin-3-yl)ethyl)amino)pyrido[2,3-d]pyrimidin-5(8H)-one |
| 108 | (S)-4-((1-(4-chloro-8-(3-fluoro-5-hydroxyphenyl)-1-oxo-2-phenyl-1,2-dihydroisoquinolin-3-yl)ethyl)amino)pyrido[2,3-d]pyrimidin-5(8H)-one |
| 109 | (S)-4-((1-(4-chloro-8-(3-fluoro-2-methylphenyl)-1-oxo-2-phenyl-1,2-dihydroisoquinolin-3-yl)ethyl)amino)pyrido[2,3-d]pyrimidin-5(8H)-one |
| 110 | (S)-4-((1-(4-chloro-8-(2-methylpyridin-3-yl)-1-oxo-2-phenyl-1,2-dihydroisoquinolin-3-yl)ethyl)amino)pyrido[2,3-d]pyrimidin-5(8H)-one |
| 111 | (S)-4-((1-(4-chloro-8-(1-methyl-1H-benzo[d]imidazol-6-yl)-1-oxo-2-phenyl-1,2-dihydroisoquinolin-3-yl)ethyl)amino)pyrido[2,3-d]pyrimidin-5(8H)-one |
| 112 | (S)-4-((1-(4-chloro-8-(2-(dimethylamino)pyrimidin-5-yl)-1-oxo-2-phenyl-1,2-dihydroisoquinolin-3-yl)ethyl)amino)pyrido[2,3-d]pyrimidin-5(8H)-one |
| 113 | (S)-4-((1-(4-chloro-8-(imidazo[1,2-a]pyridin-6-yl)-1-oxo-2-phenyl-1,2-dihydroisoquinolin-3-yl)ethyl)amino)pyrido[2,3-d]pyrimidin-5(8H)-one |
| 114 | (S)-4-((1-(4-chloro-8-(1-methyl-1H-benzo[d]imidazol-5-yl)-1-oxo-2-phenyl-1,2-dihydroisoquinolin-3-yl)ethyl)amino)pyrido[2,3-d]pyrimidin-5(8H)-one |
| 115 | (S)-4-((1-(4-chloro-1-oxo-2-phenyl-8-(6-phenylpyridin-3-yl)-1,2-dihydroisoquinolin-3-yl)ethyl)amino)pyrido[2,3-d]pyrimidin-5(8H)-one |
| 116 | (S)-4-((1-(4-chloro-S-(1-methyl-1H-indol-5-yl)-1-oxo-2-phenyl-1,2-dihydroisoquinolin-3-yl)ethyl)amino)pyrido[2,3-d]pyrimidin-5(8H)-one |
| 117 | (S)-4-((1-(4-chloro-8-(furan-2-yl)-1-oxo-2-phenyl-1,2-dihydroisoquinolin-3-yl)ethyl)amino)pyrido[2,3-d]pyrimidin-5(8H)-one |
| 118 | (S)-4-((1-(4-chloro-8-(4-cyclobutylphenyl)-l-oxo-2-phenyl-1,2-dihydroisoquinolin-3-yl)ethyl)amino)pyrido[2,3-d]pyrimidin-5(8H)-one |
| 119 | (S)-4-((1-(4-chloro-8-(5-methylpyridin-3-yl)-1-oxo-2-phenyl-1,2-dihydroisoquinolin-3-yl)ethyl)amino)pyrido[2,3-d]pyrimidin-5(8H)-one |
| 120 | (S)-4-((1-(4-chloro-8-(3,4-dihydro-2H-benzo[b][1,4]oxazin-7-yl)-1-oxo-2-phenyl-1,2-dihydroisoquinolin-3-yl)ethyl) amino)p yrido[2,3-d]pyrimidin-5(8H)-one |
| 121 | (S)-4-((1-(4-chloro-1-oxo-2-phenyl-8-(quinolin-6-yl)-1,2-dihydroisoquinolin-3-yl)ethyl)amino)pyrido[2,3-d]pyrimidin-5(8H)-one |
| 122 | (S)-4-((1-(4-chloro-8-(2-fluorophenyl)-1-oxo-2-phenyl-1,2-dihydroisoquinolin-3-yl)ethyl)amino)pyrido[2,3-d]pyrimidin-5(8H)-one |
| 123 | (S)-4-((1-(4-chloro-8-(3-fluorophenyl)-1-oxo-2-phenyl-1,2-dihydroisoquinolin-3-yl)ethyl)amino)pyrido[2,3-d]pyrimidin-5(8H)-one |
| 124 | (S)-4-((1-(4-chloro-8-(3,5-difluorophenyl)-1-oxo-2-phenyl-1,2-dihydroisoquinolin-3-yl)ethyl)amino)pyrido[2,3-d]pyrimidin-5(8H)-one |
| 125 | (S)-4-((1-(8-(1H-benzo[d]imidazol-5-yl)-4-chloro-1-oxo-2-phenyl-1,2-dihydroisoquinolin-3-yl)ethyl)amino)pyrido[2,3-d]pyrimidin-5(8H)-one |
| 126 | (S)-4-((1-(4-chloro-8-(4-hydroxy-3,5-dimethylphenyl)-1-oxo-2-phenyl-1,2-dihydroisoquinolin-3-yl) ethyl) amino)pyrido[2,3-d]pyrimidin-5(8H)-one |
| 127 | (S)-4-((1-(4-chloro-8-(5-fluoro-2-methylphenyl)-1-oxo-2-phenyl-1, 2-dihydroisoquinolin-3-yl)ethyl)amino)pyrido[2,3-d]pyrimidin-5(8H)-one |
| 128 | (S)-4-((1-(4-chloro-8-(4-fluoro-3-methylphenyl)-1-oxo-2-phenyl-1,2-dihydroisoquinolin-3-yl)ethyl)amino)pyrido[2,3-d]pyrimidin-5(8H)-one |
| 129 | (S)-4-((1-(4-chloro-8-(2,3-difluorophenyl)-1-oxo-2-phenyl-1,2-dihydroisoquinolin-3-yl)ethyl)amino)pyrido[2,3-d]pyrimidin-5(8H)-one |
| 130 | (S)-4-((1-(4-chloro-8-(4-(1-methylpiperidin-4-yl)phenyl)-1-oxo-2-phenyl-1,2-dihydroisoquinolin-3-yl) ethyl) amino)pyrido[2,3-d]pyrimidin-5(8H)-one |
| 131 | (S)-4-((1-(4-chloro-8-(4-fluoro-2-methylphenyl)-l-oxo-2-phenyl-1,2-dihydroisoquinolin-3-yl)ethyl)amino)pyrido[2,3-d]pyrimidin-5(8H)-one |
| 132 | (S)-4-((1-(4-chloro-8-(2,3-dihydro-[1,4]dioxino[2,3-b]pyridin-7-yl)-1-oxo-2-phenyl-1,2-dihydroisoquinolin-3-yl)ethyl)amino)pyrido[2,3-d]pyrimidin-5(8H)-one |
| 133 | (S)-4-((1-(4-chloro-8-(6-methoxypyridin-3-yl)-1-oxo-2-phenyl-1,2-dihydroisoquinolin-3-yl)ethyl)amino)pyrido[2,3-d]pyrimidin-5(8H)-one |
| 134 | (S)-4-((1-(4-chloro-1-oxo-2-phenyl-8-(pyridin-4-yl)-1,2-dihydroisoquinolin-3-yl)ethyl)amino)pyrido[2,3-d]pyrimidin-5(8H)-one |
| 135 | (S)-4-((1-(4-chloro-8-(2-methyl-1H-benzo[d]imidazol-5-yl)-1-oxo-2-phenyl-1,2-dihydroisoquinolin-3-yl) ethyl) amino)pyrido[2,3-d]pyrimidin-5(8H)-one |
| 136 | (S)-4-((1-(4-chloro-8-(2-methylpyrimidin-5-yl)-1-oxo-2-phenyl-1,2-dihydroisoquinolin-3-yl)ethyl)amino)pyrido[2,3-d]pyrimidin-5(8H)-one |
| 137 | (S)-4-((1-(4-chloro-1-oxo-2-phenyl-8-(quinoxalin-6-yl)-1,2-dihydroisoquinolin-3-yl)ethyl)amino)pyrido[2,3-d]pyrimidin-5(8H)-one |
| 138 | tert-butyl (S)-4-(4-(4-chloro-1-oxo-3-(1-((5-oxo-5,8-dihydropyrido[2,3-d]pyrimidin-4-yl) amino) ethyl)-2-phenyl-1,2-dihydroisoquinolin-8-yl)phenyl) piperazine-1-carboxylate |
| 139 | (S)-4-(4-chloro-1-oxo-3-(1-((5-oxo-5,8-dihydropyrido[2,3-d]pyrimidin-4-yl)amino) ethyl)-2-phenyl-1,2-dihydroisoquinolin-8-yl)-N,N-dimethylbenzamide |
| 140 | (S)-4-((1-(4-chloro-8-(5-chloro-2-fluorophenyl)-1-oxo-2-phenyl-1,2-dihydroisoquinolin-3-yl)ethyl)amino)pyrido[2,3-d]pyrimidin-5(8H)-one |
| 141 | (S)-4-((1-(4-chloro-8-(4-cyclopropylphenyl)-1-oxo-2-phenyl-1,2-dihydroisoquinolin-3-yl)ethyl)amino)pyrido[2,3-d]pyrimidin-5(8H)-one |
| 142 | (S)-4-((1-(4-chloro-l-oxo-2-phenyl-8-vinyl-1,2-dihydroisoquinolin-3-yl)ethyl)amino)pyrido[2,3-d]pyrimidin-5(8H)-one |
| 143 | (S)-4-((1-(4-chloro-S-(4-(methylthio)phenyl)-1-oxo-2-phenyl-1,2-dihydroisoquinolin-3-yl)ethyl)amino)pyrido[2,3-d]pyrimidin-5(8H)-one |
| 144 | (3)-4-((1-(S-(4-(tert-butylthio)phenyl)-4-chloro-1-oxo-2-phenyl-1,2-dihydroisoquinolin-3-yl)ethyl)amino)pyrido[2,3-d]pyrimidin-5(8H)-one |
| 145 | (S)-4-((1-(4-chloro-8-(4-(morpholine-4-carbonyl)phenyl)-1-oxo-2-phenyl-1,2-dihydroisoquinolin-3-yl) ethyl) amino)pyrido[2,3-d]pyrimidin-5(8H)-one |
| 146 | (S)-4-((1-(8-(2-(benzyloxy)pyrimidin-5-yl)-4-chloro-1-oxo-2-phenyl-1,2-dihydroisoquinolin-3-yl)ethyl)amino)pyrido[2,3-d]pyrimidin-5(8H)-one |
| 147 | (S)-4-((1-(4-chloro-1-oxo-2-phenyl-8-(6-(2,2,2-trifluoroethoxy)pyridin-3-yl)-1,2-dihydroisoquinolin-3-yl)ethyl)amino)pyrido[2,3-d]pyrimidin-5(8H)-one |
| 148 | (S)-4-((1-(4-chloro-1-oxo-2-phenyl-8-(4-(piperazin-l-yl)phenyl)-1,2-dihydroisoquinolin-3-yl)ethyl)amino)pyrido[2,3-d]pyrimidin-5(8H)-one |
| 149 | (S)-4-((1-(4-chloro-8-(2-methoxypyridin-4-yl)-1-oxo-2-phenyl-1,2-dihydroisoquinolin-3-yl)ethyl)amino)-8-(2-methoxypyridin-4-yl)pyrido[2,3-d]pyrimidin-5(8H)-one |
| 150 | (S)-4-((1-(4-chloro-8-(2-methoxypyridin-4-yl)-1-oxo-2-phenyl-1,2-dihydroisoquinolin-3-yl)ethyl)amino)pyrido[2,3-d]pyrimidin-5(8H)-one |
| 151 | (S)-4-((1-(4-chloro-1-oxo-2-phenyl-8-(4-(propylthio)phenyl)-1,2-dihydroisoquinolin-3-yl)ethyl)amino)pyrido[2,3-d]pyrimidin-5(8H)-one |
| 152 | (S)-4-((1-(4-chloro-8-(4-(cyclopropylthio)phenyl)-1-oxo-2-phenyl-1,2-dihydroisoquinolin-3-yl)ethyl)amino)pyrido[2,3-d]pyrimidin-5(8H)-one |
| 153 | (S)-4-((1-(4-chloro-1-oxo-2-phenyl-8-(2-(trifluoromethyl)pyrimidin-5-yl)-1,2-dihydroisoquinolin-3-yl)ethyl) amino)pyrido[2,3-d]pyrimidin-5(8H)-one |
| 154 | (S)-4-((1-(4-chloro-8-(4-fluoro-3,5-dimethoxyphenyl)-1-oxo-2-phenyl-1,2-dihydroisoquinolin-3-yl) ethyl) amino)pyrido[2,3-d]pyrimidin-5(8H)-one |
| 155 | (S)-4-((1-(4-chloro-8-(3,4-dimethoxyphenyl)-1-oxo-2-phenyl-1,2-dihydroisoquinolin-3-yl)ethyl)amino)pyrido[2,3-d]pyrimidin-5(8H)-one |
| 156 | (S)-4-((1-(4-chloro-8-(cyclopent-1-en-1-yl)-1-oxo-2-phenyl-1,2-dihydroisoquinolin-3-yl)ethyl)amino)pyrido[2,3-d]pyrimidin-5(8H)-one |
| 157 | (S)-4-((1-(4-chloro-8-(3-isopropoxy-5-methoxyphenyl)-1-oxo-2-phenyl-1,2-dihydroisoquinolin-3-yl) ethyl) amino)pyrido[2,3-d]pyrimidin-5(8H)-one |
| 158 | (S)-4-((1-(4-chloro-8-(3-methoxy-5-(trifluoromethoxy)phenyl)-1-oxo-2-phenyl-1,2-dihydroisoquinolin-3-yl) ethyl) amino)pyrid o[2,3-d]pyrimidin-5(8H)-one |
| 159 | (S)-4-((1-(4-chloro-8-(3-methoxy-4-(trifluoromethoxy)phenyl)-1-oxo-2-phenyl-1,2-dihydroisoquinolin-3-yl) ethyl) amino)pyrid o[2,3-d]pyrimidin-5(8H)-one |
| 160 | (S)-4-((1-(4-chloro-8-(4-(5-methyl-1,2,4-oxadiazol-3-yl)phenyl)-1-oxo-2-phenyl-1,2-dihydroisoquinolin-3-yl) ethyl) amino)p yrido[2,3-d]pyrimidin-5(8H)-one |
| 161 | (S)-4-((1-(8-(4-(5-(tert-butyl)-1,2,4-oxadiazol-3-yl)phenyl)-4-chloro-1-oxo-2-phenyl-1,2-dihydroisoquinolin-3-yl)ethyl)amino)pyrido[2,3-d]pyrimidin-5(8H)-one |
| 162 | (S)-4-((1-(4-chloro-1-oxo-2-phenyl-8-(6-((tetrahydro-2H-pyran-4-yl)oxy)pyridin-3-yl)-1,2-dihydroisoquinolin-3-yl)ethyl)amino)pyrido[2,3-d]pyrimidin-5(8H)-one |
| 163 | (S)-4-((1-(4-chloro-8-(6-(cyclopropylmethoxy)pyridin-3-yl)-1-oxo-2-phenyl-1,2-dihydroisoquinolin-3-yl) ethyl) amino)pyrido[2,3-d]pyrimidin-5(8H)-one |
| 164 | (S)-4-((1-(8-(6-(benzyloxy)pyridin-3-yl)-4-chloro-1-oxo-2-phenyl-1,2-dihydroisoquinolin-3-yl)ethyl)amino)pyrido[2,3-d]pyrimidin-5(8H)-one |
| 165 | (S)-4-((1-(4-chloro-8-(4-(methylsulfonyl)phenyl)-l-oxo-2-phenyl-1,2-dihydroisoquinolin-3-yl)ethyl)amino)pyrido[2,3-d]pyrimidin-5(8H)-one |
| 166 | (S)-4-(4-chloro-1-oxo-3-(1-((5-oxo-5,8-dihydropyrido[2,3-d]pyrimidin-4-yl)amino) ethyl)-2-phenyl-1,2-dihydroisoquinolin-8-yl)benzoic acid |
| 167 | (S)-4-(4-chloro-1-oxo-3-(1-((5-oxo-5,8-dihydropyrido[2,3-d]pyrimidin-4-yl)amino) ethyl)-2-phenyl-1,2-dihydroisoquinolin-8-yl)-N-methylbenzamide |
| 168 | (S)-4-((1-(4-chloro-8-(2-cyclopropylpyrimidin-5-yl)-1-oxo-2-phenyl-1,2-dihydroisoquinolin-3-yl)ethyl)amino)pyrido[2,3-d]pyrimidin-5(8H)-one |
| 169 | (S)-4-((1-(4-chloro-8-(3,5-dimethoxyphenyl)-1-oxo-2-phenyl-1,2-dihydroisoquinolin-3-yl)ethyl)amino)pyrido[2,3-d]pyrimidin-5(8H)-one |
| 170 | (S)-4-((1-(4-chloro-8-(5,6-dimethoxypyridin-3-yl)-1-oxo-2-phenyl-1,2-dihydroisoquinolin-3-yl)ethyl)amino)pyrido[2,3-d]pyrimidin-5(8H)-one |
| 171 | (S)-4-((1-(4-chloro-8-(2-methoxypyrimidin-5-yl)-1-oxo-2-phenyl-1,2-dihydroisoquinolin-3-yl)ethyl)amino)pyrido[2,3-d]pyrimidin-5(8H)-one |
| 172 | (S)-4-((1-(8-(6-aminopyridin-3-yl)-4-chloro-1-oxo-2-phenyl-1,2-dihydroisoquinolin-3-yl)ethyl)amino)pyrido[2,3-d]pyrimidin-5(8H)-one |
| 173 | (S)-4-((1-(4-chloro-8-(3-morpholinophenyl)-1-oxo-2-phenyl-1,2-dihydroisoquinolin-3-yl)ethyl)amino)pyrido[2,3-d]pyrimidin-5(8H)-one |
| 174 | (S)-4-((1-(4-chloro-8-(2-morpholinopyrimidin-5-yl)-l-oxo-2-phenyl-1,2-dihydroisoquinolin-3-yl)ethyl)amino)pyrido[2,3-d]pyrimidin-5(8H)-one |
| 175 | (S)-4-((1-(4-chloro-8-(6-morpholinopyridin-3-yl)-1-oxo-2-phenyl-1,2-dihydroisoquinolin-3-yl)ethyl)amino)pyrido[2,3-d]pyrimidin-5(8H)-one |
| 176 | (S)-4-((1-(4-chloro-1-oxo-2-phenyl-8-(4-(tetrahydro-2H-pyran-4-yl)phenyl)-1,2-dihydroisoquinolin-3-yl) ethyl)amino)pyrido[2,3-d]pyrimidin-5(8H)-one |
| 177 | (S)-4-((1-(4-chloro-8-(3,5-dimethoxy-4-methylphenyl)-1-oxo-2-phenyl-1,2-dihydroisoquinolin-3-yl)ethyl)amino)pyrido[2,3-d]pyrimidin-5(8H)-one |
| 178 | (S)-4-((1-(4-chloro-1-oxo-2-phenyl-8-(4-(trimethylsilyl)phenyl)-1,2-dihydroisoquinolin-3-yl)ethyl)amino)pyrido[2,3-d]pyrimidin-5(8H)-one |
| 179 | (S)-4-((1-(4-chloro-8-(4-(4-methylpiperazin-1-yl)phenyl)-1-oxo-2-phenyl-1,2-dihydroisoquinolin-3-yl) ethyl) amino)pyrido[2,3-d]pyrimidin-5(8H)-one |
| 180 | (S)-4-((1-(4-chloro-8-(2-isopropoxypyridin-4-yl)-1-oxo-2-phenyl-1,2-dihydroisoquinolin-3-yl)ethyl)amino)pyrido[2,3-d]pyrimidin-5(8H)-one |
| 181 | (S)-4-((1-(4-chloro-1-oxo-2-phenyl-8-(6-(trifluoromethyl)pyridin-3-yl)-1,2-dihydroisoquinolin-3-yl)ethyl)amino)pyrido[2,3-d]pyrimidin-5(8H)-one |
| 182 | (S)-4-((1-(4-chloro-8-(2-isopropoxypyrimidin-5-yl)-1-oxo-2-phenyl-1,2-dihydroisoquinolin-3-yl)ethyl)amino)pyrido[2,3-d]pyrimidin-5(8H)-one |
| 183 | (S)-4-((1-(4-chloro-8-(6-ethoxypyridin-3-yl)-1-oxo-2-phenyl-1,2-dihydroisoquinolin-3-yl)ethyl)amino)pyrido[2,3-d]pyrimidin-5(8H)-one |
| 184 | (S)-4-((1-(4-chloro-1-oxo-2-phenyl-8-(2-(2,2,2-trifluoroethoxy)pyrimidin-5-yl)-1,2-dihydroisoquinolin-3-yl)ethyl)amino)pyrido[2,3-d]pyrimidin-5(8H)-one |
| 185 | (S)-4-((1-(4-chloro-1-oxo-8-(6-phenoxypyridin-3-yl)-2-phenyl-1,2-dihydroisoquinolin-3-yl)ethyl)amino)pyrido[2,3-d]pyrimidin-5(8H)-one |
| 186 | (S)-4-((1-(4-chloro-8-(5-fluoro-6-methoxypyridin-3-yl)-1-oxo-2-phenyl-1,2-dihydroisoquinolin-3-yl) ethyl) amino)pyrido[2,3-d]pyrimidin-5(8H)-one |
| 187 | (S)-4-((1-(4-chloro-8-(5-chloro-6-methoxypyridin-3-yl)-1-oxo-2-phenyl-1,2-dihydroisoquinolin-3-yl) ethyl) amino)pyrido[2,3-d]pyrimidin-5(8H)-one |
| 188 | (S)-4-((1-(4-chloro-8-(2-ethoxypyrimidin-5-yl)-1-oxo-2-phenyl-1,2-dihydroisoquinolin-3-yl)ethyl)amino)pyrido[2,3-d]pyrimidin-5(8H)-one |
| 189 | (S)-4-((1-(4-chloro-8-(2,4-dimethoxypyrimidin-5-yl)-1-oxo-2-phenyl-1,2-dihydroisoquinolin-3-yl)ethyl)amino)pyrido[2,3-d]pyrimidin-5(8H)-one |
| 190 | (S)-4-((1-(4-chloro-8-(2-morpholinopyridin-4-yl)-1-oxo-2-phenyl-1,2-dihydroisoquinolin-3-yl)ethyl)amino)pyrido[2,3-d]pyrimidin-5(8H)-one |
| 191 | (S)-4-((1-(4-chloro-8-(3-fluoro-4-methoxyphenyl)-1-oxo-2-phenyl-1,2-dihydroisoquinolin-3-yl)ethyl)amino)pyrido[2,3-d]pyrimidin-5(8H)-one |
| 192 | (S)-4-((1-(4-chloro-8-(8-fluoro-3,4-dihydro-2H-benzo[b][1,4]oxazin-6-yl)-1-oxo-2-phenyl-1,2-dihydroisoquinolin-3-yl)ethyl)amino)pyrido[2,3-d]pyrimidin-5(8H)-one |
| 193 | (S)-4-(4-chloro-1-oxo-3-(1-((5-oxo-5,8-dihydropyrido[2,3-d]pyrimidin-4-yl)amino) ethyl)-2-phenyl-1,2-dihydroisoquinolin-8-yl)-N-isopropylbenzamide |
| 194 | (S)-4-((1-(4-chloro-1-oxo-2-phenyl-8-(6-(piperidin-1-yl)pyridin-3-yl)-1,2-dihydroisoquinolin-3-yl)ethyl)amino)pyrido[2,3-d]pyrimidin-5(8H)-one |
| 195 | (S)-4-((1-(4-chloro-8-(2-fluoropyridin-4-yl)-1-oxo-2-phenyl-1,2-dihydroisoquinolin-3-yl)ethyl)amino)pyrido[2,3-d]pyrimidin-5(8H)-one |
| 196 | (S)-5-(4-chloro-1-oxo-3-(1-((5-oxo-5,8-dihydropyrido[2,3-d]pyrimidin-4-yl)amino) ethyl)-2-phenyl-1,2-dihydroisoquinolin-8-yl)-N-methylpicolinamide |
| 197 | (S)-4-((1-(4-chloro-8-(6-methoxy-5-methylpyridin-3-yl)-1-oxo-2-phenyl-1,2-dihydroisoquinolin-3-yl) ethyl) amino)pyrido[2,3-d]pyrimidin-5(8H)-one |
| 198 | (S)-4-((1-(4-chloro-1-oxo-2-phenyl-8-(pyrazin-2-yl)-1,2-dihydroisoquinolin-3-yl)ethyl)amino)pyrido[2,3-d]pyrimidin-5(8H)-one |
| 199 | (S)-4-((1-(4-chloro-8-(3-ethoxy-2-fluoro-4-methoxyphenyl)-1-oxo-2-phenyl-1,2-dihydroisoquinolin-3-yl) ethyl) amino)pyrido[2,3-d]pyrimidin-5(8H)-one |
| 200 | (S)-4-((1-(8-(2-(azetidin-1-yl)pyrimidin-5-yl)-4-chloro-1-oxo-2-phenyl-1,2-dihydroisoquinolin-3-yl) ethyl) amino)pyrido[2,3-d]pyrimidin-5(8H)-one |
| 201 | (S)-5-(4-chloro-1-oxo-3-(1-((5-oxo-5,8-dihydropyrido[2,3-d]pyrimidin-4-yl)amino) ethyl)-2-phenyl-1,2-dihydroisoquinolin-8-yl)picolinamide |
| 202 | (S)-N-(5-(4-chloro-1-oxo-3-(1-((5-oxo-5,8-dihydropyrido[2,3-d]pyrimidin-4-yl)amino) ethyl)-2-phenyl-1,2-dihydroisoquinolin-8-yl)pyridin-2-yl) acetamide |
| 203 | (S)-4-((1-(4-chloro-8-(2-(cyclopropylamino)pyrimidin-5-yl)-1-oxo-2-phenyl-1,2-dihydroisoquinolin-3-yl) ethyl) amino)pyrido[2,3-d]pyrimidin-5(8H)-one |
| 204 | (S)-4-((1-(4-chloro-8-(6-methoxypyrazin-2-yl)-1-oxo-2-phenyl-1,2-dihydroisoquinolin-3-yl)ethyl)amino)pyrido[2,3-d]pyrimidin-5(8H)-one |
| 205 | (S)-4-((1-(4-chloro-8-(5-(dimethylamino)pyrazin-2-yl)-1-oxo-2-phenyl-1,2-dihydroisoquinolin-3-yl) ethyl) amino)pyrido[2,3-d]pyrimidin-5(8H)-one |
| 206 | (S)-4-((1-(8-(5-(tert-butoxy)pyrazin-2-yl)-4-chloro-1-oxo-2-phenyl-1,2-dihydroisoquinolin-3-yl)ethyl)amino)pyrido[2,3-d]pyrimidin-5(8H)-one |
| 207 | (S)-4-((1-(4-chloro-8-(5-morpholinopyrazin-2-yl)-1-oxo-2-phenyl-1,2-dihydroisoquinolin-3-yl)ethyl)amino)pyrido[2,3-d]pyrimidin-5(8H)-one |
| 208 | (S)-4-((1-(8-(2-(tert-butyl)pyrimidin-5-yl)-4-chloro-1-oxo-2-phenyl-1,2-dihydroisoquinolin-3-yl) ethyl) amino)pyrido[2,3-d]pyrimidin-5(8H)-one |
| 209 | (S)-4-((1-(4-chloro-8-(4-(isopropylsulfonyl)phenyl)-1-oxo-2-phenyl-1,2-dihydroisoquinolin-3-yl)ethyl)amino)pyrido[2,3-d]pyrimidin-5(8H)-one |
| 210 | (S)-4-((1-(4-chloro-8-(6-morpholinopyrazin-2-yl)-1-oxo-2-phenyl-1,2-dihydroisoquinolin-3-yl)ethyl)amino)pyrido[2,3-d]pyrimidin-5(8H)-one |
| 211 | (S)-4-((1-(4-chloro-8-(5-(4-methylpiperazin-1-yl)pyrazin-2-yl)-1-oxo-2-phenyl-1,2-dihydroisoquinolin-3-yl)ethyl) amino)p yrido[2,3-d]pyrimidin-5(8H)-one |
| 212 | (S)-4-((1-(8-(2-amino-4-methylpyrimidin-5-yl)-4-chloro-1-oxo-2-phenyl-1,2-dihydroisoquinolin-3-yl) ethyl)amino)pyrido[2,3-d]pyrimidin-5(8H)-one |
| 213 | (S)-4-((1-(4-chloro-1-oxo-2-phenyl-8-(quinolin-3-yl)-1,2-dihydroisoquinolin-3-yl)ethyl)amino)pyrido[2,3-d]pyrimidin-5(8H)-one |
| 214 | (S)-4-((1-(4-chloro-8-(2-(methylthio)pyrimidin-5-yl)-1-oxo-2-phenyl-1,2-dihydroisoquinolin-3-yl) ethyl)amino)pyrido[2,3-d]pyrimidin-5(8H)-one |
| 215 | (S)-4-((1-(4-chloro-8-(4-isopropylpyrimidin-5-yl)-1-oxo-2-phenyl-1,2-dihydroisoquinolin-3-yl)ethyl)amino)pyrido[2,3-d]pyrimidin-5(8H)-one |
| 216 | (S)-4-((1-(4-chloro-8-(2-(4-methylpiperazin-1-yl)pyrimidin-5-yl)-1-oxo-2-phenyl-1,2-dihydroisoquinolin-3-yl) ethyl) amino)p yrido[2,3-d]pyrimidin-5(8H)-one |
| 217 | (S)-4-((1-(4-chloro-8-(2-(diethylamino)pyrimidin-5-yl)-1-oxo-2-phenyl-1,2-dihydroisoquinolin-3-yl)ethyl)amino)pyrido[2,3-d]pyrimidin-5(8H)-one |
| 218 | (S)-4-((1-(4-chloro-8-(5-(methylsulfonyl)pyridin-3-yl)-1-oxo-2-phenyl-1,2-dihydroisoquinolin-3-yl)ethyl)amino)pyrido[2,3-d]pyrimidin-5(8H)-one |
| 219 | (S)-4-((1-(4-chloro-8-(6-(methylsulfonyl)pyridin-3-yl)-1-oxo-2-phenyl-1,2-dihydroisoquinolin-3-yl)ethyl)amino)pyrido[2,3-d]pyrimidin-5(8H)-one |
| 220 | (S)-4-((1-(4-chloro-8-(2-(isopropylamino)pyrimidin-5-yl)-1-oxo-2-phenyl-1,2-dihydroisoquinolin-3-yl)ethyl)amino)pyrido[2,3-d]pyrimidin-5(8H)-one |
| 221 | (S)-4-((1-(4-chloro-8-(5-methoxypyrazin-2-yl)-1-oxo-2-phenyl-1,2-dihydroisoquinolin-3-yl)ethyl)amino)pyrido[2,3-d]pyrimidin-5(8H)-one |
| 222 | (S)-4-((1-(4-chloro-1-oxo-2-phenyl-8-(2-(pyrrolidin-1-yl)pyrimidin-5-yl)-1,2-dihydroisoquinolin-3-yl)ethyl)amino)pyrido[2,3-d]pyrimidin-5(8H)-one |
| 223 | (S)-4-((1-(4-chloro-1-oxo-2-phenyl-8-(6-(trifluoromethoxy)pyridin-3-yl)-1,2-dihydroisoquinolin-3-yl)ethyl)amino)pyrido[2,3-d]pyrimidin-5(8H)-one |
| 224 | (S)-4-((1-(4-chloro-1-oxo-2-phenyl-8-(2-(piperidin-1-yl)pyrimidin-5-yl)-1,2-dihydroisoquinolin-3-yl)ethyl)amino)pyrido[2,3-d]pyrimidin-5(8H)-one |
| 225 | (S)-N-(5-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-2-methoxypyridin-3-yl)methanesulfonamide |
| 226 | (S)-4-((1-(4-chloro-1-oxo-2-phenyl-8-(1H-pyrazol-3-yl)-1,2-dihydroisoquinolin-3-yl)ethyl)amino)pyrido[2,3-d]pyrimidin-5(8H)-one |
| 227 | (S)-4-((1-(4-chloro-1-oxo-2-phenyl-8-(1H-pyrazol-4-yl)-1,2-dihydroisoquinolin-3-yl)ethyl)amino)pyrido[2,3-d]pyrimidin-5(8H)-one |
| 228 | (S)-4-((1-(4-chloro-8-(2-(ethylamino)pyrimidin-5-yl)-1-oxo-2-phenyl-1,2-dihydroisoquinolin-3-yl)ethyl)amino)pyrido[2,3-d]pyrimidin-5(8H)-one |
| 229 | (S)-5-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-2-methoxynicotinonitrile |
| 230 | (S)-4-((1-(4-chloro-8-(5-ethoxypyrazin-2-yl)-1-oxo-2-phenyl-1,2-dihydroisoquinolin-3-yl)ethyl)amino)pyrido[2,3-d]pyrimidin-5(8H)-one |
| 231 | (S)-4-((1-(4-chloro-8-(2-fluoro-4-(morpholinomethyl)phenyl)-1-oxo-2-phenyl-1,2-dihydroisoquinolin-3-yl) ethyl) amino)pyrid o[2,3-d]pyrimidin-5(8H)-one |
| 232 | (S)-N-(5-(4-chloro-1-oxo-3-(1-((5-oxo-5,8-dihydropyrido[2,3-d]pyrimidin-4-yl)amino) ethyl)-2-phenyl-1,2-dihydroisoquinolin-8-yl)-2-methoxypyridin-3-yl)-2,4-difluorobenzenesulfonamide |
| 233 | (S)-4-((1-(8-(benzo[c] [1,2,5]thiadiazol-5-yl)-4-chloro-1-oxo-2-phenyl-1,2-dihydroisoquinolin-3-yl) ethyl)amino)pyrido[2,3-d]pyrimidin-5(8H)-one |
| 234 | (S)-4-((1-(4-chloro-8-(5-methyl-1H-pyrazol-3-yl)-1-oxo-2-phenyl-1,2-dihydroisoquinolin-3-yl)ethyl)amino)pyrido[2,3-d]pyrimidin-5(8H)-one |
| 235 | (S)-4-((1-(8-([1,2,5]oxadiazolo[3,4-b]pyridin-6-yl)-4-chloro-1-oxo-2-phenyl-1,2-dihydroisoquinolin-3-yl) ethyl) amino)pyrid o[2,3-d]pyrimidin-5(8H)-one |
| 236 | (S)-4-((1-(4-chloro-8-(1H-indazol-5-yl)-1-oxo-2-phenyl-1,2-dihydroisoquinolin-3-yl)ethyl)amino)pyrido[2,3-d]pyrimidin-5(8H)-one |
| 237 | (S)-4-((1-(4-chloro-8-(1-methyl-1H-indazol-4-yl)-1-oxo-2-phenyl-1,2-dihydroisoquinolin-3-yl)ethyl)amino)pyrido[2,3-d]pyrimidin-5(8H)-one |
| 238 | (S)-4-((1-(4-chloro-S-(isoxazol-4-yl)-1-oxo-2-phenyl-1,2-dihydroisoquinolin-3-yl)ethyl)amino)pyrido[2,3-d]pyrimidin-5(8H)-one |
| 239 | (S)-4-((1-(4-chloro-8-(1H-indazol-7-yl)-1-oxo-2-phenyl-1,2-dihydroisoquinolin-3-yl)ethyl)amino)pyrido[2,3-d]pyrimidin-5(8H)-one |
| 240 | (S)-4-((1-(8-(benzo[c] [1,2,5]oxadiazol-5-yl)-4-chloro-1-oxo-2-phenyl-1,2-dihydroisoquinolin-3-yl)ethyl)amino)pyrido[2,3-d]pyrimidin-5(8H)-one |
| 241 | (S)-4-((1-(8-(benzo[d]oxazol-5-yl)-4-chloro-1-oxo-2-phenyl-1,2-dihydroisoquinolin-3-yl)ethyl)amino)pyrido[2,3-d]pyrimidin-5(8H)-one |
| 242 | (S)-4-((1-(4-chloro-8-(4-methylthiophen-2-yl)-1-oxo-2-phenyl-1,2-dihydroisoquinolin-3-yl)ethyl)amino)pyrido[2,3-d]pyrimidin-5(8H)-one |
| 243 | (S)-4-((1-(4-chloro-8-(3-methylthiophen-2-yl)-1-oxo-2-phenyl-1,2-dihydroisoquinolin-3-yl)ethyl)amino)pyrido[2,3-d]pyrimidin-5(8H)-one |
| 244 | (S)-4-((1-(4-chloro-8-(4-methyl-1H-pyrazol-3-yl)-1-oxo-2-phenyl-1,2-dihydroisoquinolin-3-yl)ethyl)amino)pyrido[2,3-d]pyrimidin-5(8H)-one |
| 245 | (S)-4-((1-(4-chloro-8-(morpholinomethyl)-1-oxo-2-phenyl-1,2-dihydroisoquinolin-3-yl)ethyl)amino)pyrido[2,3-d]pyrimidin-5(8H)-one |
| 246 | (S)-4-((1-(4-chloro-8-(6-(methylamino)pyridin-3-yl)-1-oxo-2-phenyl-1,2-dihydroisoquinolin-3-yl)ethyl)amino)pyrido[2,3-d]pyrimidin-5(8H)-one |
| 247 | (S)-4-((1-(4-chloro-1-oxo-2-phenyl-8-(4-(trifluoromethoxy)phenyl)-1,2-dihydroisoquinolin-3-yl)ethyl)amino)pyrido[2,3-d]pyrimidin-5(8H)-one |
| 248 | (S)-4-((1-(4-chloro-8-(2,6-dimethylpyridin-3-yl)-1-oxo-2-phenyl-1,2-dihydroisoquinolin-3-yl)ethyl)amino)pyrido[2,3-d]pyrimidin-5(8H)-one |
| 249 | (S)-4-((1-(4-chloro-8-(dimethylamino)-1-oxo-2-phenyl-1, 2-dihydroisoquinolin-3-yl)ethyl)amino)pyrido[2,3-d]pyrimidin-5(8H)-one |
| 250 | (S)-4-((1-(4-chloro-8-(4-methylpiperazin-1-yl)-1-oxo-2-phenyl-1,2-dihydroisoquinolin-3-yl)ethyl)amino)pyrido[2,3-d]pyrimidin-5(8H)-one |
| 251 | (S)-4-((1-(4-chloro-8-morpholino-1-oxo-2-phenyl-1,2-dihydroisoquinolin-3-yl)ethyl)amino)pyrido[2,3-d]pyrimidin-5(8H)-one |
| 252 | (S)-4-((1-(4-chloro-1-oxo-2-phenyl-8-(pyrrolidin-1-yl)-1,2-dihydroisoquinolin-3-yl)ethyl)amino)pyrido[2,3-d]pyrimidin-5(8H)-one |
| 253 | (S)-4-((1-(4-chloro-8-(2,3-dihydrobenzo[b][1,4]dioxin-6-yl)-1-oxo-2-phenyl-1,2-dihydroisoquinolin-3-yl) ethyl) amino)pyrid o[2,3-d]pyrimidin-5(8H)-one |
| 254 | (S)-4-((1-(8-([1,1'-biphenyl]-4-yl)-4-chloro-1-oxo-2-phenyl-1,2-dihydroisoquinolin-3-yl)ethyl)amino)pyrido[2,3-d]pyrimidin-5(8H)-one |
| 255 | (S)-4-((1-(4-chloro-1-oxo-2-phenyl-8-(p-tolyl)-1,2-dihydroisoquinolin-3-yl)ethyl)amino)pyrido[2,3-d]pyrimidin-5(8H)-one |
| 256 | (S)-4-((1-(4-chloro-8-(2-chloro-4-(trifluoromethyl)phenyl)-1-oxo-2-phenyl-1,2-dihydroisoquinolin-3-yl) ethyl) amino)pyrido[2,3-d]pyrimidin-5(8H)-one |
| 257 | (S)-4-((1-(4-chloro-8-(6-methylpyridin-3-yl)-1-oxo-2-phenyl-1,2-dihydroisoquinolin-3-yl)ethyl)amino)pyrido[2,3-d]pyrimidin-5(8H)-one |
| 258 | (S)-4-((1-(4-chloro-1-oxo-2-phenyl-8-(1H-pyrrol-2-yl)-1,2-dihydroisoquinolin-3-yl)ethyl)amino)pyrido[2,3-d]pyrimidin-5(8H)-one |
| 259 | (S)-4-(4-chloro-1-oxo-3-(1-((5-oxo-5,8-dihydropyrido[2,3-d]pyrimidin-4-yl)amino) ethyl)-2-phenyl-1,2-dihydroisoquinolin-8-yl)benzonitrile |
| 260 | (S)-4-((1-(8-(4-morpholinophenyl)-1-oxo-2-phenyl-1,2-dihydroisoquinolin-3-yl)ethyl)amino)pyrido[2,3-d]pyrimidin-5(8H)-one |
| 261 | (S)-4-((1-(1-oxo-2-phenyl-8-(4-(pyrrolidin-1-yl)phenyl)-1,2-dihydroisoquinolin-3-yl)ethyl)amino)pyrido[2,3-d]pyrimidin-5(8H)-one |
| 262 | (S)-4-((1-(8-(4-(hydroxymethyl)phenyl)-1-oxo-2-phenyl-1,2-dihydroisoquinolin-3-yl)ethyl)amino)pyrido[2,3-d]pyrimidin-5(8H)-one |
| 263 | (S)-4-((1-(8-(3-methyl-1H-indol-5-yl)-1-oxo-2-phenyl-1,2-dihydroisoquinolin-3-yl)ethyl)amino)pyrido[2,3-d]pyrimidin-5(8H)-one |
| 264 | (S)-4-((1-(8-(6-(dimethylamino)pyridin-3-yl)-1-oxo-2-phenyl-1,2-dihydroisoquinolin-3-yl)ethyl)amino)pyrido[2,3-d]pyrimidin-5(8H)-one |
| 265 | (S)-4-chloro-1-oxo-3-(1-((5-oxo-5,8-dihydropyrido[2,3-d]pyrimidin-4-yl) amino) ethyl)-2-phenyl-1,2-dihydroisoquinoline-8-carbonitrile |
| 266 | (S)-1-oxo-3-(1-((5-oxo-5,8-dihydropyrido[2,3-d]pyrimidin-4-yl) amino) ethyl)-2-phenyl-1,2-dihydroisoquinoline-8-carbonitrile |
| 267 | (S)-4-((1-(8-acetyl-4-chloro-1-oxo-2-phenyl-1,2-dihydroisoquinolin-3-yl)ethyl)amino)pyrido[2,3-d]pyrimidin-5(8H)-one |
| 268 | (S)-4-((1-(8-butryl-4-chloro-1-oxo-2-phenyl-1,2-dihydroisoquinolin-3-yl)ethyl)amino)pyrido[2,3-d]pyrimidin-5(8H)-one |
| 269 | (S)-4-((1-(4-chloro-8-(cyclopropanecarbonyl)-1-oxo-2-phenyl-1,2-dihydroisoquinolin-3-yl)ethyl)amino)pyrido[2,3-d]pyrimidin-5(8H)-one |
| 270 | (S,Z)-4-((1-(4-chloro-8-(1-(methoxyimino)ethyl)-1-oxo-2-phenyl-1,2-dihydroisoquinolin-3-yl)ethyl)amino)pyrido[2,3-d]pyrimidin-5(8H)-one |
| 271 | (S)-4-chloro-1-oxo-3-(1-((5-oxo-5,8-dihydropyrido[2,3-d]pyrimidin-4-yl) amino) ethyl)-2-phenyl-1,2-dihydroisoquinoline-8-carbaldehyde |
| 272 | 4-(((1S)-1-(4-chloro-8-(1,2-dihydroxy-2-phenylethyl)-1-oxo-2-phenyl-1,2-dihydroisoquinolin-3-yl) ethyl) amino)pyrido[2,3-d]pyrimidin-5(8H)-one |
| 273 | (S,Z)-4-chloro-1-oxo-3-(1-((5-oxo-5,8-dihydropyrido[2,3-d]pyrimidin-4-yl)amino) ethyl)-2-phenyl-1,2-dihydroisoquinoline-8-carbaldehyde oxime |
| 274 | (S)-4-chloro-N-methyl-1-oxo-3-(1-((5-oxo-5,8-dihydropyrido[2,3-d]pyrimidin-4-yl)amino)ethyl)-2-phenyl-1,2-dihydroisoquinoline-8-carboxamide |
| 275 | (S)-4-chloro-1-oxo-3-(1-((5-oxo-5,8-dihydropyrido[2,3-d]pyrimidin-4-yl) amino) ethyl)-2-phenyl-N-propyl-1,2-dihydroisoquin oline-8-carboxamide |
| 276 | (S)-4-chloro-N-(oxetan-3-yl)-1-oxo-3-(1-((5-oxo-5,8-dihydropyrido[2,3-d]pyrimidin-4-yl)amino)ethyl)-2-phenyl-1,2-dihydroisoquinoline-8-carboxamide |
| 277 | (S)-4-chloro-N-cyclobutyl-1-oxo-3-(1-((5-oxo-5,8-dihydropyrido[2,3-d]pyrimidin-4-yl)amino)ethyl)-2-phenyl-1,2-dihydroisoquinoline-8-carboxamide |
| 278 | (S)-4-chloro-1-oxo-3-(1-((5-oxo-5,8-dihydropyrido[2,3-d]pyrimidin-4-yl)amino)ethyl)-2-phenyl-N-(thiophen-2-ylmethyl)-1,2-dihydroisoquinoline-8-carboxamide |
| 279 | (S)-4-chloro-N-(furan-2-ylmethyl)-1-oxo-3-(1-((5-oxo-5,8-dihydropyrido[2,3-d]pyrimidin-4-yl)amino)ethyl)-2-phenyl-1,2-dihydroisoquinoline-8-carboxamide |
| 280 | (S)-N-benzyl-4-chloro-1-oxo-3-(1-((5-oxo-5,8-dihydropyrido[2,3-d]pyrimidin-4-yl)amino)ethyl)-2-phenyl-1,2-dihydroisoquinoline-8-carboxamide |
| 281 | (S)-4-chloro-N-(2-morpholinoethyl)-1-oxo-3-(1-((5-oxo-5,8-dihydropyrido[2,3-d]pyrimidin-4-yl)amino)ethyl)-2-phenyl-1,2-dihydroisoquinoline-8-carboxamide |
| 282 | (S)-4-chloro-N-isopropyl-1-oxo-3-(1-((5-oxo-5,8-dihydropyrido[2,3-d]pyrimidin-4-yl)amino)ethyl)-2-phenyl-1,2-dihydroisoquinoline-8-carboxamide |
| 283 | (S)-4-chloro-N-(cyclopropylmethyl)-1-oxo-3-(1-((5-oxo-5,8-dihydropyrido[2,3-d]pyrimidin-4-yl)amino)ethyl)-2-phenyl-1,2-dihydroisoquinoline-8-carboxamide |
| 284 | (S)-4-chloro-N-(1-hydroxy-2-methylpropan-2-yl)-1-oxo-3-(1-((5-oxo-5,8-dihydropyrido[2,3-d]pyrimidin-4-yl)amino)ethyl)-2-phenyl-1,2-dihydroisoquinoline-8-carboxamide |
| 285 | (S)-N-(tert-butyl)-4-chloro-1-oxo-3-(1-((5-oxo-5,8-dihydropyrido[2,3-d]pyrimidin-4-yl)amino)ethyl)-2-phenyl-1,2-dihydroisoquinoline-8-carboxamide |
| 286 | (S)-4-((1-(4-chloro-8-(morpholine-4-carbonyl)-1-oxo-2-phenyl-1,2-dihydroisoquinolin-3-yl)ethyl)amino)pyrido[2,3-d]pyrimidin-5(8H)-one |
| 287 | (S)-4-((1-(4-chloro-8-(1H-imidazol-2-yl)-1-oxo-2-phenyl-1,2-dihydroisoquinolin-3-yl)ethyl)amino)pyrido[2,3-d]pyrimidin-5(8H)-one |
| 288 | (S)-4-((1-(4-chloro-8-(4,5-dimethyl-1H-imidazol-2-yl)-1-oxo-2-phenyl-1,2-dihydroisoquinolin-3-yl)ethyl)amino)pyrido[2,3-d]pyrimidin-5(8H)-one |
| 289 | (S)-4-((1-(4-chloro-8-(4,4-dimethyl-4,5-dihydrooxazol-2-yl)-1-oxo-2-phenyl-1,2-dihydroisoquinolin-3-yl) ethyl) amino)pyrid o[2,3-d]pyrimidin-5(8H)-one |
| 290 | (S)-4-chloro-1-oxo-3-(1-((5-oxo-5,8-dihydropyrido[2,3-d]pyrimidin-4-yl)amino)ethyl)-2-phenyl-1,2-dihydroisoquinoline-8-carboxylic acid |
| 291 | (S)-4-chloro-1-oxo-3-(1-((5-oxo-5,8-dihydropyrido[2,3-d]pyrimidin-4-yl) amino)ethyl)-2-phenyl-1,2-dihydroisoquinoline-8-carboxamide |
| 292 | Ethyl (S)-4-chloro-1-oxo-3-(1-((5-oxo-5,8-dihydropyrido[2,3-d]pyrimidin-4-yl)amino)ethyl)-2-phenyl-1,2-dihydroisoquinoline-8-carboxylate |
| 293 | (S)-4-((1-(4-chloro-8-(4,6-diamino-1,3,5-triazin-2-yl)-1-oxo-2-phenyl-1,2-dihydroisoquinolin-3-yl)ethyl)amino)pyrido[2,3-d]pyrimidin-5(8H)-one |
| 294 | (S)-4-((1-(8-amino-4-chloro-1-oxo-2-phenyl-1,2-dihydroisoquinolin-3-yl)ethyl)amino)pyrido[2,3-d]pyrimidin-5(8H)-one |
| 295 | (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)pivalamide |
| 296 | (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)acetamide |
| 297 | (S)-N-(4-chloro-l-oxo-3-(l-((5-oxo-5,8-dihydropyrido[2,3-d]pyrimidin-4-yl)amino)ethyl)-2-phenyl-1,2-dihydroisoquinolin-8-yl)pentanamide |
| 298 | (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)cyclobutanecarboxamide |
| 299 | (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)benzamide |
| 300 | (S)-N-(4-chloro-1-oxo-3-(1-((5-oxo-5,8-dihydropyrido[2,3-d]pyrimidin-4-yl)amino) ethyl)-2-phenyl-1,2-dihydroisoquinolin-8-yl)methanesulfonamide |
| 301 | (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)cyclopropanesulfonamide |
| 302 | (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-2,2,2-trifluoroethane-1-sulfonamide |
| 303 | (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-4-fluorobenzenesulfonamide |
| 304 | (S)-4-((1-(4-chloro-1-oxo-2-phenyl-8-(thiophen-2-ylethynyl)-1,2-dihydroisoquinolin-3-yl)ethyl)amino)pyrido[2,3-d]pyrimidin-5(8H)-one |
| 305 | (S)-4-((1-(4-chloro-1-oxo-2-phenyl-8-(phenylethynyl)-1,2-dihydroisoquinolin-3-yl)ethyl)amino)pyrido[2,3-d]pyrimidin-5(8H)-one |
| 306 | (S)-4-((1-(4-chloro-1-oxo-2-phenyl-8-(pyridin-4-ylethynyl)-1,2-dihydroisoquinolin-3-yl)ethyl)amino)pyrido[2,3-d]pyrimidin-5(8H)-one |
| 307 | (S)-4-((1-(4-chloro-1-oxo-2-phenyl-8-(pyridin-3-ylethynyl)-1,2-dihydroisoquinolin-3-yl)ethyl)amino)pyrido[2,3-d]pyrimidin-5(8H)-one |
| 308 | (S)-4-((1-(4-chloro-1-oxo-2-phenyl-8-(pyridin-2-ylethynyl)-1,2-dihydroisoquinolin-3-yl)ethyl)amino)pyrido[2,3-d]pyrimidin-5(8H)-one |
| 309 | (S)-4-((1-(4-chloro-8-((1-methyl-1H-pyrazol-4-yl)ethynyl)-1-oxo-2-phenyl-1,2-dihydroisoquinolin-3-yl)ethyl)amino)pyrido[2,3-d]pyrimidin-5(8H)-one |
| 310 | (S)-4-((1-(8-([1,1'-biphenyl]-4-ylethynyl)-4-chloro-1-oxo-2-phenyl-1,2-dihydroisoquinolin-3-yl)ethyl)amino)pyrido[2,3-d]pyrimidin-5(8H)-one |
| 31 | (S)-4-((1-(4-chloro-8-((1-methyl-1H-pyrazol-5-yl)ethynyl)-1-oxo-2-phenyl-1,2-dihydroisoquinolin-3-yl)ethyl)amino)pyrido[2,3-d]pyrimidin-5(8H)-one |
| 312 | (S)-4-((1-(4-chloro-1-oxo-2-phenyl-8-(thiazol-2-ylethynyl)-1,2-dihydroisoquinolin-3-yl)ethyl)amino)pyrido[2,3-d]pyrimidin-5(8H)-one |
| 313 | (S)-4-((1-(4-chloro-1-oxo-2-phenyl-8-(thiazol-5-ylethynyl)-1,2-dihydroisoquinolin-3-yl)ethyl)amino)pyrido[2,3-d]pyrimidin-5(8H)-one |
| 314 | (S)-4-((1-(4-chloro-8-((4-(dimethylamino)phenyl)ethynyl)-1-oxo-2-phenyl-1,2-dihydroisoquinolin-3-yl)ethyl)amino)-5-oxo-5,8-dihydropyrido[2,3-d]pyrimidin-5(8H)-one |
| 315 | (S)-4-((1-(4-chloro-1-oxo-2-phenyl-8-((4-(trifluoromethyl)phenyl)ethynyl)-1,2-dihydroisoquinolin-3-yl)ethyl)amino)-5-oxo-5,8-dihydropyrido[2,3-d]pyrimidin-8-ium 2,2,2-trifluoroacetate |
| 316 | (S)-4-((1-(4-chloro-1-oxo-2-phenyl-8-(pyrimidin-5-ylethynyl)-1,2-dihydroisoquinolin-3-yl)ethyl)amino)pyrido[2,3-d]pyrimidin-5(8H)-one |
| 317 | (S)-4-((1-(8-(benzo[d]oxazol-2-yl)-4-chloro-1-oxo-2-phenyl-1,2-dihydroisoquinolin-3-yl)ethyl)amino)pyrido[2,3-d]pyrimidin-5(8H)-one |
| 318 | (S)-4-((1-(8-(benzo[d]oxazol-2-yl)-1-oxo-2-phenyl-1,2-dihydroisoquinolin-3-yl)ethyl)amino)pyrido[2,3-d]pyrimidin-5(8H)-one |
| 319 | (S)-4-((1-(S-(benzyl(methyl)amino)-4-chloro-1-oxo-2-phenyl-1,2-dihydroisoquinolin-3-yl)ethyl)amino)pyrido[2,3-d]pyrimidin-5(8H)-one |
| 320 | (S)-4-((1-(4-chloro-1-oxo-2-phenyl-8-(1H-pyrazol-1-yl)-1,2-dihydroisoquinolin-3-yl)ethyl)amino)pyrido[2,3-d]pyrimidin-5(8H)-one |
| 321 | (S)-4-((1-(8-(benzylamino)-4-chloro-1-oxo-2-phenyl-1,2-dihydroisoquinolin-3-yl)ethyl)amino)pyrido[2,3-d]pyrimidin-5(8H)-one |
| 322 | (S)-4-((1-(4-chloro-8-(cyclohexyl(methyl)amino)-1-oxo-2-phenyl-1,2-dihydroisoquinolin-3-yl)ethyl)amino)pyrido[2,3-d]pyrimidin-5(8H)-one |
| 323 | (S)-4-((1-(4-chloro-8-(cyclopropylamino)-1-oxo-2-phenyl-1,2-dihydroisoquinolin-3-yl)ethyl)amino)pyrido[2,3-d]pyrimidin-5(8H)-one |
| 324 | (S)-4-((1-(4-chloro-1-oxo-2-phenyl-8-((pyridin-3-ylmethyl)amino)-1,2-dihydroisoquinolin-3-yl)ethyl)amino)pyrido[2,3-d]pyrimidin-5(8H)-one |
| 325 | (S)-4-((1-(4-chloro-8-(ethylamino)-1-oxo-2-phenyl-1,2-dihydroisoquinolin-3-yl)ethyl)amino)pyrido[2,3-d]pyrimidin-5(8H)-one |
| 326 | (S)-4-((1-(4-chloro-1-oxo-2-phenyl-8-(4-phenylpiperazin-1-yl)-1,2-dihydroisoquinolin-3-yl)ethyl)amino)pyrido[2,3-d]pyrimidin-5(8H)-one |
| 327 | 4-(((S)-1-(4-chloro-1-oxo-2-phenyl-8-((1S,5R)-1,3,3-trimethyl-6-azabicyclo[3.2.1]octan-6-yl)-1,2-dihydroisoquinolin-3-yl)ethyl)amino)pyrido[2,3-d]pyrimidin-5(8H)-one |
| 328 | 4'-chloro-3'-((S)-1-((5-oxo-5,8-dihydropyrido[2,3-d]pyrimidin-4-yl)amino)ethyl)-2'-phenyl-3,4,4a,5,6,7,8,8a-octahydro-1H-[2,8' -biisoquinolin] -1' (2'H) -one |
| 329 | (S)-4'-chloro-7-methyl-3'-(1-((5-oxo-5,8-dihydropyrido[2,3-d]pyrimidin-4-yl)amino)ethyl)-2'-phenyl-3,4-dihydro-1H-[2,8'-biisoquinolin] -1' (2'H) -one |
| 330 | (S)-4-((1-(4-chloro-8-(4,7-dihydrothieno[2,3-c]pyridin-6(5H)-yl)-1-oxo-2-phenyl-1,2-dihydroisoquinolin-3-yl) ethyl) amino)p yrido[2,3-d]pyrimidin-5(8H)-one |
| 331 | 4-(((1S)-1-(4-chloro-1-oxo-2-phenyl-8-((tetrahydrofuran-3-yl)amino)-1,2-dihydroisoquinolin-3-yl)ethyl)amino)pyrido[2,3-d]pyrimidin-5(8H)-one |
| 332 | (S)-4-((1-(4-chloro-1-oxo-2-phenyl-8-((tetrahydro-2H-pyran-4-yl)amino)-1,2-dihydroisoquinolin-3-yl)ethyl)amino)pyrido[2,3-d]pyrimidin-5(8H)-one |
| 333 | (S)-4-((1-(4-chloro-1-oxo-2-phenyl-8-(4-(pyridin-2-yl)piperazin-1-yl)-1,2-dihydroisoquinolin-3-yl)ethyl)amino)pyrido[2,3-d]pyrimidin-5(8H)-one |
| 334 | (S)-4-((1-(4-chloro-8-(methyl(naphthalen-2-ylmethyl)amino)-1-oxo-2-phenyl-1,2-dihydroisoquinolin-3-yl) ethyl)amino)pyrido[2,3-d]pyrimidin-5(8H)-one |
| 335 | (S)-4-((1-(4-chloro-8-((4-chlorophenethyl)amino)-1-oxo-2-phenyl-1,2-dihydroisoquinolin-3-yl)ethyl)amino)pyrido[2,3-d]pyrimidin-5(8H)-one |
| 336 | (S)-4-((1-(4-chloro-1-oxo-2-phenyl-8-(4-phenylpiperidin-1-yl)-1,2-dihydroisoquinolin-3-yl)ethyl)amino)pyrido[2,3-d]pyrimidin-5(8H)-one |
| 337 | (S)-1-(4-chloro-1-oxo-3-((S)-1-((5-oxo-5,8-dihydropyrido[2,3-d]pyrimidin-4-yl)amino)ethyl)-2-phenyl-1,2-dihydroisoquinolin-8-yl)pyrrolidine-2-carboxamide |
| 338 | 4-(((S)-1-(4-chloro-1-oxo-2-phenyl-8-((S)-2-(pyrrolidin-1-ylmethyl)pyrrolidin-1-yl)-1,2-dihydroisoquinolin-3-yl)ethyl)amino)pyrido[2,3-d]pyrimidin-5(8H)-one |
| 339 | 4-(((1s)-1-(4-chloro-8-(2-hydroxypyrrolidin-1-yl)-1-oxo-2-phenyl-1,2-dihydroisoquinolin-3-yl)ethyl)amino)pyrido[2,3-d]pyrimidin-5(8H)-one |
| 340 | (S)-4-((1-(4-chloro-8-(isoindolin-2-yl)-1-oxo-2-phenyl-1,2-dihydroisoquinolin-3-yl)ethyl)amino)pyrido[2,3-d]pyrimidin-5(8H)-one |
| 341 | (S)-4-((1-(4-chloro-8-(indolin-1-yl)-1-oxo-2-phenyl-1,2-dihydroisoquinolin-3-yl)ethyl)amino)pyrido[2,3-d]pyrimidin-5(8H)-one |
| 342 | 4-(((1S)-1-(4-chloro-8-(hexahydrocyclopenta[c]pyrrol-2(1H)-yl)-1-oxo-2-phenyl-1,2-dihydroisoquinolin-3-yl) ethyl) amino)p yrido[2,3-d]pyrimidin-5(8H)-one |
| 343 | (S)-4-((1-(4-chloro-1-oxo-2-phenyl-8-(phenylamino)-1,2-dihydroisoquinolin-3-yl)ethyl)amino)pyrido[2,3-d]pyrimidin-5(8H)-one |
| 344 | (S)-4-((1-(4-chloro-8-((4-fluorophenyl)amino)-1-oxo-2-phenyl-1,2-dihydroisoquinolin-3-yl)ethyl)amino)pyrido[2,3-d]pyrimidin-5(8H)-one |
| 345 | (S)-4-((1-(4-chloro-8-((4-methoxyphenyl)amino)-1-oxo-2-phenyl-1,2-dihydroisoquinolin-3-yl)ethyl)amino)pyrido[2,3-d]pyrimidin-5(8H)-one |
| 346 | (S)-4-((1-(4-chloro-1-oxo-2-phenyl-8-((4-(trifluoromethyl)phenyl)amino)-1,2-dihydroisoquinolin-3-yl)ethyl)amino)pyrido[2,3-d]pyrimidin-5(8H)-one |
| 347 | (S)-4-((1-(4-chloro-1-oxo-2-phenyl-8-(pyridin-3-ylamino)-1,2-dihydroisoquinolin-3-yl)ethyl)amino)pyrido[2,3-d]pyrimidin-5(8H)-one |
| 348 | (S)-4-((1-(4-chloro-1-oxo-2-phenyl-8-(pyridin-2-ylamino)-1,2-dihydroisoquinolin-3-yl)ethyl)amino)pyrido[2,3-d]pyrimidin-5(8H)-one |
| 349 | (S)-4-((1-(4-chloro-8-(methyl(pyridin-2-yl)amino)-1-oxo-2-phenyl-1,2-dihydroisoquinolin-3-yl)ethyl)amino)pyrido[2,3-d]pyrimidin-5(8H)-one |
| 350 | (S,Z)-4-chloro-N'-hydroxy-1-oxo-3-(1-((5-oxo-5,8-dihydropyrido[2,3-d]pyrimidin-4-yl)amino)ethyl)-2-phenyl-1,2-dihydroisoquinoline-8-carboximidamide |
| 351 | (S)-4-((1-(4-chloro-8-(1,2,4-oxadiazol-3-yl)-1-oxo-2-phenyl-1,2-dihydroisoquinolin-3-yl)ethyl)amino)pyrido[2,3-d]pyrimidin-5(8H)-one |
| 352 | (S)-4-((1-(4-chloro-1-oxo-2-phenyl-8-(1H-tetrazol-5-yl)-1,2-dihydroisoquinolin-3-yl)ethyl)amino)pyrido[2,3-d]pyrimidin-5(8H)-one |
| 353 | (S)-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)boronic acid |
| 354 | (S)-4-((1-(4-chloro-8-ethyl-1-oxo-2-phenyl-1,2-dihydroisoquinolin-3-yl)ethyl)amino)pyrido[2,3-d]pyrimidin-5(8H)-one |
| 355 | (S)-4-((1-(4-chloro-8-(hydroxymethyl)-1-oxo-2-phenyl-1,2-dihydroisoquinolin-3-yl)ethyl)amino)pyrido[2,3-d]pyrimidin-5(8H)-one |
| 356 | 4-(((1s)-1-(4-chloro-1-oxo-2-phenyl-8-(thiazolidin-2-yl)-1,2-dihydroisoquinolin-3-yl)ethyl)amino)pyrido[2,3-d]pyrimidin-5(8H)-one |
| 357 | 4-(((1S)-1-(4-chloro-8-(1-hydroxy-2,2-dimethylpropyl)-1-oxo-2-phenyl-1,2-dihydroisoquinolin-3-yl) ethyl) amino)pyrido[2,3-d]pyrimidin-5(8H)-one |
| 358 | (S)-4-((1-(4-chloro-8-(5,5-dimethyl-1,3-dioxan-2-yl)-1-oxo-2-phenyl-1,2-dihydroisoquinolin-3-yl)ethyl)amino)pyrido[2,3-d]pyrimidin-5(8H)-one |
| 359 | (S)-4-((1-(4-chloro-8-((3-hydroxy-2,2-dimethylpropoxy)methyl)-1-oxo-2-phenyl-1,2-dihydroisoquinolin-3-yl) ethyl) amino)p yrido[2,3-d]pyrimidin-5(8H)-one |
| 360 | (S)-4-((1-(4-chloro-8-(2-hydroxypropan-2-yl)-1-oxo-2-phenyl-1,2-dihydroisoquinolin-3-yl)ethyl)amino)pyrido[2,3-d]pyrimidin-5(8H)-one |
| 361 | 4-(((S)-1-(4-chloro-8-((R)-1-hydroxyethyl)-1-oxo-2-phenyl-1,2-dihydroisoquinolin-3-yl)ethyl)amino)pyrido[2,3-d]pyrimidin-5(8H)-one (having a diastereomeric relationship with compound no. 441) |
| 362 | (S)-4-((1-(8-(benzyloxy)-4-chloro-1-oxo-2-phenyl-1,2-dihydroisoquinolin-3-yl)ethyl)amino)pyrido[2,3-d]pyrimidin-5(8H)-one |
| 363 | (S)-4-((1-(4-chloro-8-(2-methoxyethoxy)-1-oxo-2-phenyl-1,2-dihydroisoquinolin-3-yl)ethyl)amino)pyrido[2,3-d]pyrimidin-5(8H)-one |
| 364 | (S)-4-((1-(4-chloro-1-oxo-2-phenyl-8-(2,2,2-trifluoroethoxy)-1,2-dihydroisoquinolin-3-yl)ethyl)amino)pyrido[2,3-d]pyrimidin-5(8H)-one |
| 365 | (S)-4-((1-(4-chloro-8-methoxy-1-oxo-2-phenyl-1,2-dihydroisoquinolin-3-yl)ethyl)amino)pyrido[2,3-d]pyrimidin-5(8H)-one |
| 366 | (S)-4-((1-(4-chloro-8-(2-methoxyphenoxy)-1-oxo-2-phenyl-1,2-dihydroisoquinolin-3-yl)ethyl)amino)pyrido[2,3-d]pyrimidin-5(8H)-one |
| 367 | (S)-4-((1-(4-chloro-1-oxo-2-phenyl-8-(p-tolyloxy)-1,2-dihydroisoquinolin-3-yl)ethyl)amino)pyrido[2,3-d]pyrimidin-5(8H)-one |
| 368 | 4-(((1s)-1-(4-chloro-1-oxo-2-phenyl-8-(1-(pyridin-4-yl)ethoxy)-1,2-dihydroisoquinolin-3-yl)ethyl)amino)pyrido[2,3-d]pyrimidin-5(8H)-one |
| 369 | (S)-4-((1-(4-chloro-8-((4-chlorobenzyl)thio)-1-oxo-2-phenyl-1,2-dihydroisoquinolin-3-yl)ethyl)amino)pyrido[2,3-d]pyrimidin-5(8H)-one |
| 370 | (S)-4-((1-(4-chloro-1-oxo-2-phenyl-8-(pyridin-4-ylthio)-1,2-dihydroisoquinolin-3-yl)ethyl)amino)pyrido[2,3-d]pyrimidin-5(8H)-one |
| 371 | (S)-4-((1-(4-chloro-8-((4-chlorophenyl)thio)-1-oxo-2-phenyl-1,2-dihydroisoquinolin-3-yl)ethyl)amino)pyrido[2,3-d]pyrimidin-5(8H)-one |
| 372 | (S)-4-((1-(4-chloro-1-oxo-2-phenyl-8-(propylthio)-1,2-dihydroisoquinolin-3-yl)ethyl)amino)pyrido[2,3-d]pyrimidin-5(8H)-one |
| 373 | (S)-6-(4-chloro-1-oxo-3-(1-((5-oxo-5,8-dihydropyrido[2,3-d]pyrimidin-4-yl)amino) ethyl)-2-phenyl-1,2-dihydroisoquinolin-8-yl)-2,2-dimethyl-2H-benzo[b] [1,4]oxazin-3(5H)-one |
| 374 | (S)-4-((1-(4-chloro-1-oxo-2-phenyl-1,2-dihydroisoquinolin-3-yl)ethyl)amino)-8-(2-methoxypyridin-4-yl)pyrido[2,3-d]pyrimidin-5(8H)-one |
| 375 | (S)-8-(5-bromo-2-methoxypyridin-4-yl)-4-((1-(4-chloro-1-oxo-2-phenyl-1,2-dihydroisoquinolin-3-yl)ethyl)amino)pyrido[2,3-d]pyrimidin-5(8H)-one |
| 376 | (S)-4-((1-(4-chloro-8-(5-isopropoxypyrazin-2-yl)-1-oxo-2-phenyl-1,2-dihydroisoquinolin-3-yl)ethyl)amino)pyrido[2,3-d]pyrimidin-5(8H)-one |
| 377 | (S)-4-((1-(4-chloro-1-oxo-2-phenyl-8-(2-(thiophen-2-yl)pyrimidin-5-yl)-1,2-dihydroisoquinolin-3-yl)ethyl)amino)pyrido[2,3-d]pyrimidin-5(8H)-one |
| 378 | (S)-4-((1-(4-chloro-8-(2-(furan-2-yl)pyrimidin-5-yl)-1-oxo-2-phenyl-1,2-dihydroisoquinolin-3-yl)ethyl)amino)pyrido[2,3-d]pyrimidin-5(8H)-one |
| 379 | (S)-4-((1-(4-chloro-8-(2-ethylpyrimidin-5-yl)-1-oxo-2-phenyl-1,2-dihydroisoquinolin-3-yl)ethyl)amino)pyrido[2,3-d]pyrimidin-5(8H)-one |
| 380 | (S)-4-((1-(4-chloro-8-(1H-indol-2-yl)-1-oxo-2-phenyl-1,2-dihydroisoquinolin-3-yl)ethyl)amino)pyrido[2,3-d]pyrimidin-5(8H)-one |
| 381 | (S)-4-((1-(4-chloro-8-(2-isopropylpyrimidin-5-yl)-1-oxo-2-phenyl-1,2-dihydroisoquinolin-3-yl)ethyl)amino)pyrido[2,3-d]pyrimidin-5(8H)-one |
| 382 | (S)-4-((1-(4-chloro-8-(2-fluoropyrimidin-5-yl)-1-oxo-2-phenyl-1,2-dihydroisoquinolin-3-yl)ethyl)amino)pyrido[2,3-d]pyrimidin-5(8H)-one |
| 383 | (3)-4-((1-(8-(2-methoxypyrimidin-5-yl)-1-oxo-2-phenyl-1,2-dihydroisoquinolin-3-yl)ethyl)amino)pyrido[2,3-d]pyrimidin-5(8H)-one |
| 384 | (S)-4-((1-(8-(2-ethoxypyrimidin-5-yl)-1-oxo-2-phenyl-1,2-dihydroisoquinolin-3-yl)ethyl)amino)pyrido[2,3-d]pyrimidin-5(8H)-one |
| 385 | (S)-4-((1-(1-oxo-2-phenyl-8-(2-(trifluoromethyl)pyridin-5-yl)-1,2-dihydroisoquinolin-3-yl)ethyl)amino)pyrido[2,3-d]pyrimidin-5(8H)-one |
| 386 | (S)-4-(-(1-(8-(2-(2-hydroxypropan-2-yl)pyrimidin-5-yl)-1-oxo-2 phenyl-1,2-dihydroisoquinolin-3-yl)ethyl)amino)pyrido[2,3-d]pyrimidin-5(8H)-one |
| 387 | (S)-4-((1-(4-chloro-8-(5-methylthiophen-2-yl)-1-oxo-2-phenyl-1,2-dihydroisoquinolin-3-yl)ethyl)amino)pyrido[2,3-d]pyrimidin-5(8H)-one |
| 388 | (S)-4-((1-(4-chloro-1-oxo-2-phenyl-8-(thiophen-2-yl)-1,2-dihydroisoquinolin-3-yl)ethyl)amino)pyrido[2,3-d]pyrimidin-5(8H)-one |
| 389 | (S)-4-((1-(4-chloro-1-oxo-2-phenyl-8-(trifluoromethyl)-1,2-dihydroisoquinolin-3-yl)ethyl)amino)pyrido[2,3-d]pyrimidin-5(8H)-one |
| 390 | (S)-4-((1-(4-chloro-8-(methylsulfonyl)-1-oxo-2-phenyl-1,2-dihydroisoquinolin-3-yl)ethyl)amino)pyrido[2,3-d]pyrimidin-5(8H)-one |
| 391 | (S)-4-((1-(4-chloro-1-oxo-2-phenyl-8-(pyridin-4-ylsulfonyl)-1,2-dihydroisoquinolin-3-yl)ethyl)amino)pyrido[2,3-d]pyrimidin-5(8H)-one |
| 392 | (S)-4-((1-(4-chloro-8-(2-(methylsulfonyl)pyrimidin-5-yl)-1-oxo-2-phenyl-1,2-dihydroisoquinolin-3-yl) ethyl) amino)pyrido[2,3-d]pyrimidin-5(8H)-one |
| 393 | (S)-4-((1-(4-chloro-8-((4-chlorophenyl)sulfonyl)-1-oxo-2-phenyl-1,2-dihydroisoquinolin-3-yl)ethyl)amino)pyrido[2,3-d]pyrimidin-5(8H)-one |
| 394 | 4-(((S)-1-(4-chloro-1-oxo-2-phenyl-8-(((S)-4-(trifluoromethoxy)phenyl)sulfinyl)-1,2-dihydroisoquinolin-3-yl)ethyl)amino)pyrido[2,3-d]pyrimidin-5(8H)-one (having a diastereomeric relationship with compound no. 395) |
| 395 | 4-(((S)-1- (4-chloro-1-oxo-2-phenyl-8-(((R)-4-(trifluoromethoxy)phenyl)sulfinyl)-1,2-dihydroisoquinolin-3-yl)ethyl)amino)pyrido[2,3-d]pyrimidin-5(8H)-one (having a diastereomeric relationship with compound no. 394) |
| 396 | 4-(((S)-1-(4-chloro-1-oxo-2-phenyl-8-((S)-propylsulfinyl)-1,2-dihydroisoquinolin-3-yl)ethyl)amino)pyrido[2,3-d]pyrimidin-5(8H)-one (having a diastereomeric relationship with compound no. 397) |
| 397 | 4-(((S)-1-(4-chloro-1-oxo-2-phenyl-8-((R)-propylsulfonyl)-1,2-dihydroisoquinolin-3-yl)ethyl)amino)pyrido[2,3-d]pyrimidin-5(8H)-one (having a diastereomeric relationship with compound no. 396) |
| 398 | (S)-4-((1-(4-chloro-8-((4-fluorophenyl)thio)-1-oxo-2-phenyl-1,2-dihydroisoquinolin-3-yl)ethyl)amino)pyrido[2,3-d]pyrimidin-5(8H)-one |
| 399 | (S)-4-((1-(4-chloro-8-((4-fluorophenyl)sulfonyl)-1-oxo-2-phenyl-1,2-dihydroisoquinolin-3-yl)ethyl)amino)pyrido[2,3-d]pyrimidin-5(8H)-one |
| 400 | (S)-4-((1-(8-((1H-1,2,4-triazol-3-yl)thio)-4-chloro-1-oxo-2-phenyl-1,2-dihydroisoquinolin-3-yl)ethyl)amino)pyrido[2,3-d]pyrimidin-5(8H)-one |
| 401 | (S)-4-((1-(8-((1H-1,2,4-triazol-3-yl)sulfonyl)-4-chloro-1-oxo-2-phenyl-1,2-dihydroisoquinolin-3-yl) ethyl) amino)pyrido[2,3-d]pyrimidin-5(8H)-one |
| 402 | (S)-4-((1-(4-chloro-8-(2-chloropyrimidin-5-yl)-1-oxo-2-phenyl-1,2-dihydroisoquinolin-3-yl)ethyl)amino)pyrido[2,3-d]pyrimidin-5(8H)-one |
| 403 | (S)-4-((1-(8-(2-acetylpyrimidin-5-yl)-4-chloro-1-oxo-2-phenyl-1,2-dihydroisoquinolin-3-yl)ethyl)amino)pyrido[2,3-d]pyrimidin-5(8H)-one |
| 404 | (S)-4-((1-(4-chloro-8-(2-(2-ethoxypropan-2-yl)pyrimidin-5-yl)-1-oxo-2-phenyl-1,2-dihydroisoquinolin-3-yl) ethyl) amino)p yrido[2,3-d]pyrimidin-5(8H)-one |
| 405 | (S)-4-((1-(4-chloro-8-(2-(2-methoxypropan-2-yl)pyrimidin-5-yl)-1-oxo-2-phenyl-1,2-dihydroisoquinolin-3-yl) ethyl) amino)p yrido[2,3-d]pyrimidin-5(8H)-one |
| 406 | (S)-4-((1-(4-chloro-1-oxo-2-phenyl-8-(2-(2-propoxypropan-2-yl)pyrimidin-5-yl)-1,2-dihydroisoquinolin-3-yl)ethyl)amino)pyrido[2,3-d]pyrimidin-5(8H)-one |
| 407 | 4-(((1S)-1-(4-chloro-8-(2-(S-methylsulfonimidoyl)pyrimidin-5-yl)-1-oxo-2-phenyl-1,2-dihydroisoquinolin-3-yl) ethyl) amino)p yrido[2,3-d]pyrimidin-5(8H)-one |
| 408 | 4-(((S)-1-(4-chloro-1-oxo-2-phenyl-8-((R)-propylsulfonimidoyl)-1,2-dihydroisoquinolin-3-yl)ethyl)amino)pyrido[2,3-d]pyrimidin-5(8H)-one (having a diastereomeric relationship with compound no. 409) |
| 409 | 4-(((S)-1-(4-chloro-1-oxo-2-phenyl-8-((S)-propylsulfonimidoyl)-1,2-dihydroisoquinolin-3-yl)ethyl)amino)pyrido[2,3-d]pyrimidin-5(8H)-one (having a diastereomeric relationship with compound no. 408) |
| 410 | 4-(((S)-1-(4-chloro-1-oxo-2-phenyl-8-((R)-propan-2-ylsulfonimidoyl)-1,2-dihydroisoquinolin-3-yl)ethyl)amino)pyrido[2,3-d]pyrimidin-5(8H)-one (having a diastereomeric relationship with compound no. 411) |
| 411 | 4-(((S)-1-(4-chloro-1-oxo-2-phenyl-8-((S)-propan-2-ylsulfonimidoyl)-1,2-dihydroisoquinolin-3-yl)ethyl)amino)pyrido[2,3-d]pyrimidin-5(8H)-one (having a diastereomeric relationship with compound no. 410) |
| 412 | (S)-4-((1-(4-chloro-1-oxo-2-phenyl-8-(pyridin-2-ylthio)-1,2-dihydroisoquinolin-3-yl)ethyl)amino)pyrido[2,3-d]pyrimidin-5(8H)-one |
| 413 | 4-(((1S)-1-(4-chloro-1-oxo-2-phenyl-8-(pyridin-2-ylsulfinyl)-1,2-dihydroisoquinolin-3-yl)ethyl)amino)pyrido[2,3-d]pyrimidin-5(8H)-one |
| 414 | (S)-4-((1-(4-chloro-1-oxo-2-phenyl-8-(pyridin-2-ylsulfonyl)-1,2-dihydroisoquinolin-3-yl)ethyl)amino)pyrido[2,3-d]pyrimidin-5(8H)-one |
| 415 | (S)-4-((1-(4-chloro-8-(isopropylthio)-1-oxo-2-phenyl-1,2-dihydroisoquinolin-3-yl)ethyl)amino)pyrido[2,3-d]pyrimidin-5(8H)-one |
| 416 | (S)-4-((1-(4-chloro-8-(isopropylsulfonyl)-1-oxo-2-phenyl-1,2-dihydroisoquinolin-3-yl)ethyl)amino)pyrido[2,3-d]pyrimidin-5(8H)-one |
| 417 | (S)-4-((1-(4-chloro-1-oxo-2-phenyl-8-(thiazol-2-ylthio)-1,2-dihydroisoquinolin-3-yl)ethyl)amino)pyrido[2,3-d]pyrimidin-5(8H)-one |
| 418 | (S)-4-((1-(4-chloro-8-(oxatan-3-ylthio)-1-oxo-2-phenyl-1,2-dihydroisoquinolin-3-yl)ethyl)amino)pyrido[2,3-d]pyrimidin-5(8H)-one |
| 419 | 4-(((1S)-1-(4-chloro-1-oxo-2-phenyl-8-(pyrimidin-2-ylsulfinyl)-1,2-dihydroisoquinolin-3-yl)ethyl)amino)pyrido[2,3-d]pyrimidin-5(8H)-one |
| 420 | (S)-4-((1-(4-chloro-1-oxo-2-phenyl-8-(pyrimidin-2-ylsulfonyl)-1,2-dihydroisoquinolin-3-yl)ethyl)amino)pyrido[2,3-d]pyrimidin-5(8H)-one |
| 421 | 4-(((S)-1-(4-chloro-1-oxo-2-phenyl-8-((S)-pyrimidine-2-sulfonimidoyl)-1,2-dihydroisoquinolin-3-yl)ethyl)amino)pyrido[2,3-d]pyrimidin-5(8H)-one (having a diastereomeric relationship with compound no. 422) |
| 422 | 4-(((S)-1-(4-chloro-1-oxo-2-phenyl-8-((R)-pyrimidine-2-sulfonimidoyl)-1,2-dihydroisoquinolin-3-yl) ethyl)amino)pyrido[2,3-d]pyrimidin-5(8H)-one (having a diastereomeric relationship with compound no. 421) |
| 423 | (S)-4-((1-(4-chloro-1-oxo-2-phenyl-8-(pyrimidin-2-ylthio)-1,2-dihydroisoquinolin-3-yl)ethyl)amino)pyrido[2,3-d]pyrimidin-5(8H)-one |
| 424 | (S)-4-(-(1-(8-(2-(tert-butoxy)pyrimidin-5-yl)-4-chloro-1-oxo-2 phenyl-1,2-dihydroisoquinolin-3-yl)ethyl)amino)pyrido[2,3-d]pyrimidin-5(8H)-one |
| 425 | 4-(((1S)-1-(4-chloro-8-(S-methylsulfonimidoyl)-1-oxo-2-phenyl-1,2-dihydroisoquinolin-3-yl)ethyl)amino)pyrido[2,3-d]pyrimidin-5(8H)-one |
| 426 | (S)-4-((1-(8-(tert-butylthio)-4-chloro-1-oxo-2-phenyl-1,2-dihydroisoquinolin-3-yl)ethyl)amino)pyrido[2,3-d]pyrimidin-5(8H)-one |
| 427 | (S)-4-((1-(4-chloro-8-mercapto-1-oxo-2-phenyl-1,2-dihydroisoquinolin-3-yl)ethyl)amino)pyrido[2,3-d]pyrimidin-5(8H)-one |
| 428 | (S)-4-((1-(4-chloro-1-oxo-2-phenyl-8-(pyridin-4-yloxy)-1,2-dihydroisoquinolin-3-yl)ethyl)amino)pyrido[2,3-d]pyrimidin-5(8H)-one |
| 429 | (S)-4-((1-(4-chloro-1-oxy-2-phenyl-8-(pyridin-4-ylmethoxy)-1,2-dihydroisoquinolin-3-yl)ethyl)amino)pyrido[2,3-d]pyrimidin-5(8H)-one |
| 430 | (S)-4-((1-(4-chloro-1-oxo-2-phenyl-8-(pyridin-2-ylmethoxy)-1,2-dihydroisoquinolin-3-yl)ethyl)amino)pyrido[2,3-d]pyrimidin-5(8H)-one |
| 431 | 4-(((1S)-1-(4-chloro-8-(oxetan-3-ylsulfinyl)-1-oxo-2-phenyl-1,2-dihydroisoquinolin-3-yl)ethyl)amino)pyrido[2,3-d]pyrimidin-5(8H)-one |
| 432 | (S)-4-((1-(4-chloro-8-(oxetan-3-ylsulfonyl)-1-oxo-2-phenyl-1,2-dihydroisoquinolin-3-yl)ethyl)amino)pyrido[2,3-d]pyrimidin-5(8H)-one |
| 433 | (S)-4-(-(1-(8-(tert-butylsulfonyl)-4-chloro-1-oxo-2-phenyl-1,2 dihydroisoquinolin-3-yl)ethyl)amino)pyrido[2,3-d]pyrimidin-5(8H)-one |
| 434 | (S)-4-((1-(4-chloro-8-(ethylthio)-1-oxo-2-phenyl-1,2-dihydroisoquinolin-3-yl)ethyl)amino)pyrido[2,3-d]pyrimidin-5(8H)-one |
| 435 | 4-(((S)-1-(4-chloro-8-((R)-ethylsulfonimidoyl)-1-oxo-2-phenyl-1,2-dihydroisoquinolin-3-yl)ethyl)amino)pyrido[2,3-d]pyrimidin-5(8H)-one (having a diastereomeric relationship with compound no. 436) |
| 436 | 4-(((S)-1-(4-chloro-8-((S)-ethylsulfonimidoyl)-1-oxo-2-phenyl-1,2-dihydroisoquinolin-3-yl)ethyl)amino)pyrido[2,3-d]pyrimidin-5(8H)-one (having a diastereomeric relationship with compound no. 435) |
| 437 | (S)-4-((1-(4-chloro-1-oxo-2-phenyl-8-(pyridin-3-yloxy)-1,2-dihydroisoquinolin-3-yl)ethyl)amino)pyrido[2,3-d]pyrimidin-5(8H)-one |
| 438 | (S)-4-((1-(4-chloro-8-(2-(1-hydroxycyclobutyl)pyrimidin-5-yl)-1-oxo-2-phenyl-1,2-dihydroisoquinolin-3-yl) ethyl) amino)p yrido[2,3-d]pyrimidin-5(8H)-one |
| 439 | (S)-4-chloro-1-oxo-3-(1-((5-oxo-5,8-dihydropyrido[2,3-d]pyrimidin-4-yl)amino)ethyl)-2-phenyl-N-(pyridin-4-ylmethyl)-1,2-dihydroisoquinoline-9-carboxamide |
| 440 | (S)-4-chloro-1-oxo-3-(1-((5-oxo-5,8-dihydropyrido[2,3-d]pyrimidin-4-yl)amino)ethyl)-N,2-diphenyl-1,2-dihydroisoquinoline-9-carboxamide |
| 441 | 4-(((S)-1-(4-chloro-8-((S)-1-hydroxyethyl)-1-oxo-2-phenyl-1,2-dihydroisoquinolin-3-yl)ethyl)amino)pyrido[2,3-d]pyrimidin-5(8H)-one (having a diastereomeric relationship with compound no. 361) |

The compound of the present invention may comprise not only pharmaceutically acceptable salts, but also all the salts, isomers, hydrates and solvates, which may be prepared by a conventional method.

In the present invention, "pharmaceutically acceptable" may mean one which is physiologically acceptable; does not conventionally cause an allergic reaction such as gastrointestinal disturbance and dizziness, or other similar reactions thereto, when being administered into a human; and is conventionally used by those skilled in the art when preparing a pharmaceutical preparation.

In the present invention, the pharmaceutically acceptable salt may refer to a salt prepared from a non-toxic metal salt or an organic base.

As used herein, the term "salt" may refer to an acid addition salt formed by a pharmaceutically acceptable free acid, which may be advantageously used herein. The acid addition salt may be obtained from inorganic acids such as hydrochloric acid, nitric acid, phosphoric acid, sulfuric acid, hydrobromic acid, hydroiodic acid, nitrous acid or phosphorous acid, and non-toxic organic acids such as aliphatic mono and dicarboxylates, phenyl-substituted alkanoates, hydroxy alkanoates and alkandioate, aromatic acids, and aliphatic and aromatic sulfonic acids. These pharmaceutically non-toxic salts may comprise sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methyl benzoate, dinitro benzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycolate, malate, tartrate, methanesulfonate, propanesulfonate, naphthalene-1-sulfonate, naphthalene-2-sulfonate, or mandelate.

The acid addition salt according to the present invention may be prepared by a conventional method, for example, by dissolving the pyrido-pyrimidine derivative of the present invention in an excess amount of aqueous acid solution, and depositing the salt with a water-miscible organic solvent, for example, methanol, ethanol, acetone or acetonitrile. In addition, the acid addition salt may be prepared by evaporating solvent or an excessive amount of acid from the mixture, followed by drying or by soaking and filtering the precipitated salt.

In addition, a pharmaceutically acceptable metal salt may be prepared by using a base. An alkali metal or alkaline earth metal salt may be obtained, for example, by dissolving a compound in an excessive amount of alkali metal hydroxide or alkaline earth metal hydroxide solution, filtering the undissolved compound salt, and evaporating and drying the filtrate. In this case, it is pharmaceutically suitable to prepare sodium, potassium or calcium salt as the metal salt. The corresponding silver salt may be obtained by reacting the alkali metal or alkaline earth metal salt with an appropriate silver salt (e.g., silver nitrate).

In the present invention, "isomer" may specifically mean "stereoisomer," which may comprise diastereomers or enantiomers. More specifically, each of the diastereomers or a mixture thereof may be comprised, or each of the enantiomers or a mixture thereof (such as a racemate) may be comprised.

As one example, formulas a-1 and a-2 below may have an enantiomeric relationship, and formulas b-1 and b-2 below may also have an enantiomeric relationship, and R-S=O-R' (sulfoxide) may have a chiral center due to an asymmetric center on a sulfur atom, and thus may be present as an enantiomer.

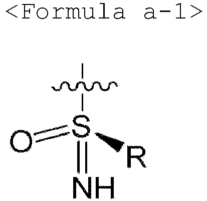

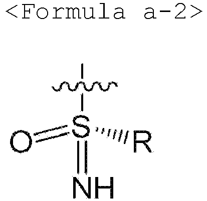

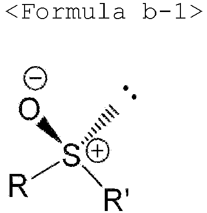

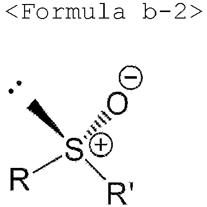

The "hydrate" of the present invention may refer to one, in which a pyrido-pyrimidine derivative, an isomer thereof or a pharmaceutically acceptable salt thereof and water are bound by a non-covalent intermolecular force, and comprises a stoichiometric or non-stoichiometric amount of water. Specifically, the hydrate may comprise water at a molar ratio of about 0.25 mol to about 10 mol based on 1 mol of an active component, more specifically about 0.5 mol, about 1 mol, about 1.5 mol, about 2 mol, about 2.5 mol, about 3 mol, about 5 mol, etc.

The "solvate" of the present invention may refer to one, in which a pyrido-pyrimidine derivative, an isomer thereof or a pharmaceutically acceptable salt thereof and water are bound by an intermolecular force, and comprises a stoichiometric or non-stoichiometric amount of solvent. Specifically, the solvate may comprise a solvent molecule at a molar ratio of about 0.25 mol to about 10 mol based on 1 mol of an active component, more specifically about 0.5 mol, about 1 mol, about 1.5 mol, about 2 mol, about 2.5 mol, about 3 mol, about 5 mol, etc.

In the present invention, the term "inhibitor" may refer to a compound that blocks or reduces the activity of an enzyme. The inhibitor may be capable of reversible or irreversible binding, and thus the term may comprise a compound that kills the substrate of the enzyme. The inhibitor may modify one or more sites on or near an enzyme active site, or may result in a conformational change elsewhere on the enzyme.

The present invention may provide a pharmaceutical composition comprising the pyrido-pyrimidine derivative of the present invention, isomers thereof or pharmaceutically acceptable salts thereof, solvates thereof or hydrates thereof as an effective ingredient.

The present invention may provide a pharmaceutical composition for preventing or treating cancer and/or metabolic diseases, comprising the pyrido-pyrimidine derivative of the present invention, isomers thereof or pharmaceutically acceptable salts thereof, solvates thereof or hydrates thereof as an effective ingredient.

The present invention may provide a pharmaceutical composition for preventing or treating PI3 kinase-associated diseases, comprising the pyrido-pyrimidine derivative of the present invention, isomers thereof or pharmaceutically acceptable salts thereof, solvates thereof or hydrates thereof as an effective ingredient.

The present invention may provide a use of the pyrido-pyrimidine derivative of the present invention, isomers thereof or pharmaceutically acceptable salts thereof for preventing or treating cancer and/or metabolic diseases.

The present invention may provide a use of the pyrido-pyrimidine derivative of the present invention, isomers thereof or pharmaceutically acceptable salts thereof, solvates thereof or hydrates thereof for preventing or treating PI3 kinase-associated diseases.

The present invention may provide a use of the pyrido-pyrimidine derivative of the present invention, isomers thereof or pharmaceutically acceptable salts thereof, solvates thereof or hydrates thereof in the manufacture of a medicament for preventing or treating PI3 kinase-associated diseases.

In addition, the present invention may provide a method for preventing or treating PI3 kinase-associated diseases comprising administering a therapeutically effective amount of the pyrido-pyrimidine derivative, isomers thereof or pharmaceutically acceptable salts thereof into a subject.

As used herein, the term "prevention" may mean all the acts, which inhibit PI3 kinase-associated diseases or delay the occurrence thereof by administering the pharmaceutical composition according to the present invention.

As used herein, the term "treatment" may mean all the acts, by which a symptom of PI3 kinase-associated diseases gets better or takes a favorable turn by administering the pharmaceutical composition according to the present invention.

The PI3 kinase-associated diseases, which are diseases to be prevented or treated by the composition of the present invention, may be a cancer disease, an autoimmune disease, and/or a respiratory disease. An example of the cancer disease may comprise liquid hematologic malignancy, ovarian cancer, cervical cancer, breast cancer, colorectal cancer, liver cancer, stomach cancer, pancreatic cancer, colon cancer, peritoneal metastatic cancer, skin cancer, bladder cancer, prostate cancer, lung cancer, osteosarcoma, fibrous tumor, brain tumor or the like. An example of the autoimmune disease may comprise rheumatoid arthritis, systemic lupus erythematosus, multiple sclerosis, diabetes, hyperthyroidism, myasthenia, autoimmune pernicious anemia or the like. An example of the respiratory disease may comprise obstructive pulmonary disease (COPD), rhinitis, asthma, chronic bronchitis, chronic pulmonary inflammatory disease, pleurisy, alveolitis, vasculitis, emphysema, pneumonia, bronchiectasis or the like.

The pharmaceutical composition of the present invention may further comprise a pharmaceutically acceptable carrier in addition to an effective ingredient. In this case, the pharmaceutically acceptable carrier may be one which is conventionally used in formulating a preparation, comprising, but not limited thereto, lactose, dextrose, sucrose, sorbitol, mannitol, starch, acacia rubber, calcium phosphate, alginate, gelatin, calcium silicate, microcrystalline cellulose, polyvinyl pyrrolidone, cellulose, water, syrup, methyl cellulose, methyl hydroxybenzoate, propyl hydroxybenzoate, talc, magnesium stearate, mineral oil and the like. In addition, the pharmaceutical composition of the present invention may further comprise lubricant, humectant, a sweetening agent, a flavoring agent, emulsifier, a suspending agent, preservative, etc. in addition to the above ingredients.

The pharmaceutical composition of the present invention may be orally or parenterally administered (for example, applied intravenously, hypodermically, intraperitoneally or locally) according to an intended method, in which a dosage thereof varies depending on a patient's condition and weight, a degree of disease, a drug form, and an administration route and time, but may be appropriately selected by those skilled in the art.

The pharmaceutical composition of the present invention may be administered in a pharmaceutically effective amount. In the present invention, the "pharmaceutically effective amount" may mean an amount enough to treat a disease at a reasonable benefit/risk ratio applicable to medical treatment, and a level of effective dose may be determined according to factors comprising a patient's disease type, severity, activity of a drug, sensitivity to the drug, an administration time, an administration route and excretion rate, a treatment period and a concurrently used drug, as well as other factors well known in a medical field. The pharmaceutical composition according to the present invention may be administered as an individual therapeutic agent or in combination with other therapeutic agents, may be administered sequentially or simultaneously with a conventional therapeutic agent, and may be administered in a single or multiple manner. Considering all the factors above, it is important to carry out an administration by an amount, in which the maximum effect may be achieved by the minimum amount without a side effect, in which such amount may be easily determined by those skilled in the art.

Specifically, the effective amount of the pharmaceutical composition of the present invention may vary depending on a patient's age, gender, condition and weight, a degree of absorption of an active ingredient into the body, an inactivation rate and excretion rate, a disease type, and a concurrently used drug, and may be generally administered in an amount of 0.001 to 160 mg per 1 kg of body weight, preferably 0.01 to 100 mg, which may be administered everyday or every other day, or administered at one time to three times a day by dividing the daily dosage of the composition. However, the effective amount may increase or decrease depending on the route of administration, the severity of disease, gender, weight, age, etc., and thus the dosage may not be intended to limit the scope of the present invention in any way.

In the present invention, "individual" may mean a subject, whose disease needs to be treated, and more specifically may refer to a mammal such as a human or non-human primate, mouse, dog, cat, horse, cow, etc.

Besides, the terms and abbreviations used in the present specification have their original meanings, unless defined otherwise.

Hereinafter, the present invention will be described in detail through Examples for better understanding of the present invention. However, the following Examples are provided only for the purpose of illustrating the present invention, and thus the scope of the present invention is not limited thereto. The Examples of the present invention are provided to more completely describe the present invention to those having ordinary skill in the art.

### Instruments and reagents used

A microwave device (Biotage, Initiator+) was used as a reactor, and prep-LC (Gilson, semi preparative medium capacity HPLC system) and MPLC (Combiflash, RF200 UV/vis) instruments were used as a device for purifying the compound of each example. The compound of each example was confirmed through ¹H-NMR (Bruker, avance III 400) and Mass (Bruker, Amazon SL) analysis, and the reagent used in the reaction of each example was purchased from Sigma-aldrich, TCI, etc.

Hereinafter, "PMB" in the compound structural formula may mean p-methoxy benzyl.

### <Preparation Example 1> Preparation of (S)-4-((1-(4,8-dichloro-1-oxo-2-phenyl-1,2-dihydroisoquinolin-3-yl)ethyl)amino)pyrido[2,3-d]pyrimidin-5(8H)-one

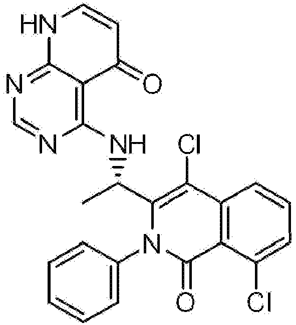

(S)-4-((1-(4,8-dichloro-1-oxo-2-phenyl-1,2-dihydroisoquinolin-3-yl)ethyl)amino)pyrido[2,3-d]pyrimidine-5(8H)-one was prepared by the same method as described in Example 10 of the International Publication WO2016/204429.

¹H NMR (400 MHz, CDCl₃) δ 1.67 (d, 3H), 5.03 (t, 1H), 6.30 (d, 1H), 7.20 (d, 1H), 7.47-7.65 (m, 6H), 7.75 (d, 1H), 7.95 (m, 1H), 8.25 (s, 1H), 10.99 (d, 1H), 12.22 (s, 1H)

### <Preparation Example 2> Preparation of (S)-4-((1-(8-chloro-1-oxo-2-phenyl-1,2-dihydroisoquinolin-3-yl)ethyl)amino)pyrido[2,3-d]pyrimidin-5(8H)-one

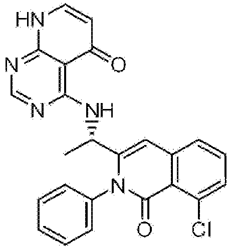

(S)-4-((1-(8-chloro-1-oxo-2-phenyl-1,2-dihydroisoquinolin-3-yl)ethyl)amino)pyrido[2,3-d]pyrimidine-5(8H)-one was prepared by the same method as described in Example 5 of the International Publication WO2016/204429.

¹H-NMR (400MHz, CD₃OD) δ ppm 1.42(d, 1H), 4.70-4.76(m, 1H), 6.15-8.20(m, 12H), 10.50(d, 1H), 12.11(s, 1H)

### <Preparation Example 3> Preparation of (S)-4-((1-(4,8-dichloro-1-oxo-2-phenyl-1,2-dihydroisoquinolin-3-yl)ethyl)amino)-8-(4-methoxybenzyl)pyrido[2,3-d]pyrimidin-5(8H)-one

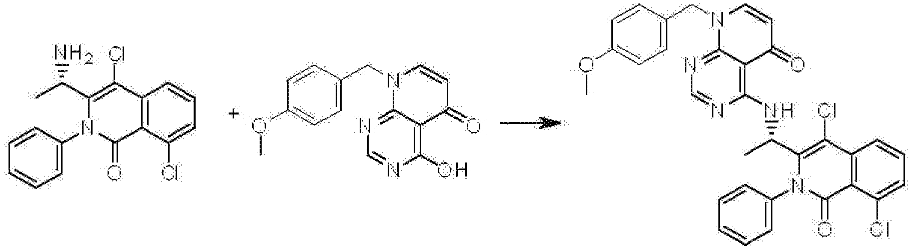

According to the above reaction formula, (S)-4-((1-(4,8-dichloro-1-oxo-2-phenyl-1,2-dihydroisoquinolin-3-yl)ethyl)amino)-8-(4-methoxybenzyl)pyrido[2,3-d]pyrimidine-5(8H)-one was prepared by the same method as described in Step 1 of Example 11 of the International Publication WO2016/204429 by using the (S)-3-(1-aminoethyl)-4,8-dichloro-2-phenylisoquinolin-1(2H)-one prepared by the method of Example 10 of the International Publication WO2016/204429.

¹H NMR (400 MHz, CDCl₃) 51.62 (d, 3H), 3.71 (s, 3H), 4.85 (t, 1H), 5.37 (s, 2H), 6.24 (d, 1H), 6.84 (d, 2H), 7.23 (d, 2H), 7.41-7.82(m, 7H), 7.96 (d, 1H), 8.14 (d, 1H), 8.42 (s, 1H), 11.05 (d, 1H)

### <Preparation Example 4> Preparation of (S)-4-((1-(8-chloro-1-oxo-2-phenyl-1,2-dihydroisoquinolin-3-yl)ethyl)amino)-8-(4-methoxybenzyl)pyrido[2,3-d]pyrimidin-5(8H)-one

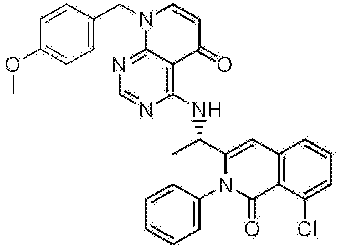

(S)-4-((1-(8-chloro-1-oxo-2-phenyl-1,2-dihydroisoquinolin-3-yl)ethyl)amino)-8-(4-methoxybenzyl)pyrido[2,3-d]pyrimidin-5(8H)-one was prepared by the same method as described in Step 2 of Example 5 of the International Publication WO2016/204429.

1H NMR (400 MHz, CDCl3) δ 1.59 (d, 3H), 3.70 (s, 3H), 4.87 (t, 1H), 5.33 (s, 2H), 6.21 (d, 1H), 6.82 (d, 2H), 7.25 (d, 2H), 7.41-7.82(m, 7H), 7.85-8.17 (m, 3H), 8.42 (s, 1H), 11.02 (d, 1H)

### <Example 1> Preparation of (S)-4-((1-(4-bromo-8-chloro-1-oxo-2-phenyl-1,2-dihydroisoquinolin-3-yl)ethyl)amino)pyrido[2,3-d]pyrimidin-5(8H)-one

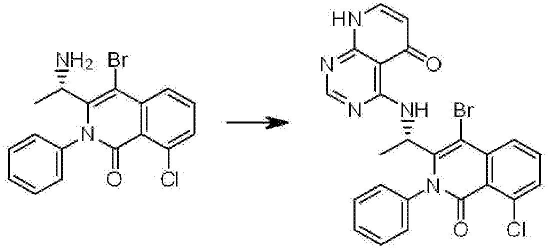

(S)-3-(1-aminoethyl)-4-bromo-8-chloro-2-phenylisoquinolin-1(2H)-one prepared by using N-bromosuccinimide instead of N-chlorosuccinimide of Step 7 by the same method as described in Preparation Example 10 of the International Publication WO2016/204429 was used to prepare (S)-4-((1-(4-bromo-8-chloro-1-oxo-2-phenyl-1,2-dihydroisoquinolin-3-yl) ethyl)amino)pyrido[2,3-d]pyrimidin-5(8H)-one by the same method as described in Example 5 of the International Publication WO2016/204429.

¹H NMR (400MHz, DMSO-d₆) δ ppm 1.60(d, 3H), 4.84(m, 1H), 6.13(d, 1H), 7.14(s, 1H), 7.30-7.80(m, 10H), 8.33(d, 1H), 10.90(br, 1H)

### <Example 2> Preparation of (S)-4-((1-(4-chloro-1-oxo-2-phenyl-8-(4,4,5,5-tetramethyl-1,3,2-dioxaborolan-2-yl)-1,2-dihydroisoquinolin-3-yl)ethyl)amino)pyrido[2,3-d]pyrimidin-5(8H)-one

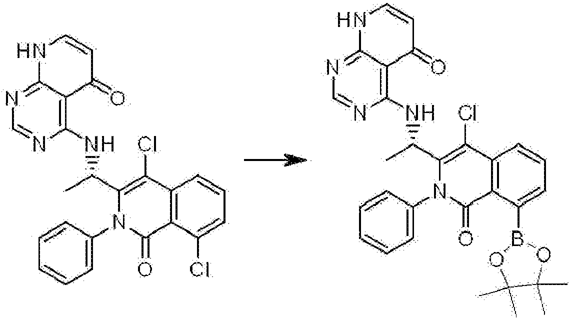

The 100 mg of (S)-4-((1-(4,8-dichloro-1-oxo-2-phenyl-1,2-**dihydroisoquinolin-3-yl)**ethyl)amino)pyrido[2,3-d]pyrimidin-5(8H)-one prepared in above Preparation Example 1, 107 mg of boron pinacol ester, 62 mg of potassium acetate, 6 mg of bis(triphenylphosphin)palladium chloride and 30 mg of 2-dicyclohexylphosphino-2',4',6'-trisisopropylbiphenyl [XPhos] were added to 1 mL of dioxane and stirred under reflux at 180°C for 15 hours. Upon completion of the reaction, the resulting mixture was cooled down to room temperature, after which a saturated aqueous solution of ammonium chloride was added, followed by extraction with ethyl acetate two to three times. A combined organic layer was washed with a saturated aqueous solution of sodium chloride, dried over anhydrous magnesium sulfate, and concentrated under reduced pressure, and then the resulting residues were purified by Prep-LC. The resulting product was vacuum-dried for five hours to obtain 30 mg of (S)-4-((1-(4-chloro-1-oxo-2-phenyl-8-(4,4,5,5-tetramethyl-1,3,2-dioxaboran-2-yl)-1,2-dihydroisoquinolin-3-yl)ethyl)amino)pyrido[2,3-d]pyrimidine-5(8H)-one.

¹H-NMR (400MHz, CDCl₃) δ ppm 1.34(d, 12H), 1.65(d, 3H), 4.97(m, 1H), 6.27(d, 1H), 7.20-7.80(m, 8H), 7.92(d, 1H), 8.24(s, 1H), 10.96(br, 1H), 12.00(br, 1H)

### <Example 3> Preparation of (S)-4-((1-(8-(6-(1H-pyrrol-1-yl)pyridin-3-yl)-4-chloro-1-oxo-2-phenyl-1,2-dihydroisoquinolin-3-yl)ethyl)amino)pyrido[2,3-d]pyrimidin-5(8H)-one

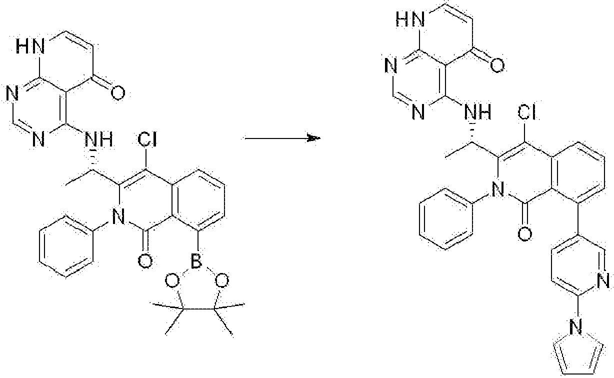

The 30 mg of (S)-4-((1-(4-chloro-1-oxo-2-phenyl-8-(4,4,5,5-tetramethyl-1,3,2-dioxaborolan-2-yl)-1,2-dihydroisoquinolin-3-yl)ethyl)amino)pyrido[2,3-d]pyrimidin-5(8H)-one prepared in above Example 2, 18 mg of 5-bromo-2-(1H-pyrrol-1-yl)pyridine, 0.09 mL of 2M aqueous potassium carbonate solution, and 4 mg of bis(triphenylphosphin)palladium chloride were added to 2 mL of dioxane and irradiated with microwaves at 170°C for 30 minutes by using a microwave reactor. Upon completion of the reaction, the resulting mixture was cooled down to room temperature, after which a saturated aqueous solution of sodium chloride was added, followed by extraction with ethyl acetate two to three times. An organic layer was dried over anhydrous sodium sulfate, and concentrated under reduced pressure, and then the resulting residues were purified by Prep-LC. The resulting product was vacuum-dried for three hours to obtain 5 mg of (S)-4-((1-(8-(6-(1H-pyrrol-1-yl)-pyridin-3-yl)-4-chloro-1-oxo-2-phenyl-1,2-dihydroisoquinolin-3-yl)ethyl)amino)pyrido[2,3-d]pyrimidin-5(8H)-one.

¹H-NMR (400MHz, CDCl₃) δ ppm 1.68(d, 3H), 5.03-5.06(m, 1H), 6.30-8.30(m, 18H), 11/02(s, 1H)

### <Examples 4 to 55>

In Examples 4 to 55, the compound of each Example was prepared by substantially the same preparation method as described in above Example 3, except for using an appropriate reactant in consideration of the structure of the corresponding compound.

### <Example 4> Preparation of (S)-4-((1-(4-chloro-1-oxo-2-phenyl-8-(pyridin-3-yl)-1,2-dihydroisoquinolin-3-yl)ethyl)amino)pyrido[2,3-d]pyrimidin-5(8H)-one

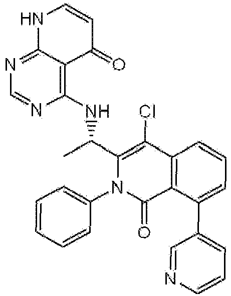

¹H-NMR (400MHz, DMSO-d₆) δ ppm 1.68(d, 3H), 5.03 (m, 1H), 6.32(d, 1H), 7.12(m, 1H), 7.30(m, 1H), 7.487.52-7.15(m, 5H), 7.62(m,1H), 7.80(t,1H), 7.90(m, 1H), 8.18(d, 1H), 8.27(s, 1H), 8.55(s, 1H), 8.63(s, 2H), 11.15(s, 1H)

### <Example 5> Preparation of (S)-4-((1-(4-chloro-8-(1-methyl-1H-pyrazol-4-yl)-1-oxo-2-phenyl-1,2-dihydroisoquinolin-3-yl)ethyl)amino)pyrido[2,3-d]pyrimidin-5(8H)-one

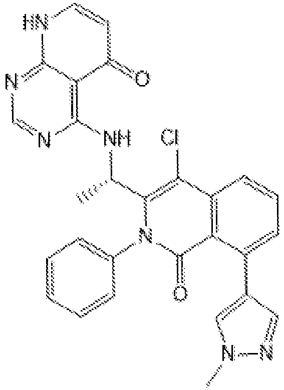

¹H-NMR (400MHz, DMSO-d₆) δ ppm 1.68(d, 3H), 3.82(s, 3H), 5.03(t, 1H), 6.25(d, 1H), 7.12(d, 1H), 7.30-7.70(m, 11H), 7.95(d, 1H), 8.25(s, 1H), 10.89(s, 1H)

### <Example 6> Preparation of (S)-4-((1-(8-(benzofuran-2-yl)-4-chloro-1-oxo-2-phenyl-1,2-dihydroisoquinolin-3-yl)ethyl)amino)pyrido[2,3-d]pyrimidin-5(8H)-one

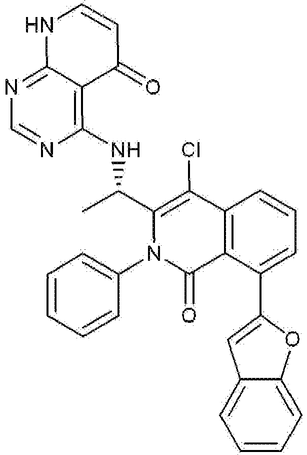

¹H-NMR (400MHz, CDCl₃) δ ppm 1.67(d, 3H), 5.03(m, 1H), 6.30(d, 1H), 6.78(s, 1H), 7.25-7.13(m, 3H), 7.80-7.40(m, 9H), 8.19(d, 1H), 8.26(s, 1H), 10.90(s, 1H)

### <Example 7> Preparation of (S)-4'-chloro-3'-(1-((5-oxo-5,8-dihydropyrido[2,3-d]pyrimidin-4-yl)amino)ethyl)-2'-phenyl-3,4-dihydro-1H-(2,8'-biisoquinolin)-1'(2'H)-one

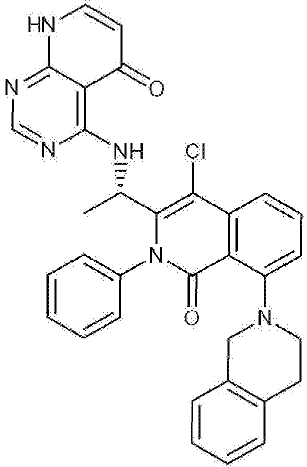

¹H-NMR (400MHz, CDCl₃) δ ppm 1.66(d, 3H), 2.26(s, 3H), 2.90-3.48(m, 4H), 5.05-5.12(m, 1H), 6.27-8.22(m, 15H), 11.02(d, 1H), 11.59(s, 1H)

### <Example 8> Preparation of 4-(((1S)-1-(4-chloro-1-oxo-2-phenyl-8-(3a,4,7,7a-tetrahydro-1H-isoindol-2(3H)-yl)-1,2-dihydroisoquinolin-3-yl)ethyl)amino)pyrido[2,3-d]pyrimidin-5(8H)-one

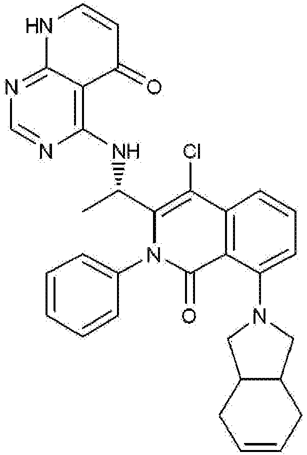

¹H-NMR (400MHz, CDCl₃) δ ppm 1.65(d, 3H), 1.85-2.56(m, 10H), 5.05-5.12(m, 1H), 6.27-8.22(m, 13H), 11.01(d, 1H), 11.49(s, 1H)

### <Example 9> Preparation of (S)-4-((1-(4-chloro-1-oxo-2-phenyl-8-(4-(pyridin-4-yl)piperazin-1-yl)-1,2-dihydroisoquinolin-3-yl)ethyl)amino)pyrido[2,3-d]pyrimidin-5(8H)-one

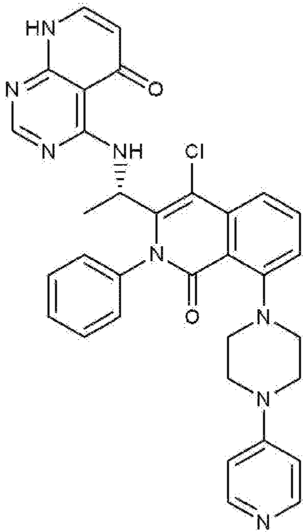

¹H-NMR (400MHz, CDCl₃) δ ppm 1.63(d, 3H), 2.06-2.56(d, 8H), 5.05-5.12(m, 1H), 6.25-8.22(m, 15H), 11.02(d, 1H), 11.59(s, 1H)

### <Example 10> Preparation of (S)-4-((1-(8-([1,4'-bipiperidin]-1'-yl)-4-chloro-1-oxo-2-phenyl-1,2-dihydroisoquinolin-3-yl)ethyl)amino)pyrido[2,3-d]pyrimidin-5(8H)-one

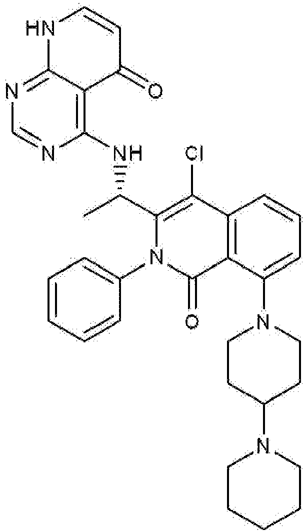

¹H-NMR (400MHz, CDCl₃) δ ppm 1.45(m, 2H), 1.63(d, 3H), 1.85-2.56(d, 16H), 5.05-5.12(m, 1H), 6.25-8.22(m, 11H), 10.98(d, 1H), 11.49(s, 1H)

### <Example 11> Preparation of (S)-4-((1-(4-chloro-8-(4-(1-methylpiperidin-4-yl)piperazin-1-yl)-1-oxo-2-phenyl-1,2-dihydroisoquinolin-3-yl)ethyl)amino)pyrido[2,3-d]pyrimidin-5(8H)-one

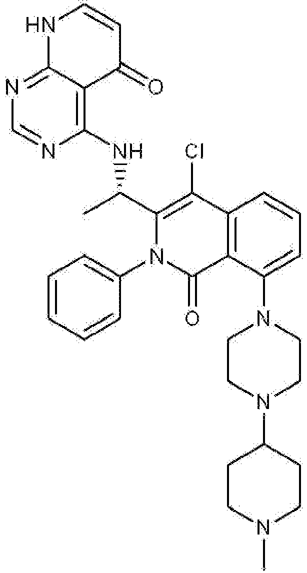

¹H-NMR (400MHz, CDCl₃) δ ppm 1.64(d, 3H), 1.85-2.56(d, 19H), 5.05-5.12(m, 1H), 6.25-8.22(m, 11H), 10.98(d, 1H), 11.49(s, 1H)

### <Example 12> Preparation of (S)-4-((1-(1-oxo-2-phenyl-8-(pyridin-3-yl)-1,2-dihydroisoquinolin-3-yl)ethyl)amino)pyrido[2,3-d]pyrimidin-5(8H)-one

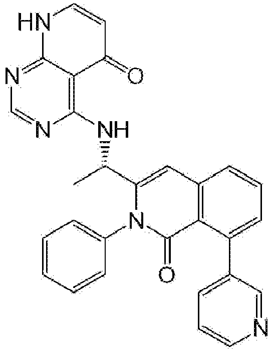

¹H-NMR (400MHz, DMSO-d₆) δ ppm 1.68(d, 3H), 5.03 (m, 1H), 6.32(d, 1H), 7.12(m, 1H), 7.30(m, 1H), 7.487.52-7.15(m, 5H), 7.62(m, 1H), 7.80(t, 1H), 7.90-8.20(m, 3H), 8.27(s, 1H), 8.55(s, 1H), 8.63(s, 2H), 11.15(s, 1H)

### <Example 13> Preparation of (S)-4-((1-(8-(6-methylpyridin-3-yl)-1-oxo-2-phenyl-1,2-dihydroisoquinolin-3-yl)ethyl)amino)pyrido[2,3-d]pyrimidin-5(8H)-one

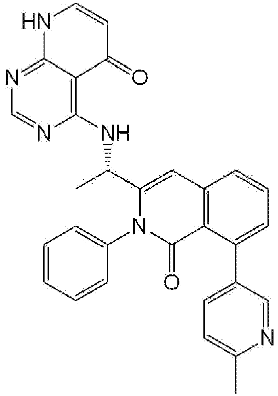

¹H-NMR (400MHz, DMSO-d₆) δ ppm 1.68(d, 3H), 2.75 (s, 3H), 5.03(m, 1H), 6.32(d, 1H), 7.12(m, 1H), 7.30(m, 1H), 7.487.52-7.15(m, 5H), 7.62(m, 1H), 7.80(t, 1H), 7.90-8.20(m, 3H), 8.27(s, 1H), 8.55(s, 1H), 8.63(s, 2H), 11.15(s, 1H)

### <Example 14> Preparation of (S)-4-((1-(8-(4-fluorophenyl)-1-oxo-2-phenyl-1,2-dihydroisoquinolin-3-yl)ethyl)amino)pyrido[2,3-d]pyrimidin-5(8H)-one

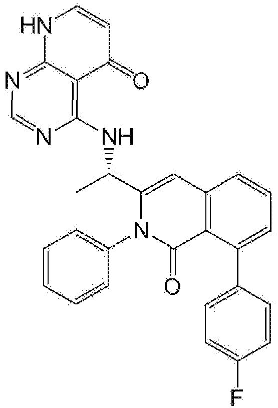

¹H-NMR (400MHz, DMSO-d₆) δ ppm 1.59(d, 3H), 4.85(m, 1H), 6.12(d, 1H), 7.65-7.15(m, 11H), 7.76(d, 1H), 7.84(t, 1H), 8.04(d, 1H), 8.30(s, 1H), 10.95(s, 1H)

### <Example 15> Preparation of (S)-4-((1-(8-(1-methyl-1H-pyrazol-4-yl)-1-oxo-2-phenyl-1,2-dihydroisoquinolin-3-yl)ethyl)amino)pyrido[2,3-d]pyrimidin-5(8H)-one

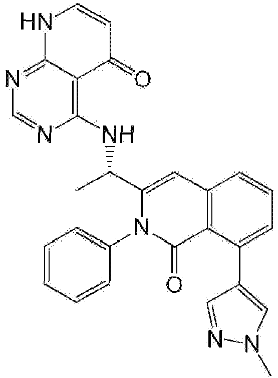

¹H-NMR (400MHz, DMSO-d₆) δ ppm 1.67(d, 3H), 3.82(s, 3H), 5.03(t, 1H), 6.24(d, 1H), 7.12(d, 1H), 7.30-7.70(m, 12H), 7.95(d, 1H), 8.25(s, 1H), 10.84(s, 1H)

### <Example 16> Preparation of (S)-4-((1-(4-bromo-8-(4-(dimethylamino)phenyl)-1-oxo-2-phenyl-1,2-dihydroisoquinolin-3-yl)ethyl)amino)pyrido[2,3-d]pyrimidin-5(8H)-one

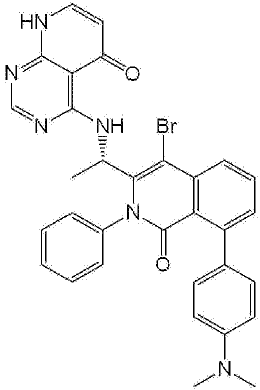

¹H-NMR (400MHz, CDCl₃) δ ppm 1.51(d, 3H), 3.80(s, 6H), 4.95(m, 1H), 6.40-7.70(m, 15H), 8.23(s, 1H), 10.59(br, 1H)

### <Example 17> Preparation of (S)-4-((1-(4-bromo-8-(6-methylpyridin-3-yl)-1-oxo-2-phenyl-1,2-dihydroisoquinolin-3-yl)ethyl)amino)pyrido[2,3-d]pyrimidin-5(8H)-one

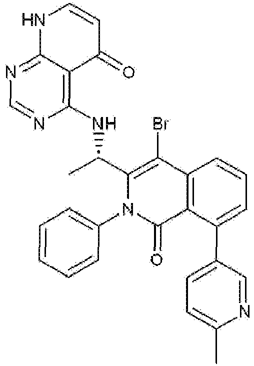

¹H-NMR (400MHz, DMSO-d₆) δ ppm 1.64(s, 3H), 1.70(d, 3H), 5.32(m, 1H), 6.34(d, 1H), 6.90-7.12(m, 2H), 7.20-7.40(m, 3H), 7.45-7.62(m, 4H), 8.16(d, 1H), 8.29(d, 1H), 10.80(br, 1H), 11.10(br, 1H)

### <Example 18> Preparation of (S)-4-((1-(4-bromo-8-(4-fluorophenyl)-1-oxo-2-phenyl-1,2-dihydroisoquinolin-3-yl)ethyl)amino)pyrido[2,3-d]pyrimidin-5(8H)-one

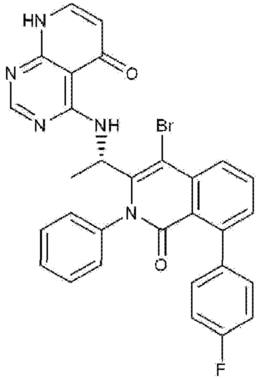

¹H-NMR (400MHz, DMSO-d₆) δ ppm 1.59(d, 3H), 4.85(m, 1H), 6.15(d, 1H), 7.65-7.20(m, 11H), 7.78(d, 1H), 7.84(t, 1H), 8.04(d, 1H), 8.30(s, 1H), 10.95(s, 1H)

### <Example 19> Preparation of (S)-4-((1-(4-bromo-8-(1-methyl-1H-pyrazol-4-yl)-1-oxo-2-phenyl-1,2-dihydroisoquinolin-3-yl)ethyl)amino)pyrido[2,3-d]pyrimidin-5(8H)-one

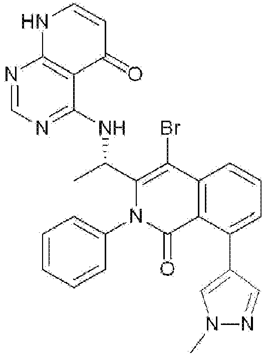

¹H-NMR (400MHz, DMSO-d₆) δ ppm 1.68(d, 3H), 3.82(s, 3H), 5.03(t, 1H), 6.33(d, 1H), 7.17(d, 1H), 7.30-7.80(m, 10H), 7.15(d, 1H), 8.27(s, 1H), 10.89(br, 1H), 10.90(br, 1H)

### <Preparation Example 20> Preparation of (S)-4-((1-(8-(4-(dimethylamino)phenyl)-1-oxo-2-phenyl-1,2-dihydroisoquinolin-3-yl)ethyl)amino)pyrido[2,3-d]pyrimidin-5(8H)-one

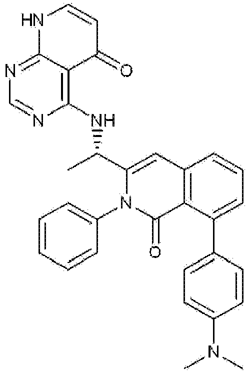

¹H-NMR (400MHz, CDCl₃) δ ppm 1.66(d, 3H), 2.54(s, 6H), 5.00-5.07(m, 1H), 6.25-8.24(m, 16H), 11.03(d, 1H), 11.63(s, 1H)

### <Example 21> Preparation of (S)-4-((1-(4-chloro-1-oxo-8-(2-oxo-2,3-dihydro-1H-benzo[d]imidazol-5-yl)-2-phenyl-1,2-dihydroisoquinolin-3-yl)ethyl)amino)pyrido[2,3-d]pyrimidin-5(8H)-one

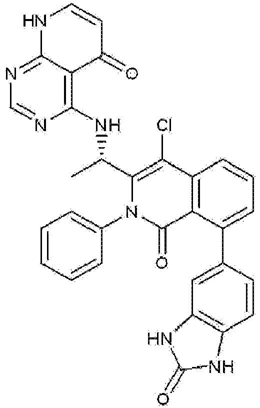

¹H-NMR (400MHz, CD₃OD) δ ppm 1.68(d, 3H), 5.06(m, 1H), 6.29(d, 1H), 6.80-7.00(m, 3H), 7.20-7.85(m, 8H), 7.00(s, 1H), 7.10-7.85(m, 8H), 8.13(d, 1H), 8.27(s, 1H), 11.10(br, 1H)

### <Example 22> Preparation of (S)-4-((1-(4-chloro-8-(2-methylbenzo[d]oxazol-6-yl)-1-oxo-2-phenyl-1,2-dihydroisoquinolin-3-yl)ethyl)amino)pyrido[2,3-d]pyrimidin-5(8H)-one

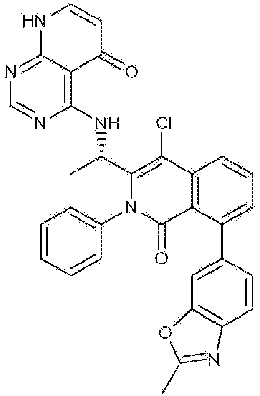

¹H-NMR (400MHz, CDCl₃) δ ppm 1.69(d, 3H), 2.57(s, 3H), 5.08(m, 1H), 6.34(d, 1H), 7.18(m, 2H), 7.30-7.80(m, 9H), 8.12(d, 1H), 8.26(s, 1H), 11.35(br, 1H)

### <Example 23> Preparation of (S)-4-(1-oxo-3-(1-((5-oxo-5,8-dihydropyrido[2,3-d]pyrimidin-4-yl)amino)ethyl)-2-phenyl-1,2-dihydroisoquinolin-8-yl)benzonitrile

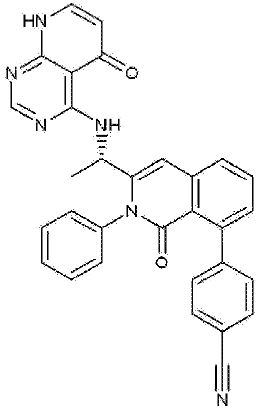

¹H-NMR (400MHz, CDCl₃) δ ppm 1.72(d, 3H), 5.13-5.20(m, 1H), 6.43-8.22(m, 16H), 11.20(d, 1H)

### <Example 24> Preparation of (S)-4-((1-(8-(benzo[d]isoxazol-6-yl)-4-chloro-1-oxo-2-phenyl-1,2-dihydroisoquinolin-3-yl)ethyl)amino)pyrido[2,3-d]pyrimidin-5(8H)-one

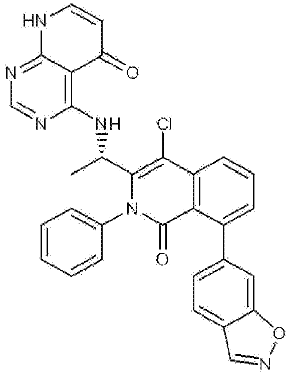

¹H-NMR (400MHz, CD₃OD) δ ppm 1.68(d, 3H), 5.02-5.09(m, 1H), 6.30-8.24(m, 15H), 10.92(d, 1H)

### <Example 25> (S)-4-((1-(4-chloro-1-oxo-2-phenyl-8-(1H-pyrrolo[2,3-b]pyridin-5-yl)-1,2-dihydroisoquinolin-3-yl)ethyl)amino)pyrido[2,3-d]pyrimidin-5(8H)-one

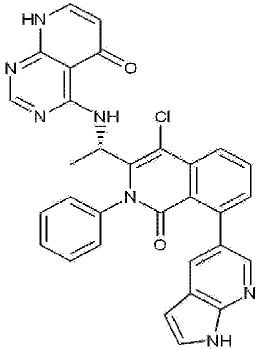

¹H-NMR (400MHz, DMSO-d₆) δ ppm 1.60(d, 3H), 4.86 (m, 1H), 6.15(d, 1H), 6.40(s, 1H), 7.30-7.95(m, 9H), 8.06(m, 1H), 8.33(s, 1H), 10.95(br), 11.60(s, 1H), 12.13(s, 1H)

### <Example 26> Preparation of (S)-4-((1-(4-chloro-8-(2-(2-hydroxypropan-2-yl)pyrimidin-5-yl)-1-oxo-2-phenyl-1,2-dihydroisoquinolin-3-yl)ethyl)amino)pyrido[2,3-d]pyrimidin-5(8H)-one

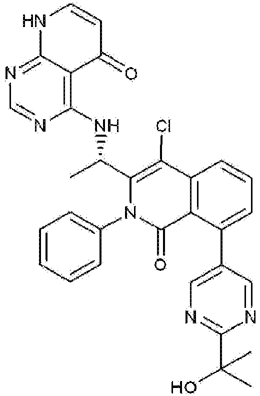

¹H-NMR (400MHz, CDCl₃) δ ppm 1.59(d, 6H), 1.79(d, 3H), 5.03-5.07(m, 1H), 6.31-8.63(m, 13H), 11.05(s, 1H), 11.06(s, 1H)

### <Example 27> Preparation of (S)-4-((1-(4-chloro-1-oxo-2-phenyl-8-(1H-pyrazolo[4,3-c]pyridin-6-yl)-1,2-dihydroisoquinolin-3-yl)ethyl)amino)pyrido[2,3-d]pyrimidin-5(8H)-one

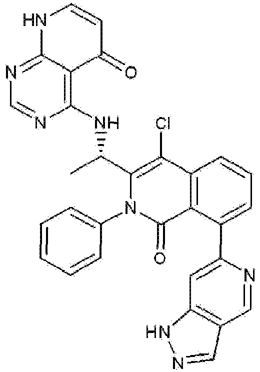

¹H-NMR (400MHz, CDCl₃) δ ppm 1.68(d, 3H), 5.06-5.10(m, 1H), 6.29-8.41(m, 14H), 11.03(s, 1H), 12.10(s, 1H)

### <Example 28> Preparation of (S)-4-((1-(4-chloro-8-(5,6-dimethoxypyrazin-2-yl)-1-oxo-2-phenyl-1,2-dihydroisoquinolin-3-yl)ethyl)amino)pyrido[2,3-d]pyrimidin-5(8H)-one

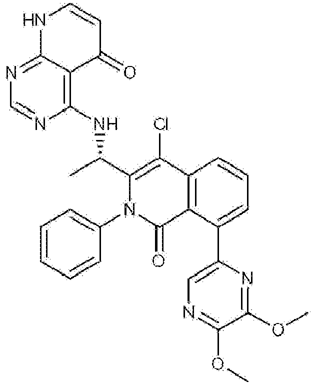

¹H-NMR (400MHz, CDCl₃) δ ppm 1.67(d, 3H), 3.93(s, 3H), 3.97(s, 3H), 5.04-5.07(m, 1H), 6.30-8.25(m, 12H), 11.05(s, 1H)

### <Example 29> Preparation of (S)-4-((1-(4-chloro-1-oxo-2-phenyl-8-(4-(thiazol-2-yl)phenyl)-1,2-dihydroisoquinolin-3-yl)ethyl)amino)pyrido[2,3-d]pyrimidin-5(8H)-one

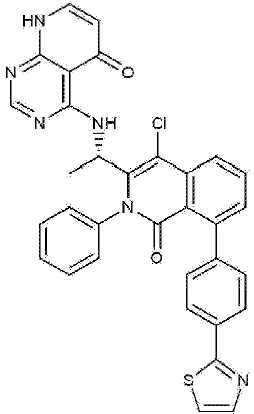

¹H-NMR (400MHz, CDCl₃) δ ppm 1.68(d, 3H), 5.01-5.08(m, 1H), 6.28-8.24(m, 17H), 11.04(s, 1H), 11.59(s, 1H)

### <Example 30> Preparation of (S)-4-((1-(4-chloro-8-(5-methoxypyrimidin-2-yl)-1-oxo-2-phenyl-1,2-dihydroisoquinolin-3-yl)ethyl)amino)pyrido[2,3-d]pyrimidin-5(8H)-one

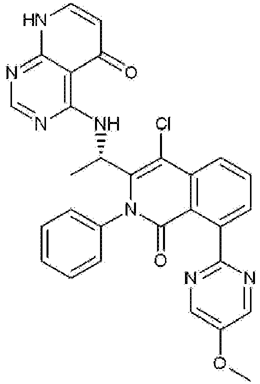

¹H-NMR (400MHz, CDCl₃) δ ppm 1.64(d, 3H), 3.89(s, 3H), 4.95-4.98(m, 1H), 6.29-8.37(m, 13H), 10.60(s, 1H), 10.95(s, 1H)

### <Example 31> Preparation of (S)-6-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)nicotinonitrile

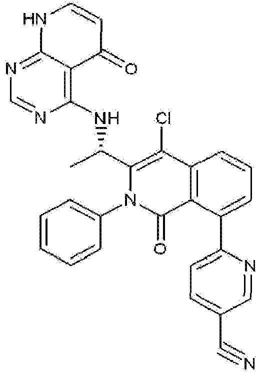

¹H-NMR (400MHz, CDCl₃) δ ppm 1.67(d, 3H), 4.94-4.95(m, 1H), 6.42-8.81(m, 14H), 10.94(s, 1H)

### <Example 32> Preparation of (S)-4-((1-(4-chloro-1-oxo-2-phenyl-8-(quinazolin-6-yl)-1,2-dihydroisoquinolin-3-yl)ethyl)amino)pyrido[2,3-d]pyrimidin-5(8H)-one

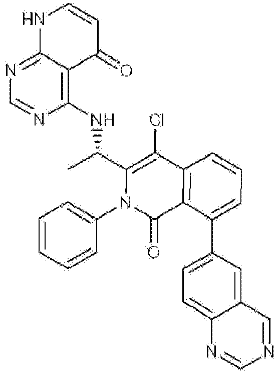

¹H-NMR (400MHz, CDCl₃) δ ppm 1.69(d, 3H), 5.04-4.09(m, 1H), 6.31-9.32(m, 15H), 11.03(s, 1H), 11.16(s, 1H)

### <Example 33> Preparation of (S)-4-((1-(4-chloro-8-(4-(isoxazol-3-yl)phenyl)-1-oxo-2-phenyl-1,2-dihydroisoquinolin-3-yl)ethyl)amino)pyrido[2,3-d]pyrimidin-5(8H)-one

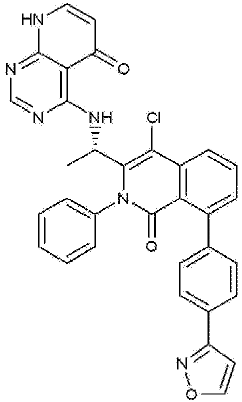

¹H-NMR (400MHz, CDCl₃) δ ppm 1.67(d, 3H), 5.03-5.08 (m, 1H), 6.30-8.13(m, 17H), 10.51(s, 1H), 10.98(s, 1H)

### <Example 34> Preparation of (S)-4-((1-(8-(6-(tert-butyl)pyridin-3-yl)-4-chloro-1-oxo-2-phenyl-1,2-dihydroisoquinolin-3-yl)ethyl)amino)pyrido[2,3-d]pyrimidin-5(8H)-one

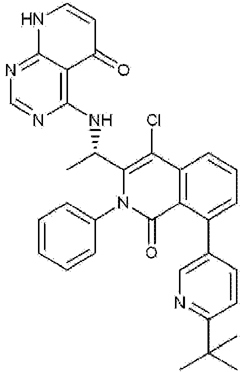

¹H-NMR (400MHz, CDCl₃) δ ppml.33(s, 9H), 1.68(d, 3H), 5.01-5.05(m, 1H), 6.29-8.43(m, 14H), 11.03(s, 1H), 11.68(s, 1H)

### <Example 35> Preparation of (S)-4-((1-(4-chloro-1-oxo-2-phenyl-8-(pyrimidin-2-yl)-1,2-dihydroisoquinolin-3-yl)ethyl)amino)pyrido[2,3-d]pyrimidin-5(8H)-one

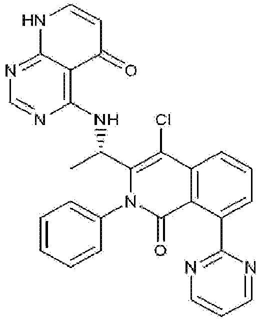

¹H-NMR (400MHz, CDCl₃) δ ppm 1.70(d, 3H), 5.08-5.11 (m, 1H), 6.38-8.41(m, 14H), 10.90(s, 1H)

### <Example 36> Preparation of (S)-4-((1-(4-chloro-8-(3-fluoro-4,5-dihydroxyphenyl)-1-oxo-2-phenyl-1,2-dihydroisoquinolin-3-yl)ethyl)amino)pyrido[2,3-d]pyrimidin-5(8H)-one

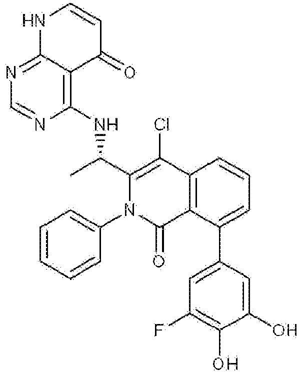

¹H-NMR (400MHz, CDCl₃) δ ppm 1.68(d, 3H), 5.07-5.11 (m, 1H), 6.29-8.23(m, 13H), 10.95(s, 1H)

### <Example 37> Preparation of (S)-4-((1-(8-([1,2,4]triazolo[4,3-a]pyridin-6-yl)-4-chloro-1-oxo-2-phenyl-1,2-dihydroisoquinolin-3-yl)ethyl)amino)pyrido[2,3-d]pyrimidin-5(8H)-one

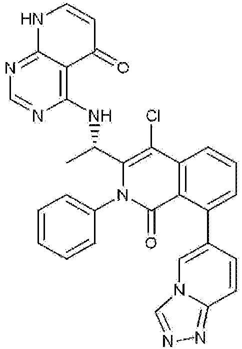

¹H-NMR (400MHz, CDCl₃) δ ppm 1.68(d, 3H), 5.04-5.07(m, 1H), 6.03-8.72(m, 15H), 10.95(s, 1H)

### <Example 38> Preparation of (S)-4-((1-(4-chloro-1-oxo-2-phenyl-1,2-dihydroisoquinolin-3-yl)ethyl)amino)pyrido[2,3-d]pyrimidin-5(8H)-one

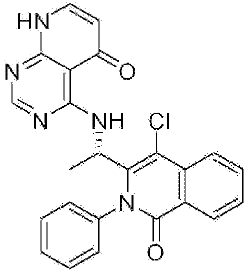

¹H-NMR (400MHz, CDCl₃) δ ppm 1.68(d, 3H), 5.07-5.11 (m, 1H), 6.29-8.23(m, 13H), 10.95(s, 1H)

### <Example 39> Preparation of (S)-2-(5-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)pyrimidin-2-yl)acetonitrile

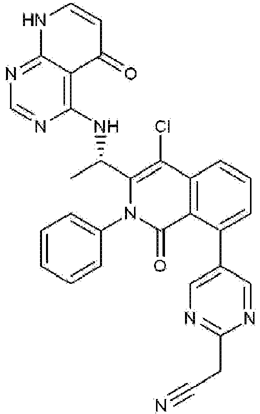

¹H-NMR (400MHz, CDCl₃) δ ppm 1.69(d, 3H), 4.08(s, 2H), 5.03-5.06(m, 1H), 6.32-8.63(m, 13H), 11.05(s, 1H), 11.76(s, 1H)

### <Example 40> Preparation of (S)-4-((1-(8-([1,2,4]triazolo[4,3-a]pyrimidin-6-yl)-4-chloro-1-oxo-2-phenyl-1,2-dihydroisoquinolin-3-yl)ethyl)amino)pyrido[2,3-d]pyrimidin-5(8H)-one

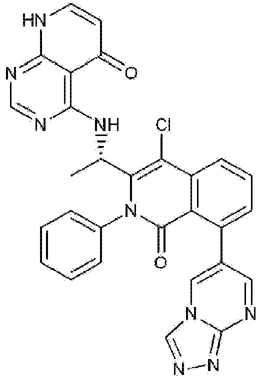

¹H-NMR (400MHz, CDCl₃) δ ppm 1.70(d, 3H), 5.04-5.07(m, 1H), 6.32-8.75(m, 14H), 11.05(s, 1H), 11.93(s, 1H)

### <Example 41> Preparation of (S)-4-((1-(4-chloro-8-(5-methoxypyridin-2-yl)-1-oxo-2-phenyl-1,2-dihydroisoquinolin-3-yl)ethyl)amino)pyrido[2,3-d]pyrimidin-5(8H)-one

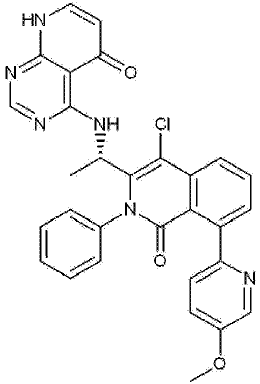

¹H-NMR (400MHz, CDCl₃) δ ppm 1.68(d, 3H), 3.89(s, 3H), 5.02-5.09(m, 1H), 6.32-8.26(m, 15H), 11.12(d, 1H), 11.59(s, 1H)

### <Example 42> Preparation of (S)-4-((1-(8-([1,2,4]triazolo[1,5-a]pyridin-6-yl)-4-chloro-1-oxo-2-phenyl-1,2-dihydroisoquinolin-3-yl)ethyl)amino)pyrido[2,3-d]pyrimidin-5(8H)-one

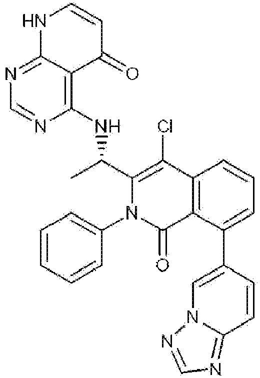

¹H-NMR (400MHz, CDCl₃) δ ppm 1.70(d, 3H), 5.05(m, 1H), 6.31(d, 1H), 7.14(d, 1H), 7.37(d, 1H), 7.40-7.85(m, 9H), 8.22(d, 1H), 8.28(s, 2H), 8.43(s, 1H), 11.03(br, 1H), 11.47(br, 1H)

### <Example 43> Preparation of (S)-4-((1-(4-chloro-8-(5-methoxypyrimidin-2-yl)-1-oxo-2-phenyl-1,2-dihydroisoquinolin-3-yl)ethyl)amino)pyrido[2,3-d]pyrimidin-5(8H)-one

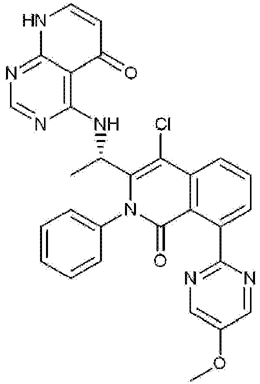

¹H-NMR (400MHz, CDCl₃) δ ppm 1.69(d, 3H), 4.01(s, 3H), 5.09-5.12(m, 1H), 6.37-8.52(m, 13H), 10.94(s, 1H)

### <Example 44> Preparation of (S)-5-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)pyrimidine-2-carbonitrile

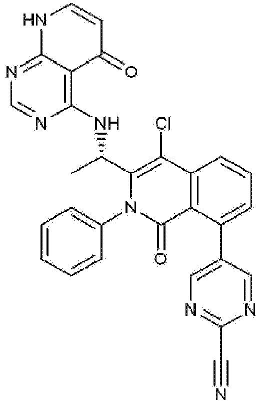

¹H-NMR (400MHz, CDCl₃) δ ppm 1.69(d, 3H), 5.02-5.08(m, 1H), 6.32-8.26(m, 13H), 11.02(s, 1H), 11.26(s, 1H)

### <Example 45> Preparation of (S)-4-((1-(4-chloro-8-(6-methoxypyrimidin-4-yl)-1-oxo-2-phenyl-1,2-dihydroisoquinolin-3-yl)ethyl)amino)pyrido[2,3-d]pyrimidin-5(8H)-one

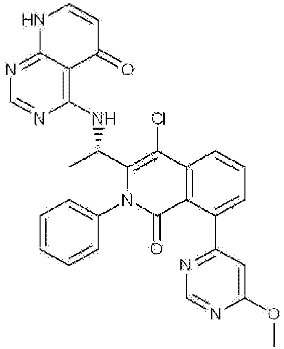

¹H-NMR (400MHz, CD₃OD) δ ppm 1.69(d, 3H), 4.06(s, 3H), 5.07-5.11(m, 1H), 6.40-8.76(m, 12H), 11.01(br, 1H)

### <Example 46> Preparation of (S)-4-((1-(4-chloro-8-(6-cyclopropylpyridin-3-yl)-1-oxo-2-phenyl-1,2-dihydroisoquinolin-3-yl)ethyl)amino)pyrido[2,3-d]pyrimidin-5(8H)-one

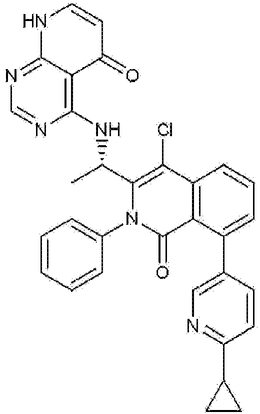

¹H-NMR (400MHz, CD₃OD) δ ppm 1.12-1.19(m, 4H), 2.10-2.14(m, 1H), 5.03-5.06(m, 1H), 6.30-8.38(m, 13H), 10.90(br, 1H)

### <Example 47> Preparation of (S)-5-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)picolinic acid

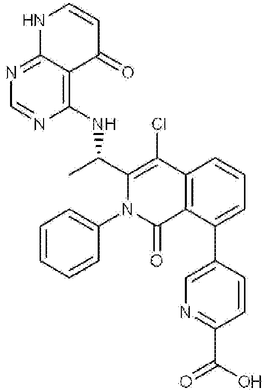

¹H-NMR (400MHz, CD₃OD) δ ppm 1.69(d, 3H), 5.07-5.11(m, 1H), 6.30-8.42(m, 13H), 11.06(br, 1H)

### <Example 48> Preparation of (S)-4-((1-(4-chloro-1-oxo-2-phenyl-8-(quinoxalin-2-yl)-1,2-dihydroisoquinolin-3-yl)ethyl)amino)pyrido[2,3-d]pyrimidin-5(8H)-one

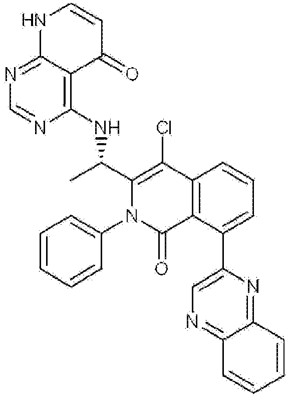

¹H-NMR (400MHz, CDCl₃) δ ppm 1.72(d, 3H), 5.12(m, 1H), 6.50(d, 1H), 7.25(m, 1H), 7.40-8.30(m, 12H), 8.42(d, 1H), 9.25(s, 1H), 11.00(br, 1H)

### <Example 49> Preparation of (S)-4-((1-(4-chloro-8-(2,6-dimethoxypyrimidin-4-yl)-1-oxo-2-phenyl-1,2-dihydroisoquinolin-3-yl)ethyl)amino)pyrido[2,3-d]pyrimidin-5(8H)-one

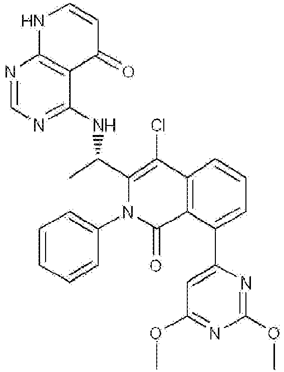

¹H-NMR (400MHz, CD₃OD) δ ppm 1.69(d, 3H), 4.02(d, 6H), 5.09-5.12(m, 1H), 6.40-8.42(m, 11H), 11.01(br, 1H)

### <Example 50> Preparation of (S)-4-((1-(4-chloro-8-(6-hydroxypyrimidin-4-yl)-1-oxo-2-phenyl-1,2-dihydroisoquinolin-3-yl)ethyl)amino)pyrido[2,3-d]pyrimidin-5(8H)-one

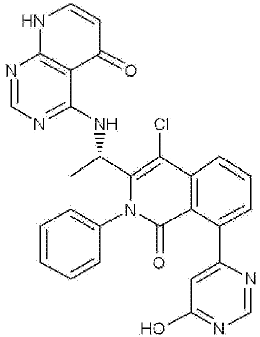

¹H-NMR (400MHz, CD₃OD) δ ppm 1.68(d, 3H), 5.07-5.08(m, 1H), 6.28-8.41(m, 12H), 11.01(br, 1H), 11.74(br, 1H)

### <Example 51> Preparation of (S)-4-((1-(4-chloro-1-oxo-2-phenyl-8-(5-(trifluoromethyl)pyrazin-2-yl)-1,2-dihydroisoquinolin-3-yl) ethyl)amino)pyrido[2,3-d]pyrimidin-5(8H)-one

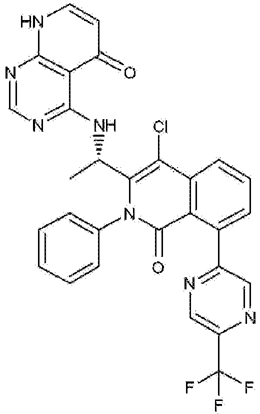

¹H-NMR (400MHz, CD₃OD) δ ppm 1.70(d, 3H), 5.09-5.12(m, 1H), 6.48-9.31(m, 12H), 10.95(br, 1H)

### <Example 52> Preparation of (S)-4-((1-(4-chloro-1-oxo-2-phenyl-8-(5-(trifluoromethyl)pyrimidin-2-yl)-1,2-dihydroisoquinolin-3-yl)ethyl)amino)pyrido[2,3-d]pyrimidin-5(8H)-one

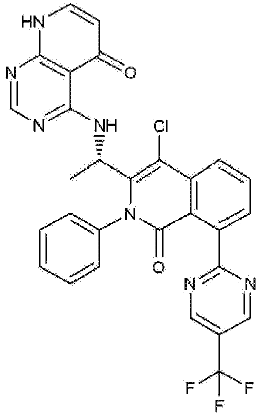

¹H-NMR (400MHz, CD₃OD) δ ppm 1.70(d, 3H), 5.08-5.11(m, 1H), 6.41-9.13(m, 12H), 10.85(br, 1H)

### <Example 53> Preparation of (S)-4-((1-(4-chloro-8-(5-fluoropyrimidin-2-yl)-1-oxo-2-phenyl-1,2-dihydroisoquinolin-3-yl)ethyl)amino)pyrido[2,3-d]pyrimidin-5(8H)-one

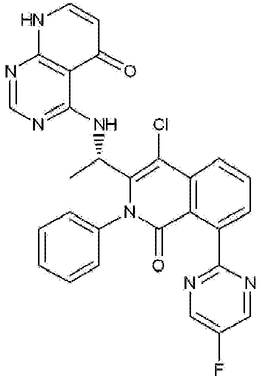

¹H-NMR (400MHz, CD₃OD) δ ppm 1.69(d, 3H), 5.06-5.10(m, 1H), 6.38-8.76(m, 12H), 10.88(br, 1H)

### <Example 54> Preparation of (S)-4-((1-(4-chloro-8-(2-cyclobutylpyrimidin-5-yl)-1-oxo-2-phenyl-1,2-dihydroisoquinolin-3-yl)ethyl)amino)pyrido[2,3-d]pyrimidin-5(8H)-one

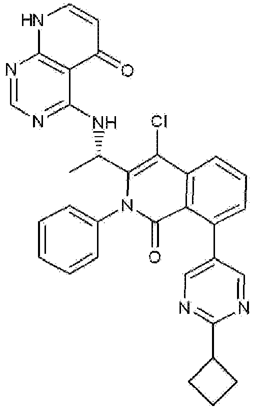

¹H-NMR (400MHz, CDCl₃) δ ppm 1.69(d, 3H), 2.01-2.12(m, 2H), 2.32-2.52(m, 4H), 3.79(q, 1H), 5.04-5.08(m, 1H), 6.32-8.59(m, 13H), 11.03(s, 1H)

### <Example 55> Preparation of (S)-4-((1-(4-chloro-8-(2-(methoxymethyl)pyrimidin-5-yl)-1-oxo-2-phenyl-1,2-dihydroisoquinolin-3-yl)ethyl)amino)pyrido[2,3-d]pyrimidin-5(8H)-one

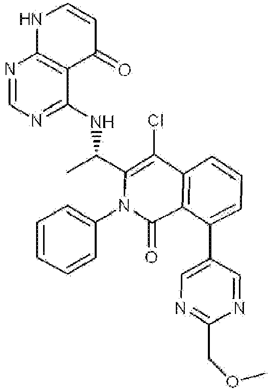

¹H-NMR (400MHz, CDCl₃) δ ppm 1.69(d, 3H), 3.54(s, 3H), 4.69(s, 2H), 5.01-5.05(m, 1H), 6.31-9.68(m, 13H), 11.00(s, 1H), 11.29(s, 1H)

### <Example 56> Preparation of (S)-4-((1-(8-(2-aminopyrimidin-5-yl)-4-chloro-1-oxo-2-phenyl-1,2-dihydroisoquinolin-3-yl)ethyl)amino)pyrido[2,3-d]pyrimidin-5(8H)-one

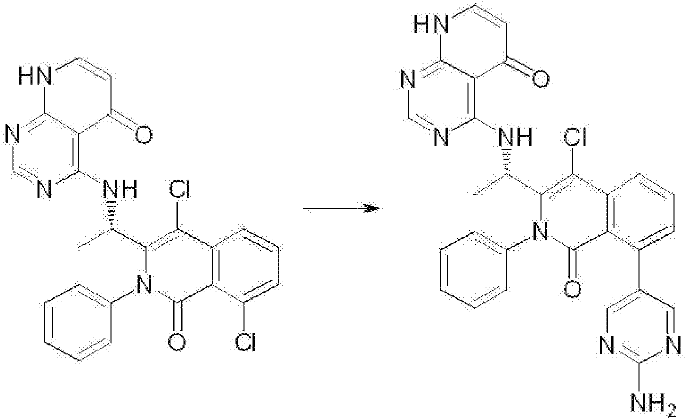

The 50 mg of (S)-4-((1-(4,8-dichloro-1-oxo-2-phenyl-1,2-**dihydroisoquinolin-3-yl)**ethyl)amino)pyrido[2,3-d]pyrimidin-5(8H)-one prepared in above Preparation Example 1, 22 mg of 2-aminopyrimidin-5-boronic acid, 0.15 mL of 2M aqueous potassium carbonate solution, and 7 mg of bis(triphenylphosphin)palladium chloride were added to 2 mL of dioxane and irradiated with microwaves at 170°C for 30 minutes by using a microwave reactor.

Upon completion of the reaction, the resulting mixture was cooled down to room temperature, after which a saturated aqueous solution of sodium chloride was added, followed by extraction with ethyl acetate two to three times. An organic layer was dried over anhydrous sodium sulfate, and concentrated under reduced pressure, and then the resulting residues were purified by Prep-LC. The resulting product was vacuum-dried for three hours to obtain 10 mg of (S)-4-((1-(8-(2-aminopyrimidin-5-yl)-4-chloro-1-oxo-2-phenyl-1,2-dihydroisoquinolin-3-yl)ethyl)amino)pyrido[2,3-d]pyrimidin-5(8H)-one.

¹H-NMR (400MHz, CDCl₃) δ ppm 1.67(d, 3H), 5.05-5.08 (m, 1H), 6.27-8.28(m, 13H), 10.80(s, 1H)

### <Examples 57 to 247>

In Examples 57 to 247, the compound of each Example was prepared by substantially the same preparation method as described in above Example 56, except for using an appropriate reactant in consideration of the structure of the corresponding compound.

### <Example 57> Preparation of 4-(((1S)-1-(4-chloro-8-(4-(1-hydroxyethyl)phenyl)-1-oxo-2-phenyl-1,2-dihydroisoquinolin-3-yl)ethyl)amino)pyrido[2,3-d]pyrimidin-5(8H)-one

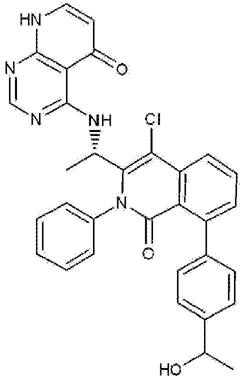

¹H-NMR (400MHz, CDCl₃) δ ppm 1.47(d, 3H), 1.67(d, 3H), 4.83-4.88(m, 1H), 5.01-5.07(m, 1H), 6.25-8.24(m, 14H), 11.02(d, 1H)

### <Example 58> Preparation of (S)-4-((1-(4-chloro-8-(6-hydroxy-5-methylpyridin-3-yl)-1-oxo-2-phenyl-1,2-dihydroisoquinolin-3-yl)ethyl)amino)pyrido[2,3-d]pyrimidin-5(8H)-one

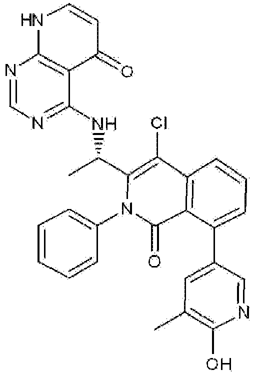

¹H-NMR (400MHz, CD₃OD) δ ppm 1.69(d, 3H), 2.10(s, 3H), 5.05-5.12(m, 1H), 6.30-8.27(m, 13H), 10.89(d, 1H)

### <Example 59> Preparation of (S)-4-((1-(4-chloro-8-(4-cyclopentylpyrimidin-5-yl)-1-oxo-2-phenyl-1,2-dihydroisoquinolin-3-yl)ethyl)amino)pyrido[2,3-d]pyrimidin-5(8H)-one

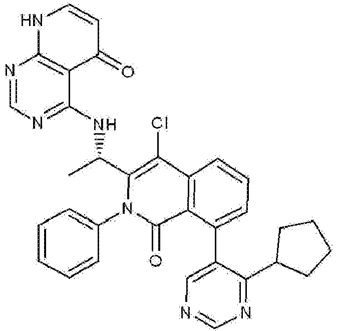

¹H-NMR (400MHz, CDCl₃) δ ppm 1.47-1.96(m, 15H), 2.75-2.84(m, 1H), 4.99-5.06(m, 1H), 6.31-9.03(m, 13H), 10.84(s, 1H), 10.99(d, 1H)

### <Example 60> Preparation of (S)-4-((1-(8-(benzo[d][1,3]dioxol-5-yl)-4-chloro-1-oxo-2-phenyl-1,2-dihydroisoquinolin-3-yl)ethyl)amino)pyrido[2,3-d]pyrimidin-5(8H)-one

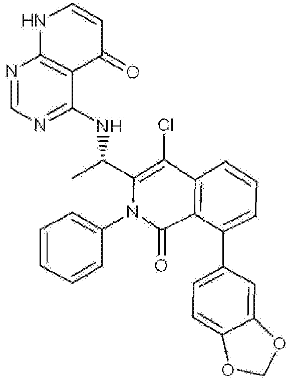

¹H-NMR (400MHz, CDCl₃) δ ppm 1.67(d, 3H), 5.01-5.08(m, 1H), 5.87-8.25(m, 16H), 11.05(d, 1H), 11.92(s, 1H)

### <Example 61> Preparation of (S)-4-((1-(4-chloro-8-(6-methoxy-5-nitropyridin-3-yl)-1-oxo-2-phenyl-1,2-dihydroisoquinolin-3-yl)ethyl)amino)pyrido[2,3-d]pyrimidin-5(8H)-one

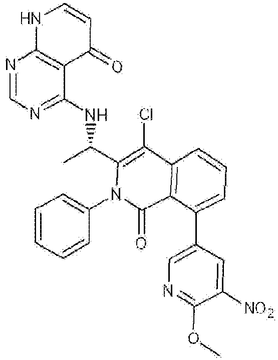

¹H-NMR (400MHz, CDCl₃) δ ppm 1.69(d, 3H), 4.07(s, 3H), 5.01-5.08(m, 1H), 6.32-8.28(m, 13H), 11.06(d, 1H), 12.00(s, 1H)

### <Example 62> Preparation of (S)-4-((1-(4-chloro-1-oxo-2,8-diphenyl-1,2-dihydroisoquinolin-3-yl)ethyl)amino)pyrido[2,3-d]pyrimidin-5(8H)-one

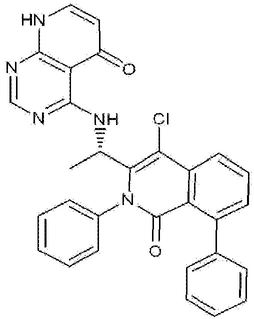

¹H-NMR (400MHz, DMSO-d₆) δ ppm 1.59(d, 3H), 4.85(m, 1H), 6.12(d, 1H), 7.65-7.15(m, 11H), 7.77(d, 1H), 7.84(t, 1H), 8.03(d, 1H), 8.31(s, 1H), 10.95(s, 1H)

### <Example 63> Preparation of (S)-4-((1-(4-chloro-8-(4-fluorophenyl)-1-oxo-2-phenyl-1,2-dihydroisoquinolin-3-yl)ethyl)amino)pyrido[2,3-d]pyrimidin-5(8H)-one

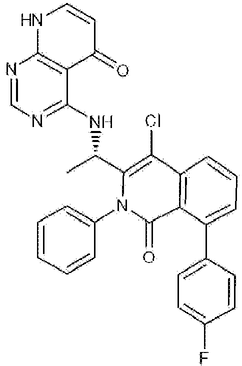

¹H-NMR (400MHz, DMSO-d₆) δ ppm 1.59(d, 3H), 4.85(m, 1H), 6.12(d, 1H), 7.65-7.15(m, 10H), 7.77(d, 1H), 7.84(t, 1H), 8.03(d, 1H), 8.31(s, 1H), 10.95(s, 1H)

### <Example 64> Preparation of (S)-4-((1-(4-chloro-8-(4-methoxyphenyl)-1-oxo-2-phenyl-1,2-dihydroisoquinolin-3-yl)ethyl)amino)pyrido[2,3-d]pyrimidin-5(8H)-one

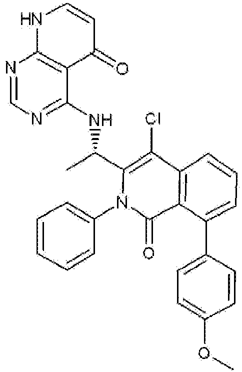

¹H-NMR (400MHz, DMSO-d₆) δ ppm 1.59(d, 3H), 4.85(m, 1H), 6.12(d, 1H), 6.83(d, 2H), 7.10(d, 2H), 7.65-7.28(m, 6H), 7.77(d, 1H), 7.82(t, 1H), 8.00(d, 1H), 8.31(s, 1H), 10.97(s, 1H)

### <Example 65> Preparation of (S)-4-((1-(8-chloro-4-cyclopropyl-1-oxo-2-phenyl-1,2-dihydroisoquinolin-3-yl)ethyl)amino)pyrido[2,3-d]pyrimidin-5(8H)-one

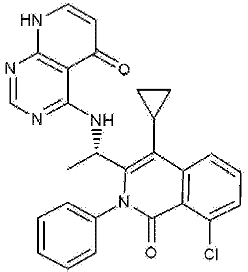

¹H-NMR (400MHz, CDCl₃) δ ppm 0.99(t, 3H), 1.66(d, 3H), 1.87(m, 1H), 2.13(m, 1H), 5.09(m, 1H), 6.29(d, 1H), 7.20-7.80(m, 8H), 8.06(d, 1H), 8.27(s, 1H), 10.95(br, 1H)

### <Example 66> Preparation of (S)-4-((1-(4-chloro-8-cyclopropyl-1-oxo-2-phenyl-1,2-dihydroisoquinolin-3-yl)ethyl)amino)pyrido[2,3-d]pyrimidin-5(8H)-one

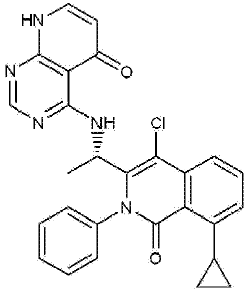

¹H-NMR (400MHz, CDCl₃) δ ppm 0.99(t, 3H), 1.68(d, 3H), 1.87(m, 1H), 2.15(m, 1H), 5.03(m, 1H), 6.30(d, 1H), 7.22(m, 1H), 7.30-7.80(m, 7H), 8.05(d, 1H), 8.30(s, 1H), 10.95(br, 1H)

### <Example 67> Preparation of (S)-4-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-3-methylbenzonitrile

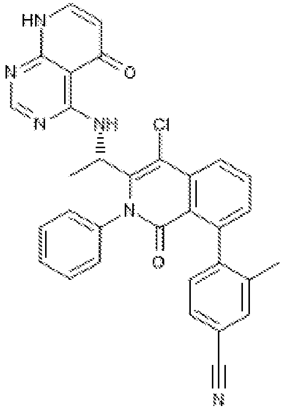

¹H-NMR (400MHz, CDCl₃) δ ppm 1.67(d, 3H), 1.98(d, 3H), 4.95-5.15(m, 1H), 6.32(d, 1H), 7.05-7.20(m, 3H), 7.30-7.80(m, 8H), 8.15(m, 1H), 8.22(d, 1H), 11.10(br, 1H), 11.90(br, 1H)

### <Example 68> Preparation of (S)-4-((1-(4-chloro-1-oxo-2-phenyl-8-(4-(trifluoromethyl)phenyl)-1,2-dihydroisoquinolin-3-yl)ethyl)amino)pyrido[2,3-d]pyrimidin-5(8H)-one

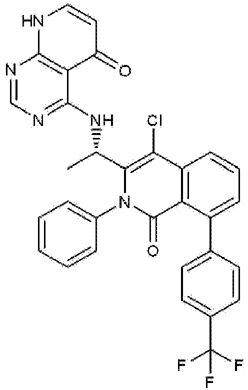

¹H-NMR (400MHz, CDCl₃) δ ppm 1.68(d, 3H), 5.04(m, 1H), 6.31(d, 1H), 7.10-7.80(m, 12H), 8.12(d, 1H), 8.24(s, 1H), 11.06(br, 1H)

### <Example 69> Preparation of (S)-1,4'-dichloro-3'-(1-((5-oxo-5,8-dihydropyrido[2,3-d]pyrimidin-4-yl)amino)ethyl)-2'-phenyl-(4,8'-biisoquinolin)-1'(2'H)-one

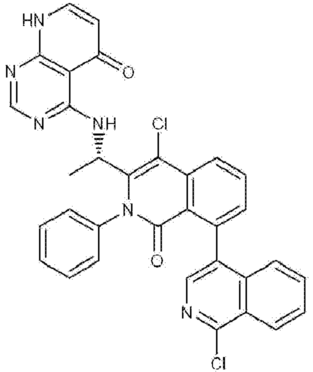

¹H-NMR (400MHz, CDCl₃) δ ppm 1.71(d, 3H), 5.05-5.090(m, 1H), 6.45-8.54(m, 16H), 10.99(d, 1H)

### <Example 70> Preparation of (S,E)-4-((1-(4-chloro-1-oxo-2-phenyl-8-styryl-1,2-dihydroisoquinolin-3-yl)ethyl)amino)pyrido[2,3-d]pyrimidin-5(8H)-one

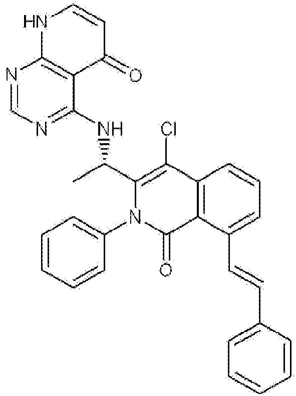

¹H-NMR (400MHz, CDCl₃) δ ppm 1.66(d, 3H), 5.03(m, 1H), 6.31(d, 1H), 6.82(d, 1H), 7.15-7.30(m, 4H), 7.40-7.80(m, 9H), 8.02(m, 1H), 8.27(s, 1H), 8.40(d, 1H), 10.70(br, 1H), 11.00(br, 1H)

### <Example 71> Preparation of (S)-4-((1-(4-chloro-8-(3,4-dichlorophenyl)-1-oxo-2-phenyl-1,2-dihydroisoquinolin-3-yl)ethyl)amino)pyrido[2,3-d]pyrimidin-5(8H)-one

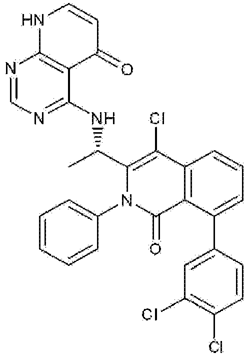

¹H-NMR (400MHz, CDC13) δ ppm 1.66(d, 3H), 5.04(m, 1H), 6.31(d, 1H), 7.06(m, 1H), 7.15(m, 1H), 7.25(m, 1H), 7.30-7.65(m, 8H), 8.13(d, 1H), 8.26(s, 1H), 11.02(br, 1.6H)

### <Example 72> Preparation of (S)-4-((1-(4-chloro-8-(4-(dimethylamino)phenyl)-1-oxo-2-phenyl-1,2-dihydroisoquinolin-3-yl)ethyl)amino)pyrido[2,3-d]pyrimidin-5(8H)-one

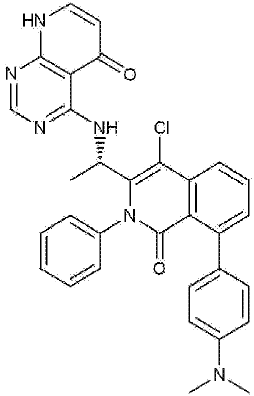

¹H-NMR (400MHz, CDCl₃) δ ppm 1.67(d, 3H), 2.90(s, 6H), 5.01-5.09(m, 1H), 6.28-8.25(m, 15H), 10.73(s, 1H), 10.99(d, 1H)

### <Example 73> Preparation of (S)-4-((1-(4-chloro-8-(1H-indol-5-yl)-1-oxo-2-phenyl-1,2-dihydroisoquinolin-3-yl)ethyl)amino)pyrido[2,3-d]pyrimidin-5(8H)-one

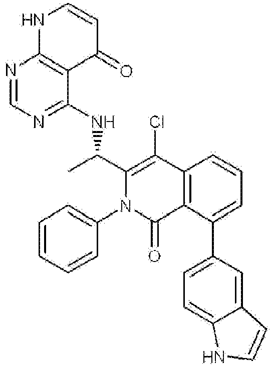

¹H-NMR (400MHz, CDCl₃) δ ppm 1.68(d, 3H), 5.02-5.09(m, 1H), 6.26-8.26(m, 16H), 11.00(d, 1H)

### <Example 74> Preparation of (S)-4-((1-(4-chloro-8-(3-methyl-1H-indol-5-yl)-1-oxo-2-phenyl-1,2-dihydroisoquinolin-3-yl)ethyl)amino)pyrido[2,3-d]pyrimidin-5(8H)-one

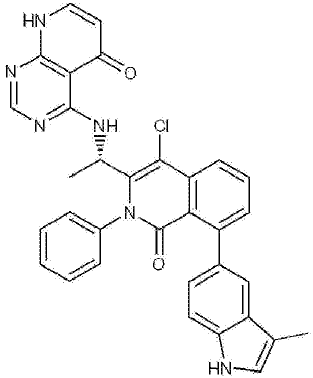

¹H-NMR (400MHz, CDCl₃) δ ppm 1.68(d, 3H), 2.23(s, 3H), 5.02-5.09(m, 1H), 6.22-8.24(m, 15H), 10.84(s, 1H), 10.99(d, 1H)

### <Example 75> Preparation of (S)-4-((1-(4-chloro-8-(4-morpholinophenyl)-1-oxo-2-phenyl-1,2-dihydroisoquinolin-3-yl)ethyl)amino)pyrido[2,3-d]pyrimidin-5(8H)-one

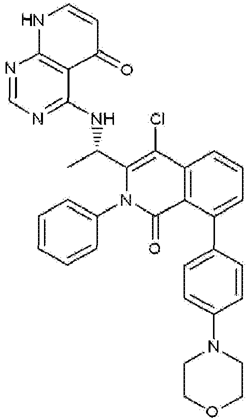

¹H-NMR (400MHz, CDCl₃) δ ppm 1.67(d, 3H), 3.14-3.83(m, 8H), 5.01-5.08(m, 1H), 6.29-8.24(m, 15H), 10.06(s, 1H), 10.95(d, 1H)

### <Example 76> Preparation of (S)-4-((1-(4-chloro-8-(4-isopropylpiperidin-1-yl)-1-oxo-2-phenyl-1,2-dihydroisoquinolin-3-yl)ethyl)amino)pyrido[2,3-d]pyrimidin-5(8H)-one

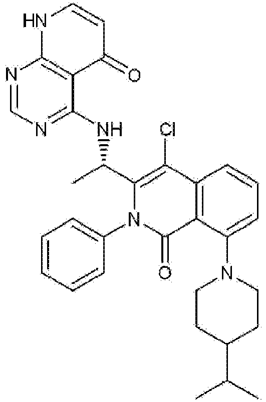

¹H-NMR (400MHz, CD₃OD) δ ppm 0.82(s, 3H), 0.83(s, 3H), 1.15(br, 1H), 1.37-1.38(m, 2H), 1.65(d, 3H), 1.66(br, 1H), 2.63(br, 2H), 3.42-3.46(m, 4H), 5.01-5.05(m, 1H), 6.27(d, 1H), 7.03-7.68(m, 9H), 8.21(s, 1H), 11.01-11.02(m, 1H)

### <Example 77> Preparation of tert-butyl (S)-(1-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)piperidin-4-yl)carbamate

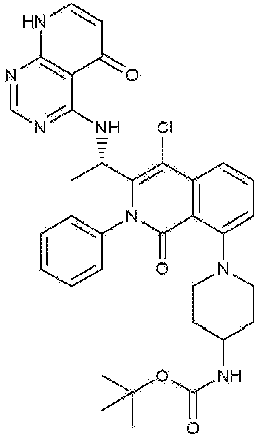

¹H-NMR (400MHz, CD₃OD) δ ppm 1.43(s, 9H), 1.65(d, 3H), 1.68-1.73(m, 2H), 1.96(br, 2H), 2.96(br, 2H), 3.37(br, 2H), 3.55(br, 1H), 5.01-5.05(m, 1H), 6.28(d, 1H), 7.02-7.65(m, 9H), 8.22(s, 1H), 10.94-10.95(m, 1H)

### <Example 78> Preparation of (S)-4-((1-(4-chloro-8-(4-(2-hydroxyphenyl)piperazin-1-yl)-1-oxo-2-phenyl-1,2-dihydroisoquinolin-3-yl)ethyl)amino)pyrido[2,3-d]pyrimidin-5(8H)-one

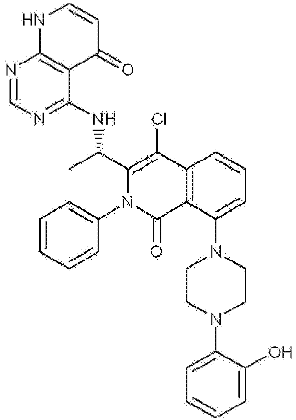

¹H-NMR (400MHz, CD₃OD) δ ppm 1.65(d, 3H), 3.03(br, 4H), 3.27(br, 4H), 5.02-5.06(m, 1H), 6.29(d, 1H), 6.82-7.70(m, 13H), 8.23(s, 1H), 10.96-10.98(m, 1H)

### <Example 79> Preparation of (S)-3-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)benzaldehyde

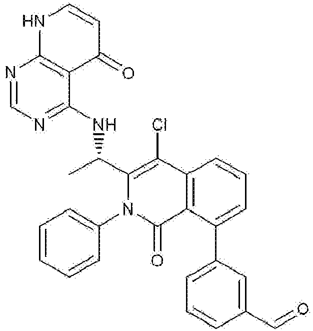

¹H-NMR (400MHz, CDCl₃) δ ppm 1.68(d, 3H), 5.00-5.07 (m, 1H), 6.30-8.27(m, 15H), 9.98(s, 1H), 10.95(d, 1H)

### <Example 80> Preparation of (S)-4-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)benzamide

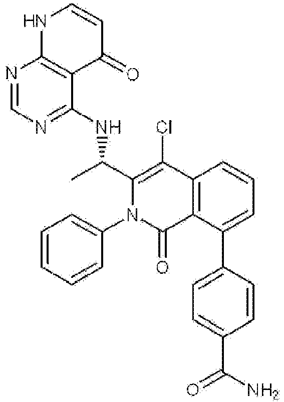

¹H-NMR (400MHz, CDCl₃) δ ppm 1.68(d, 3H), 5.01-5.08(m, 1H), 6.30-8.23(m, 15H), 10.96(d, 1H)

### <Example 81> Preparation of (S)-4-(1-oxo-3-(1-((5-oxo-5,8-dihydropyrido[2,3-d]pyrimidin-4-yl)amino)ethyl)-2-phenyl-1,2-dihydroisoquinolin-8-yl)benzamide

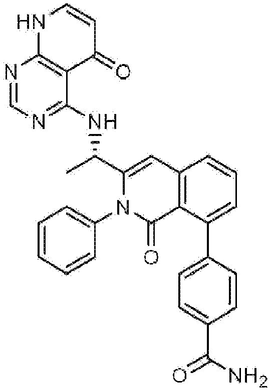

¹H-NMR (400MHz, CDCl₃) δ ppm 1.49(d, 3H), 4.90-4.97 (m, 1H), 6.32-8.19(m, 16H), 10.46(d, 1H)

### <Example 82> Preparation of (S)-4-((1-(4-chloro-8-(1,5-dimethyl-1H-pyrazol-4-yl)-1-oxo-2-phenyl-1,2-dihydroisoquinolin-3-yl)ethyl)amino)pyrido[2,3-d]pyrimidin-5(8H)-one

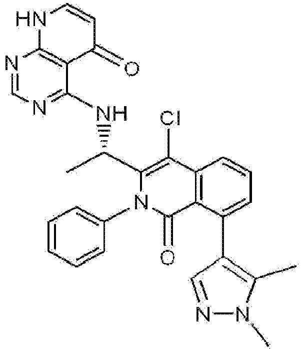

¹H-NMR (400MHz, CDCl₃) δ ppm 1.66(d, 3H), 2.05(s, 3H), 3.75(s, 3H), 5.06(m, 1H), 6.30(d, 1H), 7.10-7.80(m, 9H), 8.04(d, 1H), 8.23(s, 1H), 10.95(br, 1H)

### <Example 83> Preparation of (S)-4-((1-(4-chloro-8-(1-(2-(dimethylamino)ethyl)-lH-pyrazol-4-yl)-1-oxo-2-phenyl-1,2-dihydroisoquinolin-3-yl)ethyl)amino)pyrido[2,3-d]pyrimidin-5(8H)-one

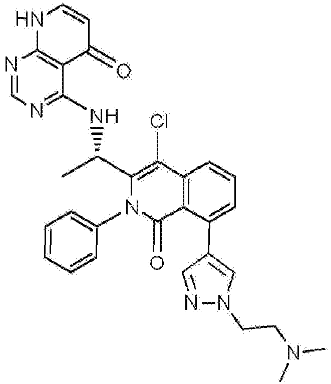

¹H-NMR (400MHz, CDCl₃) δ ppm 1.66(d, 3H), 2.35(s, 6H), 2.98(br, 2H), 4.32(br, 2H), 5.00(m, 1H), 6.30(d, 1H), 7.15-7.85(m, 10H), 8.01(d, 1H), 8.23(s, 1H), 10.96(br, 1H)

### <Example 84> Preparation of (S)-4-((1-(4-chloro-8-(4-chlorophenyl)-1-oxo-2-phenyl-1,2-dihydroisoquinolin-3-yl)ethyl)amino)pyrido[2,3-d]pyrimidin-5(8H)-one

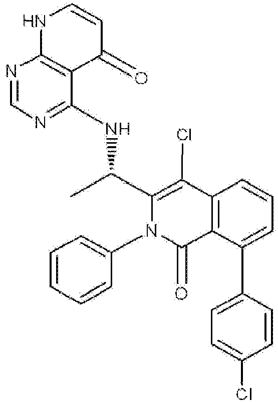

¹H-NMR (400MHz, CDCl₃) δ ppm 1.66(d, 3H), 5.03(m, 1H), 6.30(d, 1H), 7.10-7.80(m, 12H), 8.11(d, 1H), 8.24(s, 1H), 11.05(br, 1H), 1.30(br, 1H)

### <Example 85> Preparation of (S)-4-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)benzaldehyde

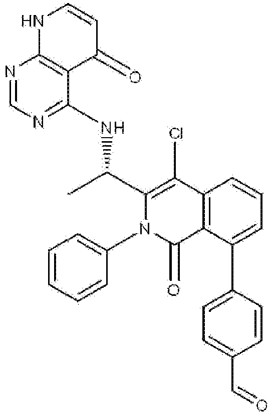

¹H-NMR (400MHz, CDCl₃) δ ppm 1.68(d, 3H), 5.01-5.08(m, 1H), 6.31-8.25(m, 15H), 9.96(s, 1H), 11.06(d, 1H)

### <Example 86> Preparation of (S)-4-((1-(4-chloro-8-(6-(dimethylamino)pyridin-3-yl)-1-oxo-2-phenyl-1,2-dihydroisoquinolin-3-yl)ethyl)amino)pyrido[2,3-d]pyrimidin-5(8H)-one

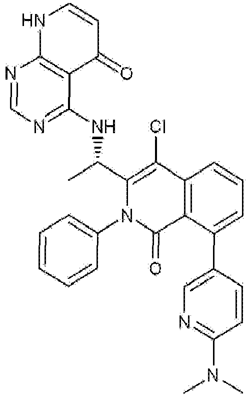

¹H-NMR (400MHz, CDCl₃) δ ppm 1.67(d, 3H), 3.01(s, 6H), 5.00-5.07(m, 1H), 6.27-8.24(m, 14H), 11.03(d, 1H), 11.91(s, 1H)

### <Example 87> Preparation of (S)-4-((1-(4-chloro-1-oxo-2-phenyl-8-(4-(pyrrolidin-1-yl)phenyl)-1,2-dihydroisoquinolin-3-yl)ethyl)amino)pyrido[2,3-d]pyrimidin-5(8H)-one

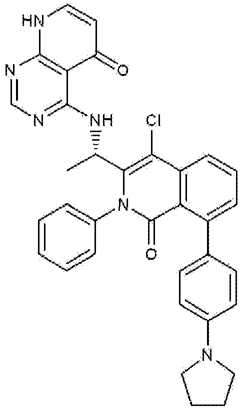

¹H-NMR (400MHz, CDCl₃) δ ppm 1.67(d, 3H), 1.90-1.94(m, 4H), 3.20-3.23(m, 4H), 5.02-5.09(m, 1H), 6.25-8.24(m, 15H), 11.02(d, 1H), 11.25(s, 1H)

### <Example 88> Preparation of (S)-4-((1-(4-chloro-1-oxo-2-phenyl-8-(4-(piperidin-1-yl)phenyl)-1,2-dihydroisoquinolin-3-yl)ethyl)amino)pyrido[2,3-d]pyrimidin-5(8H)-one

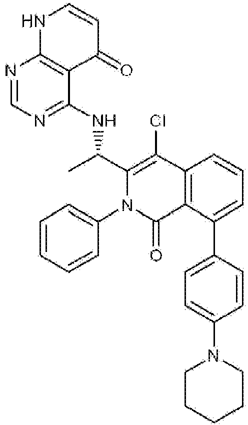

¹H-NMR (400MHz, CDCl₃) δ ppm 1.54-1.71(m, 9H), 3.10(t, 4H), 5.01-5.08(m, 1H), 6.27-8.24(m, 15H), 11.02(d, 1H), 11.28(s, 1H)

### <Example 89> Preparation of (S)-4-((1-(4-chloro-8-(1H-indol-4-yl)-1-oxo-2-phenyl-1,2-dihydroisoquinolin-3-yl)ethyl)amino)pyrido[2,3-d]pyrimidin-5(8H)-one

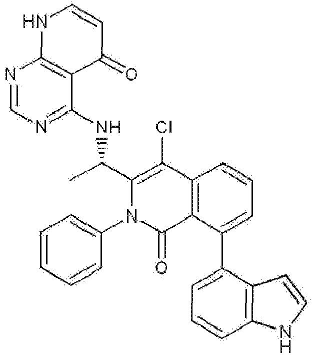

¹H-NMR (400MHz, CDCl₃) δ ppm 1.69(d, 3H), 5.02-5.09(m, 1H), 5.92-8.25(m, 17H), 11.02(d, 1H), 11.26(s, 1H)

### <Example 90> Preparation of (S)-4-((1-(8-(4-(1-methylpiperidin-4-yl)phenyl)-1-oxo-2-phenyl-1,2-dihydroisoquinolin-3-yl)ethyl)amino)pyrido[2,3-d]pyrimidin-5(8H)-one

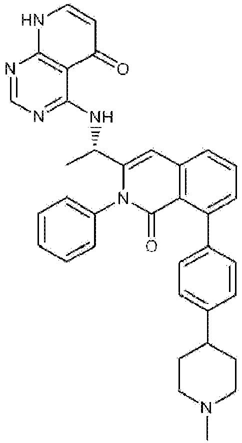

¹H-NMR (400MHz, CDCl₃) δ ppm 1.48(d, 3H), 1.74-2.99(m, 12H), 4.88-4.95(m, 1H), 6.32-8.20(m, 16H), 11.47(d, 1H)

### <Example 91> Preparation of (S)-2-(3-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)phenyl)acetonitrile

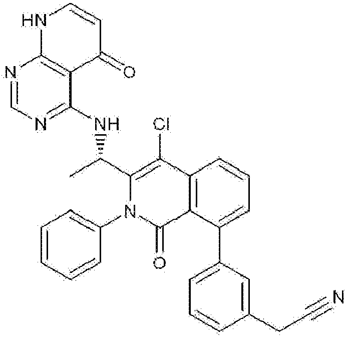

¹H-NMR (400MHz, CDCl₃) δ ppm 1.68(d, 3H), 3.74(s, 2H), 4.99-5.06(m, 1H), 6.30-8.28(m, 15H), 11.03(d, 1H), 11.39(s, 1H)

### <Example 92> Preparation of (S)-4-((1-(8-(4-(5-amino-1,3,4-thiadiazol-2-yl)phenyl)-4-chloro-1-oxo-2-phenyl-1,2-dihydroisoquinolin-3-yl)ethyl)amino)pyrido[2,3-d]pyrimidin-5(8H)-one

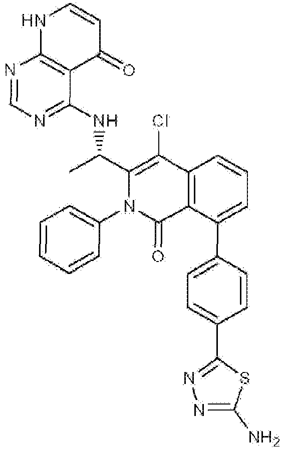

¹H-NMR (400MHz, CDCl₃) δ ppm 1.69(d, 3H), 3.37(s, 2H), 5.03-5.10(m, 1H), 6.31-8.24(m, 15H), 10.94(d, 1H)

### <Example 93> Preparation of (S)-4-((1-(4-chloro-1-oxo-2-phenyl-8-(4-(thiophen-2-yl)phenyl)-1,2-dihydroisoquinolin-3-yl)ethyl)amino)pyrido[2,3-d]pyrimidin-5(8H)-one

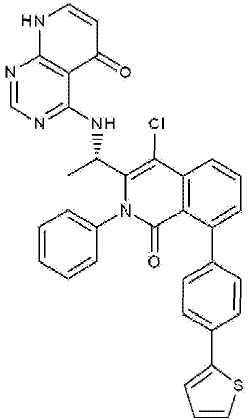

¹H-NMR (400MHz, CDCl₃) δ ppm 1.68(d, 3H), 5.00-5.07 (m, 1H), 6.29-8.24(m, 18H), 11.04(d, 1H), 11.20(s, 1H)

### <Example 94> Preparation of (S)-4-((1-(8-(4-aminophenyl)-4-chloro-1-oxo-2-phenyl-1,2-dihydroisoquinolin-3-yl)ethyl)amino)pyrido[2,3-d]pyrimidin-5(8H)-one

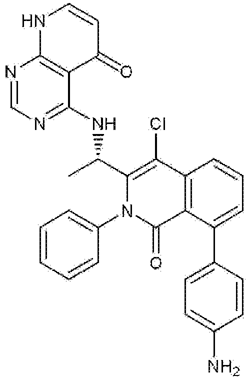

¹H-NMR (400MHz, CDCl₃) δ ppm 1.66(d, 3H), 5.00-5.07(m, 1H), 6.25-8.24(m, 15H), 11.03(d, 1H), 11.63(s, 1H)

### <Example 95> Preparation of (S)-4-((1-(4-chloro-8-(4-mercaptophenyl)-1-oxo-2-phenyl-1,2-dihydroisoquinolin-3-yl)ethyl)amino)pyrido[2,3-d]pyrimidin-5(8H)-one

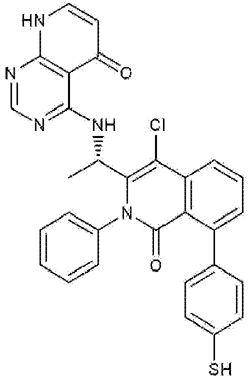

¹H-NMR (400MHz, CDCl₃) δ ppm 1.69(d, 3H), 5.01-5.08(m, 1H), 6.31-8.28(m, 15H), 11.00(d, 1H), 12.16(s, 1H)

### <Example 96> Preparation of (S)-4-((1-(4-chloro-8-(1-cyclopropyl-1H-pyrazol-4-yl)-1-oxo-2-phenyl-1,2-dihydroisoquinolin-3-yl)ethyl)amino)pyrido[2,3-d]pyrimidin-5(8H)-one

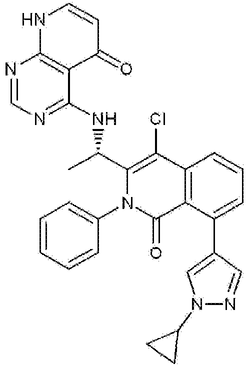

¹H-NMR (400MHz, CDCl₃) δ ppm 0.94(m, 2H), 1.10(m, 2H), 1.66(d, 3H), 3.55(m, 1H), 5.05(m, 1H), 6.27(d, 1H), 7.19(d, 1H), 7.40(d, 1H), 7.45-7.70(m, 8H), 8.02(d, 1H), 8.25(s, 1H), 11.00(br, 1H)

### <Example 97> Preparation of (S)-4-((1-(4-chloro-8-(3-chlorophenyl)-1-oxo-2-phenyl-1,2-dihydroisoquinolin-3-yl)ethyl)amino)pyrido[2,3-d]pyrimidin-5(8H)-one

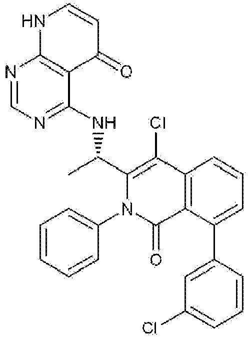

¹H-NMR (400MHz, CDCl₃) δ ppm 1.66(d, 3H), 5.04(m, 1H), 6.30(d, 1H), 7.10-7.80(m, 12H), 8.10(d, 1H), 8.26(s, 1H), 9.68(br, 1H), 10.95(br, 1H)

### <Example 98> Preparation of (S)-4-((1-(4-chloro-8-(5-methylfuran-2-yl)-1-oxo-2-phenyl-1,2-dihydroisoquinolin-3-yl)ethyl)amino)pyrido[2,3-d]pyrimidin-5(8H)-one

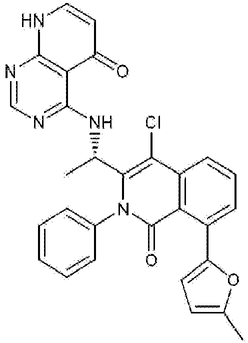

¹H-NMR (400MHz, CDCl₃) δ ppm 1.66(d, 3H), 2.28(s, 3H), 5.01-5.08(m, 1H), 5.99-8.26(m, 13H), 11.02(d, 1H), 11.63(s, 1H)

### <Example 99> Preparation of (S)-4-((1-(4-chloro-8-(4-chloro-3-methoxyphenyl)-1-oxo-2-phenyl-1,2-dihydroisoquinolin-3-yl)ethyl)amino)pyrido[2,3-d]pyrimidin-5(8H)-one

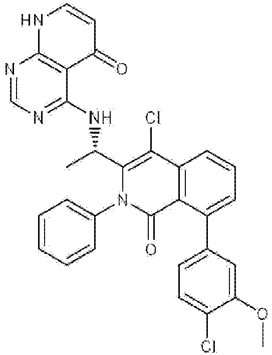

¹H-NMR (400MHz, CDCl₃) δ ppm 1.68(d, 3H), 3.81(s, 3H), 5.03-5.10(m, 1H), 6.30-8.24(m, 14H), 11.07(d, 1H), 11.66(s, 1H)

### <Example 100> Preparation of (S)-4-((1-(8-(4-chloro-3-methoxyphenyl)-1-oxo-2-phenyl-1,2-dihydroisoquinolin-3-yl)ethyl)amino)pyrido[2,3-d]pyrimidin-5(8H)-one

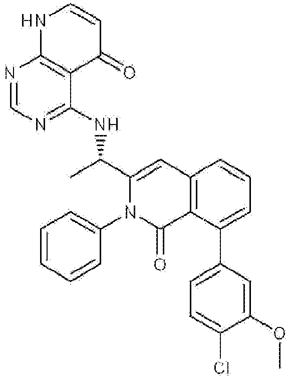

¹H-NMR (400MHz, CDCl₃) δ ppm 1.49(d, 3H), 3.84(s, 3H), 4.92-4.99(m, 1H), 6.33-8.19(m, 15H), 10.11(s, 1H), 10.51(d, 1H)

### <Example 101> Preparation of (S)-4-((1-(4-chloro-8-(6-isopropoxypyridin-3-yl)-1-oxo-2-phenyl-1,2-dihydroisoquinolin-3-yl)ethyl)amino)pyrido[2,3-d]pyrimidin-5(8H)-one

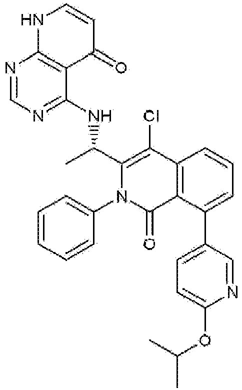

¹H-NMR (400MHz, CDCl₃) δ ppm 1.30(d, 6H), 1.68(d, 3H), 5.00-5.07(m, 1H), 5.18-5.29(m, 1H), 6.29-8.25(m, 14H), 11.06(d, 1H)

### <Example 102> Preparation of (S)-4-((1-(4-chloro-1-oxo-2-phenyl-8-(pyrimidin-5-yl)-1,2-dihydroisoquinolin-3-yl)ethyl)amino)pyrido[2,3-d]pyrimidin-5(8H)-one

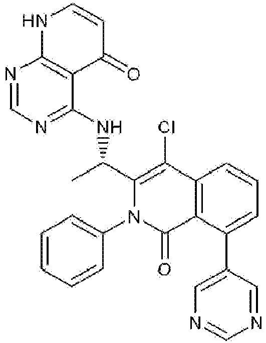

¹H-NMR (400MHz, CDCl₃) δ ppm 1.69(d, 3H), 5.02-5.09(m, 1H), 6.31-9.09(m, 14H), 11.02(d, 1H)

### <Example 103> Preparation of (S)-4-((1-(4-chloro-1-oxo-2-phenyl-8-(3,4,5-trimethoxyphenyl)-1,2-dihydroisoquinolin-3-yl)ethyl)amino)pyrido[2,3-d]pyrimidin-5(8H)-one

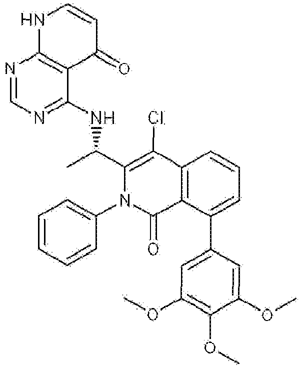

¹H-NMR (400MHz, CDCl₃) δ ppm 1.68(d, 3H), 3.77(s, 9H), 5.05-5.12(m, 1H), 6.29-8.24(m, 13H), 11.08(d, 1H), 11.73(s, 1H)

### <Example 104> Preparation of (S)-4-((1-(4-chloro-8-(3-chloro-5-fluorophenyl)-1-oxo-2-phenyl-1,2-dihydroisoquinolin-3-yl)ethyl)amino)pyrido[2,3-d]pyrimidin-5(8H)-one

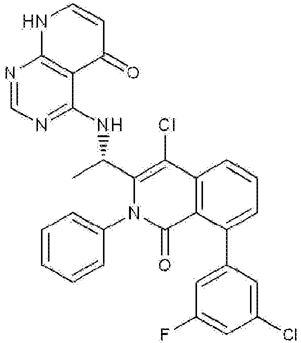

¹H-NMR (400MHz, CDCl₃) δ ppm 1.68(d, 3H), 5.05(m, 1H), 6.30(d, 1H), 6.85(d, 1H), 6.96(d, 1H), 7.00(s, 1H), 7.10-7.85(m, 8H), 8.13(d, 1H), 8.27(s, 1H), 11.10(br, 1H)

### <Example 105> Preparation of (S)-4-((1-(4-chloro-1-oxo-2-phenyl-8-(4-(pyridin-4-yl)phenyl)-1,2-dihydroisoquinolin-3-yl)ethyl)amino)pyrido[2,3-d]pyrimidin-5(8H)-one

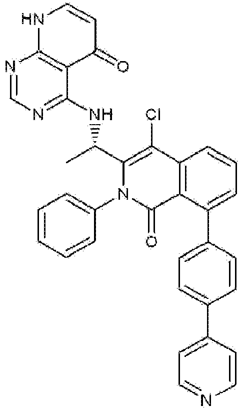

¹H-NMR (400MHz, CDCl₃) δ ppm 1.68(d, 3H), 5.05(m, 1H), 6.31(d, 1H), 7.10-7.80(m, 14H), 8.13(d, 1H), 8.25(s, 1H), 8.63(d, 2H), 11.05(br, 1H)

### <Example 106> Preparation of (S)-4-((1-(4-chloro-8-(2-chloro-3-fluorophenyl)-1-oxo-2-phenyl-1,2-dihydroisoquinolin-3-yl)ethyl)amino)pyrido[2,3-d]pyrimidin-5(8H)-one

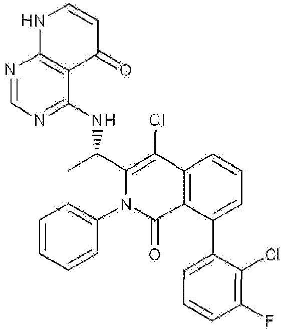

¹H-NMR (400MHz, CDCl₃) δ ppm 1.68(t, 3H), 5.03(m, 1H), 6.31(d, 1H), 7.00-7.80(m, 11H), 8.13(d, 1H), 8.23(s, 1H), 11.05(br, 1H)

### <Example 107> Preparation of (S)-4-((1-(4-chloro-8-(2-chloro-5-fluorophenyl)-1-oxo-2-phenyl-1,2-dihydroisoquinolin-3-yl)ethyl)amino)pyrido[2,3-d]pyrimidin-5(8H)-one

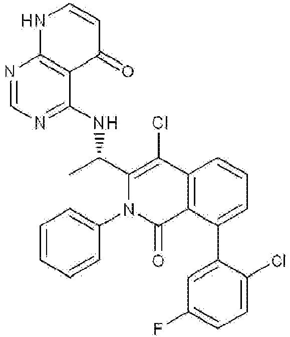

¹H-NMR (400MHz, CDCl₃) δ ppm 1.68(t, 3H), 5.03(m, 1H), 6.31(d, 1H), 6.90(m, 2H), 7.10-7.80(m, 9H), 8.13(d, 1H), 8.24(d, 1H), 11.05(br, 1H), 11.75(br, 1H)

### <Example 108> Preparation of (S)-4-((1-(4-chloro-8-(3-fluoro-5-hydroxyphenyl)-1-oxo-2-phenyl-1,2-dihydroisoquinolin-3-yl)ethyl)amino)pyrido[2,3-d]pyrimidin-5(8H)-one

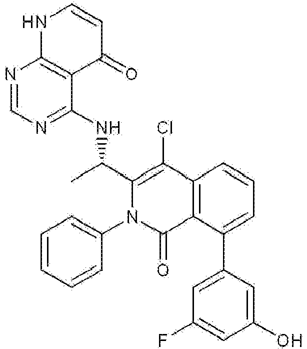

¹H-NMR (400MHz, CD₃OD) δ ppm 1.64(d, 3H), 5.03(m, 1H), 6.36(d, 1H), 6.4(m, 3H), 7.20-7.35(m, 2H), 7.40-7.80(m, 6H), 8.06(d, 1H), 8.24(s, 1H), 10.80(br, 1H)

### <Example 109> Preparation of (S)-4-((1-(4-chloro-8-(3-fluoro-2-methylphenyl)-1-oxo-2-phenyl-1,2-dihydroisoquinolin-3-yl)ethyl)amino)pyrido[2,3-d]pyrimidin-5(8H)-one

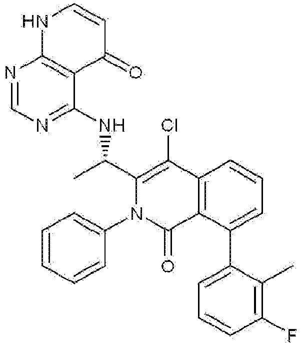

¹H-NMR (400MHz, CDCl₃) δ ppm 1.67(d, 3H), 1.88(s, 3H), 5.05(m, 1H), 6.31(d, 1H), 6.90(m, 2H), 7.00-7.30(m, 3H), 7.40-7.80(m, 6H), 8.10(m, 1H), 8.23(d, 1H), 11.05(br, 1H), 11.30(br, 1H)

### <Example 110> Preparation of (S)-4-((1-(4-chloro-8-(2-methylpyridin-3-yl)-1-oxo-2-phenyl-1,2-dihydroisoquinolin-3-yl)ethyl)amino)pyrido[2,3-d]pyrimidin-5(8H)-one

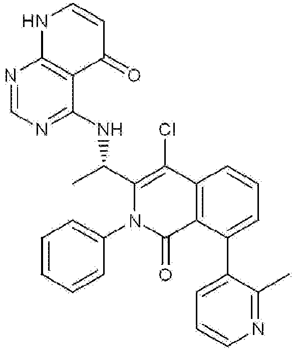

¹H-NMR (400MHz, CDCl₃) δ ppm 1.69(d, 3H), 2.23(s, 3H), 5.05(m, 1H), 6.30(d, 1H), 7.05-7.85(m, 10H), 8.13(d, 1H), 8.23(d, 1H), 8.40(d, 1H), 10.65(br, 1H), 11.00(br, 1H)

### <Example 111> Preparation of (S)-4-((1-(4-chloro-8-(1-methyl-1H-benzo[d]imidazol-6-yl)-1-oxo-2-phenyl-1,2-dihydroisoquinolin-3-yl)ethyl)amino)pyrido[2,3-d]pyrimidin-5(8H)-one

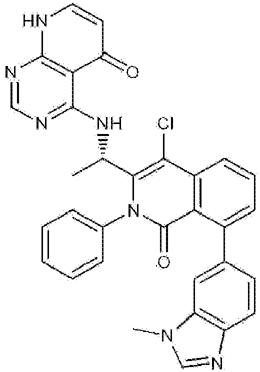

¹H-NMR (400MHz, CDCl₃) δ ppm 1.68(d, 3H), 3.71(s, 3H), 5.05(m, 1H), 6.27(d, 1H), 7.10-7.80(m, 12H), 8.00-8.50(m, 2H), 11.05(br, 1H), 12.10(br, 1H)

### <Example 112> Preparation of (S)-4-((1-(4-chloro-8-(2-(dimethylamino)pyrimidin-5-yl)-1-oxo-2-phenyl-1,2-dihydroisoquinolin-3-yl)ethyl)amino)pyrido[2,3-d]pyrimidin-5(8H)-one

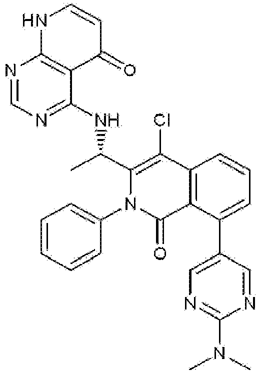

¹H-NMR (400MHz, CDCl₃) δ ppm 1.68(d, 3H), 3.14(s, 6H), 5.01-5.08(m, 1H), 6.29-8.25(m, 13H), 11.04(d, 1H), 11.91(s, 1H)

### <Example 113> Preparation of (S)-4-((1-(4-chloro-8-(imidazo[1,2-a]pyridin-6-yl)-1-oxo-2-phenyl-1,2-dihydroisoquinolin-3-yl)ethyl)amino)pyrido[2,3-d]pyrimidin-5(8H)-one

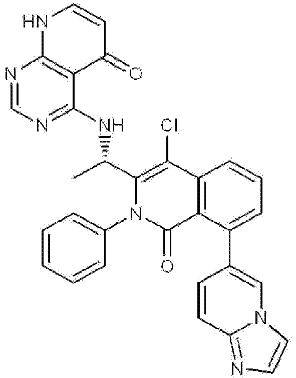

¹H-NMR (400MHz, CDCl₃) δ ppm 1.69(d, 3H), 5.03-5.10(m, 1H), 6.30-8.27(m, 16H), 10.99(d, 1H), 11.22(s, 1H)

### <Example 114> Preparation of (S)-4-((1-(4-chloro-8-ti-methyl-1H-benzo[d]imidazol-5-yl)-1-oxo-2-phenyl-1,2-dihydroisoquinolin-3-yl)ethyl)amino)pyrido[2,3-d]pyrimidin-5(8H)-one

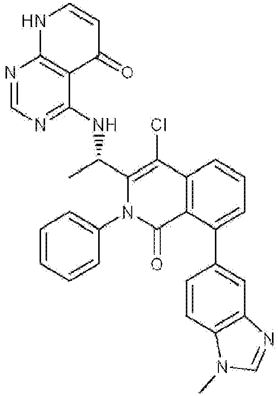

¹H-NMR (400MHz, CDCl₃) δ ppm 1.68(d, 3H), 3.96(s, 3H), 5.02-5.09(m, 1H), 6.28-8.51(m, 15H), 10.81(d, 1H)

### <Example 115> Preparation of (S)-4-((1-(4-chloro-1-oxo-2-phenyl-8-(6-phenylpyridin-3-yl)-1,2-dihydroisoquinolin-3-yl)ethyl)amino)pyrido[2,3-d]pyrimidin-5(8H)-one

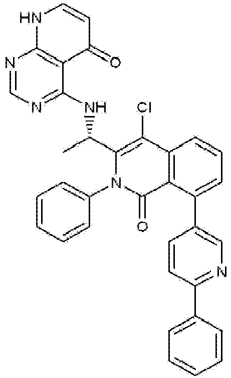

¹H-NMR (400MHz, CDCl₃) δ ppm 1.69(d, 3H), 4.99-5.06(m, 1H), 6.25-8.58(m, 19H), 11.03(d, 1H), 11.84(s, 1H)

### <Example 116> Preparation of (S)-4-((1-(4-chloro-8-(1-methyl-1H-indol-5-yl)-1-oxo-2-phenyl-1,2-dihydroisoquinolin-3-yl)ethyl)amino)pyrido[2,3-d]pyrimidin-5(8H)-one

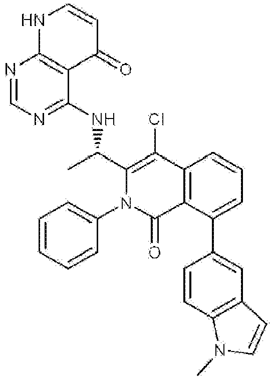

¹H-NMR (400MHz, CDCl₃) δ ppm 1.68(d, 3H), 3.70(s, 3H), 5.01-5.08(m, 1H), 6.23-8.25(m, 16H), 11.01(d, 1H), 11.15(s, 1H)

### <Example 117> Preparation of (S)-4-((1-(4-chloro-8-(furan-2-yl)-1-oxo-2-phenyl-1,2-dihydroisoquinolin-3-yl)ethyl)amino)pyrido[2,3-d]pyrimidin-5(8H)-one

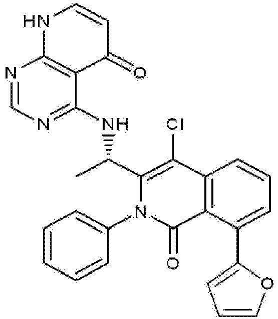

¹H-NMR (400MHz, CDCl₃) δ ppm 1.67(d, 3H), 5.01-5.08(m, 1H), 6.30-8.26(m, 14H), 11.11(d, 1H)

### <Example 118> Preparation of (S)-4-((1-(4-chloro-8-(4-cyclobutylphenyl)-1-oxo-2-phenyl-1,2-dihydroisoquinolin-3-yl)ethyl)amino)pyrido[2,3-d]pyrimidin-5(8H)-one

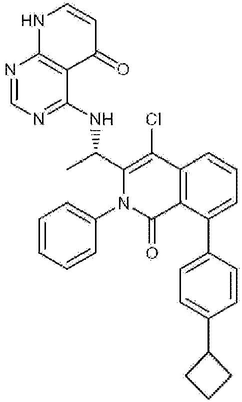

¹H-NMR (400MHz, CD₃OD) δ ppm 1.67(d, 3H), 1.70-1.85(m, 1H), 1.94-2.08(m, 1H), 2.10-2.13(m, 2H), 2.25-2.27(m, 2H), 3.40-3.55(m, 1H), 4.98-5.08(m, 1H), 6.28(d, 1H), 7.11-8.25(m, 13H), 11.05(br, 1H), 11.60(br, 1H)

### <Example 119> Preparation of (S)-4-((1-(4-chloro-8-(5-methylpyridin-3-yl)-1-oxo-2-phenyl-1,2-dihydroisoquinolin-3-yl)ethyl)amino)pyrido[2,3-d]pyrimidin-5(8H)-one

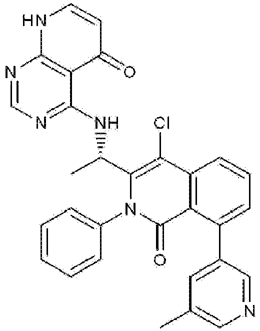

¹H-NMR (400MHz, CD₃OD) δ ppm 1.67(d, 3H), 1.70-1.85(m, 1H), 2.29(s, 3H), 4.97-5.07(m, 1H), 6.25(d, 1H), 7.11-8.25(m, 12H), 11.00(br, 1H), 11.98(br, 1H)

### <Example 120> Preparation of (S)-4-((1-(4-chloro-8-(3,4-dihydro-2H-benzo[b][1,4]oxazin-7-yl)-1-oxo-2-phenyl-1,2-dihydroisoquinolin-3-yl)ethyl)amino)pyrido[2,3-d]pyrimidin-5(8H)-one

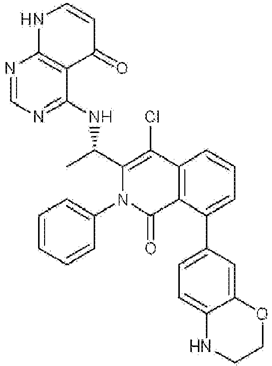

¹H-NMR (400MHz, CD₃OD) δ ppm 1.66(d, 3H), 3.23(t, 2H), 4.17(t, 2H), 4.96-5.14(m, 1H), 6.27(d, 1H), 6.47-8.28(m, 12H), 11.03(br, 1H), 11.80(br, 1H)

### <Example 121> Preparation of (S)-4-((1-(4-chloro-1-oxo-2-phenyl-8-(quinolin-6-yl)-1,2-dihydroisoquinolin-3-yl)ethyl)amino)pyrido[2,3-d]pyrimidin-5(8H)-one

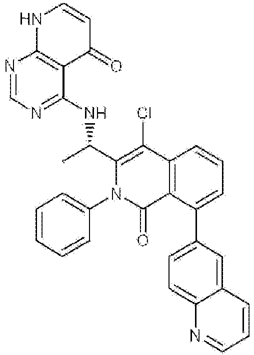

¹H-NMR (400MHz, CD₃OD) δ ppm 1.69(d, 3H), 4.97-5.13(m, 1H), 6.24(d, 1H), 7.15-8.81(m, 15H), 11.05(br, 1H), 11.195(br, 1H)

### <Example 122> Preparation of (S)-4-((1-(4-chloro-8-(2-fluorophenyl)-1-oxo-2-phenyl-1,2-dihydroisoquinolin-3-yl)ethyl)amino)pyrido[2,3-d]pyrimidin-5(8H)-one

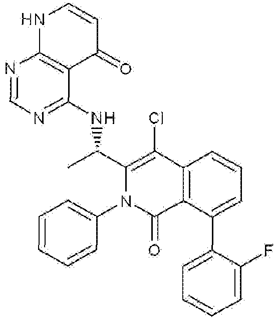

¹H-NMR (400MHz, CDCl₃) δ ppm 1.67(m, 3H), 5.05(m, 1H), 6.29(d, 1H), 6.90-7.80(m, 12H), 8.12(m, 1H), 8.24(d, 1H), 11.05(br, 1H), 12.00(br, 1H)

### <Example 123> Preparation of (S)-4-((1-(4-chloro-8-(3-fluorophenyl)-1-oxo-2-phenyl-1,2-dihydroisoquinolin-3-yl)ethyl)amino)pyrido[2,3-d]pyrimidin-5(8H)-one

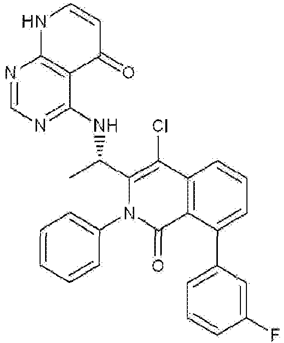

¹H-NMR (400MHz, CDCl₃) δ ppm 1.68(m, 3H), 5.05(m, 1H), 6.31(d, 1H), 6.80-7.80(m, 12H), 8.12(d, 1H), 8.24(s, 1H), 11.20(br, 1H)

### <Example 124> Preparation of (S)-4-((1-(4-chloro-8-(3,5-difluorophenyl)-1-oxo-2-phenyl-1,2-dihydroisoquinolin-3-yl)ethyl)amino)pyrido[2,3-d]pyrimidin-5(8H)-one

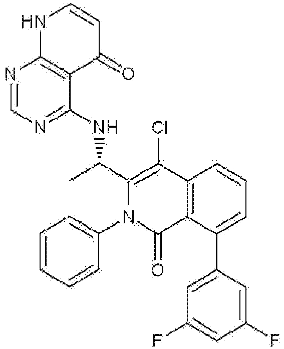

¹H-NMR (400MHz, CDCl₃) δ ppm 1.68(m, 3H), 5.05(m, 1H), 6.30(d, 1H), 6.60-6.80(m, 3H), 7.10-7.80(m, 8H), 8.12(d, 1H), 8.26(s, 1H), 11.10(br, 1H)

### <Example 125> Preparation of (S)-4-((1-(8-(1H-benzo[d]imidazol-5-yl)-4-chloro-1-oxo-2-phenyl-1,2-dihydroisoquinolin-3-yl)ethyl)amino)pyrido[2,3-d]pyrimidin-5(8H)-one

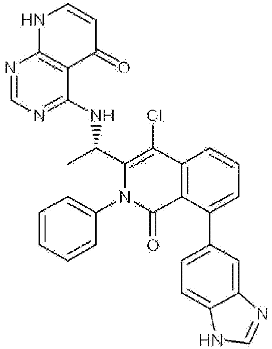

¹H-NMR (400MHz, CD₃OD) δ ppm 1.69(d, 3H), 5.06-5.13(m, 1H), 6.29-8.28(m, 15H), 10.86(d, 1H)

### <Example 126> Preparation of (S)-4-((1-(4-chloro-8-(4-hydroxy-3,5-dimethylphenyl)-1-oxo-2-phenyl-1,2-dihydroisoquinolin-3-yl)ethyl)amino)pyrido[2,3-d]pyrimidin-5(8H)-one

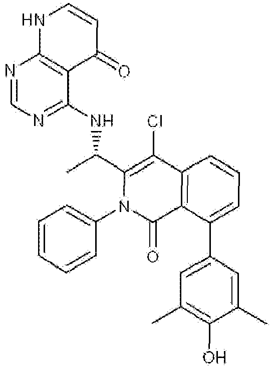

¹H-NMR (400MHz, CDCl₃) δ ppm 1.67(d, 3H), 2.08(s, 6H), 5.02-5.10(m, 1H), 6.28-8.26(m, 13H), 11.03(d, 1H), 11.41(s, 1H)

### <Example 127> Preparation of (S)-4-((1-(4-chloro-8-(5-fluoro-2-methylphenyl)-1-oxo-2-phenyl-1,2-dihydroisoquinolin-3-yl)ethyl)amino)pyrido[2,3-d]pyrimidin-5(8H)-one

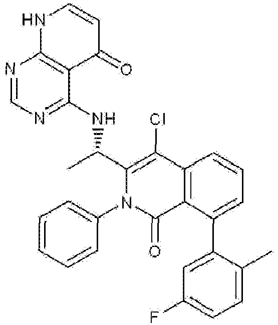

¹H-NMR (400MHz, CDCl₃) δ ppm 1.68(m, 3H), 1.91(s, 3H), 5.05(m, 1H), 6.30(d, 1H), 6.70-7.80(m, 11H), 8.10(m, 1H), 8.23(d, 1H), 11.05(br, 1H)

### <Example 128> Preparation of (S)-4-((1-(4-chloro-8-(4-fluoro-3-methylphenyl)-1-oxo-2-phenyl-1,2-dihydroi soquinolin-3-yl)ethyl)amino)pyrido[2,3-d]pyrimidin-5(8H)-one

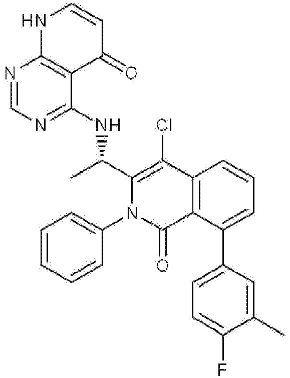

¹H-NMR (400MHz, CDCl₃) δ ppm 1.68(m, 3H), 2.19(s, 3H), 5.05(m, 1H), 6.29(d, 1H), 6.80-7.80(m, 11H), 8.09(d, 1H), 8.24(s, 1H), 11.05(br, 1H), 11.95(br, 1H)

### <Example 129> Preparation of (S)-4-((1-(4-chloro-8-(2,3-difluorophenyl)-1-oxo-2-phenyl-1,2-dihydroisoquinolin-3-yl)ethyl)amino)pyrido[2,3-d]pyrimidin-5(8H)-one

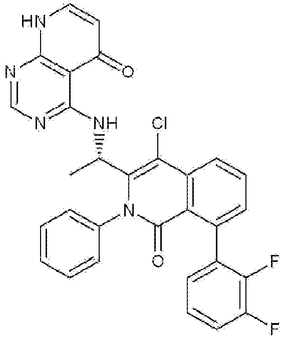

¹H-NMR (400MHz, CDCl₃) δ ppm 1.68(m, 3H), 5.03(m, 1H), 6.31(d, 1H), 6.90-7.80(m, 11H), 8.15(m, 1H), 8.25(d, 1H), 11.05(br, 1H)

### <Example 130> Preparation of (S)-4-((1-(4-chloro-8-(4-(1-methylpiperidin-4-yl)phenyl)-1-oxo-2-phenyl-1,2-dihydroisoquinolin-3-yl)ethyl)amino)pyrido[2,3-d]pyrimidin-5(8H)-one

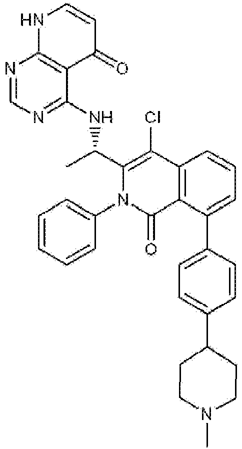

¹H-NMR (400MHz, CDCl₃) δ ppm 1.67(d, 3H), 1.82-3.07(m, 9H), 5.00-5.07(m, 1H), 6.28-8.24(m, 15H), 11.00(d, 1H)

### <Example 131> Preparation of (S)-4-((1-(4-chloro-8-(4-fluoro-2-methylphenyl)-1-oxo-2-phenyl-1,2-dihydroisoquinolin-3-yl)ethyl)amino)pyrido[2,3-d]pyrimidin-5(8H)-one

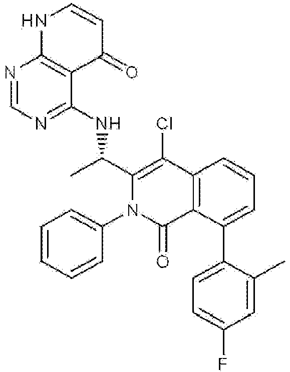

¹H-NMR (400MHz, CDCl₃) δ ppm 1.67(d, 3H), 1.96(s, 3H), 5.05(m, 1H), 6.31(d, 1H), 6.80-7.80(m, 11H), 8.10(m, 1H), 8.23(d, 1H), 11.00(br, 2H)

### <Example 132> Preparation of (S)-4-((1-(4-chloro-8-(2,3-dihydro-[1,4]dioxino[2,3-b]pyridin-7-yl)-1-oxo-2-phenyl-1,2-dihydroisoquinolin-3-yl)ethyl)amino)pyrido[2,3-d]pyrimidin-5(8H)-one

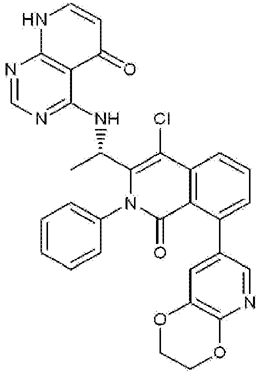

¹H-NMR (400MHz, CDCl₃) δ ppm 1.68(d, 3H), 4.21(m, 2H), 4.40(m, 2H), 5.05(m, 1H), 6.30(d, 1H), 7.10-7.80(m, 10H), 8.11(d, 1H), 8.27(s, 1H), 10.95(br, 1H)

### <Example 133> Preparation of (S)-4-((1-(4-chloro-8-(6-methoxypyridin-3-yl)-1-oxo-2-phenyl-1,2-dihydroisoquinolin-3-yl)ethyl)amino)pyrido[2,3-d]pyrimidin-5(8H)-one

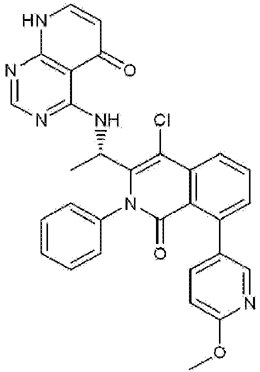

¹H-NMR (400MHz, CD₃OD) δ ppm 1.68(d, 3H), 3.88(s, 3H), 5.02-5.06(m, 1H), 6.30(d, 1H), 6.64-8.25(m, 12H), 11.02(br, 1H), 12.02(br, 1H)

### <Example 134> Preparation of (S)-4-((1-(4-chloro-1-oxo-2-phenyl-8-(pyridin-4-yl)-1,2-dihydroisoquinolin-3-yl)ethyl)amino)pyrido[2,3-d]pyrimidin-5(8H)-one

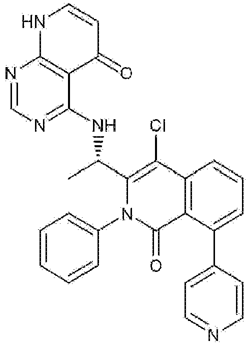

¹H-NMR (400MHz, CDCl₃) δ ppm 1.68(d, 3H), 5.01-5.08(m, 1H), 6.30-8.53(m, 15H), 11.05(d, 1H), 11.92(s, 1H)

### <Example 135> Preparation of (S)-4-((1-(4-chloro-8-(2-methyl-1H-benzo[d]imidazol-5-yl)-1-oxo-2-phenyl-1,2-dihydroisoquinolin-3-yl)ethyl)amino)pyrido[2,3-d]pyrimidin-5(8H)-one

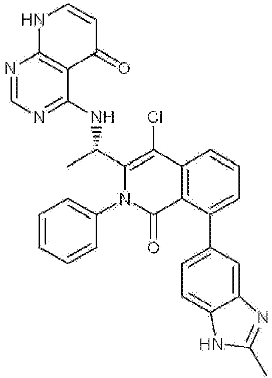

¹H-NMR (400MHz, CD₃OD) δ ppm 1.69(d, 3H), 2.39(s, 3H), 5.05-5.12(m, 1H), 6.29-8.23(m, 14H), 10.93(d, 1H)

### <Example 136> Preparation of (S)-4-((1-(4-chloro-8-(2-methylpyrimidin-5-yl)-1-oxo-2-phenyl-1,2-dihydroisoquinolin-3-yl)ethyl)amino)pyrido[2,3-d]pyrimidin-5(8H)-one

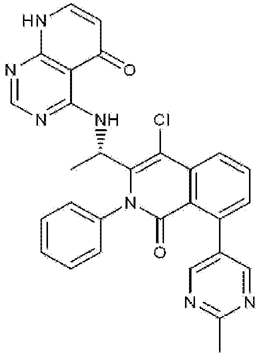

¹H-NMR (400MHz, CDCl₃) δ ppm 1.69(d, 3H), 2.70(s, 3H), 5.01-5.08(m, 1H), 6.31-8.56(m, 14H), 11.05(d, 1H), 11.79(s, 1H)

### <Example 137> Preparation of (S)-4-((1-(4-chloro-1-oxo-2-phenyl-8-(quinoxalin-6-yl)-1,2-dihydroisoquinolin-3-yl)ethyl)amino)pyrido[2,3-d]pyrimidin-5(8H)-one

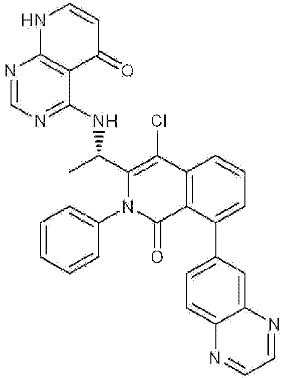

¹H-NMR (400MHz, CDCl₃) δ ppm 1.69(d, 3H), 5.01-5.08(m, 1H), 6.31-8.79(m, 16H), 10.40(s, 1H), 10.98(d, 1H)

### <Example 138> Preparation of tert-butyl (S)-4-(4-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)phenyl)piperazin-1-carboxylate

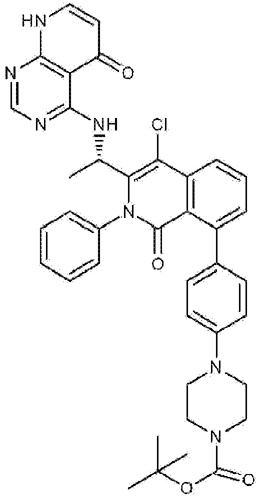

¹H-NMR (400MHz, CDCl₃) δ ppm 1.48(s, 9H), 1.67(d, 3H), 3.09-3.60(m, 8H), 5.01-5.08(m, 1H), 6.27-8.24(m, 15H), 11.05(d, 1H), 11.81(s, 1H)

### <Example 139> Preparation of (S)-4-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-N,N-dimethylbenzamide

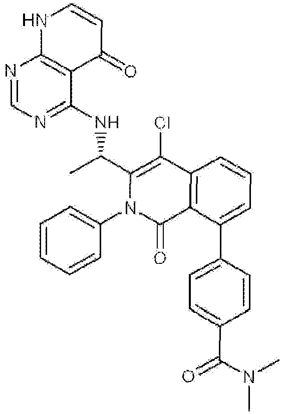

¹H-NMR (400MHz, CDCl₃) δ ppm 1.67(d, 3H), 3.07(d, 6H), 5.01-5.08(m, 1H), 6.20-8.24(m, 15H), 11.04(d, 1H), 12.05(s, 1H)

### <Example 140> Preparation of (S)-4-((1-(4-chloro-8-(5-chloro-2-fluorophenyl)-1-oxo-2-phenyl-1,2-dihydroisoquinolin-3-yl)ethyl)amino)pyrido[2,3-d]pyrimidin-5(8H)-one

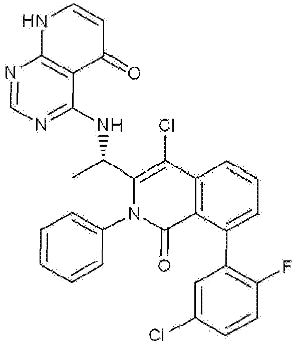

¹H-NMR (400MHz, CDCl₃) δ ppm 1.67(d, 3H), 4.97-5.07(m, 1H), 6.30-8.26(m, 14H), 11.02(d, 1H), 11.79(s, 1H)

### <Example 141> Preparation of (S)-4-((1-(4-chloro-8-(4-cyclopropylphenyl)-1-oxo-2-phenyl-1,2-dihydroisoquinolin-3-yl)ethyl)amino)pyrido[2,3-d]pyrimidin-5(8H)-one

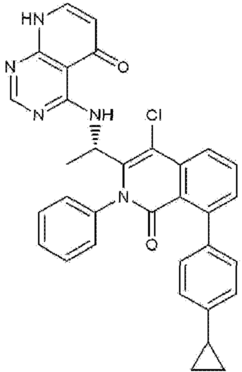

¹H-NMR (400MHz, CDCl₃) δ ppm 0.61(m, 2H), 0.85(m, 2H), 1.67(d, 3H), 1.79(m, 1H), 5.05(m, 1H), 6.27(d, 1H), 6.90-7.80(m, 12H), 8.06(d, 1H), 8.23(s, 1H), 11.05(br, 1H), 11.78(br, 1H)

### <Example 142> Preparation of (S)-4-((1-(4-chloro-1-oxo-2-phenyl-8-vinyl-1,2-dihydroisoquinolin-3-yl)ethyl)amino)pyrido[2,3-d]pyrimidin-5(8H)-one

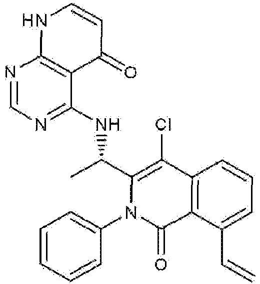

¹H-NMR (400MHz, CDCl₃) δ ppm 1.68(d, 3H), 5.06(m, 1H), 5.26(d, 1H), 5.45(d, 1H), 6.32(d, 1H), 7.15-8.05(m, 9H), 8.24(s, 1H), 11.10(br, 1H), 12.35(br)

### <Example 143> Preparation of (S)-4-((1-(4-chloro-8-(4-(methylthio)phenyl)-1-oxo-2-phenyl-1,2-dihydroisoquinolin-3-yl)ethyl)amino)pyrido[2,3-d]pyrimidin-5(8H)-one

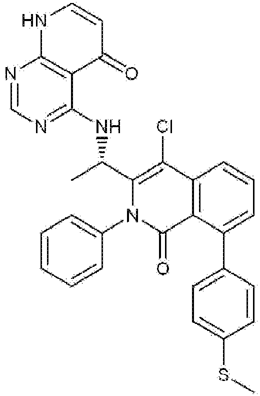

¹H-NMR (400MHz, CDCl₃) δ ppm 1.67(d, 3H), 2.42(s, 3H), 5.04(m, 1H), 6.30(d, 1H), 7.10-7.80(m, 12H), 8.07(d, 1H), 8.25(s, 1H), 11.05(br, 1H), 11.45(br, 1H)

### <Example 144> Preparation of (S)-4-((1-(8-(4-(tert-butylthio)phenyl)-4-chloro-1-oxo-2-phenyl-1,2-dihydroisoquinolin-3-yl)ethyl)amino)pyrido[2,3-d]pyrimidin-5(8H)-one

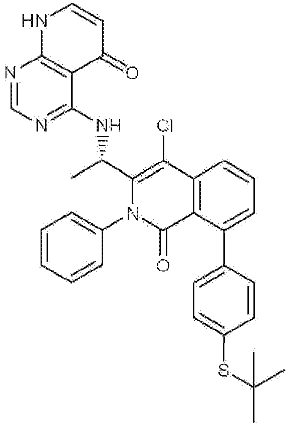

¹H-NMR (400MHz, CDCl₃) δ ppm 1.24(s, 9H), 1.67(d, 3H), 5.02(m, 1H), 6.28(d, 1H), 7.10-7.80(m, 12H), 8.10(d, 1H), 8.24(s, 1H), 11.05(br, 1H), 11.55(br, 1H)

### <Example 145> Preparation of (S)-4-((1-(4-chloro-8-(4-(morpholin-4-carbonyl)phenyl)-1-oxo-2-phenyl-1,2-dihydroisoquinolin-3-yl)ethyl)amino)pyrido[2,3-d]pyrimidin-5(8H)-one

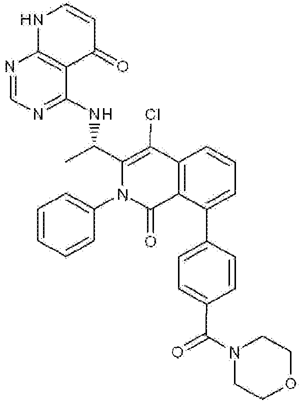

¹H-NMR (400MHz, CDCl₃) δ ppm 1.68(d, 3H), 3.66(bs, 8H), 5.00-5.07(m, 1H), 6.26-8.25(m, 15H), 11.04(d, 1H), 11.59(s, 1H)

### <Example 146> Preparation of (S)-4-((1-(8-(2-(benzyloxy)pyrimidin-5-yl)-4-chloro-1-oxo-2-phenyl-1,2-dihydroisoquinolin-3-yl)ethyl)amino)pyrido[2,3-d]pyrimidin-5(8H)-one

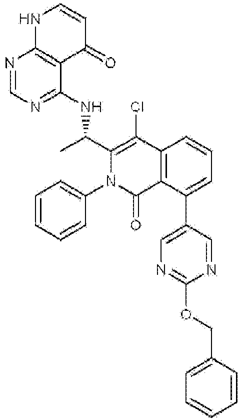

¹H-NMR (400MHz, CDCl₃) δ ppm 1.69(d, 1H), 4.99-5.06(m, 1H), 5.40(s, 2H), 6.27-8.47(m, 18H), 11.01(d, 1H), 11.71(s, 1H)

### <Example 147> Preparation of (S)-4-((1-(4-chloro-1-oxo-2-phenyl-8-(6-(2,2,2-trifluoroethoxy)pyridin-3-yl)-1,2-dihydroisoquinolin-3-yl)ethyl)amino)pyrido[2,3-d]pyrimidin-5(8H)-one

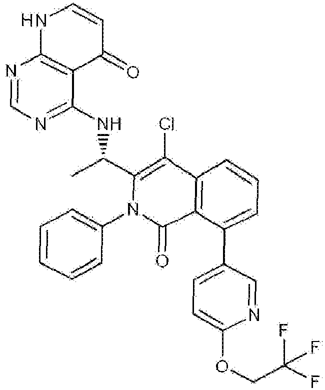

¹H-NMR (400MHz, CDCl₃) δ ppm 1.68(d, 3H), 4.67-4.74(m, 2H), 5.01-5.08(m, 1H), 6.31-8.25(m, 14H), 11.07(d, 1H), 11.89(s, 1H)

### <Example 148> Preparation of (S)-4-((1-(4-chloro-1-oxo-2-phenyl-8-(4-(piperazin-1-yl)phenyl)-1,2-dihydroisoquinolin-3-yl)ethyl)amino)pyrido[2,3-d]pyrimidin-5(8H)-one

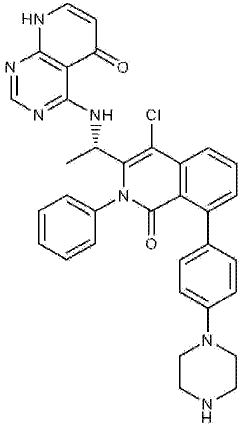

¹H-NMR (400MHz, CD₃OD) δ ppm 1.73(d, 3H), 3.28-3.44(m, 8H), 5.13-5.18(m, 1H), 6.46-8.50(m, 15H)

### <Example 149> Preparation of (S)-4-((1-(4-chloro-8-(2-methoxypyridin-4-yl)-1-oxo-2-phenyl-1,2-dihydroisoquinolin-3-yl)ethyl)amino)-8-(2-methoxypyridin-4-yl)pyrido[2,3-d]pyrimidin-5(8H)-one

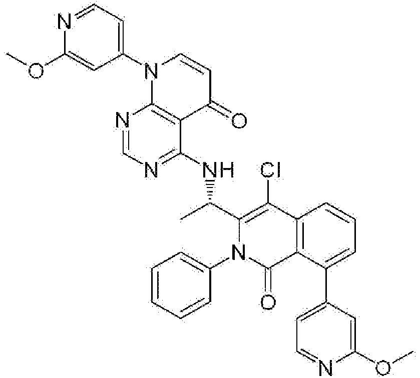

¹H-NMR (400MHz, CDCl₃) δ ppm 1.69(d, 3H), 3.90(s, 3H), 4.00(s, 3H), 5.02-5.07(m, 1H), 6.38-8.31(m, 13H), 11.04(d, 1H)

### <Example 150> Preparation of (S)-4-((1-(4-chloro-8-(2-methoxypyridin-4-yl)-1-oxo-2-phenyl-1,2-dihydroisoquinolin-3-yl)ethyl)amino)pyrido[2,3-d]pyrimidin-5(8H)-one

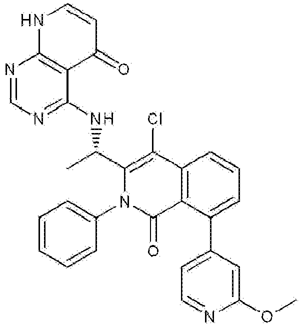

¹H-NMR (400MHz, CDCl₃) δ ppm 1.68(d, 3H), 3.89(s, 3H), 5.00-5.07(m, 1H), 6.30-8.26(m, 14H), 11.05(d, 1H), 11.73(s, 1H)

### <Example 151> Preparation of (S)-4-((1-(4-chloro-1-oxo-2-phenyl-8-(4-(propylthio)phenyl)-1,2-dihydroisoquinolin-3-yl)ethyl)amino)pyrido[2,3-d]pyrimidin-5(8H)-one

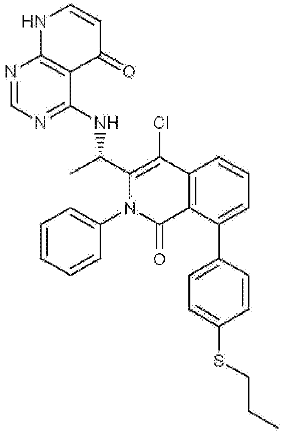

¹H-NMR (400MHz, CDCl₃) δ ppm 0.99(t, 3H), 1.64(m, 5H), 2.85(t, 2H), 5.05(m, 1H), 6.29(d, 1H), 7.10-7.80(m, 12H), 8.08(d, 1H), 8.24(s, 1H), 11.05(br, 1H), 11.35(br, 1H)

### <Example 152> Preparation of (S)-4-((1-(4-chloro-8-(4-(cyclopropylthio)phenyl)-1-oxo-2-phenyl-1,2-dihydroisoquinolin-3-yl)ethyl)amino)pyrido[2,3-d]pyrimidin-5(8H)-one

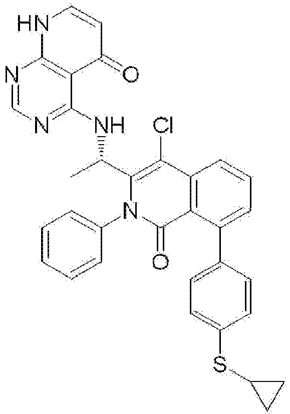

¹H-NMR (400MHz, CDCl₃) δ ppm 0.65(m, 2H), 0.98(m, 2H), 1.68(d, 3H), 2.10(m, 1H), 5.05(m, 1H), 6.29(d, 1H), 7.10-7.80(m, 12H), 8.08(d, 1H), 8.24(s, 1H), 11.05(br, 1H), 11.75(br, 1H)

### <Example 153> Preparation of (S)-4-((1-(4-chloro-1-oxo-2-phenyl-8-(2-(trifluoromethyl)pyrimidin-5-yl)-1,2-dihydroisoquinolin-3-yl)ethyl)amino)pyrido[2,3-d]pyrimidin-5(8H)-one

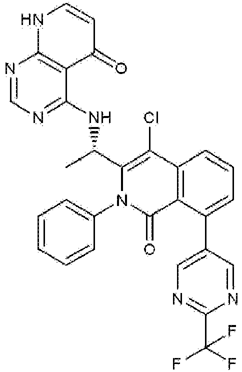

¹H-NMR (400MHz, CDCl₃) δ ppm 1.70(d, 3H), 5.02-5.09(m, 1H), 6.32-8.79(m, 13H), 10.62(s, 1H), 11.00(d, 1H)

### <Example 154> Preparation of (S)-4-((1-(4-chloro-8-(4-fluoro-3,5-dimethoxyphenyl)-1-oxo-2-phenyl-1,2-dihydroisoquinolin-3-yl)ethyl)amino)pyrido[2,3-d]pyrimidin-5(8H)-one

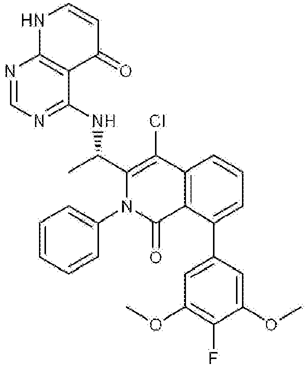

¹H-NMR (400MHz, CDCl₃) δ ppm 1.68(d, 3H), 3.79(s, 6H), 5.05-5.12(m, 1H), 6.30-8.24(m, 13H), 11.07(d, 1H), 11.50(s, 1H)

### <Example 155> Preparation of (S)-4-((1-(4-chloro-8-(3,4-dimethoxyphenyl)-1-oxo-2-phenyl-1,2-dihydroisoquinolin-3-yl)ethyl)amino)pyrido[2,3-d]pyrimidin-5(8H)-one

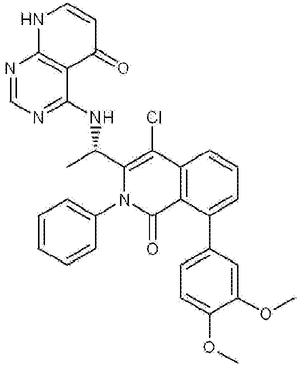

¹H-NMR (400MHz, CDCl₃) δ ppm 1.68(d, 3H), 3.79(s, 3H), 3.82(s, 3H), 5.02-5.09(m, 1H), 6.29-8.24(m, 14H), 11.06(d, 1H), 11.69(s, 1H)

### <Example 156> Preparation of (S)-4-((1-(4-chloro-8-(cyclopent-1-en-1-yl)-1-oxo-2-phenyl-1,2-dihydroisoquinolin-3-yl)ethyl)amino)pyrido[2,3-d]pyrimidin-5(8H)-one

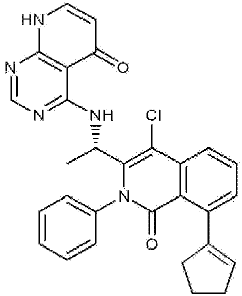

¹H-NMR (400MHz, CDCl₃) δ ppm 1.66(d, 3H), 1.93-2.62(m, 6H), 5.00-5.07(m, 1H), 5.53(t, 1H), 6.29-8.24(m, 11H), 11.04(d, 1H), 11.94(s, 1H)

### <Example 157> Preparation of (S)-4-((1-(4-chloro-8-(3-isopropoxy-5-methoxyphenyl)-1-oxo-2-phenyl-1,2-dihydroisoquinolin-3-yl)ethyl)amino)pyrido[2,3-d]pyrimidin-5(8H)-one

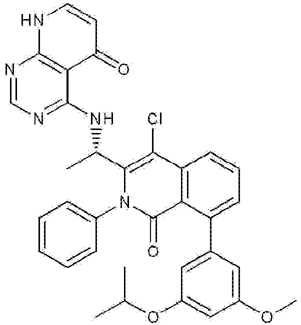

¹H-NMR (400MHz, CD₃OD) δ ppm 1.24(d, 6H), 1.67(d, 3H), 3.68(s, 3H), 4.41-4.44(m, 1H), 5.04-5.08(m, 1H), 6.27-8.25(m, 13H), 11.04(br, 1H), 11.07(br, 1H)

### <Example 158> Preparation of (S)-4-((1-(4-chloro-8-(3-methoxy-5-(trifluoromethoxy)phenyl)-1-oxo-2-phenyl-1,2-dihydroisoquinolin-3-yl)ethyl)amino)pyrido[2,3-d]pyrimidin-5(8H)-one

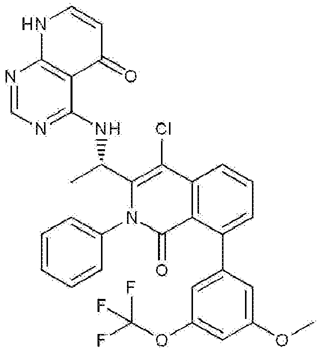

¹H-NMR (400MHz, CD₃OD) δ ppm 1.68(d, 3H), 3.73(s, 3H), 5.04-5.07(m, 1H), 6.30-8.26(m, 13H), 11.05(br, 1H), 11.82(br, 1H)

### <Example 159> Preparation of (S)-4-((1-(4-chloro-8-(3-methoxy-4-(trifluoromethoxy)phenyl)-1-oxo-2-phenyl-1,2-dihydroisoquinolin-3-yl)ethyl)amino)pyrido[2,3-d]pyrimidin-5(8H)-one

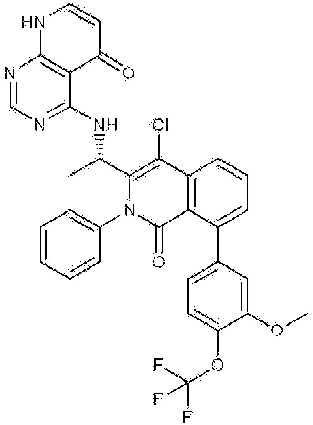

¹H-NMR (400MHz, CD₃OD) δ ppm 1.68(d, 3H), 3.78(s, 3H), 5.04-5.08(m, 1H), 6.30-8.24(m, 13H), 11.05(br, 1H), 11.52(br, 1H)

### <Example 160> Preparation of (S)-4-((1-(4-chloro-8-(4-(5-methyl-1,2,4-oxadiazol-3-yl)phenyl)-1-oxo-2-phenyl-1,2-dihydroisoquinolin-3-yl)ethyl)amino)pyrido[2,3-d]pyrimidin-5(8H)-one

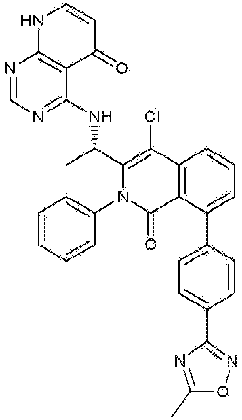

¹H-NMR (400MHz, CDCl₃) δ ppm 1.68(d, 3H), 2.61(s, 3H), 5.05(m, 1H), 6.30(d, 1H), 7.10-7.80 (m, 10H), 7.99(d, 2H), 8.12(d, 1H), 8.25(s, 1H), 11.05(br, 1H), 11.53(br, 1H)

### <Example 161> Preparation of (S)-4-((1-(8-(4-(5-(tert-butyl)-1,2,4-oxadiazol-3-yl)phenyl)-4-chloro-1-oxo-2-phenyl-1,2-dihydroisoquinolin-3-yl)ethyl)amino)pyrido[2,3-d]pyrimidin-5(8H)-one

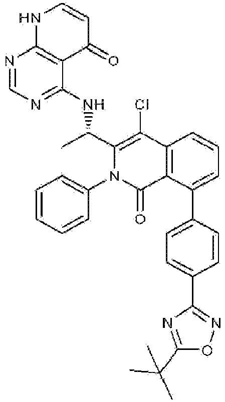

¹H-NMR (400MHz, CDCl₃) δ ppm 1.48(s, 9H), 1.68(d, 3H), 5.05(m, 1H), 6.30(d, 1H), 7.10-7.80(m, 10H), 8.02(d, 2H), 8.12(d, 1H), 8.25(s, 1H), 11.00(br, 1H)

### <Example 162> Preparation of (S)-4-((1-(4-chloro-1-oxo-2-phenyl-8-(6-((tetrahydro-2H-pyran-4-yl)oxy)pyridin-3-yl)-1,2-dihydroisoquinolin-3-yl)ethyl)amino)pyrido[2,3-d]pyrimidin-5(8H)-one

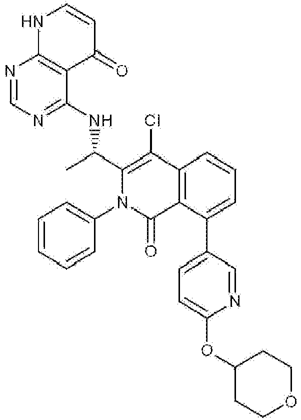

¹H-NMR (400MHz, CDCl₃) δ ppm 1.68(d, 3H), 1.80(m, 2H), 2.05(m, 2H), 3.60(m, 2H), 3.95(m, 2H), 5.05(m, 1H), 5.20(m, 1H), 6.30(d, 1H), 6.65(d, 1H), 7.10-7.80(m, 9H), 7.98(s, 1H), 8.12(d, 1H), 8.25(s, 1H), 10.68(br, 1H), 11.00(br, 1H)

### <Example 163> Preparation of (S)-4-((1-(4-chloro-8-(6-(cyclopropylmethoxy)pyridin-3-yl)-1-oxo-2-phenyl-1,2-dihydroisoquinolin-3-yl)ethyl)amino)pyrido[2,3-d]pyrimidin-5(8H)-one

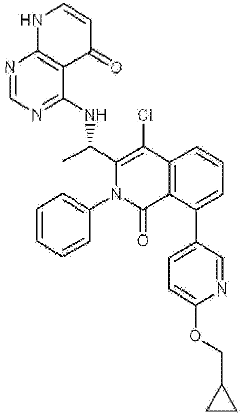

¹H-NMR (400MHz, CDCl₃) δ ppm 0.29(m, 2H), 0.56(m, 2H), 1.25(m, 1H), 1.68(d, 3H), 4.06(d, 2H), 5.04(m, 1H), 6.30(d, 1H), 6.68(d, 1H), 7.10-7.80(m, 9H), 7.98(s, 1H), 8.11(d, 1H), 8.25(s, 1H), 11.05(br, 1H), 11.95(br, 1H)

### <Example 164> Preparation of (S)-4-((1-(8-(6-(benzyloxy)pyridin-3-yl)-4-chloro-1-oxo-2-phenyl-1,2-dihydroisoquinolin-3-yl) ethyl)amino)pyrido[2 ,3-d]pyrimidin-5(8H)-one

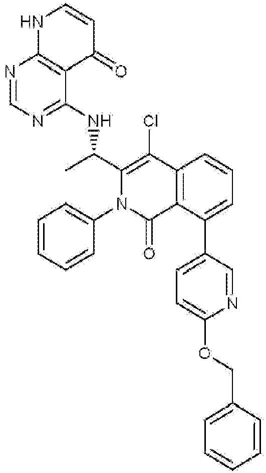

¹H-NMR (400MHz, CDCl₃) δ ppm 1.68(d, 3H), 5.04(m, 1H), 5.32(s, 2H), 6.30(d, 1H), 6.74(d, 1H), 7.10-7.80(m, 14H), 8.04(s, 1H), 8.12(d, 1H), 8.25(s, 1H), 11.05(br, 1H), 11.47(br, 1H)

### <Example 165> Preparation of (S)-4-((1-(4-chloro-8-(4-(methylsulfonyl)phenyl)-1-oxo-2-phenyl-1,2-dihydroisoquinolin-3-yl)ethyl)amino)pyrido[2,3-d]pyrimidin-5(8H)-one

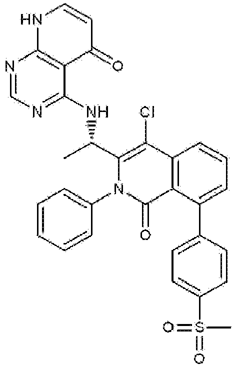

¹H-NMR (400MHz, CDCl₃) δ ppm 1.69(d, 3H), 3.03(s, 3H), 5.04(m, 1H), 6.29(d, 1H), 7.10-7.90(m, 12H), 8.15(d, 1H), 8.26(s, 1H), 11.05(br, 1H), 11.90(br, 1H)

### <Example 166> Preparation of (S)-4-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)benzoic acid

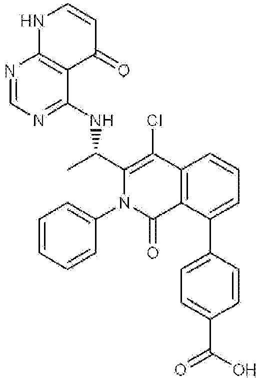

¹H-NMR (400MHz, CD₃OD) δ ppm 1.68(d, 3H), 3.78(s, 3H), 5.04-5.08(m, 1H), 6.30-8.24(m, 13H), 11.05(br, 1H), 11.52(br, 1H)

### <Example 167> Preparation of (S)-4-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-N-methylbenzamide

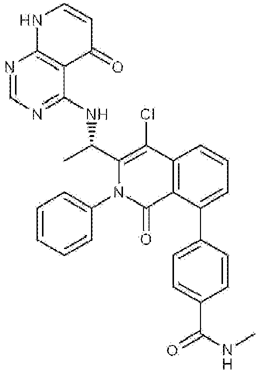

¹H-NMR (400MHz, CD₃OD) δ ppm 1.67(d, 3H), 2.90(d, 3H), 5.04-5.08(m, 1H), 6.27-8.26(m, 13H), 11.00(br, 1H), 11.82(br, 1H)

### <Example 168> Preparation of (S)-4-((1-(4-chloro-8-(2-cyclopropylpyrimidin-5-yl)-1-oxo-2-phenyl-1,2-dihydroisoquinolin-3-yl)ethyl)amino)pyrido[2,3-d]pyrimidin-5(8H)-one

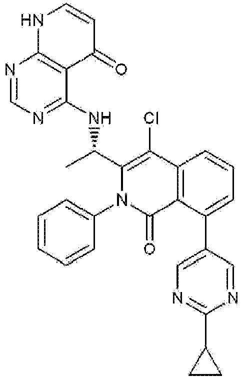

¹H-NMR (400MHz, CD₃OD) δ ppm 1.00-1.03(m, 2H), 1.08-1.10(m, 2H), 1.68(d, 3H), 2.17(m, 1H), 5.05-5.08(m, 1H), 6.30-8.47(m, 12H), 11.00(br, 1H), 12.1.2(br, 1H)

### <Example 169> Preparation of (S)-4-((1-(4-chloro-8-(3,5-dimethoxyphenyl)-1-oxo-2-phenyl-1,2-dihydroisoquinolin-3-yl)ethyl)amino)pyrido[2,3-d]pyrimidin-5(8H)-one

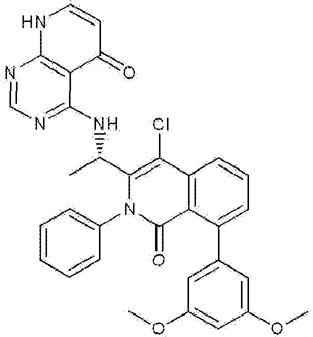

¹H-NMR (400MHz, CD₃OD) δ ppm 1.67(d, 3H), 3.70(s, 6H), 5.04-5.06(m, 1H), 6.27-8.25(m, 13H), 11.04(br, 1H), 11.68(br, 1H)

### <Example 170> Preparation of (S)-4-((1-(4-chloro-8-(5,6-dimethoxypyridin-3-yl)-1-oxo-2-phenyl-1,2-dihydroisoquinolin-3-yl)ethyl)amino)pyrido[2,3-d]pyrimidin-5(8H)-one

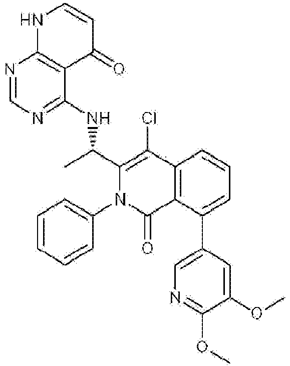

¹H-NMR (400MHz, CDCl₃) δ ppm 1.68(d, 3H), 3.93(s, 3H), 3.99(s, 3H), 5.02-5.09(m, 1H), 6.30-8.24(m, 13H), 11.00(d, 1H)

### <Example 171> Preparation of (S)-4-((1-(4-chloro-8-(2-methoxypyrimidin-5-yl)-1-oxo-2-phenyl-1,2-dihydroisoquinolin-3-yl)ethyl)amino)pyrido[2,3-d]pyrimidin-5(8H)-one

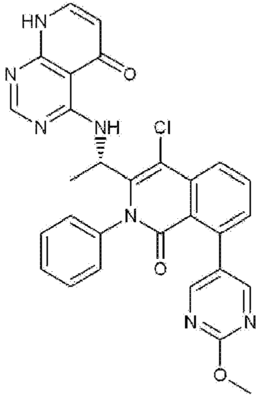

¹H-NMR (400MHz, CDCl₃) δ ppm 1.69(d, 3H), 3.97(s, 3H), 5.04(m, 1H), 6.29(d, 1H), 7.10-7.75(m, 8H), 8.15(d, 1H), 8.27(s, 1H), 8.44(s, 2H), 11.05(br, 1H), 12.10(br, 1H)

### <Example 172> Preparation of (S)-4-((1-(8-(6-aminopyridin-3-yl)-4-chloro-1-oxo-2-phenyl-1,2-dihydroisoquinolin-3-yl)ethyl)amino)pyrido[2,3-d]pyrimidin-5(8H)-one

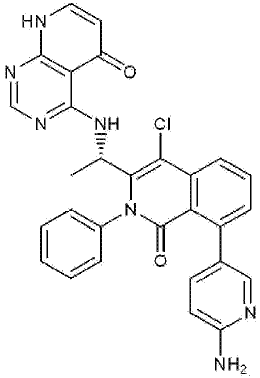

¹H-NMR (400MHz, CDCl₃+ CD₃OD) δ ppm 1.67(d, 3H), 2.35(br), 5.03(m, 1H), 6.29(d, 1H), 6.46(d, 1H), 7.10-7.80(m, 9H), 7.86(s, 1H), 8.10(d, 1H), 8.22(s, 1H), 10.96(br, 1H)

### <Example 173> Preparation of (S)-4-((1-(4-chloro-8-(3-morpholinophenyl)-1-oxo-2-phenyl-1,2-dihydroisoquinolin-3-yl)ethyl)amino)pyrido[2,3-d]pyrimidin-5(8H)-one

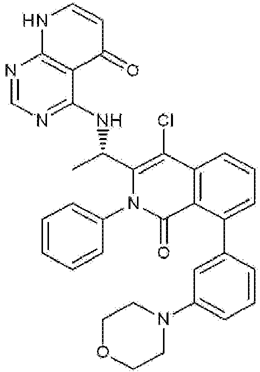

¹H-NMR (400MHz, CDCl₃) δ ppm 1.67(d, 3H), 3.09(m, 4H), 3.77(m, 4H), 5.04(m, 1H), 6.27(d, 1H), 6.74(s, 1H), 6.76(s, 2H), 7.10-7.75(m, 9H), 8.08(d, 1H), 8.24(s, 2H), 11.05(br, 1H), 11.50(br, 1H)

### <Example 174> Preparation of (S)-4-((1-(4-chloro-8-(2-morpholinopyrimidin-5-yl)-1-oxo-2-phenyl-1,2-dihydroisoquinolin-3-yl)ethyl)amino)pyrido[2,3-d]pyrimidin-5(8H)-one

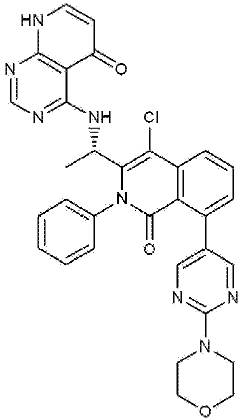

¹H-NMR (400MHz, CDCl₃) δ ppm 1.68(d, 3H), 3.74(m, 8H), 5.04(m, 1H), 6.30(d, 1H), 7.10-7.80(m, 9H), 8.11(m, 1H), 8.25(s, 2H), 11.05(br, 1H), 11.78(br, 1H)

### <Example 175> Preparation of (S)-4-((1-(4-chloro-8-(6-morpholinopyridin-3-yl)-1-oxo-2-phenyl-1,2-dihydroisoquinolin-3-yl)ethyl)amino)pyrido[2,3-d]pyrimidin-5(8H)-one

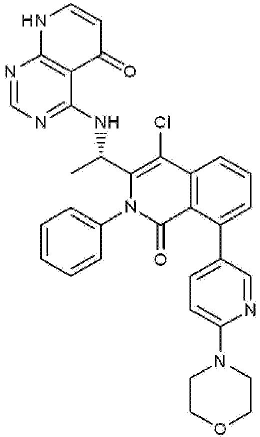

¹H-NMR (400MHz, CDCl₃) δ ppm 1.67(d, 3H), 3.45(m, 4H), 3.77(m, 4H), 5.04(m, 1H), 6.30(d, 1H), 6.56(d, 1H), 7.10-7.80(m, 10H), 8.08(m, 2H), 8.25(s, 1H), 11.05(br, 1H), 11.45(br, 1H)

### <Example 176> Preparation of (S)-4-((1-(4-chloro-1-oxo-2-phenyl-8-(4-(tetrahydro-2H-pyran-4-yl)phenyl)-1,2-dihydroisoquinolin-3-yl)ethyl)amino)pyrido[2,3-d]pyrimidin-5(8H)-one

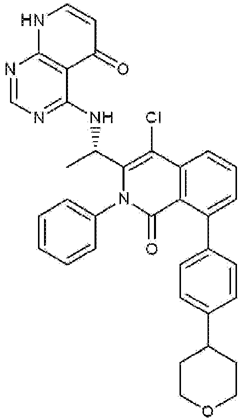

¹H-NMR (400MHz, CDCl₃) δ ppm 1.66-1.84(m, 7H), 2.67-2.75(m, 1H), 3.47-4.07(m, 4H), 5.00-5.07(m, 1H), 6.29-8.25(m, 15H), 11.03(d, 1H), 11.29(s, 1H)

### <Example 177> Preparation of (S)-4-((1-(4-chloro-8-(3,5-dimethoxy-4-methylphenyl)-1-oxo-2-phenyl-1,2-dihydroisoquinolin-3-yl)ethyl)amino)pyrido[2,3-d]pyrimidin-5(8H)-one

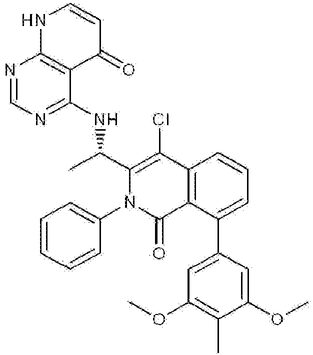

¹H-NMR (400MHz, CDCl₃) δ ppm 1.68(d, 3H), 3.72(s, 6H), 5.06-5.13(m, 1H), 6.28-8.24(m, 13H), 11.07(d, 1H), 11.49(s, 1H)

### <Example 178> Preparation of (S)-4-((1-(4-chloro-1-oxo-2-phenyl-8-(4-(trimethylsilyl)phenyl)-1,2-dihydroisoquinolin-3-yl)ethyl)amino)pyrido[2,3-d]pyrimidin-5(8H)-one

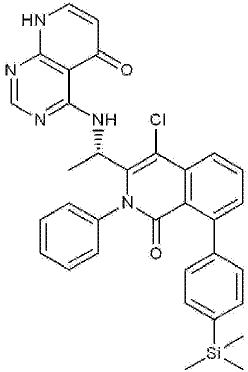

¹H-NMR (400MHz, CDCl₃) δ ppm 0.21(s, 9H), 1.67(d, 3H), 5.00-5.07(m, 1H), 6.28-8.25(m, 15H), 11.04(d, 1H), 11.45(s, 1H)

### <Example 179> Preparation of (S)-4-((1-(4-chloro-8-(4-(4-methylpiperazin-1-yl)phenyl)-1-oxo-2-phenyl-1,2-dihydroisoquinolin-3-yl)ethyl)amino)pyrido[2,3-d]pyrimidin-5(8H)-one

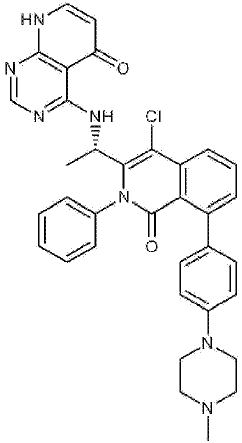

¹H-NMR (400MHz, CDCl₃) δ ppm 1.67(d, 3H), 2.33(s, 3H), 2.52-3.20(m, 8H), 5.01-5.08(m, 1H), 6.28-8.24(m, 15H), 11.01(d, 1H), 11.17(s, 1H)

### <Example 180> Preparation of (S)-4-((1-(4-chloro-8-(2-isopropoxypyridin-4-yl)-1-oxo-2-phenyl-1,2-dihydroisoquinolin-3-yl)ethyl)amino)pyrido[2,3-d]pyrimidin-5(8H)-one

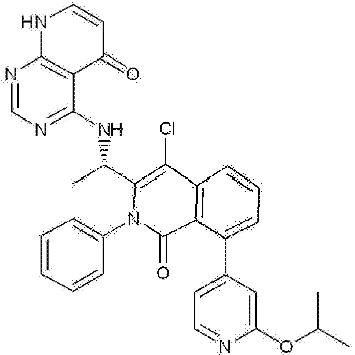

¹H-NMR (400MHz, CDCl₃) δ ppm 1.30-1.69(m, 7H), 5.00-5.07(m, 1H), 5.19-5.29(m, 1H), 6.30-8.27(m, 14H), 11.04(d, 1H), 11.68(s, 1H)

### <Example 181> Preparation of (S)-4-((1-(4-chloro-1-oxo-2-phenyl-8-(6-(trifluoromethyl)pyridin-3-yl)-1,2-dihydroisoquinolin-3-yl)ethyl)amino)pyrido[2,3-d]pyrimidin-5(8H)-one

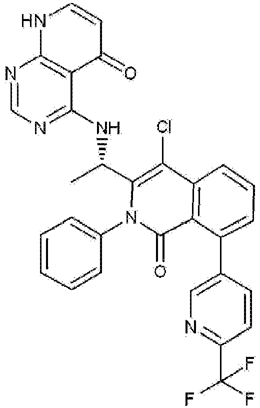

¹H-NMR (400MHz, CDCl₃) δ ppm 1.69(d, 3H), 5.00-5.07(m, 1H), 6.31-8.60(m, 14H), 11.06(d, 1H), 11.79(s, 1H)

### <Example 182> Preparation of (S)-4-((1-(4-chloro-8-(2-isopropoxypyrimidin-5-yl)-1-oxo-2-phenyl-1,2-dihydroisoquinolin-3-yl)ethyl)amino)pyrido[2,3-d]pyrimidin-5(8H)-one

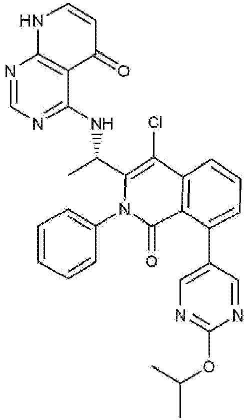

¹H-NMR (400MHz, CDCl₃) δ ppm 1.35-1.38(m, 6H), 1.70(d, 3H), 4.99-5.06(m, 1H), 5.19-5.28(m, 1H), 6.30-8.44(m, 13H), 11.04(d, 1H)

### <Example 183> Preparation of (S)-4-((1-(4-chloro-8-(6-ethoxypyridin-3-yl)-1-oxo-2-phenyl-1,2-dihydroisoquinolin-3-yl)ethyl)amino)pyrido[2,3-d]pyrimidin-5(8H)-one

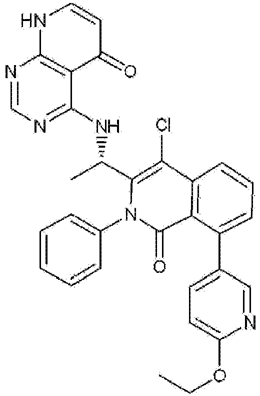

¹H-NMR (400MHz, DMSO-d₆) δ ppm 1.30(t, 3H), 1.60(d, 3H), 4.27(q, 2H), 4.86(m, 1H), 6.13(d, 1H), 6.68(d, 1H), 7.30-8.10(m, 11H), 8.32(s, 1H), 10.92(br, 1H)

### <Example 184> Preparation of (S)-4-((1-(4-chloro-1-oxo-2-phenyl-8-(2-(2,2,2-trifluoroethoxy)pyrimidin-5-yl)-1,2-dihydroisoquinolin-3-yl)ethyl)amino)pyrido[2,3-d]pyrimidin-5(8H)-one

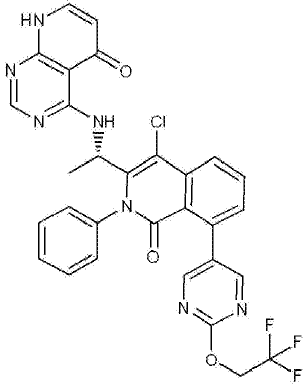

¹H-NMR (400MHz, DMSO-d₆) δ ppm 1.60(d, 3H), 4.90 (m, 1H), 5.03(q, 2H), 6.13(d, 1H), 7.30-7.80(m, 7H), 7.92(t, 1H), 8.12(d, 1H), 8.32(s, 1H), 8.54(s, 1H), 10.95(br), 12.13(s, 1H)

### <Example 185> Preparation of (S)-4-((1-(4-chloro-1-oxo-8-(6-phenoxypyridin-3-yl)-2-phenyl-1,2-dihydroisoquinolin-3-yl)ethyl)amino)pyrido[2,3-d]pyrimidin-5(8H)-one

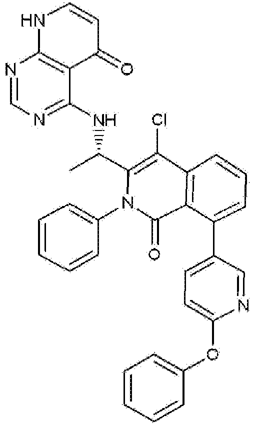

¹H-NMR (400MHz, CDCl₃) δ ppm 1.68(d, 3H), 5.04(m, 1H), 6.30(d, 1H), 6.80(d, 1H), 7.10-7.80 (m, 14H), 8.05(s, 1H), 8.11(d, 1H), 8.25(s, 1H), 11.00(br, 1H), 11.40(br, 1H)

### <Example 186> Preparation of (S)-4-((1-(4-chloro-8-(5-fluoro-6-methoxypyridin-3-yl)-1-oxo-2-phenyl-1,2-dihydroisoquinolin-3-yl)ethyl)amino)pyrido[2,3-d]pyrimidin-5(8H)-one

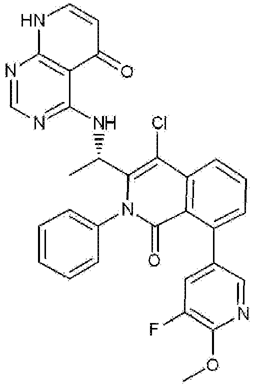

¹H-NMR (400MHz, CDCl₃) δ ppm 1.68(d, 3H), 3.98(s, 3H), 5.04(m, 1H), 6.31(d, 1H), 7.16(m, 1H), 7.27(m, 12H), 7.40-7.80(m, 7H), 8.14(d, 1H), 8.26(s, 1H), 11.05(br, 1H), 11.80(br, 1H)

### <Example 187> Preparation of (S)-4-((1-(4-chloro-8-(5-chloro-6-methoxypyridin-3-yl)-1-oxo-2-phenyl-1,2-dihydroisoquinolin-3-yl)ethyl)amino)pyrido[2,3-d]pyrimidin-5(8H)-one

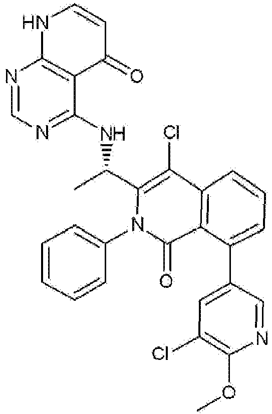

¹H-NMR (400MHz, CDCl₃) δ ppm 1.68(d, 3H), 3.98(s, 3H), 5.04(m, 1H), 6.31(d, 1H), 7.16(m, 1H), 7.27(d, 1H), 7.40-7.80(m, 7H), 7.91(s, 1H), 8.14(d, 1H), 8.26(s, 1H), 11.05(br, 1H), 11.80(br, 1H)

### <Example 188> Preparation of (S)-4-((1-(4-chloro-8-(2-ethoxypyrimidin-5-yl)-1-oxo-2-phenyl-1,2-dihydroisoquinolin-3-yl)ethyl)amino)pyrido[2,3-d]pyrimidin-5(8H)-one

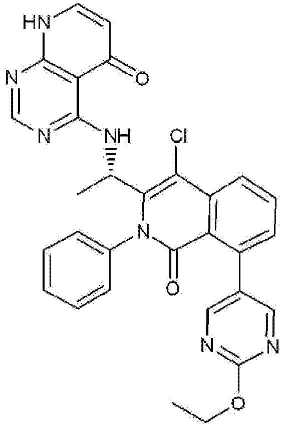

¹H-NMR (400MHz, CDCl₃) δ ppm 1.40(t, 3H), 1.69(d, 3H), 4.39(q, 2H), 5.02(m, 1H), 6.31(d, 1H), 7.14(m, 1H), 7.27(d, 1H), 7.40-7.80(m, 6H), 8.15(d, 1H), 8.28(s, 1H), 8.45(s, 2H), 11.05(br, 1H), 12.02(br, 1H)

### <Example 189> Preparation of (S)-4-((1-(4-chloro-8-(2,4-dimethoxypyrimidin-5-yl)-1-oxo-2-phenyl-1,2-dihydroisoquinolin-3-yl)ethyl)amino)pyrido[2,3-d]pyrimidin-5(8H)-one

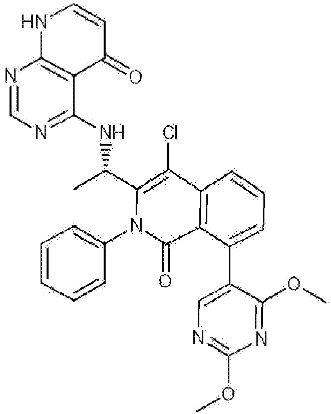

¹H-NMR (400MHz, CDCl₃) δ ppm 1.68(d, 3H), 3.84(d, 3H), 3.95(s, 3H), 5.05(m, 1H), 6.30(d, 1H), 7.15(m, 1H), 7.27(m, 1H), 7.40-7.80(m, 6H), 8.05(m, 1H), 8.12(d, 1H), 8.25(s, 1H), 10.95(br), 11.05(br)

### <Example 190> Preparation of (S)-4-((1-(4-chloro-8-(2-morpholinopyridin-4-yl)-1-oxo-2-phenyl-1,2-dihydroisoquinolin-3-yl)ethyl)amino)pyrido[2,3-d]pyrimidin-5(8H)-one

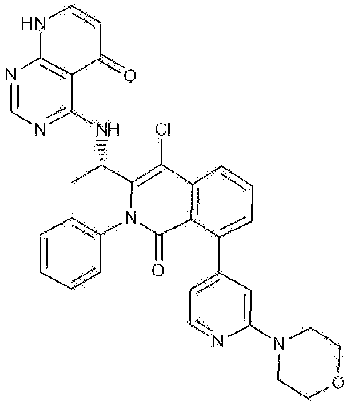

¹H-NMR (400MHz, CDCl₃) δ ppm 1.68(d, 3H), 3.43-3.76(m, 8H), 5.02-5.09(m, 1H), 6.28-8.25(m, 14H), 11.05(d, 1H), 11.76(s, 1H)

### <Example 191> Preparation of (S)-4-((1-(4-chloro-8-(3-fluoro-4-methoxyphenyl)-1-oxo-2-phenyl-1,2-dihydroisoquinolin-3-yl)ethyl)amino)pyrido[2,3-d]pyrimidin-5(8H)-one

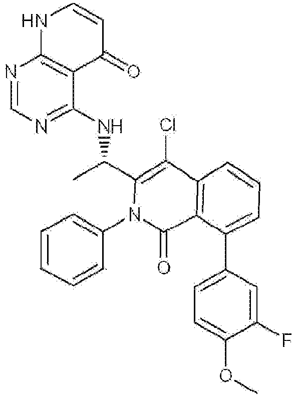

¹H-NMR (400MHz, CDCl₃) δ ppm 1.67(d, 3H), 3.83(s, 3H), 5.01-5.08(m, 1H), 6.30-8.26(m, 14H), 11.05(d, 1H), 11.84(s, 1H)

### <Example 192> Preparation of (S)-4-((1-(4-chloro-8-(8-fluoro-3,4-dihydro-2H-benzo[b][1,4]oxazin-6-yl)-1-oxo-2-phenyl-1,2-dihydroisoquinolin-3-yl) ethyl)amino)pyrido[2,3-d]pyrimidin-5(8H)-one

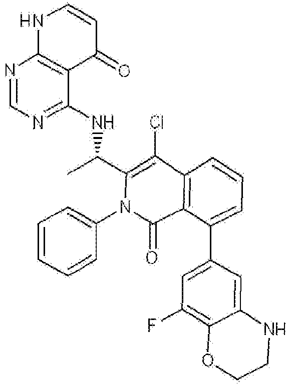

¹H-NMR (400MHz, CD₃OD) δ ppm 1.66(d, 3H), 3.46(t, 2H), 4.31(t, 2H), 5.03-5.05(m, 1H), 6.25-8.25(m, 12H), 11.00(br, 1H), 11.23(br, 1H)

### <Example 193> Preparation of (S)-4-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-N-isopropylbenzamide

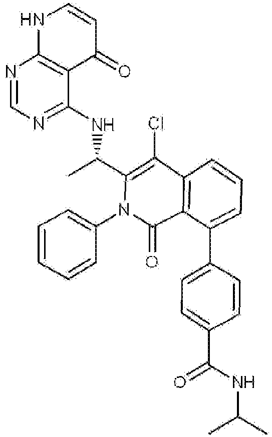

¹H-NMR (400MHz, CD₃OD) δ ppm 1.20(d, 6H), 1.66(d, 3H), 4.15-4.28(m, 1H), 5.02-5.05(m, 1H), 6.23-8.24(m, 14H), 10.98(br, 1H), 11.83(br, 1H)

### <Example 194> Preparation of (S)-4-((1-(4-chloro-1-oxo-2-phenyl-8-(6-(piperidin-1-yl)pyridin-3-yl)-1,2-dihydroisoquinolin-3-yl)ethyl)amino)pyrido[2,3-d]pyrimidin-5(8H)-one

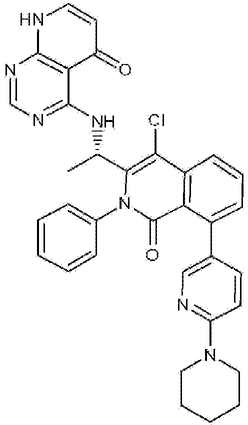

¹H-NMR (400MHz, CD₃OD) δ ppm 1.62-1.69 (m, 9H), 3.61-3.64 (m, 4H), 5.05-5.07(m, 1H), 6.25-8.15(m, 13H), 10.88(br, 1H)

### <Example 195> Preparation of (S)-4-((1-(4-chloro-8-(2-fluoropyridin-4-yl)-1-oxo-2-phenyl-1,2-dihydroisoquinolin-3-yl)ethyl)amino)pyrido[2,3-d]pyrimidin-5(8H)-one

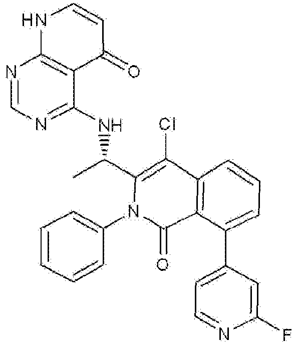

¹H-NMR (400MHz, CDCl₃) δ ppm 1.68(d, 3H), 5.02-5.09(m, 1H), 6.31-8.26(m, 14H), 11.05(d, 1H), 11.82(s, 1H)

### <Example 196> Preparation of (S)-5-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-N-methylpicolinamide

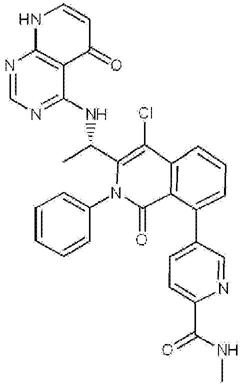

¹H-NMR (400MHz, CD₃OD) δ ppm 1.68(d, 3H), 2.97(d, 3H), 5.03-5.05(m, 1H), 6.29-8.39(m, 13H), 11.06(br, 1H), 11.90(br, 1H)

### <Example 197> Preparation of (S)-4-((1-(4-chloro-8-(6-methoxy-5-methylpyridin-3-yl)-1-oxo-2-phenyl-1,2-dihydroisoquinolin-3-yl)ethyl)amino)pyrido[2,3-d]pyrimidin-5(8H)-one

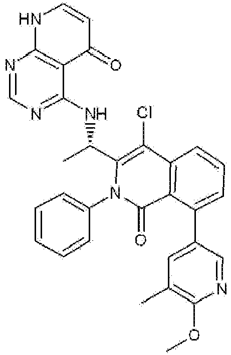

¹H-NMR (400MHz, CDCl₃) δ ppm 1.68(d, 3H), 2.12(s, 3H), 3.91(s, 3H), 5.02-5.08(m, 1H), 6.29-8.25(m, 13H), 11.04(d, 1H), 11.81(s, 1H)

### <Example 198> Preparation of (S)-4-((1-(4-chloro-1-oxo-2-phenyl-8-(pyrazin-2-yl)-1,2-dihydroisoquinolin-3-yl)ethyl)amino)pyrido[2,3-d]pyrimidin-5(8H)-one

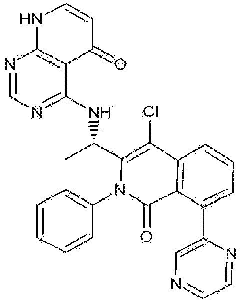

¹H-NMR (400MHz, CDCl₃) δ ppm 1.68(d, 3H), 5.01-5.08(m, 1H), 6.30-8.26(m, 14H), 10.97(d, 1H), 11.55(s, 1H)

### <Example 199> Preparation of (S)-4-((1-(4-chloro-8-(3-ethoxy-2-fluoro-4-methoxyphenyl)-1-oxo-2-phenyl-1,2-dihydroisoquinolin-3-yl)ethyl)amino)pyrido[2,3-d]pyrimidin-5(8H)-one

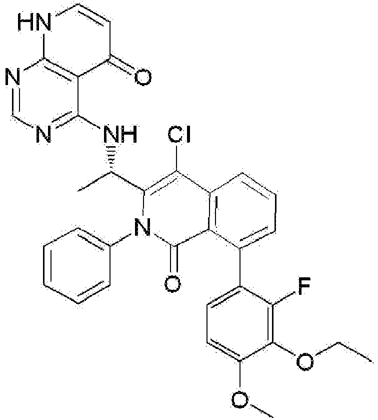

¹H-NMR (400MHz, CDCl₃) δ ppm 1.26(m, 3H), 1.67(m, 3H), 3.80(d, 3H), 4.06(m, 2H), 5.05(m, 1H), 6.30(d, 1H), 6.63(m, 1H), 6.89(m, 1H), 7.10-7.85(m, 8H), 8.08(t, 1H), 8.27(d, 1H), 11.05(br, 1H), 11.75(br, 1H)

### <Example 200> Preparation of (S)-4-((1-(8-(2-(azetidin-1-yl)pyrimidin-5-yl)-4-chloro-1-oxo-2-phenyl-1,2-dihydroisoquinolin-3-yl)ethyl)amino)pyrido[2,3-d]pyrimidin-5(8H)-one

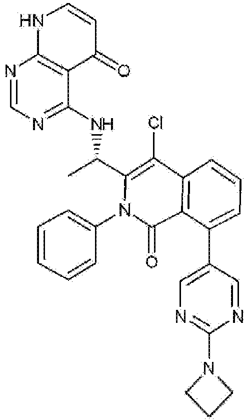

¹H-NMR (400MHz, CDCl₃) δ ppm 1.68(d, 3H), 2.31-2.43(m, 2H), 4.12-4.19(m, 4H), 5.04-5.09(m, 1H), 6.30-8.31(m, 13H), 11.00(d, 1H)

### <Example 201> Preparation of (S)-5-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)picolinamide

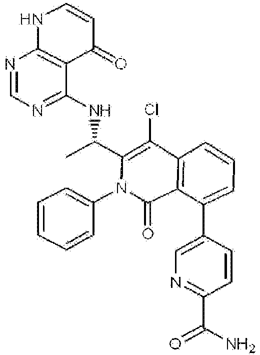

¹H-NMR (400MHz, CDCl₃) δ ppm 1.68(d, 3H), 5.00-5.07 (m, 1H), 5.97-8.46(m, 16H), 10.62(s, 1H), 10.98(d, 1H)

### <Example 202> Preparation of (S)-N-(5-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)pyridin-2-yl)acetamide

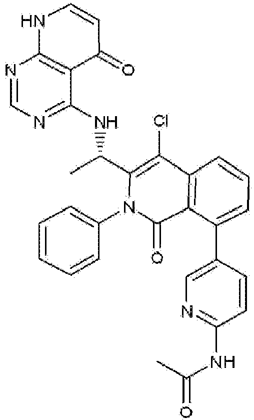

¹H-NMR (400MHz, CD₃OD) δ ppm 1.68(d, 3H), 2.19(s, 3H), 3.39(s, 1H), 5.02-5.09(m, 1H), 6.29-8.23(m, 14H), 10.95(d, 1H)

### <Example 203> Preparation of (S)-4-((1-(4-chloro-8-(2-(cyclopropylamino)pyrimidin-5-yl)-1-oxo-2-phenyl-1,2-dihydroisoquinolin-3-yl)ethyl)amino)pyrido[2,3-d]pyrimidin-5(8H)-one

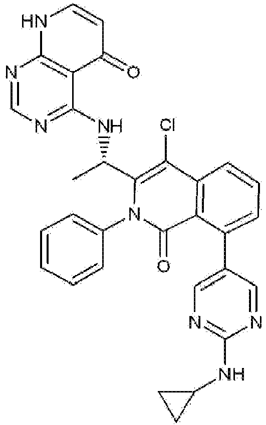

¹H-NMR (400MHz, CDCl₃) δ ppm 0.52-0.83(m, 4H), 1.68(d, 3H), 2.72-2.77(m, 1H), 5.02-5.09(m, 1H), 5.99-8.28(m, 14H), 11.03(d, 1H), 11.90(s, 1H)

### <Example 204> Preparation of (S)-4-((1-(4-chloro-8-(6-methoxypyrazin-2-yl)-1-oxo-2-phenyl-1,2-dihydroisoquinolin-3-yl)ethyl)amino)pyrido[2,3-d]pyrimidin-5(8H)-one

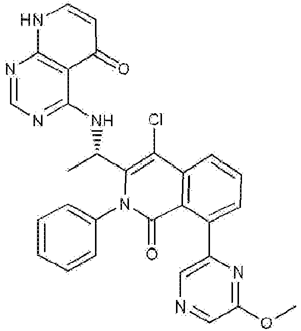

¹H-NMR (400MHz, CDCl₃) δ ppm 1.68(d, 3H), 3.88(s, 3H), 5.01-5.08(m, 1H), 6.30-8.25(m, 13H), 11.03(d, 1H)

### <Example 205> Preparation of (S)-4-((1-(4-chloro-8-(5-(dimethylamino)pyrazin-2-yl)-1-oxo-2-phenyl-1,2-dihydroisoquinolin-3-yl)ethyl)amino)pyrido[2,3-d]pyrimidin-5(8H)-one

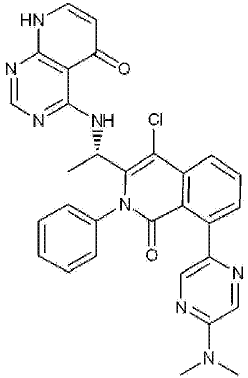

¹H-NMR (400MHz, CDCl₃) δ ppm 1.66(d, 3H), 3.09(s, 6H), 4.99-5.06(m, 1H), 6.29-8.24(m, 13H), 10.98(d, 1H)

### <Example 206> Preparation of (S)-4-((1-(8-(5-(tert-butoxy)pyrazin-2-yl)-4-chloro-1-oxo-2-phenyl-1,2-dihydroisoquinolin-3-yl)ethyl)amino)pyrido[2,3-d]pyrimidin-5(8H)-one

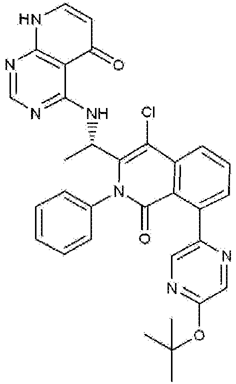

¹H-NMR (400MHz, CDCl₃) δ ppm 1.56(s, 9H), 1.66(d, 3H), 4.97-5.04(m, 1H), 6.29-8.26(m, 13H), 10.99(d, 1H)

### <Example 207> Preparation of (S)-4-((1-(4-chloro-8-(5-morpholinopyrazin-2-yl)-1-oxo-2-phenyl-1,2-dihydroisoquinolin-3-yl)ethyl)amino)pyrido[2,3-d]pyrimidin-5(8H)-one

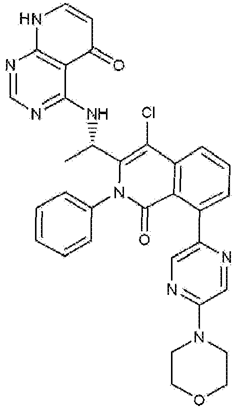

¹H-NMR (400MHz, CDCl₃) δ ppm 1.66(d, 3H), 3.53(t, 4H), 3.80(t, 4H), 4.98-5.05(m, 1H), 6.29-8.25(m, 13H), 10.98(s, 1H), 11.16(s, 1H)

### <Example 208> Preparation of (S)-4-((1-(8-(2-(tert-butyl)pyrimidin-5-yl)-4-chloro-1-oxo-2-phenyl-1,2-dihydroisoquinolin-3-yl)ethyl)amino)pyrido[2,3-d]pyrimidin-5(8H)-one

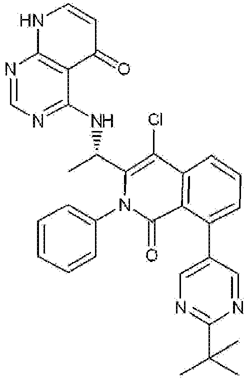

¹H-NMR (400MHz, CDCl₃) δ ppm 1.39(s, 9H), 1.69(d, 3H), 5.03-5.06(m, 1H), 6.31-8.59(m, 13H), 11.03(br, 1H), 11.56(br, 1H)

### <Example 209> Preparation of (S)-4-((1-(4-chloro-8-(4-(isopropylsulfonyl)phenyl)-1-oxo-2-phenyl-1,2-dihydroisoquinolin-3-yl)ethyl)amino)pyrido[2,3-d]pyrimidin-5(8H)-one

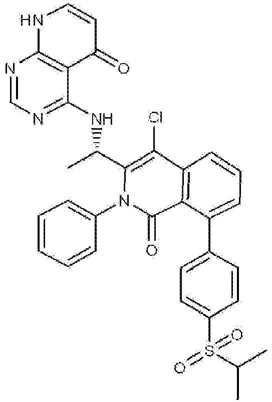

¹H-NMR (400MHz, CDCl₃) δ ppm 1.28(d, 6H), 1.70(d, 3H), 3.15(m, 1H), 5.05(m, 1H), 6.31(br, 1H), 7.15(m, 1H), 7.25(m, 1H), 7.45-7.80(m, 10H), 8.15(m, 1H), 8.28(br, 1H), 11.10(br, 1H)

### <Example 210> Preparation of (S)-4-((1-(4-chloro-8-(6-morpholinopyrazin-2-yl)-1-oxo-2-phenyl-1,2-dihydroisoquinolin-3-yl)ethyl)amino)pyrido[2,3-d]pyrimidin-5(8H)-one

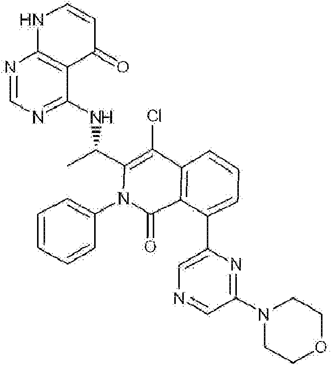

¹H-NMR (400MHz, CDCl₃) δ ppm 1.68(d, 3H), 3.51(t, 4H), 3.77(t, 4H), 5.02-5.06(m, 1H), 6.30-8.25(m, 13H), 11.00(s, 1H), 11.22(s, 1H)

### <Example 211> Preparation of (S)-4-((1-(4-chloro-8-(5-(4-methylpiperazin-1-yl)pyrazin-2-yl)-1-oxo-2-phenyl-1,2-dihydroisoquinolin-3-yl)ethyl)amino)pyrido[2,3-d]pyrimidin-5(8H)-one

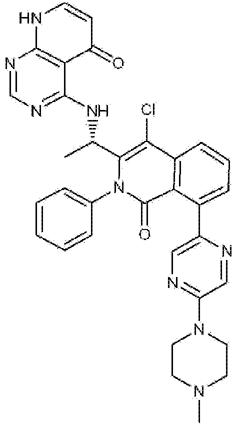

¹H-NMR (400MHz, CDCl₃) δ ppm 1.65(d, 3H), 2.38(s, 3H), 2.57(t, 4H), 3.63(t, 4H), 5.00-5.03(m, 1H), 6.28-8.23(m, 13H), 11.05(s, 1H)

### <Example 212> Preparation of (S)-4-((1-(8-(2-amino-4-methylpyrimidin-5-yl)-4-chloro-1-oxo-2-phenyl-1,2-dihydroisoquinolin-3-yl)ethyl)amino)pyrido[2,3-d]pyrimidin-5(8H)-one

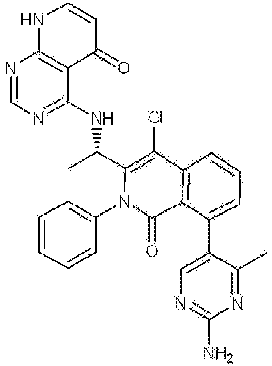

¹H-NMR (400MHz, CDCl₃) δ ppm 1.67(m, 3H), 2.08(s, 3H), 5.05(m, 1H), 6.29(d, 1H), 7.15(m, 1H), 7.23(m, 1H), 7.45-7.65(m, 5H), 7.75(m, 1H), 7.95(d, 1H), 8.12(m, 1H), 8.22(d, 1H), 10.95(br, 1H)

### <Example 213> Preparation of (S)-4-((1-(4-chloro-1-oxo-2-phenyl-8-(quinolin-3-yl)-1,2-dihydroisoquinolin-3-yl)ethyl)amino)pyrido[2,3-d]pyrimidin-5(8H)-one

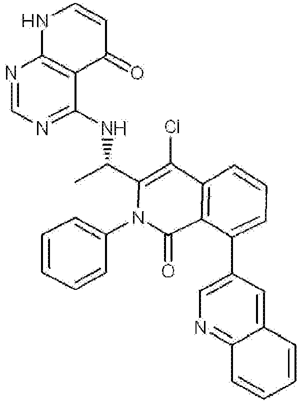

¹H-NMR (400MHz, CDCl₃) δ ppm 1.68(m, 3H), 5.05(m, 1H), 6.28(d, 1H), 7.15(br, 1H), 7.35-7.55(m, 6H), 7.60-8.30(m, 8H), 8.80(s, 1H), 11.00(br, 1H)

### <Example 214> Preparation of (S)-4-((1-(4-chloro-8-(2-(methylthio)pyrimidin-5-yl)-1-oxo-2-phenyl-1,2-dihydroisoquinolin-3-yl)ethyl)amino)pyrido[2,3-d]pyrimidin-5(8H)-one

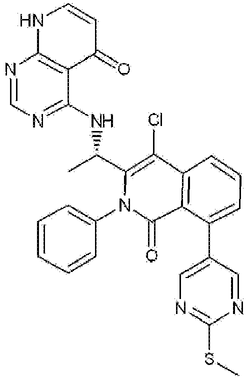

¹H-NMR (400MHz, CDCl₃) δ ppm 1.69(d, 3H), 2.52(s, 3H), 5.04(m, 1H), 6.32(d, 1H), 7.15(m, 1H), 7.28(m, 1H), 7.45-7.80(m, 6H), 8.18(d, 1H), 8.28(s, 1H), 8.45(s, 1H), 11.02(br, 1H), 11.70(br, 1H)

### <Example 215> Preparation of (S)-4-((1-(4-chloro-8-(4-isopropylpyrimidin-5-yl)-1-oxo-2-phenyl-1,2-dihydroisoquinolin-3-yl)ethyl)amino)pyrido[2,3-d]pyrimidin-5(8H)-one

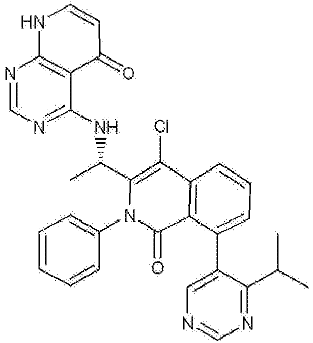

¹H-NMR (400MHz, CDCl₃) δ ppm 1.01(d, 3H), 1.18(d, 3H), 1.69(d, 3H), 2.70(s, 1H), 5.07-5.01(m, 1H), 6.30-9.05(m, 13H), 10.11(s, 1H), 11.01(s, 1H)

### <Example 216> Preparation of (S)-4-((1-(4-chloro-8-(2-(4-methylpiperazin-1-yl)pyrimidin-5-yl)-1-oxo-2-phenyl-1,2-dihydroisoquinolin-3-yl)ethyl)amino)pyrido[2,3-d]pyrimidin-5(8H)-one

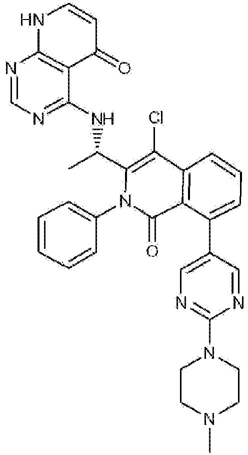

¹H-NMR (400MHz, CDCl₃) δ ppm 1.67(d, 3H), 2.35(s, 3H), 2.37(t, 4H), 3.85(t, 4H), 5.03-5.05(m, 1H), 6.29-8.25(m, 13H), 11.03(s, 1H), 11.78(s, 1H)

### <Example 217> Preparation of (S)-4-((1-(4-chloro-8-(2-(diethylamino)pyrimidin-5-yl)-1-oxo-2-phenyl-1,2-dihydroisoquinolin-3-yl)ethyl)amino)pyrido[2,3-d]pyrimidin-5(8H)-one

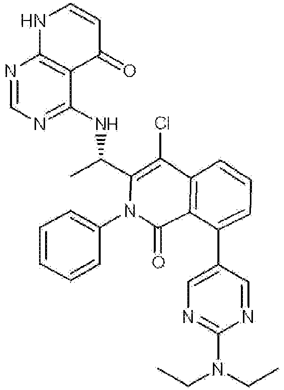

¹H-NMR (400MHz, CDCl₃) δ ppm 1.15(t, 6H), 1.68(d, 3H), 3.54-3.60(m, 4H), 5.02-5.05(m, 1H), 6.29-8.25(m, 13H), 11.04(s, 1H)

### <Example 218> Preparation of (S)-4-((1-(4-chloro-8-(5-(methylsulfonyl)pyridin-3-yl)-1-oxo-2-phenyl-1,2-dihydroisoquinolin-3-yl)ethyl)amino)pyrido[2,3-d]pyrimidin-5(8H)-one

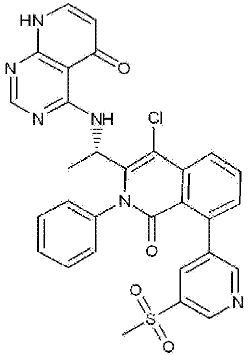

¹H-NMR (400MHz, CDCl₃) δ ppm 1.69(d, 3H), 3.06(s, 3H), 5.02(m, 1H), 6.31(d, 1H), 7.15(m, 1H), 7.30(m, 1H), 7.45-7.60(m, 4H), 7.65(m, 1H), 7.80(t, 1H), 8.10(s, 1H), 8.20(d, 1H), 8.28(s, 1H), 8.78(s, 1H), 8.99(s, 1H), 10.60(br, 1H), 10.95(br, 1H)

### <Example 219> Preparation of (S)-4-((1-(4-chloro-8-(6-(methylsulfonyl)pyridin-3-yl)-1-oxo-2-phenyl-1,2-dihydroisoquinolin-3-yl)ethyl)amino)pyrido[2,3-d]pyrimidin-5(8H)-one

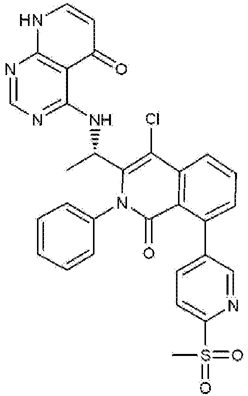

¹H-NMR (400MHz, CDCl₃) δ ppm 1.67(m, 3H), 3.21(s, 3H), 5.02(m, 1H), 6.31(d, 1H), 7.15(m, 1H), 7.25(m, 1H), 7.45-8.25(m, 9H), 8.27(s, 1H), 8.59(s, 1H), 11.05(br, 1H), 11.60(br, 1H)

### <Example 220> Preparation of (S)-4-((1-(4-chloro-8-(2-(isopropylamino)pyrimidin-5-yl)-1-oxo-2-phenyl-1,2-dihydroisoquinolin-3-yl)ethyl)amino)pyrido[2,3-d]pyrimidin-5(8H)-one

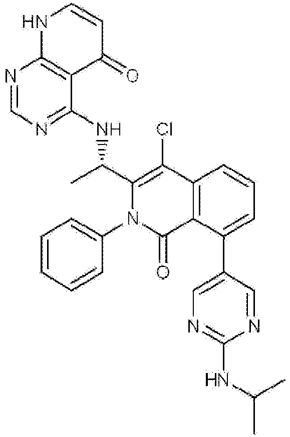

¹H-NMR (400MHz, CDCl₃) δ ppm 1.21(d, 6H), 1.68(d, 3H), 4.07-4.10(m, 1H), 5.04-5.07(m, 1H), 5.69(q, 1H), 5.69(d, 1H), 6.27-8.24(m, 13H), 11.04(s, 1H), 12.24(s, 1H)

### <Example 221> Preparation of (S)-4-((1-(4-chloro-8-(5-methoxypyrazin-2-yl)-1-oxo-2-phenyl-1,2-dihydroisoquinolin-3-yl)ethyl)amino)pyrido[2,3-d]pyrimidin-5(8H)-one

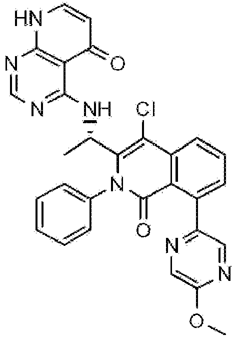

¹H-NMR (400MHz, CD₃OD) δ ppm 1.67(d, 3H), 3.93(s, 3H), 5.01-5.05(m, 1H), 6.28-8.25(m, 12H), 11.00(br, 1H), 11.73(br, 1H)

### <Example 222> Preparation of (S)-4-((1-(4-chloro-1-oxo-2-phenyl-8-(2-(pyrrolidin-1-yl)pyrimidin-5-yl)-1,2-dihydroisoquinolin-3-yl)ethyl)amino)pyrido[2,3-d]pyrimidin-5(8H)-one

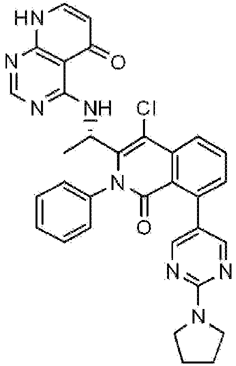

¹H-NMR (400MHz, CD₃OD) δ ppm 1.68(d, 3H), 1.94(t, 4H), 3.53(t, 4H), 5.03-5.06(m, 1H), 6.28-8.26(m, 12H), 11.03(br, 1H), 11.99(br, 1H)

### <Example 223> Preparation of (S)-4-((1-(4-chloro-1-oxo-2-phenyl-8-(6-(trifluoromethoxy)pyridin-3-yl)-1,2-dihydroisoquinolin-3-yl)ethyl)amino)pyrido[2,3-d]pyrimidin-5(8H)-one

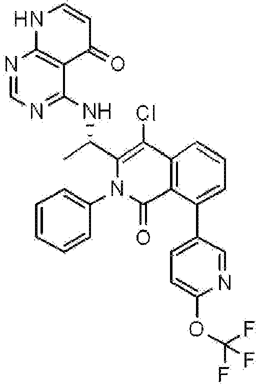

¹H-NMR (400MHz, CD₃OD) δ ppm 1.68(d, 3H), 5.02-5.04(m, 1H), 6.31-8.26(m, 13H), 11.06(br, 1H), 12.10(br, 1H)

### <Example 224> Preparation of (S)-4-((1-(4-chloro-1-oxo-2-phenyl-8-(2-(piperidin-1-yl)pyrimidin-5-yl)-1,2-dihydroisoquinolin-3-yl)ethyl)amino)pyrido[2,3-d]pyrimidin-5(8H)-one

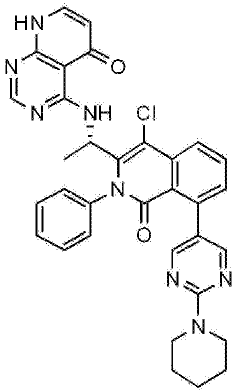

¹H-NMR (400MHz, CD₃OD) δ ppm 1.56-1.69(m, 9H), 3.72-3.74(m, 4H), 5.02-5.05(m, 1H), 6.29-8.24(m, 12H), 11.03(br, 1H), 11.88(br, 1H)

### <Example 225> Preparation of (S)-N-(5-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-2-methoxypyridin-3-yl)methanesulfonamide

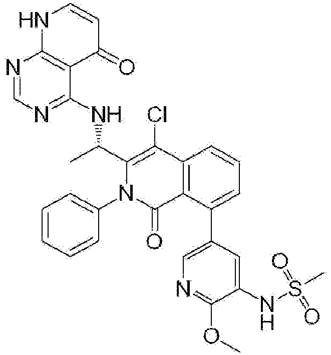

¹H-NMR (400MHz, CDCl₃) δ ppm 1.70(d, 3H), 2.85(s, 3H), 3.98(s, 3H), 5.00-5.07(m, 1H), 6.31-8.31(m, 13H), 11.01(d, 1H), 12.07(s, 1H)

### <Example 226> Preparation of (S)-4-((1-(4-chloro-1-oxo-2-phenyl-8-(1H-pyrazol-3-yl)-1,2-dihydroisoquinolin-3-yl)ethyl)amino)pyrido[2,3-d]pyrimidin-5(8H)-one

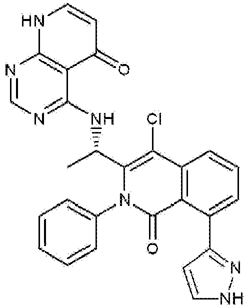

¹H-NMR (400MHz, CDCl₃) δ ppm 1.69(d, 3H), 4.93-5.00 (m, 1H), 6.30-8.42(m, 14H), 11.05(d, 1H)

### <Example 227> Preparation of (S)-4-((1-(4-chloro-1-oxo-2-phenyl-8-(1H-pyrazol-4-yl)-1,2-dihydroisoquinolin-3-yl)ethyl)amino)pyrido[2,3-d]pyrimidin-5(8H)-one

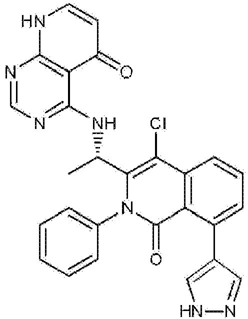

¹H-NMR (400MHz, CDCl₃) δ ppm 1.68(d, 3H), 5.01-5.08(m, 1H), 6.29-8.26(m, 14H), 10.95(d, 1H)

### <Example 228> Preparation of (S)-4-((1-(4-chloro-8-(2-(ethylamino)pyrimidin-5-yl)-1-oxo-2-phenyl-1,2-dihydroisoquinolin-3-yl)ethyl)amino)pyrido[2,3-d]pyrimidin-5(8H)-one

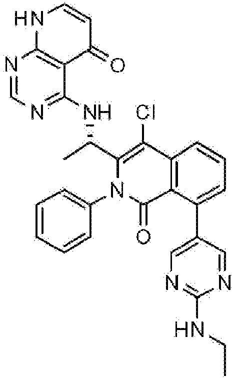

¹H-NMR (400MHz, CD₃OD) δ ppm 1.20-1.28(m, 3H), 1.68(d, 3H), 3.40-3.43(m, 2H), 5.01-5.04(m, 1H), 6.27-8.25(m, 12H), 11.02(br, 1H), 11.85(br, 1H)

### <Example 229> Preparation of (S)-5-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-2-methoxynicotinonitrile

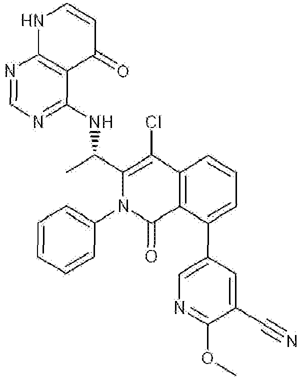

¹H-NMR (400MHz, CDCl₃) δ ppm 1.69(d, 3H), 4.02(s, 3H), 5.01-5.07(m, 1H), 6.32-8.28(m, 13H), 11.04(d, 1H), 11.62(s, 1H)

### <Example 230> Preparation of (S)-4-((1-(4-chloro-8-(5-ethoxypyrazin-2-yl)-1-oxo-2-phenyl-1,2-dihydroisoquinolin-3-yl)ethyl)amino)pyrido[2,3-d]pyrimidin-5(8H)-one

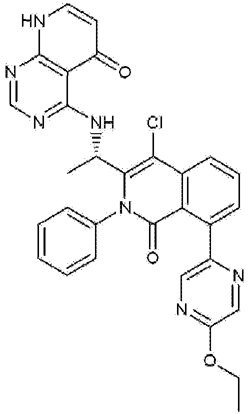

¹H-NMR (400MHz, CDCl₃) δ ppm 1.39(t, 3H), 1.66(d, 3H), 4.34(q, 2H), 4.99-5.03(m, 1H), 6.30-8.26(m, 13H), 10.45(s, 1H), 10.95(s, 1H)

### <Example 231> Preparation of (S)-4-((1-(4-chloro-8-(2-fluoro-4-(morpholinomethyl)phenyl)-1-oxo-2-phenyl-1,2-dihydroisoquinolin-3-yl)ethyl)amino)pyrido[2,3-d]pyrimidin-5(8H)-one

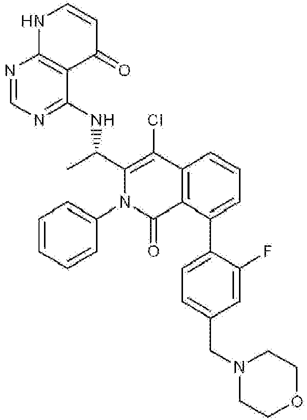

¹H-NMR (400MHz, CDCl₃) δ ppm 1.66(d, 3H), 2.47(t, 4H), 3.42(s, 2H), 3.67(t, 4H), 5.00-5.03(m, 1H), 6.28-8.25(m, 14H), 11.04(s, 1H), 11.85(s, 1H)

### <Example 232> Preparation of (S)-N-(5-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-2-methoxypyridin-3-yl)-2,4-difluorobenzenesulfonamide

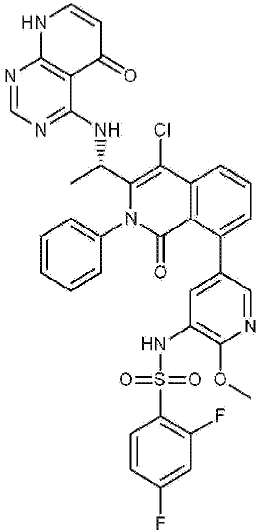

¹H-NMR (400MHz, CDCl₃) δ ppm 1.70(d, 3H), 3.83(s, 3H), 5.06-5.08(m, 1H), 6.21-8.32(m, 17H), 11.05(s, 1H), 11.50(s, 1H)

### <Example 233> Preparation of (S)-4-((1-(8-(benzo[c][1,2,5]thiadiazol-5-yl)-4-chloro-1-oxo-2-phenyl-1,2-dihydroisoquinolin-3-yl)ethyl)amino)pyrido[2,3-d]pyrimidin-5(8H)-one

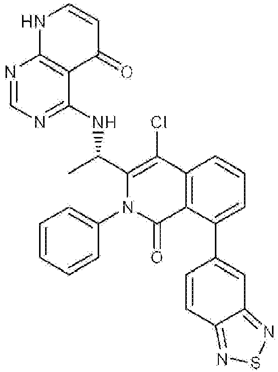

¹H-NMR (400MHz, CDCl₃) δ ppm 1.70(d, 3H), 5.01-5.08 (m, 1H), 6.30-8.26(m, 14H), 11.06(d, 1H), 11.68(s, 1H)

### <Example 234> Preparation of (S)-4-((1-(4-chloro-8-(5-methyl-1H-pyrazol-3-yl)-1-oxo-2-phenyl-1,2-dihydroisoquinolin-3-yl)ethyl)amino)pyrido[2,3-d]pyrimidin-5(8H)-one

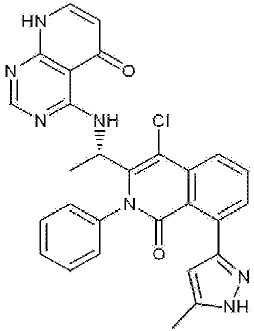

¹H-NMR (400MHz, CDCl₃) δ ppm 1.69(d, 3H), 2.32(s, 3H), 4.90-4.97(m, 1H), 6.25-8.46(m, 13H), 11.06(d, 1H)

### <Example 235> Preparation of (S)-4-((1-(8-([1,2,5]oxadiazolo[3,4-b]pyridin-6-yl)-4-chloro-1-oxo-2-phenyl-1,2-dihydroisoquinolin-3-yl)ethyl)amino)pyrido[2,3-d]pyrimidin-5(8H)-one

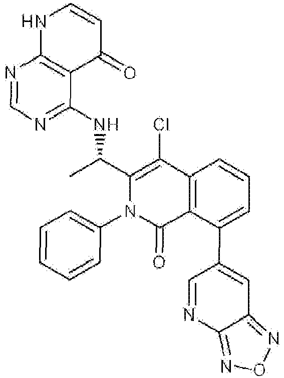

¹H-NMR (400MHz, CDCl₃) δ ppm 1.69(m, 3H), 5.02-5.09(m, 1H), 6.31-8.74(m, 13H), 10.98(d, 1H), 11.26(s, 1H)

### <Example 236> Preparation of (S)-4-((1-(4-chloro-8-(1H-indazol-5-yl)-1-oxo-2-phenyl-1,2-dihydroisoquinolin-3-yl)ethyl)amino)pyrido[2,3-d]pyrimidin-5(8H)-one

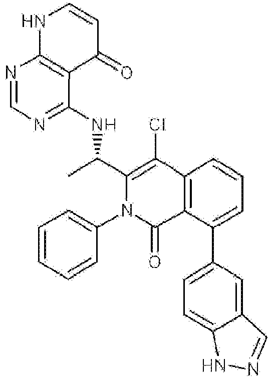

¹H-NMR (400MHz, CDCl₃) δ ppm 1.66(d, 3H), 4.94-5.01(m, 1H), 6.26-8.27(m, 16H), 10.99(d, 1H), 11.60(s, 1H)

### <Example 237> Preparation of (S)-4-((1-(4-chloro-8-(1-methyl-1H-indazol-4-yl)-1-oxo-2-phenyl-1,2-dihydroisoquinolin-3-yl)ethyl)amino)pyrido[2,3-d]pyrimidin-5(8H)-one

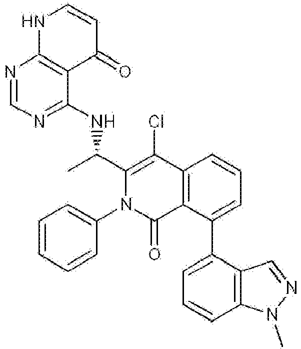

¹H-NMR (400MHz, CD₃OD) δ ppm 1.70(d, 3H), 3.99(s, 3H), 5.01-5.03(m, 1H), 6.26-8.35(m, 14H), 11.02(br, 1H), 11.80(br, 1H)

### <Example 238> Preparation of (S)-4-((1-(4-chloro-8-(isoxazol-4-yl)-1-oxo-2-phenyl-1,2-dihydroisoquinolin-3-yl)ethyl)amino)pyrido[2,3-d]pyrimidin-5(8H)-one

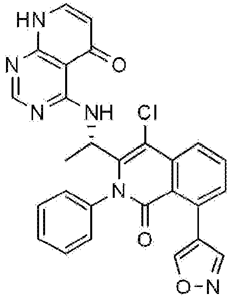

¹H-NMR (400MHz, CD₃OD) δ ppm 1.69(d, 3H), 5.02-5.05(m, 1H), 6.30-9.84(m, 12H), 11.01(br, 1H)

### <Example 239> Preparation of (S)-4-((1-(4-chloro-8-(1H-indazol-7-yl)-1-oxo-2-phenyl-1,2-dihydroisoquinolin-3-yl)ethyl)amino)pyrido[2,3-d]pyrimidin-5(8H)-one

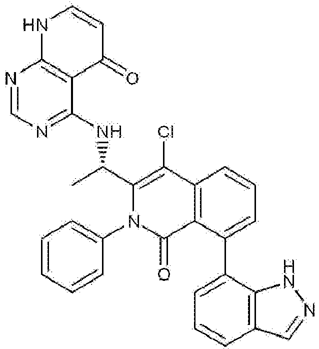

¹H-NMR (400MHz, CD₃OD) δ ppm 1.70(d, 3H), 4.88-4.90(m, 1H), 6.14-8.17(m, 14H), 10.95(br, 1H)

### <Example 240> Preparation of (S)-4-((1-(8-(benzo[c] [1,2,5]oxadiazol-5-yl)-4-chloro-1-oxo-2-phenyl-1,2-dihydroisoquinolin-3-yl)ethyl)amino)pyrido[2,3-d]pyrimidin-5(8H)-one

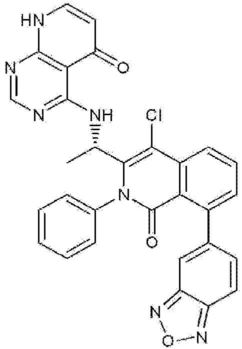

¹H-NMR (400MHz, CDCl₃) δ ppm 1.72(d, 3H), 5.06 (br, 1H), 6.32-8.28(m, 14H), 11.07(s, 1H), 11.66(s, 1H)

### <Example 241> Preparation of (S)-4-((1-(8-(benzo[d]oxazol-5-yl)-4-chloro-1-oxo-2-phenyl-1,2-dihydroisoquinolin-3-yl)ethyl)amino)pyrido[2,3-d]pyrimidin-5(8H)-one

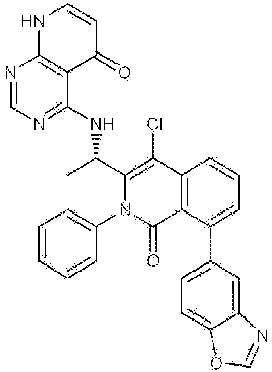

¹H-NMR (400MHz, CDCl₃) δ ppm 1.68(d, 3H), 4.98-5.01(m, 1H), 6.29-8.27(m, 15H), 10.61(s, 1H), 10.94(s, 1H)

### <Example 242> Preparation of (S)-4-((1-(4-chloro-8-(4-methylthiophen-2-yl)-1-oxo-2-phenyl-1,2-dihydroisoquinolin-3-yl)ethyl)amino)pyrido[2,3-d]pyrimidin-5(8H)-one

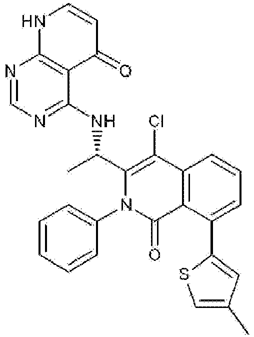

¹H-NMR (400MHz, CDCl₃) δ ppm 1.66(d, 3H), 2.19(s, 3H), 5.03(m, 1H), 6.31(d, 1H), 6.74(s, 1H), 6.83(s, 1H), 7.18(m, 1H), 7.40-7.55(m, 5H), 7.67(m, 2H), 8.18(d, 1H), 8.25(s, 1H), 11.02(br, 1H), 11.60(br, 1H)

### <Example 243> Preparation of (S)-4-((1-(4-chloro-8-(3-methylthiophen-2-yl)-1-oxo-2-phenyl-1,2-dihydroisoquinolin-3-yl)ethyl)amino)pyrido[2,3-d]pyrimidin-5(8H)-one

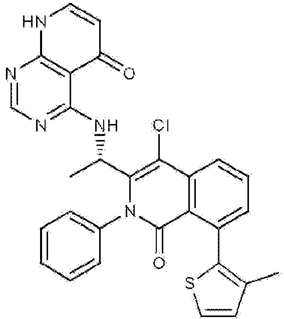

¹H-NMR (400MHz, CDCl₃) δ ppm 1.66(d, 3H), 2.03(s, 3H), 5.06(m, 1H), 6.32(d, 1H), 6.81(d, 1H), 7.17(d, 1H), 7.18(m, 1H), 7.40-7.75(m, 7H), 8.12(d, 1H), 8.25(s, 1H), 11.02(br, 1H)

### <Example 244> Preparation of (S)-4-((1-(4-chloro-8-(4-methyl-1H-pyrazol-3-yl)-1-oxo-2-phenyl-1,2-dihydroisoquinolin-3-yl)ethyl)amino)pyrido[2,3-d]pyrimidin-5(8H)-one

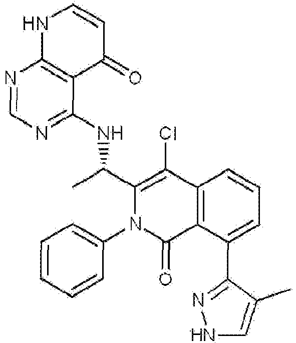

¹H-NMR (400MHz, CDCl₃) δ ppm 1.66(d, 3H), 2.02(s, 3H), 5.06(m, 1H), 6.26(d, 1H), 7.12(d, 1H), 7.35-7.56(m, 6H), 7.72(t, 1H), 7.88(m, 1H), 8.10(d, 1H), 8.36(s, 1H), 11.05(br, 1H)

### <Example 245> Preparation of (S)-4-((1-(4-chloro-8-(morpholinomethyl)-1-oxo-2-phenyl-1,2-dihydroisoquinolin-3-yl)ethyl)amino)pyrido[2,3-d]pyrimidin-5(8H)-one

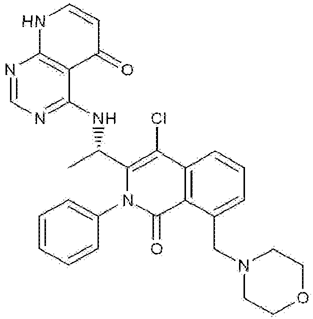

¹H-NMR (400MHz, CD₃OD) δ ppm 1.68(d, 3H), 2.55-2.65(m, 2H), 3.70-3.93(m, 3H) 5.02-5.15(m, 1H), 6.25-8.22(m, 11H), 10.95(br, 1H), 11.90(br, 1H)

### <Example 246> Preparation of (S)-4-((1-(4-chloro-8-(6-(methylamino)pyridin-3-yl)-1-oxo-2-phenyl-1,2-dihydroisoquinolin-3-yl)ethyl)amino)pyrido[2,3-d]pyrimidin-5(8H)-one

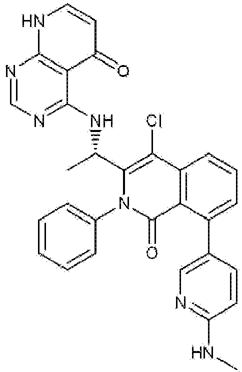

¹H-NMR (400MHz, CDCl₃) δ ppm 1.67 (d, 3H), 2.92(d, 3H), 5.03-5.06(m, 1H), 6.28-8.25(m, 14H), 10.90(s, 1H)

### <Example 247> Preparation of (S)-4-((1-(4-chloro-1-oxo-2-phenyl-8-(4-(trifluoromethoxy)phenyl)-1,2-dihydroisoquinolin-3-yl)ethyl)amino)pyrido[2,3-d]pyrimidin-5(8H)-one

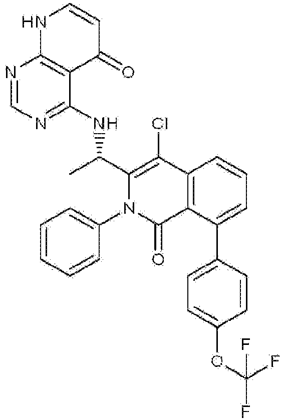

¹H-NMR (400MHz, CDCl₃) δ ppm 1.67 (d, 3H), 2.92(d, 3H), 5.01-5.08(m, 1H), 6.28-8.23(m, 15H), 11.06(s, 1H), 11.93(s, 1H)

### <Example 248> Preparation of (S)-4-((1-(4-chloro-8-(2,6-dimethylpyridin-3-yl)-1-oxo-2-phenyl-1,2-dihydroisoquinolin-3-yl)ethyl)amino)pyrido[2,3-d]pyrimidin-5(8H)-one

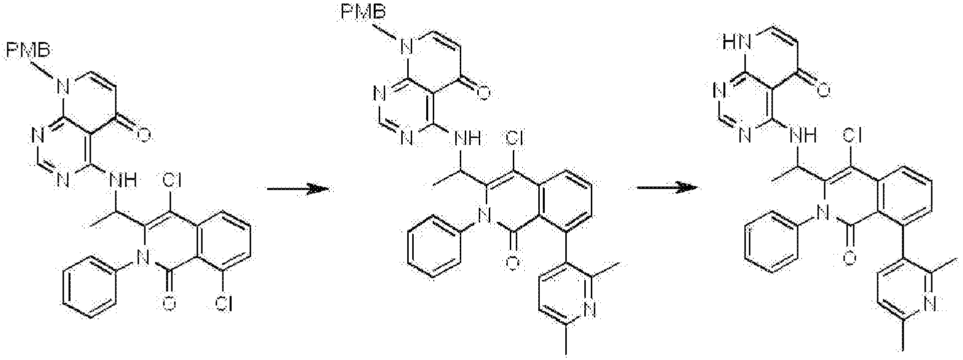

### [Step 1] Preparation of 4-((1-(4-chloro-8-(2,6-dimethylpyridin-3-yl)-1-oxo-2-phenyl-1,2-dihydroisoquinolin-3-yl)ethyl)amino)-8-(4-methoxybenzyl)pyrido[2,3-d]pyrimidin-5(8H)-one

The 80 mg of 4-((1-(4,8-dichloro-1-oxo-2-phenyl-1,2-dihydroisoquinolin-3-yl)ethyl)amino)-8-(4-methoxybenzyl)pyrido[2,3-d]pyrimidin-5(8H)-one prepared in above Preparation Example 3, 30 mg of 2,6-dimethyl-pyridin-3-boronic acid, 0.15 mL of 2M aqueous potassium carbonate solution, and 9 mg of bis(triphenylphosphin)palladium chloride were added to 2 mL of dioxane and stirred under reflux at 160°C for a day. Upon completion of the reaction, the resulting mixture was cooled down to room temperature, after which a saturated aqueous solution of sodium chloride was added, followed by extraction with ethyl acetate two to three times. An organic layer was dried over anhydrous sodium sulfate, and concentrated under reduced pressure, and then the resulting residues were purified by Prep-LC. The resulting product was vacuum-dried for three hours to obtain 10 mg of 4-((1-(4-chloro-8-(2,6-dimethylpyridin-3-yl)-1-oxo-2-phenyl-1,2-dihydroisoquinolin-3-yl)ethyl)amino)-8-(4-methoxybenzyl)pyrido [2,3-d]pyrimidin-5(8H)-one.

¹H-NMR (400 MHz, CDCl₃) δ ppm 1.67(d, 3H), 2.20 (s, 3H), 2.50 (s, 3H) 4.99-5.11(m, 1H), 5.30-5.33(m, 2H), 6.26-8.32(m, 17H), 11.13(s, 1H)

### [Step 2] Preparation of (S)-4-((1-(4-chloro-8-(2,6-dimethylpyridin-3-yl)-1-oxo-2-phenyl-1,2-dihydroisoquinolin-3-yl)ethyl)amino)pyrido[2,3-d]pyrimidin-5(8H)-one

The 10 mg of 4-((1-(4-chloro-8-(2,6-dimethylpyridin-3-yl)-1-oxo-2-phenyl-1,2-dihydroisoquinolin-3-yl) ethyl)amino)-8-(4-methoxybenzyl)pyrido[2,3-d]pyrimidin-5(8H)-one prepared in above step 1 and 1 mL of trifluoroacetic acid were added to 5 mL of methylene chloride and allowed to react for one day. After completion of the reaction, a saturated aqueous solution of sodium hydrogen carbonate was added, followed by extraction with ethyl acetate two to three times. An organic layer was dried over anhydrous sodium sulfate, and concentrated under reduced pressure, and then the resulting residues were purified by Prep-LC. The resulting product was vacuum-dried for three hours to obtain 1 mg of (S)-4-((1-(4-chloro-8-(2,6-dimethylpyridin-3-yl)-1-oxo-2-phenyl-1,2-dihydroisoquinolin-3-yl) ethyl)amino)pyrido[2,3-d]pyrimidin-5(8H)-one.

¹H-NMR (400MHz, CDCl₃) δ ppm 1.67(d, 3H), 2.19(s, 3H), 2.49(s, 3H), 5.02-5.08(m, 1H), 6.29-8.24(m, 13H), 10.19(s, 1H), 10.97(s, 1H)

### <Examples 249 to 264>

In Examples 249 to 264, the compound of each Example was prepared by substantially the same preparation method as described in above Example 248, except for using an appropriate reactant in consideration of the structure of the corresponding compound.

### <Example 249> Preparation of (S)-4-((1-(4-chloro-8-(dimethylamino)-1-oxo-2-phenyl-1,2-dihydroisoquinolin-3-yl)ethyl)amino)pyrido[2,3-d]pyrimidin-5(8H)-one

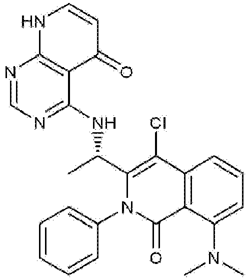

¹H-NMR (400MHz, CDCl₃) δ ppm 1.26(t, 3H), 2.35(s, 6H), 5.00(m, 1H), 6.31(d, 1H), 6.55(d, 1H), 7.07(d, 1H), 7.20(d, 1H), 7.50-7.75(m, 5H), 7.74(d(1H), 8.24(s, 1H), 9.02(t, 1H), 11.00(s, 1H)

### <Example 250> Preparation of (S)-4-((1-(4-chloro-8-(4-methylpiperazin-1-yl)-1-oxo-2-phenyl-1,2-dihydroisoquinolin-3-yl)ethyl)amino)pyrido[2,3-d]pyrimidin-5(8H)-one

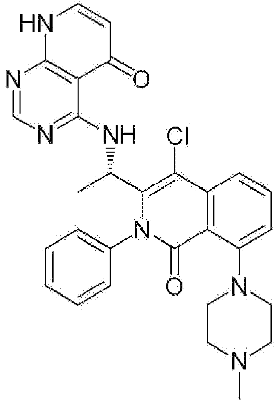

¹H-NMR (400MHz, CDCl₃) δ ppm 1.66(d, 3H), 3.22-3.41(m, 11H), 5.03-5.10(m, 1H), 6.31-8.22(m, 10H), 11.20(d, 1H)

### <Example 251> Preparation of (S)-4-((1-(4-chloro-8-morpholino-1-oxo-2-phenyl-1,2-dihydroisoquinolin-3-yl)ethyl)amino)pyrido[2,3-d]pyrimidin-5(8H)-one

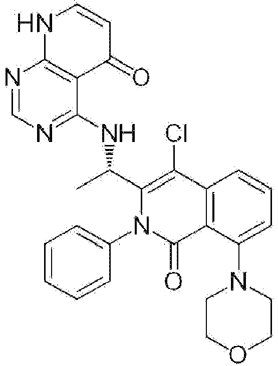

¹H-NMR (400MHz, CDCl₃) δ ppm 1.66(d, 3H), 3.22-3.85(m, 8H), 5.03-5.10(m, 1H), 6.31-8.22(m, 10H), 11.20(d, 1H)

### <Example 252> Preparation of (S)-4-((1-(4-chloro-1-oxo-2-phenyl-8-(pyrrolidin-1-yl)-1,2-dihydroisoquinolin-3-yl)ethyl)amino)pyrido[2,3-d]pyrimidin-5(8H)-one

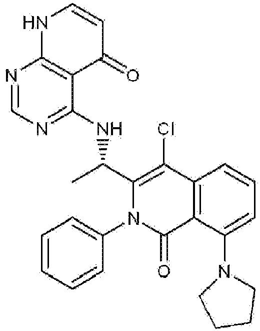

¹H-NMR (400MHz, CDCl₃) δ ppm 1.66(d, 3H), 1.80-2.50(m, 4H), 3.22-3.41(m, 4H), 5.03-5.10(m, 1H), 6.31-8.22(m, 10H), 11.20(d, 1H)

### <Example 253> Preparation of (S)-4-((1-(4-chloro-8-(2,3-dihydrobenzo[b] [1,4]dioxin-6-yl)-1-oxo-2-phenyl-1,2-dihydroisoquinolin-3-yl)ethyl)amino)pyrido[2,3-d]pyrimidin-5(8H)-one

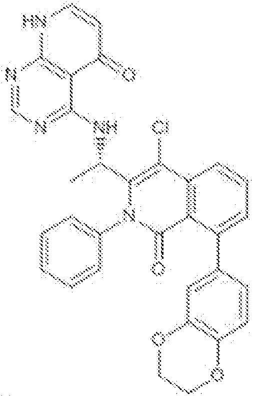

¹H-NMR (400MHz, CD₃OD) δ ppm 1.66(d, 3H), 2.66(s, 1H), 5.01-5.05(m, 1H), 6.29(d, 1H), 7.08-7.70(m, 12H), 8.07(d, 1H), 8.25(s, 1H), 11.02-11.04(m, 1H)

### <Example 254> Preparation of (S)-4-((1-(8-([1,1'-biphenyl]-4-yl)-4-chloro-1-oxo-2-phenyl-1,2-dihydroisoquinolin-3-yl)ethyl)amino)pyrido[2,3-d]pyrimidin-5(8H)-one

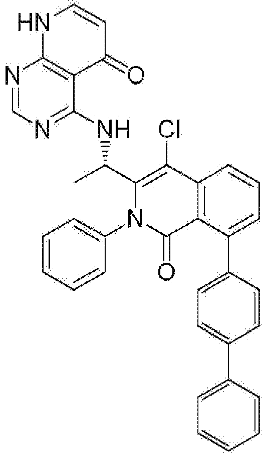

¹H-NMR (400MHz, CD₃OD) δ ppm 1.66(d, 3H), 2.66(s, 1H), 5.01-5.05(m, 1H), 6.29(d, 1H), 7.08-7.70(m, 12H), 8.07(d, 1H), 8.25(s, 1H), 11.02-11.04(m, 1H)

### <Example 255> Preparation of (S)-4-((1-(4-chloro-1-oxo-2-phenyl-8-(p-tolyl)-1,2-dihydroisoquinolin-3-yl)ethyl)amino)pyrido[2,3-d]pyrimidin-5(8H)-one

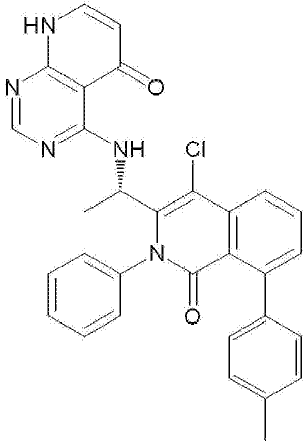

¹H-NMR (400MHz, CDCl₃) δ ppm 1.66(d, 3H), 5.04(m, 1H), 6.31(d, 1H), 7.05-7.75(m, 11H), 8.13(d, 1H), 8.26(s, 1H), 11.05(br, 1H)

### <Example 256> Preparation of (S)-4-((1-(4-chloro-8-(2-chloro-4-(trifluoromethyl)phenyl)-1-oxo-2-phenyl-1,2-dihydroisoquinolin-3-yl)ethyl)amino)pyrido[2,3-d]pyrimidin-5(8H)-one

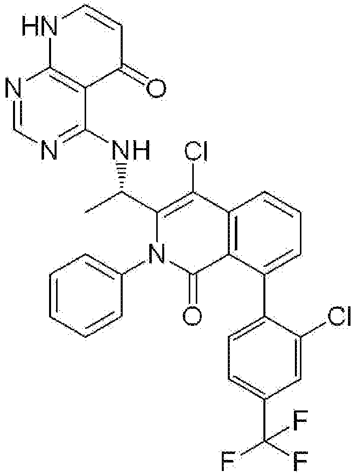

¹H-NMR (400MHz, CDCl₃) δ ppm 1.69(d, 3H), 5.05-5.12(m, 1H), 6.30-8.41(m, 14H), 11.08(d, 1H), 12.19(s, 1H)

### <Example 257> Preparation of (S)-4-((1-(4-chloro-8-(6-methylpyridin-3-yl)-1-oxo-2-phenyl-1,2-dihydroisoquinolin-3-yl)ethyl)amino)pyrido[2,3-d]pyrimidin-5(8H)-one

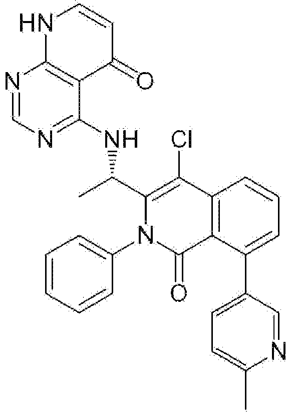

¹H-NMR (400MHz, CDCl₃) δ ppm 1.69(d, 3H), 2.79(s, 3H), 5.02-5.09(m, 1H), 6.31-8.62(m, 14H), 11.19(d, 1H)

### <Example 258> Preparation of (S)-4-((1-(4-chloro-1-oxo-2-phenyl-8-(1H-pyrrol-2-yl)-1,2-dihydroisoquinolin-3-yl)ethyl)amino)pyrido[2,3-d]pyrimidin-5(8H)-one

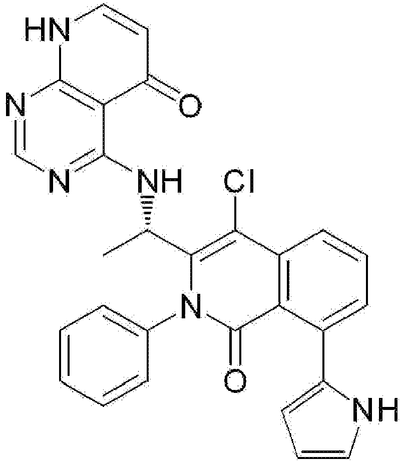

¹H-NMR (400MHz, CDCl₃) δ ppm 1.49(d, 3H), 4.95-5.02(m, 1H), 6.31-8.40(m, 14H), 11.10(d, 1H)

### <Example 259> Preparation of (S)-4-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)benzonitrile

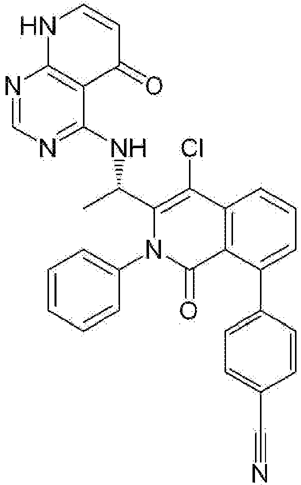

¹H-NMR (400MHz, CDCl₃) δ ppm 1.72(d, 3H), 5.13-5.20(m, 1H), 6.43-8.22(m, 15H), 11.20(d, 1H)

### <Example 260> Preparation of (S)-4-((1-(8-(4-morpholinophenyl)-1-oxo-2-phenyl-1,2-dihydroisoquinolin-3-yl)ethyl)amino)pyrido[2,3-d]pyrimidin-5(8H)-one

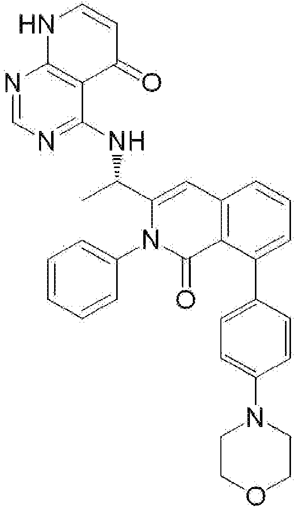

¹H-NMR (400MHz, CDCl₃) δ ppm 1.49(d, 3H), 3.10-3.82(m, 8H), 4.91-4.98(m, 1H), 6.29-8.18(m, 16H), 10.70(d, 1H), 11.91(s, 1H)

### <Example 261> Preparation of (S)-4-((1-(1-oxo-2-phenyl-8-(4-(pyrrolidin-1-yl)phenyl)-1,2-dihydroisoquinolin-3-yl)ethyl)amino)pyrido[2,3-d]pyrimidin-5(8H)-one

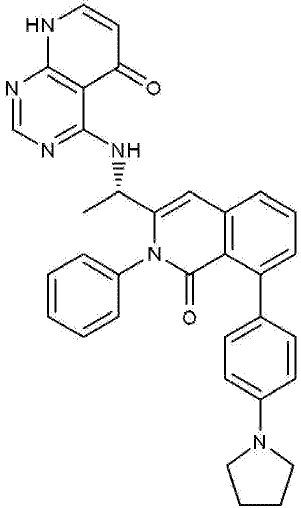

¹H-NMR (400MHz, CDCl₃) δ ppm 1.48 (d, 3H), 1.90-1.94 (m, 4H), 3.19-3.22(m, 4H), 4.91-4.98(m, 1H), 6.25-8.18(m, 16H), 10.66(d, 1H), 11.70(s, 1H)

### <Example 262> Preparation of (S)-4-((1-(8-(4-(hydroxymethyl)phenyl)-1-oxo-2-phenyl-1,2-dihydroisoquinolin-3-yl)ethyl)amino)pyrido[2,3-d]pyrimidin-5(8H)-one

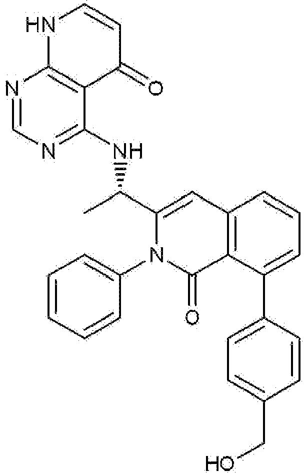

¹H-NMR (400MHz, CDCl₃) δ ppm 1.48(d, 3H), 4.68(s, 2H), 6.37-8.17(m, 16H), 10.70(d, 1H)

### <Example 263> Preparation of (S)-4-((1-(8-(3-methyl-1H-indol-5-yl)-1-oxo-2-phenyl-1,2-dihydroisoquinolin-3-yl)ethyl)amino)pyrido[2,3-d]pyrimidin-5(8H)-one

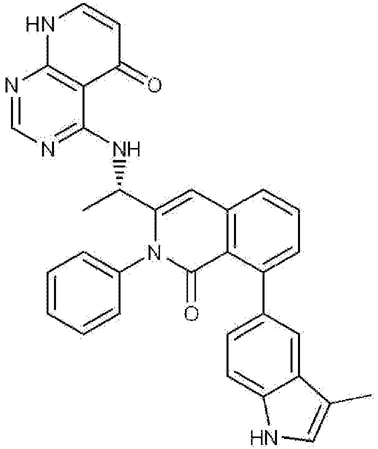

¹H-NMR (400MHz, CDCl₃) δ ppm 1.50(d, 3H), 2.27(s, 3H), 4.92-4.99(m, 1H), 6.31-8.19(m, 16H), 10.54(d, 1H)

### <Example 264> Preparation of (S)-4-((1-(8-(6-(dimethylamino)pyridin-3-yl)-1-oxo-2-phenyl-1,2-dihydroisoquinolin-3-yl)ethyl)amino)pyrido[2,3-d]pyrimidin-5(8H)-one

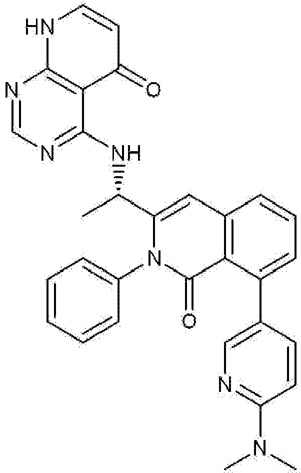

¹H-NMR (400MHz, CDCl₃) δ ppm 1.50(d, 3H), 3.27(s, 6H), 4.89-4.96(m, 1H), 6.36-8.16(m, 15H), 10.89(d, 1H)

### <Example 265> Preparation of (S)-4-chloro-1-oxo-3-(1-((5-oxo-5,8-dihydropyrido[2,3-d]pyrimidin-4-yl)amino)ethyl)-2-phenyl-1,2-dihydroisoquinoline-8-carbonitrile

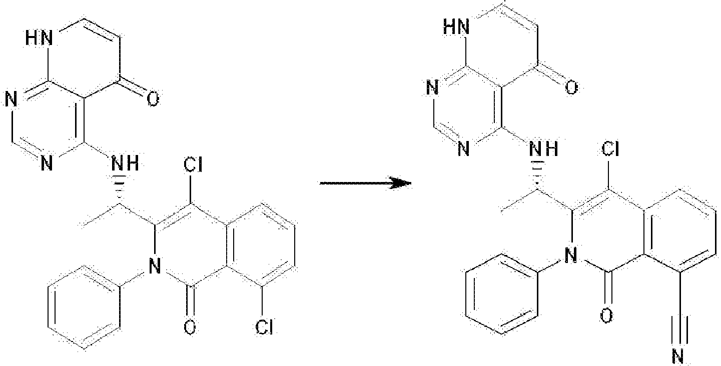

The 1 g (2.09 mmol) of (S)-4-((1-(4,8-dichloro-1-oxo-2-phenyl-1,2-dihydroisoquinolin-3-yl) ethyl)amino)pyrido[2,3-d]pyrimidin-5(8H)-one prepared in above Preparation Example 1, 24 mg (0.105 mmol) of palladium(II) acetate, 83 mg (0.21 mmol) of 2-dicyclohexylphosphino-2'-(N,N-dimethylamino)biphenyl [DavePhos, cas 213697-53-1], 220 mg (0.52 mmol) of potassium hexacyanoferrate(II) trihydrate, and 72 mg (0.52 mmol) of potassium carbonate were added to a mixed solution of 10 mL of dioxane and 10 mL of purified water, and irradiated with microwaves at 130°C for 15 hours. Upon completion of the reaction, the resulting mixture was cooled down to room temperature, filtered through diatomite, washed with acetone, and concentrated under reduced pressure to obtain residues, to which a saturated aqueous solution of ammonium chloride was then added, followed by extraction with chloroform. An organic layer was washed with saturated saline solution, dried over magnesium sulfate, concentrated, and vacuum-dried for five hours so as to obtain 0.95 g of (S)-4-chloro-1-oxo-3-(1-((5-oxo-5,8-dihydropyrido[2,3-d]pyrimidin-4-yl)amino)ethyl)-2-phenyl-1,2-dihydroisoq uinolin-8-carbonitrile as a light yellow solid. (Yield 96.9%)

¹H-NMR (400MHz, CDCl₃) δ ppm 1.68(d, 3H), 5.03(m, 1H), 6.31(d, 1H), 7.21(m, 1H), 7.45-7.65(m, 4H), 7.81(m, 2H), 7.90(d, 1H), 8.25(m, 1H), 10.90(d, 2H)

### <Example 266> Preparation of (S)-1-oxo-3-(1-((5-oxo-5,8-dihydropyrido[2,3-d]pyrimidin-4-yl)amino)ethyl)-2-phenyl-1,2-dihydroisoquinoline-8-carbonitrile

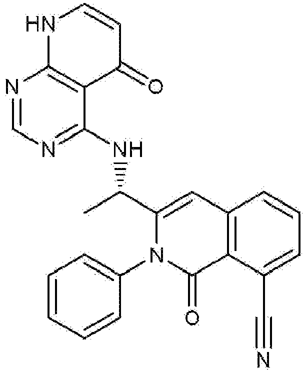

The compound was prepared by the same preparation method as that of Example 265 by using the (S)-4-((1-(8-chloro-1-oxo-2-phenyl-1,2-dihydroisoquinolin-3-yl) ethyl)amino)pyrido[2,3-d]pyrimidin-5(8H)-one prepared in above Preparation Example 2.

¹H-NMR (400MHz, CDCl₃) δ ppm 1.47(d, 3H), 4.96(m, 1H), 6.34(d, 1H), 6.67(s, 1H), 7.30-7.90(m, 9H), 8.25(s, 1H), 10.55(d, 1H)

### <Example 267> Preparation of (S)-4-((1-(8-acetyl-4-chloro-1-oxo-2-phenyl-1,2-dihydroisoquinolin-3-yl)ethyl)amino)pyrido[2,3-d]pyrimidin-5(8H)-one

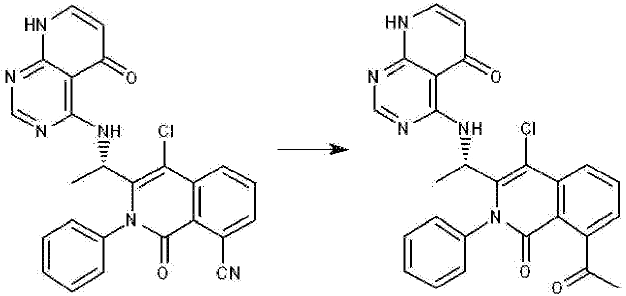

The 50 mg of (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carbonitrile prepared in above Example 265 and 0.1 mL of 3M methylmagnesium bromide THF solution were added into 2 mL of anhydrous THF at 0°C, and stirred for one to two hours. Upon completion of the reaction, a saturated aqueous solution of ammonium chloride was added, followed by extraction with ethyl acetate two to three times. An organic layer was dried over anhydrous sodium sulfate, and concentrated under reduced pressure, and then the resulting residues were purified by Prep-LC. The resulting product was vacuum-dried for three hours to obtain 10 mg of (S)-4-((1-(8-acetyl-4-chloro-1-oxo-2-phenyl-1,2-d**ihydroisoquinolin-3-yl)**ethyl)amino)pyrido[2,3-d]pyrimidin-5(8H)-one.

¹H-NMR (400MHz, CDCl₃) δ ppm 1.66(d, 3H), 2.37(s, 3H), 5.01-5.05(m, 1H), 6.26-8.25(m, 11H), 10.99(s, 1H)

### <Examples 268 and 269>

In Examples 268 and 269, the compound of each Example was prepared by substantially the same preparation method as described in above Example 267, except for using an appropriate reactant in consideration of the structure of the corresponding compound instead of methylmagnesium bromide.

### <Example 268> Preparation of (S)-4-((1-(8-butyryl-4-chloro-1-oxo-2-phenyl-1,2-dihydroisoquinolin-3-yl)ethyl)amino)pyrido[2,3-d]pyrimidin-5(8H)-one

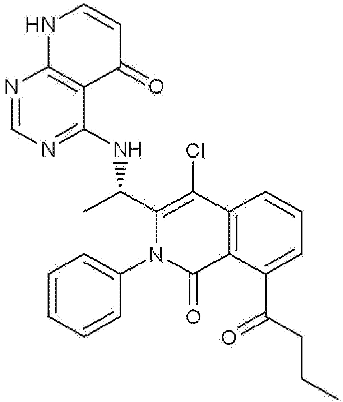

¹H-NMR (400MHz, CDCl₃) δ ppm 0.97(t, 3H), 1.53(s, 2H), 1.66(d, 3H), 2.63(t, 2H), 5.01-5.03(m, 1H), 6.27-8.22(m, 11H), 10.95(s, 1H)

### <Example 269> Preparation of (S)-4-((1-(4-chloro-8-(cyclopropanecarbonyl)-1-oxo-2-phenyl-1,2-dihydroisoquinolin-3-yl)ethyl)amino)pyrido[2,3-d]pyrimidin-5(8H)-one

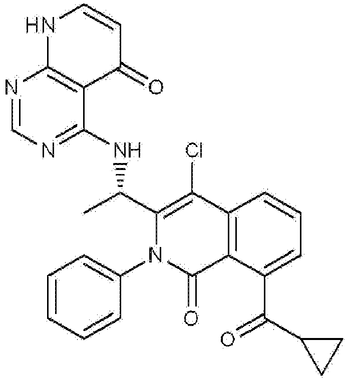

mass (513(+2))

### <Example 270> Preparation of (S,Z)-4-((1-(4-chloro-8-(1-(methoxyimino)ethyl)-1-oxo-2-phenyl-1,2-dihydroisoquinolin-3-yl)ethyl)amino)pyrido[2,3-d]pyrimidin-5(8H)-one

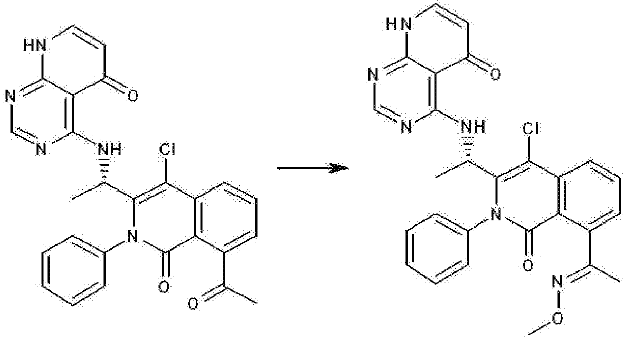

The 5 mg of methoxyamine hydrochloride and 8 mg of sodium acetate were added into 2 mL of 80% ethanol aqueous solution and stirred at room temperature for 30 minutes, after which 10 mg of (S)-4-((1-(8-acetyl-4-chloro-1-oxo-2-phenyl-1,2-dihydroisoquinolin-3-yl)ethyl)amino)pyrido[2,3-d]pyrimidin-5(8H)-one prepared in above Example 267 was added and stirred under reflux for two hours. Upon completion of the reaction, the resulting mixture was cooled down to room temperature, after which a saturated saline solution was added, followed by extraction with ethyl acetate two to three times. An organic layer was dried over anhydrous sodium sulfate, and concentrated under reduced pressure, and then the resulting residues were purified by Prep-LC. The resulting product was vacuum-dried for three hours to obtain 3.6 mg of (S,Z)-4-((1-(4-chloro-8-(1-(methoxyimino)ethyl)-1-oxo-2-phenyl-1,2-dihydroisoquinolin-3-yl) ethyl)amino)pyrido[2,3-d]pyrimidin-5(8H)-one.

¹H-NMR (400MHz, CDCl₃) δ ppm 1.67(d, 3H), 2.14(s, 3H), 3.88(s, 3H), 5.02(m, 1H), 6.30-8.26(m, 11H), 10.97(s, 1H), 11.32(s, 1H)

### <Example 271> Preparation of (S)-4-chloro-1-oxo-3-(1-((5-oxo-5,8-dihydropyrido[2,3-d]pyrimidin-4-yl)amino)ethyl)-2-phenyl-1,2-dihydroisoquinoline-8-carbaldehyde

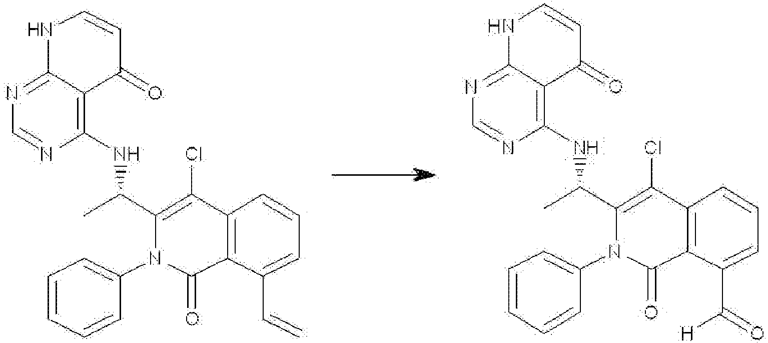

The 110 mg (0.23 mmol) of (S)-4-((1-(4-chloro-1-oxo-2-phenyl-8-vinyl-1,2-dihydroisoquinolin-3-yl)ethyl)amino)pyrido[2,3-d]pyrimidin-5(8H)-one prepared in above Example 141, 150 mg (0.702 mmol) of sodium periodate and 24 µL of 4% osmium oxide aqueous solution were added into a mixed solution of 3 mL of t-butyl alcohol and 3 mL of purified water, and heated and stirred at 40°C for 15 hours. Upon completion of the reaction, the reaction solution was concentrated, after which a saturated aqueous solution of ammonium chloride was added, followed by extraction with ethyl acetate two to three times. An organic layer was washed with saturated saline solution, dried over magnesium sulfate, and concentrated under reduced pressure to obtain residues, which were then purified through MPLC (under gradient conditions of 100% chloroform → 10% methanol chloroform solution). The resulting product was vacuum-dried for five hours to obtain 100 mg of (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carbaldehyde as a light yellow solid. (Yield 91.0%)

¹H-NMR (400MHz, CDCl₃) δ ppm 1.70(d, 3H), 5.07 (m, 1H), 6.32(d, 1H), 7.22(m, 1H), 7.40-7.85(m, 7H), 8.21(d, 1H), 8.27(s, 1H), 10.99(s, 1H), 11.02(br, 1H), 11.08(br, 1H)

### <Example 272> Preparation of 4-(((1S)-1-(4-chloro-8-(1,2-dihydroxy-2-phenylethyl)-1-oxo-2-phenyl-1,2-dihydroisoquinolin-3-yl)ethyl)amino)pyrido[2,3-d]pyrimidin-5(8H)-one

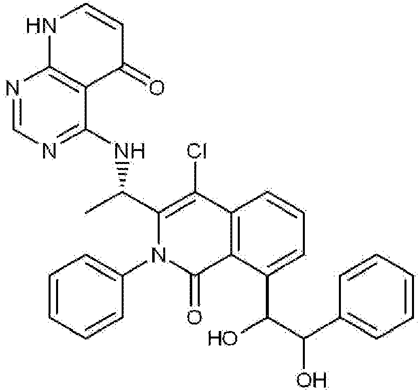

During the preparation process described in above Example 271, the reaction mixture was separated by Prep-LC from the compound prepared in above Example 70.

¹H-NMR (400MHz, CDCl₃) δ ppm 1.66(d, 3H), 5.03(m, 2H), 5.22(m, 1H), 6.22(t, 1H), 7.01(s, 2H), 7.09(s, 3H), 7.15-7.80(m, 8H), 7.95(d, 1H), 8.20(d, 1H), 11.05(br, 1H)

### <Example 273> Preparation of (S,E)-4-chloro-1-oxo-3-(1-((5-oxo-5,8-dihydropyrido[2,3-d]pyrimidin-4-yl)amino)ethyl)-2-phenyl-1,2-dihydroisoquinoline-8-carbaldehyde oxime

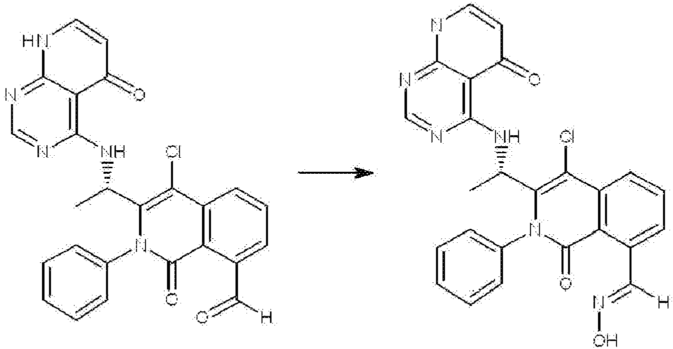

The 1 mg of hydroxyamine hydrochloride and 1 mg of sodium acetate were added into 1 mL of 80% ethanol aqueous solution and stirred at room temperature for 30 minutes, after which 2.5 mg of (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carbaldehyde prepared in above Example 271 was added and stirred under reflux for one hour. Upon completion of the reaction, the resulting mixture was cooled down to room temperature, after which a saturated saline solution was added, followed by extraction with ethyl acetate two to three times. An organic layer was dried over anhydrous sodium sulfate, and concentrated under reduced pressure, and then the resulting residues were purified by Prep-LC. The resulting product was vacuum-dried for three hours to obtain 0.9 mg of (S,E)-4-chloro-1-oxo-3-(1-((5-oxo-5,8-dihydropyrido[2,3-d]pyrimidin-4-yl)amino)ethyl)-2-phenyl-1,2-dihydroisoquinoline-8-carbaldehyde oxime.

¹H-NMR (400MHz, CDCl₃) δ ppm 1.70 (d, 3H), 5.11-5.14 (m, 1H), 6.35-9.36(m, 11H), 11.32(s, 1H)

### <Example 274> Preparation of (S)-4-chloro-N-methyl-1-oxo-3-(1-((5-oxo-5,8-dihydropyrido[2,3-d]pyrimidin-4-yl)amino)ethyl)-2-phenyl-1,2-dihydroisoquinoline-8-carboxamide

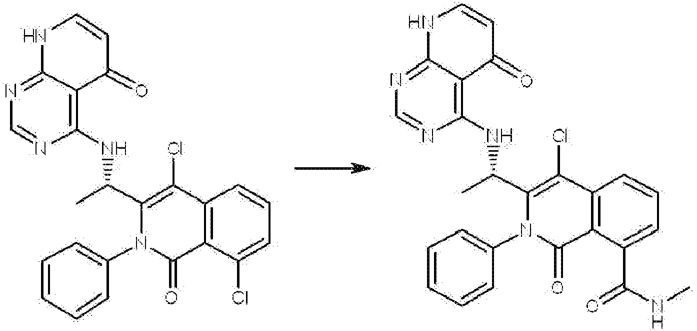

The 50 mg of (S)-4-((1-(4,8-dichloro-1-oxo-2-phenyl-1,2-dihydroisoquinolin-3-yl)ethyl)amino)pyrido[2,3-d]pyrimidin-5(8H)-one prepared in above Preparation Example 1, 2.4 mg of palladium(II) acetate, 6 mg of tri-tert-butylphosphonium tetrafluoroborate [CAS: 131274-22-1], 37 mg of cyclopentadienyl iron(II) dimer (CAS: 12154-95-9), and 7 µl of methylamine were added into 2.5 mL of dioxane, and irradiated with microwaves at 160°C for 45 minutes by using a microwave reactor. Upon completion of the reaction, the resulting mixture was cooled down to room temperature, after which a saturated saline solution was added, followed by extraction with ethyl acetate two to three times. An organic layer was dried over anhydrous sodium sulfate, and concentrated under reduced pressure, and then the resulting residues were purified by Prep-LC. The resulting product was vacuum-dried for three hours to obtain 10 mg of (S)-4-chloro-N-methyl-1-oxo-3-(1-((5-oxo-5,8-dihydropyrido[2,3-d]pyrimidin-4-yl) amino)ethyl)-2-phenyl-1,2-dihydroisoquinolin-8-carboxamide.

¹H-NMR (400MHz, CDCl₃) δ ppm 1.66(d, 3H), 2.98(d, 3H), 5.00(q, 1H), 5.66(br, 1H), 6.26-8.28(m, 11H), 10.89(br, 1H)

### <Examples 275 to 286>

In Examples 275 to 286, the compound of each Example was prepared by the same preparation method as that of Example 274, except for using an appropriate reactant in consideration of the structure of the corresponding compound instead of methylamine.

### <Example 275> Preparation of (S)-4-chloro-1-oxo-3-(1-((5-oxo-5,8-dihydropyrido[2,3-d]pyrimidin-4-yl)amino)ethyl)-2-phenyl-N-propyl-1,2-dihydroisoquinoline-8-carboxamide

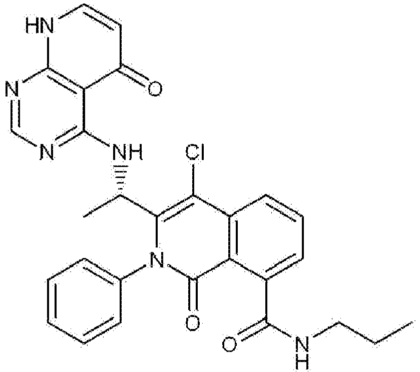

¹H-NMR (400MHz, CDCl₃) δ ppm 0.87(t, 3H), 1.50-1.56(m, 2H), 1.61(d, 3H), 3.28-3.42(m, 2H), 4.96-4.99(m, 1H), 6.07-8.16(m, 12H), 10.93(s, 1H), 11.83(s, 1H)

### <Example 276> Preparation of (S)-4-chloro-N-(oxetan-3-yl)-1-oxo-3-(1-((5-oxo-5,8-dihydropyrido[2,3-d]pyrimidin-4-yl)amino)ethyl)-2-phenyl-1,2-dihydroisoquinoline-8-carboxamide

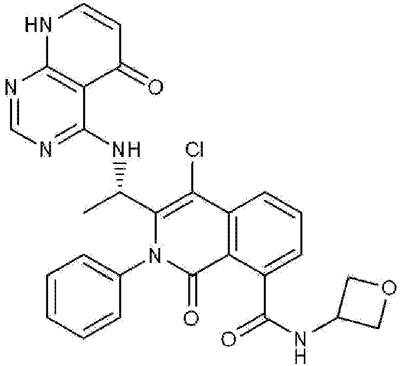

¹H-NMR (400MHz, CDCl₃) δ ppm 1.62(d, 3H), 4.56(t, 2H), 4.94-4.99(m, 3H), 5.16-5.25(m, 1H), 6.29-8.16(m, 12H), 10.24(s, 1H), 10.89(s, 1H)

### <Example 277> Preparation of (S)-4-chloro-N-cyclobutyl-1-oxo-3-(1-((5-oxo-5,8-dihydropyrido[2,3-d]pyrimidin-4-yl)amino)ethyl)-2-phenyl-1,2-dihydroisoquinoline-8-carboxamide

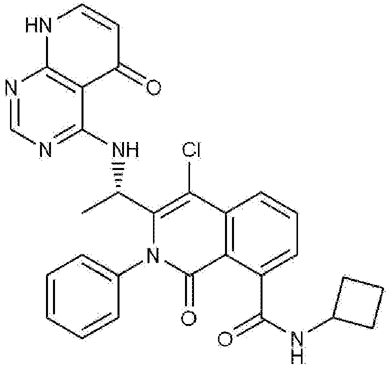

¹H-NMR (400MHz, CDCl₃) δ ppm 1.39(s, 9H), 1.69(d, 3H), 5.03-5.06(m, 1H), 6.31-8.59(m, 13H), 11.03(br, 1H), 11.56(br, 1H)

### <Example 278> Preparation of (S)-4-chloro-1-oxo-3-(1-((5-oxo-5,8-dihydropyrido[2,3-d]pyrimidin-4-yl)amino)ethyl)-2-phenyl-N-(thiophen-2-ylmethyl)-1,2-dihydroisoquinoline-8-carboxamide

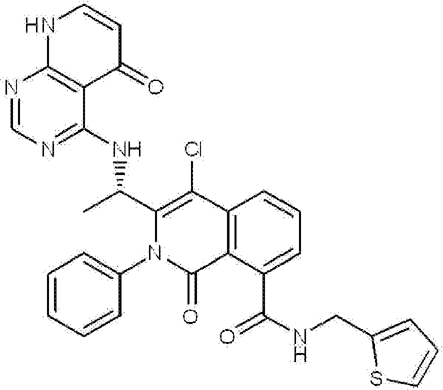

¹H-NMR (400MHz, CDCl₃) δ ppm 1.64(d, 3H), 4.76-4.85(m, 2H), 4.87-5.01(m, 1H), 6.03-8.18(m, 15H), 10.92(s, 1H), 11.01(s, 1H)

### <Example 279> Preparation of (S)-4-chloro-N-(furan-2-ylmethyl)-1-oxo-3-(1-((5-oxo-5,8-dihydropyrido[2,3-d]pyrimidin-4-yl)amino)ethyl)-2-phenyl-1,2-dihydroisoquinoline-8-carboxamide

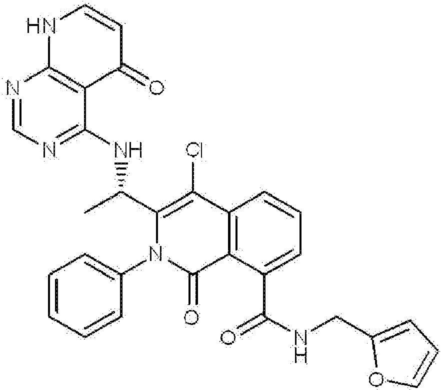

¹H-NMR (400MHz, CDCl₃) δ ppm 1.63(d, 3H), 4.58-4.95(m, 3H), 6.10-9.17(m, 15H), 10.93(s, 1H), 11.37(s, 1H)

### <Example 280> Preparation of (S)-N-benzyl-4-chloro-1-oxo-3-(1-((5-oxo-5,8-dihydropyrido[2,3-d]pyrimidin-4-yl)amino)ethyl)-2-phenyl-1,2-dihydroisoquinoline-8-carboxamide

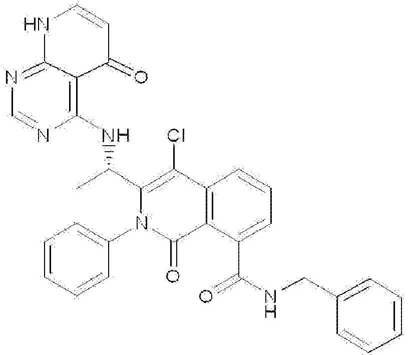

¹H-NMR (400MHz, CDCl₃) δ ppm 1.61(d, 3H), 4.55(m, 2H), 4.98(m, 1H), 6.16(d, 1H), 6.31(t, 1H), 7.15-7.80(m, 11H), 7.68(m, 2H), 8.047(m, 2H), 10.95(br, 1H)

### <Example 281> Preparation of (S)-4-chloro-N-(2-morpholinoethyl)-1-oxo-3-(1-((5-oxo-5,8-dihydropyrido[2,3-d]pyrimidin-4-yl)amino)ethyl)-2-phenyl-1,2-dihydroisoquinoline-8-carboxamide

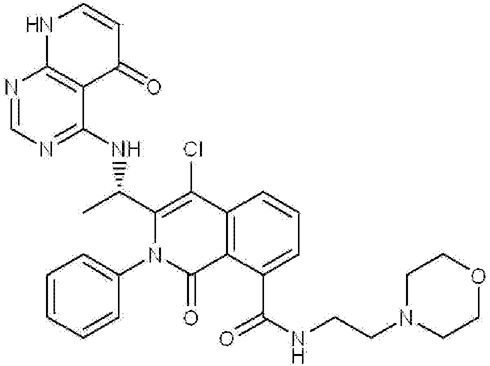

mass (598.29(-1), 600.32(+))

### <Example 282> Preparation of (S)-4-chloro-N-isopropyl-1-oxo-3-(1-((5-oxo-5,8-dihydropyrido[2,3-d]pyrimidin-4-yl)amino)ethyl)-2-phenyl-1,2-dihydroisoquinoline-8-carboxamide

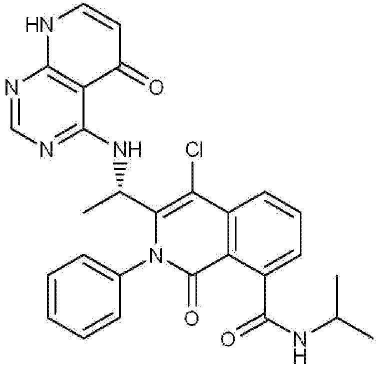

¹H-NMR (400MHz, CDCl₃) δ ppm 1.18(m, 6H), 1.63(d, 3H), 4.26(m, 1H), 4.98(m, 1H), 5.59(d, 1H), 6.21(d, 1H), 7.15(m, 1H), 7.40-7.80(m, 7H), 8.04(d, 1H), 8.19(s, 1H), 10.95(br, 1H), 11.75(br, 1H)

### <Example 283> Preparation of (S)-4-chloro-N-(cyclopropylmethyl)-1-oxo-3-(1-((5-oxo-5,8-dihydropyrido[2,3-d]pyrimidin-4-yl)amino)ethyl)-2-phenyl-1,2-dihydroisoquinoline-8-carboxamide

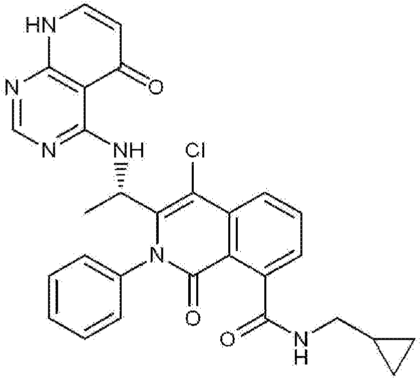

¹H-NMR (400MHz, CDCl₃) δ ppm 0.17(m, 2H), 0.42(m, 2H), 0.97(m, 1H), 1.63(d, 3H), 3.30(m, 2H), 4.98(m, 1H), 5.94(t, 1H), 6.21(d, 1H), 7.15(m, 1H), 7.40-7.80(m, 7H), 8.04(d, 1H), 8.19(s, 1H), 10.95(br, 1H), 11.80(br, 1H)

### <Example 284> Preparation of (S)-4-chloro-N-(1-hydroxy-2-methylpropan-2-yl)-1-oxo-3-(1-((5-oxo-5,8-dihydropyrido[2,3-d]pyrimidin-4-yl)amino)ethyl)-2-phenyl-1,2-dihydroisoquinoline-8-carboxamide

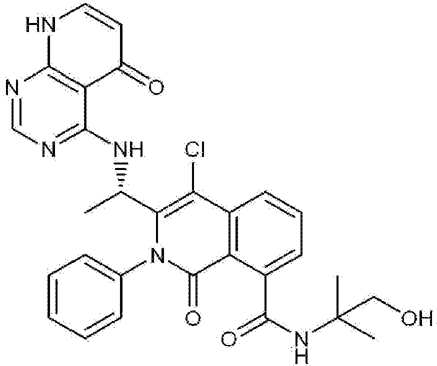

¹H-NMR (400MHz, CDCl₃) δ ppm 1.33(d, 6H), 1.58(d, 3H), 3.70-3.95(m, 2H), 4.43(br, 1H), 4.98(m, 1H), 5.65(s, 1H), 6.29(d, 1H), 7.40-8.15(m, 9H), 10.91(br, 1H), 11.45(br, 1H)

### <Example 285> Preparation of (S)-N-(tert-butyl)-4-chloro-1-oxo-3-(1-((5-oxo-5,8-dihydropyrido[2,3-d]pyrimidin-4-yl)amino)ethyl)-2-phenyl-1,2-dihydroisoquinoline-8-carboxamide

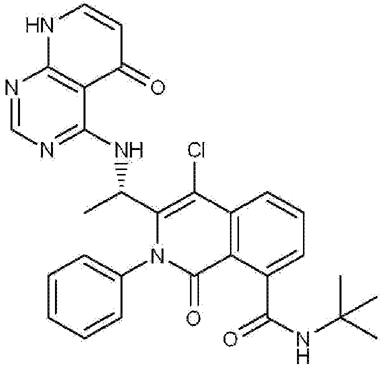

¹H-NMR (400MHz, CDCl₃) δ ppm 1.40(s, 9H), 1.64(d, 3H), 4.98(m, 1H), 5.44(s, 1H), 6.24(d, 1H), 7.15(m, 1H), 7.40-7.80(m, 7H), 8.01(d, 1H), 8.19(s, 1H), 10.92(br, 1H), 11.20(br, 1H)

### <Example 286> Preparation of (S)-4-((1-(4-chloro-8-(morpholine-4-carbonyl)-1-oxo-2-phenyl-1,2-dihydroisoquinolin-3-yl)ethyl)amino)pyrido[2,3-d]pyrimidin-5(8H)-one

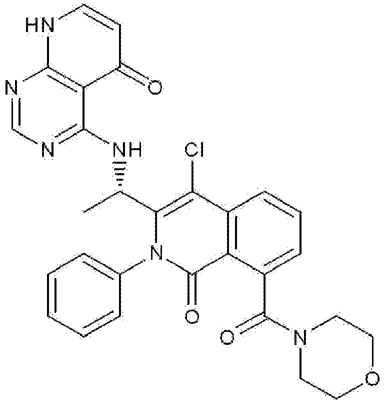

¹H-NMR (400MHz, CDCl₃) δ ppm 1.66(m, 3H), 3.10-3.95(m, 8H), 4.95(m, 1H), 6.28(d, 1H), 7.20(m, 1H), 7.30-8.25(m, 9H), 10.05(br, 1H), 10.85(br, 1H)

### <Example 287> Preparation of (S)-4-((1-(4-chloro-8-(1H-imidazol-2-yl)-1-oxo-2-phenyl-1,2-dihydroisoquinolin-3-yl)ethyl)amino)pyrido[2,3-d]pyrimidin-5(8H)-one

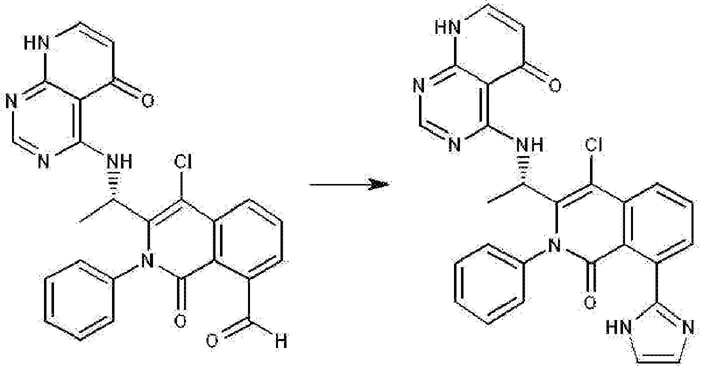

The 50 mg of (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carbaldehyde prepared in above Example 271, 0.13 mL of glyoxal and 0.14 mL of 25% ammonia water were added into 1.5 mL of ethanol at 0°C, and stirred at room temperature for two days. Upon completion of the reaction, the reaction mixture was concentrated under reduced pressure, and then the resulting residues were purified by Prep-LC. The resulting product was vacuum-dried for three hours to obtain 11.4 mg of (S)-4-((1-(4-chloro-8-(1H-imidazol-2-yl)-1-oxo-2-phenyl-1,2-dihydroisoquinolin-3-yl)ethyl)amino)pyrido[2,3-d]pyrimidin-5(8H)-one.

¹H-NMR (400MHz, CDCl₃) δ ppm 1.68(d, 3H), 4.96-5.01(m, 1H), 6.28-8.80(m, 13H), 10.97(s, 1H), 13.20(s, 1H)

### <Example 288> Preparation of (S)-4-((1-(4-chloro-8-(4,5-dimethyl-1H-imidazol-2-yl)-1-oxo-2-phenyl-1,2-dihydroisoquinolin-3-yl) ethyl)amino)pyrido[2,3-d]pyrimidin-5(8H)-one

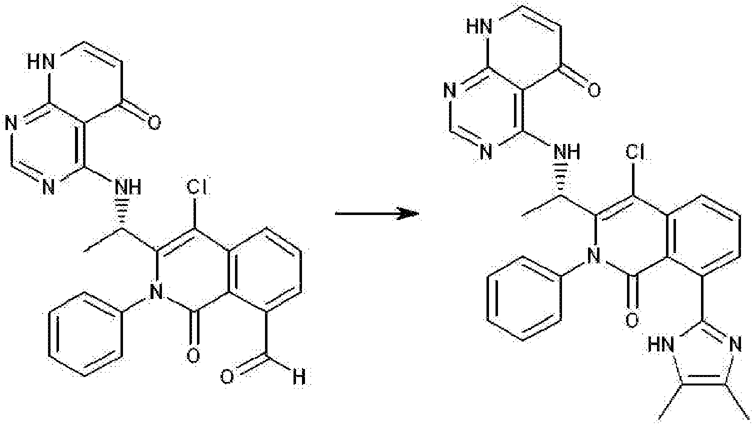

The 30 mg of (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carbaldehyde prepared in above Example 271, 10 mL of ammonium acetate and 5 mg of biacetyl were added into 1 mL of acetic acid, and stirred under reflux for two hours. Upon completion of the reaction, the resulting mixture was cooled down to room temperature and neutralized with ammonia water, after which a saturated saline solution was added, followed by extraction with ethyl acetate two to three times. An organic layer was dried over anhydrous sodium sulfate, and concentrated under reduced pressure, and then the resulting residues were purified by Prep-LC. The resulting product was vacuum-dried for three hours to obtain 6.4 mg of (S)-4-((1-(4-chloro-8-(4,5-dimethyl-1H-imidazol-2-yl)-1-oxo-2-phenyl-1,2-dihydroisoquinolin-3-yl)ethyl)amino)pyrido[2,3-d]pyrimidin-5(8H)-one.

¹H-NMR (400MHz, CDCl₃) δ ppm 1.67(d, 3H), 2.17(s, 6H), 4.93-4.97(m, 1H), 6.27-8.63(m, 11H), 10.96(s, 1H)

### <Example 289> Preparation of (S)-4-((1-(4-chloro-8-(4,4-dimethyl-4,5-dihydrooxazol-2-yl)-1-oxo-2-phenyl-1,2-dihydroisoquinolin-3-yl)ethyl)amino)pyrido[2,3-d]pyrimidin-5(8H)-one

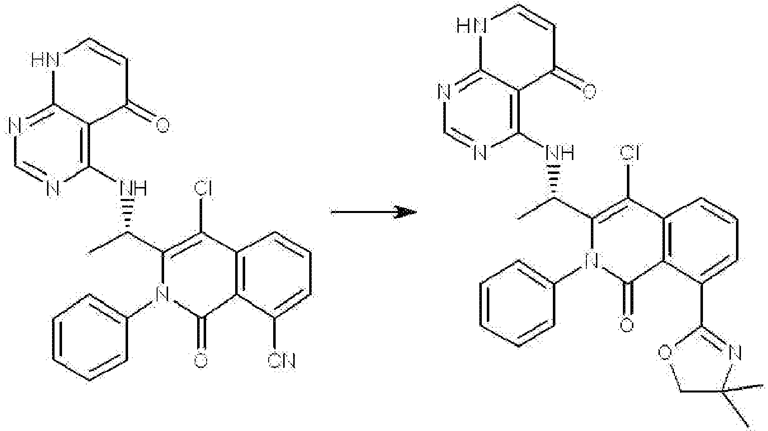

The 30 mg of (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carbonitrile prepared in above Example 265, 1 mg of zinc chloride and 16 mg of 2-amino-2-methyl-1-propanol were added into 1.5 mL of chlorobenzene, and stirred under reflux for a day. Upon completion of the reaction, the resulting mixture was cooled down to room temperature, after which a saturated aqueous solution of sodium chloride was added, followed by extraction with ethyl acetate two to three times. An organic layer was dried over anhydrous sodium sulfate, and concentrated under reduced pressure, and then the resulting residues were purified by Prep-LC. The resulting product was vacuum-dried for three hours to obtain 9.7 mg of (S)-4-((1-(4-chloro-8-(4,4-dimethyl-4,5-dihydrooxazol-2-yl)-1-oxo-2-phenyl-1,2-dihydroisoquinolin-3-yl)ethyl)amino)pyrido[2,3-d]pyrimidin-5(8H)-one.

¹H-NMR (400MHz, CDCl₃) δ ppm 1.36(s, 3H), 1.37(s, 3H), 1.63(d, 3H), 4.14(s, 2H), 4.91-4.95(q, 1H), 6.26-8.26(m, 11H), 10.94(s, 1H), 12.05(s, 1H)

### <Example 290> Preparation of (S)-4-chloro-1-oxo-3-(1-((5-oxo-5,8-dihydropyrido[2,3-d]pyrimidin-4-yl)amino)ethyl)-2-phenyl-1,2-dihydroisoquinoline-8-carboxylic acid

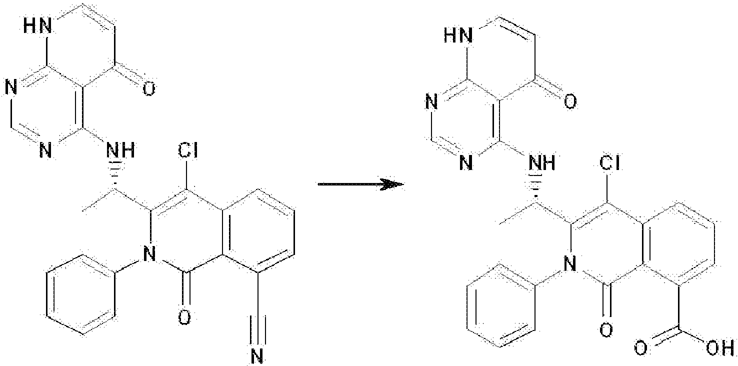

The 500 mg (1.07 mmol) of (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carbonitrile prepared in above Example 265 and 255 mg (6.4 mmol) of sodium hydroxide were added into 10 mL of 50% ethanol, and stirred under reflux for 15 hours. Upon completion of the reaction, the reaction mixture was concentrated, after which a saturated aqueous solution of ammonium chloride was added, followed by extraction with chloroform two to three times. An organic layer was washed with saturated saline solution, dried over magnesium sulfate, and concentrated to obtain residues, which were then purified through MPLC (under gradient conditions of 100% chloroform → 30% methanol chloroform solution). The resulting product was vacuum-dried for five hours to obtain 250 mg of (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carboxylic acid as a white solid. (Yield 48.1%)

¹H-NMR (400MHz, DMSO-d₆) δ ppm 1.60 (d, 3H), 4.84 (m, 1H), 6.13(d, 1H), 7.14(s, 1H), 7.30-7.80(m, 10H), 8.33(d, 1H), 10.90(br, 1H), 12.15(br, 1H)

### <Example 291> Preparation of (S)-4-chloro-1-oxo-3-(1-((5-oxo-5,8-dihydropyrido[2,3-d]pyrimidin-4-yl)amino)ethyl)-2-phenyl-1,2-dihydroisoquinoline-8-carboxamide

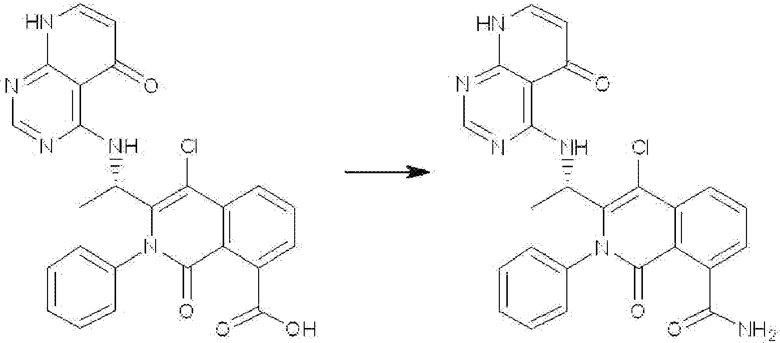

The 10 mg (0.02 mmol) of (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carboxylic acid prepared in above Example 290, 5 mg (0.033 mmol) of hydroxybenzotriazole [HOBT], 7.7 mg (0.04 mmol) of 1-ethyl-3-(3-dimethylaminopropyl)carbodiimide [EDC] and 2.2 mg (0.04 mmol) of ammonium chloride were added into 1 mL of acetonitrile, and stirred at room temperature. 14 µl (0.10 mmol) of triethylamine was added to the reaction mixture and further stirred for three hours, after which a saturated aqueous solution of ammonium chloride was added to complete the reaction, followed by extraction with ethyl acetate two to three times. An organic layer was washed with saturated saline solution, dried over magnesium sulfate, and concentrated to obtain residues, which were then purified through Prep-LC (under gradient conditions of 20% acetonitrile aqueous solution → 100% acetonitrile). The resulting product was vacuum-dried to obtain 3 mg of (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carboxamide.

¹H-NMR (400MHz, CDCl₃) δ ppm 1.66(d, 3H), 5.03(m, 1H), 6.24(d, 1H), 7.20(m, 1H), 7.30-7.60(m, 5H), 7.75(t, 2H), 8.09(d, 1H), 8.20(s, 1H), 10.80(br, 1H), 10.95(br, 1H)

### <Example 292> Preparation of ethyl (S)-4-chloro-1-oxo-3-(1-((5-oxo-5,8-dihydropyrido[2,3-d]pyrimidin-4-yl)amino)ethyl)-2-phenyl-1,2-dihydroisoquinoline-8-carboxylate

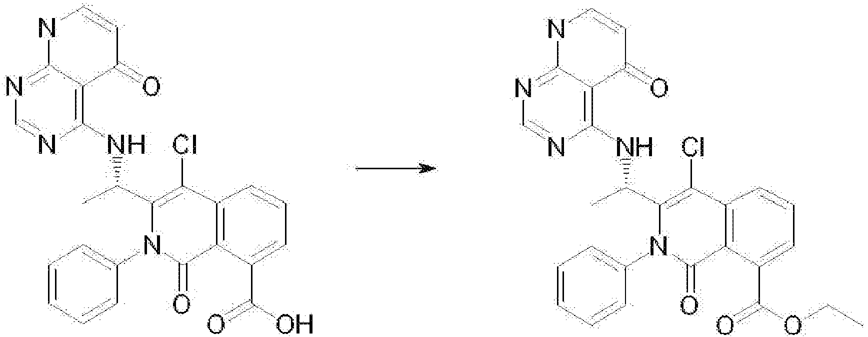

The 10 mg of (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carboxylic acid prepared in above Example 290 was added into 2 mL of ethanol and stirred. The 20 mg of 95% sulfuric acid was added dropwise and stirred under reflux for 15 hours to complete the reaction, after which a solvent was concentrated and a saturated aqueous solution of ammonium chloride was added, followed by extraction with ethyl acetate two to three times. An organic layer was washed with saturated saline solution, dried over anhydrous magnesium sulfate, and concentrated to obtain residues, which were then purified through Prep-LC. The resulting product was vacuum-dried for five hours to obtain 1.2 mg of ethyl (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carboxylate.

¹H-NMR (400MHz, CDCl₃) δ ppm 1.31(t, 3H), 1.66(d, 3H), 4.40(m, 2H), 5.01(m, 1H), 6.30(d, 1H), 7.21(m, 1H), 7.40-7.60(m, 5H), 7.74(m, 2H), 8.06(d, 1H), 8.25(s, 1H), 10.15(br, 1H), 10.89(br, 1H)

### <Example 293> Preparation of (S)-4-((1-(4-chloro-8-(4,6-diamino-1,3,5-triazin-2-yl)-1-oxo-2-phenyl-1,2-dihydroisoquinolin-3-yl)ethyl)amino)pyrido[2,3-d]pyrimidin-5(8H)-one

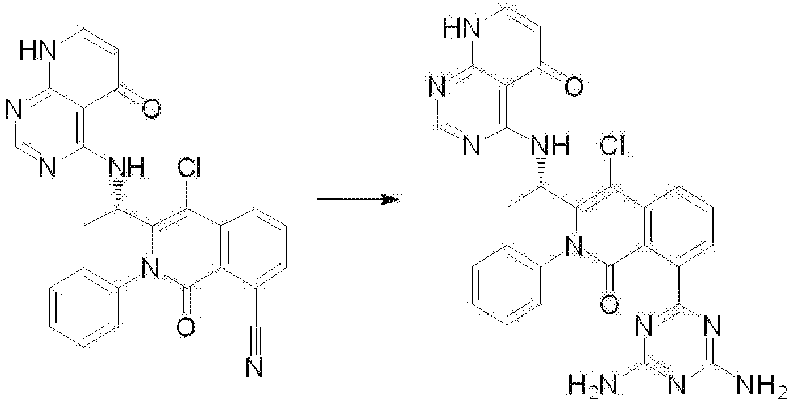

The 50 mg (0.106 mmol) of (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carbonitrile prepared in above Example 265, 11 mg (0.13 mmol) of cyanoguanidine and 3.6 mg (0.06 mmol) of potassium hydroxide were added into 2 mL of butyl alcohol, and stirred under reflux for 15 hours. Upon completion of the reaction, the resulting mixture was cooled down to room temperature, after which a saturated aqueous solution of ammonium chloride was added, followed by extraction with ethyl acetate two to three times. An organic layer was washed with saturated saline solution, dried over anhydrous magnesium sulfate, and concentrated to obtain residues, which were then purified through Prep-LC (under gradient conditions of 20% acetonitrile aqueous solution → 100% acetonitrile). The resulting product was vacuum-dried for five hours to obtain 45 mg of (S)-4-((1-(4-chloro-8-(4,6-diamino-1,3,5-triazin-2-yl)-1-oxo-2-phenyl-1,2-dihydroisoquinolin-3-yl)ethyl)amino)pyrido[2,3-d]pyrimidin-5(8H)-one as a light yellow solid. (Yield 77.5%)

¹H-NMR (400MHz, DMSO-d₆) δ ppm 1.60(d, 3H), 4.81(m, 1H), 6.13(d, 1H), 6.65(br), 7.30-8.05(m, 9H), 8.34(s, 1H), 10.88(br, 1H), 12.13(s, 1H)

### <Example 294> Preparation of (S)-4-((1-(8-amino-4-chloro-1-oxo-2-phenyl-1,2-dihydroisoquinolin-3-yl)ethyl)amino)pyrido[2,3-d]pyrimidin-5(8H)-one

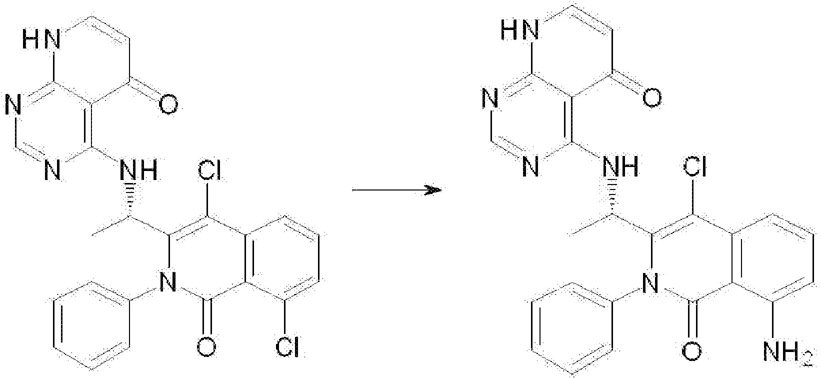

The 500 mg (1.05 mmol) of (S)-4-((1-(4,8-dichloro-1-oxo-2-phenyl-1,2-dihydroisoquinolin-3-yl)ethyl)amino)pyrido[2,3-d]pyrimidin-5(8H)-one prepared in above Preparation Example 1, 15 mg (0.105 mmol) of copper oxide(I) and 30 mg of cesium carbonate were added into a mixed solution of 1.3 mL of N-methylpyrrolidone and 1.3 mL of 29% ammonia water and irradiated with microwaves at 130°C for six hours. Upon completion of the reaction, the resulting mixture was cooled down to room temperature, after which a saturated aqueous solution of ammonium chloride was added, followed by extraction with ethyl acetate two to three times. A combined organic layer was washed with a saturated saline solution, dried over anhydrous magnesium sulfate, and concentrated. Residues were purified through MPLC (100% chloroform → 10% methanol-chloroform solution), concentrated and vacuum-dried for five hours to obtain 350 mg of (S)-4-((1-(8-amino-4-chloro-1-oxo-2-phenyl-1,2-dihydroisoquinolin-3-yl)ethyl)amino)pyrido[2,3 -d]pyrimidin-5(8H)-one as a light yellow solid. (Yield : 72.9%)

¹H-NMR (400MHz, CDCl₃) δ ppm 1.67(m, 3H), 5.02(m, 1H), 6.31(d, 1H), 6.45(s, 2H), 6.60(d, 1H), 7.15-7.80(m, 8H), 8.23(m, 1H), 10.95(br, 1H), 11.65(br, 1H)

### <Example 295> Preparation of (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)pivalamide

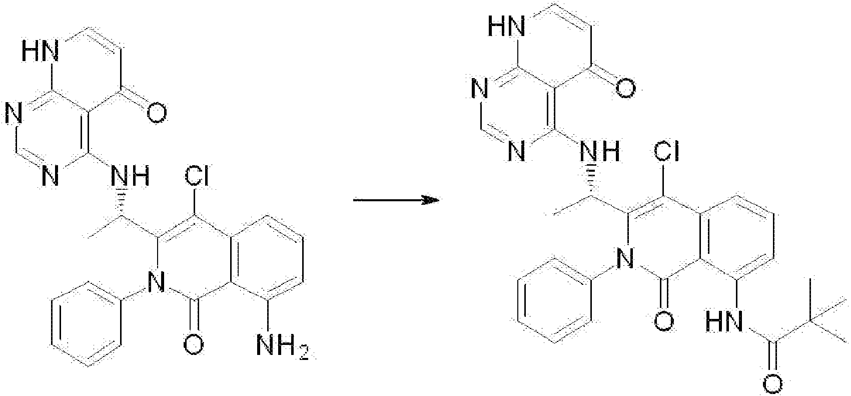

The 20 mg (0.04 mmol) of (S)-4-((1-(8-amino-4-chloro-1-oxo-2-phenyl-1,2-dihydroisoquinolin-3-yl)ethyl)amino)pyrido[2,3 - d]pyrimidin-5(8H)-one prepared in above Example 294, 11 mg (0.08 mmol) of pivaloyl chloride and 0.2 mL of triethylamine were added into 2 mL of THF and stirred at room temperature for three hours. Upon completion of the reaction, a saturated aqueous solution of ammonium chloride was added, followed by extraction with ethyl acetate two to three times. A combined organic layer was washed with a saturated saline solution, dried over anhydrous magnesium sulfate, and concentrated. The resulting product was purified through Prep-LC (under gradient conditions of 20% acetonitrile aqueous solution → 100% acetonitrile), concentrated and vacuum-dried for five hours to obtain 10 mg.

¹H-NMR (400MHz, CDCl₃) δ ppm 1.26(s, 9H), 1.66(d, 3H), 4.97(m, 1H), 6.31(d, 1H), 7.20(m, 1H), 7.45-7.75(m, 7H), 8.25(s, 1H), 8.92(m, 1H), 10.97(br, 1H), 12.84(s, 1H)

### <Examples 296 to 299>

In Examples 296 to 299, the compound corresponding to each Example was prepared by the same preparation method as described in above Example 295, except for using an appropriate reactant in consideration of the structure of the corresponding compound instead of pivaloyl chloride.

### <Example 296> Preparation of (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)acetamide

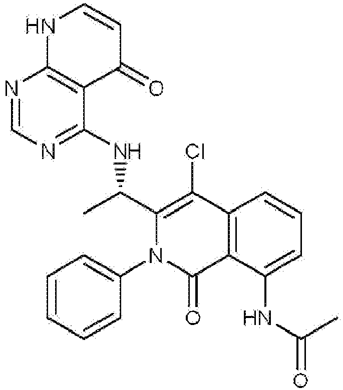

¹H-NMR (400MHz, CDCl₃) δ ppm 1.66(m, 3H), 2.17(s, 3H), 4.99(m, 1H), 6.32(d, 1H), 7.20(m, 1H), 7.45-7.80(m, 7H), 8.25(s, 1H), 8.85(m, 1H), 11.00(br, 1H), 11.93(br, 1H), 12.64(s, 1H)

### <Example 297> Preparation of (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)pentanamide

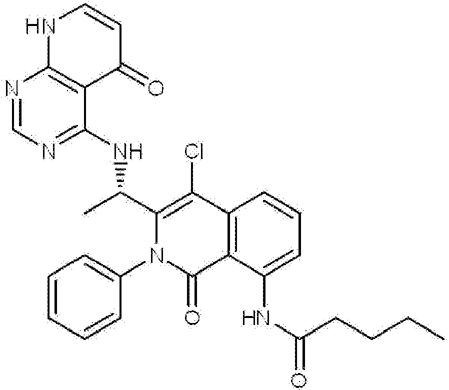

¹H-NMR (400MHz, CDCl₃) δ ppm 0.88(t, 3H), 1.33(m, 2H), 1.67(m, 5H), 2.38(t, 2H), 4.98(m, 1H), 6.31(d, 1H), 7.20(m, 1H), 7.45-7.80(m, 7H), 8.25(s, 1H), 8.89(m, 1H), 10.97(br, 1H), 12.62(s, 1H)

### <Example 298> Preparation of (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)cyclobutanecarboxamide

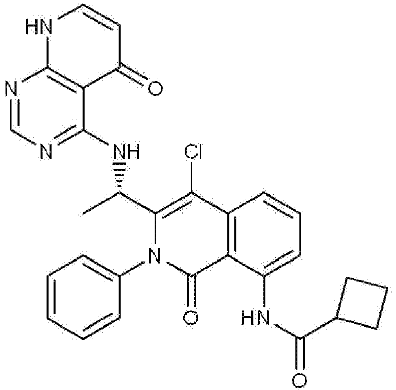

¹H-NMR (400MHz, CDCl₃) δ ppm 1.67(d, 3H), 1.80-2.40(m, 6H), 3.20(m, 1H), 4.97(m, 1H), 6.32(d, 1H), 7.20(m, 1H), 7.45-7.75(m, 7H), 8.18(s, 1H), 8.92(m, 1H), 11.07(br, 1H), 12.54(s, 1H)

### <Example 299> Preparation of (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)benzamide

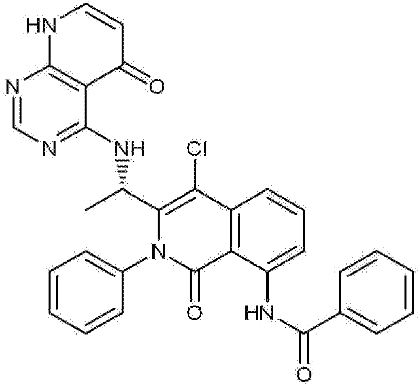

¹H-NMR (400MHz, CDCl₃) δ ppm 1.67(d, 3H), 4.97(m, 1H), 6.33(d, 1H), 7.25(m, 1H), 7.40-8.10(m, 12H), 8.25(s, 1H), 9.08(m, 1H), 11.00(br, 1H), 13.57(s, 1H)

### <Example 300> Preparation of (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)methanesulfonamide

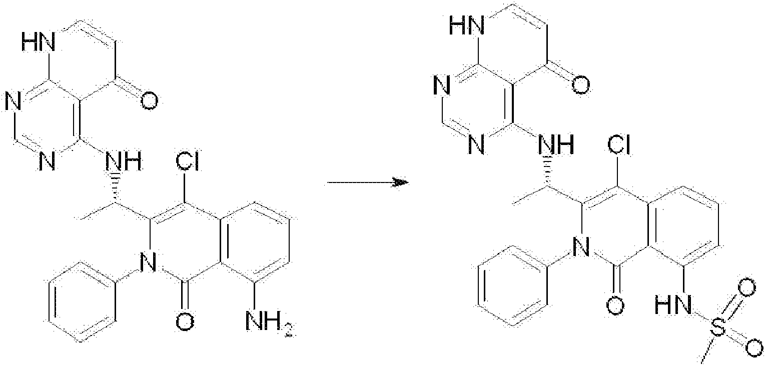

The 20 mg (0.04 mmol) of (S)-4-((1-(8-amino-4-chloro-1-oxo-2-phenyl-1,2-dihydroisoquinolin-3-yl)ethyl)amino)pyrido[2,3-d]pyrimidin-5(8H)-one prepared in above Example 294 and 0.2 mL of pyridine were added into 2 mL of methylene chloride, cooled down to 0°C and stirred. The 30 mg of methanesulfonyl chloride was added dropwise to the reaction mixture, further cooled down for two hours and stirred. Upon completion of the reaction, a saturated aqueous solution of ammonium chloride was added, followed by extraction with ethyl acetate two to three times. A combined organic layer was washed with a saturated saline solution, dried over anhydrous magnesium sulfate, and concentrated. Residues were purified through Prep-LC (under gradient conditions of 20% acetonitrile aqueous solution → 100% acetonitrile), concentrated and vacuum-dried for five hours to obtain 10 mg of (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)methanesulfonamide.

¹H-NMR (400MHz, CDCl₃) δ ppm 1.67(d, 3H), 3.05(s, 3H), 5.01(m, 1H), 6.32(d, 1H), 7.20(m, 1H), 7.45-7.80(m, 8H), 8.27(s, 1H), 11.00(br, 1H), 12.36(s, 1H)

### <Examples 301 to 303>

In Examples 301 to 303, the compound of each Example was prepared by substantially the same preparation method as described in above Example 300, except for using an appropriate reactant in consideration of the structure of the corresponding compound instead of methanesulfonyl chloride.

### <Example 301> Preparation of (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)cyclopropanesulfonamide

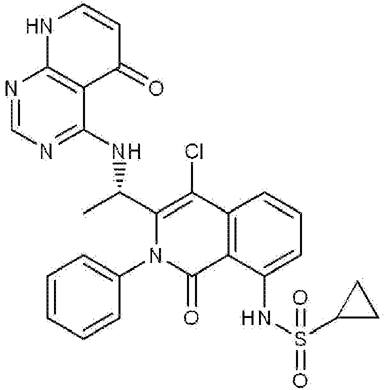

¹H-NMR (400MHz, CDCl₃) δ ppm 1.15-1.55(m, 4H), 1.65(m, 3H), 3.93(m, 1H), 5.00(m, 1H), 6.25(d, 1H), 7.20(m, 1H), 7.45-7.80(m, 7H), 8.25(s, 1H), 8.85(m, 1H), 11.00(br, 1H), 11.93(br, 1H), 12.64(s, 1H)

### <Example 302> Preparation of (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-2,2,2-trifluoroethane-1-sulfonamide

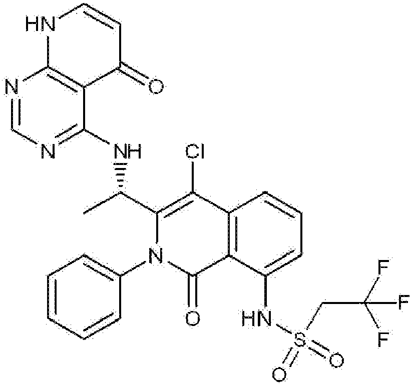

¹H-NMR (400MHz, CDCl₃) δ ppm 1.67(d, 3H), 3.88(q, 2H), 5.01(m, 1H), 6.32(d, 1H), 7.20(m, 1H), 7.45-7.80(m, 8H), 8.27(s, 1H), 10.85(br, 1H), 10.95(br, 1H), 12.96(s, 1H)

### <Example 303> Preparation of (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-4-fluorobenzenesulfonamide

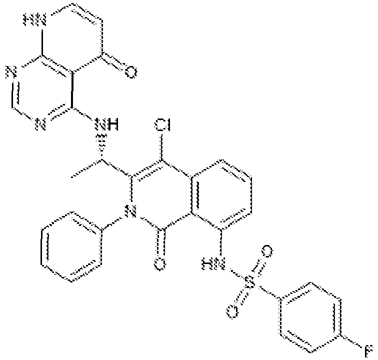

¹H-NMR (400MHz, CDCl₃) δ ppm 1.64(d, 3H), 4.97(m, 1H), 6.31(d, 1H), 7.10(m, 2H), 7.18(m, 1H), 7.51-7.77(m, 8H), 7.90(m, 2H), 8.25(s, 1H), 10.95(br, 1H), 11.30(br, 1H), 12.68(s, 1H)

### <Example 304> Preparation of (S)-4-((1-(4-chloro-1-oxo-2-phenyl-8-(thiophen-2-ylethynyl)-1,2-dihydroisoquinolin-3-yl)ethyl)amino)pyrido[2,3-d]pyrimidin-5(8H)-one

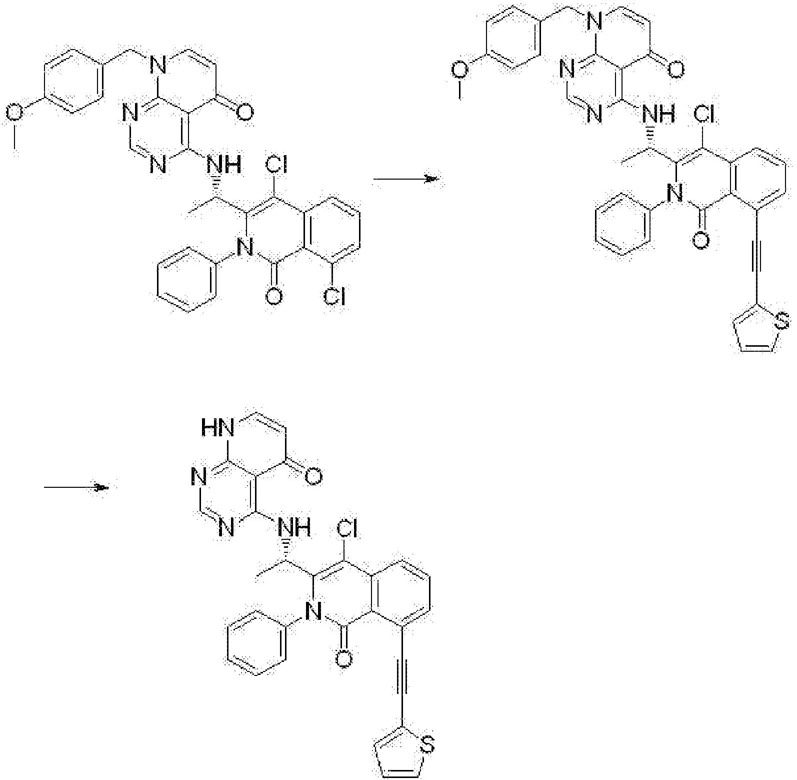

### [Step 1] Preparation of (S)-4-((1-(4-chloro-1-oxo-2-phenyl-8-(thiophen-2-ylethynyl)-1,2-dihydroisoquinolin-3-yl)ethyl)amino)-8-(4-methoxybenzyl)pyrido[2,3-d]pyrimidin-5(8H)-one

The 0.1 g of 4-((1-(4,8-dichloro-1-oxo-2-phenyl-1,2-dihydroisoquinolin-3-yl)ethyl)amino)-8-(4-methoxybenzyl)pyrido[2,3-d]pyrimidin-5(8H)-one prepared in above Preparation Example 3, 0.007 g of bis(acetonitrile)dichloropalladium(II), 0.036 g of 2-dicyclohexylphosphino-2',4',6'-trisisopropylbiphenyl and 0.163 g of cesium carbonate were added to 1.5 mL of propionitrile and stirred. To the solution, 0.036 g of 2-ethynylthiophene was dissolved in 0.5 mL of propionitrile and added dropwise, further heated and stirred at 85°C for 1.5 hours. Upon completion of the reaction, the resulting mixture was filtered through diatomite and washed with 10 mL of ethyl acetate. An organic layer was washed with 10 mL of purified water, dried over anhydrous sodium sulfate, and concentrated. The resulting product was purified through MPLC (under gradient conditions of 100% hexane → 100% ether acetate) to obtain 0.074 g of (S)-4-((1-(4-chloro-1-oxo-2-phenyl-8-(thiophen-2-ylethynyl)-1,2-dihydroisoquinolin-3-yl)ethyl)amino)-8-(4-methoxybenzyl)pyrido[2,3-d]pyrimidin-5(8H)-one.

¹H-NMR (400MHz, CDCl₃) δ ppm 1.66(d, 1H), 3.72(s, 3H), 4.95-5.02(m, 1H), 5.26(s, 2H), 6.21-8.31(m, 18H), 11.08(s, 1H)

### [Step 2] Preparation of (S)-4-((1-(4-chloro-1-oxo-2-phenyl-8-(thiophen-2-ylethynyl)-1,2-dihydroisoquinolin-3-yl)ethyl)amino)pyrido[2,3-d]pyrimidin-5(8H)-one

The 0.074 g of (S)-4-((1-(4-chloro-1-oxo-2-phenyl-8-(thiophen-2-ylethynyl)-1,2-dihydroisoquinolin-3-yl)ethyl)amino)-8-(4-methoxybenzyl)pyrido[2,3-d]pyrimidin-5(8H)-one prepared in above step 1 was dissolved in 0.4 mL of dichloromethane, after which 0.4 mL of trifluoroacetic acid and 0.1 mL of boron trifluoride diethyl etherate were added and stirred at room temperature for eight hours. Upon completion of the reaction, the reaction solution was concentrated under reduced pressure, after which 10 mL of a saturated aqueous solution of ammonium chloride was added, followed by extraction with ethyl acetate two to three times. A combined organic layer was dried over anhydrous sodium sulfate and concentrated to obtain residues, which were then purified by Prep-LC (under gradient conditions of 20% acetonitrile aqueous solution → 100% acetonitrile) to obtain 16.5 g of (S)-4-((1-(4-chloro-1-oxo-2-phenyl-8-(thiophen-2-ylethynyl)-1,2-dihydroisoquinolin-3-yl)ethyl)amino)pyrido[2,3-d]pyrimidin-5(8H)-one.

¹H-NMR (400MHz, CD₃OD) δ ppm 1.80(d, 3H), 5.08-5.17(m, 1H), 6.25-8.29(m, 14H), 11.22(d, 1H)

### <Examples 305 to 316>

In Examples 305 to 316, the compound of each Example was prepared by substantially the same preparation method as described in above Example 304, except for using an appropriate reactant in consideration of the structure of the corresponding compound instead of 2-ethynylthiophene.

### <Example 305> Preparation of (S)-4-((1-(4-chloro-1-oxo-2-phenyl-8-(phenylethynyl)-1,2-dihydroisoquinolin-3-yl)ethyl)amino)pyrido[2,3-d]pyrimidin-5(8H)-one

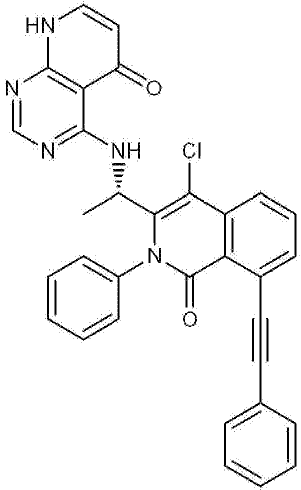

¹H-NMR (400MHz, CD₃OD) δ ppm 1.88(d, 3H), 5.22-5.29(m, 1H), 6.45-8.47(m, 16H), 11.78(d, 1H)

### <Example 4> Preparation of (S)-4-((1-(4-chloro-1-oxo-2-phenyl-8-(pyridin-4-ylethynyl)-1,2-dihydroisoquinolin-3-yl)ethyl)amino)pyrido[2,3-d]pyrimidin-5(8H)-one

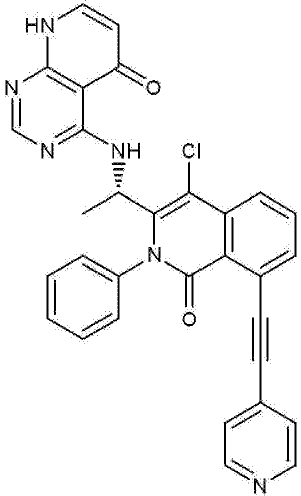

¹H-NMR (400MHz, CDCl₃) δ ppm 1.69(d, 3H), 4.99-5.06(m, 1H), 6.32-8.56(m, 15H), 11.09(d, 1H)

### <Example 307> Preparation of (S)-4-((1-(4-chloro-1-oxo-2-phenyl-8-(pyridin-3-ylethynyl)-1,2-dihydroisoquinolin-3-yl)ethyl)amino)pyrido[2,3-d]pyrimidin-5(8H)-one

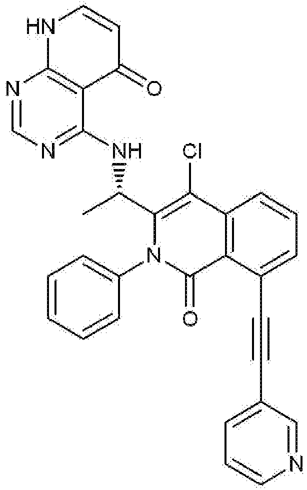

¹H-NMR (400MHz, CDCl₃) δ ppm 1.68(d, 3H), 4.98-5.05(m, 1H), 6.30-8.76(m, 15H), 11.06(d, 1H)

### <Example 308> Preparation of (S)-4-((1-(4-chloro-1-oxo-2-phenyl-8-(pyridin-2-ylethynyl)-1,2-dihydroisoquinolin-3-yl)ethyl)amino)pyrido[2,3-d]pyrimidin-5(8H)-one

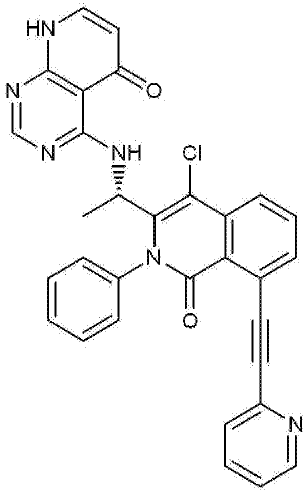

¹H-NMR (400MHz, CDCl₃) δ ppm 1.69(d, 3H), 4.98-5.05(m, 1H), 6.28-8.51(m, 15H), 11.00(d, 1H)

### <Example 309> Preparation of (S)-4-((1-(4-chloro-8-(1-methyl-1H-pyrazol-4-yl)ethynyl)-1-oxo-2-phenyl-1,2-dihydroisoquinolin-3-yl)ethyl)amino)pyrido[2,3-d]pyrimidin-5(8H)-one

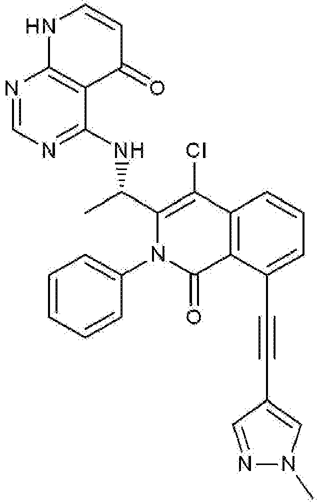

¹H-NMR (400MHz, CD₃OD) δ ppm 1.83(d, 3H), 3.818(s, 3H), 5.13-5.20(m, 1H), 6.40-8.57(m, 13H), 11.62(d, 1H)

### <Example 310> Preparation of (S)-4-((1-(8-([1,1'-biphenyl]-4-ylethynyl)-4-chloro-1-oxo-2-phenyl-1,2-dihydroisoquinolin-3-yl)ethyl)amino)pyrido[2,3-d]pyrimidin-5(8H)-one

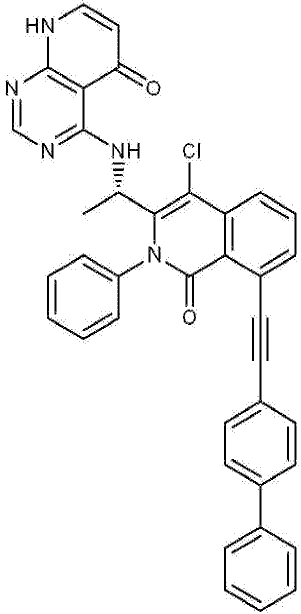

¹H-NMR (400MHz, CD₃OD) δ ppm 1.80(d, 3H), 5.09-5.16(m, 1H), 6.29-8.35(m, 20H), 11.22(d, 1H)

### <Example 311> Preparation of (S)-4-((1-(4-chloro-8-(1-methyl-1H-pyrazol-5-yl)ethynyl)-1-oxo-2-phenyl-1,2-dihydroisoquinolin-3-yl)ethyl)amino)pyrido[2,3-d]pyrimidin-5(8H)-one

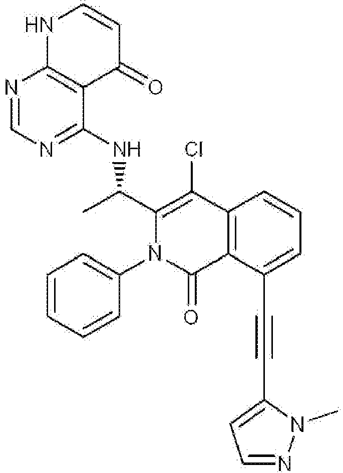

¹H-NMR (400MHz, CDCl₃) δ ppm 1.68(d, 3H), 3.97(s, 3H), 4.98-5.05(m, 1H), 6.29-8.24(m, 13H), 11.04(d, 1H), 12.26(s, 1H)

### <Example 312> Preparation of (S)-4-((1-(4-chloro-1-oxo-2-phenyl-8-(thiazol-2-ylethynyl)-1,2-dihydroisoquinolin-3-yl)ethyl)amino)pyrido[2,3-d]pyrimidin-5(8H)-one

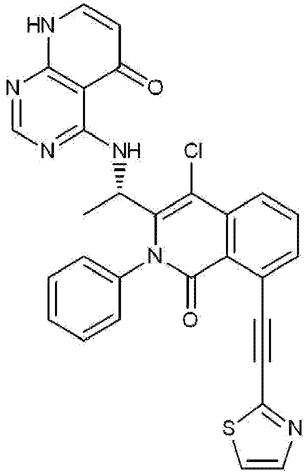

¹H-NMR (400MHz, CDCl₃) δ ppm 1.68(d, 3H), 5.00-5.07(m, 1H), 6.30-8.26(m, 13H), 11.01(d, 1H), 12.22(s, 1H)

### <Example 313> Preparation of (S)-4-((1-(4-chloro-1-oxo-2-phenyl-8-(thiazol-5-ylethynyl)-1,2-dihydroisoquinolin-3-yl)ethyl)amino)pyrido[2,3-d]pyrimidin-5(8H)-one

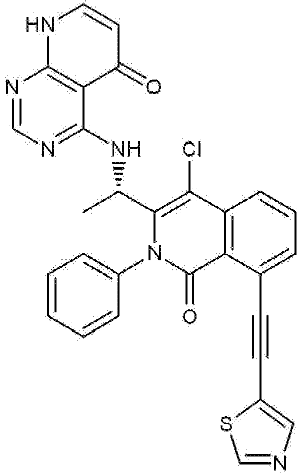

¹H-NMR (400MHz, CDCl₃) δ ppm 1.69(d, 3H), 4.50-4.57(m, 1H), 6.316-8.70(m, 13H), 11.08(d, 1H)

### <Example 314> Preparation of (S)-4-((1-(4-chloro-8-((4-(dimethylamino)phenyl)ethynyl)-1-oxo-2-phenyl-1,2-dihydroisoquinolin-3-yl)ethyl)amino)-5-oxo-5,8-dihydropyrido[2,3-d]pyrimidin-5(8H)-one

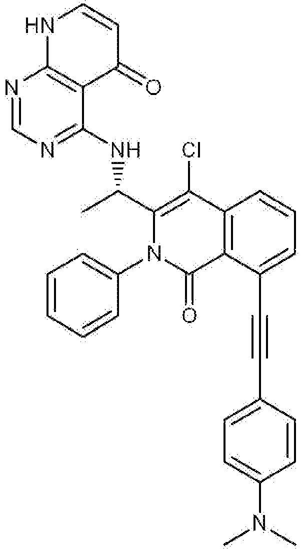

¹H-NMR (400MHz, CD₃OD) δ ppm 1.81(d, 3H), 3.04(s, 6H), 5.10-5.17(m, 1H), 6.30-8.29(m, 17H), 11.01(d, 1H)

### <Example 315> Preparation of (S)-4-((1-(4-chloro-1-oxo-2-phenyl-8-((4-(trifluoromethyl)phenyl)ethynyl)-1,2-dihydroisoquinolin-3-yl)ethyl)amino)-5-oxo-5,8-dihydropyrido[2,3-d]pyrimidin-5(8H)-one

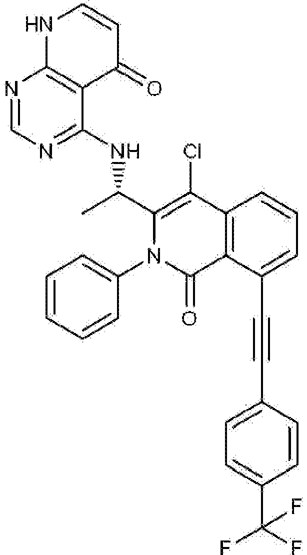

¹H-NMR (400MHz, CD₃OD) δ ppm 1.86(d, 3H), 5.18-5.25(m, 1H), 6.35-8.39(m, 17H), 10.97(d, 1H)

### <Example 316> Preparation of (S)-4-((1-(4-chloro-1-oxo-2-phenyl-8-(pyrimidin-5-ylethynyl)-1,2-dihydroisoquinolin-3-yl)ethyl)amino)pyrido[2,3-d]pyrimidin-5(8H)-one

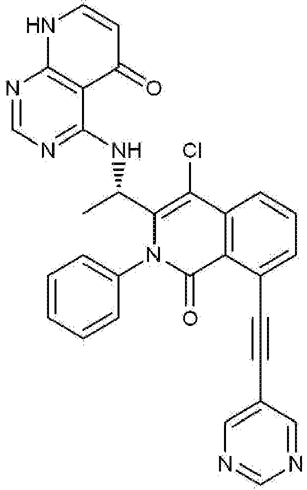

¹H-NMR (400MHz, CD₃OD) δ ppm 1.72(d, 3H), 5.06-5.13(m, 1H), 6.38-9.09(m, 14H), 11.47(d, 1H), 12.86(s, 1H)

### <Example 317> Preparation of (S)-4-((1-(8-(benzo[d]oxazol-2-yl)-4-chloro-1-oxo-2-phenyl-1,2-dihydroisoquinolin-3-yl)ethyl)amino)pyrido[2,3-d]pyrimidin-5(8H)-one

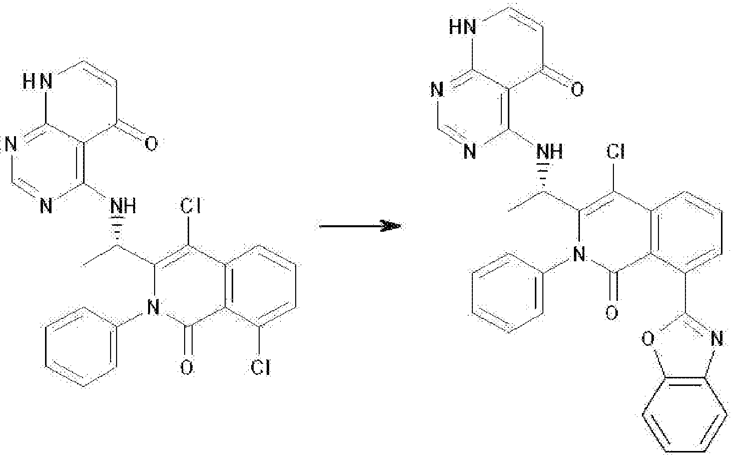

The 0.05 g of (S)-4-((1-(4,8-dichloro-1-oxo-2-phenyl-1,2-dihydroisoquinolin-3-yl)ethyl)amino)pyrido[2,3-d]pyrimidin-5(8H)-one prepared in above Preparation Example 1, 0.067 g of potassium triphosphate, 0.005 g of palladium(II) acetate, 0.008 g of di(1-adamantyl)-n-butylphosphine [cataCXium^{®} A; cas No. 321921-71-5] and 0.025 g of benzoxazole were added into 0.5 mL of N-methylpyrrolidone and irradiated with microwaves at 180°C for 30 minutes. Upon completion of the reaction, the resulting mixture was filtered through diatomite and washed with 10 mL of ethyl acetate. The residues obtained through concentration under reduced pressure were purified by Prep-LC (under gradient conditions of 20% acetonitrile aqueous solution → 100% acetonitrile) and vacuum-dried to obtain 3.3 mg of (S)-4-((1-(8-(benzo[d]oxazol-2-yl)-4-chloro-1-oxo-2-phenyl-1,2-dihydroisoquinolin-3-yl)ethyl)amino)pyrido[2,3-d]pyrimidin-5(8H)-one.

¹H-NMR (400MHz, CDCl₃) δ ppm 1.68(d, 3H), 4.95-5.02(m, 1H), 6.29-8.28(m, 15H), 10.97(d, 1H), 11.06(s, 1H)

### <Example 318> Preparation of (S)-4-((1-(8-(benzo[d]oxazol-2-yl)-1-oxo-2-phenyl-1,2-dihydroisoquinolin-3-yl)ethyl)amino)pyrido[2,3-d]pyrimidin-5(8H)-one

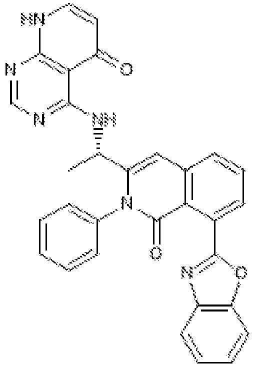

The compound was prepared by the same preparation method as that of Example 317 by using the (S)-4-((1-(8-chloro-1-oxo-2-phenyl-1,2-dihydroisoquinolin-3-yl)ethyl)amino)pyrido[2,3-d]pyrimidin-5(8H)-one prepared in above Preparation Example 2 instead of (S)-4-((1-(4,8-dichloro-1-oxo-2-phenyl-1,2-dihydroisoquinolin-3-yl)ethyl)amino)pyrido[2,3-d]pyrimidin-5(8H)-one.

¹H-NMR (400MHz, CDCl₃) δ ppm 1.48(d, 3H), 4.85-4.92(m, 1H), 6.32-8.23(m, 16H), 10.61(d, 1H), 11.03(s, 1H)

### <Example 319> Preparation of (S)-4-((1-(8-(benzyl(methyl)amino)-4-chloro-1-oxo-2-phenyl-1,2-dihydroisoquinolin-3-yl)ethyl)amino)pyrido[2,3-d]pyrimidin-5(8H)-one

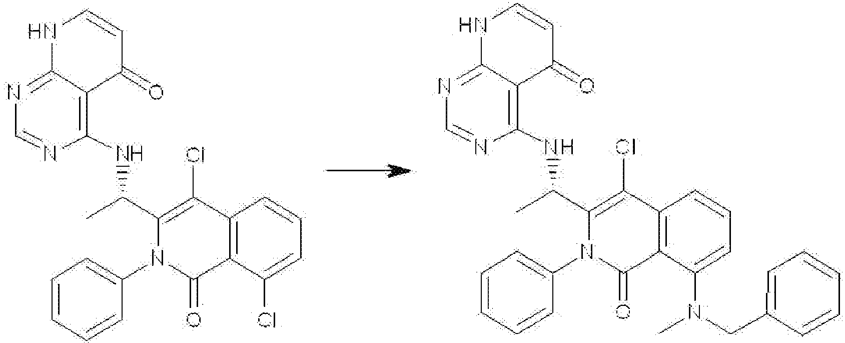

The 50 mg of (S)-4-((1-(4,8-dichloro-1-oxo-2-phenyl-1,2-dihydroisoquinolin-3-yl)ethyl)amino)pyrido[2,3-d]pyrimidin-5(8H)-one prepared in above Preparation Example 1, 10 mg of potassium carbonate and 25 mg of benzylmethylamine were added into 2 mL of dimethyl sulfoxide [DMSO], and stirred at 160°C for 15 hours. Upon completion of the reaction, a saturated aqueous solution of ammonium chloride was added, followed by extraction with ethyl acetate two to three times. A combined organic layer was washed with saturated saline solution, dried over anhydrous magnesium sulfate, and concentrated to obtain residues, which were then purified through Prep-LC (under gradient conditions of 20% acetonitrile aqueous solution → 100% acetonitrile). The resulting product was vacuum-dried for five hours to obtain 23 mg of (S)-4-((1-(8-(benzyl(methyl)amino)-4-chloro-1-oxo-2-phenyl-1,2-dihydroisoquinolin-3-yl)ethyl)amino)pyrido[2,3-d]pyrimidin-5(8H)-one.

¹H-NMR (400MHz, CDCl₃) δ ppm 1.66(d, 3H), 2.78(s, 3H), 4.41(q, 2H), 5.09(m, 1H), 6.28(d, 1H), 6.92(d, 1H), 7.08(m, 2H), 7.22(m, 4H), 7.30-7.70(m, 7H), 8.22(s, 1H), 11.05(d, 1H), 11.90(br, 1H)

### <Examples 320 to 349>

In Examples 320 to 349, the compound of each Example was prepared by substantially the same preparation method as described in above Example 319, except for using an appropriate reactant in consideration of the structure of each compound instead of benzylmethylamine.

### <Example 320> Preparation of (S)-4-((1-(4-chloro-1-oxo-2-phenyl-8-(1H-pyrazol-1-yl)-1,2-dihydroisoquinolin-3-yl)ethyl)amino)pyrido[2,3-d]pyrimidin-5(8H)-one

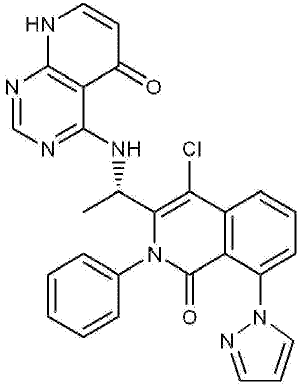

¹H-NMR (400MHz, CDCl₃) δ ppm 1.66(d, 3H), 4.98(m, 1H), 6.30(d, 1H), 6.37(s, 1H), 7.14(d, 1H), 7.40-7.80(m, 9H), 8.16(d, 1H), 8.25(s, 1H), 11.00(br, 1H)

### <Example 321> Preparation of (S)-4-((1-(8-(benzylamino)-4-chloro-l-oxo-2-phenyl-1,2-dihydroisoquinolin-3-yl)ethyl)amino)pyrido[2,3-d]pyrimidin-5(8H)-one

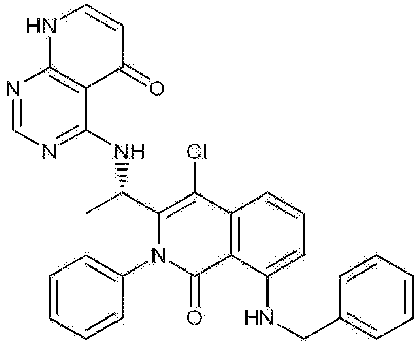

¹H-NMR (400MHz, CDCl₃) δ ppm 1.66(d, 3H), 4.40(d, 2H), 5.04(m, 1H), 6.31(d, 1H), 6.50(d, 1H), 7.10-7.80(m, 13H), 8.25(s, 1H), 9.61(t, 1H), 11.06(br, 1H), 12.05(br, 1H)

### <Example 322> Preparation of (S)-4-((1-(4-chloro-8-(cyclohexyl(methyl)amino)-1-oxo-2-phenyl-1,2-dihydroisoquinolin-3-yl)ethyl)amino)pyrido[2,3-d]pyrimidin-5(8H)-one

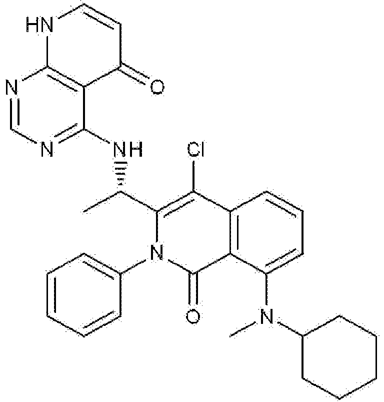

¹H-NMR (400MHz, CDCl₃) δ ppm 1.05-1.95(m, 10H), 1.64(d, 3H), 2.79(s, 3H), 3.35(m, 1H), 5.07(m, 1H), 6.28(d, 1H), 7.05(m, 1H), 7.18(m, 1H), 7.35-7.70(m, 7H), 8.21(s, 1H), 11.00(br, 1H)

### <Example 323> Preparation of (S)-4-((1-(4-chloro-8-(cyclopropylamino)-1-oxo-2-phenyl-1,2-dihydroisoquinolin-3-yl)ethyl)amino)pyrido[2,3-d]pyrimidin-5(8H)-one

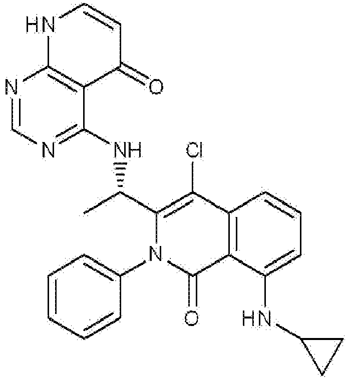

¹H-NMR (400MHz, CDCl₃) δ ppm 0.50(m, 2H), 0.74(m, 2H), 1.65(d, 3H), 2.41(m, 1H), 5.03(m, 1H), 6.31(d, 1H), 7.04(d, 1H), 7.15(m, 2H), 7.53(m, 5H), 7.72(d, 1H), 8.24(s, 1H), 9.18(s, 1H), 11.00(br, 1H), 11.87(br, 1H)

### <Example 324> Preparation of (S)-4-((1-(4-chloro-1-oxo-2-phenyl-8-((pyridin-3-ylmethyl)amino)-1,2-dihydroisoquinolin-3-yl)ethyl)amino)pyrido[2,3-d]pyrimidin-5(8H)-one

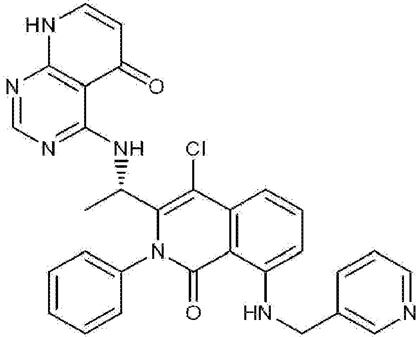

¹H-NMR (400MHz, CDCl₃) δ ppm 1.66(d, 3H), 4.43(d, 2H), 5.05(m, 1H), 6.31(d, 1H), 6.47(d, 1H), 7.20(m, 3H), 7.40-7.80(m, 6H), 8.26(s, 1H), 8.48(d, 1H), 8.59(d, 1H), 9.68(t, 1H), 11.00(br, 1H), 11.45(br, 1H)

### <Example 325> Preparation of (S)-4-((1-(4-chloro-8-(ethylamino)-1-oxo-2-phenyl-1,2-dihydroisoquinolin-3-yl)ethyl)amino)pyrido[2,3-d]pyrimidin-5(8H)-one

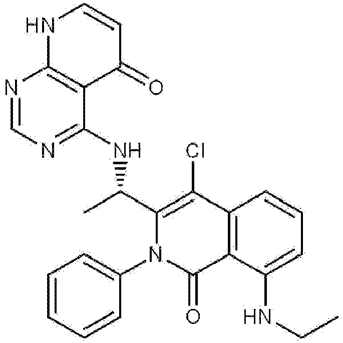

¹H-NMR (400MHz, CDCl₃) δ ppm 1.26(t, 3H), 1.65(d, 3H), 3.18(m, 2H), 5.00(m, 1H), 6.31(d, 1H), 6.55(d, 1H), 7.07(d, 1H), 7.20(d, 1H), 7.50-7.75(m, 5H), 7.74(d(1H), 8.24(s, 1H), 9.02(t, 1H), 11.00(s, 1H)

### <Example 326> Preparation of (S)-4-((1-(4-chloro-1-oxo-2-phenyl-8-(4-phenylpiperazin-1-yl)-1,2-dihydroisoquinolin-3-yl)ethyl)amino)pyrido[2,3-d]pyrimidin-5(8H)-one

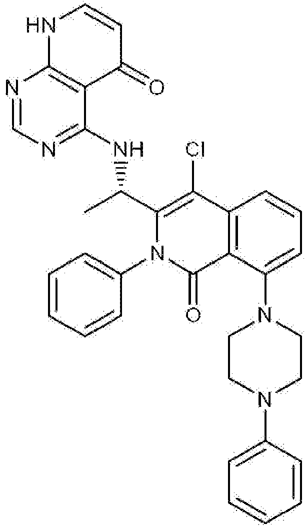

¹H-NMR (400MHz, CDCl₃) δ ppm 1.66(d, 3H), 3.22-3.41(m, 8H), 5.03-5.10(m, 1H), 6.31-8.22(m, 16H), 11.20(d, 1H)

### <Example 327> Preparation of 4-(((S)-1-(4-chloro-1-oxo-2-phenyl-8-((1S,5R)-1,3,3-trimethyl-6-azabicyclo[3.2.1]octan-6-yl)-1,2-dihydroisoquinolin-3-yl)ethyl)amino)pyrido[2,3-d]pyrimidin-5(8H)-one

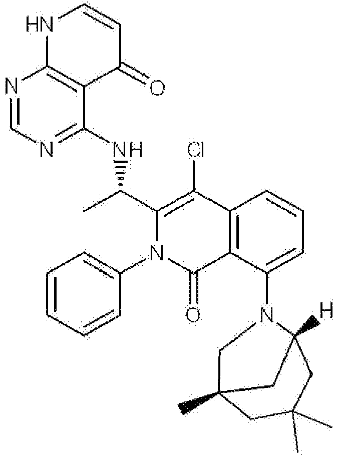

¹H-NMR (400MHz, CDCl₃) δ ppm 0.56(s, 3H), 0.87(s, 3H), 1.06(s, 3H), 1.23-1.47(m, 4H), 1.63(d, 3H), 1.62-1.64(m, 1H), 2.18(d, 1H), 2.90(d, 1H), 3.59(d, 1H), 4.37(s, 1H), 5.12-5.19(m, 1H), 6.28-8.17(m, 11H), 11.48(d, 1H), 11.61(s, 1H)

### <Example 328> Preparation of 4'-chloro-3'-((S)-1-((5-oxo-5,8-dihydropyrido[2,3-d]pyrimidin-4-yl)amino)ethyl)-2'-phenyl-3,4,4a,5,6,7,8,8a-octahydro-1H-[2,8'-biisoquinolin]-1'(2'H)-one

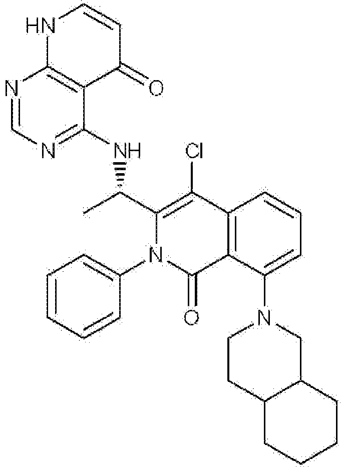

¹H-NMR (400MHz, CDCl₃) δ ppm 0.86-1.67(m, 17H), 3.23(t, 1H), 3.49(t, 1H), 5.00-5.08(m, 1H), 6.27-8.22(m, 11H), 11.02(d, 1H), 11.66(s, 1H)

### <Example 329> Preparation of (S)-4'-chloro-7-methyl-3'-(1-((5-oxo-5,8-dihydropyrido[2,3-d]pyrimidin-4-yl)amino)ethyl)-2'-phenyl-3,4-dihydro-1H-[2,8'-biisoquinolin]-1'(2'H)-one

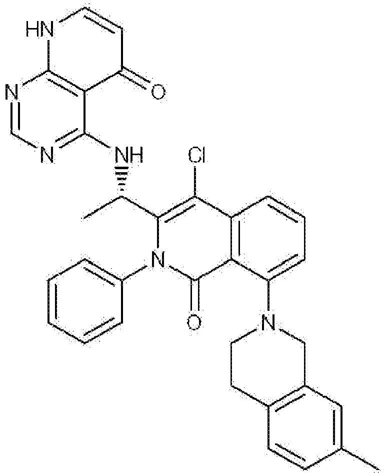

¹H-NMR (400MHz, CDCl₃) δ ppm 1.66(d, 3H), 2.26(s, 3H), 2.90-3.48(m, 4H), 4.35(s, 2H), 5.05-5.12(m, 1H), 6.26-8.23(m, 14H), 11.01(d, 1H), 11.31(s, 1H)

### <Example 330> Preparation of (S)-4-((1-(4-chloro-8-(4,7-dihydrothieno[2,3-c]pyridin-6(5H)-yl)-1-oxo-2-phenyl-1,2-dihydroisoquinolin-3-yl)ethyl)amino)pyrido[2,3-d]pyrimidin-5(8H)-one

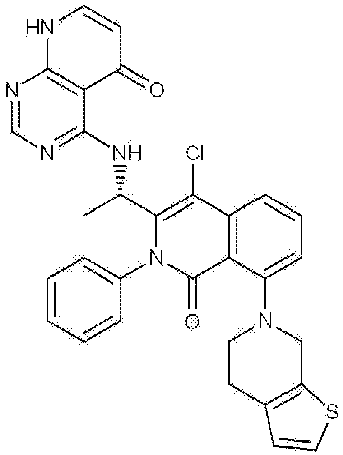

¹H-NMR (400MHz, CDCl₃) δ ppm 1.66(d, 3H), 2.26(s, 3H), 2.90-3.48(m, 4H), 4.35(s, 2H), 5.05-5.12(m, 1H), 6.27-8.22(m, 13H), 11.02(d, 1H), 11.59(s, 1H)

### <Example 331> Preparation of 4-(((1S)-1-(4-chloro-1-oxo-2-phenyl-8-((tetrahydrofuran-3-yl)amino)-1,2-dihydroisoquinolin-3-yl)ethyl)amino)pyrido[2,3-d]pyrimidin-5(8H)-one

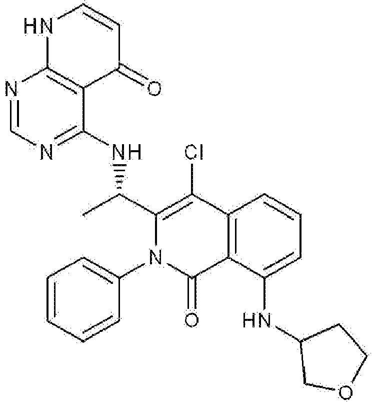

¹H-NMR (400MHz, CDCl₃) δ ppm 1.65(d, 3H), 1.87-1.95(m, 1H), 2.23-2.32(m, 1H), 3.66-4.14(m, 5H), 4.97-4.99(m, 1H), 6.30-8.26(m, 11H), 9.35(d, 1H), 10.95(d, 1H), 11.14(s, 1H)

### <Example 332> Preparation of (S)-4-((1-(4-chloro-1-oxo-2-phenyl-8-((tetrahydro-2H-pyran-4-yl)amino)-1,2-dihydroisoquinolin-3-yl)ethyl)amino)pyrido[2,3-d]pyrimidin-5(8H)-one

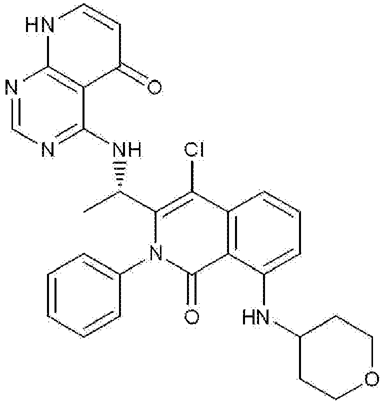

¹H-NMR (400MHz, CDCl₃) δ ppm 1.54-2.02(m, 5H), 1.99-2.02(m, 2H), 3.47-4.00(m, 5H), 4.94-5.01(m, 1H), 6.30-8.25(m, 11H), 9.25(d, 1H), 10.95(d, 1H), 11.05(s, 1H)

### <Example 333> Preparation of (S)-4-((1-(4-chloro-1-oxo-2-phenyl-8-(4-(pyridin-2-yl)piperazin-1-yl)-1,2-dihydroisoquinolin-3-yl)ethyl)amino)pyrido[2,3-d]pyrimidin-5(8H)-one

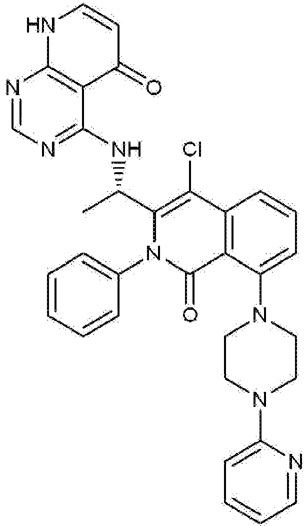

¹H-NMR (400MHz, CD₃OD) δ ppm 1.65(d, 3H), 3.20(s, 4H), 3.72(s, 4H), 5.04-5.08(m, 1H), 6.29(d, 1H), 6.58-6.63(m, 2H), 7.06-8.17(m, 13H), 8.23(s, 1H), 10.90-11.10(m, 1H), 11.30(br, 1H)

### <Example 334> Preparation of (S)-4-((1-(4-chloro-8-(methyl(naphthalen-2-ylmethyl)amino)-1-oxo-2-phenyl-1,2-dihydroisoquinolin-3-yl)ethyl)amino)pyrido[2,3-d]pyrimidin-5(8H)-one

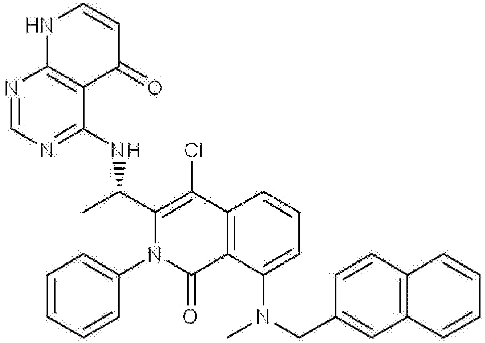

¹H-NMR (400MHz, CDCl₃) δ ppm 1.67(d, 3H), 2.83(s, 3H), 4.57(d, 2H), 5.08-5.15(m, 1H), 6.25-8.22(m, 18H), 10.82(s, 1H), 11.00(d, 1H)

### <Example 335> Preparation of (S)-4-((1-(4-chloro-8-((4-chlorophenethyl)amino)-1-oxo-2-phenyl-1,2-dihydroisoquinolin-3-yl)ethyl)amino)pyrido[2,3-d]pyrimidin-5(8H)-one

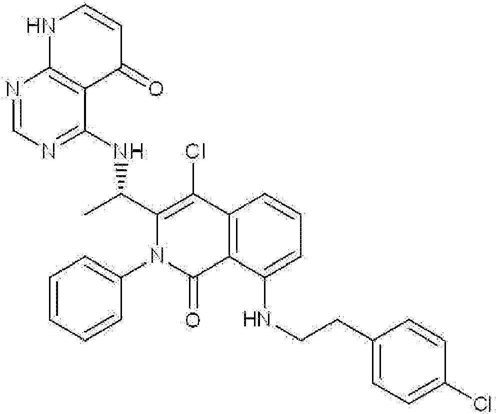

¹H-NMR (400MHz, CDCl₃) δ ppm 1.65(d, 3H), 2.89(m, 2H), 3.37(m, 2H), 5.02(m, 1H), 6.30(d, 1H), 6.58(d, 1H), 7.10-7.80(m, 12H), 8.24(s, 1H), 9.29(t, 1H), 10.70(br, 1H), 11.00(br, 1H)

### <Example 336> Preparation of (S)-4-((1-(4-chloro-1-oxo-2-phenyl-8-(4-phenylpiperidin-1-yl)-1,2-dihydroisoquinolin-3-yl)ethyl)amino)pyrido[2,3-d]pyrimidin-5(8H)-one

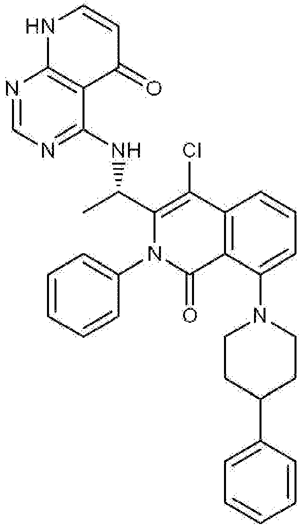

¹H-NMR (400MHz, CDCl₃) δ ppm 1.64-3.59(m, 12H), 5.01-5.08 (m, 1H), 6.27-8.22(m, 16H), 11.02(d, 1H), 11.64(s, 1H)

### <Example 337> Preparation of (S)-1-(4-chloro-1-oxo-3-((S)-1-((5-oxo-5,8-dihydropyrido[2,3-d]pyrimidin-4-yl)amino)ethyl)-2-phenyl-1,2-dihydroisoquinolin-8-yl)pyrrolidine-2-carboxamide

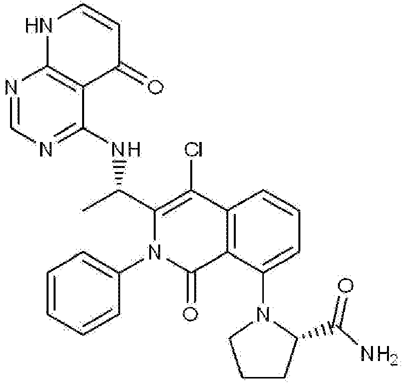

¹H-NMR (400MHz, CD₃OD) δ ppm 1.66(d, 3H), 1.71-1.78(m, 1H), 1.85-2.06(m, 2H), 2.41-2.58(m, 1H), 2.82-2.88(m, 1H), 4.02-4.03(m, 1H), 4.52(t, 1H), 5.01-5.05(m, 1H), 6.28(d, 1H), 7.10-7.98(m, 9H), 8.26(s, 1H), 11.00-11.01(m, 1H)

### <Example 338> Preparation of 4-(((S)-1-(4-chloro-1-oxo-2-phenyl-8-((S)-2-(pyrrolidin-1-ylmethyl)pyrrolidin-1-yl)-1,2-dihydroisoquinolin-3-yl)ethyl)amino)pyrido[2,3-d]pyrimidin-5(8H)-one

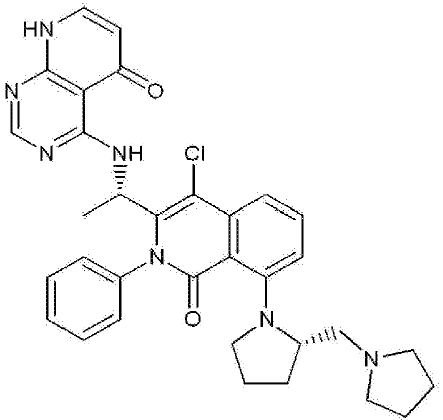

¹H-NMR (400MHz, CD₃OD) δ ppm 1.66(d, 3H), 1.67(m, 1H), 1.80-1.84(m, 3H), 1.87(m, 4H), 2.36(m, 1H), 2.62(s, 3H), 2.80(m, 1H), 3.05(m, 1H), 3.26(m, 1H), 3.85(m, 1H), 4.75(m, 1H), 5.00-5.03(m, 1H), 6.28(d, 1H), 7.14-7.67(m, 9H), 8.30(s, 1H), 11.02-11.04(m, 1H), 12.10(br, 1H)

### <Example 339> Preparation of 4-(((1S)-1-(4-chloro-8-(2-hydroxypyrrolidin-1-yl)-1-oxo-2-phenyl-1,2-dihydroisoquinolin-3-yl)ethyl)amino)pyrido[2,3-d]pyrimidin-5(8H)-one

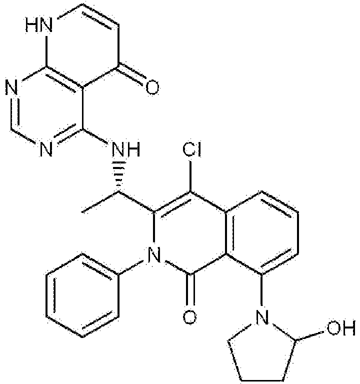

¹H-NMR (400MHz, CD₃OD) δ ppm 1.65(d, 3H), 2.01(m, 1H), 2.11(m, 1H), 3.17(m, 1H), 3.23(m, 1H), 3.84(m, 1H), 4.44(m, 1H), 5.01-520(m, 2H), 6.21(d, 1H), 6.87-7.65(m, 9H), 8.22(s, 1H), 10.88(br, 1H), 10.98-11.05(m, 1H)

### <Example 340> Preparation of (S)-4-((1-(4-chloro-8-(isoindolin-2-yl)-1-oxo-2-phenyl-1,2-dihydroisoquinolin-3-yl)ethyl)amino)pyrido[2,3-d]pyrimidin-5(8H)-one

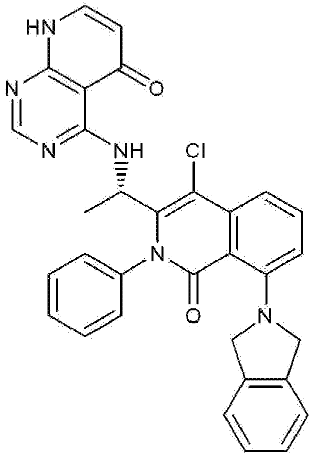

¹H-NMR (400MHz, CD₃OD) δ ppm 1.66(d, 3H), 4.64(d, 2H), 4.89(d, 2H), 5.08-5.13(m, 1H), 6.27(d, 1H), 6.98-7.68(m, 13H), 8.24(s, 1H), 10.90(br, 1H), 11.01-11.06(m, 1H)

### <Example 341> Preparation of (S)-4-((1-(4-chloro-8-(indolin-1-yl)-1-oxo-2-phenyl-1,2-dihydroisoquinolin-3-yl)ethyl)amino)pyrido[2,3-d]pyrimidin-5(8H)-one

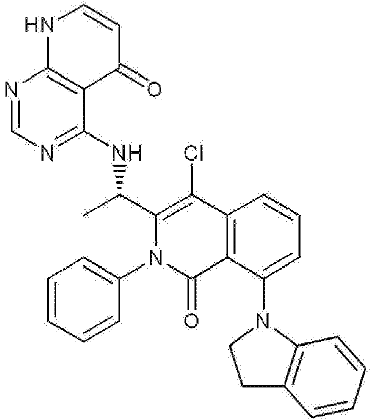

¹H-NMR (400MHz, CD₃OD) δ ppm 1.68(d, 3H), 3.01-3.15(m, 2H), 4.05(br, 2H), 5.01-5.13(m, 1H), 6.28(d, 1H), 6.98-7.68(m, 13H), 8.26(s, 1H), 10.25(br, 1H), 10.86-11.02(m, 1H)

### <Example 342> Preparation of 4-(((1S)-1-(4-chloro-8-(hexahydrocyclopenta[c]pyrrol-2(1H)-yl)-1-oxo-2-phenyl-1,2-dihydroisoquinolin-3-yl)ethyl)amino)pyrido[2,3-d]pyrimidin-5(8H)-one

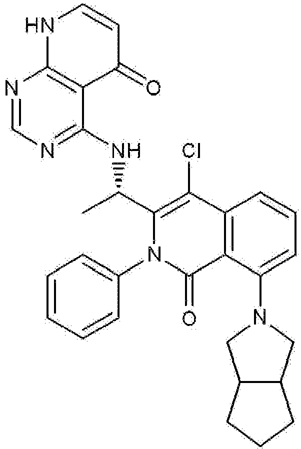

¹H-NMR (400MHz, CD₃OD) δ ppm 1.53(m, 1H), 1.53-1.55(m, 2H), 1.68(m, 4H), 1.70(m, 2H), 2.69-2.75(m, 3H), 2.82-2.91(m, 1H), 3.46-3.52(m, 1H), 3.66-3.75(m, 1H), 5.07-5.11(m, 1H), 6.27(d, 1H), 6.86-7.65(m, 9H), 8.22(s, 1H), 11.03-11.04(m, 1H), 11.85(br, 1H)

### <Example 343> Preparation of (S)-4-((1-(4-chloro-1-oxo-2-phenyl-8-(phenylamino)-1,2-dihydroisoquinolin-3-yl)ethyl)amino)pyrido[2,3-d]pyrimidin-5(8H)-one

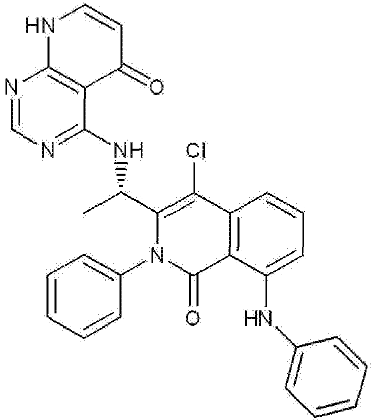

¹H-NMR (400MHz, CDCl₃) δ ppm 1.68(d, 3H), 5.03(m, 1H), 6.31(d, 1H), 7.05(m, 1H), 7.15-7.32(m, 7H), 7.41-7.80(m, 6H), 8.27(s, 1H), 10.96(s, 1H), 10.70(br, 1H), 11.40(br, 1H)

### <Example 344> Preparation of (S)-4-((1-(4-chloro-8-((4-fluorophenyl)amino)-1-oxo-2-phenyl-1,2-dihydroisoquinolin-3-yl)ethyl)amino)pyrido[2,3-d]pyrimidin-5(8H)-one

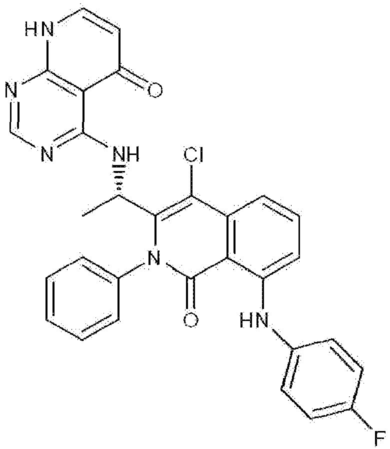

¹H-NMR (400MHz, CDCl₃) δ ppm 1.68(d, 3H), 5.03(m, 1H), 6.30(d, 1H), 6.95-7.05(m, 3H), 7.15-7.80(m, 10H), 8.25(s, 1H), 10.70(br, 1H), 10.90(s, 1H), 10.95(br, 1H)

### <Example 345> Preparation of (S)-4-((1-(4-chloro-8-((4-methoxyphenyl)amino)-1-oxo-2-phenyl-1,2-dihydroisoquinolin-3-yl)ethyl)amino)pyrido[2,3-d]pyrimidin-5(8H)-one

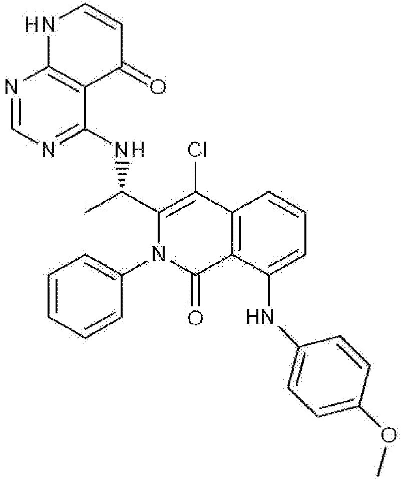

¹H-NMR (400MHz, CDCl₃) δ ppm 1.67(d, 3H), 3.79(s, 3H), 5.03(m, 1H), 6.31(d, 1H), 6.85(m, 2H), 6.92(d, 1H), 7.10-7.80(m, 10H), 8.27(s, 1H), 10.70(s, 1H), 10.95(br, 1H), 11.10(br, 1H)

### <Example 346> Preparation of (S)-4-((1-(4-chloro-1-oxo-2-phenyl-8-((4-(trifluoromethyl)phenyl)amino)-1,2-dihydroisoquinolin-3-yl)ethyl)amino)pyrido[2,3-d]pyrimidin-5(8H)-one

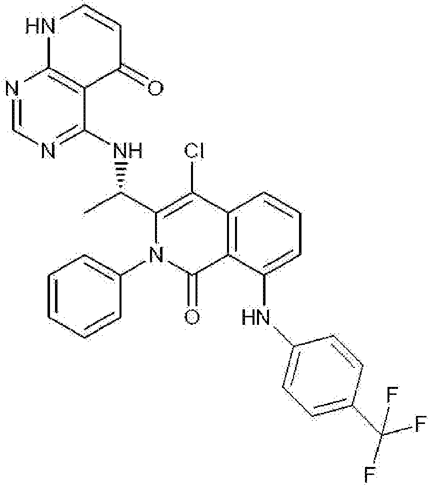

¹H-NMR (400MHz, CDCl₃) δ ppm 1.69(d, 3H), 5.03(m, 1H), 6.30(d, 1H), 7.20-7.80(m, 13H), 8.27(s, 1H), 10.45(br, 1H), 10.90(br, 1H), 11.23(s, 1H)

### <Example 347> Preparation of (S)-4-((1-(4-chloro-1-oxo-2-phenyl-8-(pyridin-3-ylamino)-1,2-dihydroisoquinolin-3-yl)ethyl)amino)pyrido[2,3-d]pyrimidin-5(8H)-one

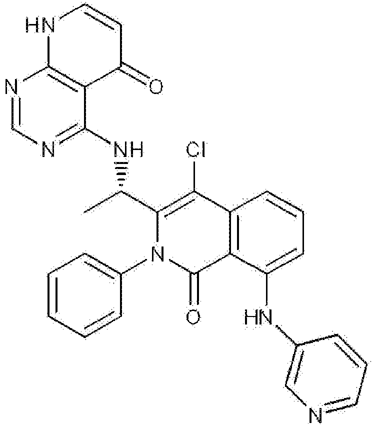

¹H-NMR (400MHz, CDCl₃) δ ppm 1.69(d, 3H), 5.01(m, 1H), 6.30(d, 1H), 7.18(d, 1H), 7.25(m, 2H), 7.35(d, 1H), 7.45-7.80(m, 7H), 8.27(s, 1H), 8.30(m, 1H), 8.55(s, 1H), 10.95(br, 1H), 11.05(s, 1H), 11.35(br, 1H)

### <Example 348> Preparation of (S)-4-((1-(4-chloro-1-oxo-2-phenyl-8-(pyridin-2-ylamino)-1,2-dihydroisoquinolin-3-yl)ethyl)amino)pyrido[2,3-d]pyrimidin-5(8H)-one

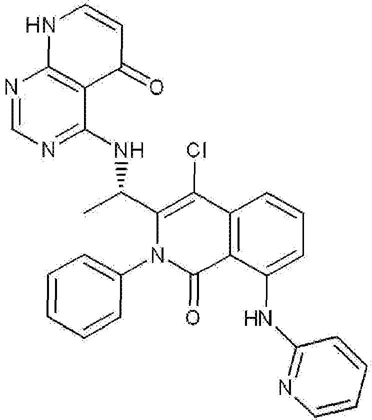

¹H-NMR (400MHz, CDCl₃) δ ppm 1.67(d, 3H), 5.00(m, 1H), 6.30(d, 1H), 6.80(m, 2H), 7.25(m, 1H), 7.40-7.80(m, 8H), 8.26(s, 1H), 8.27(m, 1H), 8.97(d, 1H), 11.00(br, 1H), 11.75(br, 1H), 12.19(s, 1H)

### <Example 349> Preparation of (S)-4-((1-(4-chloro-8-(methyl(pyridin-2-yl)amino)-1-oxo-2-phenyl-1,2-dihydroisoquinolin-3-yl)ethyl)amino)pyrido[2,3-d]pyrimidin-5(8H)-one

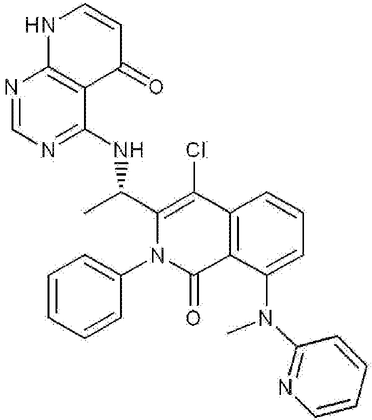

¹H-NMR (400MHz, CDCl₃) δ ppm 1.69(d, 3H), 3.34(s, 3H), 5.05(m, 1H), 6.27(m, 2H), 6.47(m, 1H), 7.05-7.30(m, 2H), 7.35-7.60(m, 6H), 7.70-7.80(m, 1H), 7.95-8.05(m, 2H), 8.15-8.30(m, 1H), 10.30(br, 1H), 10.95(br, 1H)

### <Example 350> Preparation of (S,Z)-4-chloro-N'-hydroxy-1-oxo-3-(1-((5-oxo-5,8-dihydropyrido[2,3-d]pyrimidin-4-yl)amino)ethyl)-2-phenyl-1,2-dihydroisoquinoline-8-carboximidamide

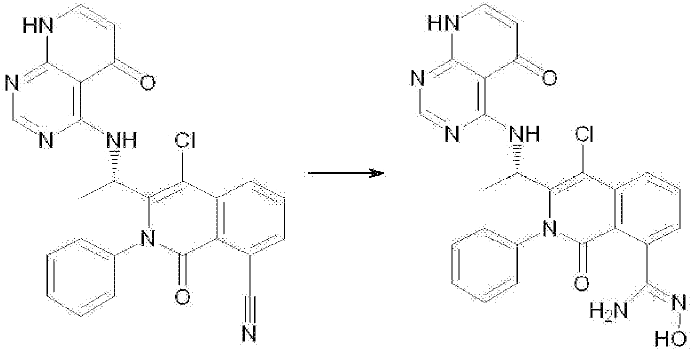

The 12 mg of hydroxyamine hydrochloride and 1 mL of saturated aqueous solution of sodium hydrogen carbonate were added into a reactor, after which 67 mg of (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carbonitrile prepared in above Example 258 was dissolved in 2 mL of ethanol and added to the solution, and stirred under reflux for 15 hours. Upon completion of the reaction, the reaction solution was concentrated, after which a saturated aqueous solution of ammonium chloride was added, followed by extraction with ethyl acetate two to three times. A combined organic layer was washed with saturated saline solution, dried over anhydrous magnesium sulfate, and concentrated to obtain residues, which were then purified through Prep-LC. The resulting product was vacuum-dried for five hours to obtain 28 mg of (S,Z)-4-chloro-N'-hydroxy-1-oxo-3-(1-((5-oxo-5,8-dihydropyrido[2,3-d]pyrimidin-4-yl)amino)ethyl)-2-phenyl-1,2-dihydroisoquinolin-8-carboximidamide.

¹H-NMR (400MHz, CDCl₃) δ ppm 1.66(d, 3H), 4.91(s, 1H), 5.05(m, 1H), 6.16(d, 1H), 7.17(m, 2H), 7.45-7.80(m, 6H), 8.05-8.30(m, 2H), 10.95(br, 1H)

### <Example 351> Preparation of (S)-4-((1-(4-chloro-8-(1,2,4-oxadiazol-3-yl)-1-oxo-2-phenyl-1,2-dihydroisoquinolin-3-yl)ethyl)amino)pyrido[2,3-d]pyrimidin-5(8H)-one

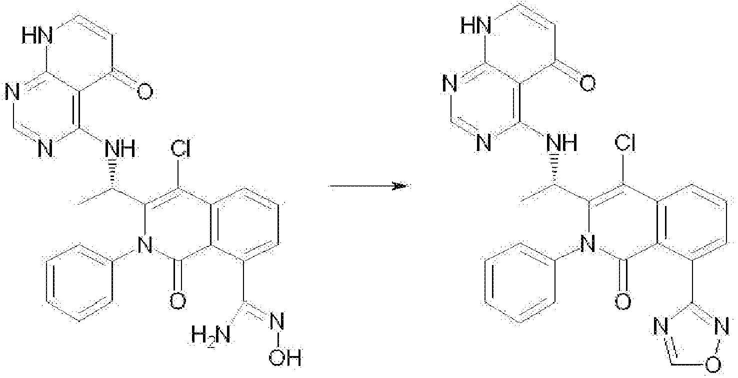

The 20 mg of (S,Z)-4-chloro-N'-hydroxy-1-oxo-3-(1-((5-oxo-5,8-dihydropyrido[2,3-d]pyrimidin-4-yl)amino)ethyl)-2-phenyl-1,2-dihydroisoquinolin-8-carboximidamide prepared in above Example 350, 1.6 mg of iron chloride (FeCl₃) and 1.2 mg of L-proline were added into 1 mL of triethyl orthoformate, and stirred under reflux for 15 hours. Upon completion of the reaction, a saturated aqueous solution of ammonium chloride was added, followed by extraction with ethyl acetate two to three times. A combined organic layer was washed with saturated saline solution, dried over anhydrous magnesium sulfate, and concentrated to obtain residues, which were then purified through Prep-LC. The resulting product was vacuum-dried for five hours to obtain 0.8 mg of (S)-4-((1-(4-chloro-8-(1,2,4-oxadiazol-3-yl)-1-oxo-2-phenyl-1,2-dihydroisoquinoline-3-yl)ethyl)amino)pyrido[2,3-d]pyrimidin-5(8H)-one.

¹H-NMR (400MHz, CDCl₃) δ ppm 1.66(d, 3H), 5.04(m, 1H), 6.32(d, 1H), 7.10-7.90(m, 8H), 8.23(m, 2H), 8.70(s, 1H), 10.95(br, 1H)

### <Example 352> Preparation of (S)-4-((1-(4-chloro-1-oxo-2-phenyl-8-(1H-tetrazol-5-yl)-1,2-dihydroisoquinolin-3-yl)ethyl)amino)pyrido[2,3-d]pyrimidin-5(8H)-one

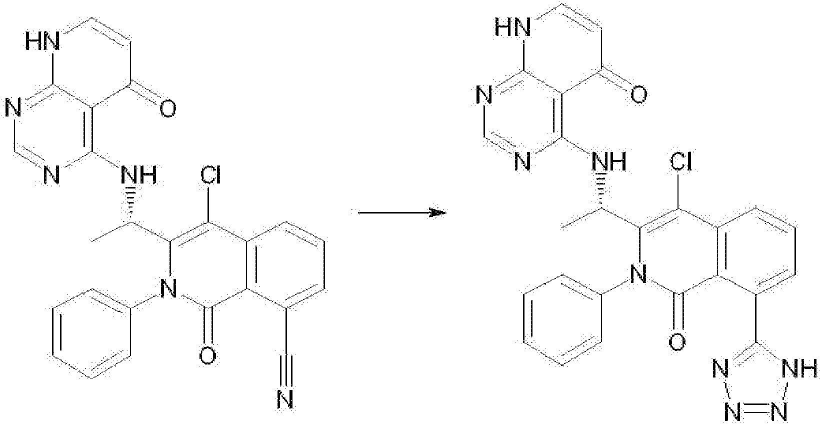

The 50 mg of (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carbonitrile prepared in above Example 265, 12 mg of ammonium chloride and 14 mg of sodium azide were added into 1 mL of dimethylformamide [DMF], and stirred under reflux at 170°C for 15 hours. Upon completion of the reaction, the resulting mixture was cooled down to room temperature, after which a saturated aqueous solution of ammonium chloride was added, followed by extraction with ethyl acetate two to three times. A combined organic layer was washed with saturated saline solution, dried over magnesium sulfate, and concentrated to obtain residues, which were then purified through Prep-LC. The resulting product was vacuum-dried for five hours to obtain 3 mg of (S)-4-((1-(4-chloro-1-oxo-2-phenyl-8-(1H-tetrazol-5-yl)-1,2-dihydroisoquinolin-3-yl)ethyl)amino)pyrido[2,3-d]pyrimidin-5(8H)-one.

¹H-NMR (400MHz, CDCl₃) δ ppm 1.66(d, 3H), 5.03(m, 1H), 6.31(d, 1H), 7.20-8.00(m, 7H), 8.25(m, 1H), 8.40(d, 1H), 8.91(d, 1H), 10.95(br, 1H)

### <Example 353> Preparation of (S)-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)boronic acid

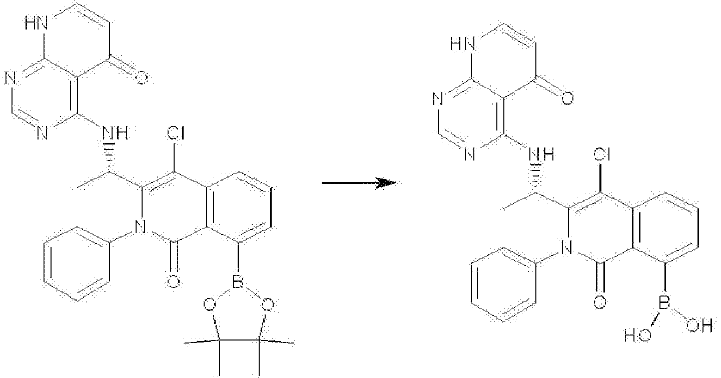

The 120 mg of (S)-4-((1-(4-chloro-1-oxo-2-phenyl-8-(4,4,5,5-tetramethyl-1,3,2-dioxaborolan-2-yl)-1,2-dihydroisoquinolin-3-yl)ethyl)amino)pyrido[2,3-d]pyrimidin-5(8H)-one prepared in above Example 2 were added into a mixed solution of 10 mL of acetone and 5 mL of purified water and stirred for five minutes, after which 135 mg of sodium periodate and 49 mg of ammonium acetate were added and stirred at room temperature for 15 hours. Upon completion of the reaction, the reaction solution was concentrated, after which a saturated aqueous solution of ammonium chloride was added, followed by extraction with ethyl acetate two to three times. A combined organic layer was washed with saturated saline solution, dried over magnesium sulfate, and concentrated to obtain residues, which were then purified through Prep-PLC. The resulting product was vacuum-dried for five hours to obtain 40 mg of (S)-(4-chloro-1-oxo-3-(1-((5-oxo-5,8-dihydropyrido[2,3-d]pyrimidin-4-yl)amino)ethyl) -2-phenyl-1,2-dihydroisoquinolin-8-yl)boronic acid.

¹H-NMR (400MHz, CD₃OD) δ ppm 1.69(d, 3H), 5.08(m, 1H), 6.28(d, 1H), 7.30-7.90(m, 8H), 7.99(d, 1H), 8.26(s, 1H)

### <Example 354> Preparation of (S)-4-((1-(4-chloro-8-ethyl-1-oxo-2-phenyl-1,2-dihydroisoquinolin-3-yl)ethyl)amino)pyrido[2,3-d]pyrimidin-5(8H)-one

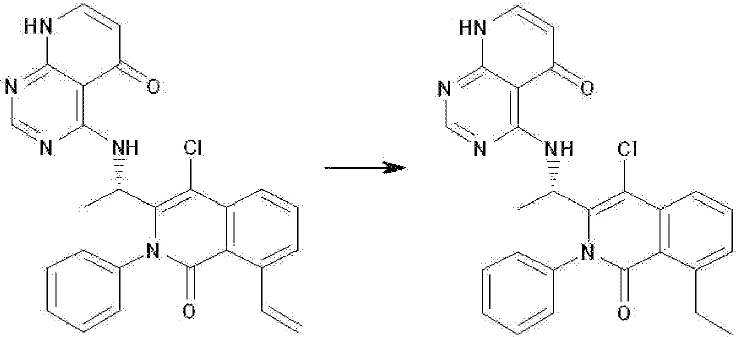

The 25 mg of (S)-4-((1-(4-chloro-1-oxo-2-phenyl-8-vinyl-1,2-dihydroisoquinolin-3-yl)ethyl)amino)pyrido[2,3-d]pyrimidin-5(8H)-one prepared in above Example 142 and 10 mg of palladium were added into 5 mL of methanol, filled with hydrogen, and stirred at room temperature for 15 hours. Upon completion of the reaction, the reaction mixture was filtered through diatomite and concentrated to obtain residues, which were then purified through Prep-LC. The resulting product was vacuum-dried for five hours to obtain 12 mg of (S)-4-((1-(4-chloro-8-ethyl-1-oxo-2-phenyl-1,2-dihydroisoquinolin-3-yl)ethyl)amino)pyrido[2,3-d]pyrimidin-5(8H)-one.

¹H-NMR (400MHz, CDCl₃) δ ppm 1.22(t, 3H), 1.68(d, 3H), 3.32(m, 2H), 5.05(m, 1H), 6.30(d, 1H), 7.20-7.80(m, 8H), 7.92(d, 1H), 8.25(s, 1H), 11.02(br, 1H), 11.55(br, 1H)

### <Example 355> Preparation of (S)-4-((1-(4-chloro-8-(hydroxymethyl)-1-oxo-2-phenyl-1,2-dihydroisoquinolin-3-yl)ethyl)amino)pyrido[2,3-d]pyrimidin-5(8H)-one

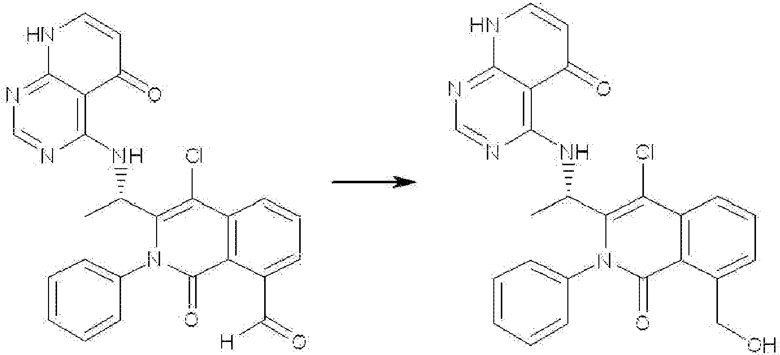

The 10 mg of (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carbaldehyde prepared in above Example 271 was added into 1 mL of anhydrous THF and stirred at room temperature. The 5 mg of lithium aluminum hydride was added to the reaction mixture and further stirred for five minutes, after which 1 mL of purified water was added to complete the reaction. A saturated aqueous solution of ammonium chloride was added to the reaction mixture, followed by extraction with ethyl acetate two to three times. The resulting combined organic layer was washed with a saturated aqueous solution of sodium chloride and dried over magnesium sulfate. The residues obtained through concentration were purified through Prep-LC. The resulting product was vacuum-dried for five hours to obtain 4 mg of (S)-4-((1-(4-chloro-8-(hydroxymethyl)-1-oxo-2-phenyl-1,2-dihydroisoquinolin-3-yl)ethyl)amino)pyrido[2,3-d]pyrimidin-5(8H)-one.

¹H-NMR (400MHz, CDCl₃) δ ppm 1.68(d, 3H), 4.84(m, 1H), 4.92(m, 2H), 5.05(m, 1H), 6.30(d, 1H), 7.25(m, 1H), 7.40-7.80(m, 7H), 8.05(d, 1H), 8.26(s, 1H), 10.45(br, 1H), 10.95(br, 1H)

### <Example 356> Preparation of 4-(((1S)-1-(4-chloro-1-oxo-2-phenyl-8-(thiazolidin-2-yl)-1,2-dihydroisoquinolin-3-yl)ethyl)amino)pyrido[2,3-d]pyrimidin-5(8H)-one

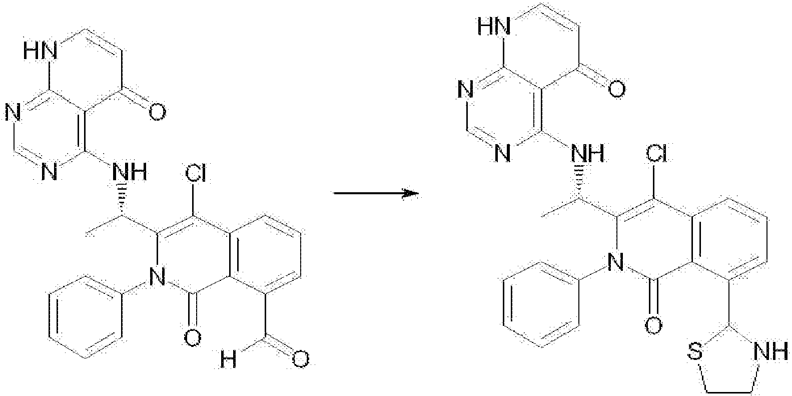

The 10 mg of (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carbaldehyde prepared in above Example 271 and 20 mg of aminoethanethiol were added into 1 mL of ethanol, and heated and stirred at 40°C for 15 hours. Upon completion of the reaction, a saturated aqueous solution of ammonium chloride was added, followed by extraction with ethyl acetate two to three times. A combined organic layer was washed with saturated saline solution, dried over anhydrous magnesium sulfate, and concentrated to obtain residues, which were then purified through Prep-LC. The resulting product was vacuum-dried for five hours to obtain 2 mg of 4-(((1S)-1-(4-chloro-1-oxo-2-phenyl-8-(thiazolidin-2-yl)-1,2-dihydroisoquinolin-3-yl)ethyl)amino)pyrido[2,3-d]pyrimidin-5(8H)-one.

¹H-NMR (400MHz, CDCl₃) δ ppm 1.66(m, 3H), 2.75-3.10(m, 4H), 3.35(m, 1H), 3.45(m, 1H), 5.05(m, 1H), 6.28(d, 1H), 7.07(s, 1H), 7.23(m, 1H), 7.40-7.80(m, 5H), 7.88(t, 1H), 7.96(d, 1H), 8.26(d, 1H), 10.95(br, 1H)

### <Example 357> Preparation of 4-(((1S)-1-(4-chloro-8-(1-hydroxy-2,2-dimethylpropyl)-1-oxo-2-phenyl-1,2-dihydroisoquinolin-3-yl)ethyl)amino)pyrido[2,3-d]pyrimidin-5(8H)-one

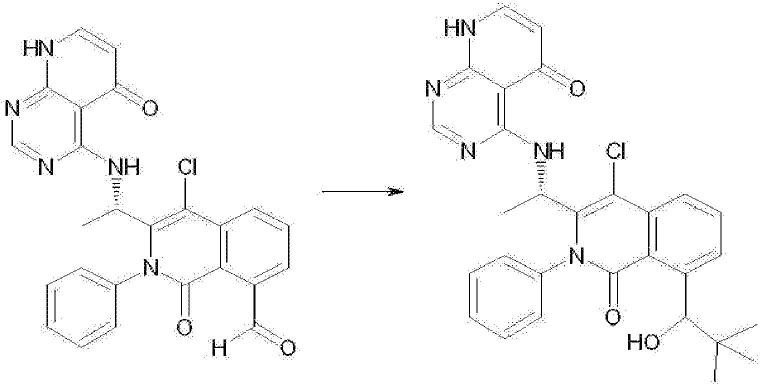

The 10 mg of (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carbaldehyde prepared in above Example 271 was added into anhydrous THF and cooled down to 0°C. The 30 mg of t-butylmagnesium chloride was dissolved in 3 mL of anhydrous THF and added dropwise, followed by stirring at room temperature for five hours. Upon completion of the reaction, 0.7 mg of 4-(((1S)-1-(4-chloro-8-(1-hydroxy-2,2-dimethylpropyl)-1-oxo-2-phenyl-1,2-dihydroisoquinolin-3-yl)ethyl)amino)pyrido[2,3-d]pyrimidin-5(8H)-one was obtained by the same process as that of Example 269 below.

¹H-NMR (400MHz, CDCl₃) δ ppm 0.87(s, 9H), 1.67(d, 3H), 5.02(m, 1H), 6.29(d, 1H), 7.20(m, 1H), 7.45-7.75(m, 7H), 8.05(m, 1H), 8.20(s, 1H), 10.90(br, 1H)

### <Example 358> Preparation of (S)-4-((1-(4-chloro-8-(5,5-dimethyl-1,3-dioxan-2-yl)-1-oxo-2-phenyl-1,2-dihydroisoquinolin-3-yl)ethyl)amino)pyrido[2,3-d]pyrimidin-5(8H)-one

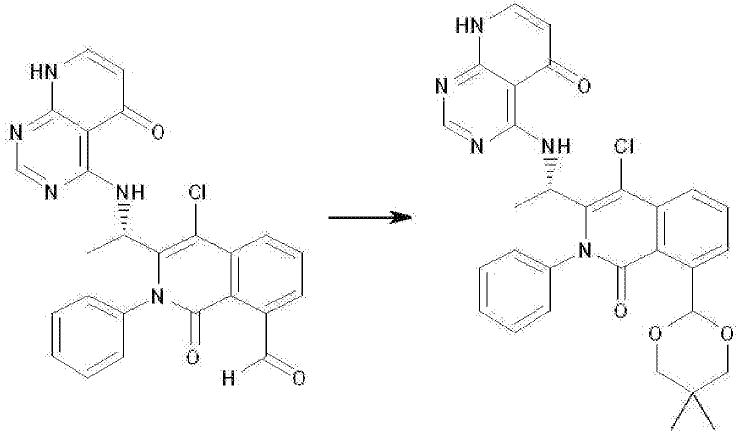

The 10 mg of (S)-4-chloro-1-oxo-3-(1-((5-oxo-5,8-dihydropyrido[2,3-d]pyrimidin-4-yl)amino)ethyl)-2-phenyl-1,2-dihydroisoquinolin-8-carbaldehyde prepared in above Example 271, 30 mg of 1,3-dihydroxy-2,2-dimethylpropane and 10 mg of p-toluenesulfonic acid were added into 5 mL of toluene, and stirred under reflux in a reactor installed with a dean-stark device for 15 hours. Upon completion of the reaction, the reaction solution was concentrated, after which a saturated aqueous solution of ammonium chloride was added, followed by extraction with ethyl acetate two to three times. A combined organic layer was washed with saturated saline solution, dried over anhydrous magnesium sulfate, and concentrated to obtain residues, which were then purified through Prep-LC. The resulting product was vacuum-dried for five hours to obtain 0.6 mg of (S)-4-((1-(4-chloro-8-(5,5-dimethyl-1,3-dioxan-2-yl)-1-oxo-2-phenyl-1,2-dihydroisoquinolin-3-yl)ethyl)amino)pyrido[2,3-d]pyrimidin-5(8H)-one.

mass(558(-1), 582(+))

### <Example 359> Preparation of (S)-4-((1-(4-chloro-8-((3-hydroxy-2,2-dimethylpropoxy)methyl)-1-oxo-2-phenyl-1,2-dihydroisoquinolin-3-yl)ethyl)amino)pyrido[2,3-d]pyrimidin-5(8H)-one

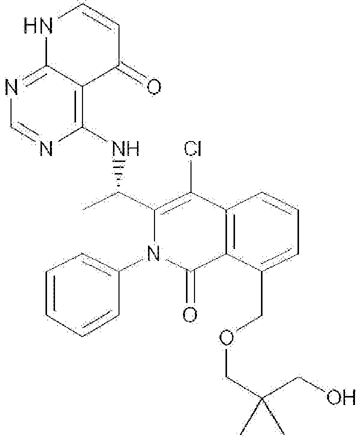

In Example 359, (S)-4-((1-(4-chloro-8-((3-hydroxy-2,2-dimethylpropoxy)methyl)-1-oxo-2-phenyl-1,2-dihydroisoquinolin-3-yl)ethyl)amino)pyrido[2,3-d]pyrimidin-5(8H)-one was obtained by substantially the same preparation method as described in above Example 358, except for using an appropriate reactant in consideration of the structure of the corresponding compound.

¹H-NMR (400MHz, CDCl₃) δ ppm 0.94(s, 6H), 1.67(d, 3H), 3.43(s, 2H), 3.45(s, 2H), 5.06(m, 1H), 5.16(q, 2H), 6.29(d, 1H), 7.20(m, 1H), 7.45-7.80(m, 7H), 7.98(d, 1H), 8.24(s, 1H), 10.92(br, 1H)

### <Example 360> Preparation of (S)-4-((1-(4-chloro-8-(2-hydroxypropan-2-yl)-1-oxo-2-phenyl-1,2-dihydroisoquinolin-3-yl)ethyl)amino)pyrido[2,3-d]pyrimidin-5(8H)-one

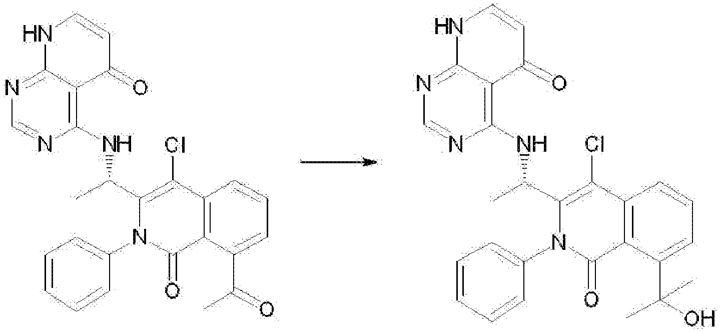

The 10 mg of (S)-4-((1-(8-acetyl-4-chloro-1-oxo-2-phenyl-1,2-dihydroisoquinolin-3-yl)ethyl)amino)pyrido[2,3-d]pyrimidin-5(8H)-one prepared in above Example 267 and 0.03 mL of 3M methylmagnesium bromide were added into 1 mL of anhydrous THF at 0°C and reacted for a day. After completion of the reaction, a saturated aqueous solution of ammonium chloride was added, followed by extraction with ethyl acetate two to three times. An organic layer was dried over anhydrous sodium sulfate, and concentrated under reduced pressure, and then the resulting residues were purified by Prep-LC. The resulting product was vacuum-dried for three hours to obtain 1 mg of (S)-4-((1-(4-chloro-8-(2-hydroxypropen-2-yl)-1-oxo-2-phenyl-1,2-dihydroisoquinolin-3-yl)ethyl)amino)pyrido[2,3-d]pyrimidin-5(8H)-one.

¹H-NMR (400MHz, CDCl₃) δ ppm 1.67-1.70(m, 9H), 5.02-5.05(m, 1H), 6.29-8.11(m, 12H), 10.95(s, 1H)

### <Example 361> Preparation of (S)-4-((1-(4-chloro-8-(4-(1-hydroxyethyl)phenyl)-1-oxo-2-phenyl-1,2-dihydroisoquinolin-3-yl)ethyl)amino)pyrido[2,3-d]pyrimidin-5(8H)-one

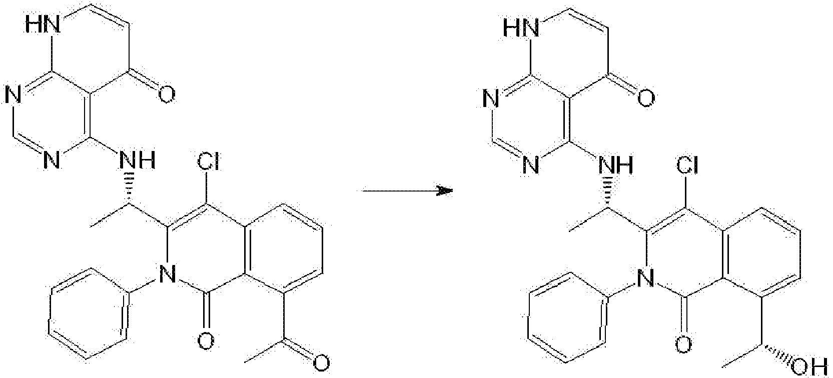

(having a diastereomeric relationship with Example 441)

The 0.03 g of (S)-4-((1-(8-amino-4-chloro-l-oxo-2-phenyl-1,2-dihydroisoquinolin-3-yl)ethyl)amino)pyrido[2,3-d]pyrimidin-5(8H)-one prepared in above Example 258 was dissolved in a mixed solvent of 1.5 mL of methylene chloride and 1.5 mL of toluene, after which 0.096 mL of 2.0M trimethylaluminum toluene solution was added and stirred at room temperature for eight hours. Upon completion of the reaction, the residues obtained through concentration under reduced pressure were then purified through Prep-LC (under gradient conditions of 20% acetonitrile aqueous solution → 100% acetonitrile). The resulting product was vacuum-dried for five hours to obtain 3 mg of (S)-4-((1-(4-chloro-8-(4-(1-hydroxyethyl)phenyl)-1-oxo-2-phenyl-1,2-dihydroisoquinolin-3-yl)ethyl)amino)pyrido[2,3-d]pyrimidin-5(8H)-one.

¹H-NMR (400MHz, CDCl₃) δ ppm 1.58-1.69(m, 6H), 2.79(s, 1H), 5.02-5.08(m, 1H), 5.51-5.56(m, 1H), 6.30-8.26(m, 11H), 10.97(d, 1H)

### <Example 362> Preparation of (S)-4-((1-(8-(benzyloxy)-4-chloro-1-oxo-2-phenyl-1,2-dihydroisoquinolin-3-yl)ethyl)amino)pyrido[2,3-d]pyrimidin-5(8H)-one

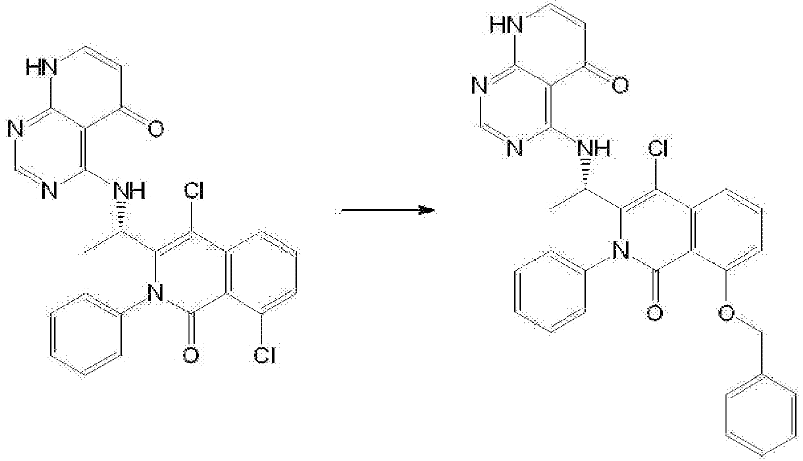

The 48 mg of (S)-4-((1-(4,8-dichloro-1-oxo-2-phenyl-1,2-dihydroisoquinolin-3-yl)ethyl)amino)pyrido[2,3-d]pyrimidin-5(8H)-one prepared in above Preparation Example 1, 22 mg of phenylmethanol and 16 mg of sodium hydroxide were added into a reactor, dissolved in 2 mL of dimethylformamide, and stirred under reflux at 150°C for 12 hours. The resulting product was cooled down to room temperature, filtered through diatomite, and concentrated under reduced pressure to obtain residues, which were then purified by Prep-LC. The resulting product was vacuum-dried for 12 hours to obtain 15 mg of (S)-4-((1-(8-(benzyloxy)-4-chloro-1-oxo-2-phenyl-1,2-dihydroisoquinolin-3-yl)ethyl)amino)pyrido[2,3-d]pyrimidin-5(8H)-one.

¹H-NMR (400MHz, CD₃OD) δ ppm 1.66(d, 3H), 5.00-5.02(m, 1H), 5.26(s, 2H), 6.27(d, 1H), 6.94-7.77(m, 14H), 8.23(s, 1H), 10.95-10.96(m, 1H), 11.20(br, 1H)

### <Examples 363 to 368>

In Examples 363 to 368, the compound of each Example was prepared by the same preparation method as that of above Example 362, except for using an appropriate reactant in consideration of the structure of the compound instead of benzylamine.

### <Example 363> Preparation of (S)-4-((1-(4-chloro-8-(2-methoxyethoxy)-1-oxo-2-phenyl-1,2-dihydroisoquinolin-3-yl)ethyl)amino)pyrido[2,3-d]pyrimidin-5(8H)-one

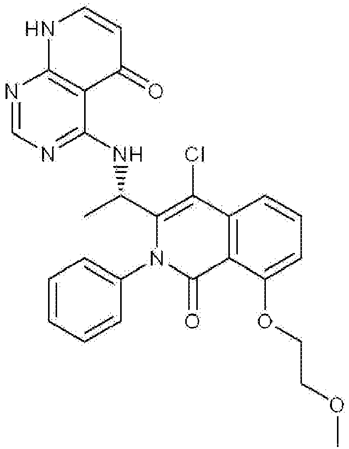

¹H-NMR (400MHz, CD₃OD) δ ppm 1.64(d, 3H), 3.40(s, 3H), 3.81-3.85(m, 2H), 4.22(t, 2H), 4.90-5.06(m, 1H), 6.29(d, 1H), 6.98-7.75(m, 9H), 8.23(s, 1H), 10.65(br, 1H), 10.80-10.91(m, 1H)

### <Example 364> Preparation of (S)-4-((1-(4-chloro-1-oxo-2-phenyl-8-(2,2,2-trifluoroethoxy)-1,2-dihydroisoquinolin-3-yl)ethyl)amino)pyrido[2,3-d]pyrimidin-5(8H)-one

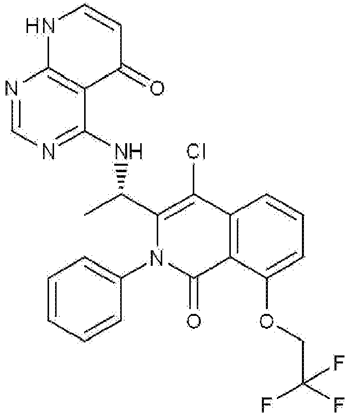

¹H-NMR (400MHz, CD₃OD) δ ppm 1.66(d, 3H), 4.44-4.50(m, 2H), 4.98-5.04(m, 1H), 6.29(d, 1H), 7.13-7.83(m, 9H), 8.25(s, 1H), 10.89-11.05(m, 1H), 11.30(br, 1H)

### <Example 365> Preparation of (S)-4-((1-(4-chloro-8-methoxy-1-oxo-2-phenyl-1,2-dihydroisoquinolin-3-yl)ethyl)amino)pyrido[2,3-d]pyrimidin-5(8H)-one

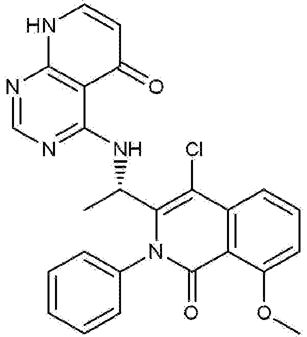

¹H-NMR (400MHz, CD₃OD) δ ppm 1.66(d, 3H), 3.94(s, 3H), 5.04-5.08(m, 1H), 6.28(d, 1H), 6.95-7.61(m, 9H), 8.22(s, 1H), 10.80-10.91(m, 1H)

### <Example 366> Preparation of (S)-4-((1-(4-chloro-8-(2-methoxyphenoxy)-1-oxo-2-phenyl-1,2-dihydroisoquinolin-3-yl)ethyl)amino)pyrido[2,3-d]pyrimidin-5(8H)-one

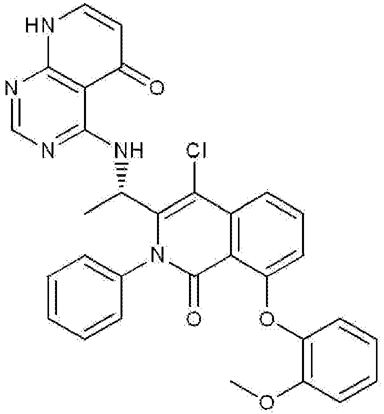

¹H-NMR (400MHz, CD₃OD) δ ppm 1.68(d, 3H), 3.72(s, 3H), 5.02-5.05(m, 1H), 6.28(d, 1H), 6.70-7.69(m, 13H), 8.25(s, 1H), 10.94-11.01(m, 1H)

### <Example 367> Preparation of (S)-4-((1-(4-chloro-1-oxo-2-phenyl-8-(p-tolyloxy)-1,2-dihydroisoquinolin-3-yl)ethyl)amino)pyrido[2,3-d]pyrimidin-5(8H)-one

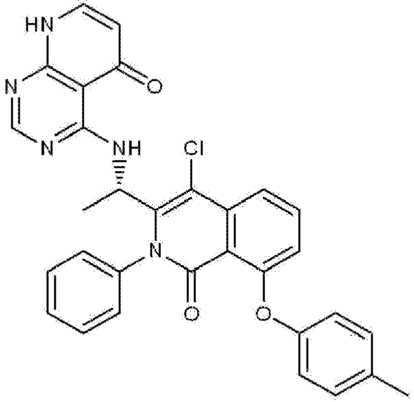

¹H-NMR (400MHz, CD₃OD) δ ppm 1.68(d, 3H), 2.30(s, 3H), 5.05-5.09(m, 1H), 6.30(d, 1H), 6.86-7.75(m, 13H), 8.25(s, 1H), 10.85(br, 1H), 10.85-10.86(m, 1H)

### <Example 368> Preparation of 4-(((1S)-1-(4-chloro-1-oxo-2-phenyl-8-(1-(pyridin-4-yl)ethoxy)-1,2-dihydroisoquinolin-3-yl)ethyl)amino)pyrido[2,3-d]pyrimidin-5(8H)-one

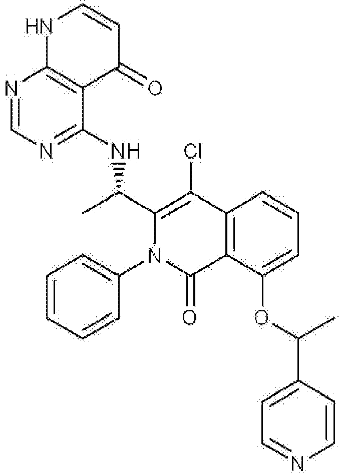

¹H-NMR (400MHz, CD₃OD) δ ppm 1.43-1.49(m, 3H), 1.68(d, 3H), 3.93(m, 1H), 5.02-5.06(m, 1H), 6.81-6.82(d, 1H), 6.96-7.92(m, 13H), 8.16(s, 1H), 10.95-10.99(m, 1H)

### <Example 369> Preparation of (S)-4-((1-(4-chloro-8-((4-chlorobenzyl)thio)-1-oxo-2-phenyl-1,2-dihydroisoquinolin-3-yl)ethyl)amino)pyrido[2,3-d]pyrimidin-5(8H)-one

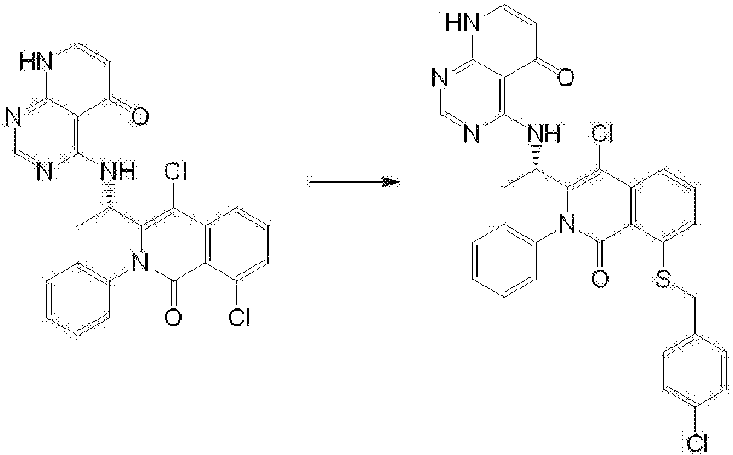

The 48 mg of (S)-4-((1-(4,8-dichloro-1-oxo-2-phenyl-1,2-dihydroisoquinolin-3-yl)ethyl)amino)pyrido[2,3-d]pyrimidin-5(8H)-one prepared in above Preparation Example 1, 32 mg of (4-chlorophenyl)methanethiol and 41 mg of potassium carbonate were added into a reactor, dissolved in 2 mL of dimethylformamide, and stirred at 150°C for 12 hours. The resulting product was cooled down to room temperature, filtered through diatomite, and concentrated under reduced pressure to obtain residues, which were then purified by Prep-LC. The resulting product was vacuum-dried for 12 hours to obtain 20 mg of (S)-4-((1-(4-chloro-8-((4-chlorobenzyl)thio)-1-oxo-2-phenyl-1,2-phenyl-1,2-dihydroisoquinolin-3-yl)ethyl)amino)pyrido[2,3-d]pyrimidin-5(8H)-one.

¹H-NMR (400MHz, CD₃OD) δ ppm 1.66(d, 3H), 4.05(s, 2H), 5.03-5.07(m, 1H), 6.30(d, 1H), 7.27-7.80(m, 11H), 8.24(s, 1H), 11.01-11.04(m, 1H)

### <Examples 370 to 372>

In Examples 370 to 372, the compound of each Example was prepared by the same preparation method as described in above Example 369, except for using an appropriate reactant in consideration of the structure of each compound instead of (4-chlorophenyl)methanethiol.

### <Example 370> Preparation of (S)-4-((1-(4-chloro-1-oxo-2-phenyl-8-(pyridin-4-ylthio)-1,2-dihydroisoquinolin-3-yl)ethyl)amino)pyrido[2,3-d]pyrimidin-5(8H)-one

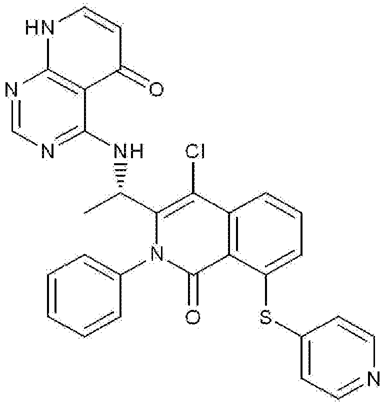

¹H-NMR (400MHz, CD₃OD) δ ppm 1.69(d, 3H), 5.01-5.04(m, 1H), 6.31(d, 1H), 6.88(d, 1H), 7.23-7.79(m, 10H), 8.27(s, 1H), 8.65(d, 1H), 10.96-10.98(m, 1H)

### <Example 371> Preparation of (S)-4-((1-(4-chloro-8-((4-chlorophenyl)thio)-1-oxo-2-phenyl-1,2-dihydroisoquinolin-3-yl)ethyl)amino)pyrido[2,3-d]pyrimidin-5(8H)-one

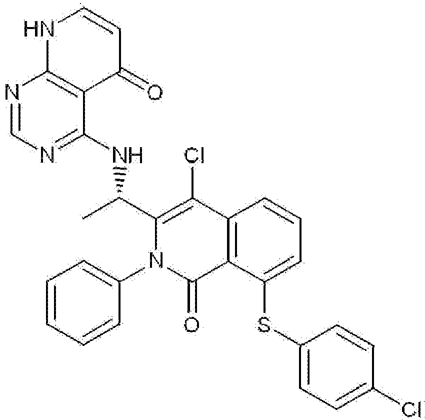

¹H-NMR (400MHz, CD₃OD) δ ppm 1.68(d, 3H), 5.03-5.07(m, 1H), 6.31(d, 1H), 6.88(d, 1H), 7.27-7.80(m, 12H), 8.27(s, 1H), 10.99-11.02(m, 1H)

### <Example 372> Preparation of (S)-4-((1-(4-chloro-1-oxo-2-phenyl-8-(propylthio)-1,2-dihydroisoquinolin-3-yl)ethyl)amino)pyrido[2,3-d]pyrimidin-5(8H)-one

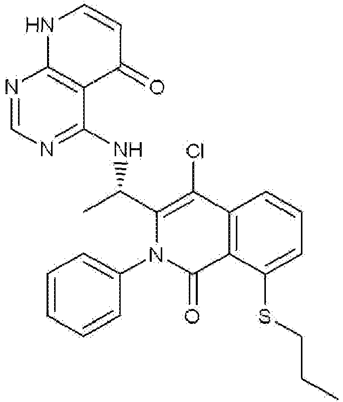

¹H-NMR (400MHz, CD₃OD) δ ppm 1.08(t, 3H), 1.66(d, 3H), 1.73-1.79(m, 2H), 2.81-2.85(m, 2H), 5.02-5.06(m, 1H), 6.30(d, 1H), 7.18-7.76(m, 7H), 8.24(s, 1H), 11.00-11.03(m, 1H)

### <Example 373> Preparation of (S)-6-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-2,2-dimethyl-2H-benzo[b][1,4]oxazin-3(4H)-one

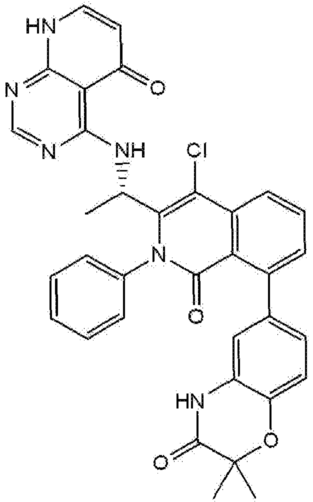

In Example 373, (S)-6-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-2,2-dimethyl-2H-benzo[b] [1,4]oxazin-3(4H)-one was obtained by substantially the same preparation method as described in above Example 56, except for using an appropriate reactant in consideration of the structure of the corresponding compound.

¹H-NMR (400MHz, CDCl₃) δ ppm 1.46(s, 3H), 1.49(s, 3H), 1.63 (d, 3H), 5.10 (m, 1H), 6.58-8.16(m, 14H), 10.86 (s, 1H)

### <Examples 374 and 375>

In Examples 374 and 375, the compound of each Example was prepared by substantially the same preparation method as described in above Example 3, except for using an appropriate reactant in consideration of the structure of the corresponding compound.

### <Example 374> Preparation of (S)-4-((1-(4-chloro-1-oxo-2-phenyl-1,2-dihydroisoquinolin-3-yl)ethyl)amino)-8-(2-methoxypyridin-4-yl)pyrido[2,3-d]pyrimidin-5(8H)-one

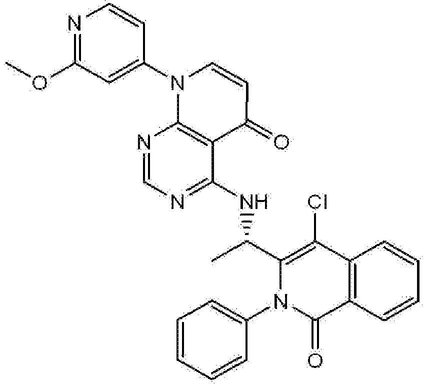

¹H-NMR (400MHz, CDCl₃) δ ppm 1.70(d, 3H), 3.99(s, 3H), 5.08-5.15(m, 1H), 6.37-8.42(m, 15H), 11.02(d, 1H)

### <Example 375> Preparation of (S)-8-(5-bromo-2-methoxypyridin-4-yl)-4-((1-(4-chloro-l-oxo-2-phenyl-1,2-dihydroisoquinolin-3-yl)ethyl)amino)pyrido[2,3-d]pyrimidin-5(8H)-one

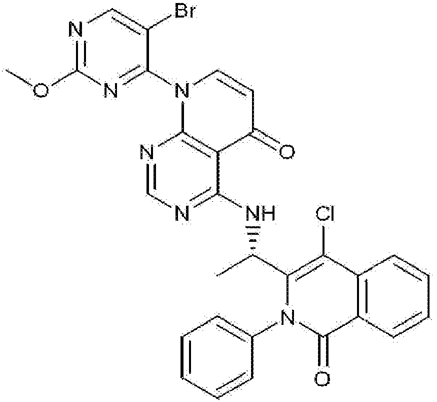

¹H-NMR (400MHz, CDCl₃) δ ppm 1.70(d, 3H), 4.07(s, 3H), 5.06-5.13(m, 1H), 6.36-8.66(m, 13H), 10.90(d, 1H)

### <Examples 376 to 378>

In Examples 376 to 378, the compound of each Example was prepared by substantially the same preparation method as described in above Example 56, except for using an appropriate reactant in consideration of the structure of the corresponding compound.

### <Example 376> Preparation of (S)-4-((1-(4-chloro-8-(5-isopropoxypyrazin-2-yl)-1-oxo-2-phenyl-1,2-dihydroisoquinolin-3-yl)ethyl)amino)pyrido[2,3-d]pyrimidin-5(8H)-one

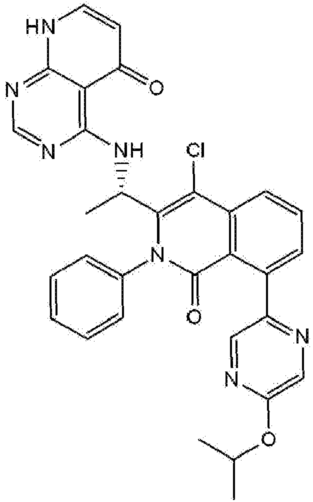

¹H-NMR(400MHz, CDC13) δ ppm 1.35(d, 6H), 1.66(d, 3H), 4.99-5.06(m, 1H), 5.20-5.27(m, 1H), 6.29-8.25(m, 13H), 10.14(s, 1H), 10.93(d, 1H)

### <Example 377> Preparation of (S)-4-((1-(4-chloro-1-oxo-2-phenyl-8-(2-(thiophen-2-yl)pyrimidin-5-yl)-1,2-dihydroisoquinolin-3-yl)ethyl)amino)pyrido[2,3-d]pyrimidin-5(8H)-one

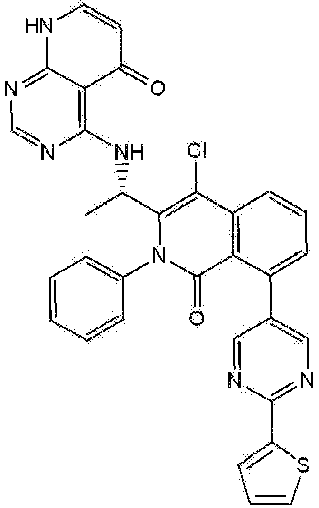

¹H-NMR (400MHz, CDCl₃) δ ppm 1.69(d, 3H), 5.05(m, 1H), 6.31(d, 1H), 7.10-7.20(m, 2H), 7.30-7.70(m, 7H), 7.80(t, 1H), 7.95(d, 1H), 8.20(d, 1H), 8.25(s, 1H), 8.60(s, 2H), 10.50(br, 1H), 11.00(br, 1H)

### <Example 378> Preparation of (S)-4-((1-(4-chloro-8-(2-(furan-2-yl)pyrimidin-5-yl)-1-oxo-2-phenyl-1,2-dihydroisoquinolin-3-yl)ethyl)amino)pyrido[2,3-d]pyrimidin-5(8H)-one

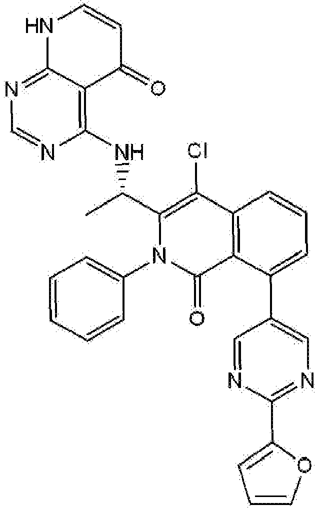

¹H-NMR (400MHz, CDCl₃) δ ppm 1.69(d, 3H), 5.05(m, 1H), 6.30(d, 1H), 6.55(m, 1H), 7.15(m, 1H), 7.30-7.85(m, 10H), 8.20(d, 1H), 8.26(s, 1H), 8.65(s, 2H), 10.72(br, 1H), 10.95(br, 1H)

### <Example 379> Preparation of (S)-4-((1-(4-chloro-8-(2-ethylpyrimidin-5-yl)-1-oxo-2-phenyl-1,2-dihydroisoquinolin-3-yl)ethyl)amino)pyrido[2,3-d]pyrimidin-5(8H)-one

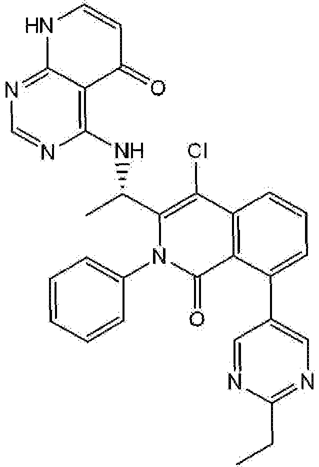

In Example 379, (S)-4-((1-(4-chloro-8-(2-ethylpyrimidin-5-yl)-1-oxo-2-phenyl-1,2-dihydroisoquinolin-3-yl)ethyl)amino)pyrido[2,3-d]pyrimidin-5(8H)-one was obtained by substantially the same preparation method as described in above Example 3, except for using an appropriate reactant in consideration of the structure of the corresponding compound.

¹H-NMR (400MHz, CDCl₃) δ ppm 1.36(t, 3H), 1.69 (d, 3H), 2.97 (3H, q), 5.05 (m, 1H) 6.31-8.58(m, 13H), 11.02 (s, 1H), 11.03 (s, 1H)

### <Examples 380 and 381>

In Examples 380 and 381, the compound of each Example was prepared by substantially the same preparation method as described in above Example 56, except for using an appropriate reactant in consideration of the structure of the corresponding compound.

### <Example 380> Preparation of (S)-4-((1-(4-chloro-8-(1H-indol-2-yl)-1-oxo-2-phenyl-1,2-dihydroisoquinolin-3-yl)ethyl)amino)pyrido[2,3-d]pyrimidin-5(8H)-one

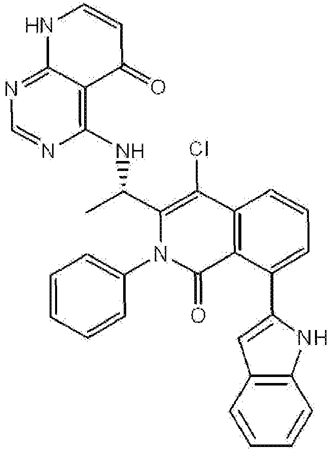

¹H-NMR (400MHz, MeOD) δ ppm 1.69(d, 3H), 3.70(s, 1H), 5.03-5.10(s, 1H), 6.28-8.24(m, 17H), 9.93(s, 1H), 10.90(d, 1H)

### <Example 381> Preparation of (S)-4-((1-(4-chloro-8-(2-isopropylpyrimidin-5-yl)-1-oxo-2-phenyl-1,2-dihydroisoquinolin-3-yl)ethyl)amino)pyrido[2,3-d]pyrimidin-5(8H)-one

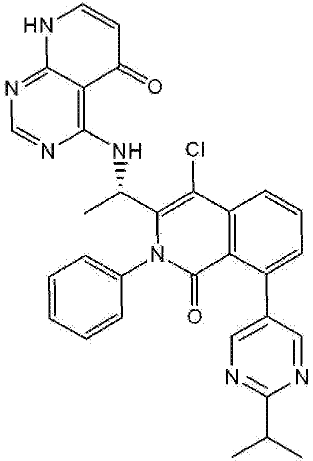

¹H-NMR (400MHz, CDCl₃) δ ppm 1.33(d, 6H), 1.69(d, 3H), 3.15-3.22(m, 1H), 4.95-5.10(m, 1H), 6.30-8.59(m, 13H), 11.05(br, 1H)

### <Example 382> Preparation of (S)-4-((1-(4-chloro-8-(2-fluoropyrimidin-5-yl)-1-oxo-2-phenyl-1,2-dihydroisoquinolin-3-yl)ethyl)amino)pyrido[2,3-d]pyrimidin-5(8H)-one

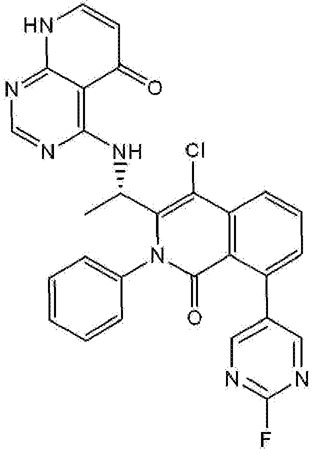

In Example 382, (S)-4-((1-(4-chloro-8-(2-fluoropyrimidin-5-yl)-1-oxo-2-phenyl-1,2-dihydroisoquinolin-3-yl)ethyl)amino)pyrido[2,3-d]pyrimidin-5(8H)-one was obtained by substantially the same preparation method as described in above Example 3, except for using an appropriate reactant in consideration of the structure of the corresponding compound.

¹H-NMR (400MHz, CDCl₃) δ ppm 1.70(d, 3H), 3.99(s, 3H), 5.05-5.11(m, 1H), 6.39-8.91(m, 13H), 10.90(br, 1H)

### <Examples 383 to 388>

In Examples 383 to 388, the compound of each Example was prepared by substantially the same preparation method as described in above Example 56, except for using an appropriate reactant in consideration of the structure of the corresponding compound.

### <Example 383> Preparation of (S)-4-((1-(8-(2-methoxypyrimidin-5-yl)-1-oxo-2-phenyl-1,2-dihydroisoquinolin-3-yl)ethyl)amino)pyrido[2,3-d]pyrimidin-5(8H)-one

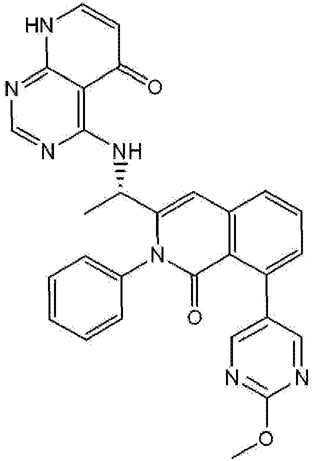

¹H-NMR (400MHz, CDCl₃) δ ppm 1.46(d, 3H), 3.99(s, 3H), 4.91-4.98(m, 1H), 6.33-8.46(m, 14H), 10.47(s, 1H), 10.55(d, 1H)

### <Example 384> Preparation of (S)-4-((1-(8-(2-ethoxypyrimidin-5-yl)-1-oxo-2-phenyl-1,2-dihydroisoquinolin-3-yl)ethyl)amino)pyrido[2,3-d]pyrimidin-5(8H)-one

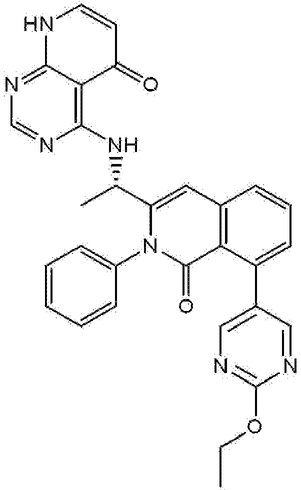

¹H-NMR (400MHz, CDCl₃) δ ppm 1.41(t, 3H), 1.49(d, 3H), 4.37-4.42(m, 2H), 4.91-4.98(m, 1H), 6.33-8.44(m, 14H), 10.56(d, 1H)

### <Example 385> Preparation of (S)-4-((1-(1-oxo-2-phenyl-8-(2-(trifluoromethyl)pyrimidin-5-yl)-1,2-dihydroisoquinolin-3-yl)ethyl)amino)pyrido[2,3-d]pyrimidin-5(8H)-one

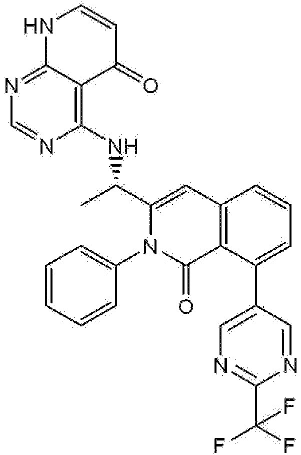

¹H-NMR (400MHz, CDCl₃) δ ppm 1.50(d, 3H), 4.92-4.99(m, 1H), 6.33-8.82(m, 14H), 10.56(d, 1H)

### <Example 386> Preparation of (S)-4-((1-(8-(2-(2-hydroxypropan-2-yl)pyrimidin-5-yl)-1-oxo-2-phenyl-1,2-dihydroisoquinolin-3-yl)ethyl)amino)pyrido[2,3-d]pyrimidin-5(8H)-one

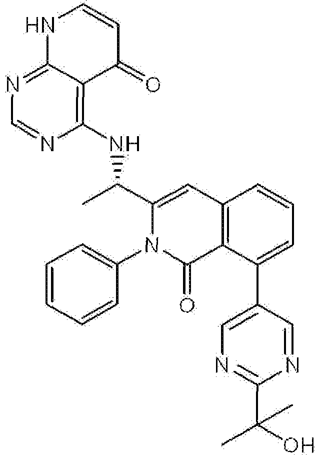

¹H-NMR (400MHz, CDCl₃) δ ppm 1.49(d, 3H), 1.60(6H, s), 2.17(s, 1H), 4.92-4.99(m, 1H), 6.33-8.66(m, 14H), 10.55(d, 1H)

### <Example 387> Preparation of (S)-4-((1-(4-chloro-8-(5-methylthiophen-2-yl)-1-oxo-2-phenyl-1,2-dihydroisoquinolin-3-yl)ethyl)amino)pyrido[2,3-d]pyrimidin-5(8H)-one

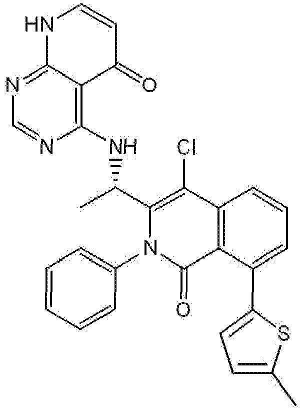

¹H-NMR (400MHz, CDCl₃) δ ppm 1.66(d, 3H), 2.43(s, 3H), 5.02-5.04(m, 1H), 6.27-8.25(m, 13H), 10.98(br, 1H)

### <Example 388> Preparation of (S)-4-((1-(4-chloro-1-oxo-2-phenyl-8-(thiophen-2-yl)-1,2-dihydroisoquinolin-3-yl)ethyl)amino)pyrido[2,3-d]pyrimidin-5(8H)-one

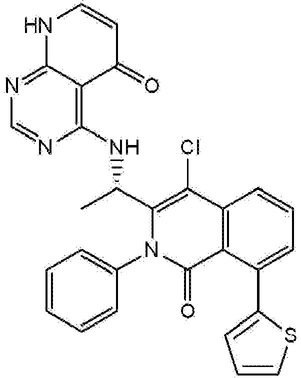

¹H-NMR (400MHz, CDCl₃) δ ppm 1.66(d, 3H), 5.01-5.05(m, 1H), 6.28-8.25(m, 14H), 11.01(br, 1H), 11.52(br, 1H)

### <Example 389> Preparation of (S)-4-((1-(4-chloro-1-oxo-2-phenyl-8-(trifluoromethyl)-1,2-dihydroisoquinolin-3-yl)ethyl)amino)pyrido[2,3-d]pyrimidin-5(8H)-one

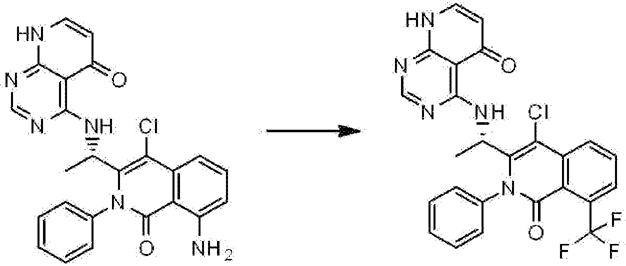

The 30 mg (1.0 eq.) of (S)-4-((1-(8-amino-4-chloro-1-oxo-2-phenyl-1,2-dihydroisoquinolin-3-yl)ethyl)amino)pyrido[2,3-d]pyrimidin-5(8H)-one prepared in above Example 294, Umemoto's reagent[cas No. 1895006-01-5] (1.5 eq.), copper (powder)(3.0 eq.) and t-butyl nitrite (3 eq.) were added into 1 mL of acetonitrile in an ice bath and reacted at room temperature for a day while stirring. After completion of the reaction, a saturated saline solution was added to the reaction mixture, followed by extraction with ethyl acetate two to three times. A combined ethyl acetate layer was dried over anhydrous magnesium sulfate (Na₂SO₄) and concentrated under reduced pressure to obtain residues, which were then purified through Prep-LC so as to obtain 1 mg of (S)-4-((1-(4-chloro-1-oxo-2-phenyl-8-(trifluoromethyl)-1,2-dihydroisoquinolin-3-yl)ethyl)amino)pyrido[2,3-d]pyrimidin-5(8H)-one.

¹H-NMR (400MHz, CDCl₃) δ ppm 1.70(d, 3H), 5.08 (m, 1H) 6.32-8.34(m, 11H), 9.94 (s, 1H), 10.92 (s, 1H)

### <Example 390> Preparation of (S)-4-((1-(4-chloro-8-(methylsulfonyl)-1-oxo-2-phenyl-1,2-dihydroisoquinolin-3-yl)ethyl)amino)pyrido[2,3-d]pyrimidin-5(8H)-one

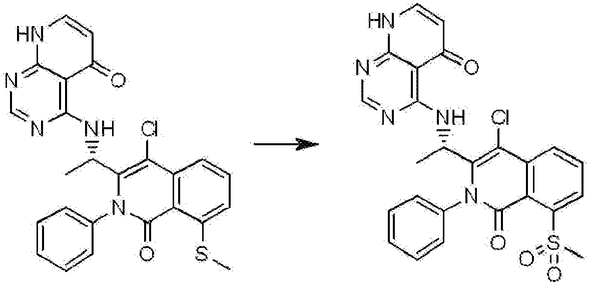

The 50 mg of (S)-4-((1-(4-chloro-8-(methylthio)-1-oxo-2-phenyl-1,2-dihydroisoquinolin-3-yl)ethyl)amino)pyrido[2,3-d]pyrimidin-5(8H)-one, prepared by substantially the same method as that of above Example 372 except for using methanethiol instead of propanethiol, was dissolved in 2 mL of CHCl₃, after which MCPBA 2.5eq was added and stirred at room temperature for two hours. Upon completion of the reaction, a saturated saline solution was added, followed by extraction with chloroform two to three times. A combined chloroform layer was dried over anhydrous magnesium sulfate and concentrated under reduced pressure to obtain residues, which were then purified through Prep-LC so as to obtain 15 mg of (S)-4-((1-(4-chloro-8-(methylsulfonyl)-1-oxo-2-phenyl-1,2-dihydroisoquinolin-3-yl)ethyl)amino)pyrido[2,3-d]pyrimidin-5(8H)-one.

¹H-NMR (400MHz, CDCl₃) δ ppm 1.68 (d, 3H), 3.57 (s, 3H), 5.01 (m, 1H) 6.29-8.55 (m, 11H), 10.95 (s, 1H)

### <Examples 391 to 397>

In Examples 391 to 397, the compound of each Example was prepared by substantially the same preparation method as described in above Example 390, except for using an appropriate reactant in consideration of the structure of the corresponding compound.

### <Example 391> Preparation of (S)-4-((1-(4-chloro-1-oxo-2-phenyl-8-(pyridin-4-ylsulfonyl)-1,2-dihydroisoquinolin-3-yl)ethyl)amino)pyrido[2,3-d]pyrimidin-5(8H)-one

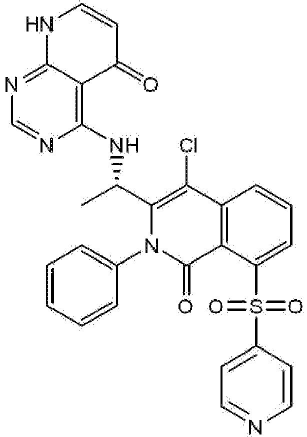

¹H-NMR (400MHz, CDCl₃) δ ppm 1.62 (d, 3H), 4.94 (m, 1H) 6.28-8.78 (m, 15H), 10.86 (s, 1H)

### <Example 392> Preparation of (S)-4-((1-(4-chloro-8-(2-(methylsulfonyl)pyrimidin-5-yl)-1-oxo-2-phenyl-1,2-dihydroisoquinolin-3-yl)ethyl)amino)pyrido[2,3-d]pyrimidin-5(8H)-one

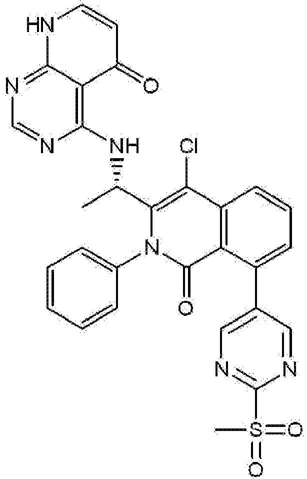

¹H-NMR (400MHz, CDCl₃) δ ppm 1.70 (d, 3H), 3.34 (s, 3H), 5.03 (m, 1H), 6.32-8.84(m, 13H), 11.04(s, 1H)

### <Example 393> Preparation of (S)-4-((1-(4-chloro-8-((4-chlorophenyl)sulfonyl)-1-oxo-2-phenyl-1,2-dihydroisoquinolin-3-yl)ethyl)amino)pyrido[2,3-d]pyrimidin-5(8H)-one

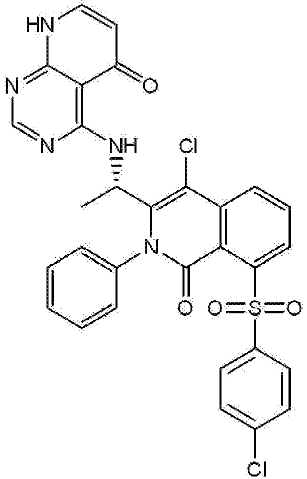

¹H-NMR (400MHz, CDCl₃) δ ppm 1.62 (d, 3H), 4.95 (m, 1H), 6.28-8.70 (m, 15H), 10.88 (s, 1H)

### <Example 394> Preparation of 4-(((S)-1-(4-chloro-1-oxo-2-phenyl-8-(((S)-4-(trifluoromethoxy)phenyl)sulfinyl)-1,2-dihydroisoquinolin-3-yl)ethyl)amino)pyrido[2,3-d]pyrimidin-5(8H)-one

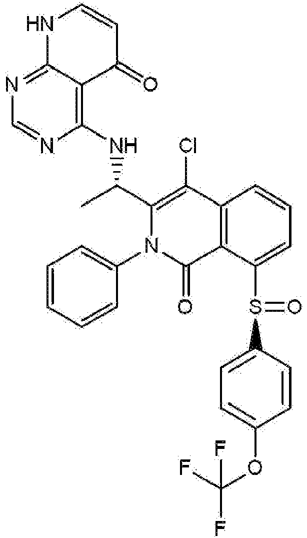

¹H-NMR (400MHz, CDCl₃) δ ppm 1.67 (d, 3H), 5.06 (m, 1H), 6.28-8.62 (m, 15H), 11.02 (s, 1H), 11.57 (s, 1H)

### <Example 395> Preparation of 4-(((S)-1-(4-chloro-1-oxo-2-phenyl-8-(((R)-4-(trifluoromethoxy)phenyl)sulfinyl)phenyl)-1,2-dihydroisoquinolin-3-yl)ethyl)amino)pyrido[2,3-d]pyrimidin-5(8H)-one

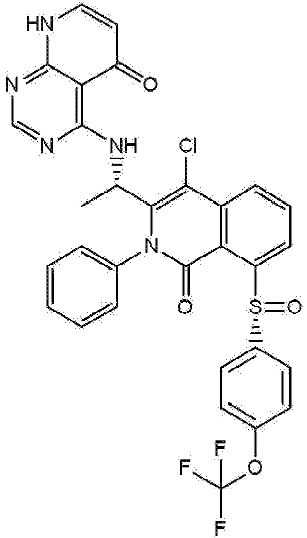

¹H-NMR (400MHz, CDCl₃) δ ppm 1.63 (d, 3H), 4.97 (m, 1H), 6.30-8.61 (m, 15H), 10.98 (s, 1H), 11.83 (s, 1H)

### <Example 396> Preparation of 4-(((S)-1-(4-chloro-1-oxo-2-phenyl-8-((S)-propylsulfinyl)-1,2-dihydroisoquinolin-3-yl)ethyl)amino)pyrido[2,3-d]pyrimidin-5(8H)-one

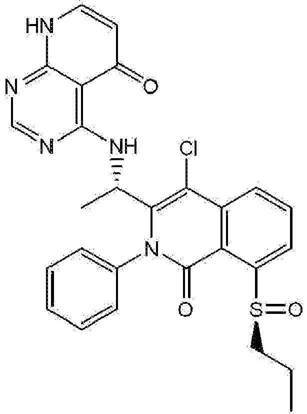

¹H-NMR (400MHz, CDCl₃) δ ppm 0.97 (t, 3H), 1.72 (d, 3H), 2.63-3.19 (m, 2H), 5.04(m, 1H), 6.31-8.39 (m, 11H), 10.90 (s, 1H)

### <Example 397> Preparation of 4-(((S)-1-(4-chloro-1-oxo-2-phenyl-8-((R)-propylsulfonyl)-1,2-dihydroisoquinolin-3-yl)ethyl)amino)pyrido[2,3-d]pyrimidin-5(8H)-one

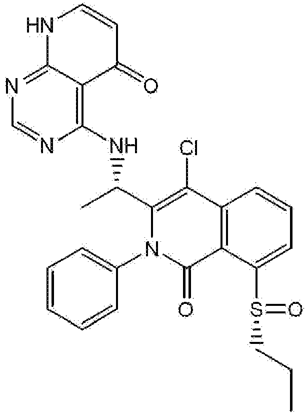

¹H-NMR (400MHz, CDCl₃) δ ppm 0.95 (t, 3H), 1.68 (d, 3H), 1.75 (q, 2H), 3.75-3.91(m, 2H), 5.02(m, 1H), 6.30-8.50(m, 11H), 11.02 (s, 1H), 11.58 (s, 1H)

### <Example 398> Preparation of (S)-4-((1-(4-chloro-8-((4-fluorophenyl)thio)-1-oxo-2-phenyl-1,2-dihydroisoquinolin-3-yl)ethyl)amino)pyrido[2,3-d]pyrimidin-5(8H)-one

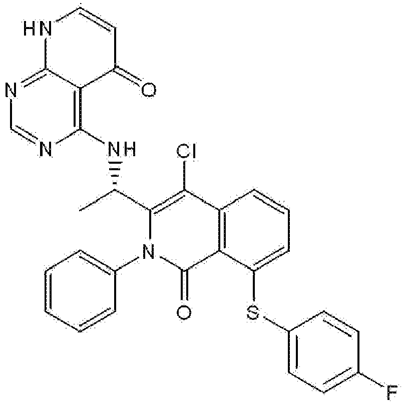

In Example 398, (S)-4-((1-(4-chloro-8-((4-fluorophenyl)thio)-1-oxo-2-phenyl-1,2-dihydroisoquinolin-3-yl)ethyl)amino)pyrido[2,3-d]pyrimidin-5(8H)-one was obtained by substantially the same preparation method as described in above Example 369, except for using an appropriate reactant in consideration of the structure of the corresponding compound.

¹H-NMR (400MHz, CDCl₃) δ ppm 1.68(d, 3H), 5.05(m, 1H), 6.32(d, 1H), 6.67(d, 1H), 7.10-7.85(m, 12H), 8.27(s, 1H), 11.00(br, 1H), 11.85(br, 1H)

### <Example 399> Preparation of (S)-4-((1-(4-chloro-8-((4-fluorophenyl)sulfonyl)-1-oxo-2-phenyl-1,2-dihydroisoquinolin-3-yl)ethyl)amino)pyrido[2,3-d]pyrimidin-5(8H)-one

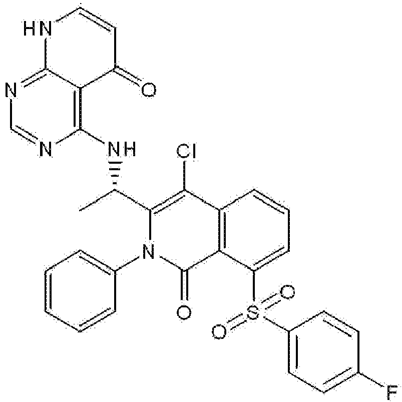

In Example 399, (S)-4-((1-(4-chloro-8-((4-fluorophenyl)sulfonyl)-1-oxo-2-phenyl-1,2-dihydroisoquinolin-3-yl)ethyl)amino)pyrido[2,3-d]pyrimidin-5(8H)-one was obtained by substantially the same preparation method as described in above Example 390, except for using an appropriate reactant in consideration of the structure of the corresponding compound.

¹H-NMR (400MHz, CDCl₃) δ ppm 1.63(d, 3H), 4.97(m, 1H), 6.30(d, 1H), 7.05(m, 3H), 7.40-7.60(m, 5H), 7.75-7.95(m, 3H), 8.19(s, 1H), 8.35(d, 1H), 8.80(d, 1H), 10.50(br, 1H), 10.90(br, 1H)

### <Example 400> Preparation of (S)-4-((1-(8-((1H-1,2,4-triazol-3-yl)thio)-4-chloro-1-oxo-2-phenyl-1,2-dihydroisoquinolin-3-yl)ethyl)amino)pyrido[2,3-d]pyrimidin-5(8H)-one

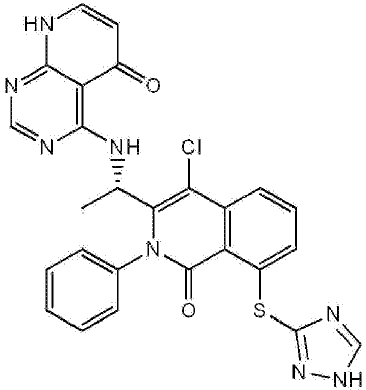

In Example 400, (S)-4-((1-(8-((1H-1,2,4-triazol-3-yl)thio)-4-chloro-l-oxo-2-phenyl-1,2-dihydroisoquinolin-3-yl)ethyl)amino)pyrido[2,3-d]pyrimidin-5(8H)-one was obtained by substantially the same preparation method as described in above Example 369, except for using an appropriate reactant in consideration of the structure of the corresponding compound.

¹H-NMR (400MHz, CDCl₃) δ ppm 1.74(d, 3H), 5.11(m, 1H), 6.48(d, 1H), 6.65(d, 1H), 7.26-7.80(m, 7H), 8.35(m, 2H), 11.95(br, 1H)

### <Example 401> Preparation of (S)-4-((1-(8-((1H-1,2,4-triazol-3-yl)sulfonyl)-4-chloro-1-oxo-2-phenyl-1,2-dihydroisoquinolin-3-yl)ethyl)amino)pyrido[2,3-d]pyrimidin-5(8H)-one

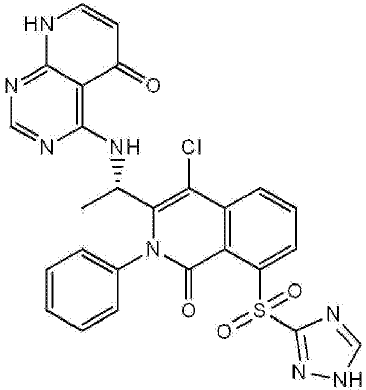

In Example 401, (S)-4-((1-(8-((1H-1,2,4-triazol-3-yl)sulfonyl)-4-chloro-1-oxo-2-phenyl-1,2-dihydroisoquinolin-3-yl)ethyl)amino)pyrido[2,3-d]pyrimidin-5(8H)-one was obtained by substantially the same preparation method as described in above Example 390, except for using an appropriate reactant in consideration of the structure of the corresponding compound.

¹H-NMR(400MHz, MeOD) δ ppm 1.66(d, 3H), 4.96(m, 1H), 6.28(d, 1H), 7.18(s, 1H), 7.40-7.70(m, 5H), 7.75(d, 1H), 8.10(t, 1H), 8.27(s, 1H), 8.45(s, 1H), 8.53(d, 1H), 8.66(d, 1H), 10.74(br, 1H)

### <Example 402> Preparation of (S)-4-((1-(4-chloro-8-(2-chloropyrimidin-5-yl)-1-oxo-2-phenyl-1,2-dihydroisoquinolin-3-yl)ethyl)amino)pyrido[2,3-d]pyrimidin-5(8H)-one

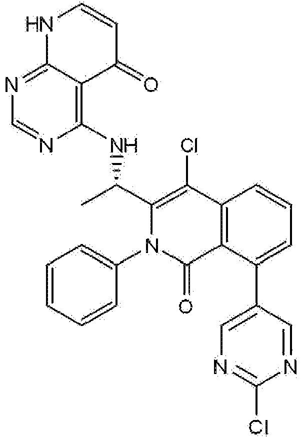

In Example 402, (S)-4-((1-(4-chloro-8-(2-chloropyrimidin-5-yl)-1-oxo-2-phenyl-1,2-dihydroisoquinolin-3-yl)ethyl)amino)pyrido[2,3-d]pyrimidin-5(8H)-one was obtained by substantially the same preparation method as described in above Example 56, except for using an appropriate reactant in consideration of the structure of the corresponding compound.

¹H-NMR (400MHz, CDCl₃) δ ppm 1.67(d, 3H), 5.02-5.06(m, 1H), 6.19-8.39(m, 13H), 11.02(br, 1H), 11.75(br, 1H)

### <Example 403> Preparation of (S)-4-((1-(8-(2-acetylpyrimidin-5-yl)-4-chloro-1-oxo-2-phenyl-1,2-dihydroisoquinolin-3-yl)ethyl)amino)pyrido[2,3-d]pyrimidin-5(8H)-one

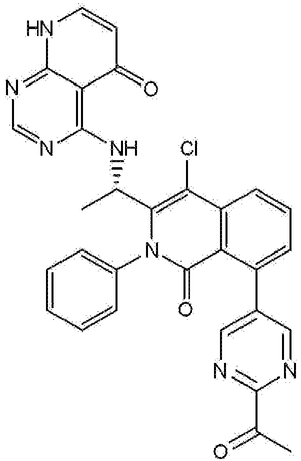

In Example 403, (S)-4-((1-(8-(2-acetylpyrimidin-5-yl)-4-chloro-l-oxo-2-phenyl-1,2-dihydroisoquinolin-3-yl)ethyl)amino)pyrido[2,3-d]pyrimidin-5(8H)-one was obtained by substantially the same preparation method as described in above Example 267, except for using an appropriate reactant in consideration of the structure of the corresponding compound.

¹H-NMR (400MHz, CDCl₃) δ ppm 1.70 (d, 3H), 2.77 (s, 3H), 5.03 (m, 1H), 6.31-8.91(m, 13H), 10.49(s, 1H), 10.97(s, 1H)

### <Example 404> Preparation of (S)-4-((1-(4-chloro-8-(2-(2-ethoxypropan-2-yl)pyrimidin-5-yl)-1-oxo-2-phenyl-1,2-dihydroisoquinolin-3-yl)ethyl)amino)pyrido[2,3-d]pyrimidin-5(8H)-one

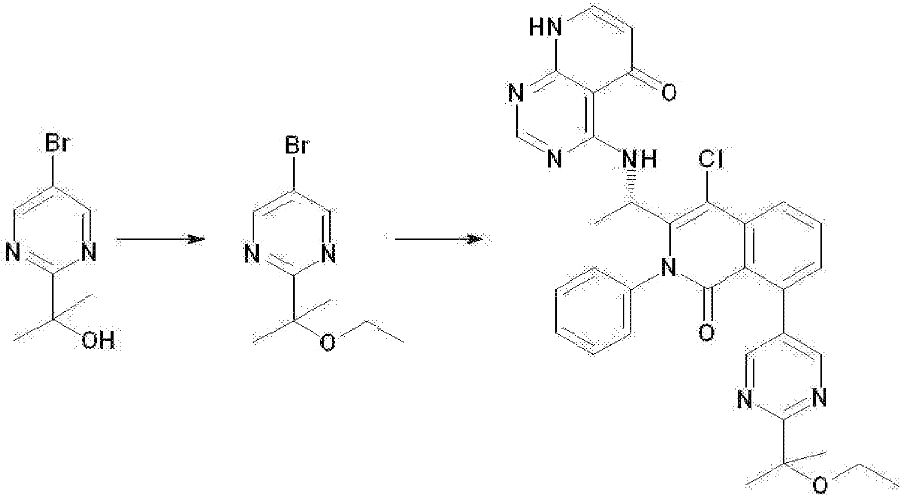

[Step 1] The 50 mg of 2-(5-bromopyrimidin-2-yl)propan-2-ol, 104 µL of ethyl iodide, and 17 mg of sodium hydride were mixed in 2 mL of DMF and stirred at 50°C for a day. After completion of the reaction, a saturated aqueous solution of ammonium chloride (NH₄Cl) was added into the reaction mixture, followed by extraction with ethyl acetate two to three times. A combined ethyl acetate layer was dried over anhydrous magnesium sulfate and concentrated under reduced pressure to obtain residues, which were then purified through MPLC so as to obtain 5-bromo-2-(2-ethoxypropan-2-yl)pyrimidine.

[Step 2] (S)-4-((1-(4-chloro-8-(2-(2-ethoxypropan-2-yl)pyrimidin-5-yl)-1-oxo-2-phenyl-1,2-dihydroisoquinolin-3-yl)ethyl)amino)pyrido[2,3-d]pyrimidin-5(8H)-one was obtained by substantially the same method as that of Example 3 except for using 5-bromo-2-(2-ethoxypropan-2-yl)pyrimidine prepared in above step 1 instead of using 5-bromo-2-(1H-pyrrol-1-yl)pyridine in Example 3.

¹H-NMR (400MHz, CDCl₃) δ ppm 1.44 (t, 3H), 1.64(s, 3H), 1.66(s, 3H), 1.69 (d, 3H), 3.32(m, 2H), 5.01 (m, 1H), 6.29-8.75 (m, 13H), 10.98(s, 1H)

### <Example 405> Preparation of (S)-4-((1-(4-chloro-8-(2-(2-methoxypropan-2-yl)pyrimidin-5-yl)-1-oxo-2-phenyl-1,2-dihydroisoquinolin-3-yl)ethyl)amino)pyrido[2,3-d]pyrimidin-5(8H)-one

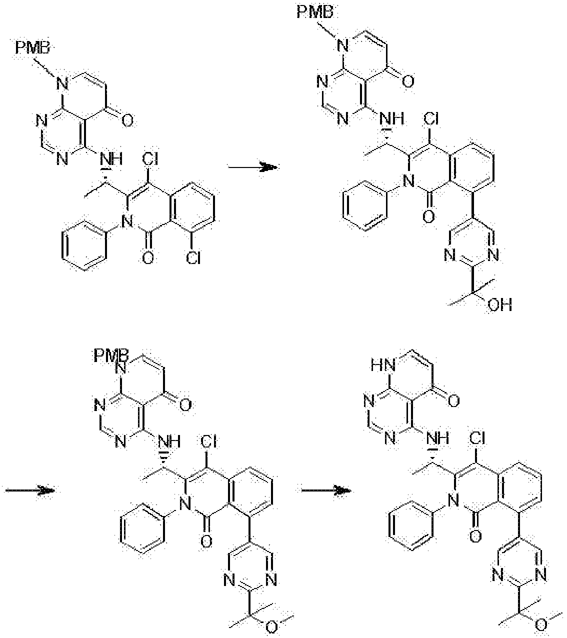

[Step 1] The 50 mg of (S)-4-((1-(4,8-dichloro-1-oxo-2-phenyl-1,2-dihydroisoquinolin-3-yl)ethyl)amino)-8-(4-methoxybenzyl)pyrido[2,3-d]pyrimidin-5(8H)-one obtained in Preparation Example 3, 22 mg of 2-(5-(4,4,5,5-tetramethyl-1,3,2-dioxaborolan-2-yl)pyrimidin-2-yl)propan-2-ol, 16.7 µL of 2M aqueous potassium carbonate solution, and 5.8 mg of bistriphenylphosphinpalladium chloride (Pd(PPh₃)₂Cl₂) were dissolved in 2 mL of dioxane and reacted at 170°C for 30 minutes. After completion of the reaction, a saturated saline solution was added, followed by extraction with ethyl acetate two to three times. A combined ethyl acetate layer was dried over anhydrous magnesium sulfate, and concentrated under reduced pressure to obtain residues, which were then purified through Prep-LC.

[Step 2] The 10 mg of (S)-4-((1-(4-chloro-8-(2-(2-hydroxypropan-2-yl)pyrimidin-5-yl)-1-oxo-2-phenyl-1,2-dihydroisoquinolin-3-yl)ethyl)amino)-8-(4-methoxybenzyl)pyrido[2,3-d]pyrimidin-5(8H)-one obtained in above step 1, 10 mg of methyl iodide and 1 mg of sodium hydride were added to 2 mL of tetrahydrofuran and stirred at room temperature. After completion of the reaction, ethyl acetate and a saturated saline solution were added, followed by extraction with ethyl acetate two to three times. A combined ethyl acetate layer was dried over anhydrous magnesium sulfate, and concentrated under reduced pressure to obtain residues, which were then purified through Prep-LC.

[Step 3] The product obtained in step 2 and TFA were excessively added to CH₂Cl₂ solvent and stirred at room temperature for a day. After completion of the reaction, the residues obtained by concentrating the reactant under reduced pressure were purified through Prep-LC so as to obtain (S)-4-((1-(4-chloro-8-(2-(2-methoxypropan-2-yl)pyrimidin-5-yl)-1-oxo-2-phenyl-1,2-dihydroisoquinolin-3-yl) ethyl)amino)pyrido[2,3-d]pyrimidin-5(8H)-one.

¹H-NMR (400MHz, CDCl₃) δ ppm 1.64 (d, 6H), 1.69 (d, 3H), 3.20 (s, 3H), 5.01 (m, 1H), 6.30-8.75 (m, 13H), 10.99(s, 1H), 11.01(s, 1H)

### <Example 406> Preparation of (S)-4-((1-(4-chloro-1-oxo-2-phenyl-8-(2-(2-propoxypropan-2-yl)pyrimidin-5-yl)-1,2-dihydroisoquinolin-3-yl) ethyl)amino)pyrido[2 ,3-d]pyrim idin-5(8H)-one

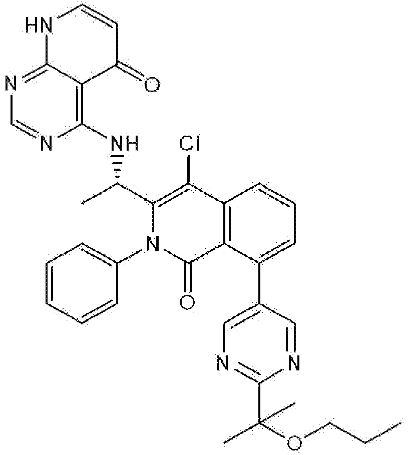

In Example 406, (S)-4-((1-(4-chloro-1-oxo-2-phenyl-8-(2-(2-propoxypropan-2-yl)pyrimidin-5-yl)-1,2-dihydroisoquinolin-3-yl)ethyl)amino)pyrido[2,3-d]pyrimidin-5(8H)-one was obtained by substantially the same preparation method as described in above Example 404, except for using an appropriate reactant in consideration of the structure of the corresponding compound.

¹H-NMR (400MHz, CDCl₃) δ ppm 0.82 (t, 3H), 1.51-1.71 (m, 11H), 3.21 (m, 2H), 5.02 (m, 1H), 6.30-8.74(m, 13H), 11.03 (s, 1H)

### <Example 407> Preparation of 4-(((1S)-1-(4-chloro-8-(2-(S-methylsulfonimidoyl)pyrimidin-5-yl)-1-oxo-2-phenyl-1,2-dihydroisoquinolin-3-yl)ethyl)amino)pyrido[2,3-d]pyrimidin-5(8H)-one

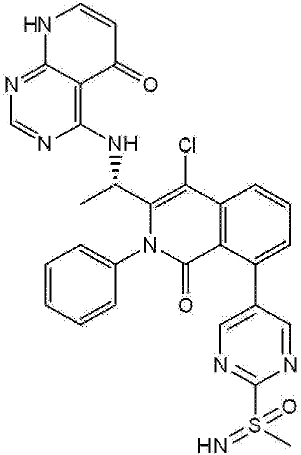

The 4-(((1S)-1-(4-chloro-8-(2-(S-methylsulfonimidoyl)pyrimidin-5-yl)-1-oxo-2-phenyl-1,2-dihydroisoquinolin-3-yl)ethyl)amino)pyrido[2,3-d]pyrimidin-5(8H)-one represented by the above formula was prepared according to a following reaction formula.

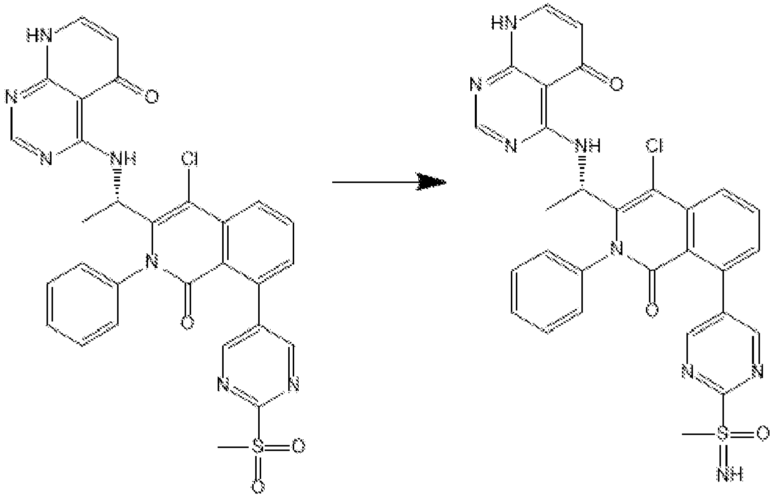

The 115 mg of (S)-4-((1-(4-chloro-8-(2-(methylsulfonyl)pyrimidin-5-yl)-1-oxo-2-phenyl-1,2-dihydroisoquinolin-3-yl)ethyl)amino)pyrido[2,3-d]pyrimidin-5(8H)-one of Example 392, 37 mg of ammonium carbamate, and 189 mg of (diacetoxyiodo)benzene were added into 1 mL of methanol and stirred at room temperature for two hours. After completion of the reaction, the mixture was filtered under reduced pressure and concentrated under reduced pressure to obtain residues, which were then purified through Prep-LC so as to obtain 4-(((1S)-1-(4-chloro-8-(2-(S-methylsulfonimidoyl)pyrimidin-5-yl)-1-oxo-2-phenyl-1,2-dihydroisoquinolin-3-yl)ethyl)amino)pyrido[2,3-d]pyrimidin-5(8H)-one.

¹H-NMR (400MHz, CDCl₃) δ ppm 1.69(d, 3H), 3.41(s, 3H), 5.06 (m, 1H), 6.31-8.75(m, 13H), 10.82(s, 1H), 11.00(s, 1H)

### <Examples 408 to 411>

In Examples 408 to 411, the compound of each Example was prepared by substantially the same preparation method as described in above Example 407, except for using an appropriate reactant in consideration of the structure of the corresponding compound.

### <Example 408> Preparation of 4-(((S)-1-(4-chloro-1-oxo-2-phenyl-8-((R)-propylsulfonimidoyl)-1,2-dihydroisoquinolin-3-yl)ethyl)amino)pyrido[2,3-d]pyrimidin-5(8H)-one

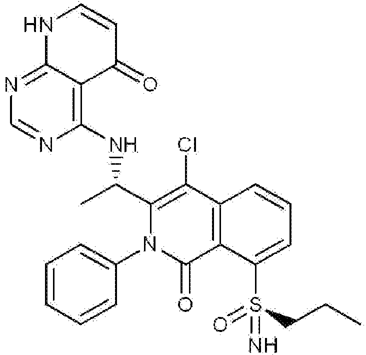

¹H-NMR (400MHz, CDCl₃) δ ppm 0.93(t, 3H), 1.61-1.83 (m, 5H), 3.56-3.68 (m, 2H), 5.12 (m, 1H), 6.29-8.53 (m, 11H), 10.94 (s, 1H), 11.05(s, 1H)

### <Example 409> Preparation of 4-(((S)-1-(4-chloro-1-oxo-2-phenyl-8-((S)-propylsulfonimidoyl)-1,2-dihydroisoquinolin-3-yl)ethyl)amino)pyrido[2,3-d]pyrimidin-5(8H)-one

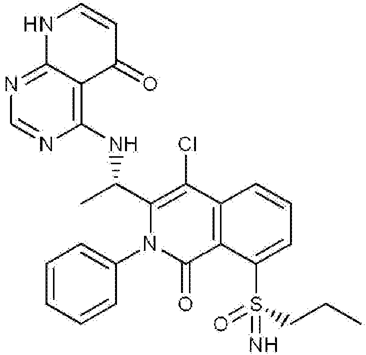

¹H-NMR (400MHz, CDCl₃) δ ppm 0.92(t, 3H), 1.53-1.90 (m, 5H) , 3.51-3.73 (m, 2H), 4.86 (m, 1H), 6.25-8.45 (m, 11H), 10.93(s, 1H), 11.63(s, 1H)

### <Example 410> Preparation of 4-(((S)-1-(4-chloro-1-oxo-2-phenyl-8-((R)-propan-2-ylsulfonimidoyl)-1,2-dihydroisoquinolin-3-yl)ethyl)amino)pyrido[2,3-d]pyrimidin-5(8H)-one

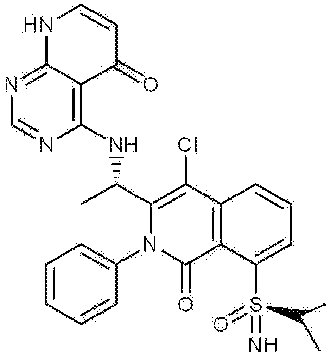

¹H-NMR (400MHz, CDCl₃) δ ppm 1.11(d, 3H), 1.43(d, 3H), 1.68 (d, 3H), 4.43(s, 1H), 5.15 (m, 1H), 6.34-8.58(m, 11H), 11.31(s, 1H)

### <Example 411> Preparation of 4-(((S)-1-(4-chloro-1-oxo-2-phenyl-8-((S)-propan-2-ylsulfonimidoyl)-1,2-dihydroisoquinolin-3-yl)ethyl)amino)pyrido[2,3-d]pyrimidin-5(8H)-one

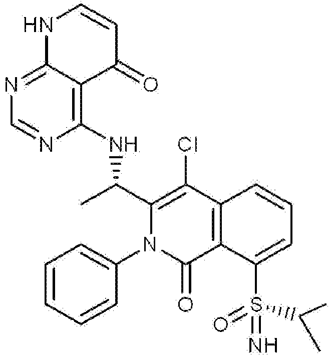

¹H-NMR (400MHz, CDCl₃) δ ppm 1.11(d, 3H), 1.43(d, 3H), 1.68 (d, 3H), 4.43(s, 1H), 5.15 (m, 1H), 6.34-8.58(m, 11H), 11.31(s, 1H)

### <Example 412> Preparation of (S)-4-((1-(4-chloro-1-oxo-2-phenyl-8-(pyridin-2-ylthio)-1,2-dihydroisoquinolin-3-yl)ethyl)amino)pyrido[2,3-d]pyrimidin-5(8H)-one

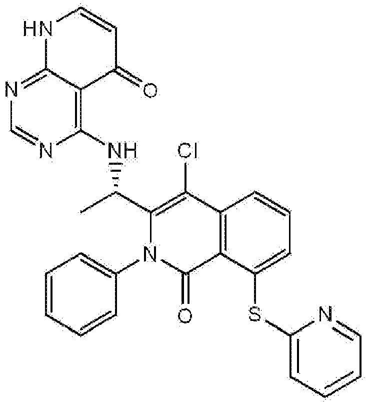

In Example 412, (S)-4-((1-(4-chloro-1-oxo-2-phenyl-8-(pyridin-2-ylthio)-1,2-dihydroisoquinolin-3-yl)ethyl)amino)pyrido[2,3-d]pyrimidin-5(8H)-one was obtained by substantially the same preparation method as described in above Example 369, except for using an appropriate reactant in consideration of the structure of the corresponding compound.

¹H-NMR (400MHz, CDCl₃) δ ppm 1.60(d, 3H), 4.90(m, 1H), 6.26(d, 1H), 6.92(m, 1H), 7.26-7.55(m, 6H), 7.77(t, 1H), 7.95(t, 1H), 8.07(d, 1H), 8.20(s, 1H), 8.40(d, 2H), 8.80(d, 1H), 10.95(br, 1H)

### <Examples 413 and 414>

In Examples 413 and 414, the compound of each Example was prepared by substantially the same preparation method as described in above Example 390, except for using an appropriate reactant in consideration of the structure of the corresponding compound.

### <Example 413> Preparation of 4-(((1S)-1-(4-chloro-1-oxo-2-phenyl-8-(pyridin-2-ylsulfinyl)-1,2-dihydroisoquinolin-3-yl)ethyl)amino)pyrido[2,3-d]pyrimidin-5(8H)-one

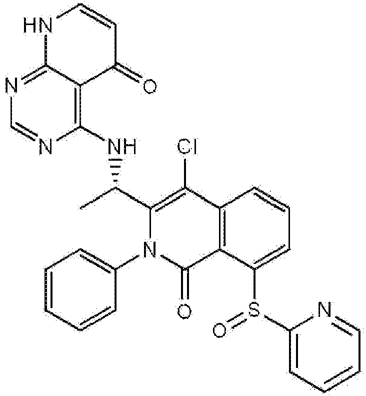

¹H-NMR (400MHz, CDCl₃) δ ppm 1.64(3H, q), 4.85-5.15(m, 1H), 6.32(m, 1H), 6.90-8.90(m, 14H), 11.00(br, 1H)

### <Example 414> Preparation of (S)-4-((1-(4-chloro-1-oxo-2-phenyl-8-(pyridin-2-ylsulfonyl)-1,2-dihydroisoquinolin-3-yl)ethyl)amino)pyrido[2,3-d]pyrimidin-5(8H)-one

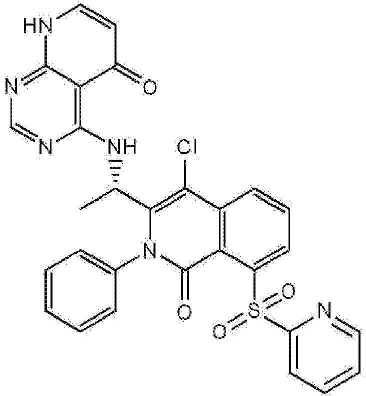

¹H-NMR (400MHz, CDCl₃) δ ppm 1.60(d, 3H), 4.90(m, 1H), 6.26(d, 1H), 6.92(m, 1H), 7.26-7.55(m, 6H), 7.77(t, 1H), 7.95(t, 1H), 8.07(d, 1H), 8.20(s, 1H), 8.40(d, 2H), 8.80(d, 1H), 10.95(br, 1H)

### <Example 415> Preparation of (S)-4-((1-(4-chloro-8-(isopropylthio)-1-oxo-2-phenyl-1,2-dihydroisoquinolin-3-yl)ethyl)amino)pyrido[2,3-d]pyrimidin-5(8H)-one

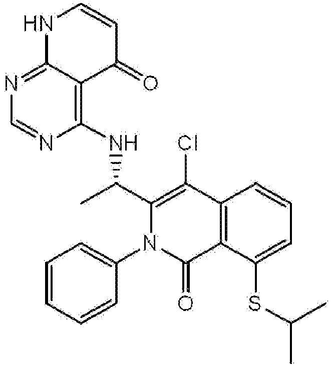

In Example 415, (S)-4-((1-(4-chloro-8-(isopropylthio)-1-oxo-2-phenyl-1,2-dihydroisoquinolin-3-yl)ethyl)amino)pyrido[2,3-d]pyrimidin-5(8H)-one was obtained by substantially the same preparation method as described in above Example 369, except for using an appropriate reactant in consideration of the structure of the corresponding compound.

¹H-NMR (400MHz, CDCl₃) δ ppm 1.38(t, 6H), 1.66(d, 3H), 3.47(m, 1H), 5.02(m, 1H), 6.30(d, 1H), 7.15-7.80(m, 9H), 8.25(s, 1H), 11.00(br, 1H), 12.10(br, 1H)

### <Example 416> Preparation of (S)-4-((1-(4-chloro-8-(isopropylsulfonyl)-1-oxo-2-phenyl-1,2-dihydroisoquinolin-3-yl)ethyl)amino)pyrido[2,3-d]pyrimidin-5(8H)-one

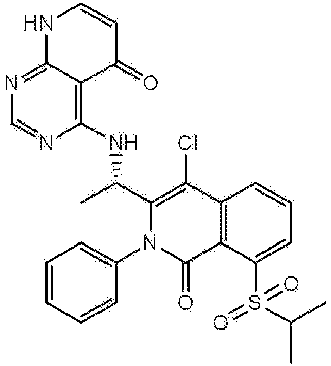

In Example 416, (S)-4-((1-(4-chloro-8-(isopropylsulfonyl)-1-oxo-2-phenyl-1,2-dihydroisoquinolin-3-yl)ethyl)amino)pyrido[2,3-d]pyrimidin-5(8H)-one was obtained by substantially the same preparation method as described in above Example 390, except for using an appropriate reactant in consideration of the structure of the corresponding compound.

¹H-NMR (400MHz, CDCl₃) δ ppm 1.23(d, 3H), 1.34(d, 3H), 1.69(d, 3H), 4.75(m, 1H), 5.01(m, 1H), 6.30(d, 1H), 7.25(m, 1H), 7.50-7.90(m, 6H), 8.30(s, 1H), 8.35(d, 1H), 8.50(d, 1H), 10.95(br, 1H)

### <Examples 417 and 418>

In Examples 417 and 418, the compound of each Example was prepared by substantially the same preparation method as described in above Example 369, except for using an appropriate reactant in consideration of the structure of the corresponding compound.

### <Example 417> Preparation of (S)-4-((1-(4-chloro-1-oxo-2-phenyl-8-(thiazol-2-ylthio)-1,2-dihydroisoquinolin-3-yl)ethyl)amino)pyrido[2,3-d]pyrimidin-5(8H)-one

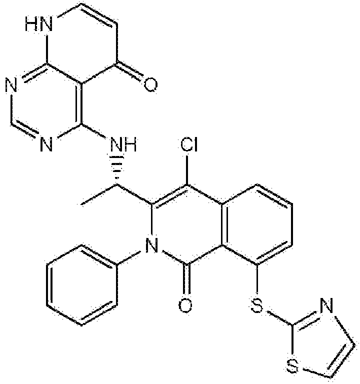

¹H-NMR (400MHz, CDCl₃) δ ppm 1.68(d, 3H), 5.03(m, 1H), 6.30(d, 1H), 6.87(d, 1H), 7.25(m, 1H), 7.40-8.10(m, 9H), 8.25(d, 1H), 11.00(br, 1H)

### <Example 418> Preparation of (S)-4-((1-(4-chloro-8-(oxetan-3-ylthio)-1-oxo-2-phenyl-1,2-dihydroisoquinolin-3-yl)ethyl)amino)pyrido[2,3-d]pyrimidin-5(8H)-one

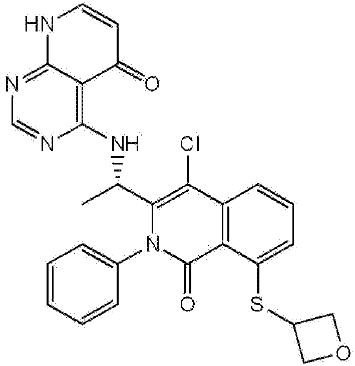

¹H-NMR (400MHz, CDCl₃) δ ppm 1.67(d, 3H), 4.50(m, 1H), 4.60(m, 1H), 5.05(m, 1H), 5.20(m, 1H), 6.30(d, 1H), 6.70(d, 1H), 7.20(m, 1H), 7.40-7.65(m, 5H), 7.75(m, 1H), 8.25(s, 1H), 10.95(br, 1H), 11.70(br, 1H)

### <Examples 419 and 420>

In Examples 419 and 420, the compound of each Example was prepared by substantially the same preparation method as described in above Example 390, except for using an appropriate reactant in consideration of the structure of the corresponding compound.

### <Example 419> Preparation of 4-(((1S)-1-(4-chloro-1-oxo-2-phenyl-8-(pyrimidin-2-ylsulfinyl)-1,2-dihydroisoquinolin-3-yl)ethyl)amino)pyrido[2,3-d]pyrimidin-5(8H)-one

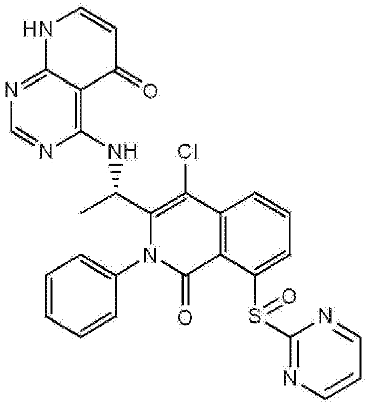

¹H-NMR (400MHz, CDCl₃) δ ppm 1.66 (d, 3H), 4.96 (m, 1H), 6.29 - 8.66 (m, 14H), 10.88 (s, 1H)

### <Example 420> Preparation of (S)-4-((1-(4-chloro-1-oxo-2-phenyl-8-(pyrimidin-2-ylsulfonyl)-1,2-dihydroisoquinolin-3-yl)ethyl)amino)pyrido[2,3-d]pyrimidin-5(8H)-one

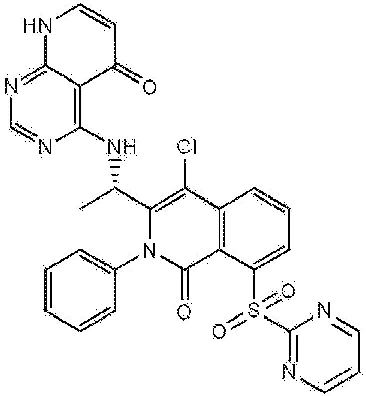

¹H-NMR (400MHz, CDCl₃) δ ppm 1.63 (d, 3H), 4.99 (m, 1H), 6.31 - 8.80 (m, 14H), 11.13 (s, 1H)

### <Examples 421 and 422>

In Examples 421 and 422, the compound of each Example was prepared by substantially the same preparation method as described in above Example 407, except for using an appropriate reactant in consideration of the structure of the corresponding compound.

### <Example 421> Preparation of 4-(((S)-1-(4-chloro-1-oxo-2-phenyl-8-((S)-pyrimidine-2-sulfonimidoyl)-1,2-dihydroisoquinolin-3-yl) ethyl)amino)pyrido[2,3-d]pyrimidin-5(8H)-one

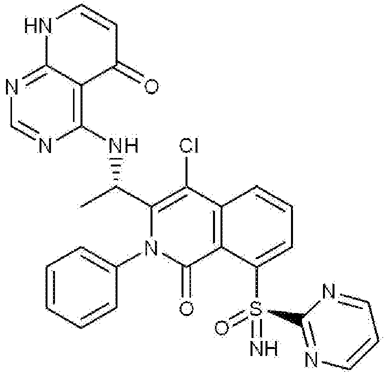

¹H-NMR (400MHz, CDCl₃) δ ppm 1.64(d, 3H), 4.99(m, 1H), 6.28-8.79(m, 14H), 10.85 (s, 1H)

### <Example 422> Preparation of 4-(((S)-1-(4-chloro-1-oxo-2-phenyl-8-((R)-pyrimidine-2-sulfonimidoyl)-1,2-dihydroisoquinolin-3-yl) ethyl)amino)pyrido[2,3-d]pyrimidin-

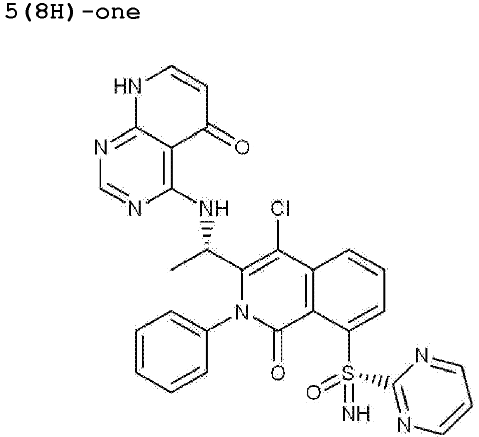

¹H-NMR (400MHz, CDCl₃) δ ppm 1.61(d, 3H), 4.88(m, 1H), 6.28-8.77(m, 14H), 10.84 (s, 1H)

### <Example 423> Preparation of (S)-4-((1-(4-chloro-1-oxo-2-phenyl-8-(pyrimidin-2-ylthio)-1,2-dihydroisoquinolin-3-yl)ethyl)amino)pyrido[2,3-d]pyrimidin-5(8H)-one

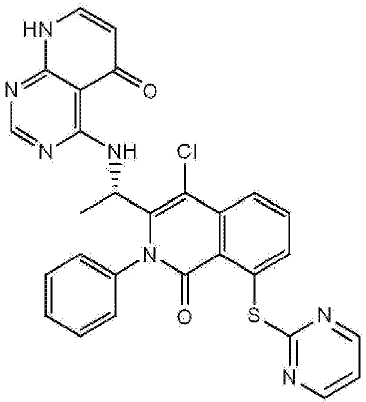

In Example 423, (S)-4-((1-(4-chloro-1-oxo-2-phenyl-8-(pyrimidin-2-ylthio)-1,2-dihydroisoquinolin-3-yl) ethyl) amino)pyrido[2,3-d]pyrimidin-5(8H)-one was obtained by substantially the same preparation method as described in above Example 369, except for using an appropriate reactant in consideration of the structure of the corresponding compound.

¹H-NMR (400MHz, CDCl₃) δ ppm 1.67 (d, 3H), 5.02 (m, 1H), 6.29 - 8.54 (m, 14H), 10.95 (m, 1H), 10.96 (m, 1H)

### <Example 424> Preparation of (S)-4-((1-(8-(2-(tert-butoxy)pyrimidin-5-yl)-4-chloro-1-oxo-2-phenyl-1,2-dihydroisoquinolin-3-yl) ethyl)amino)pyrido[2,3-d]pyrimidin-5(8H)-one

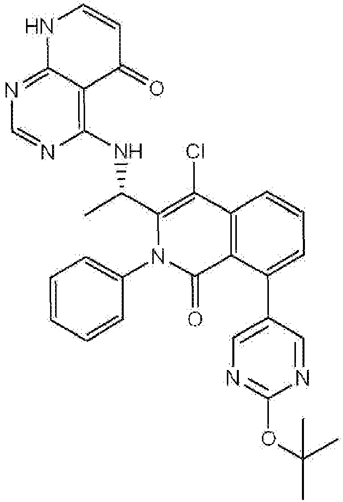

In Example 424, (S)-4-((1-(8-(2-(tert-butoxy)pyrimidin-5-yl)-4-chloro-1-oxo-2-phenyl-1,2-dihydroisoquinoline-3-yl)ethyl)amino)pyrido[2,3-d]pyrimidin-5(8H)-one was obtained by substantially the same preparation method as described in above Example 3, except for using an appropriate reactant in consideration of the structure of the corresponding compound.

¹H-NMR (400MHz, CDCl₃) δ ppm 1.64(s, 9H), 1.68(d, 3H), 4.98-5.03(m, 1H), 6.28-8.42(m, 13H), 11.02(br, 1H), 11.80(br, 1H)

### <Example 425> Preparation of 4-(((1S)-1-(4-chloro-8-(S-methylsulfonimidoyl)-1-oxo-2-phenyl-1,2-dihydroisoquinolin-3-yl)ethyl)amino)pyrido[2,3-d]pyrimidin-5(8H)-one

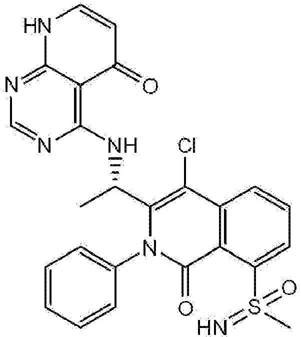

In Example 425, 4-(((1S)-1-(4-chloro-8-(S-methylsulfonimidoyl)-1-oxo-2-phenyl-1,2-dihydroisoquinolin-3-yl)ethyl)amino)pyrido[2,3-d]pyrimidin-5(8H)-one was obtained by substantially the same preparation method as described in above Example 407, except for using an appropriate reactant in consideration of the structure of the corresponding compound.

¹H-NMR (400MHz, CDCl₃) δ ppm 1.68 (d, 3H), 3.46 (s, 3H), 5.11 (m, 1H), 6.29 - 8.55 (m, 11H), 10.53 (m, 1H), 10.92 (s, 1H)

### <Examples 426 and 427>

In Examples 426 and 427, the compound of each Example was prepared by substantially the same preparation method as described in above Example 369, except for using an appropriate reactant in consideration of the structure of the corresponding compound.

### <Example 426> Preparation of (S)-4-((1-(8-(tert-butylthio)-4-chloro-1-oxo-2-phenyl-1,2-dihydroisoquinolin-3-yl)ethyl)amino)pyrido[2,3-d]pyrimidin-5(8H)-one

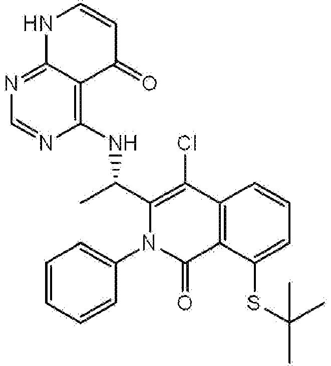

¹H-NMR (400MHz, CDCl₃) δ ppm 1.55(9H, s), 1.66(d, 3H), 5.02(m, 1H), 6.30(d, 1H), 7.17(m, 1H), 7.45-7.80(m, 8H), 8.24(s, 1H), 10.95(br, 1H), 11.30(br, 1H)

### <Example 427> Preparation of (S)-4-((1-(4-chloro-8-mercapto-1-oxo-2-phenyl-1,2-dihydroisoquinolin-3-yl)ethyl)amino)pyrido[2,3-d]pyrimidin-5(8H)-one

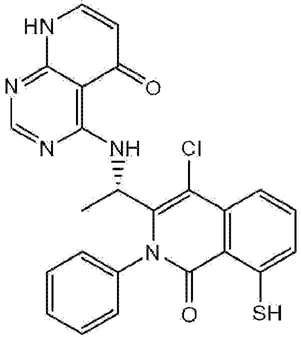

¹H-NMR (400MHz, CDCl₃) δ ppm 1.68(d, 3H), 4.95(m, 1H), 6.35(d, 1H), 7.22(m, 1H), 7.35-7.95(m, 8H), 8.32(s, 1H), 11.00(br, 1H), 12.50(br, 1H)

### <Examples 428 to 430>

In Examples 428 to 430, the compound of each Example was prepared by substantially the same preparation method as described in above Example 362, except for using an appropriate reactant in consideration of the structure of the corresponding compound.

### <Example 428> Preparation of (S)-4-((1-(4-chloro-1-oxo-2-phenyl-8-(pyridin-4-yloxy)-1,2-dihydroisoquinolin-3-yl)ethyl)amino)pyrido[2,3-d]pyrimidin-5(8H)-one

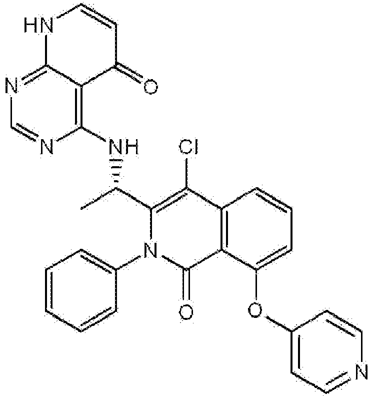

¹H-NMR (400MHz, CDCl₃) δ ppm 1.68(d, 3H), 5.05(m, 1H), 6.28(d, 1H), 6.40-6.60(m, 2H), 7.11(m, 1H), 7.25-7.85(m, 9H), 8.20-8.35(m, 2H), 10.95(br, 1H), 11.05(br, 1H)

### <Example 429> Preparation of (S)-4-((1-(4-chloro-1-oxo-2-phenyl-8-(pyridin-4-ylmethoxy)-1,2-dihydroisoquinolin-3-yl)ethyl)amino)pyrido[2,3-d]pyrimidin-5(8H)-one

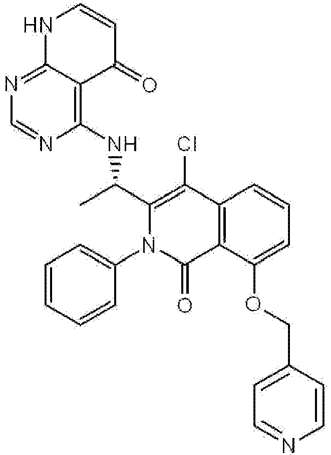

¹H-NMR (400MHz, CDCl₃) δ ppm 1.67(d, 3H), 5.02(m, 1H), 5.24(s, 2H), 6.29(d, 1H), 6.91(d, 1H), 7.23(m, 1H), 7.40-7.80(m, 9H), 8.23(s, 1H), 8.55(d, 2H), 10.95(br, 1H), 11.05(br, 1H)

### <Example 430> Preparation of (S)-4-((1-(4-chloro-1-oxo-2-phenyl-8-(pyridin-2-ylmethoxy)-1,2-dihydroisoquinolin-3-yl)ethyl)amino)pyrido[2,3-d]pyrimidin-5(8H)-one

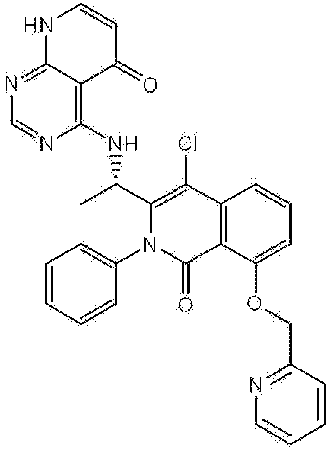

¹H-NMR (400MHz, CDCl₃) δ ppm 1.67(d, 3H), 5.02(m, 1H), 5.33(s, 2H), 6.29(d, 1H), 7.00(d, 1H), 7.15(m, 1H), 7.22(m, 1H), 7.40-7.70(m, 7H), 7.80(m, 1H), 7.92(d, 1H), 8.23(s, 1H), 8.52(m, 1H), 10.95(br, 1H), 11.10(br, 1H)

### <Examples 431 to 433>

In Examples 431 to 433, the compound of each Example was prepared by substantially the same preparation method as described in above Example 390, except for using an appropriate reactant in consideration of the structure of the corresponding compound.

### <Example 431> Preparation of 4-(((1S)-1-(4-chloro-8-(oxetan-3-ylsulfinyl)-1-oxo-2-phenyl-1,2-dihydroisoquinolin-3-yl)ethyl)amino)pyrido[2,3-d]pyrimidin-5(8H)-one

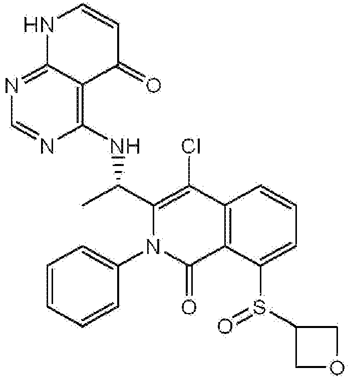

¹H-NMR (400MHz, CDCl₃) δ ppm 1.69(m, 3H), 4.20(m, 1H), 4.65(m, 1H), 4.80-5.20(m, 4H), 6.31(d, 1H), 7.22(m, 1H), 7.50-7.80(m, 5H), 7.95(m, 1H), 8.10-8.40(m, 3H), 11.00(br, 1H)

### <Example 432> Preparation of (S)-4-((1-(4-chloro-8-(oxetan-3-ylsulfonyl)-1-oxo-2-phenyl-1,2-dihydroisoquinolin-3-yl)ethyl)amino)pyrido[2,3-d]pyrimidin-5(8H)-one

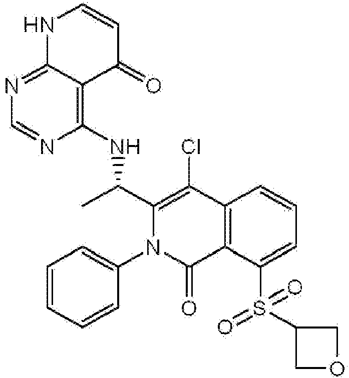

¹H-NMR (400MHz, CDCl₃) δ ppm 1.68(d, 3H), 4.75(t, 1H), 4.85(t, 1H), 4.92(t, 1H), 5.02(m, 1H), 5.07(t, 1H), 5.65(m, 1H), 6.31(d, 1H), 7.20(m, 1H), 7.50-7.65(m, 4H), 7.75(m, 1H), 7.90(t, 1H), 8.27(s, 1H), 8.38(d, 1H), 8.59(d, 1H), 10.97(br, 1H), 11.40(br, 1H)

### <Example 433> Preparation of (S)-4-((1-(8-(tert-butylsulfonyl)-4-chloro-1-oxo-2-phenyl-1,2-dihydroisoquinolin-3-yl)ethyl)amino)pyrido[2,3-d]pyrimidin-5(8H)-one

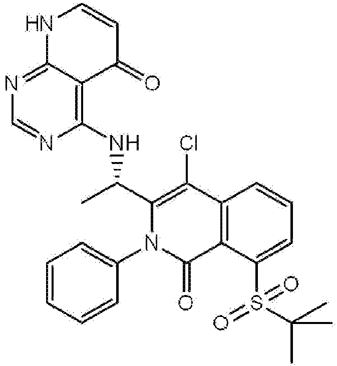

¹H-NMR (400MHz, CDCl₃) δ ppm 1.45(9H, s), 1.67(d, 3H), 5.00(m, 1H), 6.28(d, 1H), 7.20(m, 1H), 7.40-7.60(m, 4H), 7.70(m, 1H), 7.80(t, 1H), 8.24(s, 1H), 8.40(m, 2H), 11.00(br, 1H)

### <Example 434> Preparation of (S)-4-((1-(4-chloro-8-(ethylthio)-1-oxo-2-phenyl-1,2-dihydroisoquinolin-3-yl)ethyl)amino)pyrido[2,3-d]pyrimidin-5(8H)-one

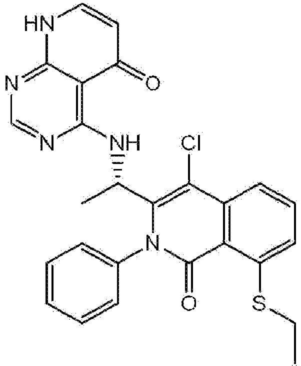

In Example 434, (S)-4-((1-(4-chloro-8-(ethylthio)-1-oxo-2-phenyl-1,2-dihydroisoquinolin-3-yl) ethyl)amino)pyrido[2,3-d]pyrimidin-5(8H)-one was obtained by substantially the same preparation method as described in above Example 369, except for using an appropriate reactant in consideration of the structure of the corresponding compound.

¹H-NMR (400MHz, CDCl₃) δ ppm 1.39 (t, 3H), 1.68 (d, 3H), 2.84-2.92(m, 2H), 5.04 (m, 1H), 6.29 - 8.24 (m, 11H), 10.82 (m, 1H), 10.92 (s, 1H)

### <Examples 435 and 436>

In Examples 435 and 436, the compound of each Example was prepared by substantially the same preparation method as described in above Example 407, except for using an appropriate reactant in consideration of the structure of the corresponding compound.

### <Example 435> Preparation of 4-(((S)-1-(4-chloro-8-((R)-ethylsulfonimidoyl)-1-oxo-2-phenyl-1,2-dihydroisoquinolin-3-yl)ethyl)amino)pyrido[2,3-d]pyrimidin-5(8H)-one

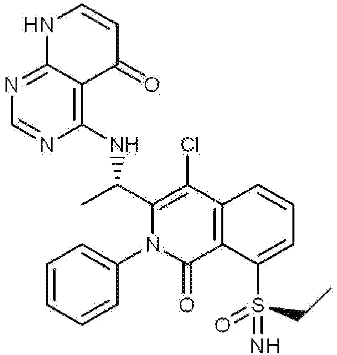

¹H-NMR (400MHz, CDCl₃) δ ppm 1.25 (t, 3H), 1.67 (d, 3H), 3.59-3.76 (m, 2H), 5.11 (m, 1H), 6.29 - 8.55 (m, 11H), 10.29 (s, 1H), 10.91 (s, 1H)

### <Example 436> Preparation of 4-(((S)-1-(4-chloro-8-((S)-ethylsulfonimidoyl)-1-oxo-2-phenyl-1,2-dihydroisoquinolin-3-yl)ethyl)amino)pyrido[2,3-d]pyrimidin-5(8H)-one

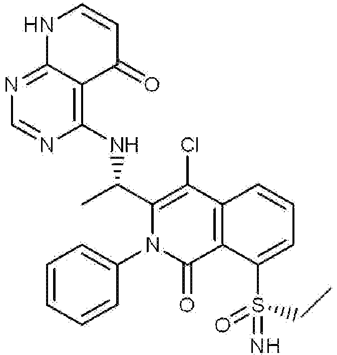

¹H-NMR (400MHz, CDCl₃) δ ppm 1.25 (t, 3H), 1.70 (d, 3H), 3.55-3.78 (m, 2H), 4.90 (m, 1H), 6.26 - 8.50 (m, 11H), 10.52 (s, 1H), 10.88 (s, 1H)

### <Example 437> Preparation of (S)-4-((1-(4-chloro-1-oxo-2-phenyl-8-(pyridin-3-yloxy)-1,2-dihydroisoquinolin-3-yl)ethyl)amino)pyrido[2,3-d]pyrimidin-5(8H)-one

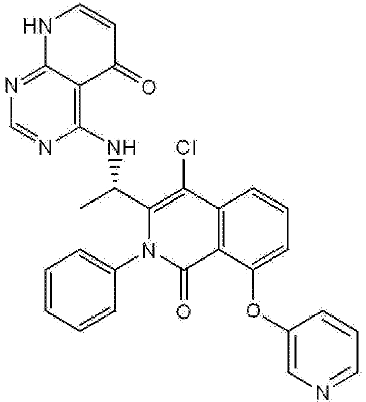

In Example 437, (S)-4-((1-(4-chloro-1-oxo-2-phenyl-8-(pyridin-3-yloxy)-1,2-dihydroisoquinolin-3-yl)ethyl)amino)pyrido[2,3-d]pyrimidin-5(8H)-one was obtained by substantially the same preparation method as described in above Example 362, except for using an appropriate reactant in consideration of the structure of the corresponding compound.

¹H-NMR (400MHz, CDCl₃) δ ppm 1.69(d, 3H), 5.05(m, 1H), 6.30(d, 1H), 7.03(d, 1H), 7.15-7.35(m, 3H), 7.40-7.60(m, 4H), 7.60-7.75(m, 2H), 7.90(m, 1H), 8.20-8.35(m, 2H), 11.00(br, 1H)

### <Example 438> Preparation of (S)-4-((1-(4-chloro-8-(2-(1-hydroxycyclobutyl)pyrimidin-5-yl)-1-oxo-2-phenyl-1,2-dihydroisoquinolin-3-yl) ethyl)amino)pyrido[2,3-d]pyrim idin-5(8H)-one

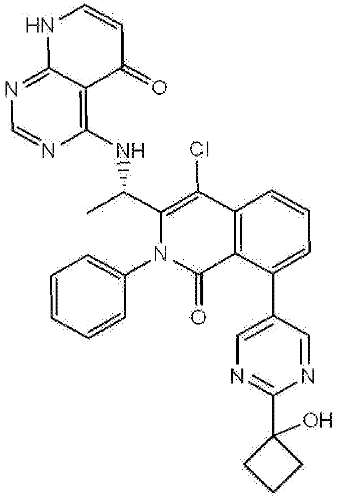

In Example 438, (S)-4-((1-(4-chloro-8-(2-(1-hydroxycyclobutyl)pyrimidin-5-yl)-1-oxo-2-phenyl-1,2-dihydroisoquinolin-3-yl) ethyl)amino)pyrido[2,3-d]pyrimidin-5(8H)-one was obtained by substantially the same preparation method as described in above Example 3, except for using an appropriate reactant in consideration of the structure of the corresponding compound.

¹H-NMR (400MHz, CDCl₃) δ ppm 1.69(d, 3H), 5.05(m, 1H), 6.30(d, 1H), 7.03(d, 1H), 7.15-7.35(m, 3H), 7.40-7.60(m, 4H), 7.60-7.75(m, 2H), 7.90(m, 1H), 8.20-8.35(m, 2H), 11.00(br, 1H)

### <Examples 439 and 440>

In Examples 439 and 440, the compound of each Example was prepared by substantially the same preparation method as described in above Example 280, except for using an appropriate reactant in consideration of the structure of the corresponding compound.

### <Example 439> Preparation of (S)-4-chloro-1-oxo-3-(1-((5-oxo-5,8-dihydropyrido[2,3-d]pyrimidin-4-yl)amino)ethyl)-2-phenyl-N-(pyridin-4-ylmethyl)-1,2-dihydroisoquinoline-8-carboxamide

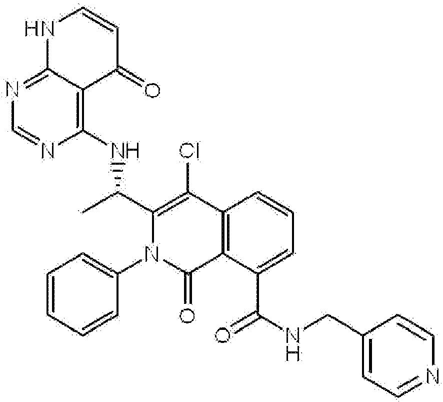

¹H-NMR (400MHz, CDCl₃) δ ppm 1.66(d, 3H), 4.63(m, 2H), 5.02(m, 1H), 6.25(d, 1H), 6.50(m, 1H), 7.20(m, 1H), 7.30-7.80(m, 10H), 8.08(m, 2H), 8.40(2H, br), 10.90(br, 1H)

### <Example 440> Preparation of (S)-4-chloro-1-oxo-3-(1-((5-oxo-5,8-dihydropyrido[2,3-d]pyrimidin-4-yl)amino)ethyl)-N,2-diphenyl-1,2-dihydroisoquinoline-8-carboxamide

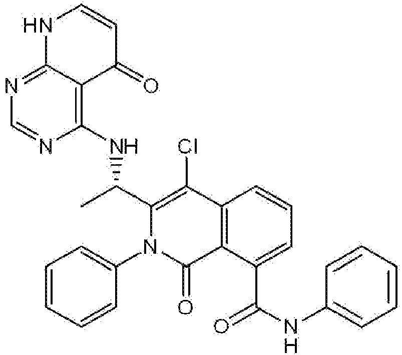

¹H-NMR (400MHz, CDCl₃) δ ppm 1.66(d, 3H), 4.98(m, 1H), 6.22(d, 1H), 7.02(m, 1H), 7.10-7.25(m, 3H), 7.35-7.80(m, 9H), 8.10(m, 1H), 8.17(s, 1H), 10.95(br, 1H), 11.02(br, 1H)

### <Example 441> Preparation of 4-(((S)-1-(4-chloro-8-((S)-1-hydroxyethyl)-1-oxo-2-phenyl-1,2-dihydroisoquinolin-3-yl)ethyl)amino)pyrido[2,3-d]pyrimidin-5(8H)-one

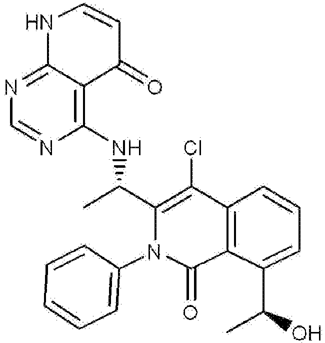

In Example 441, 4-(((S)-1-(4-chloro-8-((S)-1-hydroxyethyl)-1-oxo-2-phenyl-1,2-dihydroisoquinolin-3-yl)ethyl)amino)pyrido[2,3-d]pyrimidin-5(8H)-one was obtained by substantially the same preparation method as described in above Example 361, except for using an appropriate reactant in consideration of the structure of the corresponding compound.

¹H-NMR (400MHz, CDCl₃) δ ppm 1.59-1.70(6H, m), 4.92(1H, s), 5.02-5.07(1H, m), 5.45-5.50(1H, m), 6.29-8.26(11H, m), 10.93(1H, d)

### <Experimental Example 1> Evaluation of inhibitory activity properties against PI3K-δ

The following experiments were performed to verify the inhibitory activity against PI3K-δ with regard to each of the compounds of Examples 1 to 441 according to the present invention.

PI3K(p1108/p857) (h) was incubated in assay buffer comprising 10 µM phosphatidylinositol-4,5-bisphosphate and Mg/ATP (at a concentration as required). The reaction was initiated by the addition of a Mg/ATP mixture, and after incubation for 30 minutes at room temperature, a stop solution comprising EDTA and phosphatidylinositol-3,4,5-triphosphate was added to stop the reaction and carry out biotinylation.

A detection buffer comprising Eu (europium)-labelled anti-GST monoclonal antibody, GST-tagged GRP1 PH domain and streptavidin-allophycocyanin was added. A plate was read in a time-resolved fluorescence mode and a homogeneous time-resolved fluorescence (HTRF) signal was determined according to HTRF = 10000 x (Em 665 nm/Em 620 nm).

### <Experimental Example 2> Evaluation of inhibitory activity properties against DNA-PK

The following experiments were performed to verify the inhibitory activity against DNA-PK with regard to each of the compounds of Examples 1 to 441 according to the present invention.

DNA-PK(h) was subjected to an isothermal treatment in an assay buffer comprising 50 µM GST-cMyc-p53 and Mg/ATP (at a concentration as required). The reaction was initiated by the addition of a Mg/ATP mixture, and after incubation for 30 minutes at room temperature, a stop solution comprising EDTA was added to stop the reaction. A detection buffer comprising d2-labelled anti-GST monoclonal antibody and Europium-labelled anti-phospho Ser15 antibody for phosphorylated p53 was added. A plate was read in a time-resolved fluorescence mode and a homogeneous time-resolved fluorescence (HTRF) signal was determined according to HTRF = 10000 x (Em 665 nm/Em 620 nm).

The results of each of Experimental Examples 1 and 2 are shown in table 2 below.

**[Table 2]**

| | **EXP_VALUE(% Inhibition)** | | | | | **EXP_VALUE(% Inhibition)** | | | |
|---|---|---|---|---|---|---|---|---|---|
| | **PI3K-δ** | | **DNA-PK(h)** | | | **PI3K-δ** | | **DNA-PK(h)** | |
| **Examp le** | **0.01 µM** | **0.1 µM** | **0.01 µM** | **0.1 µM** | **Examp le** | **0.01 µM** | **0.1 µM** | **0.01 µM** | **0.1 µM** |
| **1** | 105 | 104 | 52 | 91 | **223** | 68 | - | 34 | - |
| **2** | 42 | 94 | 56 | 89 | **224** | 57 | - | 19 | - |
| **3** | 69 | - | 44 | - | **225** | 74 | - | 76 | - |
| **4** | 101 | 109 | 53 | 99 | **226** | 83 | - | 56 | - |
| **5** | 106 | 115 | 69 | 100 | **227** | 93 | - | 73 | - |
| **6** | 74 | 95 | 25 | 74 | **228** | 88 | - | 78 | - |
| **7** | 77 | 108 | 26 | 62 | **229** | 68 | - | 45 | - |
| **8** | 36 | 76 | 12 | 28 | **230** | 76 | - | 41 | - |
| **9** | 50 | 61 | 60 | 92 | **231** | 44 | - | 67 | - |
| **10** | 40 | 70 | 15 | 73 | **232** | 23 | - | 15 | - |
| **11** | 21 | 61 | 18 | 53 | **233** | 77 | - | 60 | - |
| **12** | 102 | 114 | 6 | 21 | **234** | 85 | - | 50 | - |
| **13** | 35 | 101 | 3 | 18 | **235** | 80 | - | 46 | - |
| **14** | 105 | 107 | 8 | 27 | **236** | 79 | - | 46 | - |
| **15** | 109 | 108 | 24 | 37 | **237** | 85 | - | 29 | - |
| **16** | 51 | 88 | 7 | 53 | **238** | 15 | - | 15 | - |
| **17** | 99 | 97 | 70 | 94 | **239** | 38 | - | 10 | - |
| **18** | 80 | 96 | 75 | 98 | **240** | 61 | - | 22 | - |
| **19** | 89 | 102 | 78 | 98 | **241** | 32 | - | 49 | - |
| **20** | 67 | 95 | 3 | 55 | **242** | 69 | - | 65 | - |
| **21** | 90 | 99 | 69 | 95 | **243** | 45 | - | 32 | - |
| **22** | 89 | 93 | 69 | 97 | **244** | 65 | - | 7 | - |
| **23** | 82 | 96 | 38 | 85 | **245** | 52 | - | 43 | - |
| **24** | 61 | - | 85 | - | **246** | 85 | - | 79 | - |
| **25** | 6 | - | 7 | - | **247** | 75 | - | 21 | - |
| **26** | 92 | - | 66 | - | **248** | 99 | - | 66 | - |
| **27** | 76 | - | 34 | - | **249** | 69 | 98 | 11 | 69 |
| **28** | 69 | - | 61 | - | **250** | 32 | 82 | 0 | 57 |
| **29** | 70 | - | 99 | - | **251** | 65 | 109 | 6 | 60 |
| **30** | 12 | - | 101 | - | **252** | 53 | 97 | 4 | 42 |
| **31** | 0 | - | 2 | - | **253** | 105 | 102 | 78 | 97 |
| **32** | 96 | - | 75 | - | **254** | 96 | 110 | 40 | 82 |
| **33** | 92 | - | 48 | - | **255** | 25.08 | 50.92 | 15.6 | 73.6 |
| **34** | 74 | - | 56 | - | **256** | 75 | 100 | 44 | 77 |
| **35** | 21 | - | 7 | - | **257** | 68 | 98 | 75 | 98 |
| **36** | 74 | - | 34 | - | **258** | 86 | 101 | 13 | 12 |
| **37** | 89 | - | 60 | - | **259** | 108 | 116 | 50 | 86 |
| **38** | 71 | - | 46 | - | **260** | 96 | 113 | 0 | 36 |
| **39** | 47 | - | 57 | - | **261** | 94 | 110 | 3 | 48 |
| **40** | 67 | - | 61 | - | **262** | 94 | 114 | -9 | 30 |
| **41** | 89 | - | 79 | - | **263** | 77 | 89 | 6 | 52 |
| **42** | 89 | - | 76 | - | **264** | 73 | 82 | 7 | 41 |
| **43** | 9 | - | 3 | - | **265** | 71 | 87 | 40 | 77 |
| **44** | 79 | - | 43 | - | **266** | 69 | 88 | -10 | 3 |
| **45** | 10 | - | 2 | - | **267** | 92 | - | 63 | - |
| **46** | 42 | - | 27 | - | **268** | 76 | - | 27 | - |
| **47** | 86 | - | 48 | - | **269** | 37 | - | 26 | - |
| **48** | 7 | - | -5 | - | **270** | 63 | - | 60 | - |
| **49** | 23 | - | -3 | - | **271** | 60 | - | 19 | - |
| **50** | 96 | - | 53 | - | **272** | 17 | 39 | 10 | 37 |
| **51** | 6 | - | -15 | - | **273** | 44 | - | 36 | - |
| **52** | 13 | - | -2 | - | **274** | 99 | - | 18 | - |
| **53** | 11 | - | -3 | - | **275** | 104 | - | 8 | - |
| **54** | 55 | - | 42 | - | **276** | 96 | - | 29 | - |
| **55** | 59 | - | 44 | - | **277** | 99 | - | 8 | - |
| **56** | 81 | - | 78 | - | **278** | 104 | - | 6 | - |
| **57** | 97 | - | 80 | - | **279** | 105 | - | 11 | - |
| **58** | 95 | - | 87 | - | **280** | 91 | - | 15 | - |
| **59** | 70 | - | 32 | - | **281** | 75 | - | 13 | - |
| **60** | 94 | - | 74 | - | **282** | 79 | - | 14 | - |
| **61** | 89 | - | 67 | - | **283** | 81 | - | 12 | - |
| **62** | 99 | 109 | 54 | 89 | **284** | 25 | - | 10 | - |
| **63** | 95 | 96 | 44 | 84 | **285** | 22 | - | 15 | - |
| **64** | 93 | 101 | 51 | 86 | **286** | 52 | - | 23 | - |
| **65** | 89 | 110 | 58 | 90 | **287** | 38 | - | 28 | - |
| **66** | 72 | 103 | 59 | 92 | **288** | 28 | - | 8 | - |
| **67** | 82 | 92 | 56 | 86 | **289** | 10 | - | 27 | - |
| **68** | 57 | 13 | 48 | 73 | **290** | 80 | 102 | 39 | 76 |
| **69** | 3.3 | 14.54 | 3 | 2.7 | **291** | 91 | 101 | 31 | 78 |
| **70** | 10.25 | 29.88 | -1.2 | 23.6 | **292** | 99 | 112 | 79 | 99 |
| **71** | 17 | 44 | 16 | 41 | **293** | 24 | - | 1 | - |
| **72** | 99 | 112 | 58 | 96 | **294** | 65 | - | 49 | - |
| **73** | 72 | 86 | 53 | 92 | **295** | 24 | - | 56 | - |
| **74** | 65 | 88 | 54 | 90 | **296** | 46 | - | 30 | - |
| **75** | 79 | 86 | 82 | 99 | **297** | 9 | - | 45 | - |
| **76** | 14 | 43 | 1 | 20 | **298** | 11 | - | 50 | - |
| **77** | 45 | 78 | 9 | 64 | **299** | 13 | - | 17 | - |
| **78** | 46 | 78 | 18 | 56 | **300** | 63 | - | 44 | - |
| **79** | 114 | 111 | 82 | 98 | **301** | 41 | - | 14 | - |
| **80** | 110 | 101 | 90 | 106 | **302** | 16 | - | 16 | - |
| **81** | 97 | 105 | 22 | 76 | **303** | 12 | - | 6 | - |
| **82** | 73 | 94 | 74 | 94 | **304** | 71 | 101 | 27 | 71 |
| **83** | 56 | 89 | 66 | 94 | **305** | 83 | 99 | 20 | 62 |
| **84** | 49 | 91 | 16 | 65 | **306** | 57 | 105 | 75 | 95 |
| **85** | 73 | 95 | 56 | 91 | **307** | 83 | 114 | 80 | 95 |
| **86** | 67 | 94 | 72 | 95 | **308** | 28.39 | 60.27 | 21.5 | 70.3 |
| **87** | 38 | 83 | 31 | 79 | **309** | 42.37 | 65.57 | 23.2 | 64.8 |
| **88** | 42 | 88 | 33 | 78 | **310** | 31.48 | 20.4 | -2.5 | 3.2 |
| **89** | 58 | 92 | 17 | 52 | **311** | 18.16 | 39.45 | 47.1 | 82.2 |
| **90** | 66 | 90 | 22 | 63 | **312** | 46.81 | 77.12 | 39.8 | 84.3 |
| **91** | 89 | 101 | 77 | 98 | **313** | 20.19 | 55.04 | 47.2 | 84.6 |
| **92** | 91 | 97 | 63 | 92 | **314** | 76 | 96 | 25 | 75 |
| **93** | 46 | 79 | 13 | 45 | **315** | 39 | 89 | 18 | 62 |
| **94** | 84 | 95 | 78 | 100 | **316** | 43 | 91 | 88 | 99 |
| **95** | 3 | 14 | 9 | 16 | **317** | 45 | 86 | 64 | 96 |
| **96** | 86 | 97 | 78 | 100 | **318** | 23 | 65 | 19 | 35 |
| **97** | 84 | 99 | 35 | 87 | **319** | 18 | 62 | 7 | 25 |
| **98** | 66 | 91 | 84 | 98 | **320** | 35.94 | 83.32 | 12.3 | 57.3 |
| **99** | 83 | 108 | 54 | 95 | **321** | 22 | 68 | 10 | 34 |
| **100** | 82 | 104 | 17 | 54 | **322** | 11 | 30 | -11 | 21 |
| **101** | 90 | 102 | 68 | 94 | **323** | 22 | 65 | 8 | 28 |
| **102** | 110 | 105 | 73 | 94 | **324** | 19 | 58 | 30 | 74 |
| **103** | 91 | 103 | 91 | 101 | **325** | 22 | 65 | 12 | 59 |
| **104** | 63 | 95 | 16 | 66 | **326** | 17 | 51 | -4 | 24 |
| **105** | 75 | 103 | 12 | 75 | **327** | 4 | 25 | -13 | 8 |
| **106** | 53 | 87 | 14 | 59 | **328** | 17 | 42 | 2 | 12 |
| **107** | 59 | 91 | 24 | 54 | **329** | 19 | 55 | 4 | 23 |
| **108** | 99 | 102 | 7 | 66 | **330** | 30 | 72 | 10 | 32 |
| **109** | 61 | 94 | 10 | 65 | **331** | 24 | 74 | 31 | 76 |
| **110** | 93 | 97 | 57 | 91 | **332** | 30 | 75 | -1 | 33 |
| **111** | 80 | 96 | 87 | 98 | **333** | 46 | 79 | 17 | 62 |
| **112** | 92 | 96 | 78 | 97 | **334** | 9 | 49 | 10 | 27 |
| **113** | 82 | 93 | 79 | 98 | **335** | 31 | 95 | 13 | 52 |
| **114** | 87 | 95 | 77 | 97 | **336** | 6 | 19 | 7 | 14 |
| **115** | 78 | 96 | 15 | 72 | **337** | 27 | 70 | 13 | 63 |
| **116** | 73 | 101 | 32 | 91 | **338** | 3 | 19 | -2 | 35 |
| **117** | 82 | 89 | 86 | 99 | **339** | 69 | 20 | 8 | 52 |
| **118** | 29 | 81 | 26 | 74 | **340** | 43 | 91 | 21 | 67 |
| **119** | 84 | 82 | 96 | 101 | **341** | 62 | 91 | 13 | 56 |
| **120** | 65 | 95 | 72 | 98 | **342** | 15 | 45 | 9 | 58 |
| **121** | 84 | 95 | 76 | 98 | **343** | 8 | - | -1 | - |
| **122** | 68 | 88 | 61 | 92 | **344** | 13 | - | -7 | - |
| **123** | 66 | 94 | 58 | 93 | **345** | 10 | - | 0 | - |
| **124** | 83 | 99 | 44 | 86 | **346** | 9 | - | 1 | - |
| **125** | 68 | 95 | 79 | 97 | **347** | 11 | - | 28 | - |
| **126** | 74 | 94 | 70 | 96 | **348** | 5 | - | -1 | - |
| **127** | 89 | 92 | 38 | 81 | **349** | 72 | - | 15 | - |
| **128** | 101 | 105 | 59 | 97 | **350** | 109 | 117 | 15 | 55 |
| **129** | 75 | 67 | 56 | 91 | **351** | 50 | 91 | 45 | 89 |
| **130** | 85 | 89 | 98 | 102 | **352** | 27 | 75 | 34 | 76 |
| **131** | 81 | 91 | 54 | 92 | **353** | 84 | 94 | 19 | 62 |
| **132** | 91 | 94 | 89 | 102 | **354** | 95 | 106 | 63 | 94 |
| **133** | 78 | 88 | 79 | 99 | **355** | 78 | - | 47 | - |
| **134** | 85 | 92 | 83 | 97 | **356** | 62 | - | 34 | - |
| **135** | 78 | 97 | 85 | 100 | **357** | 11 | - | 15 | - |
| **136** | 83 | 93 | 75 | 97 | **358** | 37 | - | -1 | - |
| **137** | 84 | 92 | 87 | 100 | **359** | 46 | - | 8 | - |
| **138** | 33 | 81 | 32 | 75 | **360** | 6 | - | -4 | - |
| **139** | 51 | 94 | 74 | 96 | **361** | 56 | - | 48 | - |
| **140** | 42 | 87 | 38 | 84 | **362** | 21 | 64 | 29 | 67 |
| **141** | 39 | 79 | 31 | 74 | **363** | 26 | 76 | 35 | 80 |
| **142** | 67 | 97 | 50 | 87 | **364** | 29 | 76 | 21 | 82 |
| **143** | 48 | 94 | 25 | 80 | **365** | 31 | 84 | 28 | 65 |
| **144** | 17 | 72 | 4 | 48 | **366** | 41 | 85 | 23 | 69 |
| **145** | 88 | - | 93 | - | **367** | 51 | 93 | 21 | 67 |
| **146** | 84 | - | 35 | - | **368** | 72 | 94 | 50 | 85 |
| **147** | 69 | - | 48 | - | **369** | 5 | 27 | 14 | 54 |
| **148** | 92 | - | 89 | - | **370** | 69 | 96 | 39 | 76 |
| **149** | 2 | - | 7 | - | **371** | 2 | 10 | 0 | 4 |
| **150** | 105 | - | 93 | - | **372** | 3 | 19 | 38 | 79 |
| **151** | 44 | - | 23 | - | **373** | - | 48 | - | 36 |
| **152** | 58 | - | 36 | - | **374** | - | 21 | - | 14 |
| **153** | 77 | - | 46 | - | **375** | - | 13 | - | 9 |
| **154** | 89 | - | 71 | - | **376** | - | 49 | - | 18 |
| **155** | 95 | - | 87 | - | **377** | - | 59 | - | 30 |
| **156** | 82 | - | 42 | - | **378** | - | 61 | - | 18 |
| **157** | 57 | - | 52 | - | **379** | - | 80 | - | 74 |
| **158** | 32 | - | 18 | - | **380** | - | 76 | - | 19 |
| **159** | 28 | - | 19 | - | **381** | - | 81 | - | 69 |
| **160** | 94 | - | 74 | - | **382** | - | 16 | - | 5 |
| **161** | 24 | - | 7 | - | **383** | - | 79 | - | 10 |
| **162** | 81 | - | 63 | - | **384** | - | 85 | - | 40 |
| **163** | 70 | - | 45 | - | **385** | - | 75 | - | 4 |
| **164** | 46 | - | 20 | - | **386** | - | 85 | - | 16 |
| **165** | 96 | - | 91 | - | **387** | - | 72 | - | 58 |
| **166** | 59 | - | 67 | - | **388** | - | 80 | - | 68 |
| **167** | 44 | - | 81 | - | **389** | - | 90 | - | 32 |
| **168** | 58 | - | 67 | - | **390** | - | 89 | - | 18 |
| **169** | 40 | - | 67 | - | **391** | - | 98 | - | 22 |
| **170** | 35 | - | 66 | - | **392** | - | 94 | - | 45 |
| **171** | 58 | - | 62 | - | **393** | - | 84 | - | 17 |
| **172** | 53 | - | 82 | - | **394** | - | 27 | - | -2 |
| **173** | 40 | - | 86 | - | **395** | - | 12 | - | 15 |
| **174** | 65 | - | 67 | - | **396** | - | 29 | - | 46 |
| **175** | 70 | - | 80 | - | **397** | - | 94 | - | 34 |
| **176** | 19 | - | 48 | - | **398** | - | 7 | - | 3 |
| **177** | 28 | - | 54 | - | **399** | - | 92 | - | 23 |
| **178** | 9 | - | -4 | - | **400** | - | 16 | - | -2 |
| **179** | 86 | - | 91 | - | **401** | - | 42 | - | 24 |
| **180** | 83 | - | 59 | - | **402** | - | 71 | - | 16 |
| **181** | 101 | - | 61 | - | **403** | - | 80 | - | 46 |
| **182** | 85 | - | 64 | - | **404** | - | 70 | - | 78 |
| **183** | 85 | - | 68 | - | **405** | - | 69 | - | 74 |
| **184** | 78 | - | 38 | - | **406** | - | 72 | - | 71 |
| **185** | 95 | - | 33 | - | **407** | - | 67 | - | 70 |
| **186** | 94 | - | 49 | - | **408** | - | 71 | - | 28 |
| **187** | 79 | - | 45 | - | **409** | - | 17 | - | 53 |
| **188** | 101 | - | 67 | - | **410** | - | 14 | - | 23 |
| **189** | 81 | - | 54 | - | **411** | - | 4 | - | 37 |
| **190** | 87 | - | 85 | - | **412** | - | 29 | - | 15 |
| **191** | 85 | - | 102 | - | **413** | - | 16 | - | 30 |
| **192** | 72 | - | 25 | - | **414** | - | 44 | - | 32 |
| **193** | 72 | - | 71 | - | **415** | - | 0 | - | 23 |
| **194** | 59 | - | 31 | - | **416** | - | 13 | - | 52 |
| **195** | 118 | - | 101 | - | **417** | - | 26 | - | 48 |
| **196** | 127 | - | 101 | - | **418** | - | 1 | - | 29 |
| **197** | 91 | - | 61 | - | **419** | - | 7 | - | 47 |
| **198** | 90 | - | 63 | - | **420** | - | 37 | - | 36 |
| **199** | 35 | - | 60 | - | **421** | - | 9 | - | 7 |
| **200** | 77 | - | 72 | - | **422** | - | 9 | - | 18 |
| **201** | 80 | - | 73 | - | **423** | - | 20 | - | 22 |
| **202** | 78 | - | 70 | - | **424** | - | 38 | - | 56 |
| **203** | 106 | - | 79 | - | **425** | - | 86 | - | 16 |
| **204** | 73 | - | 66 | - | **426** | - | 12 | - | 8 |
| **205** | 49 | - | 47 | - | **427** | - | 38 | - | 7 |
| **206** | 45 | - | 29 | - | **428** | - | 86 | - | 13 |
| **207** | 57 | - | 52 | - | **429** | - | 47 | - | 63 |
| **208** | 68 | - | 58 | - | **430** | - | 44 | - | 68 |
| **209** | 67 | - | 77 | - | **431** | - | 32 | - | 57 |
| **210** | 73 | - | 56 | - | **432** | - | 87 | - | 47 |
| **211** | 80 | - | 55 | - | **433** | - | 8 | - | 24 |
| **212** | 94 | - | 57 | - | **434** | - | 8 | - | 32 |
| **213** | 98 | - | 69 | - | **435** | - | 24 | - | 18 |
| **214** | 99 | - | 70 | - | **436** | - | 7 | - | 9 |
| **215** | 73 | - | 44 | - | **437** | - | 33 | - | 53 |
| **216** | 96 | - | 75 | - | **438** | - | 87 | - | 64 |
| **217** | 71 | - | 66 | - | **439** | - | 92 | - | 16 |
| **218** | 99 | - | 85 | - | **440** | - | 92 | - | 16 |
| **219** | 97 | - | 82 | - | **441** | 54 | - | 35 | - |
| **220** | 87 | - | 73 | - | | | | | |
| **221** | 67 | - | 27 | - | | | | | |
| **222** | 75 | - | 55 | - | | | | | |

The present invention has been described with reference to preferred exemplary embodiments herein, but it will be understood by those skilled in the art that the present invention may be variously changed and modified without departing from the spirit and field of the present invention, as described in the following scope of patent claims.

## Claims

1. A pyrido-pyrimidine derivative represented by following formula X, isomers thereof or pharmaceutically acceptable salts thereof, solvates thereof or hydrates thereof: 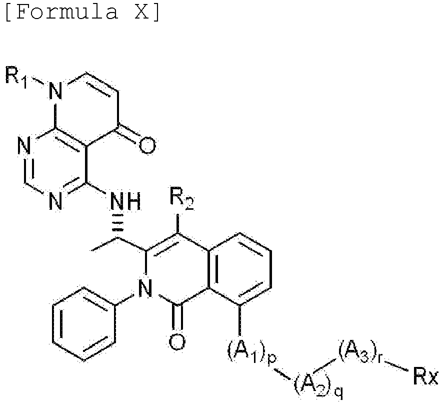 in above formula X,
R₁ is a hydrogen atom or C₂₋₁₂ heteroaryl group, in which at least one or more hydrogen atoms of the C₂₋₁₂ heteroaryl group may be each independently substituted with a halogen atom or -O-Ra (in which Ra is H, NH₂, OH, -CF₃ or C₁₋₁₀ alkyl group) ;
R₂ is a hydrogen atom, a halogen atom or C₃₋₁₀ cycloalkyl group; Rx is R₃ or Rcy;
R₃ is a hydrogen atom, a halogen atom, -CN, OH, -O-Ra (in which Ra is H, NH₂, OH, -CF₃ or C₁₋₁₀ alkyl group), -NH₂, -CF₃ or C₁₋₁₀ alkyl group;
Rcy is C₃₋₁₀ cycloalkyl, C₃₋₁₀ cycloalkenyl, C₂₋₁₀ heterocycloalkyl, C₂₋₁₀ heterocycloalkenyl, 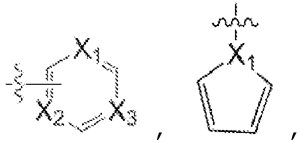 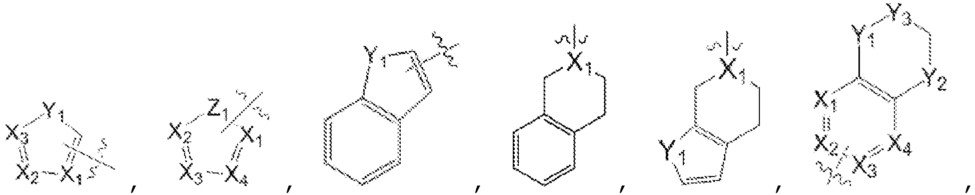 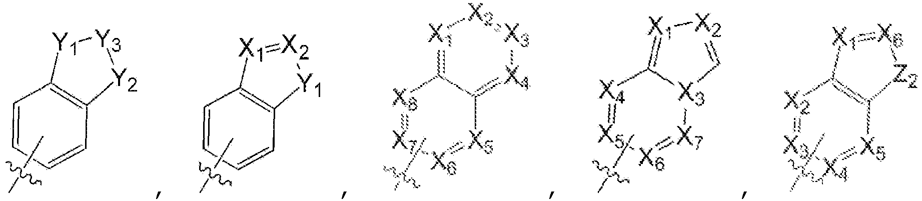 or 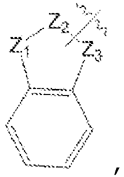
at least one or more hydrogen atoms of Rcy may be substituted or unsubstituted with R₄;
R₄ is a halogen atom, -CN, -OH, -NH₂, -NO₂, -CF₃, -O-Ra, - N(Ra) (Rb), -S(=O) (=N-Ra)-Rb, -S(Ra)₃, -S-Ra, -S(=O)₂-Ra, -C(=O)-Ra, -C(=O)-N(Ra) (Rb), -C(=O)-O-Ra, -N(Ra)-C(=O)-Rb, -N(Ra)-C(=O)-O-Rb, -N (Ra)-S (=O)₂-Rb, C₁₋₁₀ alkyl group or - (A₄)*ₛ*-(A₅)ₜ-R₅, which are same as or different from each other if R₄ substituted for Cy is 2 or more, and Ra and Rb of R₄ are each independently H, NH₂, OH, -CF₃ or C₁₋₁₀ alkyl group;
in R₄, at least one or more hydrogen atoms of C₁₋₁₀ alkyl group may be substituted or unsubstituted with -OH, C₁₋₁₀ alkoxy, -CN, - CF₃ or -N(Ra) (Rb), in which Ra and Rb are each independently H, NH₂, OH, -CF₃ or C₁₋₁₀ alkyl group;
A₁ and A₃ are each independently C₁₋₁₀ alkylene, C₂₋₁₀ alkenylene or C₂₋₁₀ alkynylene, in which at least one or more hydrogen atoms of A₁ and A₃ may be each independently substituted or unsubstituted with a halogen atom, -OH, -NO₂ or -CF₃;
A₂ is -N(Ra)-, -N(Ra)-C(=O)-, -N(Ra)-C(=O)-O-, -C(=O)-N(Ra)-, -N(Ra)-S(=O)-, -N(Ra)-S(=O)₂-, -C(Ra)=N-, -S(=O) (=N-Ra)-, -C(=O)-, -C(=O)-O-, -O-, -S-, -S(=O)-, -S(=O)₂- or -B(Ra)-, in which Ra of A₂ is H, NH₂, OH, -CF₃ or C₁₋₁₀ alkyl group;
A₄ is C₁₋₁₀ alkylene, C₂₋₁₀ alkenylene or C₂₋₁₀ alkynylene;
A₅ is -N(Ra)-, -N(Ra)-S(=O)₂-, -C(=O)-, -O-Ra-, -O-, or -S-, in which Ra of A₅ is H, NH₂, OH, -CF₃ or C₁₋₁₀ alkyl group;
R₅ is C₃₋₁₀ cycloalkyl, C₃₋₁₀ cycloalkenyl, C₂₋₁₀ heterocycloalkyl, C₂₋₁₀ heterocycloalkenyl, 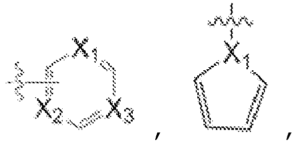 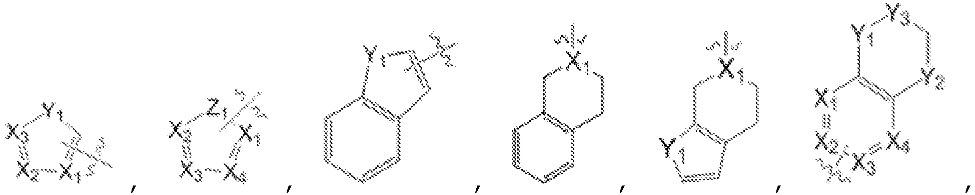 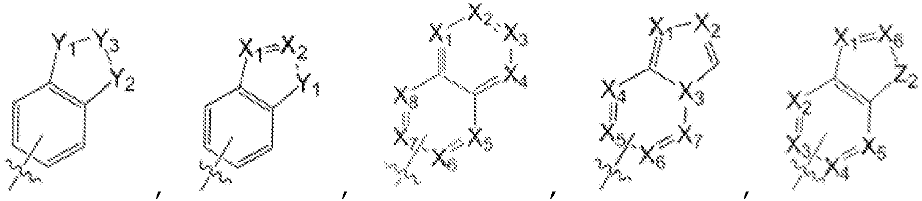 or 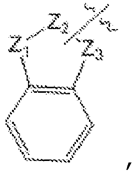 and at least one or more hydrogen atoms of R₅ may be substituted or unsubstituted with a halogen atom, NH₂, O-Ra (Ra is H, NH₂, OH, -CF₃ or C₁₋₁₀ alkyl group) or C₁₋₁₀ alkyl group;
in each of Rcy and R₅, X₁ to X₈ are each independently N or CH;
Y₁ and Y₂ are each independently NH, O or S, and Y₃ is C=O;
Z₁ to Z₃ are each independently NH or CH₂; and
p, q, r, s and t are each independently an integer of 0 or 1.

2. The pyrido-pyrimidine derivative, isomers thereof or pharmaceutically acceptable salts thereof, solvates thereof or hydrates thereof according to claim 1,
wherein in formula X, R₁ to R₃, A₁ to A₃, Rcy, Ra, Rb, p, q and r are the same as defined in claim 1,
wherein Rx of formula X is Rcy, p, r and q are 0, Rcy is 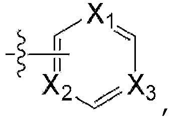 and X₁ to X₃ are CH, in which, if H is unsubstituted with R₄, R₂ is Cl as a halogen atom;
Rx of formula X is Rcy, p and r are 0, q is 1, A₂ is N(Ra)-, Ra is a hydrogen atom, Rcy is C₂₋₁₀heterocycloalkyl or 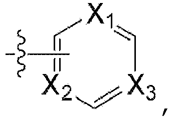 and if X₁ to X₃ are CH, R₂ is Cl as a halogen atom;
if Rx of formula X is R₃; p, q and r are 0; and R₃ is a hydrogen atom, R₂ is Cl as a halogen atom;
if Rx of formula X is R₃; p, q and r are 0; and R₃ is a hydrogen atom, R₁ is C₂₋₁₂ heteroaryl group, in which at least one or more hydrogen atoms of C₂₋₁₂ heteroaryl group may be each independently substituted with a halogen atom or -O-Ra (in which Ra is C₁₋₁₀ alkyl);
if Rx of formula X is R₃; p, q and r are 0; and R₃ is CN, R₂ is a hydrogen atom or Cl as a halogen atom;
if Rx of formula X is R₃; p, q and r are 0; and R₃ is a halogen atom, R₂ is Br as a halogen atom or C₃₋₁₀ cycloalkyl group;
if Rx of formula X is R₃; p and r are 0, q is 1; A₂ is N(Ra)-, S-, O- or S (=O)₂-; R₃ is C₁₋₁₀ alkyl; and Ra is a hydrogen atom or C₁₋₁₀ alkyl, R₂ is Cl as a halogen atom; and
if Rx of formula X is R₃; p, r and q are 1; A₁ is C₁₋₁₀ alkylene; A₂ is O-; and A₃ is C₁₋₁₀ alkylene, R₃ is OH.

3. The pyrido-pyrimidine derivative, isomers thereof or pharmaceutically acceptable salts thereof, solvates thereof or hydrates thereof according to claim 1, wherein the compound represented by above formula X is a compound represented by formula 1-1 below: 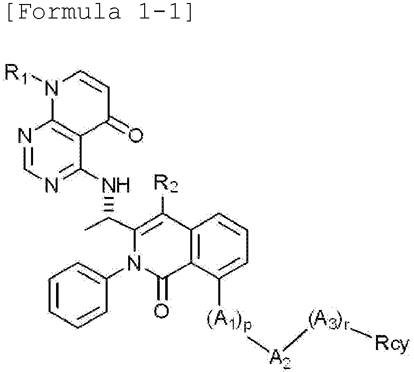 in above formula 1-1,
R₁ is H or C₂₋₁₂ heteroaryl group, in which at least one or more hydrogen atoms of the C₂₋₁₂ heteroaryl group may be each independently substituted with a halogen atom or -O-Ra (in which Ra is H, NH₂, OH, -CF₃ or C₁₋₁₀ alkyl group) ;
R₂ is H, a halogen atom or C₃₋₁₀ cycloalkyl group;
Rcy is C₃₋₁₀ cycloalkyl, C₃₋₁₀ cycloalkenyl, C₂₋₁₀ heterocycloalkyl, C₂₋₁₀ heterocycloalkenyl, 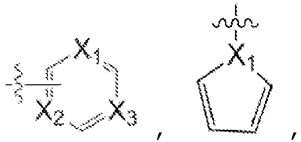 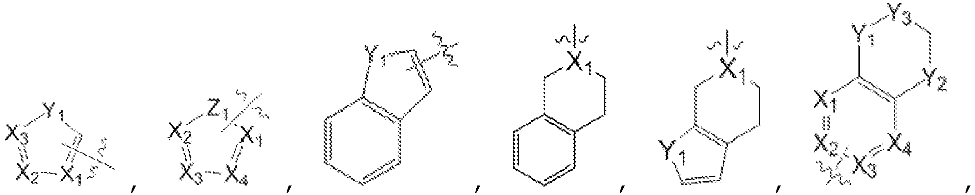 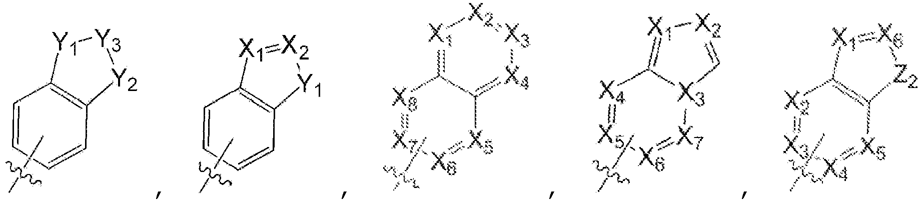 or 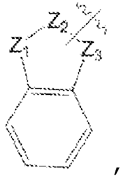
at least one or more hydrogen atoms of Rcy may be substituted or unsubstituted with R₄;
R₄ is a halogen atom, -CN, -OH, NO₂, -CF₃, -O-Ra, -N(Ra) (Rb), -S(=O)(=N-Ra)-Rb, -S(Ra)₃, -S-Ra, S(=O)₂-Ra, -C(=O)-Ra, -C(=O)-N(Ra)(Rb), -C(=O)-O-Ra, -N(Ra)-C(=O)-Ra, -N(Ra)-C(=O)-O-Ra, - N(Ra)-S(=O) ₂-Rb, C₁₋₁₀ alkyl group or - (A₄)*ₛ*-(A₅)ₜ-R₅, which are same as or different from each other if R₄ substituted for Cy is 2 or more, and Ra and Rb of R₄ are each independently H, NH₂, OH, -CF₃ or C₁₋₁₀ alkyl group;
in R₄, at least one or more hydrogen atoms of C₁₋₁₀ alkyl group may be substituted or unsubstituted with OH, C₁₋₁₀ alkoxy, CN, -CF₃ or -N(Ra)(Rb), in which Ra and Rb of R₄ are each independently H, NH₂, OH, -CF₃ or C₁₋₁₀ alkyl group;
A₁ and A₃ are each independently C₁₋₁₀ alkylene, C₂₋₁₀ alkenylene or C₂₋₁₀ alkynylene, in which at least one or more hydrogen atoms of A₁ and A₃ may be each independently substituted with a halogen atom, -OH, NO₂ or -CF₃;
A₂ is N(Ra)-, N(Ra)-C(=O)-, N(Ra)-C(=O)-O-, C(=O)-N(Ra)-, N(Ra)-S(=O)-, N(Ra)-S (=O)₂-, C(Ra)=N-, S(=O)(=N-Ra)-, C(=O)-, C(=O)-O-, O-, S-, S(=O)-, S(=O)₂- or B(Ra)-, in which Ra of A₂ is H, NH₂, OH, -CF₃ or C₁₋₁₀ alkyl group;
A₄ is C₁₋₁₀ alkylene, C₂₋₁₀ alkenylene or C₂₋₁₀ alkynylene;
A₅ is N(Ra)-, N(Ra)-S(=O)₂-, C(=O)-, O-Ra-, O-, or S-, in which Ra of A₅ is H, NH₂, OH, -CF₃ or C₁₋₁₀ alkyl group;
R₅ is C₃₋₁₀ cycloalkyl, C₃₋₁₀ cycloalkenyl, C₂₋₁₀ heterocycloalkyl, C₂₋₁₀ heterocycloalkenyl, 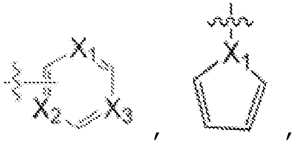 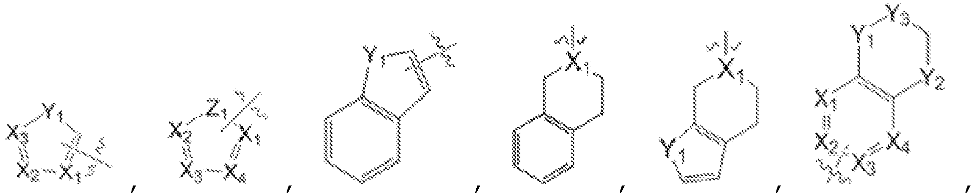 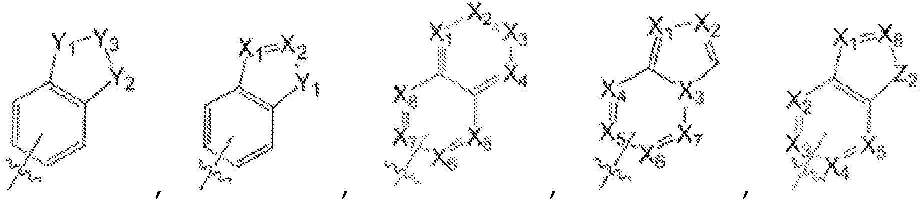 or 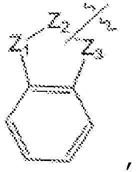 and at least one or more hydrogen atoms of R₅ may be substituted with a halogen atom, NH₂, OH or C₁₋₁₀ alkyl group;
in each of Rcy and R₅, X₁ to X₈ are each independently N or CH;
Y₁ and Y₂ are each independently NH, O or S, and Y₃ is C=O;
Z₁ to Z₃ are each independently NH or CH₂; and
p, r, s and t are each independently an integer of 0 or 1.

4. The pyrido-pyrimidine derivative, isomers thereof or pharmaceutically acceptable salts thereof, solvates thereof or hydrates thereof according to claim 1, wherein the compound represented by above formula X is a compound represented by formula 1-2 below: 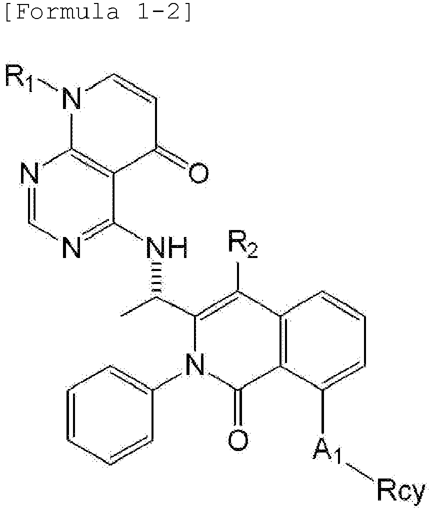 in above formula 1-2,
R₁ is H or C₂₋₁₂ heteroaryl group, in which at least one or more hydrogen atoms of the C₂₋₁₂ heteroaryl group may be each independently substituted with a halogen atom or -O-Ra (in which Ra is H, NH₂, OH, -CF₃ or C₁₋₁₀ alkyl group) ;
R₂ is H, a halogen atom or C₃₋₁₀ cycloalkyl group;
Rcy is C₃₋₁₀ cycloalkyl, C₃₋₁₀ cycloalkenyl, C₂₋₁₀ heterocycloalkyl, C₂₋₁₀ heterocycloalkenyl, 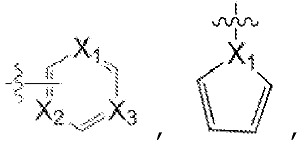 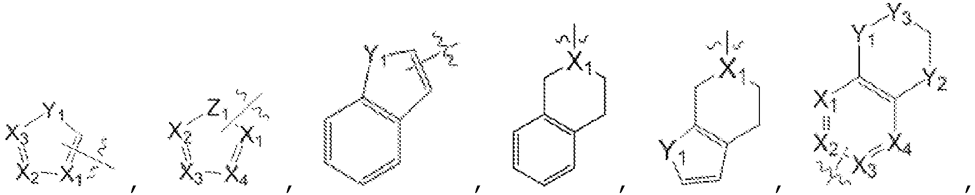 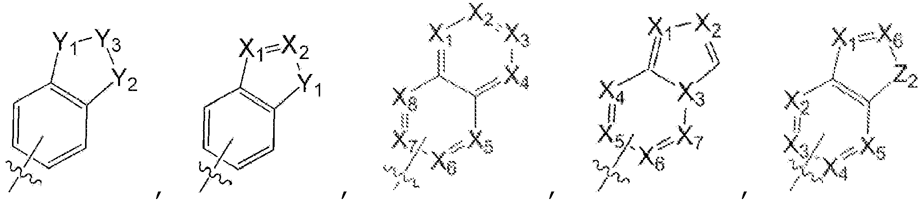 or 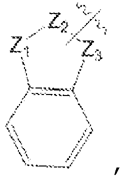
at least one or more hydrogen atoms of Rcy may be substituted or unsubstituted with R₄;
R₄ is a halogen atom, -CN, -OH, NO₂, -CF₃, O-Ra, -N(Ra)(Rb), S(=O)(=N-Ra) -Rb, S(Ra)₃, S-Ra, S(=O)₂-Ra, C(=O)-Ra, C(=O)-N(Ra)(Rb), C(=O)-O-Ra, N(Ra)-C(=O)-Ra, N(Ra)-C(=O)-O-Ra, N(Ra)-S(=O)₂-Rb, C₁₋₁₀ alkyl group or -(A₄)ₛ-(A₅)ₜ-R₅, which are same as or different from each other if R₄ substituted for Cy is 2 or more, and Ra and Rb of R₄ are each independently H, NH₂, OH, -CF₃ or C₁₋₁₀ alkyl group;
in R₄, at least one or more hydrogen atoms of C₁₋₁₀ alkyl group may be substituted with OH, C₁₋₁₀ alkoxy, CN, -CF₃ or -N(Ra)(Rb), in which Ra and Rb are each independently H, NH₂, OH, -CF₃ or C₁₋₁₀ alkyl group;
A₁ is C₂₋₁₀ alkenylene or C₂₋₁₀ alkynylene, in which at least one or more hydrogen atoms of A₁ may be each independently substituted or unsubstituted with a halogen atom, -OH, NO₂ or -CF₃;
A₄ is C₁₋₁₀ alkylene, C₂₋₁₀ alkenylene or C₂₋₁₀ alkynylene;
A₅ is N(Ra)-, N(Ra)-S(=O)₂-, C(=O)-, O-Ra-, O-, or S-, in which Ra of A₅ is H, NH₂, OH, -CF₃ or C₁₋₁₀ alkyl group;
R₅ is C₃₋₁₀ cycloalkyl, C₃₋₁₀ cycloalkenyl, C₂₋₁₀ heterocycloalkyl, C₂₋₁₀ heterocycloalkenyl, 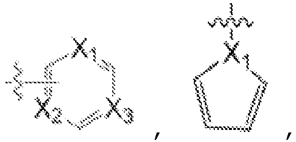 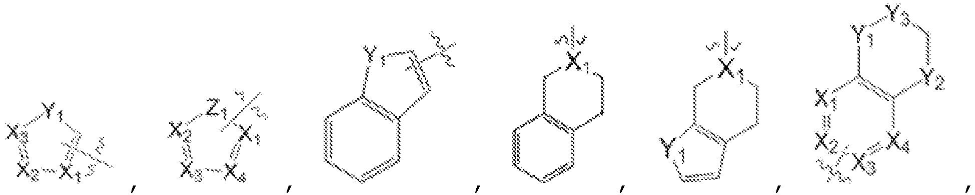 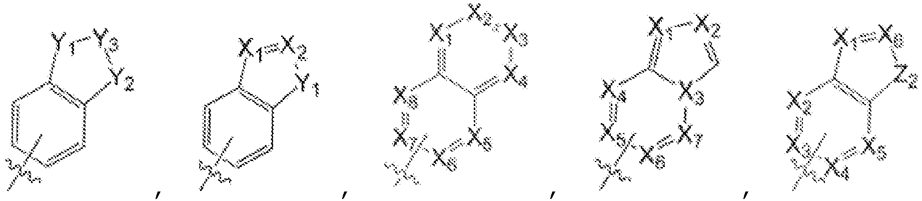 or 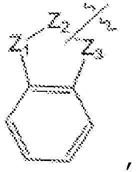 and at least one or more hydrogen atoms of R₅ may be substituted with a halogen atom, NH₂, OH or C₁₋₁₀ alkyl group;
in each of Rcy and R₅, X₁ to X₈ are each independently N or CH;
Y₁ and Y₂ are each independently NH, O or S, and Y₃ is C=O;
Z₁ to Z₃ are each independently NH or CH₂; and
s and t are each independently an integer of 0 or 1.

5. The pyrido-pyrimidine derivative, isomers thereof or pharmaceutically acceptable salts thereof, solvates thereof or hydrates thereof according to claim 1, wherein the compound represented by above formula X is a compound represented by formula 1-3 below: 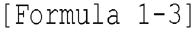 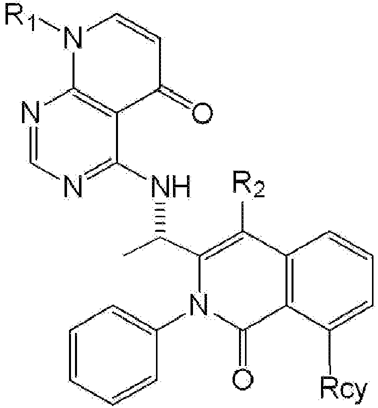 in above formula 1-3,
R₁ is H or C₂₋₁₂ heteroaryl group, in which at least one or more hydrogen atoms of the C₂₋₁₂ heteroaryl group may be each independently substituted with a halogen atom or -O-Ra (in which Ra is H, NH₂, OH, -CF₃ or C₁₋₁₀ alkyl group) ;
R₂ is H, a halogen atom or C₃₋₁₀ cycloalkyl group;
Rcy is C₃₋₁₀ cycloalkyl, C₃₋₁₀ cycloalkenyl, C₂₋₁₀ heterocycloalkyl, C₂₋₁₀ heterocycloalkenyl, 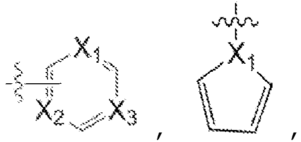 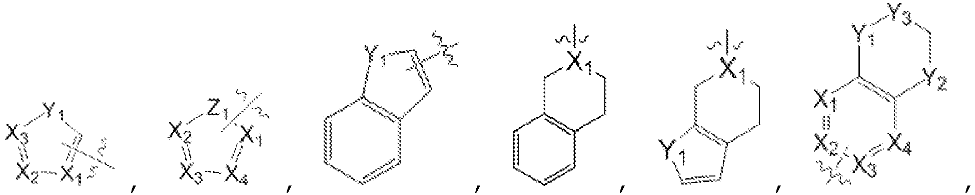 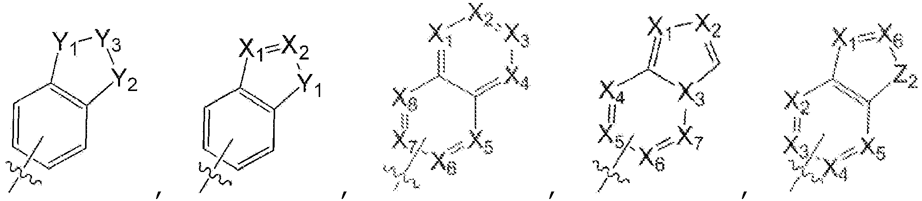 or 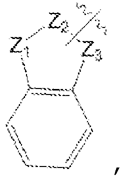
at least one or more hydrogen atoms of Rcy may be substituted or unsubstituted with R₄;
R₄ is a halogen atom, -CN, -OH, NO₂, -CF₃, O-Ra, -N(Ra)(Rb), S(=O)(=N-Ra) -Rb, S(Ra)₃, S-Ra, S(=O)₂-Ra, C(=O)-Ra, C(=O)-N(Ra)(Rb), C(=O)-O-Ra, N(Ra)-C(=O)-Ra, N(Ra)-C(=O)-O-Ra, N(Ra)-S(=O)₂-Rb, C₁₋₁₀ alkyl group or -(A₄)ₛ-(A₅)ₜ-R₅, which are same as or different from each other if R₄ substituted for Cy is 2 or more, and Ra and Rb are each independently H, NH₂, OH, -CF₃ or C₁₋₁₀ alkyl group;
in R₄, at least one or more hydrogen atoms of C₁₋₁₀ alkyl group may be substituted or unsubstituted with OH, C₁₋₁₀ alkoxy, CN, -CF₃ or -N(Ra)(Rb), in which Ra and Rb are each independently H, NH₂, OH, -CF₃ or C₁₋₁₀ alkyl group;
A₄ is C₁₋₁₀ alkylene, C₂₋₁₀ alkenylene or C₂₋₁₀ alkynylene;
A₅ is N(Ra)-, N(Ra)-S(=O)₂-, C(=O)-, O-Ra-, O-, or S-, in which Ra of A₅ is H, NH₂, OH, -CF₃ or C₁₋₁₀ alkyl group;
R₅ is C₃₋₁₀ cycloalkyl, C₃₋₁₀ cycloalkenyl, C₂₋₁₀ heterocycloalkyl, C₂₋₁₀ heterocycloalkenyl, 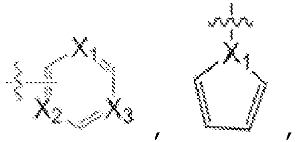 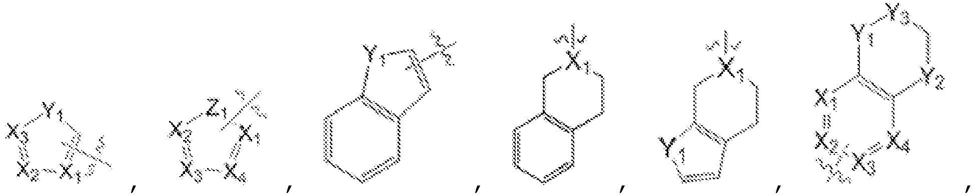 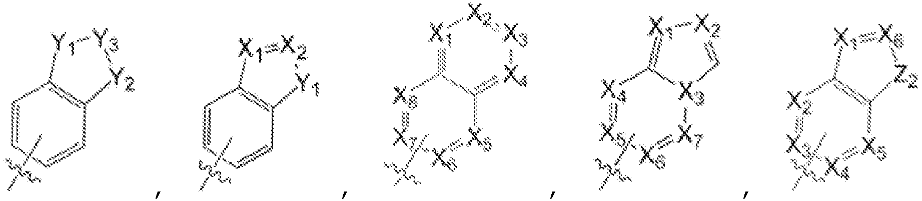 or 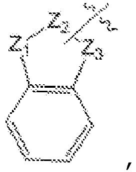 and at least one or more hydrogen atoms of R₅ may be substituted or unsubstituted with a halogen atom, NH₂, OH or C₁₋₁₀ alkyl group;
in each of Rcy and R₅, X₁ to X₈ are each independently N or CH;
Y₁ and Y₂ are each independently NH, O or S, and Y₃ is C=O;
Z₁ to Z₃ are each independently NH or CH₂; and
s and t are each independently an integer of 0 or 1.

6. The pyrido-pyrimidine derivative, isomers thereof or pharmaceutically acceptable salts thereof, solvates thereof or hydrates thereof according to claim 1, wherein the compound represented by above formula X is a compound represented by formula 1-4 below: 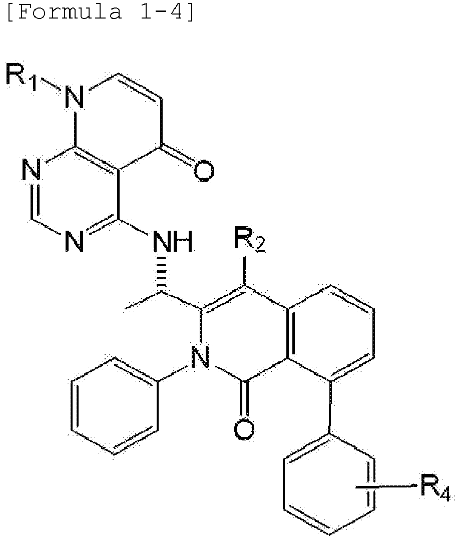 in above formula 1-4,
R₁ is H or C₂₋₁₂ heteroaryl group, in which at least one or more hydrogen atoms of the C₂₋₁₂ heteroaryl group may be each independently substituted with a halogen atom or -O-Ra (in which Ra is H, NH₂, OH, -CF₃ or C₁₋₁₀ alkyl group) ;
R₂ is H, a halogen atom or C₃₋₁₀ cycloalkyl group;
R₄ is a halogen atom, -CN, -OH, NO₂, -CF₃, O-Ra, -N(Ra)(Rb), S(=O)(=N-Ra) -Rb, S(Ra)₃, S-Ra, S(=O)₂-Ra, C(=O)-Ra, C(=O)-N(Ra)(Rb), C(=O)-O-Ra, N(Ra)-C(=O)-Ra, N(Ra)-C(=O)-O-Ra, N(Ra)-S(=O)₂-Rb, C₁₋₁₀ alkyl group or -(A₄)ₛ-(A₅)ₜ-R₅, which are same as or different from each other if R₄ is 2 or more, and Ra and Rb of R₄ are each independently H, NH₂, OH, -CF₃ or C₁₋₁₀ alkyl group;
in R₄, at least one or more hydrogen atoms of C₁₋₁₀ alkyl group may be substituted or unsubstituted with OH, C₁₋₁₀ alkoxy, CN, -CF₃ or -N(Ra)(Rb), in which Ra and Rb are each independently H, NH₂, OH, -CF₃ or C₁₋₁₀ alkyl group;
A₄ is C₁₋₁₀ alkylene, C₂₋₁₀ alkenylene or C₂₋₁₀ alkynylene;
A₅ is N(Ra)-, N(Ra)-S(=O)₂-, C(=O)-, O-Ra-, O-, or S-, in which Ra of A₅ is H, NH₂, OH, -CF₃ or C₁₋₁₀ alkyl group;
R₅ is C₃₋₁₀ cycloalkyl, C₃₋₁₀ cycloalkenyl, C₂₋₁₀ heterocycloalkyl, C₂₋₁₀ heterocycloalkenyl, 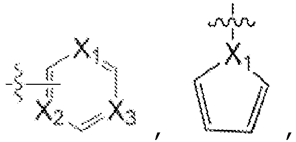 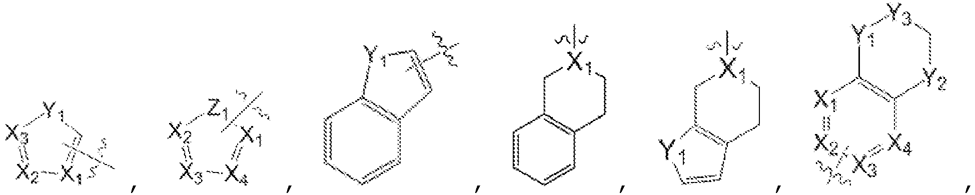 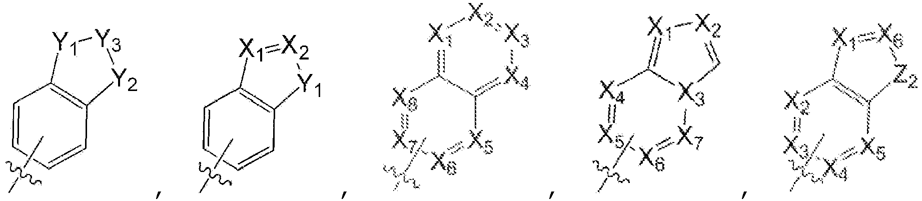 or 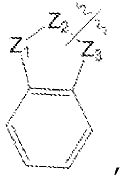 and at least one or more hydrogen atoms of R₅ may be substituted or unsubstituted with a halogen atom, NH₂, OH or C₁₋₁₀ alkyl group;
X₁ to X₈ are each independently N or CH;
Y₁ and Y₂ are each independently NH, O or S, and Y₃ is C=O;
Z₁ to Z₃ are each independently NH or CH₂; and
s and t are each independently an integer of 0 or 1.

7. The pyrido-pyrimidine derivative, isomers thereof or pharmaceutically acceptable salts thereof, solvates thereof or hydrates thereof according to claim 1, wherein the compound represented by above formula X is a compound represented by formula 2-1 below: 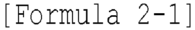 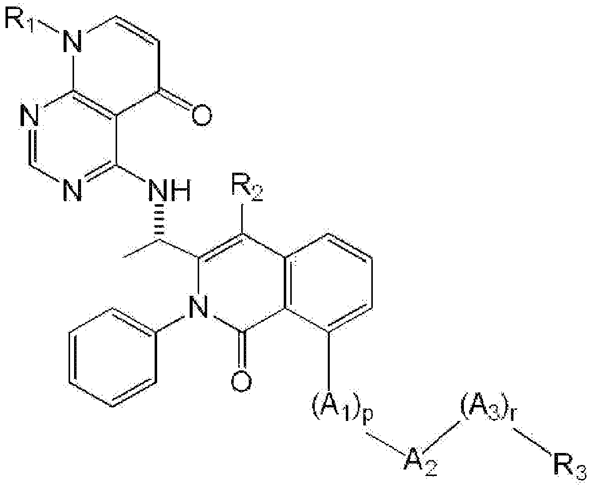 in above formula 2-1,
R₁ is H or C₂₋₁₂ heteroaryl group, in which at least one or more hydrogen atoms of the C₂₋₁₂ heteroaryl group may be each independently substituted with a halogen atom or -O-Ra (in which Ra is H, NH₂, OH, -CF₃ or C₁₋₁₀ alkyl group) ;
R₂ is H, a halogen atom or C₃₋₁₀ cycloalkyl group;
R₃ is H, a halogen atom, CN, OH, O-Ra (in which Ra is H, NH₂, OH, -CF₃ or C₁₋₁₀ alkyl group), NH₂, -CF₃ or C₁₋₁₀ alkyl group;
A₁ and A₃ are each independently C₁₋₁₀ alkylene, C₂₋₁₀ alkenylene or C₂₋₁₀ alkynylene, in which at least one or more hydrogen atoms of A₁ and A₃ may be each independently substituted or unsubstituted with a halogen atom, -OH, NO₂ or -CF₃;
A₂ is N(Ra)-C(=O)-, N(Ra)-C(=O)-O-, C(=O)-N(Ra)-, N(Ra)-S(=O)-, N(Ra)-S(=O)₂-, C(Ra)=N-, S(=O)(=N-Ra)-, C(=O)-, C(=O)-O-, S(=O)- or B(Ra)-, in which Ra of A₂ is H, NH₂, OH, -CF₃ or C₁₋₁₀ alkyl group; and
p and r are each independently 0 or 1.

8. The pyrido-pyrimidine derivative, isomers thereof or pharmaceutically acceptable salts thereof, solvates thereof or hydrates thereof according to claim 1, wherein the compound represented by above formula X is a compound represented by formula 2-2 below: 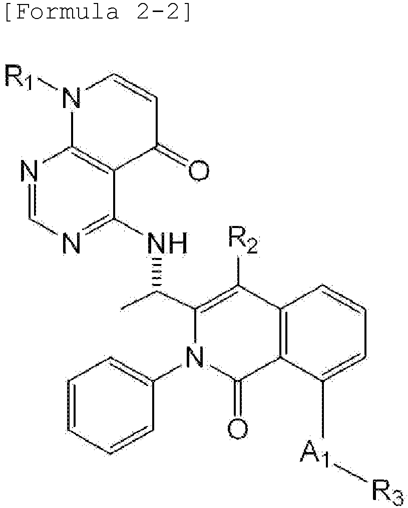 in above formula 2-2,
R₁ is H or C₂₋₁₂ heteroaryl group, in which at least one or more hydrogen atoms of the C₂₋₁₂ heteroaryl group may be each independently substituted with a halogen atom or -O-Ra (in which Ra is H, NH₂, OH, -CF₃ or C₁₋₁₀ alkyl group) ;
R₂ is H, a halogen atom or C₃₋₁₀ cycloalkyl group;
if A₁ is C₂₋₁₀ alkenylene or C₂₋₁₀ alkynylene, R₃ is H, a halogen atom, CN, OH, O-Ra (in which Ra is H, OH, -CF₃ or C₁₋₁₀ alkyl group), NH₂, -CF₃ or C₁₋₁₀ alkyl group; and
if A₁ is C₁₋₁₀ alkylene, R₃ is a halogen atom, CN, OH, O-Ra (in which Ra is H, OH, -CF₃ or C₁₋₁₀ alkyl group), NH₂ or -CF₃.

9. The pyrido-pyrimidine derivative, isomers thereof or pharmaceutically acceptable salts thereof, solvates thereof or hydrates thereof according to claim 1, wherein in formula X, Rcy and R₅ are each independently C₃₋₁₀ cycloalkylene, C₃₋₁₀ cycloalkenyl, C₂₋₁₀ heterocycloalkylene, C₂₋₁₀ heterocycloalkenyl, 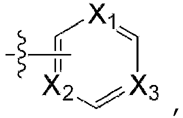 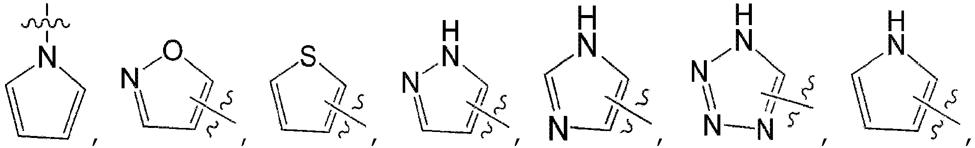 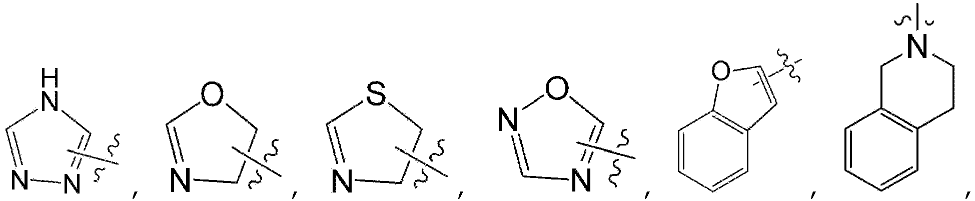 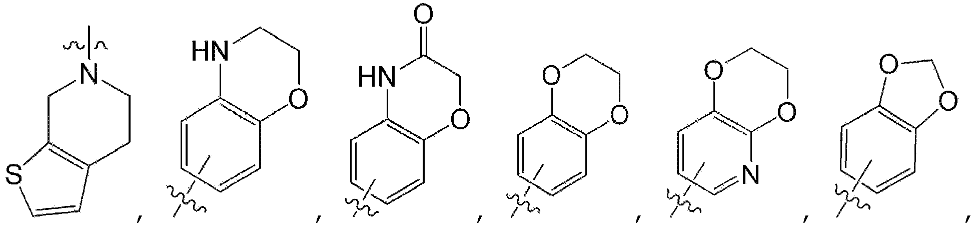 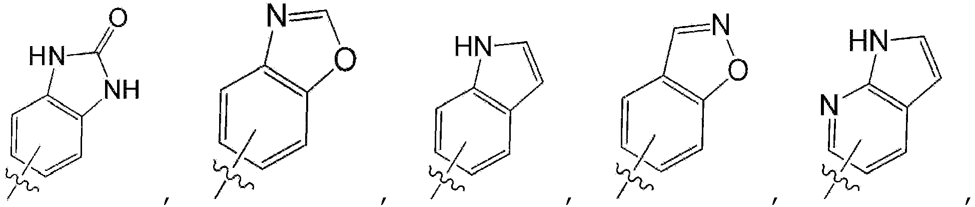 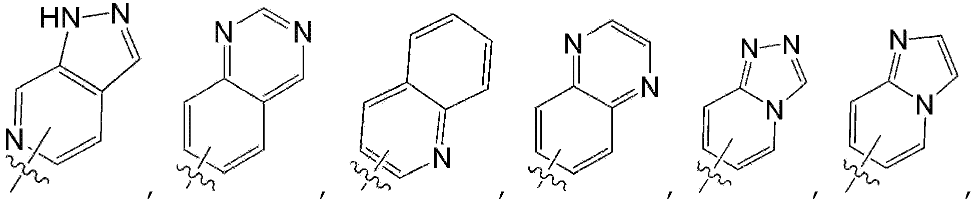 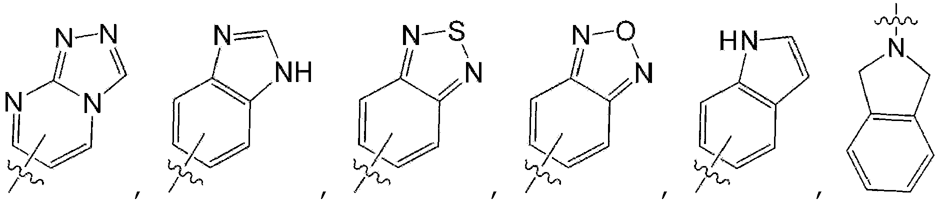 or

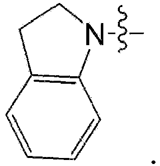

10. The pyrido-pyrimidine derivative represented by formula X, isomers thereof or pharmaceutically acceptable salts thereof, solvates thereof or hydrates thereof of claim 1, wherein the compound represented by above formula X is any one of compounds below:
[001] (S)-4-((1-(4-bromo-8-chloro-1-oxo-2-phenyl-1,2-dihydroisoquinolin-3-yl)ethyl)amino)pyrido[2,3-d]pyrimidin-5(8H)-one
[002] (S)-4-((1-(4-chloro-1-oxo-2-phenyl-8-(4,4,5,5-tetramethyl-1,3,2-dioxaboran-2-yl)-1,2-dihydroisoquinolin-3-yl)ethyl)amino)pyrido[2,3-d]pyrimidin-5(8H)-one
[003] (S)-4-((1-(8-(6-(1H-pyrrol-1-yl)pyridin-3-yl)-4-chloro-1-oxo-2-phenyl-1,2-dihydroisoquinolin-3-yl)ethyl)amino)pyrido[2,3-d]pyrimidin-5(8H)-one
[004] (S)-4-((1-(4-chloro-1-oxo-2-phenyl-8-(pyridin-3-yl)-1,2-dihydroisoquinolin-3-yl)ethyl)amino)pyrido[2,3-d]pyrimidin-5(8H)-one
[005] (S)-4-((1-(4-chloro-8-(1-methyl-1H-pyrazol-4-yl)-1-oxo-2-phenyl-1,2-dihydroisoquinolin-3-yl)ethyl)amino)pyrido[2,3-d]pyrimidin-5(8H)-one
[006] (S)-4-((1-(8-(benzofuran-2-yl)-4-chloro-1-oxo-2-phenyl-1,2-dihydroisoquinolin-3-yl)ethyl)amino)pyrido[2,3-d]pyrimidin-5(8H)-one
[007] (S)-4'-chloro-3'-(1-((5-oxo-5,8-dihydropyrido[2,3-d]pyrimidin-4-yl)amino)ethyl-2'-phenyl-3,4-dihydro-1H-(2,8'-biisoquinolin)-1'(2'H)-one
[008] 4-(((1S)-1-(4-chloro-1-oxo-2-phenyl-8-(3a,4,7,7a-tetrahydro-1-H-isoindol-2(3H-yl)-1,2-dihydroisoquinolin-3-yl)ethyl)amino)pyrido[2,3-d]pyrimidin-5(8H)-one
[009] (S)-4-((1-(4-chloro-1-oxo-2-phenyl-8-(4-(pyridin-4-yl)piperazin-1-yl)-1,2-dihydroisoquinolin-3-yl)ethyl)amino)pyrido[2,3-d]pyrimidin-5(8H)-one
[010] (S)-4-((1-(8-([1,4'-bipiperidin]-1'-yl)-4-chloro-1-oxo-2-phenyl-1,2-dihydroisoquinolin-3-yl)ethyl)amino)pyrido[2,3-d]pyrimidin-5(8H)-one
[011] (S)-4-((1-(4-chloro-8-(4-(1-methylpiperidin-4-yl)piperazin-1-yl)-1-oxo-2-phenyl-1,2-dihydroisoquinolin-3-yl)ethyl)amino)pyrido[2,3-d]pyrimidin-5(8H)-one
[012] (S)-4-((1-(1-oxo-2-phenyl-8-(pyridin-3-yl)-1,2-dihydroisoquinolin-3-yl)ethyl)amino)pyrido[2,3-d]pyrimidin-5(8H)-one
[013] (S)-4-((1-(8-(6-methylpyridin-3-yl)-1-oxo-2-phenyl-1,2-dihydroisoquinolin-3-yl)ethyl)amino)pyrido[2,3-d]pyrimidin-5(8H)-one
[014] (S)-4-((1-(8-(4-fluorophenyl)-1-oxo-2-phenyl-1,2-dihydroisoquinolin-3-yl)ethyl)amino)pyrido[2,3-d]pyrimidin-5(8H)-one
[015] (S)-4-((1-(8-(1-methyl-1H-pyrazol-4-yl)-1-oxo-2-phenyl-1,2-dihydroisoquinolin-3-yl)ethyl)amino)pyrido[2,3-d]pyrimidin-5(8H)-one
[016] (S)-4-((1-(4-bromo-8-(4-(dimethylamino)phenyl)-1-oxo-2-phenyl-1,2-dihydroisoquinolin-3-yl)ethyl)amino)pyrido[2,3-d]pyrimidin-5(8H)-one
[017] (S)-4-((1-(4-bromo-8-(6-methylpyridin-3-yl)-1-oxo-2-phenyl-1,2-dihydroisoquinolin-3-yl)ethyl)amino)pyrido[2,3-d]pyrimidin-5(8H)-one
[018] (S)-4-((1-(4-bromo-8-(4-fluorophenyl)-1-oxo-2-phenyl-1,2-dihydroisoquinolin-3-yl)ethyl)amino)pyrido[2,3-d]pyrimidin-5(8H)-one
[019] (S)-4-((1-(4-bromo-8-(1-methyl-1H-pyrazol-4-yl)-1-oxo-2-phenyl-1,2-dihydroisoquinolin-3-yl)ethyl)amino)pyrido[2,3-d]pyrimidin-5(8H)-one
[020] (S)-4-((1-(8-(4-(dimethylamino)phenyl)-1-oxo-2-phenyl-1,2-dihydroisoquinolin-3-yl)ethyl)amino)pyrido[2,3-d]pyrimidin-5(8H)-one
[021] (S)-4-((1-(4-chloro-1-oxo-8-(2-oxo-2,3-dihydro-1H-benzo[d]imidazol-5-yl)-2-phenyl-1,2-dihydroisoquinolin-3-yl)ethyl)amino)pyrido[2,3-d]pyrimidin-5(8H)-one
[022] (S)-4-((1-(4-chloro-8-(2-methylbenzo[d]oxazol-6-yl)-1-oxo-2-phenyl-1,2-dihydroisoquinolin-3-yl)ethyl)amino)pyrido[2,3-d]pyrimidin-5(8H)-one
[023] (S)-4-(1-oxo-3-(1-((5-oxo-5,8-dihydropyrido[2,3-d]pyrimidin-4-yl)amino)ethyl)-2-phenyl-1,2-dihydroisoquinolin-8-yl)benzonitrile
[024] (S)-4-((1-(8-(benzo[d]isoxazol-6-yl)-4-chloro-1-oxo-2-phenyl-1,2-dihydroisoquinolin-3-yl)ethyl)amino)pyrido[2,3-d]pyrimidin-5(8H)-one
[025] (S)-4-((1-(4-chloro-1-oxo-2-phenyl-8-(1H-pyrrolo[2,3-b]pyridin-5-yl)-1,2-dihydroisoquinolin-3-yl)ethyl)amino)pyrido[2,3-d]pyrimidin-5(8H)-one
[026] (S)-4-((1-(4-chloro-8-(2-(2-hydroxypropan-2-yl)pyrimidin-5-yl)-1-oxo-2-phenyl-1,2-dihydroisoquinolin-3-yl)ethyl)amino)pyrido[2,3-d]pyrimidin-5(8H)-one
[027] (S)-4-((1-(4-chloro-1-oxo-2-phenyl-8-(1H-pyrazolo[4,3-c]pyridin-6-yl)-1,2-dihydroisoquinolin-3-yl)ethyl)amino)pyrido[2,3-d]pyrimidin-5(8H)-one
[028] (S)-4-((1-(4-chloro-8-(5,6-dimethoxypyrazin-2-yl)-1-oxo-2-phenyl-1,2-dihydroisoquinolin-3-yl)ethyl)amino)pyrido[2,3-d]pyrimidin-5(8H)-one
[029] (S)-4-((1-(4-chloro-1-oxo-2-phenyl-8-(4-(triazol-2-yl)phenyl)-1,2-dihydroisoquinolin-3-yl)ethyl)amino)pyrido[2,3-d]pyrimidin-5(8H)-one
[030] (S)-4-((1-(4-chloro-8-(5-methoxypyrimidin-2-yl)-1-oxo-2-phenyl-1,2-dihydroisoquinolin-3-yl)ethyl)amino)pyrido[2,3-d]pyrimidin-5(8H)-one
[031] (S)-6-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)nicotinonitrile
[032] (S)-4-((1-(4-chloro-1-oxo-2-phenyl-8-(quinazolin-6-yl)-1,2-dihydroisoquinolin-3-yl)ethyl)amino)pyrido[2,3-d]pyrimidin-5(8H)-one
[033] (S)-4-((1-(4-chloro-8-(4-(isoxazol-3-yl)phenyl)-1-oxo-2-phenyl-1,2-dihydroisoquinolin-3-yl)ethyl)amino)pyrido[2,3-d]pyrimidin-5(8H)-one
[034] (S)-4-((1-(8-(6-(tert-butyl)pyridin-3-yl)-4-chloro-1-oxo-2-phenyl-1,2-dihydroisoquinolin-3-yl)ethyl)amino)pyrido[2,3-d]pyrimidin-5(8H)-one
[035] (S)-4-((1-(4-chloro-1-oxo-2-phenyl-8-(pyrimidin-2-yl)-1,2-dihydroisoquinolin-3-yl)ethyl)amino)pyrido[2,3-d]pyrimidin-5(8H)-one
[036] (S)-4-((1-(4-chloro-8-(3-fluoro-4,5-dihydroxyphenyl)-1-oxo-2-phenyl-1,2-dihydroisoquinolin-3-yl)ethyl)amino)pyrido[2,3-d]pyrimidin-5(8H)-one
[037] (S)-4-((1-(8-([1,2,4]tetrazolo[4,3-a]pyridin-6-yl)-4-chloro-1-oxo-2-phenyl -1,2-dihydroisoquinolin-3-yl)ethyl)amino)pyrido[2,3-d]pyrimidin-5(8H)-one
[038] (S)-4-((1-(4-chloro-1-oxo-2-phenyl-1,2-dihydroisoquinolin-3-yl)ethyl)amino)pyrido[2,3-d]pyrimidin-5(8H)-one
[039] (S)-2-(S-(4-chloro-1-oxo-3-(1-((5-oxo-5,8-dihyropyrido[2,3-d]pyrimidin-4-yl)amino)ethyl)-2-phenyl-1,2-dihydroisoquinolin-8-yl)pyrimidin-2-yl)acetonitrile
[040] (S)-4-((1-(8-([1,2,4]triazolo[4,3-a]pyrimidin-6-yl)-4-chloro-1-oxo-2-phenyl-1,2-dihydroisoquinolin-3-yl)ethyl)amino)pyrido[2,3-d]pyrimidin-5(8H)-one
[041] (S)-4-((1-(4-chloro-8-(5-methoxypyridin-2-yl)-1-oxo-2-phenyl-1,2-dihydroisoquinolin-3-yl)ethyl)amino)pyrido[2,3-d]pyrimidin-5(8H)-one
[042] (S)-4-((1-(8-([1,2,4]triazolo[1,5-a]pyridin-6-yl)-4-chloro-1-oxo-2-phenyl-1,2-dihydroisoquinolin-3-yl)ethyl)amino)pyrido[2,3-d]pyrimidin-5(8H)-one
[043] (S)-4-((1-(4-chloro-1-oxo-2-phenyl-1,2-dihydroisoquinolin-3-yl)ethyl)amino)-8-(5-methoxypyrimidin-2-yl)pyrido[2,3-d]pyrimidin-5(8H)-one
[044] (S)-5-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)pyrimidine-2-carbonitrile
[045] (S)-4-((1-(4-chloro-8-(6-methoxypyrimidin-4-yl)-1-oxo-2-phenyl-1,2-dihydroisoquinolin-3-yl)ethyl)amino)pyrido[2,3-d]pyrimidin-5(8H)-one
[046] (S)-4-((1-(4-chloro-8-(6-cyclopropylpyridin-3-yl)-1-oxo-2-phenyl-1,2-dihydroisoquinolin-3-yl)ethyl)amino)pyrido[2,3-d]pyrimidin-5(8H)-one
[047] (S)-S-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)picolinic acid
[048] (S)-4-((1-(4-chloro-1-oxo-2-phenyl-8-(quinoxalin-2-yl)-1,2-dihydroisoquinolin-3-yl)ethyl)amino)pyrido[2,3-d]pyrimidin-5(8H)-one
[049] (S)-4-((1-(4-chloro-8-(2,6-dimethoxypyrimidin-4-yl)-1-oxo-2-phenyl-1,2-dihydroisoquinolin-3-yl)ethyl)amino)pyrido[2,3-d]pyrimidin-5(8H)-one
[050] (S)-4-((1-(4-chloro-8-(6-hydroxypyrimidin-4-yl)-1-oxo-2-phenyl-1,2-dihydroisoquinolin-3-yl)ethyl)amino)pyrido[2,3-d]pyrimidin-5(8H)-one
[051] (S)-4-((1-(4-chloro-1-oxo-2-phenyl-8-(5-(trifluoromethyl)pyrazin-2-yl)-1,2-dihydroquinolin-3-yl)ethyl)amino)pyrido[2,3-d]pyrimidin-5(8H)-one
[052] (S)-4-((1-(4-chloro-1-oxo-2-phenyl-8-(5-(trifluoromethyl)pyrimidin-2-yl)-1,2-dihydroisoquinolin-3-yl)ethyl)amino)pyrido[2,3-d]pyrimidin-5(8H)-one
[053] (S)-4-((1-(4-chloro-8-(5-fluoropyrimidin-2-yl)-1-oxo-2-phenyl-1,2-dihydroisoquinolin-3-yl)ethyl)amino)pyrido[2,3-d]pyrimidin-5(8H)-one
[054] (S)-4-((1-(4-chloro-8-(2-cyclobutylpyrimidin-5-yl)-1-oxo-2-phenyl-1,2-dihydroisoquinolin-3-yl)ethyl)amino)pyrido[2,3-d]pyrimidin-5(8H)-one
[055] (S)-4-((1-(4-chloro-8-(2-(methoxymethyl)pyrimidin-5-yl)-1-oxo-2-phenyl-1,2-dihydroisoquinolin-3-yl)ethyl)amino)pyrido[2,3-d]pyrimidin-5(8H)-one
[056] (S)-4-((1-(8-(2-aminopyrimidin-5-yl)-4-chloro-1-oxo-2-phenyl-1,2-dihydroisoquinolin-3-yl)ethyl)amino)pyrido[2,3-d]pyrimidin-5(8H)-one
[057] 4-(((1S)-1-(4-chloro-8-(4-(1-hydroxyethyl)phenyl)-1-oxo-2-phenyl-1,2-dihydroisoquinolin-3-yl)ethyl)amino)pyrido[2,3-d]pyrimidin-5(8H)-one
[058] (S)-4-((1-(4-chloro-8-(6-hydroxy-5-methylpyridin-3-yl)-1-oxo-2-phenyl-1,2-dihydroisoquinolin-3-yl)ethyl)amino)pyrido[2,3-d]pyrimidin-5(8H)-one
[059] (S)-4-((1-(4-chloro-8-(4-cyclopentylpyrimidin-5-yl)-1-oxo-2-phenyl-1,2-dihydroisoquinolin-3-yl)ethyl)amino)pyrido[2,3-d]pyrimidin-5(8H)-one
[060] (S)-4-((1-(8-(benzo[d][1,3]dioxol-5-yl)-4-chloro-1-oxo-2-phenyl-1,2-dihydroisoquinolin-3-yl)ethyl)amino)pyrido[2,3-d]pyrimidin-5(8H)-one
[061] (S)-4-((1-(4-chloro-8-(6-methoxy-5-nitropyrimidin-3-yl)-1-oxo-2-phenyl-1,2-dihydroisoquinolin-3-yl)ethyl)amino)pyrido[2,3-d]pyrimidin-5(8H)-one
[062] (S)-4-((1-(4-chloro-1-oxo-2,8-diphenyl-1,2-dihydroisoquinolin-3-yl)ethyl)amino)pyrido[2,3-d]pyrimidin-5(8H)-one
[063] (S)-4-((1-(4-chloro-8-(4-fluorophenyl)-1-oxo-2-phenyl-1,2-dihydroisoquinolin-3-yl)ethyl)amino)pyrido[2,3-d]pyrimidin-5(8H)-one
[064] (S)-4-((1-(4-chloro-8-(4-methoxyphenyl)-1-oxo-2-phenyl-1,2-dihydroisoquinolin-3-yl)ethyl)amino)pyrido[2,3-d]pyrimidin-5(8H)-one
[065] (S)-4-((1-(8-chloro-4-cyclopropyl-1-oxo-2-phenyl-1,2-dihydroisoquinolin-3-yl)ethyl)amino)pyrido[2,3-d]pyrimidin-5(8H)-one
[066] (S)-4-((1-(4-chloro-8-cyclopropyl-1-oxo-2-phenyl-1,2-dihydroisoquinolin-3-yl)ethyl)amino)pyrido[2,3-d]pyrimidin-5(8H)-one
[067] (S)-4-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-3-methylbenzonitrile
[068] (S)-4-((1-(4-chloro-1-oxo-2-phenyl-8-(4-(trifluoromethyl)phenyl)-1,2-dihydroisoquinolin-3-yl)ethyl)amino)pyrido[2,3-d]pyrimidin-5(8H)-one
[069] (S)-1,4'-dichloro-3'-(1-((5-oxo-5,8-dihydropyrido[2,3-d]pyrimidin-4-yl)amino)ethyl)-2'-phenyl-(4,8'-biisoquinolin)-1'(2'H)-one
[070] (S,E)-4-((1-(4-chloro-1-oxo-2-phenyl-8-styryl-1,2-dihydroisoquinolin-3-yl)ethyl)amino)pyrido[2,3-d]pyrimidin-5(8H)-one
[071] (S)-4-((1-(4-chloro-8-(3,4-dichlorophenyl)-1-oxo-2-phenol-1,2-dihydroisoquinolin-3-yl)ethyl)amino)pyrido[2,3-d]pyrimidin-5(8H)-one
[072] (S)-4-((1-(4-chloro-8-(4-(dimethylamino)phenyl)-1-oxo-2-phenyl-1,2-dihydroisoquinolin-3-yl)ethyl)amino)pyrido[2,3-d]pyrimidin-5(8H)-one
[073] (S)-4-((1-(4-chloro-8-(1H-indol-5-yl)-1-oxo-2-phenyl-1,2-dihydroisoquinolin-3-yl)ethyl)amino)pyrido[2,3-d]pyrimidin-5(8H)-one
[074] (S)-4-((1-(4-chloro-8-(3-methyl-1H-indol-5-yl)-1-oxo-2-phenyl-1,2-dihydroisoquinolin-3-yl)ethyl)amino)pyrido[2,3-d]pyrimidin-5(8H)-one
[075] (S)-4-((1-(4-chloro-8-(4-morpholinophenyl)-1-oxo-2-phenyl-1,2-dihydroisoquinolin-3-yl)ethyl)amino)pyrido[2,3-d]pyrimidin-5(8H)-one
[076] (S)-4-((1-(4-chloro-8-(4-isopropylpiperidin-1-yl)-1-oxo-2-phenyl-1,2-dihydroisoquinolin-3-yl)ethyl)amino)pyrido[2,3-d]pyrimidin-5(8H)-one
[077] tert-butyl (S)-(1-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)piperidin-4-yl)carbamate
[078] (S)-4-((1-(4-chloro-8-(4-(2-hydroxyphenyl)piperazin-1-yl)-1-oxo-2-phenyl-1,2-dihydroisoquinolin-3-yl)ethyl)amino)pyrido[2,3-d]pyrimidin-5(8H)-one
[079] (S)-3-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)benzaldehyde
[080] (S)-4-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)benzamide
[081] (S)-4-(1-oxo-3-(1-((5-oxo-5,8-dihydropyrido[2,3-d]pyrimidin-4-yl)amino)ethyl)-2-phenyl-1,2-dihydroisoquinolin-8-yl)benzamide
[082] (S)-4-((1-(4-chloro-8-(1,5-dimethyl-1H-pyrazol-4-yl)-1-oxo-2-phenyl-1,2-dihydroisoquinolin-3-yl)ethyl)amino)pyrido[2,3-d]pyrimidin-5(8H)-one
[083] (S)-4-((1-(4-chloro-8-(1-(2-(dimethylamino)ethyl)-1H-pyrazol-4-yl)-1-oxo-2-phenyl-1,2-dihydroisoquinolin-3-yl)ethyl)amino)pyrido[2,3-d]pyrimidin-5(8H)-one
[084] (S)-4-((1-(4-chloro-8-(4-chlorophenyl)-1-oxo-2-phenyl-1,2-dihydroisoquinolin-3-yl)ethyl)amino)pyrido[2,3-d]pyrimidin-5(8H)-one
[085] (S)-4-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)benzaldehyde
[086] (S)-4-((1-(4-chloro-8-(6-(dimethylamino)pyridin-3-yl)-1-oxo-2-phenyl-1,2-dihydroisoquinolin-3-yl)ethyl)amino)pyrido[2,3-d]pyrimidin-5(8H)-one
[087] (S)-4-((1-(4-chloro-1-oxo-2-phenyl-8-(4-(pyrrolidin-1-yl)phenyl)-1,2-dihydroisoquinolin-3-yl)ethyl)amino)pyrido[2,3-d]pyrimidin-5(8H)-one
[088] (S)-4-((1-(4-chloro-1-oxo-2-phenyl-8-(4-(piperidin-1-yl)phenyl)-1,2-dihydroisoquinolin-3-yl)ethyl)amino)pyrido[2,3-d]pyrimidin-5(8H)-one
[089] (S)-4-((1-(4-chloro-8-(1H-indol-4-yl)-1-oxo-2-phenyl-1,2-dihydroisoquinolin-3-yl)ethyl)amino)pyrido[2,3-d]pyrimidin-5(8H)-one
[090] (S)-4-((1-(8-(4-(1-methylpiperidin-4-yl)phenyl)-1-oxo-2-phenyl-1,2-dihydroisoquinolin-3-yl)ethyl)amino)pyrido[2,3-d]pyrimidin-5(8H)-one
[091] (S)-2-(3-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)phenyl)acetonitrile
[092] (S)-4-((1-(8-(4-(5-amino-1,3,4-thiadiazol-2-yl)phenyl)-4-chloro-1-oxo-2-phenyl-1,2-dihydroisoquinolin-3-yl)ethyl)amino)pyrido[2,3-d]pyrimidin-5(8H)-one
[093] (S)-4-((1-(4-chloro-1-oxo-2-phenyl-8-(4-(thiophen-2-yl)phenyl)-1,2-dihydroisoquinolin-3-yl)ethyl)amino)pyrido[2,3-d]pyrimidin-5(8H)-one
[094] (S)-4-((1-(8-(4-aminophenyl)-4-chloro-1-oxo-2-phenyl-1,2-dihydroisoquinolin-3-yl)ethyl)amino)pyrido[2,3-d]pyrimidin-5(8H)-one
[095] (S)-4-((1-(4-chloro-8-(4-mercaptophenyl)-1-oxo-2-phenyl-1,2-dihydroisoquinolin-3-yl)ethyl)amino)pyrido[2,3-d]pyrimidin-5(8H)-one
[096] (S)-4-((1-(4-chloro-8-(1-cyclopropyl-1H-pyrazol-4-yl)-1-oxo-2-phenyl-1,2-dihydroisoquinolin-3-yl)ethyl)amino)pyrido[2,3-d]pyrimidin-5(8H)-one
[097] (S)-4-((1-(4-chloro-8-(3-chlorophenyl)-1-oxo-2-phenyl-1,2-dihydroisoquinolin-3-yl)ethyl)amino)pyrido[2,3-d]pyrimidin-5(8H)-one
[098] (S)-4-((1-(4-chloro-8-(5-methylfuran-2-yl)-1-oxo-2-phenyl-1,2-dihydroisoquinolin-3-yl)ethyl)amino)pyrido[2,3-d]pyrimidin-5(8H)-one
[099] (S)-4-((1-(4-chloro-8-(4-chloro-3-methoxyphenyl)-1-oxo-2-phenyl-1,2-dihydroisoquinolin-3-yl)ethyl)amino)pyrido[2,3-d]pyrimidin-5(8H)-one
[100] (S)-4-((1-(8-(4-chloro-3-methoxyphenyl)-1-oxo-2-phenyl-1,2-dihydroisoquinolin-3-yl)ethyl)amino)pyrido[2,3-d]pyrimidin-5(8H)-one
[101] (S)-4-((1-(4-chloro-8-(6-isopropoxypyridin-3-yl)-1-oxo-2-phenyl-1,2-dihydroisoquinolin-3-yl)ethyl)amino)pyrido[2,3-d]pyrimidin-5(8H)-one
[102] (S)-4-((1-(4-chloro-1-oxo-2-phenyl-8-(pyrimidin-5-yl)-1,2-dihydroisoquinolin-3-yl)ethyl)amino)pyrido[2,3-d]pyrimidin-5(8H)-one
[103] (S)-4-((1-(4-chloro-1-oxo-2-phenyl-8-(3,4,5-trimethoxyphenyl)-1,2-dihydroisoquinolin-3-yl)ethyl)amino)pyrido[2,3-d]pyrimidin-5(8H)-one
[104] (S)-4-((1-(4-chloro-8-(3-chloro-5-fluorophenyl)-1-oxo-2-phenyl-1,2-dihydroisoquinolin-3-yl)ethyl)amino)pyrido[2,3-d]pyrimidin-5(8H)-one
[105] (S)-4-((1-(4-chloro-1-oxo-2-phenyl-8-(4-(pyridin-4-yl)phenyl)-1,2-dihydroisoquinolin-3-yl)ethyl)amino)pyrido[2,3-d]pyrimidin-5(8H)-one
[106] (S)-4-((1-(4-chloro-8-(2-chloro-3-fluorophenyl)-1-oxo-2-phenyl-1,2-dihydroisoquinolin-3-yl)ethyl)amino)pyrido[2,3-d]pyrimidin-5(8H)-one
[107] (S)-4-((1-(4-chloro-8-(2-chloro-5-fluorophenyl)-1-oxo-2-phenyl-1,2-dihydroisoquinolin-3-yl)ethyl)amino)pyrido[2,3-d]pyrimidin-5(8H)-one
[108] (S)-4-((1-(4-chloro-8-(3-fluoro-5-hydroxyphenyl)-1-oxo-2-phenyl-1,2-dihydroisoquinolin-3-yl)ethyl)amino)pyrido[2,3-d]pyrimidin-5(8H)-one
[109] (S)-4-((1-(4-chloro-8-(3-fluoro-2-methylphenyl)-1-oxo-2-phenyl-1,2-dihydroisoquinolin-3-yl)ethyl)amino)pyrido[2,3-d]pyrimidin-5(8H)-one
[110] (S)-4-((1-(4-chloro-8-(2-methylpyridin-3-yl)-1-oxo-2-phenyl-1,2-dihydroisoquinolin-3-yl)ethyl)amino)pyrido[2,3-d]pyrimidin-5(8H)-one
[111] (S)-4-((1-(4-chloro-8-(1-methyl-1H-benzo[d]imidazol-6-yl)-1-oxo-2-phenyl-1,2-dihydroisoquinolin-3-yl)ethyl)amino)pyrido[2,3-d]pyrimidin-5(8H)-one
[112] (S)-4-((1-(4-chloro-8-(2-(dimethylamino)pyrimidin-5-yl)-1-oxo-2-phenyl-1,2-dihydroisoquinolin-3-yl)ethyl)amino)pyrido[2,3-d]pyrimidin-5(8H)-one
[113] (S)-4-((1-(4-chloro-8-(imidazo[1,2-a]pyridin-6-yl)-1-oxo-2-phenyl-1,2-dihydroisoquinolin-3-yl)ethyl)amino)pyrido[2,3-d]pyrimidin-5(8H)-one
[114] (S)-4-((1-(4-chloro-8-(1-methyl-1H-benzo[d]imidazol-5-yl)-1-oxo-2-phenyl-1,2-dihydroisoquinolin-3-yl)ethyl)amino)pyrido[2,3-d]pyrimidin-5(8H)-one
[115] (S)-4-((1-(4-chloro-1-oxo-2-phenyl-8-(6-phenylpyridin-3-yl)-1,2-dihydroisoquinolin-3-yl)ethyl)amino)pyrido[2,3-d]pyrimidin-5(8H)-one
[116] (S)-4-((1-(4-chloro-8-(1-methyl-1H-indol-5-yl)-1-oxo-2-phenyl-1,2-dihydroisoquinolin-3-yl)ethyl)amino)pyrido[2,3-d]pyrimidin-5(8H)-one
[117] (S)-4-((1-(4-chloro-8-(furan-2-yl)-1-oxo-2-phenyl-1,2-dihydroisoquinolin-3-yl)ethyl)amino)pyrido[2,3-d]pyrimidin-5(8H)-one
[118] (S)-4-((1-(4-chloro-8-(4-cyclobutylphenyl)-1-oxo-2-phenyl-1,2-dihydroisoquinolin-3-yl)ethyl)amino)pyrido[2,3-d]pyrimidin-5(8H)-one
[119] (S)-4-((1-(4-chloro-8-(5-methylpyridin-3-yl)-1-oxo-2-phenyl-1,2-dihydroisoquinolin-3-yl)ethyl)amino)pyrido[2,3-d]pyrimidin-5(8H)-one
[120] (S)-4-((1-(4-chloro-8-(3,4-dihydro-2H-benzo[b][1,4]oxazin-7-yl)-1-oxo-2-phenyl-1,2-dihydroisoquinolin-3-yl)ethyl)amino)pyrido[2,3-d]pyrimidin-5(8H)-one
[121] (S)-4-((1-(4-chloro-1-oxo-2-phenyl-8-(quinolin-6-yl)-1,2-dihydroisoquinolin-3-yl)ethyl)amino)pyrido[2,3-d]pyrimidin-5(8H)-one
[122] (S)-4-((1-(4-chloro-8-(2-fluorophenyl)-1-oxo-2-phenyl-1,2-dihydroisoquinolin-3-yl)ethyl)amino)pyrido[2,3-d]pyrimidin-5(8H)-one
[123] (S)-4-((1-(4-chloro-8-(3-fluorophenyl)-1-oxo-2-phenyl-1,2-dihydroisoquinolin-3-yl)ethyl)amino)pyrido[2,3-d]pyrimidin-5(8H)-one
[124] (S)-4-((1-(4-chloro-8-(3,5-difluorophenyl)-1-oxo-2-phenyl-1,2-dihydroisoquinolin-3-yl)ethyl)amino)pyrido[2,3-d]pyrimidin-5(8H)-one
[125] (S)-4-((1-(8-(1H-benzo[d]imidazol-5-yl)-4-chloro-1-oxo-2-phenyl-1,2-dihydroisoquinolin-3-yl)ethyl)amino)pyrido[2,3-d]pyrimidin-5(8H)-one
[126] (S)-4-((1-(4-chloro-8-(4-hydroxy-3,5-dimethylphenyl)-1-oxo-2-phenyl-1,2-dihydroisoquinolin-3-yl)ethyl)amino)pyrido[2,3-d]pyrimidin-5(8H)-one
[127] (S)-4-((1-(4-chloro-8-(5-fluoro-2-methylphenyl)-1-oxo-2-phenyl-1,2-dihydroisoquinolin-3-yl)ethyl)amino)pyrido[2,3-d]pyrimidin-5(8H)-one
[128] (S)-4-((1-(4-chloro-8-(4-fluoro-3-methylphenyl)-1-oxo-2-phenyl-1,2-dihydroisoquinolin-3-yl)ethyl)amino)pyrido[2,3-d]pyrimidin-5(8H)-one
[129] (S)-4-((1-(4-chloro-8-(2,3-difluorophenyl)-1-oxo-2-phenyl-1,2-dihydroisoquinolin-3-yl)ethyl)amino)pyrido[2,3-d]pyrimidin-5(8H)-one
[130] (S)-4-((1-(4-chloro-8-(4-(1-methylpiperidin-4-yl)phenyl)-1-oxo-2-phenyl-1,2-dihydroisoquinolin-3-yl)ethyl)amino)pyrido[2,3-d]pyrimidin-5(8H)-one
[131] (S)-4-((1-(4-chloro-8-(4-fluoro-2-methylphenyl)-1-oxo-2-phenyl-1,2-dihydroisoquinolin-3-yl)ethyl)amino)pyrido[2,3-d]pyrimidin-5(8H)-one
[132] (S)-4-((1-(4-chloro-8-(2,3-dihydro-[1,4]dioxino[2,3-b]pyridin-7-yl)-1-oxo-2-phenyl-1,2-dihydroisoquinolin-3-yl)ethyl)amino)pyrido[2,3-d]pyrimidin-5(8H)-one
[133] (S)-4-((1-(4-chloro-8-(6-methoxypyridin-3-yl)-1-oxo-2-phenyl-1,2-dihydroisoquinolin-3-yl)ethyl)amino)pyrido[2,3-d]pyrimidin-5(8H)-one
[134] (S)-4-((1-(4-chloro-1-oxo-2-phenyl-8-(pyridin-4-yl)-1,2-dihydroisoquinolin-3-yl)ethyl)amino)pyrido[2,3-d]pyrimidin-5(8H)-one
[135] (S)-4-((1-(4-chloro-8-(2-methyl-1H-benzo[d]imidazol-5-yl)-1-oxo-2-phenyl-1,2-dihydroisoquinolin-3-yl)ethyl)amino)pyrido[2,3-d]pyrimidin-5(8H)-one
[136] (S)-4-((1-(4-chloro-8-(2-methylpyrimidin-5-yl)-1-oxo-2-phenyl-1,2-dihydroisoquinolin-3-yl)ethyl)amino)pyrido[2,3-d]pyrimidin-5(8H)-one
[137] (S)-4-((1-(4-chloro-1-oxo-2-phenyl-8-(quinoxalin-6-yl)-1,2-dihydroisoquinolin-3-yl)ethyl)amino)pyrido[2,3-d]pyrimidin-5(8H)-one
[138] tert-butyl (S)-4-(4-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)phenyl)piperazine-1-carboxylate
[139] (S)-4-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-N,N-dimethylbenzamide
[140] (S)-4-((1-(4-chloro-8-(5-chloro-2-fluorophenyl)-1-oxo-2-phenyl-1,2-dihydroisoquinolin-3-yl)ethyl)amino)pyrido[2,3-d]pyrimidin-5(8H)-one
[141] (S)-4-((1-(4-chloro-8-(4-cyclopropylphenyl)-1-oxo-2-phenyl-1,2-dihydroisoquinolin-3-yl)ethyl)amino)pyrido[2,3-d]pyrimidin-5(8H)-one
[142] (S)-4-((1-(4-chloro-1-oxo-2-phenyl-8-vinyl-1,2-dihydroisoquinolin-3-yl)ethyl)amino)pyrido[2,3-d]pyrimidin-5(8H)-one
[143] (S)-4-((1-(4-chloro-8-(4-(methylthio)phenyl)-1-oxo-2-phenyl-1,2-dihydroisoquinolin-3-yl)ethyl)amino)pyrido[2,3-d]pyrimidin-5(8H)-one
[144] (S)-4-((1-(8-(4-(tert-butylthio)phenyl)-4-chloro-1-oxo-2-phenyl-1,2-dihydroisoquinolin-3-yl)ethyl)amino)pyrido[2,3-d]pyrimidin-5(8H)-one
[145] (S)-4-((1-(4-chloro-8-(4-(morpholine-4-carbonyl)phenyl)-1-oxo-2-phenyl-1,2-dihydroisoquinolin-3-yl)ethyl)amino)pyrido[2,3-d]pyrimidin-5(8H)-one
[146] (S)-4-((1-(8-(2-(benzyloxy)pyrimidin-5-yl)-4-chloro-1-oxo-2-phenyl-1,2-dihydroisoquinolin-3-yl)ethyl)amino)pyrido[2,3-d]pyrimidin-5(8H)-one
[147] (S)-4-((1-(4-chloro-1-oxo-2-phenyl-8-(6-(2,2,2-trifluoroethoxy)pyridin-3-yl)-1,2-dihydroisoquinolin-3-yl)ethyl)amino)pyrido[2,3-d]pyrimidin-5(8H)-one
[148] (S)-4-((1-(4-chloro-1-oxo-2-phenyl-8-(4-(piperazin-1-yl)phenyl)-1,2-dihydroisoquinolin-3-yl)ethyl)amino)pyrido[2,3-d]pyrimidin-5(8H)-one
[149] (S)-4-((1-(4-chloro-8-(2-methoxypyridin-4-yl)-1-oxo-2-phenyl-1,2-dihydroisoquinolin-3-yl)ethyl)amino)-8-(2-methoxypyridin-4-yl)pyrido[2,3-d]pyrimidin-5(8H)-one
[150] (S)-4-((1-(4-chloro-8-(2-methoxypyridin-4-yl)-1-oxo-2-phenyl-1,2-dihydroisoquinolin-3-yl)ethyl)amino)pyrido[2,3-d]pyrimidin-5(8H)-one
[151] (S)-4-((1-(4-chloro-1-oxo-2-phenyl-8-(4-(propylthio)phenyl)-1,2-dihydroisoquinolin-3-yl)ethyl)amino)pyrido[2,3-d]pyrimidin-5(8H)-one
[152] (S)-4-((1-(4-chloro-8-(4-(cyclopropylthio)phenyl)-1-oxo-2-phenyl-1,2-dihydroisoquinolin-3-yl)ethyl)amino)pyrido[2,3-d]pyrimidin-5(8H)-one
[153] (S)-4-((1-(4-chloro-1-oxo-2-phenyl-8-(2-(trifluoromethyl)pyrimidin-5-yl)-1,2-dihydroisoquinolin-3-yl)ethyl)amino)pyrido[2,3-d]pyrimidin-5(8H)-one
[154] (S)-4-((1-(4-chloro-8-(4-fluoro-3,5-dimethoxyphenyl)-1-oxo-2-phenyl-1,2-dihydroisoquinolin-3-yl)ethyl)amino)pyrido[2,3-d]pyrimidin-5(8H)-one
[155] (S)-4-((1-(4-chloro-8-(3,4-dimethoxyphenyl)-1-oxo-2-phenyl-1,2-dihydroisoquinolin-3-yl)ethyl)amino)pyrido[2,3-d]pyrimidin-5(8H)-one
[156] (S)-4-((1-(4-chloro-8-(cyclopent-1-en-1-yl)-1-oxo-2-phenyl-1,2-dihydroisoquinolin-3-yl)ethyl)amino)pyrido[2,3-d]pyrimidin-5(8H)-one
[157] (S)-4-((1-(4-chloro-8-(3-isopropoxy-5-methoxyphenyl)-1-oxo-2-phenyl-1,2-dihydroisoquinolin-3-yl)ethyl)amino)pyrido[2,3-d]pyrimidin-5(8H)-one
[158] (S)-4-((1-(4-chloro-8-(3-methoxy-5-(trifluoromethoxy)phenyl)-1-oxo-2-phenyl-1,2-dihydroisoquinolin-3-yl)ethyl)amino)pyrido[2,3-d]pyrimidin-5(8H)-one
[159] (S)-4-((1-(4-chloro-8-(3-methoxy-4-(trifluoromethoxy)phenyl)-1-oxo-2-phenyl-1,2-dihydroisoquinolin-3-yl)ethyl)amino)pyrido[2,3-d]pyrimidin-5(8H)-one
[160] (S)-4-((1-(4-chloro-8-(4-(5-methyl-1,2,4-oxadiazol-3-yl)phenyl)-1-oxo-2-phenyl-1,2-dihydroisoquinolin-3-yl)ethyl)amino)pyrido[2,3-d]pyrimidin-5(8H)-one
[161] (S)-4-((1-(8-(4-(5-(tert-butyl)-1,2,4-oxadiazol-3-yl)phenyl)-4-chloro-1-oxo-2-phenyl-1,2-dihydroisoquinolin-3-yl)ethyl)amino)pyrido[2,3-d]pyrimidin-5(8H)-one
[162] (S)-4-((1-(4-chloro-1-oxo-2-phenyl-8-(6-((tetrahydro-2H-pyran-4-yl)oxy)pyridin-3-yl)-1,2-dihydroisoquinolin-3-yl)ethyl)amino)pyrido[2,3-d]pyrimidin-5(8H)-one
[163] (S)-4-((1-(4-chloro-8-(6-(cyclopropylmethoxy)pyridin-3-yl)-1-oxo-2-phenyl-1,2-dihydroisoquinolin-3-yl)ethyl)amino)pyrido[2,3-d]pyrimidin-5(8H)-one
[164] (S)-4-((1-(8-(6-(benzyloxy)pyridin-3-yl)-4-chloro-1-oxo-2-phenyl-1,2-dihydroisoquinolin-3-yl)ethyl)amino)pyrido[2,3-d]pyrimidin-5(8H)-one
[165] (S)-4-((1-(4-chloro-8-(4-(methylsulfonyl)phenyl)-1-oxo-2-phenyl-1,2-dihydroisoquinolin-3-yl)ethyl)amino)pyrido[2,3-d]pyrimidin-5(8H)-one
[166] (S)-4-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)benzoic acid
[167] (S)-4-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-N-methylbenzamide
[168] (S)-4-((1-(4-chloro-8-(2-cyclopropylpyrimidin-5-yl)-1-oxo-2-phenyl-1,2-dihydroisoquinolin-3-yl)ethyl)amino)pyrido[2,3-d]pyrimidin-5(8H)-one
[169] (S)-4-((1-(4-chloro-8-(3,5-dimethoxyphenyl)-1-oxo-2-phenyl-1,2-dihydroisoquinolin-3-yl)ethyl)amino)pyrido[2,3-d]pyrimidin-5(8H)-one
[170] (S)-4-((1-(4-chloro-8-(5,6-dimethoxypyridin-3-yl)-1-oxo-2-phenyl-1,2-dihydroisoquinolin-3-yl)ethyl)amino)pyrido[2,3-d]pyrimidin-5(8H)-one
[171] (S)-4-((1-(4-chloro-8-(2-methoxypyrimidin-5-yl)-1-oxo-2-phenyl-1,2-dihydroisoquinolin-3-yl)ethyl)amino)pyrido[2,3-d]pyrimidin-5(8H)-one
[172] (S)-4-((1-(8-(6-aminopyridin-3-yl)-4-chloro-1-oxo-2-phenyl-1,2-dihydroisoquinolin-3-yl)ethyl)amino)pyrido[2,3-d]pyrimidin-5(8H)-one
[173] (S)-4-((1-(4-chloro-8-(3-morpholinophenyl)-1-oxo-2-phenyl-1,2-dihydroisoquinolin-3-yl)ethyl)amino)pyrido[2,3-d]pyrimidin-5(8H)-one
[174] (S)-4-((1-(4-chloro-8-(2-morpholinopyrimidin-5-yl)-1-oxo-2-phenyl-1,2-dihydroisoquinolin-3-yl)ethyl)amino)pyrido[2,3-d]pyrimidin-5(8H)-one
[175] (S)-4-((1-(4-chloro-8-(6-morpholinopyridin-3-yl)-1-oxo-2-phenyl-1,2-dihydroisoquinolin-3-yl)ethyl)amino)pyrido[2,3-d]pyrimidin-5(8H)-one
[176] (S)-4-((1-(4-chloro-1-oxo-2-phenyl-8-(4-(tetrahydro-2H-pyran-4-yl)phenyl)-1,2-dihydroisoquinolin-3-yl)ethyl)amino)pyrido[2,3-d]pyrimidin-5(8H)-one
[177] (S)-4-((1-(4-chloro-8-(3,5-dimethoxy-4-methylphenyl)-1-oxo-2-phenyl-1,2-dihydroisoquinolin-3-yl)ethyl)amino)pyrido[2,3-d]pyrimidin-5(8H)-one
[178] (S)-4-((1-(4-chloro-1-oxo-2-phenyl-8-(4-(trimethylsilyl)phenyl)-1,2-dihydroisoquinolin-3-yl)ethyl)amino)pyrido[2,3-d]pyrimidin-5(8H)-one
[179] (S)-4-((1-(4-chloro-8-(4-(4-methylpiperazin-1-yl)phenyl)-1-oxo-2-phenyl-1,2-dihydroisoquinolin-3-yl)ethyl)amino)pyrido[2,3-d]pyrimidin-5(8H)-one
[180] (S)-4-((1-(4-chloro-8-(2-isopropoxypyridin-4-yl)-1-oxo-2-phenyl-1,2-dihydroisoquinolin-3-yl)ethyl)amino)pyrido[2,3-d]pyrimidin-5(8H)-one
[181] (S)-4-((1-(4-chloro-1-oxo-2-phenyl-8-(6-(trifluoromethyl)pyridin-3-yl)-1,2-dihydroisoquinolin-3-yl)ethyl)amino)pyrido[2,3-d]pyrimidin-5(8H)-one
[182] (S)-4-((1-(4-chloro-8-(2-isopropoxypyrimidin-5-yl)-1-oxo-2-phenyl-1,2-dihydroisoquinolin-3-yl)ethyl)amino)pyrido[2,3-d]pyrimidin-5(8H)-one
[183] (S)-4-((1-(4-chloro-8-(6-ethoxypyridin-3-yl)-1-oxo-2-phenyl-1,2-dihydroisoquinolin-3-yl) ethyl)amino)pyrido[2,3-d]pyrimidin-5(8H)-one
[184] (S)-4-((1-(4-chloro-1-oxo-2-phenyl-8-(2-(2,2,2-trifluoroethoxy)pyrimidin-5-yl)-1,2-dihydroisoquinolin-3-yl)ethyl)amino)pyrido[2,3-d]pyrimidin-5(8H)-one
[185] (S)-4-((1-(4-chloro-l-oxo-8-(6-phenoxypyridin-3-yl)-2-phenyl-1,2-dihydroisoquinolin-3-yl) ethyl)amino)pyrido[2,3-d]pyrimidin-5(8H)-one
[186] (S)-4-((1-(4-chloro-8-(5-fluoro-6-methoxypyridin-3-yl)-1-oxo-2-phenyl-1,2-dihydroisoquinolin-3-yl)ethyl)amino)pyrido[2,3-d]pyrimidin-5(8H)-one
[187] (S)-4-((1-(4-chloro-8-(5-chloro-6-methoxypyridin-3-yl)-1-oxo-2-phenyl-1,2-dihydroisoquinolin-3-yl)ethyl)amino)pyrido[2,3-d]pyrimidin-5(8H)-one
[188] (S)-4-((1-(4-chloro-8-(2-ethoxypyrimidin-5-yl)-1-oxo-2-phenyl-1,2-dihydroisoquinolin-3-yl) ethyl)amino)pyrido[2,3-d]pyrimidin-5(8H)-one
[189] (S)-4-((1-(4-chloro-8-(2,4-dimethoxypyrimidin-5-yl)-1-oxo-2-phenyl-1,2-dihydroisoquinolin-3-yl)ethyl)amino)pyrido[2,3-d]pyrimidin-5(8H)-one
[190] (S)-4-((1-(4-chloro-8-(2-morpholinopyridin-4-yl)-1-oxo-2-phenyl-1,2-dihydroisoquinolin-3-yl) ethyl)amino)pyrido[2,3-d]pyrimidin-5(8H)-one
[191] (S)-4-((1-(4-chloro-8-(3-fluoro-4-methoxyphenyl)-1-oxo-2-phenyl-1,2-dihydroisoquinolin-3-yl) ethyl)amino)pyrido[2,3-d]pyrimidin-5(8H)-one
[192] (S)-4-((1-(4-chloro-8-(8-fluoro-3,4-dihydro-2H-benzo[b] [1,4]oxazin-6-yl)-1-oxo-2-phenyl-1,2-dihydroisoquinolin-3-yl)ethyl)amino)pyrido[2,3-d]pyrimidin-5(8H)-one
[193] (S)-4-(4-chloro-1-oxo-3-(1-((5-oxo-5,8-dihydropyrido[2,3-d]pyrimidin-4-yl) amino)ethyl)-2-phenyl-1,2-dihydroisoquinolin-8-yl)-N-isopropylbenzamide
[194] (S)-4-((1-(4-chloro-1-oxo-2-phenyl-8-(6-(piperidin-1-yl)pyridin-3-yl)-1,2-dihydroisoquinolin-3-yl)ethyl)amino)pyrido[2,3-d]pyrimidin-5(8H)-one
[195] (S)-4-((1-(4-chloro-8-(2-fluoropyridin-4-yl)-1-oxo-2-phenyl-1,2-dihydroisoquinolin-3-yl) ethyl)amino)pyrido[2,3-d]pyrimidin-5(8H)-one
[196] (S)-5-(4-chloro-1-oxo-3-(1-((5-oxo-5,8-dihydropyrido[2,3-d]pyrimidin-4-yl) amino)ethyl)-2-phenyl-1,2-dihydroisoquinolin-8-yl)-N-methylpicolinamide
[197] (S)-4-((1-(4-chloro-8-(6-methoxy-5-methylpyridin-3-yl)-1-oxo-2-phenyl-1,2-dihydroisoquinolin-3-yl)ethyl)amino)pyrido[2,3-d]pyrimidin-5(8H)-one
[198] (S)-4-((1-(4-chloro-1-oxo-2-phenyl-8-(pyrazin-2-yl)-1,2-dihydroisoquinolin-3-yl)ethyl)amino)pyrido[2,3-d]pyrimidin-5(8H)-one
[199] (S)-4-((1-(4-chloro-8-(3-ethoxy-2-fluoro-4-methoxyphenyl)-1-oxo-2-phenyl-1,2-dihydroisoquinolin-3-yl)ethyl)amino)pyrido[2,3-d]pyrimidin-5(8H)-one
[200] (S)-4-((1-(8-(2-(azetidin-1-yl)pyrimidin-5-yl)-4-chloro-1-oxo-2-phenyl-1,2-dihydroisoquinolin-3-yl)ethyl)amino)pyrido[2,3-d]pyrimidin-5(8H)-one
[201] (S)-5-(4-chloro-1-oxo-3-(1-((5-oxo-5,8-dihydropyrido[2,3-d]pyrimidin-4-yl) amino)ethyl)-2-phenyl-1,2-dihydroisoquinolin-8-yl)picolinamide
[202] (S)-N-(5-(4-chloro-1-oxo-3-(1-((5-oxo-5,8-dihydropyrido[2,3-d]pyrimidin-4-yl) amino)ethyl)-2-phenyl-1,2-dihydroisoquinolin-8-yl)pyridin-2-yl) acetamide
[203] (S)-4-((1-(4-chloro-8-(2-(cyclopropylamino)pyrimidin-5-yl)-1-oxo-2-phenyl-1,2-dihydroisoquinolin-3-yl)ethyl)amino)pyrido[2,3-d]pyrimidin-5(8H)-one
[204] (S)-4-((1-(4-chloro-8-(6-methoxypyrazin-2-yl)-1-oxo-2-phenyl-1,2-dihydroisoquinolin-3-yl) ethyl)amino)pyrido[2,3-d]pyrimidin-5(8H)-one
[205] (S)-4-((1-(4-chloro-8-(5-(dimethylamino)pyrazin-2-yl)-1-oxo-2-phenyl-1,2-dihydroisoquinolin-3-yl)ethyl)amino)pyrido[2,3-d]pyrimidin-5(8H)-one
[206] (S)-4-((1-(8-(5-(tert-butoxy)pyrazin-2-yl)-4-chloro-1-oxo-2-phenyl-1,2-dihydroisoquinolin-3-yl)ethyl)amino)pyrido[2,3-d]pyrimidin-5(8H)-one
[207] (S)-4-((1-(4-chloro-8-(5-morpholinopyrazin-2-yl)-1-oxo-2-phenyl-1,2-dihydroisoquinolin-3-yl) ethyl)amino)pyrido[2,3-d]pyrimidin-5(8H)-one
[208] (S)-4-((1-(8-(2-(tert-butyl)pyrimidin-5-yl)-4-chloro-1-oxo-2-phenyl-1,2-dihydroisoquinolin-3-yl)ethyl)amino)pyrido[2,3-d]pyrimidin-5(8H)-one
[209] (S)-4-((l-(4-chloro-8-(4-(isopropylsulfonyl)phenyl)-1-oxo-2-phenyl-1,2-dihydroisoquinolin-3-yl)ethyl)amino)pyrido[2,3-d]pyrimidin-5(8H)-one
[210] (S)-4-((1-(4-chloro-8-(6-morpholinopyrazin-2-yl)-1-oxo-2-phenyl-1,2-dihydroisoquinolin-3-yl) ethyl)amino)pyrido[2,3-d]pyrimidin-5(8H)-one
[211] (S)-4-((1-(4-chloro-8-(5-(4-methylpiperazin-1-yl)pyrazin-2-yl)-1-oxo-2-phenyl-1,2-dihydroisoquinolin-3-yl)ethyl)amino)pyrido[2,3-d]pyrimidin-5(8H)-one
[212] (S)-4-((1-(8-(2-amino-4-methylpyrimidin-5-yl)-4-chloro-1-oxo-2-phenyl-1,2-dihydroisoquinolin-3-yl)ethyl)amino)pyrido[2,3-d]pyrimidin-5(8H)-one
[213] (S)-4-((1-(4-chloro-1-oxo-2-phenyl-8-(quinolin-3-yl)-1,2-dihydroisoquinolin-3-yl)ethyl)amino)pyrido[2,3-d]pyrimidin-5(8H)-one
[214] (S)-4-((1-(4-chloro-8-(2-(methylthio)pyrimidin-5-yl)-1-oxo-2-phenyl-1,2-dihydroisoquinolin-3-yl)ethyl)amino)pyrido[2,3-d]pyrimidin-5(8H)-one
[215] (S)-4-((1-(4-chloro-8-(4-isopropylpyrimidin-5-yl)-1-oxo-2-phenyl-1,2-dihydroisoquinolin-3-yl) ethyl)amino)pyrido[2,3-d]pyrimidin-5(8H)-one
[216] (S)-4-((1-(4-chloro-8-(2-(4-methylpiperazin-1-yl)pyrimidin-5-yl)-1-oxo-2-phenyl-1,2-dihydroisoquinolin-3-yl)ethyl)amino)pyrido[2,3-d]pyrimidin-5(8H)-one
[217] (S)-4-((1-(4-chloro-8-(2-(diethylamino)pyrimidin-5-yl)-1-oxo-2-phenyl-1,2-dihydroisoquinolin-3-yl)ethyl)amino)pyrido[2,3-d]pyrimidin-5(8H)-one
[218] (S)-4-((1-(4-chloro-8-(5-(methylsulfonyl)pyridin-3-yl)-1-oxo-2-phenyl-1,2-dihydroisoquinolin-3-yl)ethyl)amino)pyrido[2,3-d]pyrimidin-5(8H)-one
[219] (S)-4-((1-(4-chloro-8-(6-(methylsulfonyl)pyridin-3-yl)-1-oxo-2-phenyl-1,2-dihydroisoquinolin-3-yl)ethyl)amino)pyrido[2,3-d]pyrimidin-5(8H)-one
[220] (S)-4-((1-(4-chloro-8-(2-(isopropylamino)pyrimidin-5-yl)-1-oxo-2-phenyl-1,2-dihydroisoquinolin-3-yl)ethyl)amino)pyrido[2,3-d]pyrimidin-5(8H)-one
[221] (S)-4-((1-(4-chloro-8-(5-methoxypyrazin-2-yl)-1-oxo-2-phenyl-1,2-dihydroisoquinolin-3-yl) ethyl)amino)pyrido[2,3-d]pyrimidin-5(8H)-one
[222] (S)-4-((1-(4-chloro-1-oxo-2-phenyl-8-(2-(pyrrolidin-1-yl)pyrimidin-5-yl)-1,2-dihydroisoquinolin-3-yl)ethyl)amino)pyrido[2,3-d]pyrimidin-5(8H)-one
[223] (S)-4-((1-(4-chloro-1-oxo-2-phenyl-8-(6-(trifluoromethoxy)pyridin-3-yl)-1,2-dihydroisoquinolin-3-yl)ethyl)amino)pyrido[2,3-d]pyrimidin-5(8H)-one
[224] (S)-4-((1-(4-chloro-1-oxo-2-phenyl-8-(2-(piperidin-1-yl)pyrimidin-5-yl)-1,2-dihydroisoquinolin-3-yl)ethyl)amino)pyrido[2,3-d]pyrimidin-5(8H)-one
[225] (S)-N-(5-(4-chloro-1-oxo-3-(1-((5-oxo-5,8-dihydropyrido[2,3-d]pyrimidin-4-yl) amino)ethyl)-2-phenyl-1,2-dihydroisoquinolin-8-yl)-2-methoxypyridin-3-yl)methanesulfonamide
[226] (S)-4-((1-(4-chloro-1-oxo-2-phenyl-8-(1H-pyrazol-3-yl)-1,2-dihydroisoquinolin-3-yl)ethyl)amino)pyrido[2,3-d]pyrimidin-5(8H)-one
[227] (S)-4-((1-(4-chloro-1-oxo-2-phenyl-8-(1H-pyrazol-4-yl)-1,2-dihydroisoquinolin-3-yl)ethyl)amino)pyrido[2,3-d]pyrimidin-5(8H)-one
[228] (S)-4-((1-(4-chloro-8-(2-(ethylamino)pyrimidin-5-yl)-1-oxo-2-phenyl-1,2-dihydroisoquinolin-3-yl)ethyl)amino)pyrido[2,3-d]pyrimidin-5(8H)-one
[229] (S)-5-(4-chloro-1-oxo-3-(1-((5-oxo-5,8-dihydropyrido[2,3-d]pyrimidin-4-yl) amino)ethyl)-2-phenyl-1,2-dihydroisoquinolin-8-yl)-2-methoxynicotinonitrile
[230] (S)-4-((1-(4-chloro-8-(5-ethoxypyrazin-2-yl)-1-oxo-2-phenyl-1,2-dihydroisoquinolin-3-yl) ethyl)amino)pyrido[2,3-d]pyrimidin-5(8H)-one
[231] (S)-4-((1-(4-chloro-8-(2-fluoro-4-(morpholinomethyl)phenyl)-1-oxo-2-phenyl-1,2-dihydroisoquinolin-3-yl)ethyl)amino)pyrido[2,3-d]pyrimidin-5(8H)-one
[232] (S)-N-(5-(4-chloro-1-oxo-3-(1-((5-oxo-5,8-dihydropyrido[2,3-d]pyrimidin-4-yl) amino)ethyl)-2-phenyl-1,2-dihydroisoquinolin-8-yl)-2-methoxypyridin-3-yl)-2,4-difluorobenzenesulfonamide
[233] (S)-4-((1-(8-(benzo[c] [1,2,5]thiadiazol-5-yl)-4-chloro-1-oxo-2-phenyl-1,2-dihydroisoquinolin-3-yl)ethyl)amino)pyrido[2,3-d]pyrimidin-5(8H)-one
[234] (S)-4-((1-(4-chloro-8-(5-methyl-1H-pyrazol-3-yl)-1-oxo-2-phenyl-1,2-dihydroisoquinolin-3-yl) ethyl)amino)pyrido[2,3-d]pyrimidin-5(8H)-one
[235] (S)-4-((1-(8-([1,2,5]oxadiazolo[3,4-b]pyridin-6-yl)-4-chloro-1-oxo-2-phenyl-1,2-dihydroisoquinolin-3-yl)ethyl)amino)pyrido[2,3-d]pyrimidin-5(8H)-one
[236] (S)-4-((1-(4-chloro-8-(1H-indazol-5-yl)-1-oxo-2-phenyl-1,2-dihydroisoquinolin-3-yl)ethyl)amino)pyrido[2,3-d]pyrimidin-5(8H)-one
[237] (S)-4-((1-(4-chloro-8-(1-methyl-1H-indazol-4-yl)-1-oxo-2-phenyl-1,2-dihydroisoquinolin-3-yl) ethyl)amino)pyrido[2,3-d]pyrimidin-5(8H)-one
[238] (S)-4-((1-(4-chloro-8-(isoxazol-4-yl)-1-oxo-2-phenyl-1,2-dihydroisoquinolin-3-yl)ethyl)amino)pyrido[2,3-d]pyrimidin-5(8H)-one
[239] (S)-4-((1-(4-chloro-8-(1H-indazol-7-yl)-1-oxo-2-phenyl-1,2-dihydroisoquinolin-3-yl) ethyl)amino)pyrido[2,3-d]pyrimidin-5(8H)-one
[240] (S)-4-((1-(8-(benzo[c][1,2,5]oxadiazol-5-yl)-4-chloro-1-oxo-2-phenyl-1,2-dihydroisoquinolin-3-yl)ethyl)amino)pyrido[2,3-d]pyrimidin-5(8H)-one
[241] (S)-4-((1-(8-(benzo[d]oxazol-5-yl)-4-chloro-1-oxo-2-phenyl-1,2-dihydroisoquinolin-3-yl) ethyl)amino)pyrido[2,3-d]pyrimidin-5(8H)-one
[242] (S)-4-((1-(4-chloro-8-(4-methylthiophen-2-yl)-1-oxo-2-phenyl-1,2-dihydroisoquinolin-3-yl) ethyl)amino)pyrido[2,3-d]pyrimidin-5(8H)-one
[243] (S)-4-((1-(4-chloro-8-(3-methylthiophen-2-yl)-1-oxo-2-phenyl-1,2-dihydroisoquinolin-3-yl) ethyl)amino)pyrido[2,3-d]pyrimidin-5(8H)-one
[244] (S)-4-((1-(4-chloro-8-(4-methyl-1H-pyrazol-3-yl)-1-oxo-2-phenyl-1,2-dihydroisoquinolin-3-yl)ethyl)amino)pyrido[2,3-d]pyrimidin-5(8H)-one
[245] (S)-4-((1-(4-chloro-8-(morpholinomethyl)-1-oxo-2-phenyl-1,2-dihydroisoquinolin-3-yl) ethyl)amino)pyrido[2,3-d]pyrimidin-5(8H)-one
[246] (S)-4-((1-(4-chloro-8-(6-(methylamino)pyridin-3-yl)-1-oxo-2-phenyl-1,2-dihydroisoquinolin-3-yl)ethyl)amino)pyrido[2,3-d]pyrimidin-5(8H)-one
[247] (S)-4-((1-(4-chloro-1-oxo-2-phenyl-8-(4-(trifluoromethoxy)phenyl)-1,2-dihydroisoquinolin-3-yl)ethyl)amino)pyrido[2,3-d]pyrimidin-5(8H)-one
[248] (S)-4-((1-(4-chloro-8-(2,6-dimethylpyridin-3-yl)-1-oxo-2-phenyl-1,2-dihydroisoquinolin-3-yl)ethyl)amino)pyrido[2,3-d]pyrimidin-5(8H)-one
[249] (S)-4-((1-(4-chloro-8-(dimethylamino)-1-oxo-2-phenyl-1,2-dihydroisoquinolin-3-yl) ethyl)amino)pyrido[2,3-d]pyrimidin-5(8H)-one
[250] (S)-4-((1-(4-chloro-8-(4-methylpiperazin-1-yl)-1-oxo-2-phenyl-1,2-dihydroisoquinolin-3-yl) ethyl)amino)pyrido[2,3-d]pyrimidin-5(8H)-one
[251] (S)-4-((1-(4-chloro-8-morpholino-1-oxo-2-phenyl-1,2-dihydroisoquinolin-3-yl)ethyl)amino)pyrido[2,3-d]pyrimidin-5(8H)-one
[252] (S)-4-((1-(4-chloro-1-oxo-2-phenyl-8-(pyrrolidin-1-yl)-1,2-dihydroisoquinolin-3-yl) ethyl)amino)pyrido[2,3-d]pyrimidin-5(8H)-one
[253] (S)-4-((1-(4-chloro-8-(2,3-dihydrobenzo[b] [1,4]dioxin-6-yl)-1-oxo-2-phenyl-1,2-dihydroisoquinolin-3-yl)ethyl)amino)pyrido[2,3-d]pyrimidin-5(8H)-one
[254] (S)-4-((1-(8-([1,1'-biphenyl]-4-yl)-4-chloro-1-oxo-2-phenyl-1,2-dihydroisoquinolin-3-yl) ethyl)amino)pyrido[2,3-d]pyrimidin-5(8H)-one
[255] (S)-4-((1-(4-chloro-1-oxo-2-phenyl-8-(p-tolyl)-1,2-dihydroisoquinolin-3-yl)ethyl)amino)pyrido[2,3-d]pyrimidin-5(8H)-one
[256] (S)-4-((1-(4-chloro-8-(2-chloro-4-(trifluoromethyl)phenyl)-1-oxo-2-phenyl-1,2-dihydroisoquinolin-3-yl)ethyl)amino)pyrido[2,3-d]pyrimidin-5(8H)-one
[257] (S)-4-((1-(4-chloro-8-(6-methylpyridin-3-yl)-1-oxo-2-phenyl-1,2-dihydroisoquinolin-3-yl) ethyl)amino)pyrido[2,3-d]pyrimidin-5(8H)-one
[258] (S)-4-((1-(4-chloro-1-oxo-2-phenyl-8-(1H-pyrrol-2-yl)-1,2-dihydroisoquinolin-3-yl) ethyl)amino)pyrido[2,3-d]pyrimidin-5(8H)-one
[259] (S)-4-(4-chloro-1-oxo-3-(1-((5-oxo-5,8-dihydropyrido[2,3-d]pyrimidin-4-yl) amino)ethyl)-2-phenyl-1,2-dihydroisoquinolin-8-yl)benzonitrile
[260] (S)-4-((1-(8-(4-morpholinophenyl)-1-oxo-2-phenyl-1,2-dihydroisoquinolin-3-yl)ethyl)amino)pyrido[2,3-d]pyrimidin-5(8H)-one
[261] (S)-4-((1-(1-oxo-2-phenyl-8-(4-(pyrrolidin-1-yl)phenyl)-1,2-dihydroisoquinolin-3-yl)ethyl)amino)pyrido[2,3-d]pyrimidin-5(8H)-one
[262] (S)-4-((1-(8-(4-(hydroxymethyl)phenyl)-1-oxo-2-phenyl-1,2-dihydroisoquinolin-3-yl) ethyl)amino)pyrido[2,3-d]pyrimidin-5(8H)-one
[263] (S)-4-((1-(8-(3-methyl-1H-indol-5-yl)-1-oxo-2-phenyl-1,2-dihydroisoquinolin-3-yl) ethyl)amino)pyrido[2,3-d]pyrimidin-5(8H)-one
[264] (S)-4-((1-(8-(6-(dimethylamino)pyridin-3-yl)-1-oxo-2-phenyl-1,2-dihydroisoquinolin-3-yl) ethyl)amino)pyrido[2,3-d]pyrimidin-5(8H)-one
[265] (S)-4-chloro-1-oxo-3-(1-((5-oxo-5,8-dihydropyrido[2,3-d]pyrimidin-4-yl) amino)ethyl)-2-phenyl-1,2-dihydroisoquinoline-8-carbonitrile
[266] (S)-1-oxo-3-(1-((5-oxo-5,8-dihydropyrido[2,3-d]pyrimidin-4-yl)amino)ethyl)-2-phenyl-1,2-dihydroisoquinoline-8-carbonitrile
[267] (S)-4-((1-(8-acetyl-4-chloro-1-oxo-2-phenyl-1,2-dihydroisoquinolin-3-yl)ethyl)amino)pyrido[2,3-d]pyrimidin-5(8H)-one
[268] (S)-4-((1-(8-butryl-4-chloro-1-oxo-2-phenyl-1,2-dihydroisoquinolin-3-yl)ethyl)amino)pyrido[2,3-d]pyrimidin-5(8H)-one
[269] (S)-4-((1-(4-chloro-8-(cyclopropanecarbonyl)-1-oxo-2-phenyl-1,2-dihydroisoquinolin-3-yl) ethyl)amino)pyrido[2,3-d]pyrimidin-5(8H)-one
[270] (S,Z)-4-((1-(4-chloro-8-(1-(methoxyimino)ethyl)-1-oxo-2-phenyl-1,2-dihydroisoquinolin-3-yl)ethyl)amino)pyrido[2,3-d]pyrimidin-5(8H)-one
[271] (S)-4-chloro-1-oxo-3-(1-((5-oxo-5,8-dihydropyrido[2,3-d]pyrimidin-4-yl) amino)ethyl)-2-phenyl-1,2-dihydroisoquinoline-8-carbaldehyde
[272] 4-(((1S)-1-(4-chloro-8-(1,2-dihydroxy-2-phenylethyl)-1-oxo-2-phenyl-1,2-dihydroisoquinolin-3-yl)ethyl)amino)pyrido[2,3-d]pyrimidin-5(8H)-one
[273] (S,Z)-4-chloro-1-oxo-3-(1-((5-oxo-5,8-dihydropyrido[2,3-d]pyrimidin-4-yl)amino)ethyl)-2-phenyl-1,2-dihydroisoquinoline-8-carbaldehyde oxime
[274] (S)-4-chloro-N-methyl-1-oxo-3-(1-((5-oxo-5,8-dihydropyrido[2,3-d]pyrimidin-4-yl)amino)ethyl)-2-phenyl-1,2-dihydroisoquinoline-8-carboxamide
[275] (S)-4-chloro-1-oxo-3-(1-((5-oxo-5,8-dihydropyrido[2,3-d]pyrimidin-4-yl)amino)ethyl)-2-phenyl-N-propyl-1,2-dihydroisoquinoline-8-carboxamide
[276] (S)-4-chloro-N-(oxetan-3-yl)-1-oxo-3-(1-((5-oxo-5,8-dihydropyrido[2,3-d]pyrimidin-4-yl)amino)ethyl)-2-phenyl-1,2-dihydroisoquinoline-8-carboxamide
[277] (S)-4-chloro-N-cyclobutyl-1-oxo-3-(1-((5-oxo-5,8-dihydropyrido[2,3-d]pyrimidin-4-yl)amino)ethyl)-2-phenyl-1,2-dihydroisoquinoline-8-carboxamide
[278] (S)-4-chloro-1-oxo-3-(1-((5-oxo-5,8-dihydropyrido[2,3-d]pyrimidin-4-yl) amino)ethyl)-2-phenyl-N-(thiophen-2-ylmethyl)-1,2-dihydroisoquinoline-8-carboxamide
[279] (S)-4-chloro-N-(furan-2-ylmethyl)-1-oxo-3-(1-((5-oxo-5, 8-dihydropyrido[2,3-d]pyrimidin-4-yl) amino)ethyl)-2-phenyl-1,2-dihydroisoquinoline-8-carboxamide
[280] (S)-N-benzyl-4-chloro-1-oxo-3-(1-((5-oxo-5,8-dihydropyrido[2,3-d]pyrimidin-4-yl)amino)ethyl)-2-phenyl-1,2-dihydroisoquinoline-8-carboxamide
[281] (S)-4-chloro-N-(2-morpholinoethyl)-1-oxo-3-(1-((5-oxo-5,8-dihydropyrido[2,3-d]pyrimidin-4-yl)amino)ethyl)-2-phenyl-1,2-dihydroisoquinoline-8-carboxamide
[282] (S)-4-chloro-N-isopropyl-1-oxo-3-(1-((5-oxo-5,8-dihydropyrido[2,3-d]pyrimidin-4-yl) amino)ethyl)-2-phenyl-1,2-dihydroisoquinoline-8-carboxamide
[283] (S)-4-chloro-N-(cyclopropylmethyl)-1-oxo-3-(1-((5-oxo-5, 8-dihydropyrido[2,3-d]pyrimidin-4-yl) amino)ethyl)-2-phenyl-1,2-dihydroisoquinoline-8-carboxamide
[284] (S)-4-chloro-N-(1-hydroxy-2-methylpropan-2-yl)-1-oxo-3-(1-((5-oxo-5,8-dihydropyrido[2,3-d]pyrimidin-4-yl)amino)ethyl)-2-phenyl-1,2-dihydroisoquinoline-8-carboxamide
[285] (S)-N-(tert-butyl)-4-chloro-1-oxo-3-(1-((5-oxo-5, 8-dihydropyrido[2,3-d]pyrimidin-4-yl) amino)ethyl)-2-phenyl-1,2-dihydroisoquinoline-8-carboxamide
[286] (S)-4-((1-(4-chloro-8-(morpholine-4-carbonyl)-1-oxo-2-phenyl-1,2-dihydroisoquinolin-3-yl)ethyl)amino)pyrido[2,3-d]pyrimidin-5(8H)-one
[287] (S)-4-((1-(4-chloro-8-(1H-imidazol-2-yl)-1-oxo-2-phenyl-1,2-dihydroisoquinolin-3-yl) ethyl)amino)pyrido[2,3-d]pyrimidin-5(8H)-one
[288] (S)-4-((1-(4-chloro-8-(4,5-dimethyl-1H-imidazol-2-yl)-1-oxo-2-phenyl-1,2-dihydroisoquinolin-3-yl)ethyl)amino)pyrido[2,3-d]pyrimidin-5(8H)-one
[289] (S)-4-((1-(4-chloro-8-(4,4-dimethyl-4,5-dihydrooxazol-2-yl)-1-oxo-2-phenyl-1,2-dihydroisoquinolin-3-yl)ethyl)amino)pyrido[2,3-d]pyrimidin-5(8H)-one
[290] (S)-4-chloro-1-oxo-3-(1-((5-oxo-5,8-dihydropyrido[2,3-d]pyrimidin-4-yl)amino)ethyl)-2-phenyl-1,2-dihydroisoquinoline-8-carboxylic acid
[291] (S)-4-chloro-1-oxo-3-(1-((5-oxo-5,8-dihydropyrido[2,3-d]pyrimidin-4-yl)amino)ethyl)-2-phenyl-1,2-dihydroisoquinoline-8-carboxamide
[292] Ethyl (S)-4-chloro-1-oxo-3-(1-((5-oxo-5,8-dihydropyrido[2,3-d]pyrimidin-4-yl)amino)ethyl)-2-phenyl-1,2-dihydroisoquinoline-8-carboxylate
[293] (S)-4-((1-(4-chloro-8-(4,6-diamino-1,3,5-triazin-2-yl)-1-oxo-2-phenyl-1,2-dihydroisoquinolin-3-yl)ethyl)amino)pyrido[2,3-d]pyrimidin-5(8H)-one
[294] (S)-4-((1-(8-amino-4-chloro-1-oxo-2-phenyl-1,2-dihydroisoquinolin-3-yl)ethyl)amino)pyrido[2,3-d]pyrimidin-5(8H)-one
[295] (S)-N-(4-chloro-1-oxo-3-(1-((5-oxo-5,8-dihydropyrido[2,3-d]pyrimidin-4-yl) amino)ethyl)-2-phenyl-1,2-dihydroisoquinolin-8-yl)pivalamide
[296] (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)acetamide
[297] (S)-N-(4-chloro-1-oxo-3-(1-((5-oxo-5,8-dihydropyrido[2,3-d]pyrimidin-4-yl) amino)ethyl)-2-phenyl-1,2-dihydroisoquinolin-8-yl)pentanamide
[298] (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)cyclobutanecarboxamide
[299] (S)-N-(4-chloro-1-oxo-3-(1-((5-oxo-5,8-dihydropyrido[2,3-d]pyrimidin-4-yl) amino)ethyl)-2-phenyl-1,2-dihydroisoquinolin-8-yl)benzamide
[300] (S)-N-(4-chloro-1-oxo-3-(1-((5-oxo-5,8-dihydropyrido[2,3-d]pyrimidin-4-yl) amino)ethyl)-2-phenyl-1,2-dihydroisoquinolin-8-yl)methanesulfonamide
[301] (S)-N-(4-chloro-1-oxo-3-(1-((5-oxo-5,8-dihydropyrido[2,3-d]pyrimidin-4-yl) amino)ethyl)-2-phenyl-1,2-dihydroisoquinolin-8-yl)cyclopropanesulfonamide
[302] (S)-N-(4-chloro-1-oxo-3-(1-((5-oxo-5,8-dihydropyrido[2,3-d]pyrimidin-4-yl) amino)ethyl)-2-phenyl-1,2-dihydroisoquinolin-8-yl)-2,2,2-trifluoroethane-1-sulfonamide
[303] (S)-N-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-4-fluorobenzenesulfonamide
[304] (S)-4-((1-(4-chloro-1-oxo-2-phenyl-8-(thiophen-2-ylethynyl)-1,2-dihydroisoquinolin-3-yl) ethyl)amino)pyrido[2,3-d]pyrimidin-5(8H)-one
[305] (S)-4-((1-(4-chloro-1-oxo-2-phenyl-8-(phenylethynyl)-1,2-dihydroisoquinolin-3-yl)ethyl)amino)pyrido[2,3-d]pyrimidin-5(8H)-one
[306] (S)-4-((1-(4-chloro-1-oxo-2-phenyl-8-(pyridin-4-ylethynyl)-1,2-dihydroisoquinolin-3-yl) ethyl)amino)pyrido[2,3-d]pyrimidin-5(8H)-one
[307] (S)-4-((1-(4-chloro-1-oxo-2-phenyl-8-(pyridin-3-ylethynyl)-1,2-dihydroisoquinolin-3-yl) ethyl)amino)pyrido[2,3-d]pyrimidin-5(8H)-one
[308] (S)-4-((1-(4-chloro-1-oxo-2-phenyl-8-(pyridin-2-ylethynyl)-1,2-dihydroisoquinolin-3-yl) ethyl)amino)pyrido[2,3-d]pyrimidin-5(8H)-one
[309] (S)-4-((1-(4-chloro-8-((1-methyl-1H-pyrazol-4-yl)ethynyl)-1-oxo-2-phenyl-1,2-dihydroisoquinolin-3-yl)ethyl)amino)pyrido[2,3-d]pyrimidin-5(8H)-one
[310] (S)-4-((1-(8-([1,1'-biphenyl]-4-ylethynyl)-4-chloro-1-oxo-2-phenyl-1,2-dihydroisoquinolin-3-yl)ethyl)amino)pyrido[2,3-d]pyrimidin-5(8H)-one
[311] (S)-4-((1-(4-chloro-8-((1-methyl-1H-pyrazol-5-yl)ethynyl)-1-oxo-2-phenyl-1,2-dihydroisoquinolin-3-yl)ethyl)amino)pyrido[2,3-d]pyrimidin-5(8H)-one
[312] (S)-4-((1-(4-chloro-1-oxo-2-phenyl-8-(thiazol-2-ylethynyl)-1,2-dihydroisoquinolin-3-yl) ethyl)amino)pyrido[2,3-d]pyrimidin-5(8H)-one
[313] (S)-4-((1-(4-chloro-1-oxo-2-phenyl-8-(thiazol-5-ylethynyl)-1,2-dihydroisoquinolin-3-yl) ethyl)amino)pyrido[2,3-d]pyrimidin-5(8H)-one
[314] (S)-4-((1-(4-chloro-8-((4-(dimethylamino)phenyl)ethynyl)-1-oxo-2-phenyl-1,2-dihydroisoquinolin-3-yl)ethyl)amino)-5-oxo-5, 8-dihydropyrido[2, 3-d]pyrimidin-5 (8H)-one
[315] (S)-4-((1-(4-chloro-1-oxo-2-phenyl-8-((4-(trifluoromethyl)phenyl)ethynyl)-1,2-dihydroisoquinolin-3-yl)ethyl)amino)-5-oxo-5,8-dihydropyrido[2,3-d]pyrimidin-8-ium 2,2,2-trifluoroacetate
[316] (S)-4-((1-(4-chloro-1-oxo-2-phenyl-8-(pyrimidin-5-ylethynyl)-1,2-dihydroisoquinolin-3-yl) ethyl)amino)pyrido[2,3-d]pyrimidin-5(8H)-one
[317] (S)-4-((1-(8-(benzo[d]oxazol-2-yl)-4-chloro-1-oxo-2-phenyl-1,2-dihydroisoquinolin-3-yl) ethyl)amino)pyrido[2,3-d]pyrimidin-5(8H)-one
[318] (S)-4-((1-(8-(benzo[d]oxazol-2-yl)-1-oxo-2-phenyl-1,2-dihydroisoquinolin-3-yl) ethyl)amino)pyrido[2,3-d]pyrimidin-5(8H)-one
[319] (S)-4-((1-(8-(benzyl(methyl)amino)-4-chloro-1-oxo-2-phenyl-1,2-dihydroisoquinolin-3-yl)ethyl)amino)pyrido[2,3-d]pyrimidin-5(8H)-one
[320] (S)-4-((1-(4-chloro-1-oxo-2-phenyl-8-(1H-pyrazol-1-yl)-1,2-dihydroisoquinolin-3-yl)ethyl)amino)pyrido[2,3-d]pyrimidin-5(8H)-one
[321] (S)-4-((1-(8-(benzylamino)-4-chloro-1-oxo-2-phenyl-1,2-dihydroisoquinolin-3-yl) ethyl)amino)pyrido[2,3-d]pyrimidin-5(8H)-one
[322] (S)-4-((1-(4-chloro-8-(cyclohexyl(methyl)amino)-1-oxo-2-phenyl-1,2-dihydroisoquinolin-3-yl)ethyl)amino)pyrido[2,3-d]pyrimidin-5(8H)-one
[323] (S)-4-((1-(4-chloro-8-(cyclopropylamino)-1-oxo-2-phenyl-1,2-dihydroisoquinolin-3-yl) ethyl)amino)pyrido[2,3-d]pyrimidin-5(8H)-one
[324] (S)-4-((1-(4-chloro-1-oxo-2-phenyl-8-((pyridin-3-ylmethyl)amino)-1,2-dihydroisoquinolin-3-yl)ethyl)amino)pyrido[2,3-d]pyrimidin-5(8H)-one
[325] (S)-4-((1-(4-chloro-8-(ethylamino)-1-oxo-2-phenyl-1,2-dihydroisoquinolin-3-yl) ethyl)amino)pyrido[2,3-d]pyrimidin-5(8H)-one
[326] (S)-4-((1-(4-chloro-1-oxo-2-phenyl-8-(4-phenylpiperazin-1-yl)-1,2-dihydroisoquinolin-3-yl)ethyl)amino)pyrido[2,3-d]pyrimidin-5(8H)-one
[327] 4-(((S)-1-(4-chloro-1-oxo-2-phenyl-8-((1S,5R)-1,3,3-trimethyl-6-azabicyclo[3.2.1]octan-6-yl)-1,2-dihydroisoquinolin-3-yl)ethyl)amino)pyrido[2,3-d]pyrimidin-5(8H)-one
[328] 4'-chloro-3'-((S)-1-((5-oxo-5,8-dihydropyrido[2,3-d]pyrimidin-4-yl) amino)ethyl)-2'-phenyl-3,4,4a,5,6,7,8,8a-octahydro-1H-[2,8'-biisoquinolin]-1' (2'H)-one
[329] (S)-4'-chloro-7-methyl-3'-(1-((5-oxo-5,8-dihydropyrido[2,3-d]pyrimidin-4-yl) amino)ethyl)-2'-phenyl-3,4-dihydro-1H-[2,8'-biisoquinolin]-1' (2'H)-one
[330] (S)-4-((1-(4-chloro-8-(4,7-dihydrothieno[2,3-c]pyridin-6(5H)-yl)-1-oxo-2-phenyl-1,2-dihydroisoquinolin-3-yl)ethyl)amino)pyrido[2,3-d]pyrimidin-5(8H)-one
[331] 4-(((1S)-1-(4-chloro-1-oxo-2-phenyl-8-((tetrahydrofuran-3-yl)amino)-1,2-dihydroisoquinolin-3-yl)ethyl)amino)pyrido[2,3-d]pyrimidin-5(8H)-one
[332] (S)-4-((1-(4-chloro-1-oxo-2-phenyl-8-((tetrahydro-2H-pyran-4-yl)amino)-1,2-dihydroisoquinolin-3-yl)ethyl)amino)pyrido[2,3-d]pyrimidin-5(8H)-one
[333] (S)-4-((1-(4-chloro-1-oxo-2-phenyl-8-(4-(pyridin-2-yl)piperazin-1-yl)-1,2-dihydroisoquinolin-3-yl)ethyl)amino)pyrido[2,3-d]pyrimidin-5(8H)-one
[334] (S)-4-((1-(4-chloro-8-(methyl(naphthalen-2-ylmethyl)amino)-1-oxo-2-phenyl-1,2-dihydroisoquinolin-3-yl)ethyl)amino)pyrido[2,3-d]pyrimidin-5(8H)-one
[335] (S)-4-((1-(4-chloro-8-((4-chlorophenethyl)amino)-1-oxo-2-phenyl-1,2-dihydroisoquinolin-3-yl)ethyl)amino)pyrido[2,3-d]pyrimidin-5(8H)-one
[336] (S)-4-((1-(4-chloro-1-oxo-2-phenyl-8-(4-phenylpiperidin-1-yl)-1,2-dihydroisoquinolin-3-yl)ethyl)amino)pyrido[2,3-d]pyrimidin-5(8H)-one
[337] (S)-1-(4-chloro-1-oxo-3-((S)-1-((5-oxo-5,8-dihydropyrido[2,3-d]pyrimidin-4-yl) amino)ethyl)-2-phenyl-1,2-dihydroisoquinolin-8-yl)pyrrolidine-2-carboxamide
[338] 4-(((S)-1-(4-chloro-1-oxo-2-phenyl-8-((S)-2-(pyrrolidin-1-ylmethyl)pyrrolidin-1-yl)-1,2-dihydroisoquinolin-3-yl)ethyl)amino)pyrido[2,3-d]pyrimidin-5(8H)-one
[339] 4-(((1S)-1-(4-chloro-8-(2-hydroxypyrrolidin-1-yl)-1-oxo-2-phenyl-1,2-dihydroisoquinolin-3-yl)ethyl)amino)pyrido[2,3-d]pyrimidin-5(8H)-one
[340] (S)-4-((1-(4-chloro-8-(isoindolin-2-yl)-1-oxo-2-phenyl-1,2-dihydroisoquinolin-3-yl) ethyl)amino)pyrido[2,3-d]pyrimidin-5(8H)-one
[341] (S)-4-((1-(4-chloro-8-(indolin-1-yl)-1-oxo-2-phenyl-1,2-dihydroisoquinolin-3-yl)ethyl)amino)pyrido[2,3-d]pyrimidin-5(8H)-one
[342] 4-(((1S)-1-(4-chloro-8-(hexahydrocyclopenta[c]pyrrol-2(1H)-yl)-1-oxo-2-phenyl-1,2-dihydroisoquinolin-3-yl)ethyl)amino)pyrido[2,3-d]pyrimidin-5(8H)-one
[343] (S)-4-((1-(4-chloro-1-oxo-2-phenyl-8-(phenylamino)-1,2-dihydroisoquinolin-3-yl)ethyl)amino)pyrido[2,3-d]pyrimidin-5(8H)-one
[344] (S)-4-((1-(4-chloro-8-((4-fluorophenyl)amino)-1-oxo-2-phenyl-1,2-dihydroisoquinolin-3-yl)ethyl)amino)pyrido[2,3-d]pyrimidin-5(8H)-one
[345] (S)-4-((1-(4-chloro-8-((4-methoxyphenyl)amino)-1-oxo-2-phenyl-1,2-dihydroisoquinolin-3-yl) ethyl)amino)pyrido[2,3-d]pyrimidin-5(8H)-one
[346] (S)-4-((1-(4-chloro-1-oxo-2-phenyl-8-((4-(trifluoromethyl)phenyl)amino)-1,2-dihydroisoquinolin-3-yl)ethyl)amino)pyrido[2,3-d]pyrimidin-5(8H)-one
[347] (S)-4-((1-(4-chloro-1-oxo-2-phenyl-8-(pyridin-3-ylamino)-1,2-dihydroisoquinolin-3-yl)ethyl)amino)pyrido[2,3-d]pyrimidin-5(8H)-one
[348] (S)-4-((1-(4-chloro-1-oxo-2-phenyl-8-(pyridin-2-ylamino)-1,2-dihydroisoquinolin-3-yl)ethyl)amino)pyrido[2,3-d]pyrimidin-5(8H)-one
[349] (S)-4-((1-(4-chloro-8-(methyl(pyridin-2-yl)amino)-1-oxo-2-phenyl-1,2-dihydroisoquinolin-3-yl)ethyl)amino)pyrido[2,3-d]pyrimidin-5(8H)-one
[350] (S,Z)-4-chloro-N'-hydroxy-1-oxo-3-(1-((5-oxo-5,8-dihydropyrido[2,3-d]pyrimidin-4-yl)amino)ethyl)-2-phenyl-1,2-dihydroisoquinoline-8-carboximidamide
[351] (S)-4-((1-(4-chloro-8-(1,2,4-oxadiazol-3-yl)-1-oxo-2-phenyl-1,2-dihydroisoquinolin-3-yl) ethyl)amino)pyrido[2,3-d]pyrimidin-5(8H)-one
[352] (S)-4-((1-(4-chloro-1-oxo-2-phenyl-8-(1H-tetrazol-5-yl)-1,2-dihydroisoquinolin-3-yl)ethyl)amino)pyrido[2,3-d]pyrimidin-5(8H)-one
[353] (S)-(4-chloro-1-oxo-3-(1-((5-oxo-5,8-dihydropyrido[2,3-d]pyrimidin-4-yl) amino)ethyl)-2-phenyl-1,2-dihydroisoquinolin-8-yl)boronic acid
[354] (S)-4-((1-(4-chloro-8-ethyl-1-oxo-2-phenyl-1,2-dihydroisoquinolin-3-yl)ethyl)amino)pyrido[2,3-d]pyrimidin-5(8H)-one
[355] (S)-4-((1-(4-chloro-8-(hydroxymethyl)-1-oxo-2-phenyl-1,2-dihydroisoquinolin-3-yl) ethyl)amino)pyrido[2,3-d]pyrimidin-5(8H)-one
[356] 4-(((1S)-1-(4-chloro-1-oxo-2-phenyl-8-(thiazolidin-2-yl)-1,2-dihydroisoquinolin-3-yl)ethyl)amino)pyrido[2,3-d]pyrimidin-5(8H)-one
[357] 4-(((1S)-1-(4-chloro-8-(1-hydroxy-2,2-dimethylpropyl)-1-oxo-2-phenyl-1,2-dihydroisoquinolin-3-yl)ethyl)amino)pyrido[2,3-d]pyrimidin-5(8H)-one
[358] (S)-4-((1-(4-chloro-8-(5,5-dimethyl-1,3-dioxan-2-yl)-1-oxo-2-phenyl-1,2-dihydroisoquinolin-3-yl)ethyl)amino)pyrido[2,3-d]pyrimidin-5(8H)-one
[359] (S)-4-((1-(4-chloro-8-((3-hydroxy-2,2-dimethylpropoxy)methyl)-1-oxo-2-phenyl-1,2-dihydroisoquinolin-3-yl)ethyl)amino)pyrido[2,3-d]pyrimidin-5(8H)-one
[360] (S)-4-((1-(4-chloro-8-(2-hydroxypropan-2-yl)-1-oxo-2-phenyl-1,2-dihydroisoquinolin-3-yl) ethyl)amino)pyrido[2,3-d]pyrimidin-5(8H)-one
[361] 4-(((S)-1-(4-chloro-8-((R)-1-hydroxyethyl)-1-oxo-2-phenyl-1,2-dihydroisoquinolin-3-yl) ethyl)amino)pyrido[2,3-d]pyrimidin-5(8H)-one (having a diastereomeric relationship with compound no. 441)
[362] (S)-4-((1-(8-(benzyloxy)-4-chloro-1-oxo-2-phenyl-1,2-dihydroisoquinolin-3-yl)ethyl)amino)pyrido[2,3-d]pyrimidin-5(8H)-one
[363] (S)-4-((1-(4-chloro-8-(2-methoxyethoxy)-1-oxo-2-phenyl-1,2-dihydroisoquinolin-3-yl) ethyl)amino)pyrido[2,3-d]pyrimidin-5(8H)-one
[364] (S)-4-((1-(4-chloro-1-oxo-2-phenyl-8-(2,2,2-trifluoroethoxy)-1,2-dihydroisoquinolin-3-yl)ethyl)amino)pyrido[2,3-d]pyrimidin-5(8H)-one
[365] (S)-4-((1-(4-chloro-8-methoxy-1-oxo-2-phenyl-1,2-dihydroisoquinolin-3-yl)ethyl)amino)pyrido[2,3-d]pyrimidin-5(8H)-one
[366] (S)-4-((1-(4-chloro-8-(2-methoxyphenoxy)-1-oxo-2-phenyl-1,2-dihydroisoquinolin-3-yl) ethyl)amino)pyrido[2,3-d]pyrimidin-5(8H)-one
[367] (S)-4-((1-(4-chloro-1-oxo-2-phenyl-8-(p-tolyloxy)-1,2-dihydroisoquinolin-3-yl) ethyl)amino)pyrido[2,3-d]pyrimidin-5(8H)-one
[368] 4-(((1S)-1-(4-chloro-1-oxo-2-phenyl-8-(1-(pyridin-4-yl)ethoxy)-1,2-dihydroisoquinolin-3-yl)ethyl)amino)pyrido[2,3-d]pyrimidin-5(8H)-one
[369] (S)-4-((1-(4-chloro-8-((4-chlorobenzyl)thio)-1-oxo-2-phenyl-1,2-dihydroisoquinolin-3-yl) ethyl)amino)pyrido[2,3-d]pyrimidin-5(8H)-one
[370] (S)-4-((1-(4-chloro-1-oxo-2-phenyl-8-(pyridin-4-ylthio)-1,2-dihydroisoquinolin-3-yl) ethyl)amino)pyrido[2,3-d]pyrimidin-5(8H)-one
[371] (S)-4-((1-(4-chloro-8-((4-chlorophenyl)thio)-1-oxo-2-phenyl-1,2-dihydroisoquinolin-3-yl) ethyl)amino)pyrido[2,3-d]pyrimidin-5(8H)-one
[372] (S)-4-((1-(4-chloro-1-oxo-2-phenyl-8-(propylthio)-1,2-dihydroisoquinolin-3-yl) ethyl)amino)pyrido[2,3-d]pyrimidin-5(8H)-one
[373] (S)-6-(4-chloro-1-oxo-3-(1-((5-oxo-5,8-dihydropyrido[2,3-d]pyrimidin-4-yl)amino)ethyl)-2-phenyl-1,2-dihydroisoquinolin-8-yl)-2,2-dimethyl-2H-benzo[b] [1,4]oxazin-3(5H)-one
[374] (S)-4-((1-(4-chloro-1-oxo-2-phenyl-1,2-dihydroisoquinolin-3-yl)ethyl)amino)-8-(2-methoxypyridin-4-yl)pyrido[2,3-d]pyrimidin-5(8H)-one
[375] (S)-8-(5-bromo-2-methoxypyridin-4-yl)-4-((1-(4-chloro-1-oxo-2-phenyl-1,2-dihydroisoquinolin-3-yl)ethyl)amino)pyrido[2,3-d]pyrimidin-5(8H)-one
[376] (S)-4-((1-(4-chloro-8-(5-isopropoxypyrazin-2-yl)-1-oxo-2-phenyl-1,2-dihydroisoquinolin-3-yl)ethyl)amino)pyrido[2,3-d]pyrimidin-5(8H)-one
[377] (S)-4-((1-(4-chloro-1-oxo-2-phenyl-8-(2-(thiophen-2-yl)pyrimidin-5-yl)-1,2-dihydroisoquinolin-3-yl)ethyl)amino)pyrido[2,3-d]pyrimidin-5(8H)-one
[378] (S)-4-((1-(4-chloro-8-(2-(furan-2-yl)pyrimidin-5-yl)-1-oxo-2-phenyl-1,2-dihydroisoquinolin-3-yl)ethyl)amino)pyrido[2,3-d]pyrimidin-5(8H)-one
[379] (S)-4-((1-(4-chloro-8-(2-ethylpyrimidin-5-yl)-1-oxo-2-phenyl-1,2-dihydroisoquinolin-3-yl) ethyl)amino)pyrido[2,3-d]pyrimidin-5(8H)-one
[380] (S)-4-((1-(4-chloro-8-(1H-indol-2-yl)-1-oxo-2-phenyl-1,2-dihydroisoquinolin-3-yl) ethyl)amino)pyrido[2,3-d]pyrimidin-5(8H)-one
[381] (S)-4-((1-(4-chloro-8-(2-isopropylpyrimidin-5-yl)-1-oxo-2-phenyl-1,2-dihydroisoquinolin-3-yl)ethyl)amino)pyrido[2,3-d]pyrimidin-5(8H)-one
[382] (S)-4-((1-(4-chloro-8-(2-fluoropyrimidin-5-yl)-1-oxo-2-phenyl-1,2-dihydroisoquinolin-3-yl) ethyl)amino)pyrido[2,3-d]pyrimidin-5(8H)-one
[383] (S)-4-((1-(8-(2-methoxypyrimidin-5-yl)-1-oxo-2-phenyl-1,2-dihydroisoquinolin-3-yl) ethyl)amino)pyrido[2,3-d]pyrimidin-5(8H)-one
[384] (S)-4-((1-(8-(2-ethoxypyrimidin-5-yl)-1-oxo-2-phenyl-1,2-dihydroisoquinolin-3-yl) ethyl)amino)pyrido[2,3-d]pyrimidin-5(8H)-one
[385] (S)-4-((1-(1-oxo-2-phenyl-8-(2-(trifluoromethyl)pyridin-5-yl)-1,2-dihydroisoquinolin-3-yl)ethyl)amino)pyrido[2,3-d]pyrimidin-5(8H)-one
[386] (S)-4-((1-(8-(2-(2-hydroxypropan-2-yl)pyrimidin-5-yl)-1-oxo-2-phenyl-1,2-dihydroisoquinolin-3-yl)ethyl)amino)pyrido[2,3-d]pyrimidin-5(8H)-one
[387] (S)-4-((1-(4-chloro-8-(5-methylthiophen-2-yl)-1-oxo-2-phenyl-1,2-dihydroisoquinolin-3-yl) ethyl)amino)pyrido[2,3-d]pyrimidin-5(8H)-one
[388] (S)-4-((1-(4-chloro-1-oxo-2-phenyl-8-(thiophen-2-yl)-1,2-dihydroisoquinolin-3-yl)ethyl)amino)pyrido[2,3-d]pyrimidin-5(8H)-one
[389] (S)-4-((1-(4-chloro-1-oxo-2-phenyl-8-(trifluoromethyl)-1,2-dihydroisoquinolin-3-yl)ethyl)amino)pyrido[2,3-d]pyrimidin-5(8H)-one
[390] (S)-4-((1-(4-chloro-8-(methylsulfonyl)-1-oxo-2-phenyl-1,2-dihydroisoquinolin-3-yl) ethyl)amino)pyrido[2,3-d]pyrimidin-5(8H)-one
[391] (S)-4-((1-(4-chloro-1-oxo-2-phenyl-8-(pyridin-4-ylsulfonyl)-1,2-dihydroisoquinolin-3-yl) ethyl)amino)pyrido[2,3-d]pyrimidin-5(8H)-one
[392] (S)-4-((1-(4-chloro-8-(2-(methylsulfonyl)pyrimidin-5-yl)-1-oxo-2-phenyl-1,2-dihydroisoquinolin-3-yl)ethyl)amino)pyrido[2,3-d]pyrimidin-5(8H)-one
[393] (S)-4-((1-(4-chloro-8-((4-chlorophenyl)sulfonyl)-1-oxo-2-phenyl-1,2-dihydroisoquinolin-3-yl)ethyl)amino)pyrido[2,3-d]pyrimidin-5(8H)-one
[394] 4-(((S)-1-(4-chloro-1-oxo-2-phenyl-8-(((S)-4-(trifluoromethoxy)phenyl)sulfinyl)-1,2-dihydroisoquinolin-3-yl)ethyl)amino)pyrido[2,3-d]pyrimidin-5(8H)-one (having a diastereomeric relationship with compound no. 395)
[395] 4-(((S)-1-(4-chloro-1-oxo-2-phenyl-8-(((R)-4-(trifluoromethoxy)phenyl)sulfinyl)-1,2-dihydroisoquinolin-3-yl)ethyl)amino)pyrido[2,3-d]pyrimidin-5(8H)-one (having a diastereomeric relationship with compound no. 394)
[396] 4-(((S)-1-(4-chloro-1-oxo-2-phenyl-8-((S)-propylsulfinyl)-1,2-dihydroisoquinolin-3-yl)ethyl)amino)pyrido[2,3-d]pyrimidin-5(8H)-one (having a diastereomeric relationship with compound no. 397)
[397] 4-(((S)-1-(4-chloro-1-oxo-2-phenyl-8-((R)-propylsulfonyl)-1,2-dihydroisoquinolin-3-yl)ethyl)amino)pyrido[2,3-d]pyrimidin-5(8H)-one (having a diastereomeric relationship with compound no. 396)
[398] (S)-4-((1-(4-chloro-8-((4-fluorophenyl)thio)-1-oxo-2-phenyl-1,2-dihydroisoquinolin-3-yl) ethyl)amino)pyrido[2,3-d]pyrimidin-5(8H)-one
[399] (S)-4-((1-(4-chloro-8-((4-fluorophenyl)sulfonyl)-1-oxo-2-phenyl-1,2-dihydroisoquinolin-3-yl)ethyl)amino)pyrido[2,3-d]pyrimidin-5(8H)-one
[400] (S)-4-((1-(8-((1H-1,2,4-triazol-3-yl)thio)-4-chloro-1-oxo-2-phenyl-1,2-dihydroisoquinolin-3-yl)ethyl)amino)pyrido[2,3-d]pyrimidin-5(8H)-one
[401] (S)-4-((1-(8-((1H-1,2,4-triazol-3-yl)sulfonyl)-4-chloro-1-oxo-2-phenyl-1,2-dihydroisoquinolin-3-yl)ethyl)amino)pyrido[2,3-d]pyrimidin-5(8H)-one
[402] (S)-4-((1-(4-chloro-8-(2-chloropyrimidin-5-yl)-1-oxo-2-phenyl-1,2-dihydroisoquinolin-3-yl) ethyl)amino)pyrido[2,3-d]pyrimidin-5(8H)-one
[403] (S)-4-((1-(8-(2-acetylpyrimidin-5-yl)-4-chloro-1-oxo-2-phenyl-1,2-dihydroisoquinolin-3-yl) ethyl)amino)pyrido[2,3-d]pyrimidin-5(8H)-one
[404] (S)-4-((1-(4-chloro-8-(2-(2-ethoxypropan-2-yl)pyrimidin-5-yl)-1-oxo-2-phenyl-1,2-dihydroisoquinolin-3-yl)ethyl)amino)pyrido[2,3-d]pyrimidin-5(8H)-one
[405] (S)-4-((1-(4-chloro-8-(2-(2-methoxypropan-2-yl)pyrimidin-5-yl)-1-oxo-2-phenyl-1,2-dihydroisoquinolin-3-yl)ethyl)amino)pyrido[2,3-d]pyrimidin-5(8H)-one
[406] (S)-4-((1-(4-chloro-1-oxo-2-phenyl-8-(2-(2-propoxypropan-2-yl)pyrimidin-5-yl)-1,2-dihydroisoquinolin-3-yl)ethyl)amino)pyrido[2,3-d]pyrimidin-5(8H)-one
[407] 4-(((1S)-1-(4-chloro-8-(2-(S-methylsulfonimidoyl)pyrimidin-5-yl)-1-oxo-2-phenyl-1,2-dihydroisoquinolin-3-yl)ethyl)amino)pyrido[2,3-d]pyrimidin-5(8H)-one
[408] 4-(((S)-1-(4-chloro-1-oxo-2-phenyl-8-((R)-propylsulfonimidoyl)-1,2-dihydroisoquinolin-3-yl)ethyl)amino)pyrido[2,3-d]pyrimidin-5(8H)-one (having a diastereomeric relationship with compound no. 409)
[409] 4-(((S)-1-(4-chloro-1-oxo-2-phenyl-8-((S)-propylsulfonimidoyl)-1,2-dihydroisoquinolin-3-yl)ethyl)amino)pyrido[2,3-d]pyrimidin-5(8H)-one (having a diastereomeric relationship with compound no. 408)
[410] 4-(((S)-1-(4-chloro-1-oxo-2-phenyl-8-((R)-propan-2-ylsulfonimidoyl)-1,2-dihydroisoquinolin-3-yl)ethyl)amino)pyrido[2,3-d]pyrimidin-5(8H)-one (having a diastereomeric relationship with compound no. 411)
[411] 4-(((S)-1-(4-chloro-1-oxo-2-phenyl-8-((S)-propan-2-ylsulfonimidoyl)-1,2-dihydroisoquinolin-3-yl)ethyl)amino)pyrido[2,3-d]pyrimidin-5(8H)-one (having a diastereomeric relationship with compound no. 410)
[412] (S)-4-((1-(4-chloro-1-oxo-2-phenyl-8-(pyridin-2-ylthio)-1,2-dihydroisoquinolin-3-yl)ethyl)amino)pyrido[2,3-d]pyrimidin-5(8H)-one
[413] 4-(((1S)-1-(4-chloro-1-oxo-2-phenyl-8-(pyridin-2-ylsulfinyl)-1,2-dihydroisoquinolin-3-yl) ethyl)amino)pyrido[2,3-d]pyrimidin-5(8H)-one
[414] (S)-4-((1-(4-chloro-1-oxo-2-phenyl-8-(pyridin-2-ylsulfonyl)-1,2-dihydroisoquinolin-3-yl) ethyl)amino)pyrido[2,3-d]pyrimidin-5(8H)-one
[415] (S)-4-((1-(4-chloro-8-(isopropylthio)-1-oxo-2-phenyl-1,2-dihydroisoquinolin-3-yl)ethyl)amino)pyrido[2,3-d]pyrimidin-5(8H)-one
[416] (S)-4-((1-(4-chloro-8-(isopropylsulfonyl)-1-oxo-2-phenyl-1,2-dihydroisoquinolin-3-yl) ethyl)amino)pyrido[2,3-d]pyrimidin-5(8H)-one
[417] (S)-4-((1-(4-chloro-1-oxo-2-phenyl-8-(thiazol-2-ylthio)-1,2-dihydroisoquinolin-3-yl) ethyl)amino)pyrido[2,3-d]pyrimidin-5(8H)-one
[418] (S)-4-((1-(4-chloro-8-(oxatan-3-ylthio)-1-oxo-2-phenyl-1,2-dihydroisoquinolin-3-yl)ethyl)amino)pyrido[2,3-d]pyrimidin-5(8H)-one
[419] 4-(((1S)-1-(4-chloro-1-oxo-2-phenyl-8-(pyrimidin-2-ylsulfinyl)-1,2-dihydroisoquinolin-3-yl)ethyl)amino)pyrido[2,3-d]pyrimidin-5(8H)-one
[420] (S)-4-((1-(4-chloro-1-oxo-2-phenyl-8-(pyrimidin-2-ylsulfonyl)-1,2-dihydroisoquinolin-3-yl)ethyl)amino)pyrido[2,3-d]pyrimidin-5(8H)-one
[421] 4-(((S)-1-(4-chloro-1-oxo-2-phenyl-8-((S)-pyrimidin-2-sulfonimidoyl)-1,2-dihydroisoquinolin-3-yl)ethyl)amino)pyrido[2,3-d]pyrimidin-5(8H)-one (having a diastereomeric relationship with compound no. 422)
[422] 4-(((S)-1-(4-chloro-1-oxo-2-phenyl-8-((R)-pyrimidine-2-sulfonimidoyl)-1,2-dihydroisoquinolin-3-yl)ethyl)amino)pyrido[2,3-d]pyrimidin-5(8H)-one (having a diastereomeric relationship with compound no. 421)
[423] (S)-4-((1-(4-chloro-1-oxo-2-phenyl-8-(pyrimidine-2-ylthio)-1,2-dihydroisoquinolin-3-yl) ethyl)amino)pyrido[2,3-d]pyrimidin-5(8H)-one
[424] (S)-4-((1-(8-(2-(tert-butoxy)pyrimidin-5-yl)-4-chloro-1-oxo-2-phenyl-1,2-dihydroisoquinolin-3-yl)ethyl)amino)pyrido[2,3-d]pyrimidin-5(8H)-one
[425] 4-(((1S)-1-(4-chloro-8-(S-methylsulfonimidoyl)-1-oxo-2-phenyl-1,2-dihydroisoquinolin-3-yl) ethyl)amino)pyrido[2,3-d]pyrimidin-5(8H)-one
[426] (S)-4-((1-(8-(tert-butylthio)-4-chloro-1-oxo-2-phenyl-1,2-dihydroisoquinolin-3-yl) ethyl)amino)pyrido[2,3-d]pyrimidin-5(8H)-one
[427] (S)-4-((1-(4-chloro-8-mercapto-1-oxo-2-phenyl-1,2-dihydroisoquinolin-3-yl)ethyl)amino)pyrido[2,3-d]pyrimidin-5(8H)-one
[428] (S)-4-((1-(4-chloro-1-oxo-2-phenyl-8-(pyridin-4-yloxy)-1,2-dihydroisoquinolin-3-yl)ethyl)amino)pyrido[2,3-d]pyrimidin-5(8H)-one
[429] (S)-4-((1-(4-chloro-1-oxy-2-phenyl-8-(pyridin-4-ylmethoxy)-1,2-dihydroisoquinolin-3-yl) ethyl)amino)pyrido[2,3-d]pyrimidin-5(8H)-one
[430] (S)-4-((1-(4-chloro-1-oxo-2-phenyl-8-(pyridin-2-ylmethoxy)-1,2-dihydroisoquinolin-3-yl)ethyl)amino)pyrido[2,3-d]pyrimidin-5(8H)-one
[431] 4-(((1S)-1-(4-chloro-8-(oxetan-3-ylsulfinyl)-1-oxo-2-phenyl-1,2-dihydroisoquinolin-3-yl) ethyl)amino)pyrido[2,3-d]pyrimidin-5(8H)-one
[432] (S)-4-((1-(4-chloro-8-(oxetan-3-ylsulfonyl)-1-oxo-2-phenyl-1,2-dihydroisoquinolin-3-yl) ethyl)amino)pyrido[2,3-d]pyrimidin-5(8H)-one
[433] (S)-4-((1-(8-(tert-butylsulfonyl)-4-chloro-1-oxo-2-phenyl-1,2-dihydroisoquinolin-3-yl) ethyl)amino)pyrido[2,3-d]pyrimidin-5(8H)-one
[434] (S)-4-((1-(4-chloro-8-(ethylthio)-1-oxo-2-phenyl-1,2-dihydroisoquinolin-3-yl)ethyl)amino)pyrido[2,3-d]pyrimidin-5(8H)-one
[435] 4-(((S)-1-(4-chloro-8-((R)-ethylsulfonimidoyl)-1-oxo-2-phenyl-1,2-dihydroisoquinolin-3-yl) ethyl)amino)pyrido[2,3-d]pyrimidin-5(8H)-one (having a diastereomeric relationship with compound no. 436)
[436] 4-(((S)-1-(4-chloro-8-((S)-ethylsulfonimidoyl)-1-oxo-2-phenyl-1,2-dihydroisoquinolin-3-yl) ethyl)amino)pyrido[2,3-d]pyrimidin-5(8H)-one (having a diastereomeric relationship with compound no. 435)
[437] (S)-4-((1-(4-chloro-1-oxo-2-phenyl-8-(pyridin-3-yloxy)-1,2-dihydroisoquinolin-3-yl)ethyl)amino)pyrido[2,3-d]pyrimidin-5(8H)-one
[438] (S)-4-((1-(4-chloro-8-(2-(1-hydroxycyclobutyl)pyrimidin-5-yl)-1-oxo-2-phenyl-1,2-dihydroisoquinolin-3-yl)ethyl)amino)pyrido[2,3-d]pyrimidin-5(8H)-one
[439] (S)-4-chloro-1-oxo-3-(1-((5-oxo-5,8-dihydropyrido[2,3-d]pyrimidin-4-yl) amino)ethyl)-2-phenyl-N-(pyridin-4-ylmethyl)-1,2-dihydroisoquinoline-9-carboxamide
[440] (S)-4-chloro-1-oxo-3-(1-((5-oxo-5,8-dihydropyrido[2,3-d]pyrimidin-4-yl) amino)ethyl)-N,2-diphenyl-1,2-dihydroisoquinoline-9-carboxamide
[441] 4-(((S)-1-(4-chloro-8-((S)-1-hydroxyethyl)-1-oxo-2-phenyl-1,2-dihydroisoquinolin-3-yl) ethyl)amino)pyrido[2,3-d]pyrimidin-5(8H)-one (having a diastereomeric relationship with compound no. 361)

11. A pharmaceutical composition comprising, the pyrido-pyrimidine derivative, isomers thereof or pharmaceutically acceptable salts thereof, solvates thereof or hydrates thereof according to any one of claims 1 to 10, as an effective ingredient.

12. The pharmaceutical composition according to claim 11, wherein the pharmaceutical composition is for preventing or treating PI3 kinase-associated diseases.

13. The pharmaceutical composition according to claim 11, wherein the pyrido-pyrimidine derivative, isomers thereof or pharmaceutically acceptable salts thereof, solvates thereof or hydrates thereof inhibit at least one kinase of PI3K-δ and DNA-PK.

14. The pharmaceutical composition according to claim 12, wherein the PI3 kinase-associated disease is at least one selected from the group consisting of cancer diseases comprising liquid hematologic malignancy, ovarian cancer, cervical cancer, breast cancer, colorectal cancer, liver cancer, stomach cancer, pancreatic cancer, colon cancer, peritoneal metastatic cancer, skin cancer, bladder cancer, prostate cancer, lung cancer, osteosarcoma, fibrous tumor or brain tumor; autoimmune diseases comprising rheumatoid arthritis, systemic lupus erythematosus, multiple sclerosis, diabetes, hyperthyroidism, myasthenia or autoimmune pernicious anemia; and respiratory diseases comprising chronic obstructive pulmonary disease (COPD), rhinitis, asthma, chronic bronchitis, chronic pulmonary inflammatory disease, pleurisy, alveolitis, vasculitis, emphysema, pneumonia or bronchiectasis.

15. A method for preventing or treating PI3 kinase-associated diseases, the method comprising administering a therapeutically effective amount of the pyrido-pyrimidine derivative, isomers thereof or pharmaceutically acceptable salts thereof, solvates thereof or hydrates thereof according to any one of claims 1 to 10, into a subject.

16. Use of the pyrido-pyrimidine derivative, isomers thereof or pharmaceutically acceptable salts thereof, solvates thereof or hydrates thereof according to any one of claims 1 to 10, for preventing or treating PI3 kinase-associated diseases.

17. Use of the pyrido-pyrimidine derivative, isomers thereof or pharmaceutically acceptable salts thereof, solvates thereof or hydrates thereof according to any one of claims 1 to 10, in the manufacture of a medicament for treating PI3 kinase-associated diseases.
